(19) Europäisches Patentamt
European Patent Office
Office européen des brevets

(11) Publication number: 0 342 456 A2

(12) **EUROPEAN PATENT APPLICATION**

(21) Application number: 89108194.5

(22) Date of filing: 05.05.89

(51) Int. Cl.4: C07D 417/12 , A01N 47/36 , //C07D283/00,C07D285/00

(30) Priority: 10.05.88 JP 113029/88
04.10.88 JP 250400/88
24.12.88 JP 327199/88

(43) Date of publication of application: 23.11.89 Bulletin 89/47

(84) Designated Contracting States: AT BE CH DE ES FR GB GR IT LI LU NL SE

(71) Applicant: NISSAN CHEMICAL INDUSTRIES LTD.
7-1, 3-chome Kanda-Nishiki-cho
Chiyoda-ku Tokyo(JP)

(72) Inventor: Makino, Kenji Nissan Chemical Industries Ltd.
Chuo Kenkyusho, 722-1, Tsuboi-cho
Funabashi-shi Chiba-ken(JP)
Inventor: Sato, Toshiaki Nissan Chemical Industries Ltd.
Chuo Kenkyusho, 722-1, Tsuboi-cho
Funabashi-shi Chiba-ken(JP)
Inventor: Morimoto, Katsushi Nissan Chemical Industries Ltd.
Chuo Kenkyusho, 722-1, Tsuboi-cho
Funabashi-shi Chiba-ken(JP)
Inventor: Akiyama, Shigeaki Nissan Chemical Industries Ltd.
Chuo Kenkyusho, 722-1, Tsuboi-cho
Funabashi-shi Chiba-ken(JP)
Inventor: Suzuki, Hideaki Nissan Chemical Industries Ltd.
Chuo Kenkyusho, 722-1, Tsuboi-cho
Funabashi-shi Chiba-ken(JP)
Inventor: Suzuki, Koichi Nissan Chemical Industries Ltd.
Seibutsukagaku Kenkyusho 1470
Oaza-shiraoka
Shiraoka-machi Minamisaitama-gun
Saitama(JP)
Inventor: Nawamaki, Tsutomu Nissan Chemical Industries Ltd.
Seibutsukagaku Kenkyusho 1470
Oaza-shiraoka
Shiraoka-machi Minamisaitama-gun
Saitama(JP)
Inventor: Watanabe, Shigeomi Nissan Chemical Industries Ltd.
Seibutsukagaku Kenkyusho 1470
Oaza-shiraoka
Shiraoka-machi Minamisaitama-gun
Saitama(JP)

(74) Representative: Wächtershäuser, Günter, Dr.
Tal 29
D-8000 München 2(DE)

(54) **Sultamsulfonamide derivatives and herbicides.**

(57) A sultamsulfonamide derivative having the formula:

EP 0 342 456 A2

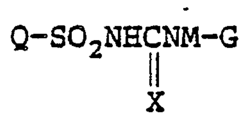

(I)

wherein Q is

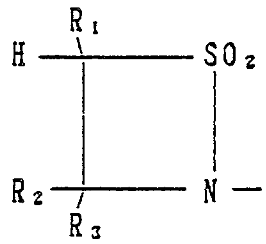

,

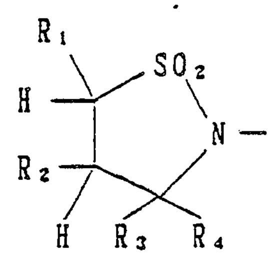

,

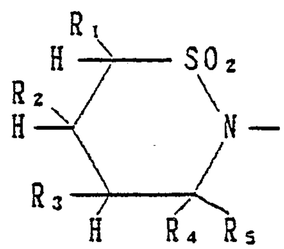

,

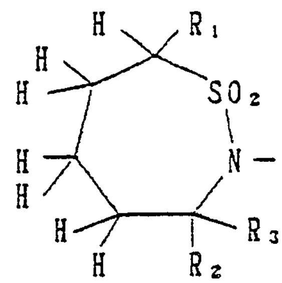

or

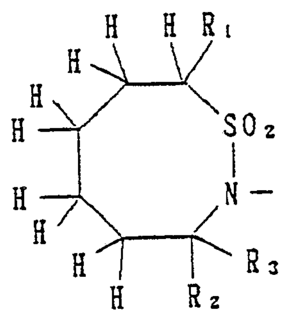

wherein each of $R_1$, $R_2$, $R_3$, $R_4$ and $R_5$ is hydrogen, $C_1$-$C_6$ alkyl, $C_2$-$C_6$ alkenyl, $C_2$-$C_6$ alkynyl, halogen, trifluoromethyl, nitro, cyano, amino, $C_1$-$C_6$ alkoxy, phenoxy (provided that this phenoxy may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylthio, phenylthio (provided that this phenylthio may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylsulfonyl, benzenesulfonyl (provided that this benzenesulfonyl may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylcarbonyl, carboxylic acid, $C_1$-$C_6$ alkoxycarbonyl, $C_1$-$C_6$ monoalkylaminocarbonyl, $C_1$-$C_6$ dialkylaminocarbonyl, $C_2$-$C_6$ acylamino, $C_1$-$C_6$ halogenoalkyl, $C_1$-$C_6$ alkyl substituted by cyano, $C_1$-$C_6$ alkyl substituted by hydroxyl, $C_1$-$C_6$ alkyl substituted by amino, $C_1$-$C_6$ alkyl substituted by $C_1$-$C_6$ alkoxy, $C_1$-$C_6$ alkyl substituted by $C_1$-$C_6$ halogenoalkoxy, $C_1$-$C_6$ alkyl substituted by $C_1$-$C_6$ alkylthio, $C_1$-$C_6$ alkyl substituted by $C_1$-$C_6$ alkylsulfonyl, $C_1$-$C_6$ alkyl substituted by $C_1$-$C_6$ alkylcarbonyl, $C_1$-$C_6$ alkyl substituted by carboxylic acid, $C_1$-$C_6$ alkyl substituted by $C_1$-$C_6$ alkoxycarbonyl, $C_1$-$C_6$ alkyl substituted by $C_2$-$C_6$ alkenyloxy, $C_1$-$C_6$ alkyl substituted by $C_2$-$C_6$ alkynyloxy, $C_1$-$C_6$ alkyl substituted by $C_2$-$C_6$ acylamino, $C_1$-$C_6$ alkyl substituted by $C_1$-$C_6$ alkylsulfonyloxy, $C_2$-$C_6$ alkenyl substituted by $C_1$-$C_6$ alkoxycarbonyl, $C_2$-$C_6$ alkynyl substituted by cyano, phenyl (provided that this phenyl may be substituted by halogen, trifluoromethyl, $C_1$-$C_6$ alkyl, $C_1$-$C_6$ alkoxy or $C_1$-$C_6$ alkoxycarbonyl) or $C_1$-$C_6$ alkyl substituted by phenyl (provided that this phenyl may be substituted by halogen, trifluoromethyl, $C_1$-$C_6$ alkyl, $C_1$-$C_6$ alkoxy or $C_1$-$C_6$ alkoxycarbonyl); X is oxygen or sulfur; M is hydrogen, $C_1$-$C_6$ alkyl, $C_2$-$C_6$ alkenyl cr $C_2$-$C_6$ alkynyl; and G is

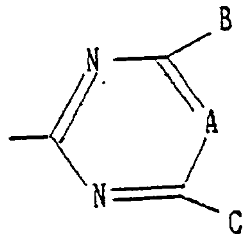

wherein A is CH or nitrogen, and each of B and C is $C_1$-$C_4$ alkyl, $C_1$-$C_4$ alkoxy, $C_1$-$C_4$ halogenoalkyl, $C_1$-$C_4$ halogenoalkoxy, halogen or $C_1$-$C_4$ monoalkylamino, or an agriculturally useful salt thereof.

## SULTAMSULFONAMIDE DERIVATIVES AND HERBICIDES

The present invention relates to novel sultamsulfonamide derivatives and agriculturally useful salts thereof, and herbicides containing such derivatives and salts as active ingredients.

It is essential to employ herbicides for protecting important crop plants such as rice (Oryza sativa), wheat (Triticum vulgare), corn (Zea mays), soybean (Glycine max), cotton (Gossypium spp.) and beet (Beta vulgaris), from weeds and increasing the yield of such crop plants. Recently, it is particularly desired to develop a selective herbicide capable of selectively controlling weeds without adversely affecting crop plants even when applied to the foliages of crop plants and weeds simultaneously in a field where such crop plants and weeds exsist together. Further, with a view to preventing the pollution of environment, reducing costs for transportation and application, researches have been conducted for many years for developping a compound which exhibits high herbicidal effects at a dose as low as possible. Some of compounds having such properties are now practically used as selective herbicides. However, a new compound having such properties is still in demand.

As the prior art having a structure similar to the compound of the present invention, urea compounds having a sulfamide structure are disclosed in Japanese Unexamined Patent Publication No. 48973/1985. However, compounds having a sultam structure like the compounds of the present invention have not been known, and they are novel compounds.

The present inventors have conducted extensive researches to develop a compound having selectivity for important crop plants for many years and have studied the herbicidal properties of many compounds with an aim to find a compound having high herbicidal activities and selectivity. As a result, they have found that sultamsulfonamide derivatives having the formula:

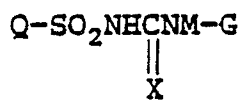 (I)

wherein Q is

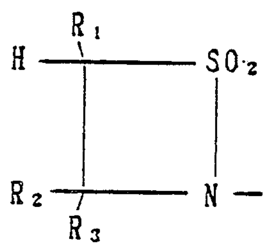 ,

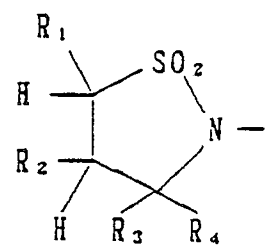 ,

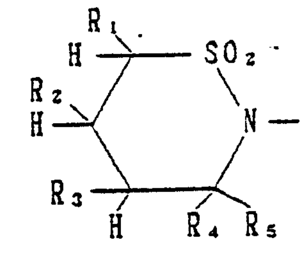
,
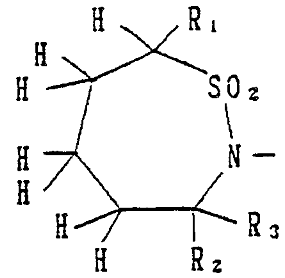
or
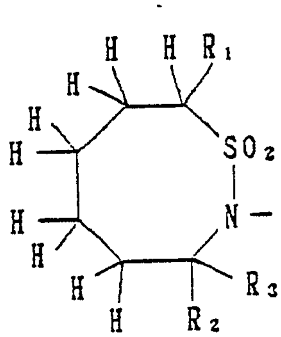

wherein each of $R_1$, $R_2$, $R_3$, $R_4$ and $R_5$ is hydrogen, $C_1$-$C_6$ alkyl, $C_2$-$C_6$ alkenyl, $C_2$-$C_6$ alkynyl, halogen, trifluoromethyl, nitro, cyano, amino, $C_1$-$C_6$ alkoxy, phenoxy (provided that this phenoxy may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylthio, phenylthio (provided that this phenylthio may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylsulfonyl, benzenesulfonyl (provided that this benzenesulfonyl may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylcarbonyl, carboxylic acid, $C_1$-$C_6$ alkoxycarbonyl, $C_1$-$C_6$ monoalkylaminocarbonyl, $C_1$-$C_6$ dialkylaminocarbonyl, $C_2$-$C_6$ acylamino, $C_1$-$C_6$ halogenoalkyl, $C_1$-$C_6$ alkyl substituted by cyano, $C_1$-$C_6$ alkyl substituted by hydroxyl, $C_1$-$C_6$ alkyl substituted by amino, $C_1$-$C_6$ alkyl substituted by $C_1$-$C_6$ alkoxy, $C_1$-$C_6$ alkyl substituted by $C_1$-$C_6$ halogenoalkoxy, $C_1$-$C_6$ alkyl substituted by $C_1$-$C_6$ alkylthio, $C_1$-$C_6$ alkyl substituted by $C_1$-$C_6$ alkylsulfonyl, $C_1$-$C_6$ alkyl substituted by $C_1$-$C_6$ alkylcarbonyl, $C_1$-$C_6$ alkyl substituted by carboxylic acid, $C_1$-$C_6$ alkyl substituted by $C_1$-$C_6$ alkoxycarbonyl, $C_1$-$C_6$ alkyl substituted by $C_2$-$C_6$ alkenyloxy, $C_1$-$C_6$ alkyl substituted by $C_2$-$C_6$ alkynyloxy, $C_1$-$C_6$ alkyl substituted by $C_2$-$C_6$ acylamino, $C_1$-$C_6$ alkyl substituted by $C_1$-$C_6$ alkylsulfonyloxy, $C_2$-$C_6$ alkenyl substituted by $C_1$-$C_6$ alkoxycarbonyl, $C_2$-$C_6$ alkynyl substituted by cyano, phenyl (provided that this phenyl may be substituted by halogen, trifluoromethyl, $C_1$-$C_6$ alkyl, $C_1$-$C_6$ alkoxy or $C_1$-$C_6$ alkoxycarbonyl) or $C_1$-$C_6$ alkyl substituted by phenyl (provided that this phenyl may be substituted by halogen, trifluoromethyl, $C_1$-$C_6$ alkyl, $C_1$-$C_6$ alkoxy or $C_1$-$C_6$ alkoxycarbonyl); X is oxygen or sulfur; M is hydrogen, $C_1$-$C_6$ alkyl, $C_2$-$C_6$ alkenyl or $C_2$-$C_6$ alkynyl; and G is

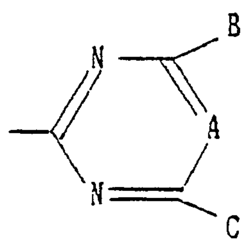

wherein A is CH or nitrogen, and each of B and C is $C_1$-$C_4$ alkyl, $C_1$-$C_4$ alkoxy, $C_1$-$C_4$ halogenoalkyl, $C_1$-$C_4$ halogenoalkoxy, halogen or $C_1$-$C_4$ monoalkylamino, or an agriculturally useful salt thereof, have particularly strong herbicidal activities against many weeds in both soil treatment and foliage treatment and have high safety to wheat (Triticum vulgare), corn (Zea mays), cotton (Gossypium spp.), soybean (Glycine max), beet (Beta vulgaris), rice (Oryza sativa) and the like. The present invention has been accomplished on the basis of this discovery. On the other hand, the compounds of the present invention exhibit high herbicidal activities at an extremely low dose as compared with conventional herbicides, and they are useful as herbicides for orchards or non-agricultural fields.

The compound of the formula I of the present invention can readily be prepared by one of the following

Reaction Schemes 1 to 4.

## Reaction Scheme 1

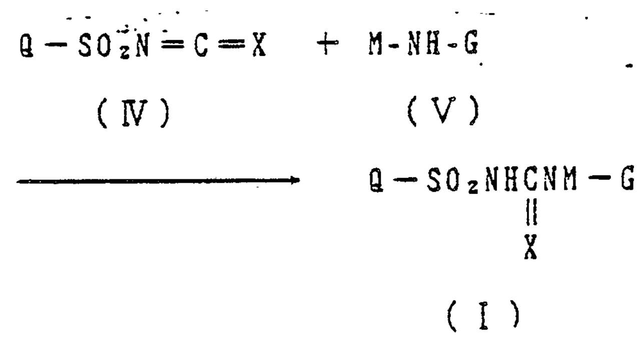

(IV) (V) (I)

In the formulas, Q, G, X and M are as defined above.

Namely, the sultamsulfonyliso(thio)cyanate derivative (IV) is dissolved in a thoroughly dried inert solvent such as dioxane, acetonitrile or acetone, and the pyrimidine or triazine derivative of the formula V is added thereto. In general, the reaction rapidly proceeds under stirring to obtain the compound of the formula I of the present invention. When the reaction hardly proceeds, a small amount of a suitable base such as triethylamine, triethylenediamine, pyridine, sodium alkoxide, sodium hydride or potassium carbonate, may be added, whereby the reaction will readily proceed.

## Reaction Scheme 2

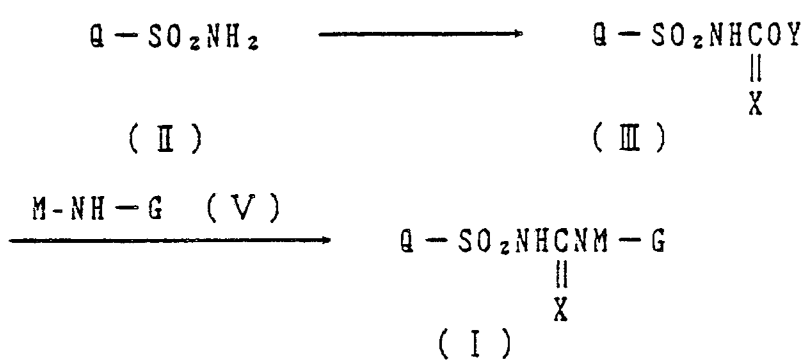


(II) (III) (I)

In the formulas, Q, G, X and M are as defined above, and Y is $C_1$-$C_6$ alkyl or phenyl.

Namely, the sultamsulfonamide derivative (II) is reacted with a chloroformic acid (thio) ester or a carbonic acid (thio) ester in a solvent such as acetone, methyl ethyl ketone or acetonitrile in the presence of a base such as potassium carbonate to obtain the compound (III). Then, the compound (III) is heated with the compound (V) in a solvent such as toluene to obtain the compound (I) of the present invention.

## Reaction Scheme 3

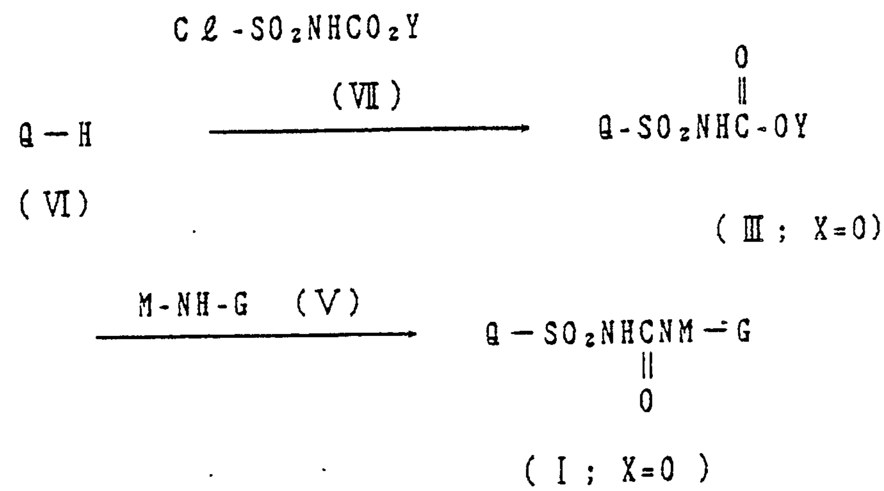


(VI) (III; X=0) (I; X=0)

In the formulas, Q, G, Y and M are as defined above.

The reaction of the sultam (VI) with the phenyl N-chlorosulfonylcarbamate (VII: Y = phenyl) or the alkyl N-chlorosulfonylcarbamate (VII: Y = lower alkyl) is conducted by using 0.5 to 3.0 mols, preferably from 0.9 to 1.2 mols of the carbamate derivative (VII) relative to one mol of the sultam (VI).

The reaction temperature may be selected within a range of from -50 to 100° C, and the preferred range is -20 to 30° C.

This reaction is conducted by using various base. The base is used in an amount of from 0.5 to 4.0 mols relative to one mol of the sultam (VI). As such a base, there may be suitably employed a metal hydride such as sodium hydride, a metal alkoxide such as sodium ethoxide, an alkyl metal such as n-butyl lithium, an organic base such as triethylamine, pyridine or 1,8-diazabicyclo[5,4,0]-7-undecene (DBU] or an inorganic base such as potassium hydroxide or sodium hydroxide.

Particularly preferred are metal hydrides.

As the solvent suitable for this reaction, there may be mentioned a solvent inert to this reaction, for example, an aromatic hydrocarbon such as benzene, toluene or xylene, a halogenated hydrocarbon such as dichloromethane, chloroform or carbontetrachloride, an ether such as ethyl ether, isopropyl ether, dioxane or tetrahydrofuran, a nitrile such as acetonitrile or propionitrile, a hydrocarbon such as petroleum ether, petroleum benzene or hexane, a ketone such as acetone or methyl ethyl ketone, an ester such as ethyl acetate or an amide such as dimethylformamide, dimethylacetamide or hexamethylphosphoric triamide.

These solvents may be used alone or in combination.

Particularly preferred are ethers or amides.

Then, the phenyl N-sultamsulfonylcarbamate (III: X = O and Y = phenyl) or alkyl N-sultamsulfonylcarbamate (III: X = O and Y = lower alkyl) and the compound (V) are heated in a solvent such as benzene or toluene to obtain the compound (I) of the present invention.

## Reaction Scheme 4

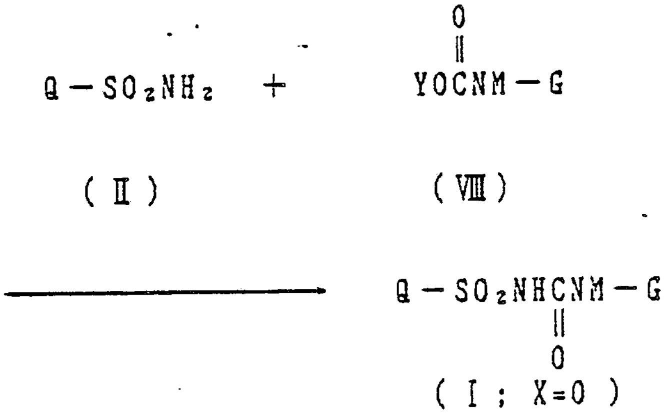

(II) (VIII)

(I; X=O)

In the above formulas, Q, G, Y and M are as defined above.

Namely, the sultamsulfonamide derivative (II) is reacted with the carbamate derivative (VIII) in a solvent such as acetone, acetonitrile or dioxane in the presence of an inorganic base such as potassium carbonate or an organic base such as triethylamine or DBU to obtain the compound (I) of the present invention.

## Reaction Scheme 5

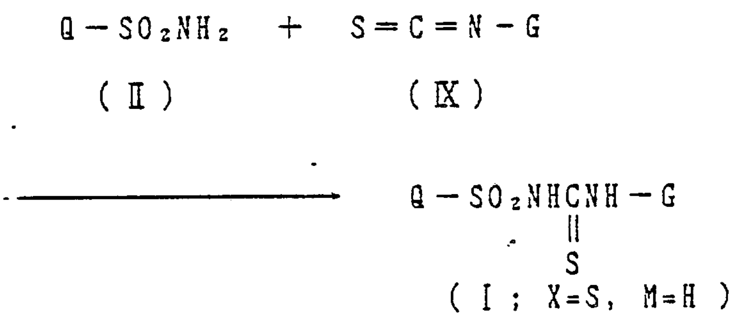

(II) (IX)

(I; X=S, M=H)

In the above formulas, Q and G are as defined above.

Namely, the sultamsulfonamide derivative (II) is reacted with the isothiocyanate derivative (IX) in a solvent such as acetone, acetonitrile or dioxane in the presence of an inorganic base such as potassium carbonate or an organic base such as triethylamine or DBU to obtain the compound (I) of the present invention.

In the formula I, the compound of the present invention wherein M is $C_1$-$C_6$ alkyl, $C_2$-$C_6$ alkenyl or $C_2$-$C_6$ alkynyl, can be prepared by using Reaction Scheme 1, 2, 3 or 4 and can also be prepared by directly alkylating, alkenylating or alkynylating the compound (I: M = H).

The sultamsulfonyliso(thio)cyanate derivative (IV) as the starting material used in Reaction Scheme 1 can be prepared from the sultamsulfonamide derivative (II) in accordance with the method disclosed in European Patent Publication No. 87,780 or Japanese Unexamined Patent Publication No. 13266/1980.

Further, the sultamsulfonylisocyanate (IV: X = O) can also be prepared by Reaction Scheme 6 in accordance with the method disclosed in Japanese Unexamined Patent Publication No. 81320/1974.

Reaction Scheme 6

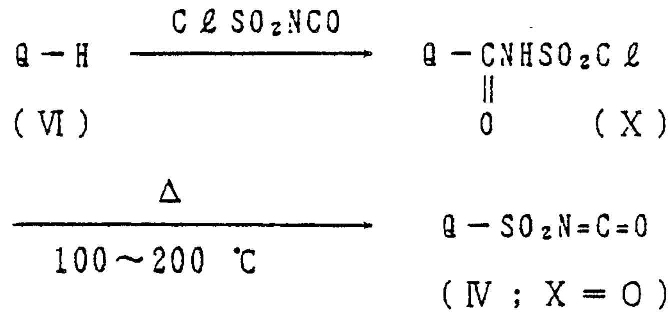


In the formulas, Q is as defined above.

The sultamsulfonamide derivative (II), the phenyl N-sultamsulfonyl(thio)carbamate (III: Y = phenyl) and the alkyl N-sultamsulfonyl(thio)carbamate (III: Y = lower alkyl) which are used as the intermediates of the present invention, are also novel compounds. The sultamsulfonamide derivative (II) can be prepared from the sultam derivative (VI) by Reaction Scheme 7 or 8.

Reaction Scheme 7

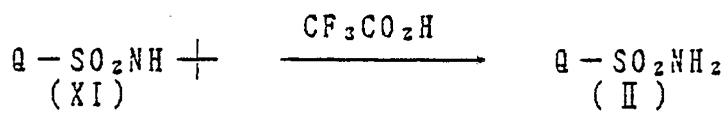


In the formulas, Q is as defined above.

In Reaction Scheme 7, the removal of tert-butyl is conducted by using trifluoroacetic acid.

The amount of trifluoroacetic acid may optionally be selected from equimolar to excessive amount. Trifluoroacetic acid may be used as a solvent.

The reaction temperature may be selected within a range of from -50 to 80 °C. Preferred is a range of from -20 to 30 °C.

When a solvent is used in this reaction, there may be used a solvent inert to this reaction, for example, an aromatic hydrocarbon such as benzene, toluene or xylene, a haloganated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzene or hexane, a ketone such as acetone or methyl ethyl ketone, an ester such as ethyl acetate or an amide such as dimethylformamide, dimethylacetamide or hexamethylphosphoric triamide. These solvents may be used alone or in combination.

## Reaction Scheme 8

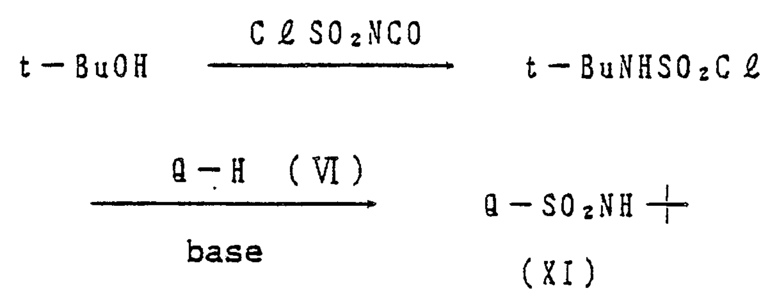


(XI)

In the formulas, Q is as defined above.

In Reaction Scheme 8, the reaction of 2-methyl-2-propanol with chlorosulfonyl isocyanate can be conducted by a method known per se such as the one disclosed in Japanese Unexamined Patent Publication No. 101323/1975.

The reaction of the sultam (VI) with tert-butylsulfamoyl chloride is conducted by using from 0.5 to 3.0 mols of tert-butylsulfamoyl chloride. Preferred is a range of from 0.9 to 1.2 mols.

The reaction temperature may be selected within a range of from -50 to 100° C. Preferred is a range of from -20 to 30° C.

This reaction is conducted by using various bases. The base is used in an amount of from 0.5 to 4.0 mols relative to one mol of the sultam (VI). Preferred is a range of from 0.8 to 2.2 mols. As such a suitable base, there may be employed, for example, a metal hydride such as sodium hydride, a metal alkoxide such as sodium ethoxide, an alkyl metal such as n-butyl lithium, an organic base such as triethylamine, pyridine or 1,8-diazabicyclo[5,4,0]-7-undecene (DBU) or an inorganic base such as potassium hydroxide or sodium hydroxide.

Particularly preferred are metal hydrides.

The solvent suitable for this reaction is a solvent inert to this reaction, for example, an aromatic hydrocarbon such as benzene, toluene or xylene, a halogenated hydrocarbon such as dichloromethane, chloroform or carbon tetrachloride, an ether such as ethyl ether, isopropyl ether, dioxane or tetrahydrofuran, a nitrile such as acetonitrile or propionitrile, a hydrocarbon such as petroleum ether, petroleum benzene or hexane, a ketone such as acetone or methyl ethyl ketone, an ester such as ethyl acetate or an amide such as dimethylformamide, dimethylacetamide or hexamethylphosphoric triamide.

These solvents may be used alone or in combination.

Particularly preferred are ethers or amides.

In Reaction Scheme 3, the phenyl N-chlorosulfonylcarbamate (VII: Y = phenyl) and the alkyl N-chlorosulfonylcarbamate (VII: Y = lower alkyl) can be prepared by a method known per se as disclosed in Chemishe Berichte, Vol. 96, p. 56 (1963).

The sultam (VI) used as the starting material of the above reaction can readily be prepared with reference to Journal of Organic Chemistry, Vol. 28, p.3537 (1963), Journal of Organic Chemistry, Vol. 48, p.537 (1983), Tetrahedron Letters, Vol. 24, p. 2131 (1983), Chemical Abstracts, Vol. 62, 14484g (1965) and German Patent No. 1300933.

As a representative example, preparation of 1,4-butanesultam will be given as Reaction Scheme 9.

Reaction Scheme 9

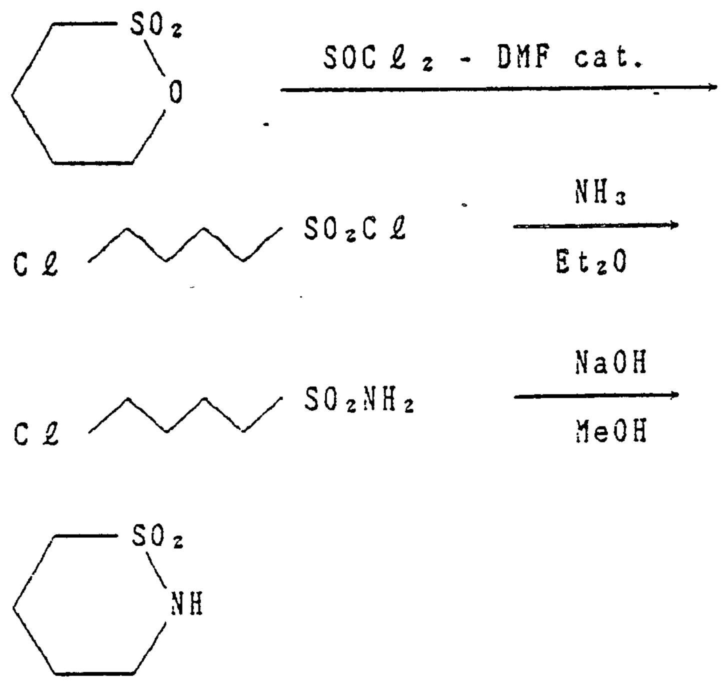


Now, the present invention will be described in further detail with reference to Examples. However, it should be understood that the present invention is by no means restricted to such specific Examples.

EXAMPLE 1

Preparation of 1,4-butanesultam-N-sulfonamide

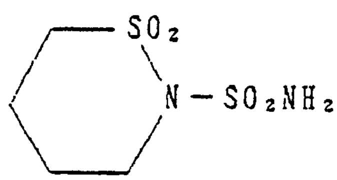

1) To 50 mℓ of dry THF containing 3.23 g (74 mmol) of 55% sodium hydride, 10 g (74 mmol) of 1,4-butanesultam dissolved in 50 mℓ of dry THF was added at room temperature, and the mixture was stirred at room temperature for 14 hours.

Then, the reaction system was cooled to -70°C, and 14.0 g (81.6 mmol) of tert-butylsulfamoyl chloride was dropwise added thereto. Then, the temperature was gradually raised to room temperature, and stirring was continued at room temperature for 24 hours.

The reaction mixture was poured into an aqueous sodium hydrogencarbonate solution-ice and adjusted to pH 6 with concentrated hydrochloric acid. The reaction product was extracted with chloroform.

The chloroform layer was washed with water and then dried over anhydrous magnesium sulfate. Then, the solvent was distilled off under reduced pressure. The residue thereby obtained was washed with n-hexane to obtain 5.3 g of the desired N-tert-butylsulfamoyl-1,4-butanesultam.

Melting point: 87-88°C

2) 5.2 g (19.3 mmol) of N-tert-butylsulfamoyl-1,4-butanesultam was added to 80 mℓ of trifluoroacetic acid, and the mixture was stirred at room temperature for 2 hours. Then, trifluoroacetic acid was distilled off under reduced pressure. Crude solid thereby obtained was washed with diethyl ether and with chloroform to

obtain 1.8 g of the desired 1,4-butanesultam-N-sulfonamide.
Melting point: 130-131 °C

The structures and physical properties of the compounds prepared in the same manner as in Example 1 are shown below.

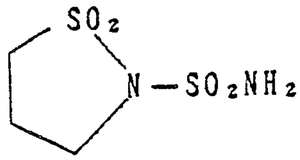

Melting point: 152-153°C

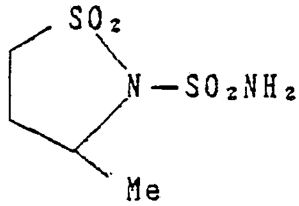

Melting point: 137-138°C

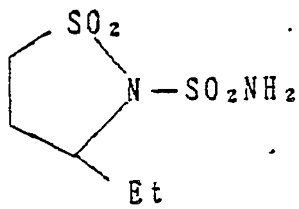

Melting point: 113-114°C

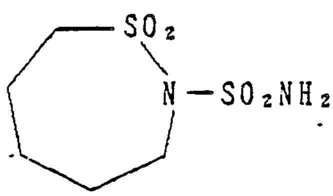

Melting point: 111-112°C

EXAMPLE 2

Preparation of phenyl N-(2,4-butanesultam-N-sulfonyl)carbamate

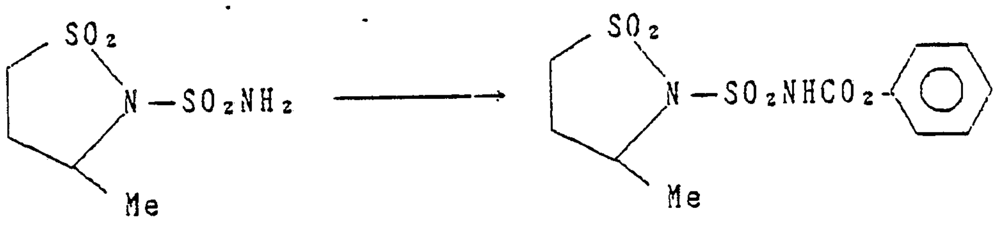

1.0 g (4.67 mmol) of 2,4-butanesultam-N-sulfonamide was dissolved in 10 mℓ of dry acetonitrile, and 970 mg (7.02 mmol) of anhydrous potassium carbonate and 880 mg (5.62 mmol) of phenyl chloroformate were added thereto. The mixture was refluxed under heating for 1 hour.

After cooling, the reaction mixture was poured into 100 mℓ of ice water containing 1.8 g of concentrated hydrochloric acid and extracted with chloroform. The chloroform layer was washed with water and then dried over anhydrous magnesium sulfate. Then, the solvent was distilled off. The residue thereby obtained was washed with isopropyl ether to obtain 1.2 g of the desired phenyl N-(2,4-butanesultam-N-sulfonyl)-carbamate.

Melting point: 125-126 °C

EXAMPLE 3

Preparation of phenyl N-(1-methoxycarbonyl-1,3-propanesultam-N-sulfonyl)carbamate

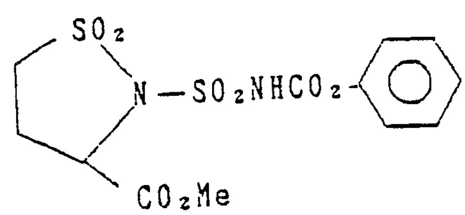

To 100 mℓ of dry THF containing 2.87 g (65.8 mmol) of 55% sodium hydride, 5.6 g (31.3 mmol) of 1-methoxycarbonyl-1,3-propanesultam dissolved in 20 mℓ of dry THF was added under cooling with ice. Then, the mixture was stirred at room temperature for 14 hours under nitrogen atmosphere.

Then, the reaction system was cooled to 0 °C, and 30 mℓ of a dry THF solution cotaining 8.1 g (34.4 mmol) of phenyl N-chlorosulfonylcarbamate was dorpwise added thereto. Then, the temperature was gradually raised to room temperature, and stirring was continued at room temperature for 3 hours.

The reaction mixture was poured into 500 mℓ of ice water containing 10.4 g of concentrated hydrochloric acid and extracted with diethyl ether. The diethyl ether layer was washed sequentially with water and with an aqueous saturated sodium chloride solution and then dried over anhydrous magnesium sulfate. Then, the solvent was distilled off under reduced pressure. The residue thereby obtained was washed with benzene to obtain 10.0 g of the desired phenyl N-(1-methoxycarbonyl-1,3-propanesultam-N-sulfonyl)carbamate.

Melting point: 84-85 °C

The structures and physical properties of the compounds prepared in the same manner as in Examples 2 and 3 are shown below.

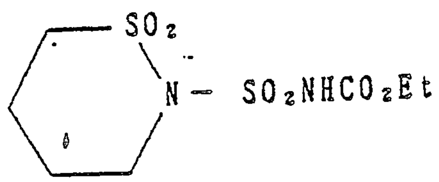

(solid)

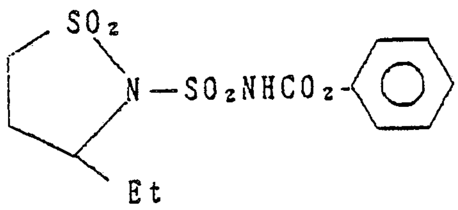

(oil)

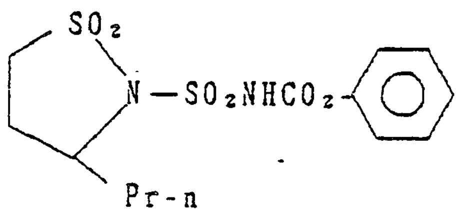

(oil)

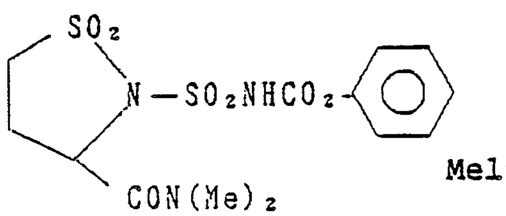

Melting point: 70-71°C

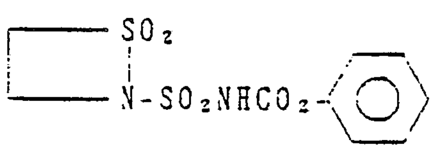

Melting point: 92-93°C

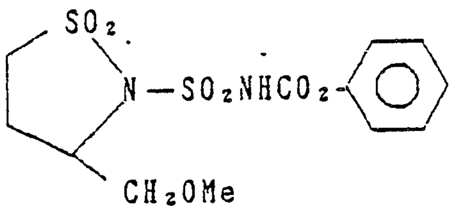

(oil)

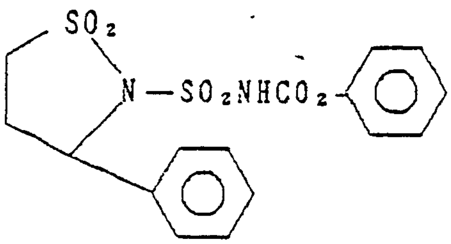

(oil)

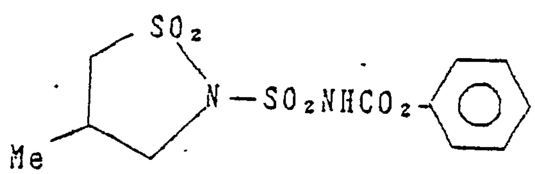

(oil)

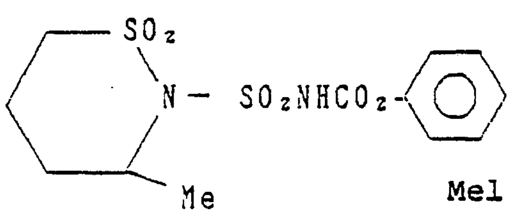

Melting point: 144-145°C

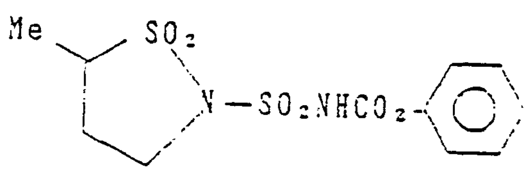

Melting point: 141-142°C

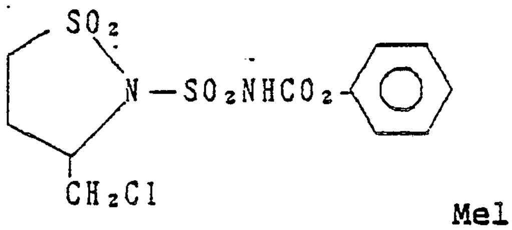

Melting point: 149-150°C

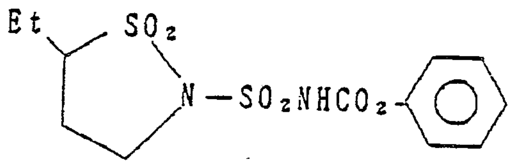

Melting point: 128-129°C

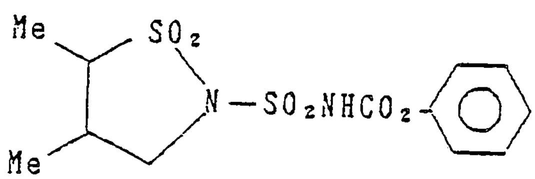

(oil)

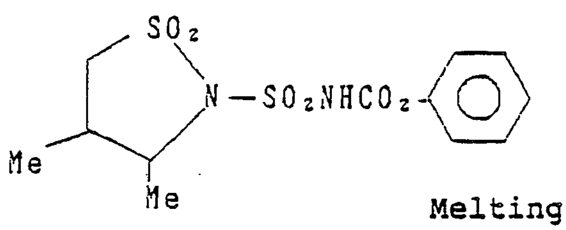

Melting point: 112-113°C

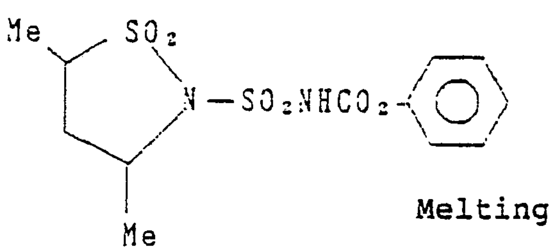

Melting point: 128-129°C

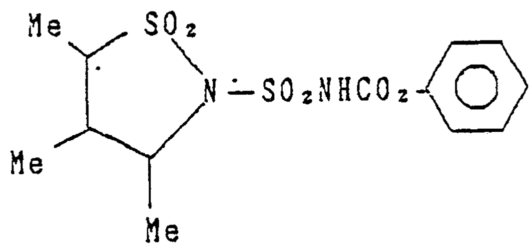

(oil)

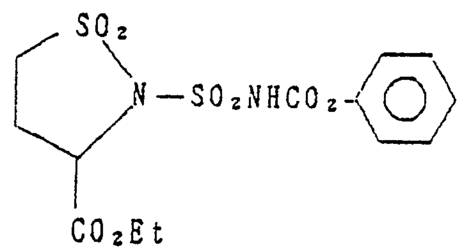

(oil)

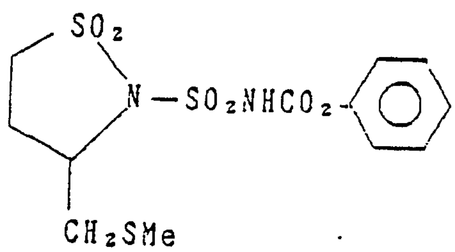

(oil)

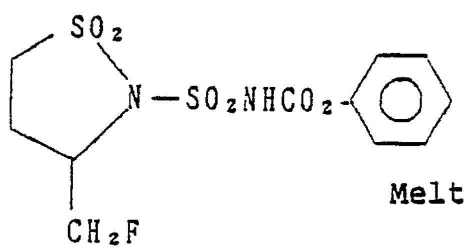

Melting point: 70-73°C

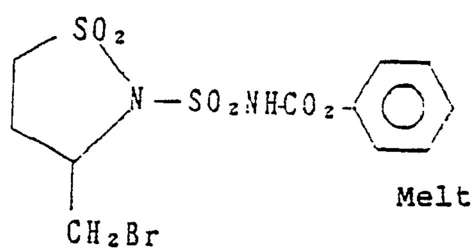

Melting point: 147-149°C

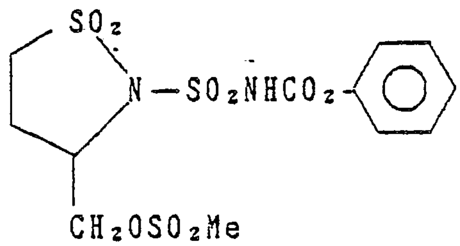

(solid)

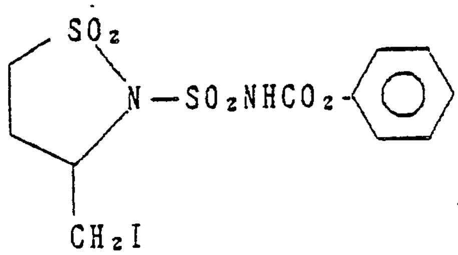

Melting point: 125-128°C

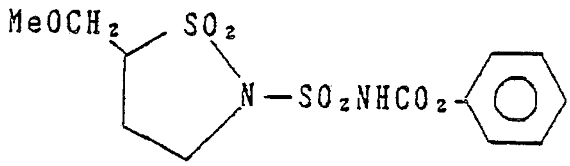

Melting point: 114-117°C

EXAMPLE 4

Preparation of 1-(1,4-butanesultam-N-sulfonyl)-3-(4,6-dimethoxypyrimidin-2-yl)urea

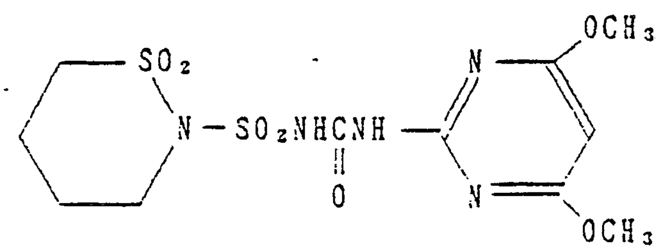

(Compound No. 1)

To a mixture of 400 mg (1.87 mmol) of 1,4-butanesultam-N-sulfonamide, 515 mg (1.87 mmol) of phenyl N-(4,6-dimethoxypyrimidin-2-yl) carbamate and 10 mℓ of dry acetonitrile, 285 mg (1.87 mmol) of 1,8-diazabicyclo[5,4,0]-7-undecene (DBU) was added, and the mixture was stirred at room temperature for 15 minutes. 50 mℓ of ice water was added to the reaction mixture, and the mixture was subjected to filtration. The filtrate was acidified with concentrated hydrochloric acid. Precipitated solid was collected by filtration

and washed with water and then with acetonitrile and dried to obtain 0.42 g of the desired 1-(1,4-butanesultam-N-N-sulfonyl)-3-(4,6-dimethoxpyrimidin-2-yl)urea.
Melting point: 173-174° C

EXAMPLE 5

Preparation of 1-(1-methoxycarbonyl-1,3-propanesultam-N-sulfonyl)-3-(4,6-dimethoxypyrimidin-2-yl)urea

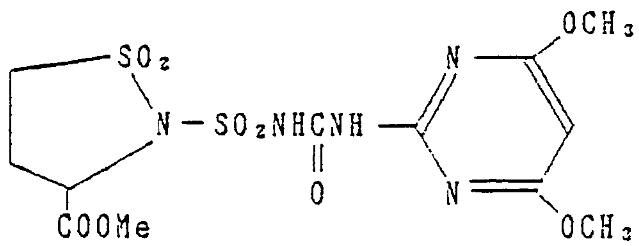

(Compound No. 2)

A mixture of 1.9 g (5.03 mmol) of phenyl N-(1-methoxycarbonyl-1,3-propanesultam-N-sulfonyl)-carbamate, 780 mg (5.03 mmol) of 2 amino-4,6- dimethoxypyrimidine and 20 mℓ of dry benzene was refluxed under heating for 20 minutes. After cooling, precipitated solid was collected by filtration to obtain 1.1 g of the desired 1-(1-methoxycarbonyl-1,3-propanesultam-N-sulfonyl)-3-(4,6-dimethoxypyrimidin-2-yl)-urea.
Melting point: 173-174° C

EXAMPLE 6

Preparation of 1-(1,4-butanesultam-N-sulfonyl)-3-(4,6-dimethoxypyrimidin-2-yl)thiourea

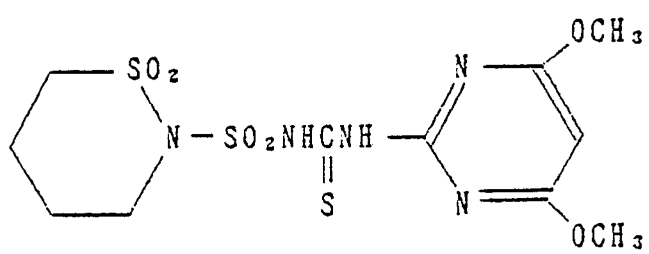

(Compound No. 3)

214 mg (1.0 mmol) of 1,4-butanesultam-N-sulfonamide and 207 mg (1.05 mmol) of 4,6-dimethoxy-2-isothiocyanatopyrimidine were dissolved in 10 mℓ of dry acetone, and then 145 mg (1.05 mmol) of anhydrous potassium carbonate was added thereto. The mixture was stirred at room temperature for 15 hours. Then, precipitated solid was collected by filtration and suspended in 30 mℓ of water. The suspension was adjusted to a pH of from 1 to 2 with diluted hydrochloric acid, and precipitated solid was collected by filtration, washed with water and then washed with a small amount of acetonitrile and diethyl ether to obtain

190 mg of 1-(1,4 butanesultam-N-sulfonyl)-3-(4,6-dimethoxypyrimidin-2-yl)thiourea.
Melting point: 215-216 °C

EXAMPLE 7

Preparation of 1-(2,4-butanesultam-N-sulfonyl)-3-methyl-3-(4,6-dimethoxypyrimidin-2-yl)urea

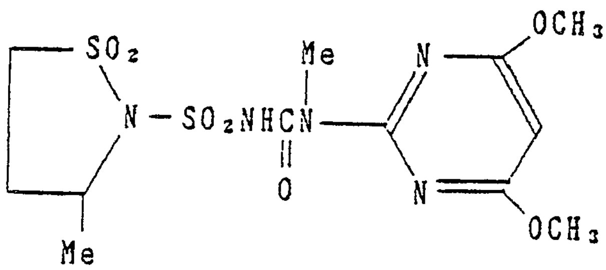

(Compound No. 37)

470 mg (1.19 mmol) of 1-(2,4-butanesultam-N-sulfonyl)-3-(4,6 dimethoxypyrimidin-2-yl)urea was dissolved in 10 mℓ of dry tetrahydrofuran, and 270 mg (2.41 mmol) of potassium t-butoxide and 270 mg (2.14 mmol) of dimethyl sulfate were sequentially added thereto. The reaction mixture was stirred at room temperature for 3 hours, and then the solvent was distilled off under reduced pressure. 20 mℓ of 5% hydrochloric acid and a small amount of ethanol were sequentially added thereto. Precipitated solid was collected by filtration, washed with water and then washed with a small amount of diethyl ether to obtain 270 mg of 1-(2,4-butanesultam-N-sulfonyl)-3-methyl-3-(4,6-dimethoxypyrimidin-2-yl)urea.
Melting point: 139-140 °C

The structure and physical properties of the compounds prepared in the same manner as in Example 4, 5 and 6 are shown below.

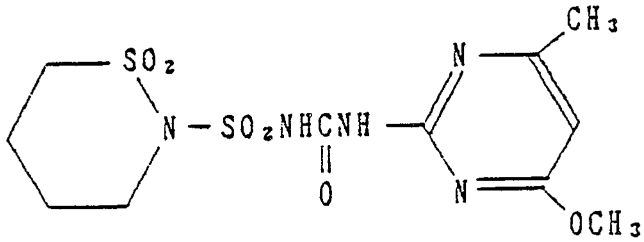

(Compound No. 4) Melting point: 153-154°C

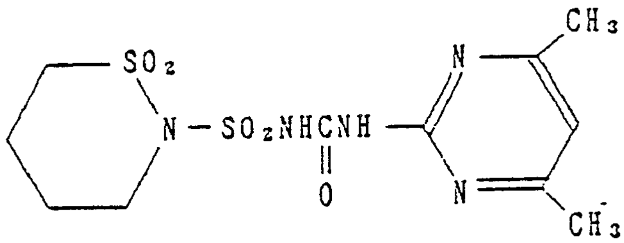

(Compound No. 5) Melting point: 165-166°C

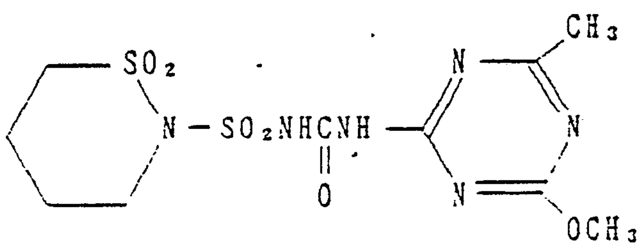

(Compound No. 6) Melting point: 159-160°C

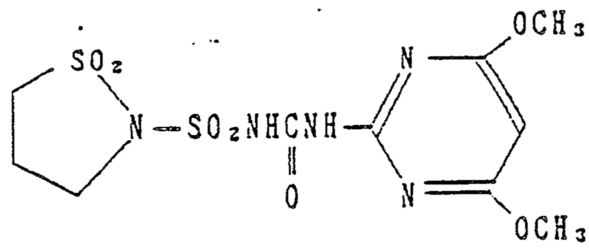

(Compound No. 7) Melting point: 190-191°C

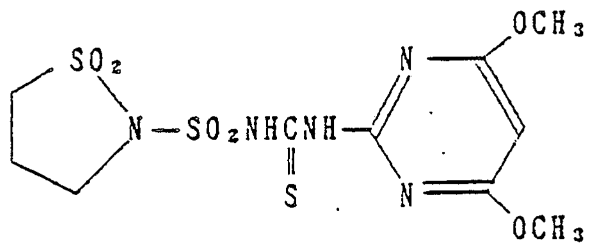

(Compound No. 8) Melting point: 180-181°C

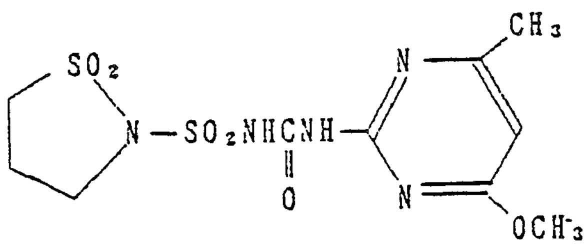

(Compound No. 9) Melting point: 172-173°C

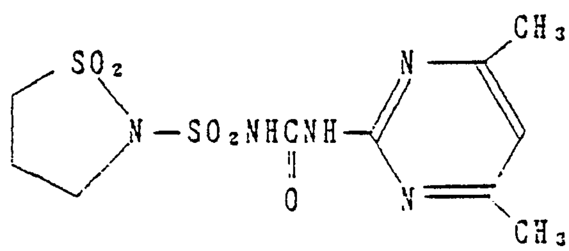

(Compound No. 10) Melting point: 170-171°C

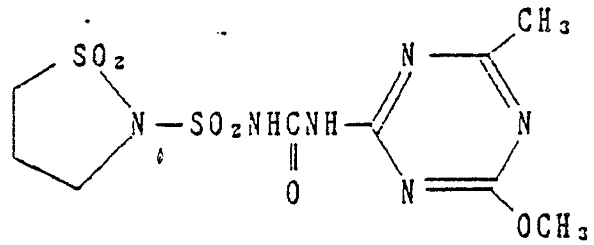

(Compound No. 11) Melting point: 156-157°C

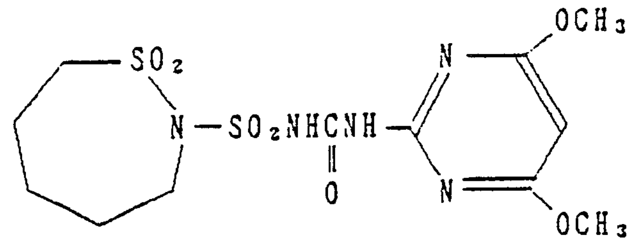

(Compound No. 12) Melting point: 159-160°C

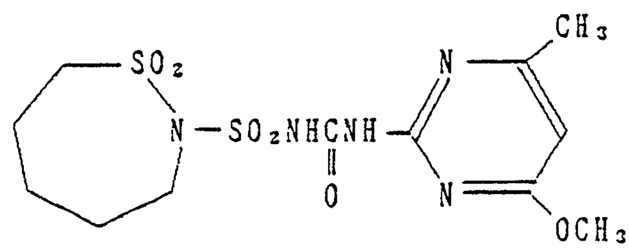

(Compound No. 13) Melting point: 162-163°C

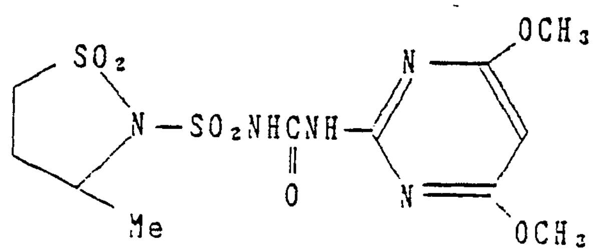

(Compound No. 14) Melting point: 173-174°C

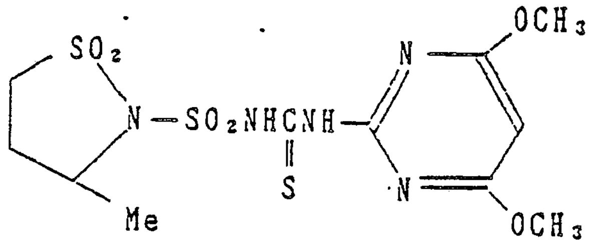

(Compound No. 15) Melting point: 160-161°C

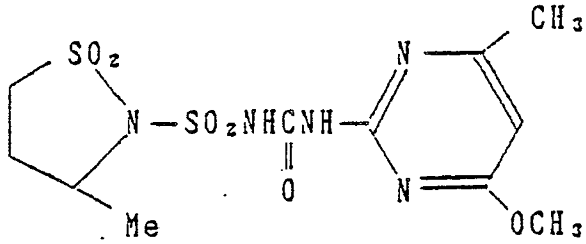

(Compound No. 16) Melting point: 159-160°C

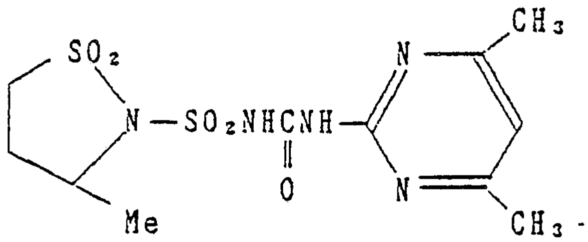

(Compound No. 17) Melting point: 174-175°C

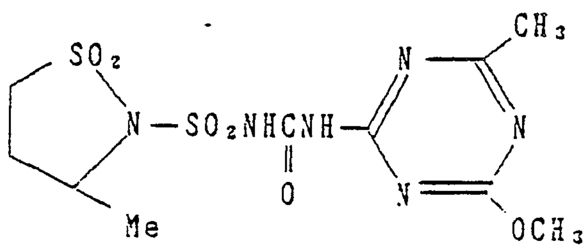

(Compound No. 18) Melting point: 156-157°C

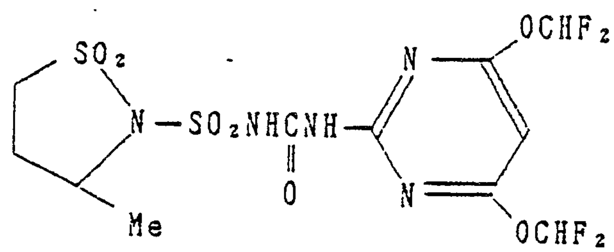

(Compound No. 19) Melting point: 140-142°C

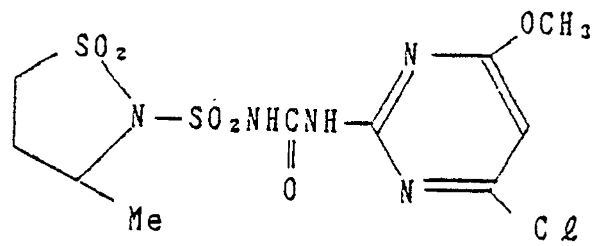

(Compound No. 20) Melting point: 154-155°C

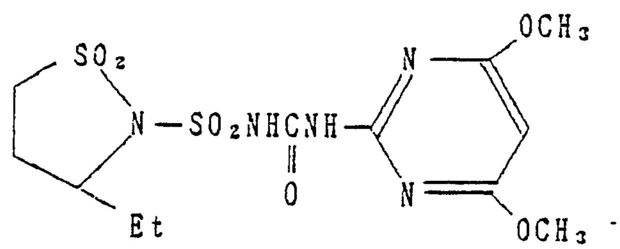

(Compound No. 21) Melting point: 162-164°C

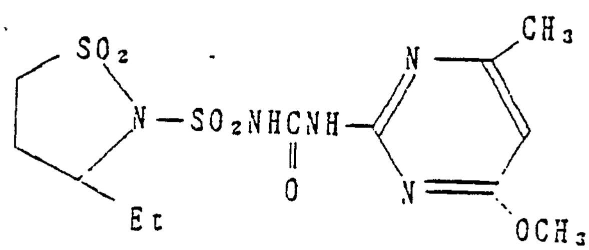

(Compound No. 22) Melting point: 118-120°C

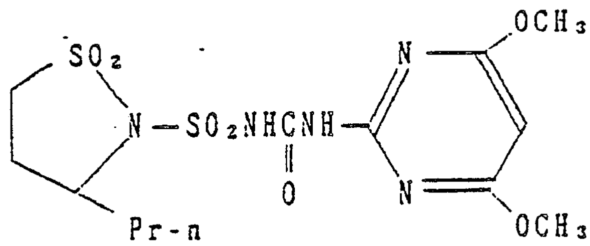

(Compound No. 23) Melting point: 138-140°C

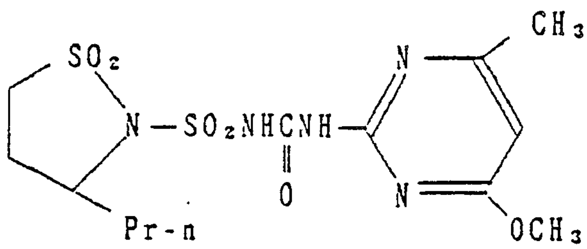

(Compound No. 24) Melting point: 142-145°C

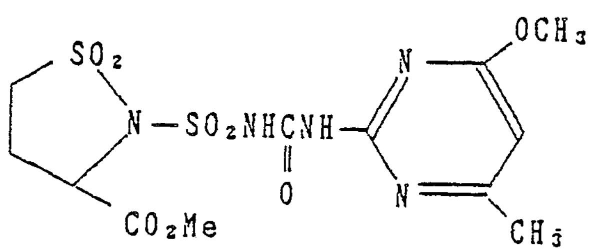

(Compound No. 25) Melting point: 182-183°C

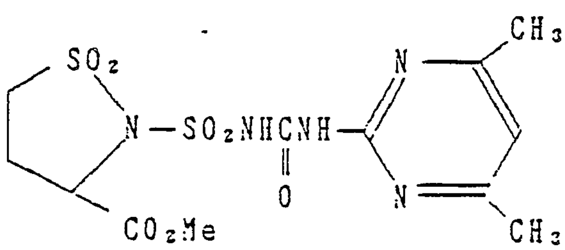

(Compound No. 26) Melting point: 188-189°C

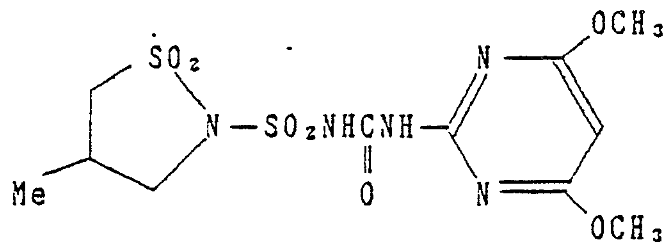

(Compound No. 27) Melting point: 156-158°C

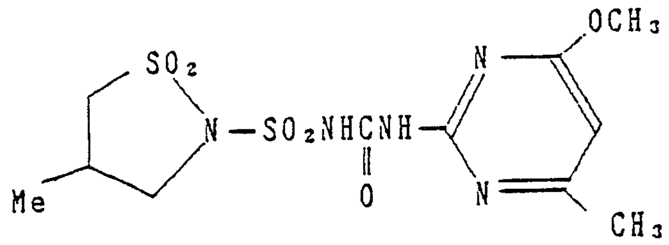

(Compound No. 28) Melting point: 155-158°C

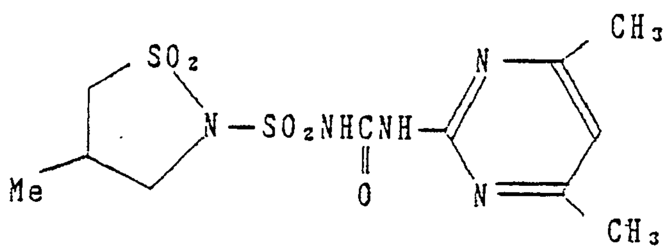

(Compound No. 29) Melting point: 157-158°C

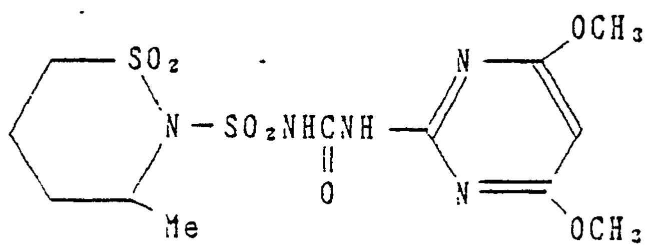

(Compound No. 30) Melting point: 173-174°C

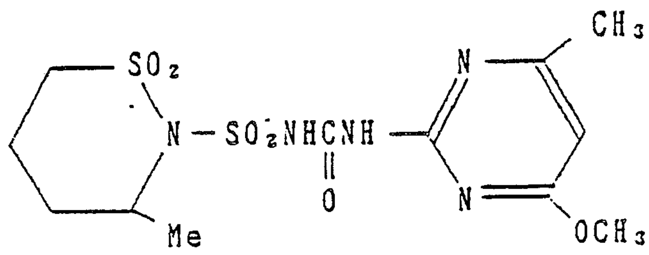

(Compound No. 31) Melting point: 158-159°C

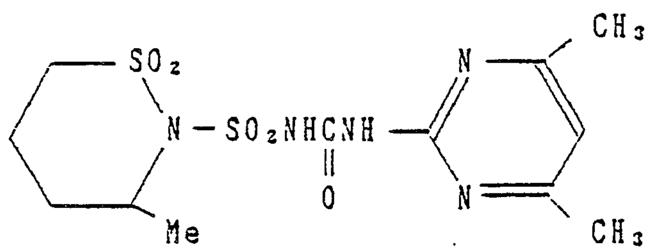

(Compound No. 32) Melting point: 158-159°C

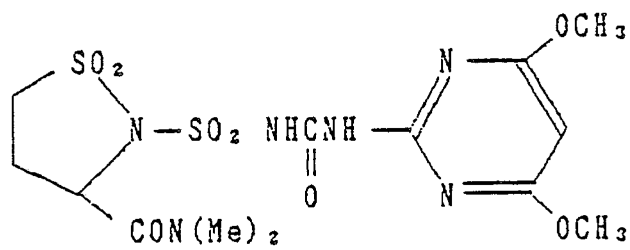

(Compound No. 33) Melting point: 143-144°C

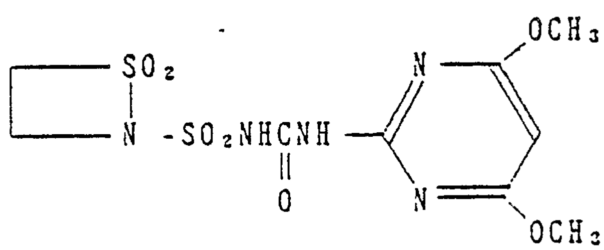

(Compound No. 34) Melting point: 171-172°C

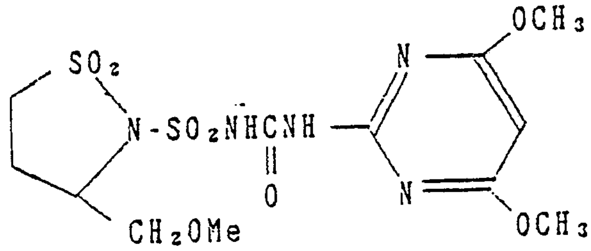

(Compound No. 35) Melting point: 150-152°C

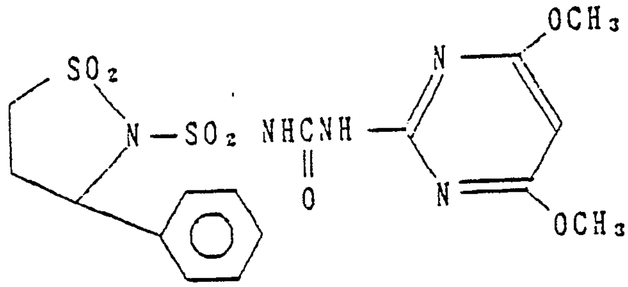

(Compound No. 36) Melting point: 199-200°C

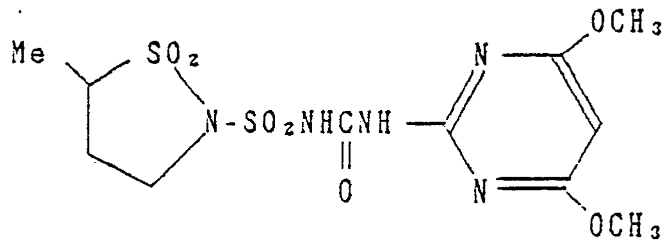

(Compound No. 38) Melting point: 178-179°C

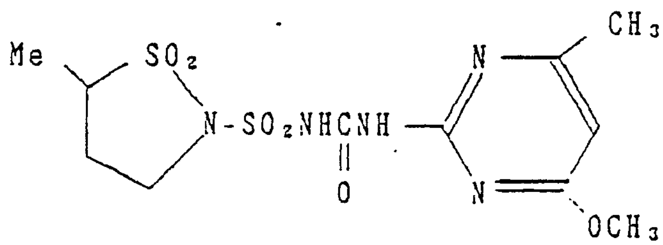

(Compound No. 39) Melting point: 179-180°C

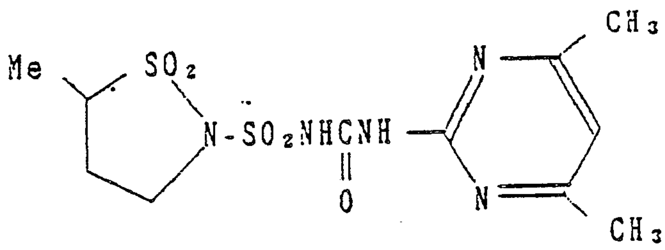

(Compound No. 40) Melting point: 176-177°C

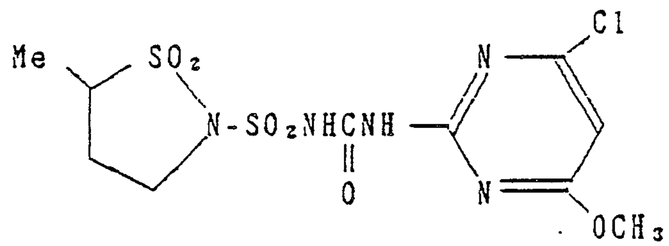

(Compound No. 41) Melting point: 166-167°C

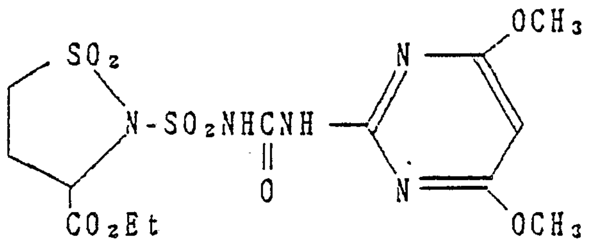

(Compound No. 42) Melting point: 136-139°C

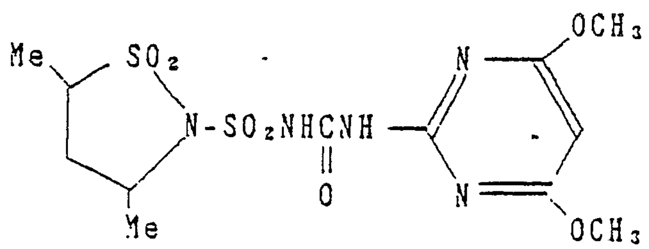

(Compound No. 43) Melting point: 174-175°C

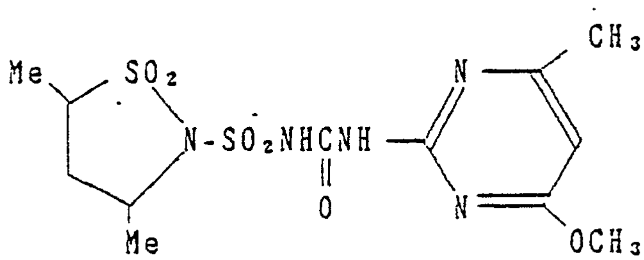

(Compound No. 44) Melting point: 179-180°C

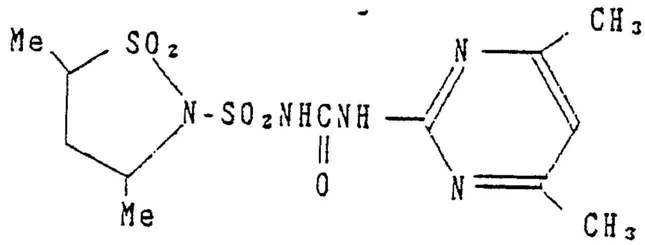

(Compound No. 45) Melting point: 157-158°C

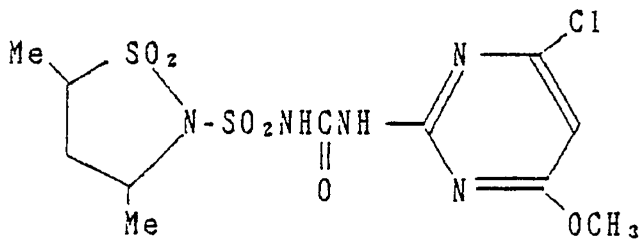

(Compound No. 46) Melting point: 183-184°C

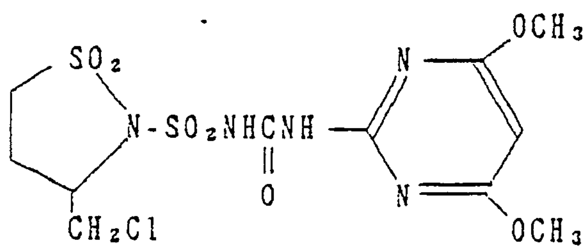

(Compound No. 47) Melting point: 178-180°C

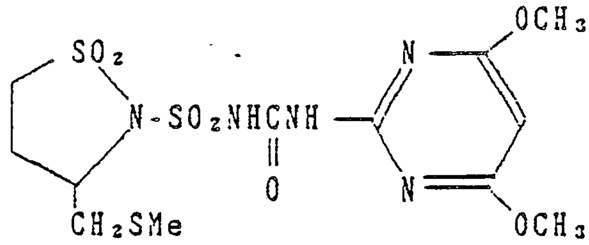

(Compound No. 48) Melting point: 137-140°C

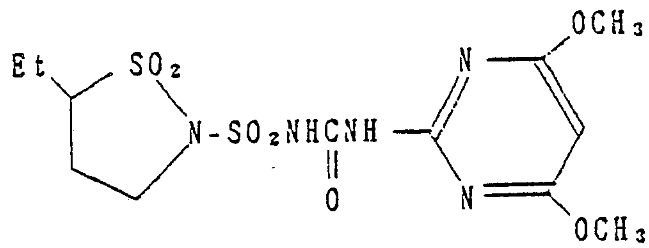

(Compound No. 49) Melting point: 179-180°C

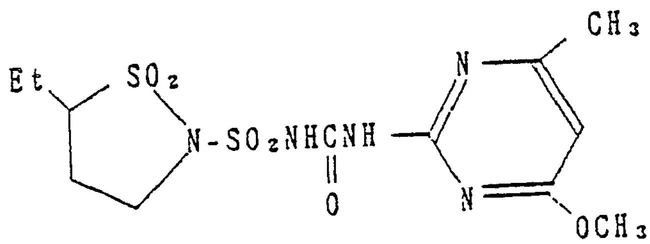

(Compound No. 50) Melting point: 163-164°C

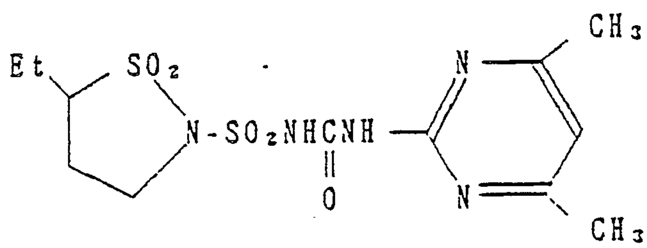

(Compound No. 51) Melting point: 174-175°C

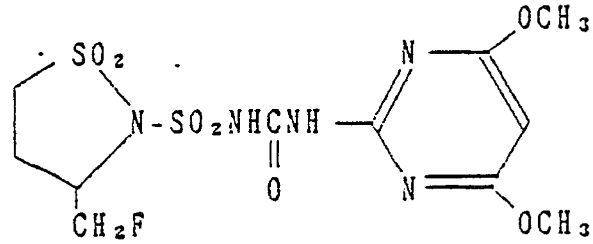

(Compound No. 52) Melting point: 161-166°C

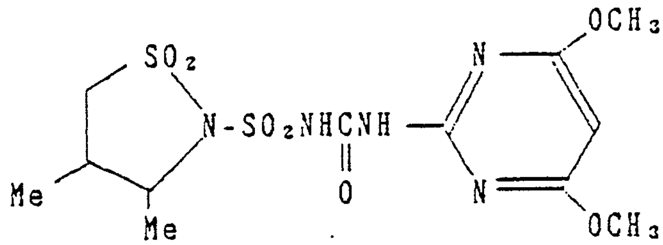

(Compound No. 53) Melting point: 189-190°C

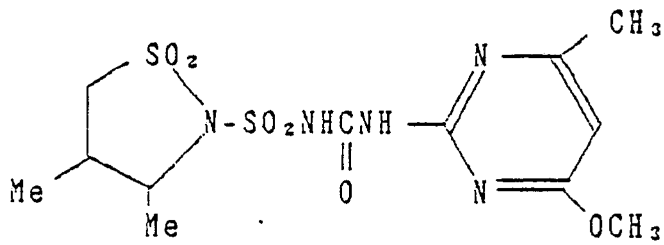

(Compound No. 54) Melting point: 143-144°C

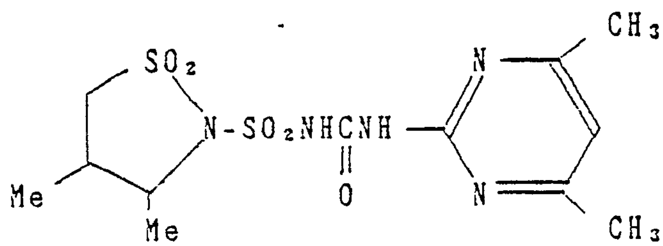

(Compound No. 55) Melting point: 140-141°C

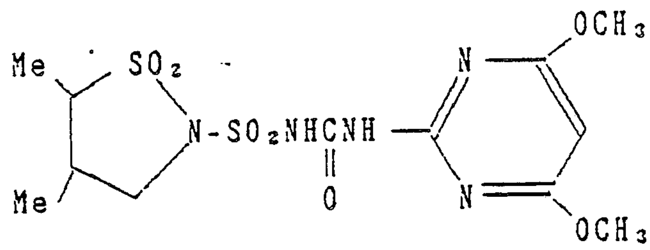

(Compound No. 56) Melting point: 168-169°C

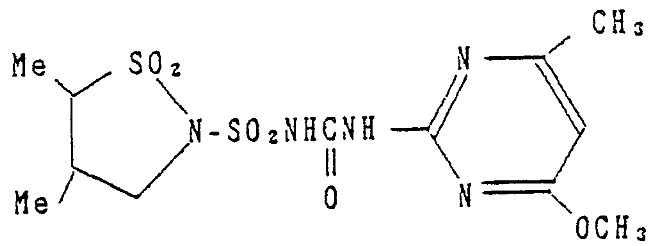

(Compound No. 57) Melting point: 171-172°C

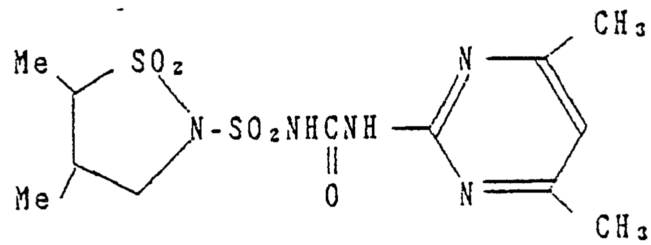

(Compound No. 58) Melting point: 191-192°C

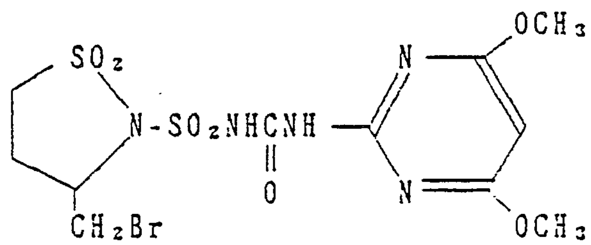

(Compound No. 59) Melting point: 157-159°C

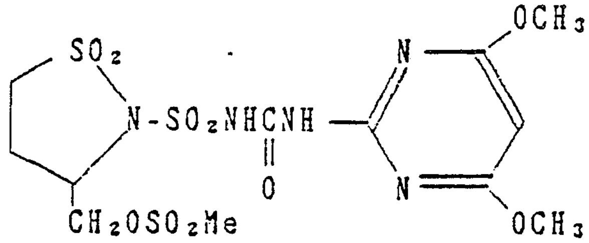

(Compound No. 60) Melting point: 173-174°C

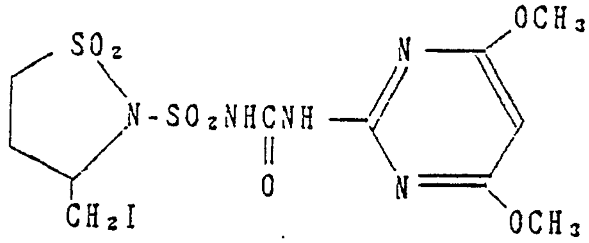

(Compound No. 61) Melting point: 172-173°C

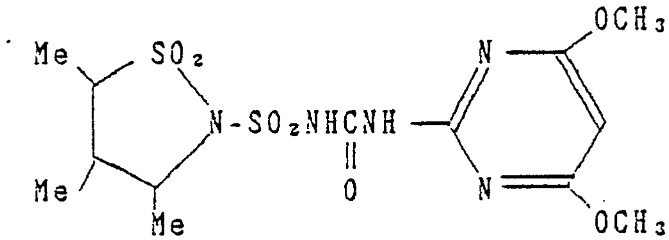

(Compound No. 62) Melting point: 172-174°C

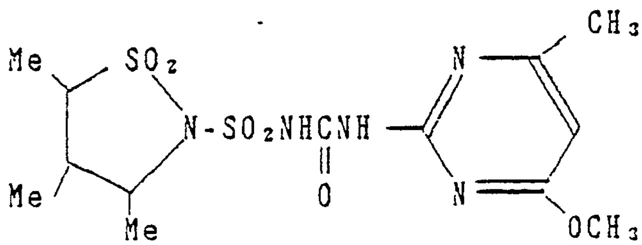

(Compound No. 63) Melting point: 171-174°C

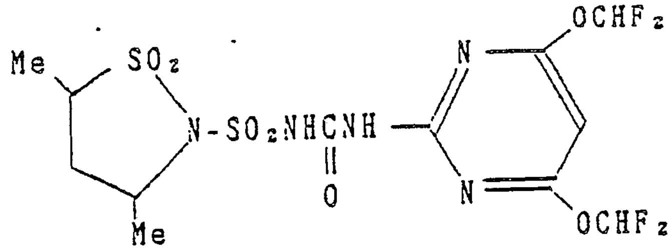

(Compound No. 64) Melting point: 177-178°C

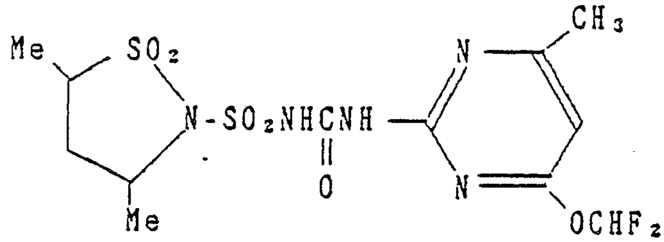

(Compound No. 65) Melting point: 170-171°C

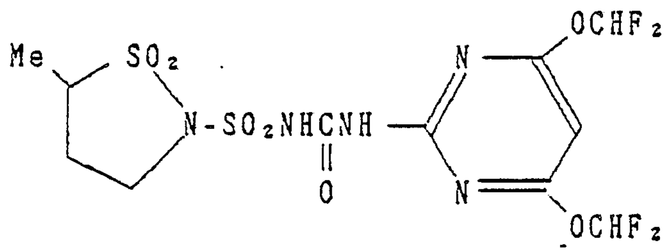

(Compound No. 66) Melting point: 170-171°C

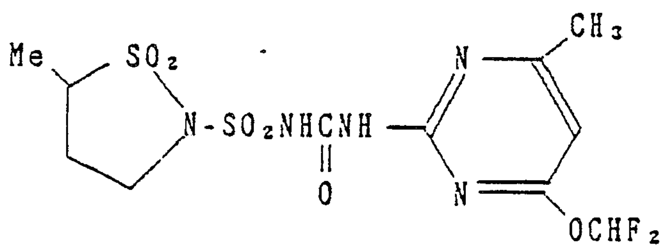

(Compound No. 67) Melting point: 166-167°C

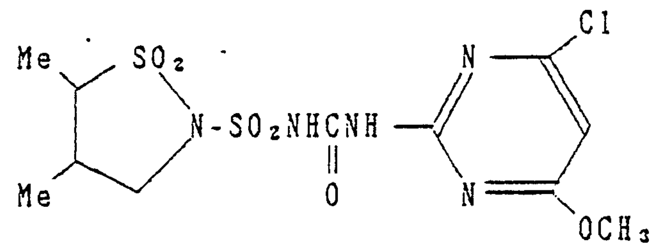

(Compound No. 68) Melting point: 169-170°C

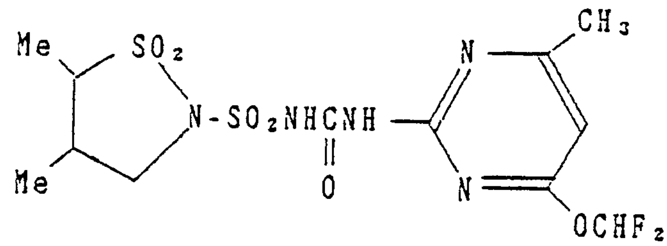

(Compound No. 69) Melting point: 168-169°C

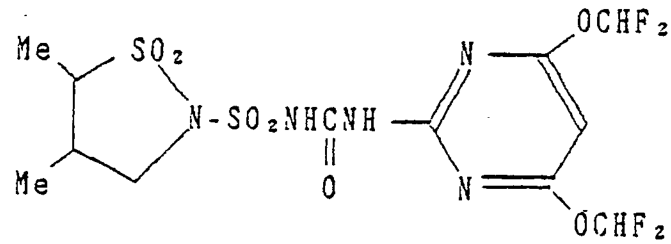

(Compound No. 70) Melting point: 179-180°C

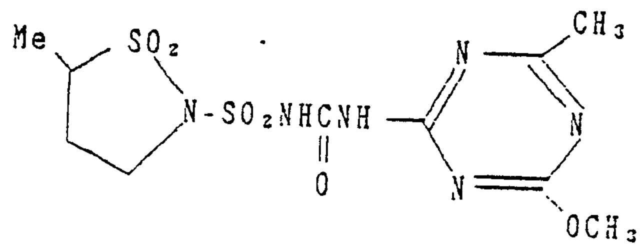

(Compound No. 71) Melting point: 149-150°C

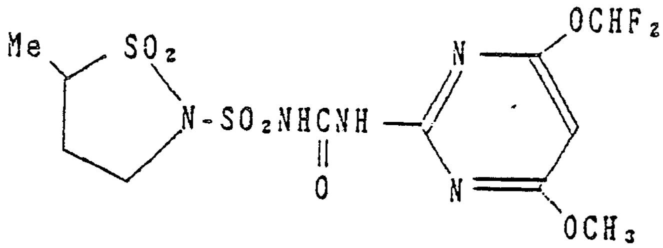

(Compound No. 72) Melting point: 168-169°C

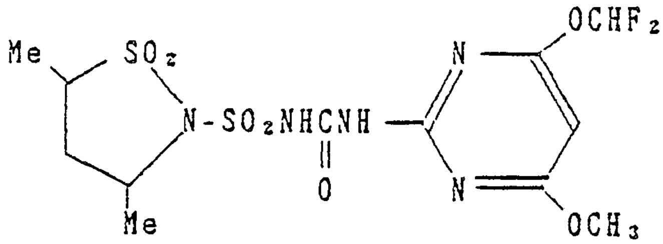

(Compound No. 73) Melting point: 168-170°C

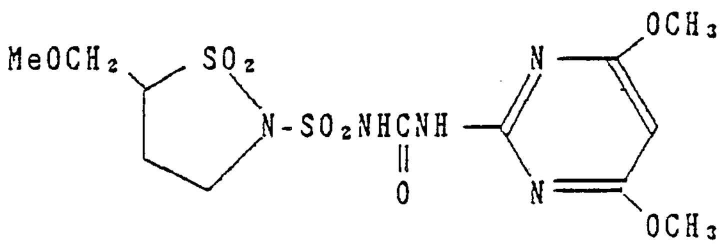

(Compound No. 74) Melting point: 160-161°C

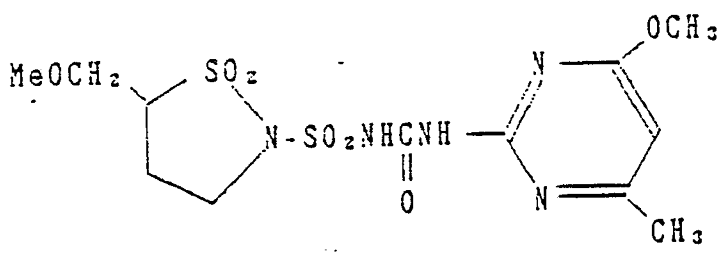

(Compound No. 75) Melting point: 158-160°C

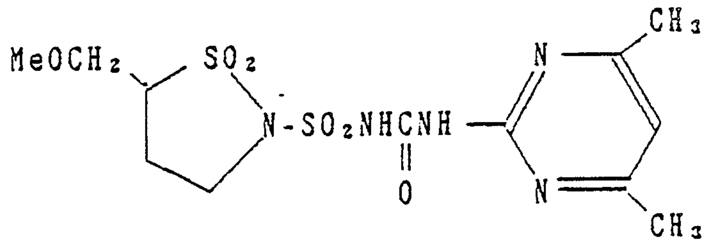

(Compound No. 76) Melting point: 157-160°C

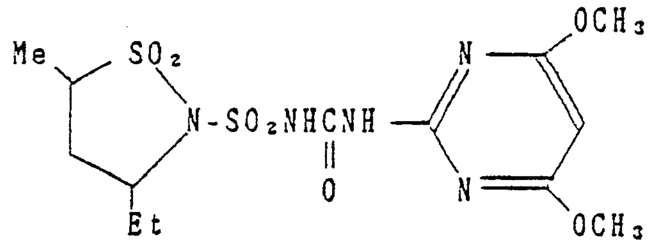

(Compound No. 77) Melting point: 166-167°C

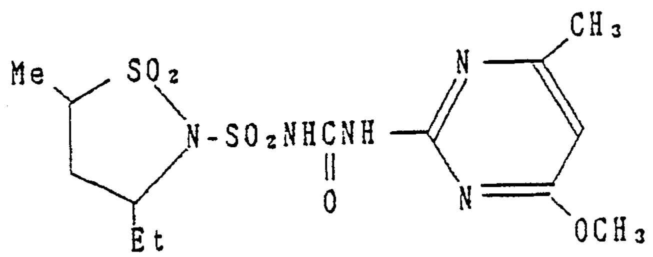

(Compound No. 78) Melting point: 158-159°C

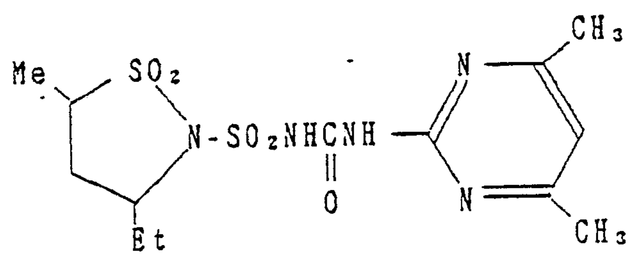

(Compound No. 79) Melting point: 159-160°C

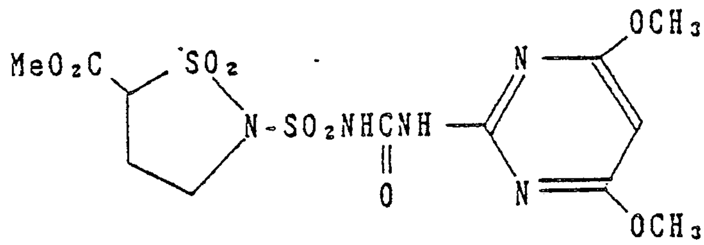

(Compound No. 80) Melting point: 148-151°C

Now, examples of the compounds included in the present invention are shown in Tables 1 to 10 together with the compounds prepared in the above Examples. However, it should be understood that the present invention is by no means restricted to such specific compounds.

The reference symbols in Tables have the following meanings.

Me: methyl, Et: ethyl, Pr-n: n-propyl, Pr-i: isopropyl, Bu-n: n-butyl, Bu-sec: sec-butyl, Bu-t: tert-butyl, Pen-n: n-pentyl, and Ph: phenyl. Gn has the following meanings.

Ga = G1 ~ G13, Gb = G1 ~ G6, Gc = G1 ~ G3

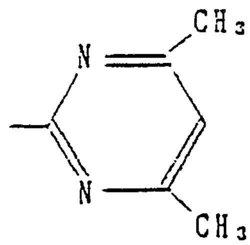

G1

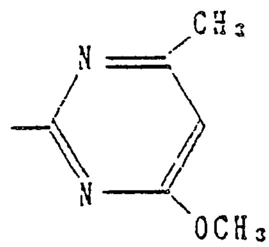

G2

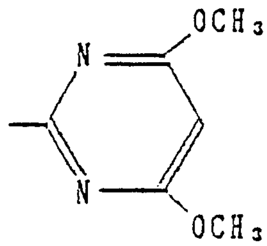

G3

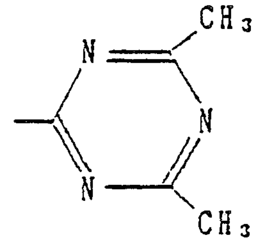

G4

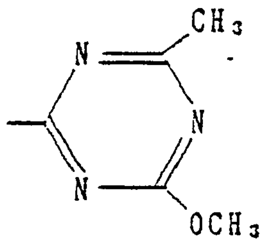

G5

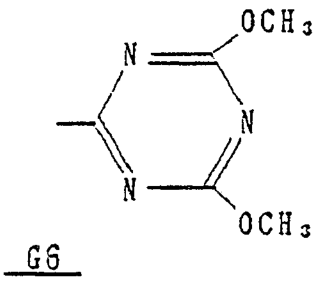

G6

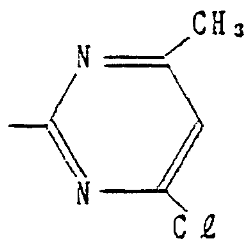

G7

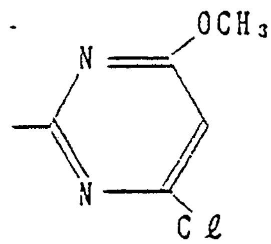

G8

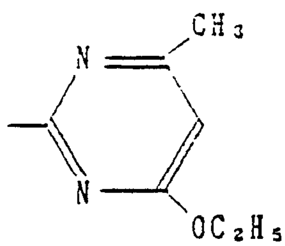

G9

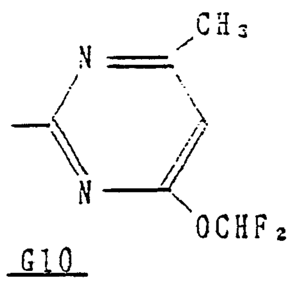

G10

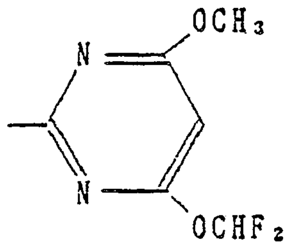

G11

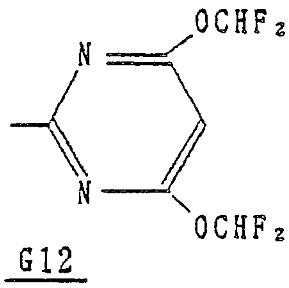

G12

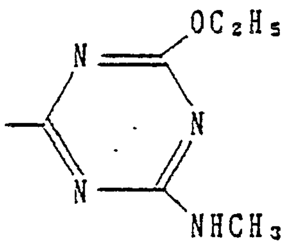

G13

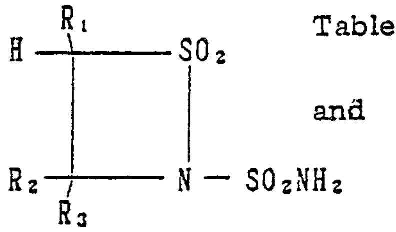

Table 1

and

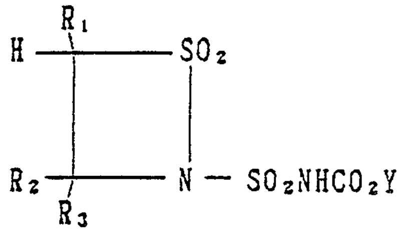

(Y=Me, Et, Pr-n, Bu-n or Ph)

| $R_1$ | $R_2$ | $R_3$ |
|---|---|---|
| H | H | H |
| H | Me | H |
| H | Et | H |
| H | Pr-n | H |
| H | Pr-i | H |
| H | Bu-n | H |
| Me | H | H |
| Et | H | H |
| Pr-n | H | H |
| Pr-i | H | H |
| Bu-n | H | H |
| H | Me | Me |
| H | Me | Et |
| H | Me | Pr-n |
| H | Me | Pr-i |
| H | Me | Bu-n |
| H | Et | Et |
| H | Et | Pr-n |
| H | Et | Pr-i |
| H | Pr-n | Pr-n |
| Me | H | H |
| Me | Me | H |
| Me | Et | H |
| Me | Pr-n | H |
| Me | Pr-i | H |
| Me | Bu-n | H |
| Et | H | H |
| Et | Me | H |
| Et | Et | H |
| Et | Pr-n | H |
| Et | Pr-i | H |

| $R_1$ | $R_2$ | $R_3$ |
|---|---|---|
| Et | Bu-n | H |
| Pr-n | H | H |
| Pr-n | Me | H |
| Pr-n | Et | H |
| Pr-n | Pr-n | H |
| Pr-n | Pr-i | H |
| Pr-n | Bu-n | H |
| Pr-i | H | H |
| Pr-i | Me | H |
| Pr-i | Et | H |
| Pr-i | Pr-n | H |
| Pr-i | Pr-i | H |
| Pr-i | Bu-n | H |
| Bu-n | H | H |
| Bu-n | Me | H |
| Bu-n | Et | H |
| Bu-n | Pr-n | H |
| Bu-n | Pr-i | H |
| Bu-n | Bu-n | H |
| Me | Me | Me |
| Me | Me | Et |
| Me | Me | Pr-n |
| Me | Me | Pr-i |
| Me | Et | Et |
| Me | Et | Pr-n |
| Me | Et | Pr-i |
| Et | Me | Me |
| Et | Me | Et |
| Et | Me | Pr-n |
| Et | Me | Pr-i |
| Et | Et | Et |
| Et | Et | Pr-n |
| Et | Et | Pr-i |
| Pr-n | Me | Me |
| Pr-n | Me | Et |
| Pr-n | Me | Pr-n |
| Pr-n | Me | Pr-i |

| $R_1$ | $R_2$ | $R_3$ |
|---|---|---|
| Pr-n | Et | Et |
| Pr-n | Et | Pr-n |
| Pr-n | Et | Pr-i |
| Pr-i | Me | Me |
| Pr-i | Me | Et |
| Pr-i | Me | Pr-n |
| Pr-i | Me | Pr-i |
| Pr-i | Et | Et |
| Pr-i | Et | Pr-n |
| Pr-i | Et | Pr-i |
| H | Ph | H |
| H | Ph-2-Cl | H |
| H | Ph-3-Cl | H |
| H | Ph-4-Cl | H |
| H | $CH_2Ph$ | H |
| Ph | H | H |
| Ph-2-Cl | H | H |
| Ph-3-Cl | H | H |
| Ph-4-Cl | H | H |
| $CH_2Ph$ | H | H |
| Me | Ph | H |
| Me | Ph-2-Cl | H |
| Me | Ph-3-Cl | H |
| Me | Ph-4-Cl | H |
| Me | $CH_2Ph$ | H |
| Et | Ph | H |
| Et | Ph-2-Cl | H |
| Et | Ph-3-Cl | H |
| Et | Ph-4-Cl | H |
| Et | $CH_2Ph$ | H |
| Pr-n | Ph | H |
| Pr-n | Ph-2-Cl | H |
| Pr-n | Ph-3-Cl | H |
| Pr-n | Ph-4-Cl | H |
| Pr-n | $CH_2Ph$ | H |
| Ph | Me | H |
| Ph-2-Cl | Me | H |
| Ph-3-Cl | Me | H |
| Ph-4-Cl | Me | H |

| $R_1$ | $R_2$ | $R_3$ |
|---|---|---|
| $CH_2Ph$ | Me | H |
| Ph | Et | H |
| Ph-2-Cl | Et | H |
| Ph-3-Cl | Et | H |
| Ph-4-Cl | Et | H |
| $CH_2Ph$ | Et | H |
| Ph | Pr-n | H |
| Ph-2-Cl | Pr-n | H |
| Ph-3-Cl | Pr-n | H |
| Ph-4-Cl | Pr-n | H |
| $CH_2Ph$ | Pr-n | H |

Table 2

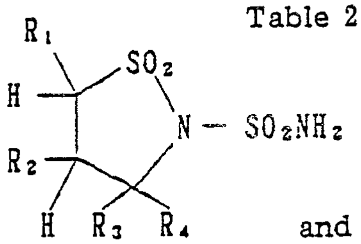

and

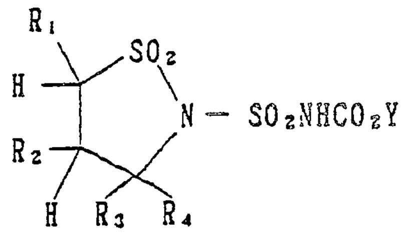

(Y=Me, Et, Pr-n, Bu-n or Ph)

| $R_1$ | $R_2$ | $R_3$ | $R_4$ |
|---|---|---|---|
| H | H | H | H |
| Me | H | H | H |
| H | Me | H | H |
| H | H | Me | H |
| Et | H | H | H |
| H | Et | H | H |
| H | H | Et | H |
| Pr-n | H | H | H |
| H | Pr-n | H | H |
| H | H | Pr-n | H |
| Pr-i | H | H | H |
| H | Pr-i | H | H |
| H | H | Pr-i | H |
| Bu-n | H | H | H |
| H | Bu-n | H | H |
| H | H | Bu-n | H |
| H | H | Me | Me |
| Me | H | Me | Me |
| H | Me | Me | Me |
| Et | H | Me | Me |
| H | Et | Me | Me |
| Pr-n | H | Me | Me |
| H | Pr-n | Me | Me |
| Pr-i | H | Me | Me |
| H | Pr-i | Me | Me |
| Bu-n | H | Me | Me |
| H | Bu-n | Me | Me |
| Me | Me | Me | Me |
| Me | Et | Me | Me |
| Me | Pr-n | Me | Me |

| $R_1$ | $R_2$ | $R_3$ | $R_4$ |
|---|---|---|---|
| Me | Bu-n | Me | Me |
| Et | Me | Me | Me |
| Et | Et | Me | Me |
| Et | Pr-n | Me | Me |
| Et | Bu-n | Me | Me |
| Pr-n | Me | Me | Me |
| Pr-n | Et | Me | Me |
| Pr-n | Pr-n | Me | Me |
| Pr-n | Bu-n | Me | Me |
| Bu-n | Me | Me | Me |
| Bu-n | Et | Me | Me |
| Bu-n | Pr-n | Me | Me |
| Bu-n | Bu-n | Me | Me |
| F | H | Me | Me |
| Cl | H | Me | Me |
| Br | H | Me | Me |
| I | H | Me | Me |
| $CH=CH_2$ | H | Me | Me |
| $CH_2CH=CH_2$ | H | Me | Me |
| $C\equiv CH$ | H | Me | Me |
| $CH_2C\equiv CH$ | H | Me | Me |
| $CH_2F$ | H | Me | Me |
| $CHF_2$ | H | Me | Me |
| CN | H | Me | Me |
| $NH_2$ | H | Me | Me |
| NHCOMe | H | Me | Me |
| $CH_2NH_2$ | H | Me | Me |
| $CH_2NHCOMe$ | H | Me | Me |
| $CH_2OCHF_2$ | H | Me | Me |
| $CH_2OCF_3$ | H | Me | Me |
| $CF_3$ | H | Me | Me |
| $NO_2$ | H | Me | Me |
| OMe | H | Me | Me |
| OEt | H | Me | Me |
| OPr-n | H | Me | Me |
| OBu-n | H | Me | Me |
| OPen-n | H | Me | Me |
| SMe | H | Me | Me |
| SEt | H | Me | Me |
| SPr-n | H | Me | Me |

| $R_1$ | $R_2$ | $R_3$ | $R_4$ |
|---|---|---|---|
| SBu-n | H | Me | Me |
| SPen-n | H | Me | Me |
| $SO_2Me$ | H | Me | Me |
| $SO_2Et$ | H | Me | Me |
| $SO_2Pr$-n | H | Me | Me |
| COMe | H | Me | Me |
| COEt | H | Me | Me |
| COPr-n | H | Me | Me |
| $CO_2H$ | H | Me | Me |
| $CO_2Me$ | H | Me | Me |
| $CO_2Et$ | H | Me | Me |
| $CO_2Pr$-n | H | Me | Me |
| $CO_2Bu$-n | H | Me | Me |
| $CO_2Pen$-n | H | Me | Me |
| $CH_2Cl$ | H | Me | Me |
| $CH_2Br$ | H | Me | Me |
| $CH_2I$ | H | Me | Me |
| $CH_2CF_3$ | H | Me | Me |
| $CH_2CN$ | H | Me | Me |
| $CH_2OH$ | H | Me | Me |
| $CH_2OMe$ | H | Me | Me |
| $CH_2OEt$ | H | Me | Me |
| $CH_2OPr$-n | H | Me | Me |
| $CH_2SMe$ | H | Me | Me |
| $CH_2SEt$ | H | Me | Me |
| $CH_2SPr$-n | H | Me | Me |
| $CH_2SO_2Me$ | H | Me | Me |
| $CH_2SO_2Et$ | H | Me | Me |
| $CH_2SO_2Pr$-n | H | Me | Me |
| $CH_2COMe$ | H | Me | Me |
| $CH_2COEt$ | H | Me | Me |
| $CH_2COPr$-n | H | Me | Me |
| $CH_2CO_2H$ | H | Me | Me |
| $CH_2CO_2Me$ | H | Me | Me |
| $CH_2CO_2Et$ | H | Me | Me |
| $CH_2CO_2Pr$-n | H | Me | Me |
| $CH_2OCH_2CH=CH_2$ | H | Me | Me |
| $CH_2OCH_2C\equiv CH$ | H | Me | Me |
| $CH=CHCO_2Me$ | H | Me | Me |
| $CH=CHCO_2Et$ | H | Me | Me |

| $R_1$ | $R_2$ | $R_3$ | $R_4$ |
|---|---|---|---|
| $CH=CHCO_2Pr$-n | H | Me | Me |
| CH=CHCN | H | Me | Me |
| Ph | H | Me | Me |
| $CH_2Ph$ | H | Me | Me |
| OPh | H | Me | Me |
| SPh | H | Me | Me |
| $SO_2Ph$ | H | Me | Me |
| CONHMe | H | Me | Me |
| CONHEt | H | Me | Me |
| CONHPr-n | H | Me | Me |
| CONHPr-i | H | Me | Me |
| CONHBu-n | H | Me | Me |
| CONHBu-sec | H | Me | Me |
| CONHBu-t | H | Me | Me |
| $CON(Me)_2$ | H | Me | Me |
| $CON(Et)_2$ | H | Me | Me |
| $CON(Pr$-$n)_2$ | H | Me | Me |
| H | F | Me | Me |
| H | Cl | Me | Me |
| H | Br | Me | Me |
| H | I | Me | Me |
| H | $CH=CH_2$ | Me | Me |
| H | $CH_2CH=CH_2$ | Me | Me |
| H | $C\equiv CH$ | Me | Me |
| H | $CH_2C\equiv CH$ | Me | Me |
| H | $CH_2F$ | Me | Me |
| H | $CHF_2$ | Me | Me |
| H | CN | Me | Me |
| H | $NH_2$ | Me | Me |
| H | NHCOMe | Me | Me |
| H | $CH_2NH_2$ | Me | Me |
| H | $CH_2NHCOMe$ | Me | Me |
| H | $CH_2OCHF_2$ | Me | Me |
| H | $CH_2OCF_3$ | Me | Me |
| H | $CF_3$ | Me | Me |
| H | $NO_2$ | Me | Me |
| H | OMe | Me | Me |
| H | OEt | Me | Me |
| H | OPr-n | Me | Me |
| H | OBu-n | Me | Me |

| $R_1$ | $R_2$ | $R_3$ | $R_4$ |
|---|---|---|---|
| H | OPen-n | Me | Me |
| H | SMe | Me | Me |
| H | SEt | Me | Me |
| H | SPr-n | Me | Me |
| H | SBu-n | Me | Me |
| H | SPen-n | Me | Me |
| H | $SO_2Me$ | Me | Me |
| H | $SO_2Et$ | Me | Me |
| H | $SO_2Pr$-n | Me | Me |
| H | COMe | Me | Me |
| H | COEt | Me | Me |
| H | COPr-n | Me | Me |
| H | $CO_2H$ | Me | Me |
| H | $CO_2Me$ | Me | Me |
| H | $CO_2Et$ | Me | Me |
| H | $CO_2Pr$-n | Me | Me |
| H | $CO_2Bu$-n | Me | Me |
| H | $CO_2Pen$-n | Me | Me |
| H | $CH_2Cl$ | Me | Me |
| H | $CH_2Br$ | Me | Me |
| H | $CH_2I$ | Me | Me |
| H | $CH_2CF_3$ | Me | Me |
| H | $CH_2CN$ | Me | Me |
| H | $CH_2OH$ | Me | Me |
| H | $CH_2OMe$ | Me | Me |
| H | $CH_2OEt$ | Me | Me |
| H | $CH_2OPr$-n | Me | Me |
| H | $CH_2SMe$ | Me | Me |
| H | $CH_2SEt$ | Me | Me |
| H | $CH_2SPr$-n | Me | Me |
| H | $CH_2SO_2Me$ | Me | Me |
| H | $CH_2SO_2Et$ | Me | Me |
| H | $CH_2SO_2Pr$-n | Me | Me |
| H | $CH_2COMe$ | Me | Me |
| H | $CH_2COEt$ | Me | Me |
| H | $CH_2COPr$-n | Me | Me |
| H | $CH_2CO_2H$ | Me | Me |
| H | $CH_2CO_2Me$ | Me | Me |
| H | $CH_2CO_2Et$ | Me | Me |
| H | $CH_2CO_2Pr$-n | Me | Me |

| $R_1$ | $R_2$ | $R_3$ | $R_4$ |
|---|---|---|---|
| H | $CH_2OCH_2CH=CH_2$ | Me | Me |
| H | $CH_2OCH_2C\equiv CH$ | Me | Me |
| H | $CH=CHCO_2Me$ | Me | Me |
| H | $CH=CHCO_2Et$ | Me | Me |
| H | $CH=CHCO_2Pr$-n | Me | Me |
| H | CH=CHCN | Me | Me |
| H | Ph | Me | Me |
| H | $CH_2Ph$ | Me | Me |
| H | OPh | Me | Me |
| H | SPh | Me | Me |
| H | $SO_2Ph$ | Me | Me |
| H | CONHMe | Me | Me |
| H | CONHEt | Me | Me |
| H | CONHPr-n | Me | Me |
| H | CONHPr-i | Me | Me |
| H | CONHBu-n | Me | Me |
| H | CONHBu-sec | Me | Me |
| H | CONHBu-t | Me | Me |
| H | $CON(Me)_2$ | Me | Me |
| H | $CON(Et)_2$ | Me | Me |
| H | $CON(Pr\text{-}n)_2$ | Me | Me |
| F | Me | Me | Me |
| Cl | Me | Me | Me |
| Br | Me | Me | Me |
| I | Me | Me | Me |
| $CH=CH_2$ | Me | Me | Me |
| $CH_2CH=CH_2$ | Me | Me | Me |
| $C\equiv CH$ | Me | Me | Me |
| $CH_2C\equiv CH$ | Me | Me | Me |
| $CH_2F$ | Me | Me | Me |
| $CHF_2$ | Me | Me | Me |
| CN | Me | Me | Me |
| $NH_2$ | Me | Me | Me |
| NHCOMe | Me | Me | Me |
| $CH_2NH_2$ | Me | Me | Me |
| $CH_2NHCOMe$ | Me | Me | Me |
| $CH_2OCHF_2$ | Me | Me | Me |
| $CH_2OCF_3$ | Me | Me | Me |
| $CF_3$ | Me | Me | Me |
| $NO_2$ | Me | Me | Me |

| $R_1$ | $R_2$ | $R_3$ | $R_4$ |
|---|---|---|---|
| OMe | Me | Me | Me |
| OEt | Me | Me | Me |
| OPr-n | Me | Me | Me |
| OBu-n | Me | Me | Me |
| OPen-n | Me | Me | Me |
| SMe | Me | Me | Me |
| SEt | Me | Me | Me |
| SPr-n | Me | Me | Me |
| SBu-n | Me | Me | Me |
| SPen-n | Me | Me | Me |
| $SO_2Me$ | Me | Me | Me |
| $SO_2Et$ | Me | Me | Me |
| $SO_2Pr$-n | Me | Me | Me |
| COMe | Me | Me | Me |
| COEt | Me | Me | Me |
| COPr-n | Me | Me | Me |
| $CO_2H$ | Me | Me | Me |
| $CO_2Me$ | Me | Me | Me |
| $CO_2Et$ | Me | Me | Me |
| $CO_2Pr$-n | Me | Me | Me |
| $CO_2Bu$-n | Me | Me | Me |
| $CO_2Pen$-n | Me | Me | Me |
| $CH_2Cl$ | Me | Me | Me |
| $CH_2Br$ | Me | Me | Me |
| $CH_2I$ | Me | Me | Me |
| $CH_2CF_3$ | Me | Me | Me |
| $CH_2CN$ | Me | Me | Me |
| $CH_2OH$ | Me | Me | Me |
| $CH_2OMe$ | Me | Me | Me |
| $CH_2OEt$ | Me | Me | Me |
| $CH_2OPr$-n | Me | Me | Me |
| $CH_2SMe$ | Me | Me | Me |
| $CH_2SEt$ | Me | Me | Me |
| $CH_2SPr$-n | Me | Me | Me |
| $CH_2SO_2Me$ | Me | Me | Me |
| $CH_2SO_2Et$ | Me | Me | Me |
| $CH_2SO_2Pr$-n | Me | Me | Me |
| $CH_2COMe$ | Me | Me | Me |
| $CH_2COEt$ | Me | Me | Me |
| $CH_2COPr$-n | Me | Me | Me |

| $R_1$ | $R_2$ | $R_3$ | $R_4$ |
|---|---|---|---|
| $CH_2CO_2Me$ | Me | Me | Me |
| $CH_2CO_2Et$ | Me | Me | Me |
| $CH_2CO_2Pr$-n | Me | Me | Me |
| $CH_2OCH_2CH=CH_2$ | Me | Me | Me |
| $CH_2OCH_2C \equiv CH$ | Me | Me | Me |
| $CH=CHCO_2Me$ | Me | Me | Me |
| $CH=CHCO_2Et$ | Me | Me | Me |
| $CH=CHCO_2Pr$-n | Me | Me | Me |
| CH=CHCN | Me | Me | Me |
| Ph | Me | Me | Me |
| $CH_2Ph$ | Me | Me | Me |
| OPh | Me | Me | Me |
| SPh | Me | Me | Me |
| $SO_2Ph$ | Me | Me | Me |
| CONHMe | Me | Me | Me |
| CONHEt | Me | Me | Me |
| CONHPr-n | Me | Me | Me |
| CONHPr-i | Me | Me | Me |
| CONHBu-n | Me | Me | Me |
| CONHBu-sec | Me | Me | Me |
| CONHBu-t | Me | Me | Me |
| $CON(Me)_2$ | Me | Me | Me |
| $CON(Et)_2$ | Me | Me | Me |
| $CON(Pr$-$n)_2$ | Me | Me | Me |
| F | Cl | Me | Me |
| Cl | Cl | Me | Me |
| Br | Cl | Me | Me |
| I | Cl | Me | Me |
| $CH=CH_2$ | Cl | Me | Me |
| $CH_2CH=CH_2$ | Cl | Me | Me |
| $C \equiv CH$ | Cl | Me | Me |
| $CH_2C \equiv CH$ | Cl | Me | Me |
| $CH_2F$ | Cl | Me | Me |
| $CHF_2$ | Cl | Me | Me |
| CN | Cl | Me | Me |
| $NH_2$ | Cl | Me | Me |
| NHCOMe | Cl | Me | Me |
| $CH_2NH_2$ | Cl | Me | Me |
| $CH_2NHCOMe$ | Cl | Me | Me |
| $CH_2OCHF_2$ | Cl | Me | Me |

| $R_1$ | $R_2$ | $R_3$ | $R_4$ |
|---|---|---|---|
| $CH_2OCF_3$ | Cl | Me | Me |
| $CF_3$ | Cl | Me | Me |
| $NO_2$ | Cl | Me | Me |
| OMe | Cl | Me | Me |
| OEt | Cl | Me | Me |
| OPr-n | Cl | Me | Me |
| OBu-n | Cl | Me | Me |
| OPen-n | Cl | Me | Me |
| SMe | Cl | Me | Me |
| SEt | Cl | Me | Me |
| SPr-n | Cl | Me | Me |
| SBu-n | Cl | Me | Me |
| SPen-n | Cl | Me | Me |
| $SO_2Me$ | Cl | Me | Me |
| $SO_2Et$ | Cl | Me | Me |
| $SO_2Pr$-n | Cl | Me | Me |
| COMe | Cl | Me | Me |
| COEt | Cl | Me | Me |
| COPr-n | Cl | Me | Me |
| $CO_2H$ | Cl | Me | Me |
| $CO_2Me$ | Cl | Me | Me |
| $CO_2Et$ | Cl | Me | Me |
| $CO_2Pr$-n | Cl | Me | Me |
| $CO_2Bu$-n | Cl | Me | Me |
| $CO_2Pen$-n | Cl | Me | Me |
| $CH_2Cl$ | Cl | Me | Me |
| $CH_2Br$ | Cl | Me | Me |
| $CH_2I$ | Cl | Me | Me |
| $CH_2CF_3$ | Cl | Me | Me |
| $CH_2CN$ | Cl | Me | Me |
| $CH_2OH$ | Cl | Me | Me |
| $CH_2OMe$ | Cl | Me | Me |
| $CH_2OEt$ | Cl | Me | Me |
| $CH_2OPr$-n | Cl | Me | Me |
| $CH_2SMe$ | Cl | Me | Me |
| $CH_2SEt$ | Cl | Me | Me |
| $CH_2SPr$-n | Cl | Me | Me |
| $CH_2SO_2Me$ | Cl | Me | Me |
| $CH_2SO_2Et$ | Cl | Me | Me |
| $CH_2SO_2Pr$-n | Cl | Me | Me |

| $R_1$ | $R_2$ | $R_3$ | $R_4$ |
|---|---|---|---|
| $CH_2COMe$ | Cl | Me | Me |
| $CH_2COEt$ | Cl | Me | Me |
| $CH_2COPr$-n | Cl | Me | Me |
| $CH_2CO_2H$ | Cl | Me | Me |
| $CH_2CO_2Me$ | Cl | Me | Me |
| $CH_2CO_2Et$ | Cl | Me | Me |
| $CH_2CO_2Pr$-n | Cl | Me | Me |
| $CH_2OCH_2CH=CH_2$ | Cl | Me | Me |
| $CH_2OCH_2C\equiv CH$ | Cl | Me | Me |
| $CH=CHCO_2Me$ | Cl | Me | Me |
| $CH=CHCO_2Et$ | Cl | Me | Me |
| $CH=CHCO_2Pr$-n | Cl | Me | Me |
| CH=CHCN | Cl | Me | Me |
| Ph | Cl | Me | Me |
| $CH_2Ph$ | Cl | Me | Me |
| OPh | Cl | Me | Me |
| SPh | Cl | Me | Me |
| $SO_2Ph$ | Cl | Me | Me |
| CONHMe | Cl | Me | Me |
| CONHEt | Cl | Me | Me |
| CONHPr-n | Cl | Me | Me |
| CONHPr-i | Cl | Me | Me |
| CONHBu-n | Cl | Me | Me |
| CONHBu-sec | Cl | Me | Me |
| CONHBu-t | Cl | Me | Me |
| $CON(Me)_2$ | Cl | Me | Me |
| $CON(Et)_2$ | Cl | Me | Me |
| $CON(Pr\text{-}n)_2$ | Cl | Me | Me |
| H | H | Me | Et |
| F | H | Me | Et |
| Cl | H | Me | Et |
| Br | H | Me | Et |
| I | H | Me | Et |
| $CH=CH_2$ | H | Me | Et |
| $CH_2CH=CH_2$ | H | Me | Et |
| $C\equiv CH$ | H | Me | Et |
| $CH_2C\equiv CH$ | H | Me | Et |
| $CH_2F$ | H | Me | Et |
| $CHF_2$ | H | Me | Et |
| CN | H | Me | Et |

| $R_1$ | $R_2$ | $R_3$ | $R_4$ |
|---|---|---|---|
| $NH_2$ | H | Me | Et |
| NHCOMe | H | Me | Et |
| $CH_2NH_2$ | H | Me | Et |
| $CH_2NHCOMe$ | H | Me | Et |
| $CH_2OCHF_2$ | H | Me | Et |
| $CH_2OCF_3$ | H | Me | Et |
| $CF_3$ | H | Me | Et |
| $NO_2$ | H | Me | Et |
| OMe | H | Me | Et |
| OEt | H | Me | Et |
| OPr-n | H | Me | Et |
| OBu-n | H | Me | Et |
| OPen-n | H | Me | Et |
| SMe | H | Me | Et |
| SEt | H | Me | Et |
| SPr-n | H | Me | Et |
| SBu-n | H | Me | Et |
| SPen-n | H | Me | Et |
| $SO_2Me$ | H | Me | Et |
| $SO_2Et$ | H | Me | Et |
| $SO_2Pr$-n | H | Me | Et |
| COMe | H | Me | Et |
| COEt | H | Me | Et |
| COPr-n | H | Me | Et |
| $CO_2H$ | H | Me | Et |
| $CO_2Me$ | H | Me | Et |
| $CO_2Et$ | H | Me | Et |
| $CO_2Pr$-n | H | Me | Et |
| $CO_2Bu$-n | H | Me | Et |
| $CO_2Pen$-n | H | Me | Et |
| $CH_2Cl$ | H | Me | Et |
| $CH_2Br$ | H | Me | Et |
| $CH_2I$ | H | Me | Et |
| $CH_2CF_3$ | H | Me | Et |
| $CH_2CN$ | H | Me | Et |
| $CH_2OH$ | H | Me | Et |
| $CH_2OMe$ | H | Me | Et |
| $CH_2OEt$ | H | Me | Et |
| $CH_2OPr$-n | H | Me | Et |
| $CH_2SMe$ | H | Me | Et |

| $R_1$ | $R_2$ | $R_3$ | $R_4$ |
|---|---|---|---|
| $CH_2SEt$ | H | Me | Et |
| $CH_2SPr$-n | H | Me | Et |
| $CH_2SO_2Me$ | H | Me | Et |
| $CH_2SO_2Et$ | H | Me | Et |
| $CH_2SO_2Pr$-n | H | Me | Et |
| $CH_2COMe$ | H | Me | Et |
| $CH_2COEt$ | H | Me | Et |
| $CH_2COPr$-n | H | Me | Et |
| $CH_2CO_2H$ | H | Me | Et |
| $CH_2CO_2Me$ | H | Me | Et |
| $CH_2CO_2Et$ | H | Me | Et |
| $CH_2CO_2Pr$-n | H | Me | Et |
| $CH_2OCH_2CH=CH_2$ | H | Me | Et |
| $CH_2OCH_2C\equiv CH$ | H | Me | Et |
| $CH=CHCO_2Me$ | H | Me | Et |
| $CH=CHCO_2Et$ | H | Me | Et |
| $CH=CHCO_2Pr$-n | H | Me | Et |
| CH=CHCN | H | Me | Et |
| Ph | H | Me | Et |
| $CH_2Ph$ | H | Me | Et |
| OPh | H | Me | Et |
| SPh | H | Me | Et |
| $SO_2Ph$ | H | Me | Et |
| CONHMe | H | Me | Et |
| CONHEt | H | Me | Et |
| CONHPr-n | H | Me | Et |
| CONHPr-i | H | Me | Et |
| CONHBu-n | H | Me | Et |
| CONHBu-sec | H | Me | Et |
| CONHBu-t | H | Me | Et |
| $CON(Me)_2$ | H | Me | Et |
| $CON(Et)_2$ | H | Me | Et |
| $CON(Pr\text{-}n)_2$ | H | Me | Et |
| H | H | Me | Pr-n |
| F | H | Me | Pr-n |
| Cl | H | Me | Pr-n |
| Br | H | Me | Pr-n |
| I | H | Me | Pr-n |
| $CH=CH_2$ | H | Me | Pr-n |
| $CH_2CH=CH_2$ | H | Me | Pr-n |

| $R_1$ | $R_2$ | $R_3$ | $R_4$ |
|---|---|---|---|
| $C\equiv CH$ | H | Me | Pr-n |
| $CH_2C\equiv CH$ | H | Me | Pr-n |
| $CH_2F$ | H | Me | Pr-n |
| $CHF_2$ | H | Me | Pr-n |
| CN | H | Me | Pr-n |
| $NH_2$ | H | Me | Pr-n |
| NHCOMe | H | Me | Pr-n |
| $CH_2NH_2$ | H | Me | Pr-n |
| $CH_2NHCOMe$ | H | Me | Pr-n |
| $CH_2OCHF_2$ | H | Me | Pr-n |
| $CH_2OCF_3$ | H | Me | Pr-n |
| $CF_3$ | H | Me | Pr-n |
| $NO_2$ | H | Me | Pr-n |
| OMe | H | Me | Pr-n |
| OEt | H | Me | Pr-n |
| OPr-n | H | Me | Pr-n |
| OBu-n | H | Me | Pr-n |
| OPen-n | H | Me | Pr-n |
| SMe | H | Me | Pr-n |
| SEt | H | Me | Pr-n |
| SPr-n | H | Me | Pr-n |
| SBu-n | H | Me | Pr-n |
| SPen-n | H | Me | Pr-n |
| $SO_2Me$ | H | Me | Pr-n |
| $SO_2Et$ | H | Me | Pr-n |
| $SO_2Pr$-n | H | Me | Pr-n |
| COMe | H | Me | Pr-n |
| COEt | H | Me | Pr-n |
| COPr-n | H | Me | Pr-n |
| $CO_2H$ | H | Me | Pr-n |
| $CO_2Me$ | H | Me | Pr-n |
| $CO_2Et$ | H | Me | Pr-n |
| $CO_2Pr$-n | H | Me | Pr-n |
| $CO_2Bu$-n | H | Me | Pr-n |
| $CO_2Pen$-n | H | Me | Pr-n |
| $CH_2Cl$ | H | Me | Pr-n |
| $CH_2Br$ | H | Me | Pr-n |
| $CH_2I$ | H | Me | Pr-n |
| $CH_2CF_3$ | H | Me | Pr-n |
| $CH_2CN$ | H | Me | Pr-n |

| $R_1$ | $R_2$ | $R_3$ | $R_4$ |
|---|---|---|---|
| $CH_2OH$ | H | Me | Pr-n |
| $CH_2OMe$ | H | Me | Pr-n |
| $CH_2OEt$ | H | Me | Pr-n |
| $CH_2OPr$-n | H | Me | Pr-n |
| $CH_2SMe$ | H | Me | Pr-n |
| $CH_2SEt$ | H | Me | Pr-n |
| $CH_2SPr$-n | H | Me | Pr-n |
| $CH_2SO_2Me$ | H | Me | Pr-n |
| $CH_2SO_2Et$ | H | Me | Pr-n |
| $CH_2SO_2Pr$-n | H | Me | Pr-n |
| $CH_2COMe$ | H | Me | Pr-n |
| $CH_2COEt$ | H | Me | Pr-n |
| $CH_2COPr$-n | H | Me | Pr-n |
| $CH_2CO_2H$ | H | Me | Pr-n |
| $CH_2CO_2Me$ | H | Me | Pr-n |
| $CH_2CO_2Et$ | H | Me | Pr-n |
| $CH_2CO_2Pr$-n | H | Me | Pr-n |
| $CH_2OCH_2CH=CH_2$ | H | Me | Pr-n |
| $CH_2OCH_2C\equiv CH$ | H | Me | Pr-n |
| $CH=CHCO_2Me$ | H | Me | Pr-n |
| $CH=CHCO_2Et$ | H | Me | Pr-n |
| $CH=CHCO_2Pr$-n | H | Me | Pr-n |
| CH=CHCN | H | Me | Pr-n |
| Ph | H | Me | Pr-n |
| $CH_2Ph$ | H | Me | Pr-n |
| OPh | H | Me | Pr-n |
| SPh | H | Me | Pr-n |
| $SO_2Ph$ | H | Me | Pr-n |
| CONHMe | H | Me | Pr-n |
| CONHEt | H | Me | Pr-n |
| CONHPr-n | H | Me | Pr-n |
| CONHPr-i | H | Me | Pr-n |
| CONHBu-n | H | Me | Pr-n |
| CONHBu-sec | H | Me | Pr-n |
| CONHBu-t | H | Me | Pr-n |
| $CON(Me)_2$ | H | Me | Pr-n |
| $CON(Et)_2$ | H | Me | Pr-n |
| $CON(Pr-n)_2$ | H | Me | Pr-n |
| H | H | Me | Pr-i |
| F | H | Me | Pr-i |

| $R_1$ | $R_2$ | $R_3$ | $R_4$ |
|---|---|---|---|
| Cl | H | Me | Pr-i |
| Br | H | Me | Pr-i |
| I | H | Me | Pr-i |
| $CH=CH_2$ | H | Me | Pr-i |
| $CH_2CH=CH_2$ | H | Me | Pr-i |
| $C\equiv CH$ | H | Me | Pr-i |
| $CH_2C\equiv CH$ | H | Me | Pr-i |
| $CH_2F$ | H | Me | Pr-i |
| $CHF_2$ | H | Me | Pr-i |
| CN | H | Me | Pr-i |
| $NH_2$ | H | Me | Pr-i |
| NHCOMe | H | Me | Pr-i |
| $CH_2NH_2$ | H | Me | Pr-i |
| $CH_2NHCOMe$ | H | Me | Pr-i |
| $CH_2OCHF_2$ | H | Me | Pr-i |
| $CH_2OCF_3$ | H | Me | Pr-i |
| $CF_3$ | H | Me | Pr-i |
| $NO_2$ | H | Me | Pr-i |
| OMe | H | Me | Pr-i |
| OEt | H | Me | Pr-i |
| OPr-n | H | Me | Pr-i |
| OBu-n | H | Me | Pr-i |
| OPen-n | H | Me | Pr-i |
| SMe | H | Me | Pr-i |
| SEt | H | Me | Pr-i |
| SPr-n | H | Me | Pr-i |
| SBu-n | H | Me | Pr-i |
| SPen-n | H | Me | Pr-i |
| $SO_2Me$ | H | Me | Pr-i |
| $SO_2Et$ | H | Me | Pr-i |
| $SO_2Pr$-n | H | Me | Pr-i |
| COMe | H | Me | Pr-i |
| COEt | H | Me | Pr-i |
| COPr-n | H | Me | Pr-i |
| $CO_2H$ | H | Me | Pr-i |
| $CO_2Me$ | H | Me | Pr-i |
| $CO_2Et$ | H | Me | Pr-i |
| $CO_2Pr$-n | H | Me | Pr-i |
| $CO_2Bu$-n | H | Me | Pr-i |
| $CO_2Pen$-n | H | Me | Pr-i |

| $R_1$ | $R_2$ | $R_3$ | $R_4$ |
|---|---|---|---|
| $CH_2Cl$ | H | Me | Pr-i |
| $CH_2Br$ | H | Me | Pr-i |
| $CH_2I$ | H | Me | Pr-i |
| $CH_2CF_3$ | H | Me | Pr-i |
| $CH_2CN$ | H | Me | Pr-i |
| $CH_2OH$ | H | Me | Pr-i |
| $CH_2OMe$ | H | Me | Pr-i |
| $CH_2OEt$ | H | Me | Pr-i |
| $CH_2OPr$-n | H | Me | Pr-i |
| $CH_2SMe$ | H | Me | Pr-i |
| $CH_2SEt$ | H | Me | Pr-i |
| $CH_2SPr$-n | H | Me | Pr-i |
| $CH_2SO_2Me$ | H | Me | Pr-i |
| $CH_2SO_2Et$ | H | Me | Pr-i |
| $CH_2SO_2Pr$-n | H | Me | Pr-i |
| $CH_2COMe$ | H | Me | Pr-i |
| $CH_2COEt$ | H | Me | Pr-i |
| $CH_2COPr$-n | H | Me | Pr-i |
| $CH_2CO_2H$ | H | Me | Pr-i |
| $CH_2CO_2Me$ | H | Me | Pr-i |
| $CH_2CO_2Et$ | H | Me | Pr-i |
| $CH_2CO_2Pr$-n | H | Me | Pr-i |
| $CH_2OCH_2CH=CH_2$ | H | Me | Pr-i |
| $CH_2OCH_2C\equiv CH$ | H | Me | Pr-i |
| $CH=CHCO_2Me$ | H | Me | Pr-i |
| $CH=CHCO_2Et$ | H | Me | Pr-i |
| $CH=CHCO_2Pr$-n | H | Me | Pr-i |
| CH=CHCN | H | Me | Pr-i |
| Ph | H | Me | Pr-i |
| $CH_2Ph$ | H | Me | Pr-i |
| OPh | H | Me | Pr-i |
| SPh | H | Me | Pr-i |
| $SO_2Ph$ | H | Me | Pr-i |
| CONHMe | H | Me | Pr-i |
| CONHEt | H | Me | Pr-i |
| CONHPr-n | H | Me | Pr-i |
| CONHPr-i | H | Me | Pr-i |
| CONHBu-n | H | Me | Pr-i |
| CONHBu-sec | H | Me | Pr-i |
| CONHBu-t | H | Me | Pr-i |

| $R_1$ | $R_2$ | $R_3$ | $R_4$ |
|---|---|---|---|
| $CON(Me)_2$ | H | Me | Pr-i |
| $CON(Et)_2$ | H | Me | Pr-i |
| $CON(Pr\text{-}n)_2$ | H | Me | Pr-i |
| H | H | Et | Et |
| F | H | Et | Et |
| Cl | H | Et | Et |
| Br | H | Et | Et |
| I | H | Et | Et |
| $CH=CH_2$ | H | Et | Et |
| $CH_2CH=CH_2$ | H | Et | Et |
| $C\equiv CH$ | H | Et | Et |
| $CH_2C\equiv CH$ | H | Et | Et |
| $CH_2F$ | H | Et | Et |
| $CHF_2$ | H | Et | Et |
| CN | H | Et | Et |
| $NH_2$ | H | Et | Et |
| NHCOMe | H | Et | Et |
| $CH_2NH_2$ | H | Et | Et |
| $CH_2NHCOMe$ | H | Et | Et |
| $CH_2OCHF_2$ | H | Et | Et |
| $CH_2OCF_3$ | H | Et | Et |
| $CF_3$ | H | Et | Et |
| $NO_2$ | H | Et | Et |
| OMe | H | Et | Et |
| OEt | H | Et | Et |
| OPr-n | H | Et | Et |
| OBu-n | H | Et | Et |
| OPen-n | H | Et | Et |
| SMe | H | Et | Et |
| SEt | H | Et | Et |
| SPr-n | H | Et | Et |
| SBu-n | H | Et | Et |
| SPen-n | H | Et | Et |
| $SO_2Me$ | H | Et | Et |
| $SO_2Et$ | H | Et | Et |
| $SO_2Pr\text{-}n$ | H | Et | Et |
| COMe | H | Et | Et |
| COEt | H | Et | Et |
| COPr-n | H | Et | Et |
| $CO_2H$ | H | Et | Et |

| $R_1$ | $R_2$ | $R_3$ | $R_4$ |
|---|---|---|---|
| $CO_2Me$ | H | Et | Et |
| $CO_2Et$ | H | Et | Et |
| $CO_2Pr$-n | H | Et | Et |
| $CO_2Bu$-n | H | Et | Et |
| $CO_2Pen$-n | H | Et | Et |
| $CH_2Cl$ | H | Et | Et |
| $CH_2Br$ | H | Et | Et |
| $CH_2I$ | H | Et | Et |
| $CH_2CF_3$ | H | Et | Et |
| $CH_2CN$ | H | Et | Et |
| $CH_2OH$ | H | Et | Et |
| $CH_2OMe$ | H | Et | Et |
| $CH_2OEt$ | H | Et | Et |
| $CH_2OPr$-n | H | Et | Et |
| $CH_2SMe$ | H | Et | Et |
| $CH_2SEt$ | H | Et | Et |
| $CH_2SPr$-n | H | Et | Et |
| $CH_2SO_2Me$ | H | Et | Et |
| $CH_2SO_2Et$ | H | Et | Et |
| $CH_2SO_2Pr$-n | H | Et | Et |
| $CH_2COMe$ | H | Et | Et |
| $CH_2COEt$ | H | Et | Et |
| $CH_2COPr$-n | H | Et | Et |
| $CH_2CO_2H$ | H | Et | Et |
| $CH_2CO_2Me$ | H | Et | Et |
| $CH_2CO_2Et$ | H | Et | Et |
| $CH_2CO_2Pr$-n | H | Et | Et |
| $CH_2OCH_2CH=CH_2$ | H | Et | Et |
| $CH_2OCH_2C\equiv CH$ | H | Et | Et |
| $CH=CHCO_2Me$ | H | Et | Et |
| $CH=CHCO_2Et$ | H | Et | Et |
| $CH=CHCO_2Pr$-n | H | Et | Et |
| CH=CHCN | H | Et | Et |
| Ph | H | Et | Et |
| $CH_2Ph$ | H | Et | Et |
| OPh | H | Et | Et |
| SPh | H | Et | Et |
| $SO_2Ph$ | H | Et | Et |
| CONHMe | H | Et | Et |
| CONHEt | H | Et | Et |

| $R_1$ | $R_2$ | $R_3$ | $R_4$ |
|---|---|---|---|
| CONHPr-n | H | Et | Et |
| CONHPr-i | H | Et | Et |
| CONHBu-n | H | Et | Et |
| CONHBu-sec | H | Et | Et |
| CONHBu-t | H | Et | Et |
| $CON(Me)_2$ | H | Et | Et |
| $CON(Et)_2$ | H | Et | Et |
| $CON(Pr-n)_2$ | H | Et | Et |
| H | H | F | H |
| H | H | Cl | H |
| H | H | Br | H |
| H | H | I | H |
| H | H | $CH=CH_2$ | H |
| H | H | $CH_2CH=CH_2$ | H |
| H | H | $C\equiv CH$ | H |
| H | H | $CH_2C\equiv CH$ | H |
| H | H | $CH_2F$ | H |
| H | H | $CHF_2$ | H |
| H | H | CN | H |
| H | H | $NH_2$ | H |
| H | H | NHCOMe | H |
| H | H | $CH_2NH_2$ | H |
| H | H | $CH_2NHCOMe$ | H |
| H | H | $CH_2OCHF_2$ | H |
| H | H | $CH_2OCF_3$ | H |
| H | H | $CF_3$ | H |
| H | H | $NO_2$ | H |
| H | H | OMe | H |
| H | H | OEt | H |
| H | H | OPr-n | H |
| H | H | OBu-n | H |

| $R_1$ | $R_2$ | $R_3$ | $R_4$ |
|---|---|---|---|
| H | H | OPen-n | H |
| H | H | SMe | H |
| H | H | SEt | H |
| H | H | SPr-n | H |
| H | H | SBu-n | H |
| H | H | SPen-n | H |
| H | H | $SO_2Me$ | H |
| H | H | $SO_2Et$ | H |
| H | H | $SO_2Pr$-n | H |
| H | H | COMe | H |
| H | H | COEt | H |
| H | H | COPr-n | H |
| H | H | $CO_2H$ | H |
| H | H | $CO_2Me$ | H |
| H | H | $CO_2Et$ | H |
| H | H | $CO_2Pr$-n | H |
| H | H | $CO_2Bu$-n | H |
| H | H | $CO_2Pen$-n | H |
| H | H | $CH_2Cl$ | H |
| H | H | $CH_2Br$ | H |
| H | H | $CH_2I$ | H |
| H | H | $CH_2CF_3$ | H |
| H | H | $CH_2CN$ | H |
| H | H | $CH_2OH$ | H |
| H | H | $CH_2OMe$ | H |
| H | H | $CH_2OEt$ | H |
| H | H | $CH_2OPr$-n | H |
| H | H | $CH_2SMe$ | H |
| H | H | $CH_2SEt$ | H |
| H | H | $CH_2SPr$-n | H |
| H | H | $CH_2SO_2Me$ | H |
| H | H | $CH_2SO_2Et$ | H |
| H | H | $CH_2SO_2Pr$-n | H |
| H | H | $CH_2OSO_2Me$ | H |
| H | H | $CH_2COMe$ | H |
| H | H | $CH_2COEt$ | H |
| H | H | $CH_2COPr$-n | H |
| H | H | $CH_2CO_2H$ | H |
| H | H | $CH_2CO_2Me$ | H |
| H | H | $CH_2CO_2Et$ | H |
| H | H | $CH_2CO_2Pr$-n | H |

| $R_1$ | $R_2$ | $R_3$ | $R_4$ |
|---|---|---|---|
| H | H | $CH_2OCH_2CH=CH_2$ | H |
| H | H | $CH_2OCH_2C\equiv CH$ | H |
| H | H | $CH=CHCO_2Me$ | H |
| H | H | $CH=CHCO_2Et$ | H |
| H | H | $CH=CHCO_2Pr$-n | H |
| H | H | CH=CHCN | H |
| H | H | Ph | H |
| H | H | Ph-2-F | H |
| H | H | Ph-3-F | H |
| H | H | Ph-4-F | H |
| H | H | Ph-2-Cl | H |
| H | H | Ph-3-Cl | H |
| H | H | Ph-4-Cl | H |
| H | H | Ph-2-Br | H |
| H | H | Ph-3-Br | H |
| H | H | Ph-4-Br | H |
| H | H | Ph-2-$CF_3$ | H |
| H | H | Ph-3-$CF_3$ | H |
| H | H | Ph-4-$CF_3$ | H |
| H | H | Ph-2-Me | H |
| H | H | Ph-3-Me | H |
| H | H | Ph-4-Me | H |
| H | H | Ph-2-Et | H |
| H | H | Ph-2-OMe | H |
| H | H | Ph-3-OMe | H |
| H | H | Ph-4-OMe | H |
| H | H | Ph-2-OEt | H |
| H | H | Ph-2-$CO_2Me$ | H |
| H | H | Ph-2-$CO_2Et$ | H |
| H | H | Ph-3-$CO_2Me$ | H |
| H | H | Ph-4-$CO_2Me$ | H |
| H | H | $CH_2Ph$ | H |
| H | H | $CH_2Ph$-2-F | H |
| H | H | $CH_2Ph$-3-F | H |
| H | H | $CH_2Ph$-4-F | H |
| H | H | $CH_2Ph$-2-Cl | H |
| H | H | $CH_2Ph$-3-Cl | H |
| H | H | $CH_2Ph$-4-Cl | H |
| H | H | $CH_2Ph$-2-Br | H |
| H | H | $CH_2Ph$-3-Br | H |

| $R_1$ | $R_2$ | $R_3$ | $R_4$ |
|---|---|---|---|
| H | H | $CH_2Ph$-4-Br | H |
| H | H | $CH_2Ph$-2-$CF_3$ | H |
| H | H | $CH_2Ph$-3-$CF_3$ | H |
| H | H | $CH_2Ph$-4-$CF_3$ | H |
| H | H | $CH_2Ph$-2-Me | H |
| H | H | $CH_2Ph$-3-Me | H |
| H | H | $CH_2Ph$-4-Me | H |
| H | H | $CH_2Ph$-2-Et | H |
| H | H | $CH_2Ph$-2-OMe | H |
| H | H | $CH_2Ph$-3-OMe | H |
| H | H | $CH_2Ph$-4-OMe | H |
| H | H | $CH_2Ph$-2-OEt | H |
| H | H | $CH_2Ph$-2-$CO_2Me$ | H |
| H | H | $CH_2Ph$-2-$CO_2Et$ | H |
| H | H | $CH_2Ph$-3-$CO_2Me$ | H |
| H | H | $CH_2Ph$-4-$CO_2Me$ | H |
| H | H | OPh | H |
| H | H | OPh-2-Cl | H |
| H | H | OPh-3-Cl | H |
| H | H | OPh-4-Cl | H |
| H | H | OPh-2-F | H |
| H | H | OPh-3-F | H |
| H | H | OPh-4-F | H |
| H | H | OPh-2-$CF_3$ | H |
| H | H | OPh-3-$CF_3$ | H |
| H | H | OPh-4-$CF_3$ | H |
| H | H | OPh-2-Me | H |
| H | H | OPh-3-Me | H |
| H | H | OPh-4-Me | H |
| H | H | OPh-2-Et | H |
| H | H | OPh-2-OMe | H |
| H | H | OPh-3-OMe | H |
| H | H | OPh-4-OMe | H |
| H | H | OPh-2-OEt | H |
| H | H | OPh-2-$CO_2Me$ | H |
| H | H | OPh-3-$CO_2Me$ | H |
| H | H | OPh-4-$CO_2Me$ | H |
| H | H | OPh-2-$CO_2Et$ | H |
| H | H | SPh | H |
| H | H | SPh-2-Cl | H |

| $R_1$ | $R_2$ | $R_3$ | $R_4$ |
|---|---|---|---|
| H | H | SPh-3-Cl | H |
| H | H | SPh-4-Cl | H |
| H | H | SPh-2-F | H |
| H | H | SPh-3-F | H |
| H | H | SPh-4-F | H |
| H | H | SPh-2-$CF_3$ | H |
| H | H | SPh-3-$CF_3$ | H |
| H | H | SPh-4-$CF_3$ | H |
| H | H | SPh-2-Me | H |
| H | H | SPh-3-Me | H |
| H | H | SPh-4-Me | H |
| H | H | SPh-2-Et | H |
| H | H | SPh-2-OMe | H |
| H | H | SPh-3-OMe | H |
| H | H | SPh-4-OMe | H |
| H | H | SPh-2-OEt | H |
| H | H | SPh-2-$CO_2$Me | H |
| H | H | SPh-3-$CO_2$Me | H |
| H | H | SPh-4-$CO_2$Me | H |
| H | H | SPh-2-$CO_2$Et | H |
| H | H | $SO_2$Ph | H |
| H | H | $SO_2$Ph-2-Cl | H |
| H | H | $SO_2$Ph-3-Cl | H |
| H | H | $SO_2$Ph-4-Cl | H |
| H | H | $SO_2$Ph-2-F | H |
| H | H | $SO_2$Ph-3-F | H |
| H | H | $SO_2$Ph-4-F | H |
| H | H | $SO_2$Ph-2-$CF_3$ | H |
| H | H | $SO_2$Ph-3-$CF_3$ | H |
| H | H | $SO_2$Ph-4-$CF_3$ | H |
| H | H | $SO_2$Ph-2-Me | H |
| H | H | $SO_2$Ph-3-Me | H |
| H | H | $SO_2$Ph-4-Me | H |
| H | H | $SO_2$Ph-2-Et | H |
| H | H | $SO_2$Ph-2-OMe | H |
| H | H | $SO_2$Ph-3-OMe | H |
| H | H | $SO_2$Ph-4-OMe | H |
| H | H | $SO_2$Ph-2-OEt | H |
| H | H | $SO_2$Ph-2-$CO_2$Me | H |
| H | H | $SO_2$Ph-3-$CO_2$Me | H |

| $R_1$ | $R_2$ | $R_3$ | $R_4$ |
|---|---|---|---|
| H | H | $SO_2Ph$-4-$CO_2Me$ | H |
| H | H | $SO_2Ph$-2-$CO_2Et$ | H |
| H | H | CONHMe | H |
| H | H | CONHEt | H |
| H | H | CONHPr-n | H |
| H | H | CONHPr-i | H |
| H | H | CONHBu-n | H |
| H | H | CONHBu-sec | H |
| H | H | CONHBu-t | H |
| H | H | $CON(Me)_2$ | H |
| H | H | $CON(Et)_2$ | H |
| H | H | $CON(Pr\text{-}n)_2$ | H |
| F | H | H | H |
| Cl | H | H | H |
| Br | H | H | H |
| I | H | H | H |
| $CH=CH_2$ | H | H | H |
| $CH_2CH=CH_2$ | H | H | H |
| $C\equiv CH$ | H | H | H |
| $CH_2C\equiv CH$ | H | H | H |
| $CH_2F$ | H | H | H |
| $CHF_2$ | H | H | H |
| CN | H | H | H |
| $NH_2$ | H | H | H |
| NHCOMe | H | H | H |
| $CH_2NH_2$ | H | H | H |
| $CH_2NHCOMe$ | H | H | H |
| $CH_2OCHF_2$ | H | H | H |
| $CH_2OCF_3$ | H | H | H |
| $CF_3$ | H | H | H |
| $NO_2$ | H | H | H |
| OMe | H | H | H |
| OEt | H | H | H |
| OPr-n | H | H | H |
| OBu-n | H | H | H |
| OPen-n | H | H | H |
| SMe | H | H | H |
| SEt | H | H | H |
| SPr-n | H | H | H |
| SBu-n | H | H | H |

| $R_1$ | $R_2$ | $R_3$ | $R_4$ |
|---|---|---|---|
| SPen-n | H | H | H |
| $SO_2Me$ | H | H | H |
| $SO_2Et$ | H | H | H |
| $SO_2Pr$-n | H | H | H |
| COMe | H | H | H |
| COEt | H | H | H |
| COPr-n | H | H | H |
| $CO_2H$ | H | H | H |
| $CO_2Me$ | H | H | H |
| $CO_2Et$ | H | H | H |
| $CO_2Pr$-n | H | H | H |
| $CO_2Bu$-n | H. | H | H |
| $CO_2Pen$-n | H | H | H |
| $CH_2Cl$ | H | H | H |
| $CH_2Br$ | H | H | H |
| $CH_2I$ | H | H | H |
| $CH_2CF_3$ | H | H | H |
| $CH_2CN$ | H | H | H |
| $CH_2OH$ | H | H | H |
| $CH_2OMe$ | H | H | H |
| $CH_2OEt$ | H | H | H |
| $CH_2OPr$-n | H | H | H |
| $CH_2SMe$ | H | H | H |
| $CH_2SEt$ | H | H | H |
| $CH_2SPr$-n | H | H | H |
| $CH_2SO_2Me$ | H | H | H |
| $CH_2SO_2Et$ | H | H | H |
| $CH_2SO_2Pr$-n | H | H | H |
| $CH_2COMe$ | H | H | H |
| $CH_2COEt$ | H | H | H |
| $CH_2COPr$-n | H | H | H |
| $CH_2CO_2H$ | H | H | H |
| $CH_2CO_2Me$ | H | H | H |
| $CH_2CO_2Et$ | H | H | H |
| $CH_2CO_2Pr$-n | H | H | H |
| $CH_2OCH_2CH=CH_2$ | H | H | H |
| $CH_2OCH_2C\equiv CH$ | H | H | H |
| $CH=CHCO_2Me$ | H | H | H |
| $CH=CHCO_2Et$ | H | H | H |
| $CH=CHCO_2Pr$-n | H | H | H |

| $R_1$ | $R_2$ | $R_3$ | $R_4$ |
|---|---|---|---|
| CH=CHCN | H | H | H |
| Ph | H | H | H |
| Ph-2-F | H | H | H |
| Ph-3-F | H | H | H |
| Ph-4-F | H | H | H |
| Ph-2-Cl | H | H | H |
| Ph-3-Cl | H | H | H |
| Ph-4-Cl | H | H | H |
| Ph-2-Br | H | H | H |
| Ph-3-Br | H | H | H |
| Ph-4-Br | H | H | H |
| Ph-2-$CF_3$ | H | H | H |
| Ph-3-$CF_3$ | H | H | H |
| Ph-4-$CF_3$ | H | H | H |
| Ph-2-Me | H | H | H |
| Ph-3-Me | H | H | H |
| Ph-4-Me | H | H | H |
| Ph-2-Et | H | H | H |
| Ph-2-OMe | H | H | H |
| Ph-3-OMe | H | H | H |
| Ph-4-OMe | H | H | H |
| Ph-2-OEt | H | H | H |
| Ph-2-$CO_2$Me | H | H | H |
| Ph-2-$CO_2$Et | H | H | H |
| Ph-3-$CO_2$Me | H | H | H |
| Ph-4-$CO_2$Me | H | H | H |
| $CH_2$Ph | H | H | H |
| $CH_2$Ph-2-F | H | H | H |
| $CH_2$Ph-3-F | H | H | H |
| $CH_2$Ph-4-F | H | H | H |
| $CH_2$Ph-2-Cl | H | H | H |
| $CH_2$Ph-3-Cl | H | H | H |
| $CH_2$Ph-4-Cl | H | H | H |
| $CH_2$Ph-2-Br | H | H | H |
| $CH_2$Ph-3-Br | H | H | H |
| $CH_2$Ph-4-Br | H | H | H |
| $CH_2$Ph-2-$CF_3$ | H | H | H |
| $CH_2$Ph-3-$CF_3$ | H | H | H |
| $CH_2$Ph-4-$CF_3$ | H | H | H |
| $CH_2$Ph-2-Me | H | H | H |

| $R_1$ | $R_2$ | $R_3$ | $R_4$ |
|---|---|---|---|
| $CH_2Ph$-3-Me | H | H | H |
| $CH_2Ph$-4-Me | H | H | H |
| $CH_2Ph$-2-Et | H | H | H |
| $CH_2Ph$-2-OMe | H | H | H |
| $CH_2Ph$-3-OMe | H | H | H |
| $CH_2Ph$-4-OMe | H | H | H |
| $CH_2Ph$-2-OEt | H | H | H |
| $CH_2Ph$-2-$CO_2Me$ | H | H | H |
| $CH_2Ph$-2-$CO_2Et$ | H | H | H |
| $CH_2Ph$-3-$CO_2Me$ | H | H | H |
| $CH_2Ph$-4-$CO_2Me$ | H | H | H |
| OPh | H | H | H |
| OPh-2-Cl | H | H | H |
| OPh-3-Cl | H | H | H |
| OPh-4-Cl | H | H | H |
| OPh-2-F | H | H | H |
| OPh-3-F | H | H | H |
| OPh-4-F | H | H | H |
| OPh-2-$CF_3$ | H | H | H |
| OPh-3-$CF_3$ | H | H | H |
| OPh-4-$CF_3$ | H | H | H |
| OPh-2-Me | H | H | H |
| OPh-3-Me | H | H | H |
| OPh-4-Me | H | H | H |
| OPh-2-Et | H | H | H |
| OPh-2-OMe | H | H | H |
| OPh-3-OMe | H | H | H |
| OPh-4-OMe | H | H | H |
| OPh-2-OEt | H | H | H |
| OPh-2-$CO_2Me$ | H | H | H |
| OPh-3-$CO_2Me$ | H | H | H |
| OPh-4-$CO_2Me$ | H | H | H |
| OPh-2-$CO_2Et$ | H | H | H |
| SPh | H | H | H |
| SPh-2-Cl | H | H | H |
| SPh-3-Cl | H | H | H |
| SPh-4-Cl | H | H | H |
| SPh-2-F | H | H | H |
| SPh-3-F | H | H | H |
| SPh-4-F | H | H | H |

| $R_1$ | $R_2$ | $R_3$ | $R_4$ |
|---|---|---|---|
| SPh-2-$CF_3$ | H | H | H |
| SPh-3-$CF_3$ | H | H | H |
| SPh-4-$CF_3$ | H | H | H |
| SPh-2-Me | H | H | H |
| SPh-3-Me | H | H | H |
| SPh-4-Me | H | H | H |
| SPh-2-Et | H | H | H |
| SPh-2-OMe | H | H | H |
| SPh-3-OMe | H | H | H |
| SPh-4-OMe | H | H | H |
| SPh-2-OEt | H | H | H |
| SPh-2-$CO_2$Me | H | H | H |
| SPh-3-$CO_2$Me | H | H | H |
| SPh-4-$CO_2$Me | H | H | H |
| SPh-2-$CO_2$Et | H | H | H |
| $SO_2$Ph | H | H | H |
| $SO_2$Ph-2-Cl | H | H | H |
| $SO_2$Ph-3-Cl | H | H | H |
| $SO_2$Ph-4-Cl | H | H | H |
| $SO_2$Ph-2-F | H | H | H |
| $SO_2$Ph-3-F | H | H | H |
| $SO_2$Ph-4-F | H | H | H |
| $SO_2$Ph-2-$CF_3$ | H | H | H |
| $SO_2$Ph-3-$CF_3$ | H | H | H |
| $SO_2$Ph-4-$CF_3$ | H | H | H |
| $SO_2$Ph-2-Me | H | H | H |
| $SO_2$Ph-3-Me | H | H | H |
| $SO_2$Ph-4-Me | H | H | H |
| $SO_2$Ph-2-Et | H | H | H |
| $SO_2$Ph-2-OMe | H | H | H |
| $SO_2$Ph-3-OMe | H | H | H |
| $SO_2$Ph-4-OMe | H | H | H |
| $SO_2$Ph-2-OEt | H | H | H |
| $SO_2$Ph-2-$CO_2$Me | H | H | H |
| $SO_2$Ph-3-$CO_2$Me | H | H | H |
| $SO_2$Ph-4-$CO_2$Me | H | H | H |
| $SO_2$Ph-2-$CO_2$Et | H | H | H |
| CONHMe | H | H | H |
| CONHEt | H | H | H |
| CONHPr-n | H | H | H |

| $R_1$ | $R_2$ | $R_3$ | $R_4$ |
|---|---|---|---|
| CONHPr-i | H | H | H |
| CONHBu-n | H | H | H |
| CONHBu-sec | H | H | H |
| CONHBu-t | H | H | H |
| $CON(Me)_2$ | H | H | H |
| $CON(Et)_2$ | H | H | H |
| $CON(Pr-n)_2$ | H | H | H |

| $R_1$ | $R_2$ | $R_3$ | $R_4$ |
|---|---|---|---|
| H | F | H | H |
| H | Cl | H | H |
| H | Br | H | H |
| H | I | H | H |
| H | $CH=CH_2$ | H | H |
| H | $CH_2CH=CH_2$ | H | H |
| H | $C\equiv CH$ | H | H |
| H | $CH_2C\equiv CH$ | H | H |
| H | $CH_2F$ | H | H |
| H | $CHF_2$ | H | H |
| H | CN | H | H |
| H | $NH_2$ | H | H |
| H | NHCOMe | H | H |
| H | $CH_2NH_2$ | H | H |
| H | $CH_2NHCOMe$ | H | H |
| H | $CH_2OCHF_2$ | H | H |
| H | $CH_2OCF_3$ | H | H |
| H | $CF_3$ | H | H |
| H | $NO_2$ | H | H |
| H | OMe | H | H |
| H | OEt | H | H |
| H | OPr-n | H | H |
| H | OBu-n | H | H |
| H | OPen-n | H | H |
| H | SMe | H | H |
| H | SEt | H | H |
| H | SPr-n | H | H |
| H | SBu-n | H | H |
| H | SPen-n | H | H |
| H | $SO_2Me$ | H | H |
| H | $SO_2Et$ | H | H |
| H | $SO_2Pr$-n | H | H |
| H | COMe | H | H |
| H | COEt | H | H |
| H | COPr-n | H | H |
| H | $CO_2H$ | H | H |
| H | $CO_2Me$ | H | H |
| H | $CO_2Et$ | H | H |
| H | $CO_2Pr$-n | H | H |
| H | $CO_2Bu$-n | H | H |

| $R_1$ | $R_2$ | $R_3$ | $R_4$ |
|---|---|---|---|
| H | $CO_2Pen$-n | H | H |
| H | $CH_2Cl$ | H | H |
| H | $CH_2Br$ | H | H |
| H | $CH_2I$ | H | H |
| H | $CH_2CF_3$ | H | H |
| H | $CH_2CN$ | H | H |
| H | $CH_2OH$ | H | H |
| H | $CH_2OMe$ | H | H |
| H | $CH_2OEt$ | H | H |
| H | $CH_2OPr$-n | H | H |
| H | $CH_2SMe$ | H | H |
| H | $CH_2SEt$ | H | H |
| H | $CH_2SPr$-n | H | H |
| H | $CH_2SO_2Me$ | H | H |
| H | $CH_2SO_2Et$ | H | H |
| H | $CH_2SO_2Pr$-n | H | H |
| H | $CH_2COMe$ | H | H |
| H | $CH_2COEt$ | H | H |
| H | $CH_2COPr$-n | H | H |
| H | $CH_2CO_2H$ | H | H |
| H | $CH_2CO_2Me$ | H | H |
| H | $CH_2CO_2Et$ | H | H |
| H | $CH_2CO_2Pr$-n | H | H |
| H | $CH_2OCH_2CH=CH_2$ | H | H |
| H | $CH_2OCH_2C\equiv CH$ | H | H |
| H | $CH=CHCO_2Me$ | H | H |
| H | $CH=CHCO_2Et$ | H | H |
| H | $CH=CHCO_2Pr$-n | H | H |
| H | CH=CHCN | H | H |
| H | Ph | H | H |
| H | Ph-2-F | H | H |
| H | Ph-3-F | H | H |
| H | Ph-4-F | H | H |
| H | Ph-2-Cl | H | H |
| H | Ph-3-Cl | H | H |
| H | Ph-4-Cl | H | H |
| H | Ph-2-Br | H | H |
| H | Ph-3-Br | H | H |
| H | Ph-4-Br | H | H |
| H | Ph-2-$CF_3$ | H | H |

| $R_1$ | $R_2$ | $R_3$ | $R_4$ |
|---|---|---|---|
| H | Ph-3-$CF_3$ | H | H |
| H | Ph-4-$CF_3$ | H | H |
| H | Ph-2-Me | H | H |
| H | Ph-3-Me | H | H |
| H | Ph-4-Me | H | H |
| H | Ph-2-Et | H | H |
| H | Ph-2-OMe | H | H |
| H | Ph-3-OMe | H | H |
| H | Ph-4-OMe | H | H |
| H | Ph-2-OEt | H | H |
| H | Ph-2-$CO_2$Me | H | H |
| H | Ph-2-$CO_2$Et | H | H |
| H | Ph-3-$CO_2$Me | H | H |
| H | Ph-4-$CO_2$Me | H | H |
| H | $CH_2$Ph | H | H |
| H | $CH_2$Ph-2-F | H | H |
| H | $CH_2$Ph-3-F | H | H |
| H | $CH_2$Ph-4-F | H | H |
| H | $CH_2$Ph-2-Cl | H | H |
| H | $CH_2$Ph-3-Cl | H | H |
| H | $CH_2$Ph-4-Cl | H | H |
| H | $CH_2$Ph-2-Br | H | H |
| H | $CH_2$Ph-3-Br | H | H |
| H | $CH_2$Ph-4-Br | H | H |
| H | $CH_2$Ph-2-$CF_3$ | H | H |
| H | $CH_2$Ph-3-$CF_3$ | H | H |
| H | $CH_2$Ph-4-$CF_3$ | H | H |
| H | $CH_2$Ph-2-Me | H | H |
| H | $CH_2$Ph-3-Me | H | H |
| H | $CH_2$Ph-4-Me | H | H |
| H | $CH_2$Ph-2-Et | H | H |
| H | $CH_2$Ph-2-OMe | H | H |
| H | $CH_2$Ph-3-OMe | H | H |
| H | $CH_2$Ph-4-OMe | H | H |
| H | $CH_2$Ph-2-OEt | H | H |
| H | $CH_2$Ph-2-$CO_2$Me | H | H |
| H | $CH_2$Ph-2-$CO_2$Et | H | H |
| H | $CH_2$Ph-3-$CO_2$Me | H | H |
| H | $CH_2$Ph-4-$CO_2$Me | H | H |
| H | OPh | H | H |

| $R_1$ | $R_2$ | $R_3$ | $R_4$ |
|---|---|---|---|
| H | OPh-2-Cl | H | H |
| H | OPh-3-Cl | H | H |
| H | OPh-4-Cl | H | H |
| H | OPh-2-F | H | H |
| H | OPh-3-F | H | H |
| H | OPh-4-F | H | H |
| H | OPh-2-$CF_3$ | H | H |
| H | OPh-3-$CF_3$ | H | H |
| H | OPh-4-$CF_3$ | H | H |
| H | OPh-2-Me | H | H |
| H | OPh-3-Me | H | H |
| H | OPh-4-Me | H | H |
| H | OPh-2-Et | H | H |
| H | OPh-2-OMe | H | H |
| H | OPh-3-OMe | H | H |
| H | OPh-4-OMe | H | H |
| H | OPh-2-OEt | H | H |
| H | OPh-2-$CO_2$Me | H | H |
| H | OPh-3-$CO_2$Me | H | H |
| H | OPh-4-$CO_2$Me | H | H |
| H | OPh-2-$CO_2$Et | H | H |
| H | SPh | H | H |
| H | SPh-2-Cl | H | H |
| H | SPh-3-Cl | H | H |
| H | SPh-4-Cl | H | H |
| H | SPh-2-F | H | H |
| H | SPh-3-F | H | H |
| H | SPh-4-F | H | H |
| H | SPh-2-$CF_3$ | H | H |
| H | SPh-3-$CF_3$ | H | H |
| H | SPh-4-$CF_3$ | H | H |
| H | SPh-2-Me | H | H |
| H | SPh-3-Me | H | H |
| H | SPh-4-Me | H | H |
| H | SPh-2-Et | H | H |
| H | SPh-2-OMe | H | H |
| H | SPh-3-OMe | H | H |
| H | SPh-4-OMe | H | H |
| H | SPh-2-OEt | H | H |
| H | SPh-2-$CO_2$Me | H | H |

| $R_1$ | $R_2$ | $R_3$ | $R_4$ |
|---|---|---|---|
| H | SPh-3-$CO_2$Me | H | H |
| H | SPh-4-$CO_2$Me | H | H |
| H | SPh-2-$CO_2$Et | H | H |
| H | $SO_2$Ph | H | H |
| H | $SO_2$Ph-2-Cl | H | H |
| H | $SO_2$Ph-3-Cl | H | H |
| H | $SO_2$Ph-4-Cl | H | H |
| H | $SO_2$Ph-2-F | H | H |
| H | $SO_2$Ph-3-F | H | H |
| H | $SO_2$Ph-4-F | H | H |
| H | $SO_2$Ph-2-$CF_3$ | H | H |
| H | $SO_2$Ph-3-$CF_3$ | H | H |
| H | $SO_2$Ph-4-$CF_3$ | H | H |
| H | $SO_2$Ph-2-Me | H | H |
| H | $SO_2$Ph-3-Me | H | H |
| H | $SO_2$Ph-4-Me | H | H |
| H | $SO_2$Ph-2-Et | H | H |
| H | $SO_2$Ph-2-OMe | H | H |
| H | $SO_2$Ph-3-OMe | H | H |
| H | $SO_2$Ph-4-OMe | H | H |
| H | $SO_2$Ph-2-OEt | H | H |
| H | $CH_2$Ph-2-$CO_2$Me | H | H |
| H | $CH_2$Ph-3-$CO_2$Me | H | H |
| H | $CH_2$Ph-4-$CO_2$Me | H | H |
| H | $CH_2$Ph-2-$CO_2$Et | H | H |
| H | CONHMe | H | H |
| H | CONHEt | H | H |
| H | CONHPr-n | H | H |
| H | CONHPr-i | H | H |
| H | CONHBu-n | H | H |
| H | CONHBu-sec | H | H |
| H | CONHBu-t | H | H |
| H | $CON(Me)_2$ | H | H |
| H | $CON(Et)_2$ | H | H |
| H | $CON(Pr-n)_2$ | H | H |
| Me | Me | H | H |
| Me | H | Me | H |
| Me | Et | H | H |
| Me | H | Et | H |
| Me | Pr-n | H | H |

| $R_1$ | $R_2$ | $R_3$ | $R_4$ |
|---|---|---|---|
| Me | H | Pr-n | H |
| Me | Pr-i | H | H |
| Me | H | Pr-i | H |
| Me | Bu-n | H | H |
| Me | H | Bu-n | H |
| Et | Me | H | H |
| Et | H | Me | H |
| Et | Et | H | H |
| Et | H | Et | H |
| Et | Pr-n | H | H |
| Et | H | Pr-n | H |
| Et | Pr-i | H | H |
| Et | H | Pr-i | H |
| Et | Bu-n | H | H |
| Et | H | Bu-n | H |
| Pr-n | Me | H | H |
| Pr-n | H | Me | H |
| Pr-n | Et | H | H |
| Pr-n | H | Et | H |
| Pr-n | Pr-n | H | H |
| Pr-n | H | Pr-n | H |
| Pr-n | Pr-i | H | H |
| Pr-n | H | Pr-i | H |
| Pr-n | Bu-n | H | H |
| Pr-n | H | Bu-n | H |
| Pr-i | Me | H | H |
| Pr-i | H | Me | H |
| Pr-i | Et | H | H |
| Pr-i | H | Et | H |
| Pr-i | Pr-n | H | H |
| Pr-i | H | Pr-n | H |
| Pr-i | Pr-i | H | H |
| Pr-i | H | Pr-i | H |
| Pr-i | Bu-n | H | H |
| Pr-i | H | Bu-n | H |
| Bu-n | Me | H | H |
| Bu-n | H | Me | H |
| Bu-n | Et | H | H |
| Bu-n | H | Et | H |
| Bu-n | Pr-n | H | H |

| $R_1$ | $R_2$ | $R_3$ | $R_4$ |
|---|---|---|---|
| Bu-n | H | Pr-n | H |
| Bu-n | Pr-i | H | H |
| Bu-n | H | Pr-i | H |
| Bu-n | Bu-n | H | H |
| Bu-n | H | Bu-n | H |
| H | Me | Me | H |
| H | Me | Et | H |
| H | Me | Pr-n | H |
| H | Me | Pr-i | H |
| H | Me | Bu-n | H |
| H | Et | Me | H |
| H | Et | Et | H |
| H | Et | Pr-n | H |
| H | Et | Pr-i | H |
| H | Et | Bu-n | H |
| H | Pr-n | Me | H |
| H | Pr-n | Et | H |
| H | Pr-n | Pr-n | H |
| H | Pr-n | Pr-i | H |
| H | Pr-n | Bu-n | H |
| H | Pr-i | Me | H |
| H | Pr-i | Et | H |
| H | Pr-i | Pr-n | H |
| H | Pr-i | Pr-i | H |
| H | Pr-i | Bu-n | H |
| H | Bu-n | Me | H |
| H | Bu-n | Et | H |
| H | Bu-n | Pr-n | H |
| H | Bu-n | Pr-i | H |
| H | Bu-n | Bu-n | H |
| Me | Me | Me | H |
| Me | Me | Et | H |
| Me | Me | Pr-n | H |
| Me | Me | Pr-i | H |
| Me | Me | Bu-n | H |
| Me | Et | Me | H |
| Me | Et | Et | H |
| Me | Et | Pr-n | H |
| Me | Et | Pr-i | H |
| Me | Et | Bu-n | H |

| $R_1$ | $R_2$ | $R_3$ | $R_4$ |
|---|---|---|---|
| Me | Pr-n | Me | H |
| Me | Pr-n | Et | H |
| Me | Pr-n | Pr-n | H |
| Me | Pr-n | Pr-i | H |
| Me | Pr-n | Bu-n | H |
| Me | Pr-i | Me | H |
| Me | Pr-i | Et | H |
| Me | Pr-i | Pr-n | H |
| Me | Pr-i | Pr-i | H |
| Me | Pr-i | Bu-n | H |
| Me | Bu-n | Me | H |
| Me | Bu-n | Et | H |
| Me | Bu-n | Pr-n | H |
| Me | Bu-n | Pr-i | H |
| Me | Bu-n | Bu-n | H |
| Et | Me | Me | H |
| Et | Me | Et | H |
| Et | Me | Pr-n | H |
| Et | Me | Pr-i | H |
| Et | Me | Bu-n | H |
| Et | Et | Me | H |
| Et | Et | Et | H |
| Et | Et | Pr-n | H |
| Et | Et | Pr-i | H |
| Et | Et | Bu-n | H |
| Et | Pr-n | Me | H |
| Et | Pr-n | Et | H |
| Et | Pr-n | Pr-n | H |
| Et | Pr-n | Pr-i | H |
| Et | Pr-n | Bu-n | H |
| Et | Pr-i | Me | H |
| Et | Pr-i | Et | H |
| Et | Pr-i | Pr-n | H |
| Et | Pr-i | Pr-i | H |
| Et | Pr-i | Bu-n | H |
| Et | Bu-n | Me | H |
| Et | Bu-n | Et | H |
| Et | Bu-n | Pr-n | H |
| Et | Bu-n | Pr-i | H |
| Et | Bu-n | Bu-n | H |

| $R_1$ | $R_2$ | $R_3$ | $R_4$ |
|---|---|---|---|
| Pr-n | Me | Me | H |
| Pr-n | Me | Et | H |
| Pr-n | Me | Pr-n | H |
| Pr-n | Me | Pr-i | H |
| Pr-n | Me | Bu-n | H |
| Pr-n | Et | Me | H |
| Pr-n | Et | Et | H |
| Pr-n | Et | Pr-n | H |
| Pr-n | Et | Pr-i | H |
| Pr-n | Et | Bu-n | H |
| Pr-n | Pr-n | Me | H |
| Pr-n | Pr-n | Et | H |
| Pr-n | Pr-n | Pr-n | H |
| Pr-n | Pr-n | Pr-i | H |
| Pr-n | Pr-n | Bu-n | H |
| Pr-n | Pr-i | Me | H |
| Pr-n | Pr-i | Et | H |
| Pr-n | Pr-i | Pr-n | H |
| Pr-n | Pr-i | Pr-i | H |
| Pr-n | Pr-i | Bu-n | H |
| Pr-n | Bu-n | Me | H |
| Pr-n | Bu-n | Et | H |
| Pr-n | Bu-n | Pr-n | H |
| Pr-n | Bu-n | Pr-i | H |
| Pr-n | Bu-n | Bu-n | H |
| Pr-i | Me | Me | H |
| Pr-i | Me | Et | H |
| Pr-i | Me | Pr-n | H |
| Pr-i | Me | Pr-i | H |
| Pr-i | Me | Bu-n | H |
| Pr-i | Et | Me | H |
| Pr-i | Et | Et | H |
| Pr-i | Et | Pr-n | H |
| Pr-i | Et | Pr-i | H |
| Pr-i | Et | Bu-n | H |
| Pr-i | Pr-n | Me | H |
| Pr-i | Pr-n | Et | H |
| Pr-i | Pr-n | Pr-n | H |
| Pr-i | Pr-n | Pr-i | H |
| Pr-i | Pr-n | Bu-n | H |

| $R_1$ | $R_2$ | $R_3$ | $R_4$ |
|---|---|---|---|
| Pr-i | Pr-i | Me | H |
| Pr-i | Pr-i | Et | H |
| Pr-i | Pr-i | Pr-n | H |
| Pr-i | Pr-i | Pr-i | H |
| Pr-i | Pr-i | Bu-n | H |
| Pr-i | Bu-n | Me | H |
| Pr-i | Bu-n | Et | H |
| Pr-i | Bu-n | Pr-n | H |
| Pr-i | Bu-n | Pr-i | H |
| Pr-i | Bu-n | Bu-n | H |
| Bu-n | Me | Me | H |
| Bu-n | Me | Et | H |
| Bu-n | Me | Pr-n | H |
| Bu-n | Me | Pr-i | H |
| Bu-n | Me | Bu-n | H |
| Bu-n | Et | Me | H |
| Bu-n | Et | Et | H |
| Bu-n | Et | Pr-n | H |
| Bu-n | Et | Pr-i | H |
| Bu-n | Et | Bu-n | H |
| Bu-n | Pr-n | Me | H |
| Bu-n | Pr-n | Et | H |
| Bu-n | Pr-n | Pr-n | H |
| Bu-n | Pr-n | Pr-i | H |
| Bu-n | Pr-n | Bu-n | H |
| Bu-n | Pr-i | Me | H |
| Bu-n | Pr-i | Et | H |
| Bu-n | Pr-i | Pr-n | H |
| Bu-n | Pr-i | Pr-i | H |
| Bu-n | Pr-i | Bu-n | H |
| Bu-n | Bu-n | Me | H |
| Bu-n | Bu-n | Et | H |
| Bu-n | Bu-n | Pr-n | H |
| Bu-n | Bu-n | Pr-i | H |
| Bu-n | Bu-n | Bu-n | H |
| F | H | Me | H |
| Cl | H | Me | H |
| Br | H | Me | H |
| I | H | Me | H |
| $CH=CH_2$ | H | Me | H |

| $R_1$ | $R_2$ | $R_3$ | $R_4$ |
|---|---|---|---|
| $CH_2CH{=}CH_2$ | H | Me | H |
| $C{\equiv}CH$ | H | Me | H |
| $CH_2C{\equiv}CH$ | H | Me | H |
| $CH_2F$ | H | Me | H |
| $CHF_2$ | H | Me | H |
| CN | H | Me | H |
| $NH_2$ | H | Me | H |
| NHCOMe | H | Me | H |
| $CH_2NH_2$ | H | Me | H |
| $CH_2NHCOMe$ | H | Me | H |
| $CH_2OCHF_2$ | H | Me | H |
| $CH_2OCF_3$ | H | Me | H |
| $CF_3$ | H | Me | H |
| $NO_2$ | H | Me | H |
| OMe | H | Me | H |
| OEt | H | Me | H |
| OPr-n | H | Me | H |
| OBu-n | H | Me | H |
| OPen-n | H | Me | H |
| SMe | H | Me | H |
| SEt | H | Me | H |
| SPr-n | H | Me | H |
| SBu-n | H | Me | H |
| SPen-n | H | Me | H |
| $SO_2Me$ | H | Me | H |
| $SO_2Et$ | H | Me | H |
| $SO_2Pr$-n | H | Me | H |
| COMe | H | Me | H |
| COEt | H | Me | H |
| COPr-n | H | Me | H |
| $CO_2H$ | H | Me | H |
| $CO_2Me$ | H | Me | H |
| $CO_2Et$ | H | Me | H |
| $CO_2Pr$-n | H | Me | H |
| $CO_2Bu$-n | H | Me | H |
| $CO_2Pen$-n | H | Me | H |
| $CH_2Cl$ | H | Me | H |
| $CH_2Br$ | H | Me | H |
| $CH_2I$ | H | Me | H |
| $CH_2CF_3$ | H | Me | H |

| $R_1$ | $R_2$ | $R_3$ | $R_4$ |
|---|---|---|---|
| $CH_2CN$ | H | Me | H |
| $CH_2OH$ | H | Me | H |
| $CH_2OMe$ | H | Me | H |
| $CH_2OEt$ | H | Me | H |
| $CH_2OPr$-n | H | Me | H |
| $CH_2SMe$ | H | Me | H |
| $CH_2SEt$ | H | Me | H |
| $CH_2SPr$-n | H | Me | H |
| $CH_2SO_2Me$ | H | Me | H |
| $CH_2SO_2Et$ | H | Me | H |
| $CH_2SO_2Pr$-n | H | Me | H |
| $CH_2COMe$ | H | Me | H |
| $CH_2COEt$ | H | Me | H |
| $CH_2COPr$-n | H | Me | H |
| $CH_2CO_2H$ | H | Me | H |
| $CH_2CO_2Me$ | H | Me | H |
| $CH_2CO_2Et$ | H | Me | H |
| $CH_2CO_2Pr$-n | H | Me | H |
| $CH_2OCH_2CH=CH_2$ | H | Me | H |
| $CH_2OCH_2C\equiv CH$ | H | Me | H |
| $CH=CHCO_2Me$ | H | Me | H |
| $CH=CHCO_2Et$ | H | Me | H |
| $CH=CHCO_2Pr$-n | H | Me | H |
| CH=CHCN | H | Me | H |
| Ph | H | Me | H |
| Ph-2-F | H | Me | H |
| Ph-3-F | H | Me | H |
| Ph-4-F | H | Me | H |
| Ph-2-Cl | H | Me | H |
| Ph-3-Cl | H | Me | H |
| Ph-4-Cl | H | Me | H |
| Ph-2-Br | H | Me | H |
| Ph-3-Br | H | Me | H |
| Ph-4-Br | H | Me | H |
| Ph-2-$CF_3$ | H | Me | H |
| Ph-3-$CF_3$ | H | Me | H |
| Ph-4-$CF_3$ | H | Me | H |
| Ph-2-Me | H | Me | H |
| Ph-3-Me | H | Me | H |
| Ph-4-Me | H | Me | H |

| $R_1$ | $R_2$ | $R_3$ | $R_4$ |
|---|---|---|---|
| Ph-2-Et | H | Me | H |
| Ph-2-OMe | H | Me | H |
| Ph-3-OMe | H | Me | H |
| Ph-4-OMe | H | Me | H |
| Ph-2-OEt | H | Me | H |
| Ph-2-$CO_2$Me | H | Me | H |
| Ph-2-$CO_2$Et | H | Me | H |
| Ph-3-$CO_2$Me | H | Me | H |
| Ph-4-$CO_2$Me | H | Me | H |
| $CH_2$Ph | H | Me | H |
| $CH_2$Ph-2-F | H | Me | H |
| $CH_2$Ph-3-F | H | Me | H |
| $CH_2$Ph-4-F | H | Me | H |
| $CH_2$Ph-2-Cl | H | Me | H |
| $CH_2$Ph-3-Cl | H | Me | H |
| $CH_2$Ph-4-Cl | H | Me | H |
| $CH_2$Ph-2-Br | H | Me | H |
| $CH_2$Ph-3-Br | H | Me | H |
| $CH_2$Ph-4-Br | H | Me | H |
| $CH_2$Ph-2-$CF_3$ | H | Me | H |
| $CH_2$Ph-3-$CF_3$ | H | Me | H |
| $CH_2$Ph-4-$CF_3$ | H | Me | H |
| $CH_2$Ph-2-Me | H | Me | H |
| $CH_2$Ph-3-Me | H | Me | H |
| $CH_2$Ph-4-Me | H | Me | H |
| $CH_2$Ph-2-Et | H | Me | H |
| $CH_2$Ph-2-OMe | H | Me | H |
| $CH_2$Ph-3-OMe | H | Me | H |
| $CH_2$Ph-4-OMe | H | Me | H |
| $CH_2$Ph-2-OEt | H | Me | H |
| $CH_2$Ph-2-$CO_2$Me | H | Me | H |
| $CH_2$Ph-2-$CO_2$Et | H | Me | H |
| $CH_2$Ph-3-$CO_2$Me | H | Me | H |
| $CH_2$Ph-4-$CO_2$Me | H | Me | H |
| OPh | H | Me | H |
| OPh-2-Cl | H | Me | H |
| OPh-3-Cl | H | Me | H |
| OPh-4-Cl | H | Me | H |
| OPh-2-F | H | Me | H |
| OPh-3-F | H | Me | H |

| $R_1$ | $R_2$ | $R_3$ | $R_4$ |
|---|---|---|---|
| OPh-4-F | H | Me | H |
| OPh-2-$CF_3$ | H | Me | H |
| OPh-3-$CF_3$ | H | Me | H |
| OPh-4-$CF_3$ | H | Me | H |
| OPh-2-Me | H | Me | H |
| OPh-3-Me | H | Me | H |
| OPh-4-Me | H | Me | H |
| OPh-2-Et | H | Me | H |
| OPh-2-OMe | H | Me | H |
| OPh-3-OMe | H | Me | H |
| OPh-4-OMe | H | Me | H |
| OPh-2-OEt | H | Me | H |
| OPh-2-$CO_2$Me | H | Me | H |
| OPh-3-$CO_2$Me | H | Me | H |
| OPh-4-$CO_2$Me | H | Me | H |
| OPh-2-$CO_2$Et | H | Me | H |
| SPh | H | Me | H |
| SPh-2-Cl | H | Me | H |
| SPh-3-Cl | H | Me | H |
| SPh-4-Cl | H | Me | H |
| SPh-2-F | H | Me | H |
| SPh-3-F | H | Me | H |
| SPh-4-F | H | Me | H |
| SPh-2-$CF_3$ | H | Me | H |
| SPh-3-$CF_3$ | H | Me | H |
| SPh-4-$CF_3$ | H | Me | H |
| SPh-2-Me | H | Me | H |
| SPh-3-Me | H | Me | H |
| SPh-4-Me | H | Me | H |
| SPh-2-Et | H | Me | H |
| SPh-2-OMe | H | Me | H |
| SPh-3-OMe | H | Me | H |
| SPh-4-OMe | H | Me | H |
| SPh-2-OEt | H | Me | H |
| SPh-2-$CO_2$Me | H | Me | H |
| SPh-3-$CO_2$Me | H | Me | H |
| SPh-4-$CO_2$Me | H | Me | H |
| SPh-2-$CO_2$Et | H | Me | H |
| $SO_2$Ph | H | Me | H |
| $SO_2$Ph-2-Cl | H | Me | H |

| $R_1$ | $R_2$ | $R_3$ | $R_4$ |
|---|---|---|---|
| $SO_2Ph$-3-Cl | H | Me | H |
| $SO_2Ph$-4-Cl | H | Me | H |
| $SO_2Ph$-2-F | H | Me | H |
| $SO_2Ph$-3-F | H | Me | H |
| $SO_2Ph$-4-F | H | Me | H |
| $SO_2Ph$-2-$CF_3$ | H | Me | H |
| $SO_2Ph$-3-$CF_3$ | H | Me | H |
| $SO_2Ph$-4-$CF_3$ | H | Me | H |
| $SO_2Ph$-2-Me | H | Me | H |
| $SO_2Ph$-3-Me | H | Me | H |
| $SO_2Ph$-4-Me | H | Me | H |
| $SO_2Ph$-2-Et | H | Me | H |
| $SO_2Ph$-2-OMe | H | Me | H |
| $SO_2Ph$-3-OMe | H | Me | H |
| $SO_2Ph$-4-OMe | H | Me | H |
| $SO_2Ph$-2-OEt | H | Me | H |
| $CH_2Ph$-2-$CO_2Me$ | H | Me | H |
| $CH_2Ph$-3-$CO_2Me$ | H | Me | H |
| $CH_2Ph$-4-$CO_2Me$ | H | Me | H |
| $CH_2Ph$-2-$CO_2Et$ | H | Me | H |
| CONHMe | H | Me | H |
| CONHEt | H | Me | H |
| CONHPr-n | H | Me | H |
| CONHPr-i | H | Me | H |
| CONHBu-n | H | Me | H |
| CONHBu-sec | H | Me | H |
| CONHBu-t | H | Me | H |
| $CON(Me)_2$ | H | Me | H |
| $CON(Et)_2$ | H | Me | H |
| $CON(Pr\text{-}n)_2$ | H | Me | H |
| F | H | Et | H |
| Cl | H | Et | H |
| Br | H | Et | H |
| I | H | Et | H |
| $CH=CH_2$ | H | Et | H |
| $CH_2CH=CH_2$ | H | Et | H |
| $C\equiv CH$ | H | Et | H |
| $CH_2C\equiv CH$ | H | Et | H |
| $CH_2F$ | H | Et | H |
| $CHF_2$ | H | Et | H |

| $R_1$ | $R_2$ | $R_3$ | $R_4$ |
|---|---|---|---|
| CN | H | Et | H |
| $NH_2$ | H | Et | H |
| NHCOMe | H | Et | H |
| $CH_2NH_2$ | H | Et | H |
| $CH_2NHCOMe$ | H | Et | H |
| $CH_2OCHF_2$ | H | Et | H |
| $CH_2OCF_3$ | H | Et | H |
| $CF_3$ | H | Et | H |
| $NO_2$ | H | Et | H |
| OMe | H | Et | H |
| OEt | H | Et | H |
| OPr-n | H | Et | H |
| OBu-n | H | Et | H |
| OPen-n | H | Et | H |
| SMe | H | Et | H |
| SEt | H | Et | H |
| SPr-n | H | Et | H |
| SBu-n | H | Et | H |
| SPen-n | H | Et | H |
| $SO_2Me$ | H | Et | H |
| $SO_2Et$ | H | Et | H |
| $SO_2Pr$-n | H | Et | H |
| COMe | H | Et | H |
| COEt | H | Et | H |
| COPr-n | H | Et | H |
| $CO_2H$ | H | Et | H |
| $CO_2Me$ | H | Et | H |
| $CO_2Et$ | H | Et | H |
| $CO_2Pr$-n | H | Et | H |
| $CO_2Bu$-n | H | Et | H |
| $CO_2Pen$-n | H | Et | H |
| $CH_2Cl$ | H | Et | H |
| $CH_2Br$ | H | Et | H |
| $CH_2I$ | H | Et | H |
| $CH_2CF_3$ | H | Et | H |
| $CH_2CN$ | H | Et | H |
| $CH_2OH$ | H | Et | H |
| $CH_2OMe$ | H | Et | H |
| $CH_2OEt$ | H | Et | H |
| $CH_2OPr$-n | H | Et | H |

| $R_1$ | $R_2$ | $R_3$ | $R_4$ |
|---|---|---|---|
| $CH_2SMe$ | H | Et | H |
| $CH_2SEt$ | H | Et | H |
| $CH_2SPr$-n | H | Et | H |
| $CH_2SO_2Me$ | H | Et | H |
| $CH_2SO_2Et$ | H | Et | H |
| $CH_2SO_2Pr$-n | H | Et | H |
| $CH_2COMe$ | H | Et | H |
| $CH_2COEt$ | H | Et | H |
| $CH_2COPr$-n | H | Et | H |
| $CH_2CO_2H$ | H | Et | H |
| $CH_2CO_2Me$ | H | Et | H |
| $CH_2CO_2Et$ | H | Et | H |
| $CH_2CO_2Pr$-n | H | Et | H |
| $CH_2OCH_2CH=CH_2$ | H | Et | H |
| $CH_2OCH_2C\equiv CH$ | H | Et | H |
| $CH=CHCO_2Me$ | H | Et | H |
| $CH=CHCO_2Et$ | H | Et | H |
| $CH=CHCO_2Pr$-n | H | Et | H |
| CH=CHCN | H | Et | H |
| Ph | H | Et | H |
| $CH_2Ph$ | H | Et | H |
| OPh | H | Et | H |
| SPh | H | Et | H |
| $SO_2Ph$ | H | Et | H |
| CONHMe | H | Et | H |
| CONHEt | H | Et | H |
| CONHPr-n | H | Et | H |
| CONHPr-i | H | Et | H |
| CONHBu-n | H | Et | H |
| CONHBu-sec | H | Et | H |
| CONHBu-t | H | Et | H |
| $CON(Me)_2$ | H | Et | H |
| $CON(Et)_2$ | H | Et | H |
| $CON(Pr\text{-}n)_2$ | H | Et | H |
| F | Me | H | H |
| Cl | Me | H | H |
| Br | Me | H | H |
| I | Me | H | H |
| $CH=CH_2$ | Me | H | H |
| $CH_2CH=CH_2$ | Me | H | H |

| $R_1$ | $R_2$ | $R_3$ | $R_4$ |
|---|---|---|---|
| $C\equiv CH$ | Me | H | H |
| $CH_2C\equiv CH$ | Me | H | H |
| $CH_2F$ | Me | H | H |
| $CHF_2$ | Me | H | H |
| CN | Me | H | H |
| $NH_2$ | Me | H | H |
| NHCOMe | Me | H | H |
| $CH_2NH_2$ | Me | H | H |
| $CH_2NHCOMe$ | Me | H | H |
| $CH_2OCHF_2$ | Me | H | H |
| $CH_2OCF_3$ | Me | H | H |
| $CF_3$ | Me | H | H |
| $NO_2$ | Me | H | H |
| OMe | Me | H | H |
| OEt | Me | H | H |
| OPr-n | Me | H | H |
| OBu-n | Me | H | H |
| OPen-n | Me | H | H |
| SMe | Me | H | H |
| SEt | Me | H | H |
| SPr-n | Me | H | H |
| SBu-n | Me | H | H |
| SPen-n | Me | H | H |
| $SO_2Me$ | Me | H | H |
| $SO_2Et$ | Me | H | H |
| $SO_2Pr$-n | Me | H | H |
| COMe | Me | H | H |
| COEt | Me | H | H |
| COPr-n | Me | H | H |
| $CO_2H$ | Me | H | H |
| $CO_2Me$ | Me | H | H |
| $CO_2Et$ | Me | H | H |
| $CO_2Pr$-n | Me | H | H |
| $CO_2Bu$-n | Me | H | H |
| $CO_2Pen$-n | Me | H | H |
| $CH_2Cl$ | Me | H | H |
| $CH_2Br$ | Me | H | H |
| $CH_2I$ | Me | H | H |
| $CH_2CF_3$ | Me | H | H |
| $CH_2CN$ | Me | H | H |

| $R_1$ | $R_2$ | $R_3$ | $R_4$ |
|---|---|---|---|
| $CH_2OH$ | Me | H | H |
| $CH_2OMe$ | Me | H | H |
| $CH_2OEt$ | Me | H | H |
| $CH_2OPr$-n | Me | H | H |
| $CH_2SMe$ | Me | H | H |
| $CH_2SEt$ | Me | H | H |
| $CH_2SPr$-n | Me | H | H |
| $CH_2SO_2Me$ | Me | H | H |
| $CH_2SO_2Et$ | Me | H | H |
| $CH_2SO_2Pr$-n | Me | H | H |
| $CH_2COMe$ | Me | H | H |
| $CH_2COEt$ | Me | H | H |
| $CH_2COPr$-n | Me | H | H |
| $CH_2CO_2H$ | Me | H | H |
| $CH_2CO_2Me$ | Me | H | H |
| $CH_2CO_2Et$ | Me | H | H |
| $CH_2CO_2Pr$-n | Me | H | H |
| $CH_2OCH_2CH=CH_2$ | Me | H | H |
| $CH_2OCH_2C\equiv CH$ | Me | H | H |
| $CH=CHCO_2Me$ | Me | H | H |
| $CH=CHCO_2Et$ | Me | H | H |
| $CH=CHCO_2Pr$-n | Me | H | H |
| CH=CHCN | Me | H | H |
| Ph | Me | H | H |
| $CH_2Ph$ | Me | H | H |
| OPh | Me | H | H |
| SPh | Me | H | H |
| $SO_2Ph$ | Me | H | H |
| CONHMe | Me | H | H |
| CONHEt | Me | H | H |
| CONHPr-n | Me | H | H |
| CONHPr-i | Me | H | H |
| CONHBu-n | Me | H | H |
| CONHBu-sec | Me | H | H |
| CONHBu-t | Me | H | H |
| $CON(Me)_2$ | Me | H | H |
| $CON(Et)_2$ | Me | H | H |
| $CON(Pr\text{-}n)_2$ | Me | H | H |
| F | Et | H | H |
| Cl | Et | H | H |

| $R_1$ | $R_2$ | $R_3$ | $R_4$ |
|---|---|---|---|
| Br | Et | H | H |
| I | Et | H | H |
| $CH=CH_2$ | Et | H | H |
| $CH_2CH=CH_2$ | Et | H | H |
| $C\equiv CH$ | Et | H | H |
| $CH_2C\equiv CH$ | Et | H | H |
| $CH_2F$ | Et | H | H |
| $CHF_2$ | Et | H | H |
| CN | Et | H | H |
| $NH_2$ | Et | H | H |
| NHCOMe | Et | H | H |
| $CH_2NH_2$ | Et | H | H |
| $CH_2NHCOMe$ | Et | H | H |
| $CH_2OCHF_2$ | Et | H | H |
| $CH_2OCF_3$ | Et | H | H |
| $CF_3$ | Et | H | H |
| $NO_2$ | Et | H | H |
| OMe | Et | H | H |
| OEt | Et | H | H |
| OPr-n | Et | H | H |
| OBu-n | Et | H | H |
| OPen-n | Et | H | H |
| SMe | Et | H | H |
| SEt | Et | H | H |
| SPr-n | Et | H | H |
| SBu-n | Et | H | H |
| SPen-n | Et | H | H |
| $SO_2Me$ | Et | H | H |
| $SO_2Et$ | Et | H | H |
| $SO_2Pr$-n | Et | H | H |
| COMe | Et | H | H |
| COEt | Et | H | H |
| COPr-n | Et | H | H |
| $CO_2H$ | Et | H | H |
| $CO_2Me$ | Et | H | H |
| $CO_2Et$ | Et | H | H |
| $CO_2Pr$-n | Et | H | H |
| $CO_2Bu$-n | Et | H | H |
| $CO_2Pen$-n | Et | H | H |
| $CH_2Cl$ | Et | H | H |

| $R_1$ | $R_2$ | $R_3$ | $R_4$ |
|---|---|---|---|
| $CH_2Br$ | Et | H | H |
| $CH_2I$ | Et | H | H |
| $CH_2CF_3$ | Et | H | H |
| $CH_2CN$ | Et | H | H |
| $CH_2OH$ | Et | H | H |
| $CH_2OMe$ | Et | H | H |
| $CH_2OEt$ | Et | H | H |
| $CH_2OPr$-n | Et | H | H |
| $CH_2SMe$ | Et | H | H |
| $CH_2SEt$ | Et | H | H |
| $CH_2SPr$-n | Et | H | H |
| $CH_2SO_2Me$ | Et | H | H |
| $CH_2SO_2Et$ | Et | H | H |
| $CH_2SO_2Pr$-n | Et | H | H |
| $CH_2COMe$ | Et | H | H |
| $CH_2COEt$ | Et | H | H |
| $CH_2COPr$-n | Et | H | H |
| $CH_2CO_2H$ | Et | H | H |
| $CH_2CO_2Me$ | Et | H | H |
| $CH_2CO_2Et$ | Et | H | H |
| $CH_2CO_2Pr$-n | Et | H | H |
| $CH_2OCH_2CH=CH_2$ | Et | H | H |
| $CH_2OCH_2C\equiv CH$ | Et | H | H |
| $CH=CHCO_2Me$ | Et | H | H |
| $CH=CHCO_2Et$ | Et | H | H |
| $CH=CHCO_2Pr$-n | Et | H | H |
| CH=CHCN | Et | H | H |
| Ph | Et | H | H |
| $CH_2Ph$ | Et | H | H |
| OPh | Et | H | H |
| SPh | Et | H | H |
| $SO_2Ph$ | Et | H | H |
| CONHMe | Et | H | H |
| CONHEt | Et | H | H |
| CONHPr-n | Et | H | H |
| CONHPr-i | Et | H | H |
| CONHBu-n | Et | H | H |
| CONHBu-sec | Et | H | H |
| CONHBu-t | Et | H | H |
| $CON(Me)_2$ | Et | H | H |

| $R_1$ | $R_2$ | $R_3$ | $R_4$ |
|---|---|---|---|
| $CON(Et)_2$ | Et | H | H |
| $CON(Pr\text{-}n)_2$ | Et | H | H |
| F | H | Pr-n | H |
| Cl | H | Pr-n | H |
| Br | H | Pr-n | H |
| I | H | Pr-n | H |
| $CH=CH_2$ | H | Pr-n | H |
| $CH_2CH=CH_2$ | H | Pr-n | H |
| $C\equiv CH$ | H | Pr-n | H |
| $CH_2C\equiv CH$ | H | Pr-n | H |
| $CH_2F$ | H | Pr-n | H |
| $CHF_2$ | H | Pr-n | H |
| CN | H | Pr-n | H |
| $NH_2$ | H | Pr-n | H |
| NHCOMe | H | Pr-n | H |
| $CH_2NH_2$ | H | Pr-n | H |
| $CH_2NHCOMe$ | H | Pr-n | H |
| $CH_2OCHF_2$ | H | Pr-n | H |
| $CH_2OCF_3$ | H | Pr-n | H |
| $CF_3$ | H | Pr-n | H |
| $NO_2$ | H | Pr-n | H |
| OMe | H | Pr-n | H |
| OEt | H | Pr-n | H |
| OPr-n | H | Pr-n | H |
| OBu-n | H | Pr-n | H |
| OPen-n | H | Pr-n | H |
| SMe | H | Pr-n | H |
| SEt | H | Pr-n | H |
| SPr-n | H | Pr-n | H |
| SBu-n | H | Pr-n | H |
| SPen-n | H | Pr-n | H |
| $SO_2Me$ | H | Pr-n | H |
| $SO_2Et$ | H | Pr-n | H |
| $SO_2Pr\text{-}n$ | H | Pr-n | H |
| COMe | H | Pr-n | H |
| COEt | H | Pr-n | H |
| COPr-n | H | Pr-n | H |
| $CO_2H$ | H | Pr-n | H |
| $CO_2Me$ | H | Pr-n | H |
| $CO_2Et$ | H | Pr-n | H |

| $R_1$ | $R_2$ | $R_3$ | $R_4$ |
|---|---|---|---|
| $CO_2Pr$-n | H | Pr-n | H |
| $CO_2Bu$-n | H | Pr-n | H |
| $CO_2Pen$-n | H | Pr-n | H |
| $CH_2Cl$ | H | Pr-n | H |
| $CH_2Br$ | H | Pr-n | H |
| $CH_2I$ | H | Pr-n | H |
| $CH_2CF_3$ | H | Pr-n | H |
| $CH_2CN$ | H | Pr-n | H |
| $CH_2OH$ | H | Pr-n | H |
| $CH_2OMe$ | H | Pr-n | H |
| $CH_2OEt$ | H | Pr-n | H |
| $CH_2OPr$-n | H | Pr-n | H |
| $CH_2SMe$ | H | Pr-n | H |
| $CH_2SEt$ | H | Pr-n | H |
| $CH_2SPr$-n | H | Pr-n | H |
| $CH_2SO_2Me$ | H | Pr-n | H |
| $CH_2SO_2Et$ | H | Pr-n | H |
| $CH_2SO_2Pr$-n | H | Pr-n | H |
| $CH_2COMe$ | H | Pr-n | H |
| $CH_2COEt$ | H | Pr-n | H |
| $CH_2COPr$-n | H | Pr-n | H |
| $CH_2CO_2H$ | H | Pr-n | H |
| $CH_2CO_2Me$ | H | Pr-n | H |
| $CH_2CO_2Et$ | H | Pr-n | H |
| $CH_2CO_2Pr$-n | H | Pr-n | H |
| $CH_2OCH_2CH{=}CH_2$ | H | Pr-n | H |
| $CH_2OCH_2C{\equiv}CH$ | H | Pr-n | H |
| $CH{=}CHCO_2Me$ | H | Pr-n | H |
| $CH{=}CHCO_2Et$ | H | Pr-n | H |
| $CH{=}CHCO_2Pr$-n | H | Pr-n | H |
| $CH{=}CHCN$ | H | Pr-n | H |
| Ph | H | Pr-n | H |
| $CH_2Ph$ | H | Pr-n | H |
| OPh | H | Pr-n | H |
| SPh | H | Pr-n | H |
| $SO_2Ph$ | H | Pr-n | H |
| CONHMe | H | Pr-n | H |
| CONHEt | H | Pr-n | H |
| CONHPr-n | H | Pr-n | H |
| CONHPr-i | H | Pr-n | H |

| $R_1$ | $R_2$ | $R_3$ | $R_4$ |
|---|---|---|---|
| CONHBu-n | H | Pr-n | H |
| CONHBu-sec | H | Pr-n | H |
| CONHBu-t | H | Pr-n | H |
| $CON(Me)_2$ | H | Pr-n | H |
| $CON(Et)_2$ | H | Pr-n | H |
| $CON(Pr\text{-}n)_2$ | H | Pr-n | H |
| F | H | Cl | H |
| Cl | H | Cl | H |
| Br | H | Cl | H |
| I | H | Cl | H |
| $CH=CH_2$ | H | Cl | H |
| $CH_2CH=CH_2$ | H | Cl | H |
| $C\equiv CH$ | H | Cl | H |
| $CH_2C\equiv CH$ | H | Cl | H |
| $CH_2F$ | H | Cl | H |
| $CHF_2$ | H | Cl | H |
| CN | H | Cl | H |
| $NH_2$ | H | Cl | H |
| NHCOMe | H | Cl | H |
| $CH_2NH_2$ | H | Cl | H |
| $CH_2NHCOMe$ | H | Cl | H |
| $CH_2OCHF_2$ | H | Cl | H |
| $CH_2OCF_3$ | H | Cl | H |
| $CF_3$ | H | Cl | H |
| $NO_2$ | H | Cl | H |
| OMe | H | Cl | H |
| OEt | H | Cl | H |
| OPr-n | H | Cl | H |
| OBu-n | H | Cl | H |
| OPen-n | H | Cl | H |
| SMe | H | Cl | H |
| SEt | H | Cl | H |
| SPr-n | H | Cl | H |
| SBu-n | H | Cl | H |
| SPen-n | H | Cl | H |
| $SO_2Me$ | H | Cl | H |
| $SO_2Et$ | H | Cl | H |
| $SO_2Pr\text{-}n$ | H | Cl | H |
| COMe | H | Cl | H |
| COEt | H | Cl | H |

| $R_1$ | $R_2$ | $R_3$ | $R_4$ |
|---|---|---|---|
| COPr-n | H | Cl | H |
| $CO_2H$ | H | Cl | H |
| $CO_2Me$ | H | Cl | H |
| $CO_2Et$ | H | Cl | H |
| $CO_2Pr$-n | H | Cl | H |
| $CO_2Bu$-n | H | Cl | H |
| $CO_2Pen$-n | H | Cl | H |
| $CH_2Cl$ | H | Cl | H |
| $CH_2Br$ | H | Cl | H |
| $CH_2I$ | H | Cl | H |
| $CH_2CF_3$ | H | Cl | H |
| $CH_2CN$ | H | Cl | H |
| $CH_2OH$ | H | Cl | H |
| $CH_2OMe$ | H | Cl | H |
| $CH_2OEt$ | H | Cl | H |
| $CH_2OPr$-n | H | Cl | H |
| $CH_2SMe$ | H | Cl | H |
| $CH_2SEt$ | H | Cl | H |
| $CH_2SPr$-n | H | Cl | H |
| $CH_2SO_2Me$ | H | Cl | H |
| $CH_2SO_2Et$ | H | Cl | H |
| $CH_2SO_2Pr$-n | H | Cl | H |
| $CH_2COMe$ | H | Cl | H |
| $CH_2COEt$ | H | Cl | H |
| $CH_2COPr$-n | H | Cl | H |
| $CH_2CO_2H$ | H | Cl | H |
| $CH_2CO_2Me$ | H | Cl | H |
| $CH_2CO_2Et$ | H | Cl | H |
| $CH_2CO_2Pr$-n | H | Cl | H |
| $CH_2OCH_2CH=CH_2$ | H | Cl | H |
| $CH_2OCH_2C\equiv CH$ | H | Cl | H |
| $CH=CHCO_2Me$ | H | Cl | H |
| $CH=CHCO_2Et$ | H | Cl | H |
| $CH=CHCO_2Pr$-n | H | Cl | H |
| CH=CHCN | H | Cl | H |
| Ph | H | Cl | H |
| $CH_2Ph$ | H | Cl | H |
| OPh | H | Cl | H |
| SPh | H | Cl | H |
| $SO_2Ph$ | H | Cl | H |

| $R_1$ | $R_2$ | $R_3$ | $R_4$ |
|---|---|---|---|
| CONHMe | H | Cl | H |
| CONHEt | H | Cl | H |
| CONHPr-n | H | Cl | H |
| CONHPr-i | H | Cl | H |
| CONHBu-n | H | Cl | H |
| CONHBu-sec | H | Cl | H |
| CONHBu-t | H | Cl | H |
| $CON(Me)_2$ | H | Cl | H |
| $CON(Et)_2$ | H | Cl | H |
| $CON(Pr\text{-}n)_2$ | H | Cl | H |
| F | H | $NO_2$ | H |
| Cl | H | $NO_2$ | H |
| Br | H | $NO_2$ | H |
| I | H | $NO_2$ | H |
| $CH=CH_2$ | H | $NO_2$ | H |
| $CH_2CH=CH_2$ | H | $NO_2$ | H |
| $C\equiv CH$ | H | $NO_2$ | H |
| $CH_2C\equiv CH$ | H | $NO_2$ | H |
| $CH_2F$ | H | $NO_2$ | H |
| $CHF_2$ | H | $NO_2$ | H |
| CN | H | $NO_2$ | H |
| $NH_2$ | H | $NO_2$ | H |
| NHCOMe | H | $NO_2$ | H |
| $CH_2NH_2$ | H | $NO_2$ | H |
| $CH_2NHCOMe$ | H | $NO_2$ | H |
| $CH_2OCHF_2$ | H | $NO_2$ | H |
| $CH_2OCF_3$ | H | $NO_2$ | H |
| $CF_3$ | H | $NO_2$ | H |
| $NO_2$ | H | $NO_2$ | H |
| OMe | H | $NO_2$ | H |
| OEt | H | $NO_2$ | H |
| OPr-n | H | $NO_2$ | H |
| OBu-n | H | $NO_2$ | H |
| OPen-n | H | $NO_2$ | H |
| SMe | H | $NO_2$ | H |
| SEt | H | $NO_2$ | H |
| SPr-n | H | $NO_2$ | H |
| SBu-n | H | $NO_2$ | H |
| SPen-n | H | $NO_2$ | H |
| $SO_2Me$ | H | $NO_2$ | H |

| $R_1$ | $R_2$ | $R_3$ | $R_4$ |
|---|---|---|---|
| $SO_2Et$ | H | $NO_2$ | H |
| $SO_2Pr$-n | H | $NO_2$ | H |
| COMe | H | $NO_2$ | H |
| COEt | H | $NO_2$ | H |
| COPr-n | H | $NO_2$ | H |
| $CO_2H$ | H | $NO_2$ | H |
| $CO_2Me$ | H | $NO_2$ | H |
| $CO_2Et$ | H | $NO_2$ | H |
| $CO_2Pr$-n | H | $NO_2$ | H |
| $CO_2Bu$-n | H | $NO_2$ | H |
| $CO_2Pen$-n | H | $NO_2$ | H |
| $CH_2Cl$ | H | $NO_2$ | H |
| $CH_2Br$ | H | $NO_2$ | H |
| $CH_2I$ | H | $NO_2$ | H |
| $CH_2CF_3$ | H | $NO_2$ | H |
| $CH_2CN$ | H | $NO_2$ | H |
| $CH_2OH$ | H | $NO_2$ | H |
| $CH_2OMe$ | H | $NO_2$ | H |
| $CH_2OEt$ | H | $NO_2$ | H |
| $CH_2OPr$-n | H | $NO_2$ | H |
| $CH_2SMe$ | H | $NO_2$ | H |
| $CH_2SEt$ | H | $NO_2$ | H |
| $CH_2SPr$-n | H | $NO_2$ | H |
| $CH_2SO_2Me$ | H | $NO_2$ | H |
| $CH_2SO_2Et$ | H | $NO_2$ | H |
| $CH_2SO_2Pr$-n | H | $NO_2$ | H |
| $CH_2COMe$ | H | $NO_2$ | H |
| $CH_2COEt$ | H | $NO_2$ | H |
| $CH_2COPr$-n | H | $NO_2$ | H |
| $CH_2CO_2H$ | H | $NO_2$ | H |
| $CH_2CO_2Me$ | H | $NO_2$ | H |
| $CH_2CO_2Et$ | H | $NO_2$ | H |
| $CH_2CO_2Pr$-n | H | $NO_2$ | H |
| $CH_2OCH_2CH=CH_2$ | H | $NO_2$ | H |
| $CH_2OCH_2C\equiv CH$ | H | $NO_2$ | H |
| $CH=CHCO_2Me$ | H | $NO_2$ | H |
| $CH=CHCO_2Et$ | H | $NO_2$ | H |
| $CH=CHCO_2Pr$-n | H | $NO_2$ | H |
| CH=CHCN | H | $NO_2$ | H |
| Ph | H | $NO_2$ | H |

| $R_1$ | $R_2$ | $R_3$ | $R_4$ |
|---|---|---|---|
| $CH_2Ph$ | H | $NO_2$ | H |
| OPh | H | $NO_2$ | H |
| SPh | H | $NO_2$ | H |
| $SO_2Ph$ | H | $NO_2$ | H |
| CONHMe | H | $NO_2$ | H |
| CONHEt | H | $NO_2$ | H |
| CONHPr-n | H | $NO_2$ | H |
| CONHPr-i | H | $NO_2$ | H |
| CONHBu-n | H | $NO_2$ | H |
| CONHBu-sec | H | $NO_2$ | H |
| CONHBu-t | H | $NO_2$ | H |
| $CON(Me)_2$ | H | $NO_2$ | H |
| $CON(Et)_2$ | H | $NO_2$ | H |
| $CON(Pr\text{-}n)_2$ | H | $NO_2$ | H |
| F | H | $CF_3$ | H |
| Cl | H | $CF_3$ | H |
| Br | H | $CF_3$ | H |
| I | H | $CF_3$ | H |
| $CH=CH_2$ | H | $CF_3$ | H |
| $CH_2CH=CH_2$ | H | $CF_3$ | H |
| $C\equiv CH$ | H | $CF_3$ | H |
| $CH_2C\equiv CH$ | H | $CF_3$ | H |
| $CH_2F$ | H | $CF_3$ | H |
| $CHF_2$ | H | $CF_3$ | H |
| CN | H | $CF_3$ | H |
| $NH_2$ | H | $CF_3$ | H |
| NHCOMe | H | $CF_3$ | H |
| $CH_2NH_2$ | H | $CF_3$ | H |
| $CH_2NHCOMe$ | H | $CF_3$ | H |
| $CH_2OCHF_2$ | H | $CF_3$ | H |
| $CH_2OCF_3$ | H | $CF_3$ | H |
| $CF_3$ | H | $CF_3$ | H |
| $NO_2$ | H | $CF_3$ | H |
| OMe | H | $CF_3$ | H |
| OEt | H | $CF_3$ | H |
| OPr-n | H | $CF_3$ | H |
| OBu-n | H | $CF_3$ | H |
| OPen-n | H | $CF_3$ | H |
| SMe | H | $CF_3$ | H |
| SEt | H | $CF_3$ | H |

| $R_1$ | $R_2$ | $R_3$ | $R_4$ |
|---|---|---|---|
| SPr-n | H | $CF_3$ | H |
| SBu-n | H | $CF_3$ | H |
| SPen-n | H | $CF_3$ | H |
| $SO_2Me$ | H | $CF_3$ | H |
| $SO_2Et$ | H | $CF_3$ | H |
| $SO_2Pr$-n | H | $CF_3$ | H |
| COMe | H | $CF_3$ | H |
| COEt | H | $CF_3$ | H |
| COPr-n | H | $CF_3$ | H |
| $CO_2H$ | H | $CF_3$ | H |
| $CO_2Me$ | H | $CF_3$ | H |
| $CO_2Et$ | H | $CF_3$ | H |
| $CO_2Pr$-n | H | $CF_3$ | H |
| $CO_2Bu$-n | H | $CF_3$ | H |
| $CO_2Pen$-n | H | $CF_3$ | H |
| $CH_2Cl$ | H | $CF_3$ | H |
| $CH_2Br$ | H | $CF_3$ | H |
| $CH_2I$ | H | $CF_3$ | H |
| $CH_2CF_3$ | H | $CF_3$ | H |
| $CH_2CN$ | H | $CF_3$ | H |
| $CH_2OH$ | H | $CF_3$ | H |
| $CH_2OMe$ | H | $CF_3$ | H |
| $CH_2OEt$ | H | $CF_3$ | H |
| $CH_2OPr$-n | H | $CF_3$ | H |
| $CH_2SMe$ | H | $CF_3$ | H |
| $CH_2SEt$ | H | $CF_3$ | H |
| $CH_2SPr$-n | H | $CF_3$ | H |
| $CH_2SO_2Me$ | H | $CF_3$ | H |
| $CH_2SO_2Et$ | H | $CF_3$ | H |
| $CH_2SO_2Pr$-n | H | $CF_3$ | H |
| $CH_2COMe$ | H | $CF_3$ | H |
| $CH_2COEt$ | H | $CF_3$ | H |
| $CH_2COPr$-n | H | $CF_3$ | H |
| $CH_2CO_2H$ | H | $CF_3$ | H |
| $CH_2CO_2Me$ | H | $CF_3$ | H |
| $CH_2CO_2Et$ | H | $CF_3$ | H |
| $CH_2CO_2Pr$-n | H | $CF_3$ | H |
| $CH_2OCH_2CH=CH_2$ | H | $CF_3$ | H |
| $CH_2OCH_2C\equiv CH$ | H | $CF_3$ | H |
| $CH=CHCO_2Me$ | H | $CF_3$ | H |

| $R_1$ | $R_2$ | $R_3$ | $R_4$ |
|---|---|---|---|
| $CH=CHCO_2Et$ | H | $CF_3$ | H |
| $CH=CHCO_2Pr$-n | H | $CF_3$ | H |
| CH=CHCN | H | $CF_3$ | H |
| Ph | H | $CF_3$ | H |
| $CH_2Ph$ | H | $CF_3$ | H |
| OPh | H | $CF_3$ | H |
| SPh | H | $CF_3$ | H |
| $SO_2Ph$ | H | $CF_3$ | H |
| CONHMe | H | $CF_3$ | H |
| CONHEt | H | $CF_3$ | H |
| CONHPr-n | H | $CF_3$ | H |
| CONHPr-i | H | $CF_3$ | H |
| CONHBu-n | H | $CF_3$ | H |
| CONHBu-sec | H | $CF_3$ | H |
| CONHBu-t | H | $CF_3$ | H |
| $CON(Me)_2$ | H | $CF_3$ | H |
| $CON(Et)_2$ | H | $CF_3$ | H |
| $CON(Pr$-$n)_2$ | H | $CF_3$ | H |

| $R_1$ | $R_2$ | $R_3$ | $R_4$ |
|---|---|---|---|
| F | H | OMe | H |
| Cl | H | OMe | H |
| Br | H | OMe | H |
| I | H | OMe | H |
| $CH=CH_2$ | H | OMe | H |
| $CH_2CH=CH_2$ | H | OMe | H |
| $C\equiv CH$ | H | OMe | H |
| $CH_2C\equiv CH$ | H | OMe | H |
| $CH_2F$ | H | OMe | H |
| $CHF_2$ | H | OMe | H |
| CN | H | OMe | H |
| $NH_2$ | H | OMe | H |
| NHCOMe | H | OMe | H |
| $CH_2NH_2$ | H | OMe | H |
| $CH_2NHCOMe$ | H | OMe | H |
| $CH_2OCHF_2$ | H | OMe | H |
| $CH_2OCF_3$ | H | OMe | H |
| $CF_3$ | H | OMe | H |
| $NO_2$ | H | OMe | H |
| OMe | H | OMe | H |
| OEt | H | OMe | H |
| OPr-n | H | OMe | H |
| OBu-n | H | OMe | H |
| OPen-n | H | OMe | H |
| SMe | H | OMe | H |
| SEt | H | OMe | H |
| SPr-n | H | OMe | H |
| SBu-n | H | OMe | H |
| SPen-n | H | OMe | H |
| $SO_2Me$ | H | OMe | H |
| $SO_2Et$ | H | OMe | H |
| $SO_2Pr$-n | H | OMe | H |
| COMe | H | OMe | H |
| COEt | H | OMe | H |
| COPr-n | H | OMe | H |
| $CO_2H$ | H | OMe | H |
| $CO_2Me$ | H | OMe | H |
| $CO_2Et$ | H | OMe | H |
| $CO_2Pr$-n | H | OMe | H |
| $CO_2Bu$-n | H | OMe | H |

| $R_1$ | $R_2$ | $R_3$ | $R_4$ |
|---|---|---|---|
| $CO_2Pen$-n | H | OMe | H |
| $CH_2Cl$ | H | OMe | H |
| $CH_2Br$ | H | OMe | H |
| $CH_2I$ | H | OMe | H |
| $CH_2CF_3$ | H | OMe | H |
| $CH_2CN$ | H | OMe | H |
| $CH_2OH$ | H | OMe | H |
| $CH_2OMe$ | H | OMe | H |
| $CH_2OEt$ | H | OMe | H |
| $CH_2OPr$-n | H | OMe | H |
| $CH_2SMe$ | H | OMe | H |
| $CH_2SEt$ | H | OMe | H |
| $CH_2SPr$-n | H | OMe | H |
| $CH_2SO_2Me$ | H | OMe | H |
| $CH_2SO_2Et$ | H | OMe | H |
| $CH_2SO_2Pr$-n | H | OMe | H |
| $CH_2COMe$ | H | OMe | H |
| $CH_2COEt$ | H | OMe | H |
| $CH_2COPr$-n | H | OMe | H |
| $CH_2CO_2H$ | H | OMe | H |
| $CH_2CO_2Me$ | H | OMe | H |
| $CH_2CO_2Et$ | H | OMe | H |
| $CH_2CO_2Pr$-n | H | OMe | H |
| $CH_2OCH_2CH=CH_2$ | H | OMe | H |
| $CH_2OCH_2C\equiv CH$ | H | OMe | H |
| $CH=CHCO_2Me$ | H | OMe | H |
| $CH=CHCO_2Et$ | H | OMe | H |
| $CH=CHCO_2Pr$-n | H | OMe | H |
| CH=CHCN | H | OMe | H |
| Ph | H | OMe | H |
| $CH_2Ph$ | H | OMe | H |
| OPh | H | OMe | H |
| SPh | H | OMe | H |
| $SO_2Ph$ | H | OMe | H |
| CONHMe | H | OMe | H |
| CONHEt | H | OMe | H |
| CONHPr-n | H | OMe | H |
| CONHPr-i | H | OMe | H |
| CONHBu-n | H | OMe | H |
| CONHBu-sec | H | OMe | H |

| $R_1$ | $R_2$ | $R_3$ | $R_4$ |
|---|---|---|---|
| CONHBu-t | H | OMe | H |
| CON(Me)$_2$ | H | OMe | H |
| CON(Et)$_2$ | H | OMe | H |
| CON(Pr-n)$_2$ | H | OMe | H |
| F | H | SMe | H |
| Cl | H | SMe | H |
| Br | H | SMe | H |
| I | H | SMe | H |
| CH=CH$_2$ | H | SMe | H |
| CH$_2$CH=CH$_2$ | H | SMe | H |
| C≡CH | H | SMe | H |
| CH$_2$C≡CH | H | SMe | H |
| CH$_2$F | H | SMe | H |
| CHF$_2$ | H | SMe | H |
| CN | H | SMe | H |
| NH$_2$ | H | SMe | H |
| NHCOMe | H | SMe | H |
| CH$_2$NH$_2$ | H | SMe | H |
| CH$_2$NHCOMe | H | SMe | H |
| CH$_2$OCHF$_2$ | H | SMe | H |
| CH$_2$OCF$_3$ | H | SMe | H |
| CF$_3$ | H | SMe | H |
| NO$_2$ | H | SMe | H |
| OMe | H | SMe | H |
| OEt | H | SMe | H |
| OPr-n | H | SMe | H |
| OBu-n | H | SMe | H |
| OPen-n | H | SMe | H |
| SMe | H | SMe | H |
| SEt | H | SMe | H |
| SPr-n | H | SMe | H |
| SBu-n | H | SMe | H |
| SPen-n | H | SMe | H |
| SO$_2$Me | H | SMe | H |
| SO$_2$Et | H | SMe | H |
| SO$_2$Pr-n | H | SMe | H |
| COMe | H | SMe | H |
| COEt | H | SMe | H |
| COPr-n | H | SMe | H |
| CO$_2$H | H | SMe | H |

| $R_1$ | $R_2$ | $R_3$ | $R_4$ |
|---|---|---|---|
| $CO_2Me$ | H | SMe | H |
| $CO_2Et$ | H | SMe | H |
| $CO_2Pr$-n | H | SMe | H |
| $CO_2Bu$-n | H | SMe | H |
| $CO_2Pen$-n | H | SMe | H |
| $CH_2Cl$ | H | SMe | H |
| $CH_2Br$ | H | SMe | H |
| $CH_2I$ | H | SMe | H |
| $CH_2CF_3$ | H | SMe | H |
| $CH_2CN$ | H | SMe | H |
| $CH_2OH$ | H | SMe | H |
| $CH_2OMe$ | H | SMe | H |
| $CH_2OEt$ | H | SMe | H |
| $CH_2OPr$-n | H | SMe | H |
| $CH_2SMe$ | H | SMe | H |
| $CH_2SEt$ | H | SMe | H |
| $CH_2SPr$-n | H | SMe | H |
| $CH_2SO_2Me$ | H | SMe | H |
| $CH_2SO_2Et$ | H | SMe | H |
| $CH_2SO_2Pr$-n | H | SMe | H |
| $CH_2COMe$ | H | SMe | H |
| $CH_2COEt$ | H | SMe | H |
| $CH_2COPr$-n | H | SMe | H |
| $CH_2CO_2H$ | H | SMe | H |
| $CH_2CO_2Me$ | H | SMe | H |
| $CH_2CO_2Et$ | H | SMe | H |
| $CH_2CO_2Pr$-n | H | SMe | H |
| $CH_2OCH_2CH=CH_2$ | H | SMe | H |
| $CH_2OCH_2C\equiv CH$ | H | SMe | H |
| $CH=CHCO_2Me$ | H | SMe | H |
| $CH=CHCO_2Et$ | H | SMe | H |
| $CH=CHCO_2Pr$-n | H | SMe | H |
| $CH=CHCN$ | H | SMe | H |
| Ph | H | SMe | H |
| $CH_2Ph$ | H | SMe | H |
| OPh | H | SMe | H |
| SPh | H | SMe | H |
| $SO_2Ph$ | H | SMe | H |
| CONHMe | H | SMe | H |
| CONHEt | H | SMe | H |

| $R_1$ | $R_2$ | $R_3$ | $R_4$ |
|---|---|---|---|
| CONHPr-n | H | SMe | H |
| CONHPr-i | H | SMe | H |
| CONHBu-n | H | SMe | H |
| CONHBu-sec | H | SMe | H |
| CONHBu-t | H | SMe | H |
| $CON(Me)_2$ | H | SMe | H |
| $CON(Et)_2$ | H | SMe | H |
| $CON(Pr\text{-}n)_2$ | H | SMe | H |
| F | H | $SO_2Me$ | H |
| Cl | H | $SO_2Me$ | H |
| Br | H | $SO_2Me$ | H |
| I | H | $SO_2Me$ | H |
| $CH=CH_2$ | H | $SO_2Me$ | H |
| $CH_2CH=CH_2$ | H | $SO_2Me$ | H |
| $C\equiv CH$ | H | $SO_2Me$ | H |
| $CH_2C\equiv CH$ | H | $SO_2Me$ | H |
| $CH_2F$ | H | $SO_2Me$ | H |
| $CHF_2$ | H | $SO_2Me$ | H |
| CN | H | $SO_2Me$ | H |
| $NH_2$ | H | $SO_2Me$ | H |
| NHCOMe | H | $SO_2Me$ | H |
| $CH_2NH_2$ | H | $SO_2Me$ | H |
| $CH_2NHCOMe$ | H | $SO_2Me$ | H |
| $CH_2OCHF_2$ | H | $SO_2Me$ | H |
| $CH_2OCF_3$ | H | $SO_2Me$ | H |
| $CF_3$ | H | $SO_2Me$ | H |
| $NO_2$ | H | $SO_2Me$ | H |
| OMe | H | $SO_2Me$ | H |
| OEt | H | $SO_2Me$ | H |
| OPr-n | H | $SO_2Me$ | H |
| OBu-n | H | $SO_2Me$ | H |
| OPen-n | H | $SO_2Me$ | H |
| SMe | H | $SO_2Me$ | H |
| SEt | H | $SO_2Me$ | H |
| SPr-n | H | $SO_2Me$ | H |
| SBu-n | H | $SO_2Me$ | H |
| SPen-n | H | $SO_2Me$ | H |
| $SO_2Me$ | H | $SO_2Me$ | H |
| $SO_2Et$ | H | $SO_2Me$ | H |
| $SO_2Pr\text{-}n$ | H | $SO_2Me$ | H |

| $R_1$ | $R_2$ | $R_3$ | $R_4$ |
|---|---|---|---|
| COMe | H | $SO_2Me$ | H |
| COEt | H | $SO_2Me$ | H |
| COPr-n | H | $SO_2Me$ | H |
| $CO_2H$ | H | $SO_2Me$ | H |
| $CO_2Me$ | H | $SO_2Me$ | H |
| $CO_2Et$ | H | $SO_2Me$ | H |
| $CO_2Pr$-n | H | $SO_2Me$ | H |
| $CO_2Bu$-n | H | $SO_2Me$ | H |
| $CO_2Pen$-n | H | $SO_2Me$ | H |
| $CH_2Cl$ | H | $SO_2Me$ | H |
| $CH_2Br$ | H | $SO_2Me$ | H |
| $CH_2I$ | H | $SO_2Me$ | H |
| $CH_2CF_3$ | H | $SO_2Me$ | H |
| $CH_2CN$ | H | $SO_2Me$ | H |
| $CH_2OH$ | H | $SO_2Me$ | H |
| $CH_2OMe$ | H | $SO_2Me$ | H |
| $CH_2OEt$ | H | $SO_2Me$ | H |
| $CH_2OPr$-n | H | $SO_2Me$ | H |
| $CH_2SMe$ | H | $SO_2Me$ | H |
| $CH_2SEt$ | H | $SO_2Me$ | H |
| $CH_2SPr$-n | H | $SO_2Me$ | H |
| $CH_2SO_2Me$ | H | $SO_2Me$ | H |
| $CH_2SO_2Et$ | H | $SO_2Me$ | H |
| $CH_2SO_2Pr$-n | H | $SO_2Me$ | H |
| $CH_2COMe$ | H | $SO_2Me$ | H |
| $CH_2COEt$ | H | $SO_2Me$ | H |
| $CH_2COPr$-n | H | $SO_2Me$ | H |
| $CH_2CO_2H$ | H | $SO_2Me$ | H |
| $CH_2CO_2Me$ | H | $SO_2Me$ | H |
| $CH_2CO_2Et$ | H | $SO_2Me$ | H |
| $CH_2CO_2Pr$-n | H | $SO_2Me$ | H |
| $CH_2OCH_2CH=CH_2$ | H | $SO_2Me$ | H |
| $CH_2OCH_2C\equiv CH$ | H | $SO_2Me$ | H |
| $CH=CHCO_2Me$ | H | $SO_2Me$ | H |
| $CH=CHCO_2Et$ | H | $SO_2Me$ | H |
| $CH=CHCO_2Pr$-n | H | $SO_2Me$ | H |
| CH=CHCN | H | $SO_2Me$ | H |
| Ph | H | $SO_2Me$ | H |
| $CH_2Ph$ | H | $SO_2Me$ | H |
| OPh | H | $SO_2Me$ | H |

| $R_1$ | $R_2$ | $R_3$ | $R_4$ |
|---|---|---|---|
| SPh | H | $SO_2Me$ | H |
| $SO_2Ph$ | H | $SO_2Me$ | H |
| CONHMe | H | $SO_2Me$ | H |
| CONHEt | H | $SO_2Me$ | H |
| CONHPr-n | H | $SO_2Me$ | H |
| CONHPr-i | H | $SO_2Me$ | H |
| CONHBu-n | H | $SO_2Me$ | H |
| CONHBu-sec | H | $SO_2Me$ | H |
| CONHBu-t | H | $SO_2Me$ | H |
| $CON(Me)_2$ | H | $SO_2Me$ | H |
| $CON(Et)_2$ | H | $SO_2Me$ | H |
| $CON(Pr\text{-}n)_2$ | H | $SO_2Me$ | H |
| F | H | COMe | H |
| Cl | H | COMe | H |
| Br | H | COMe | H |
| I | H | COMe | H |
| $CH{=}CH_2$ | H | COMe | H |
| $CH_2CH{=}CH_2$ | H | COMe | H |
| $C\equiv CH$ | H | COMe | H |
| $CH_2C\equiv CH$ | H | COMe | H |
| $CH_2F$ | H | COMe | H |
| $CHF_2$ | H | COMe | H |
| CN | H | COMe | H |
| $NH_2$ | H | COMe | H |
| NHCOMe | H | COMe | H |
| $CH_2NH_2$ | H | COMe | H |
| $CH_2NHCOMe$ | H | COMe | H |
| $CH_2OCHF_2$ | H | COMe | H |
| $CH_2OCF_3$ | H | COMe | H |
| $CF_3$ | H | COMe | H |
| $NO_2$ | H | COMe | H |
| OMe | H | COMe | H |
| OEt | H | COMe | H |
| OPr-n | H | COMe | H |
| OBu-n | H | COMe | H |
| OPen-n | H | COMe | H |
| SMe | H | COMe | H |
| SEt | H | COMe | H |
| SPr-n | H | COMe | H |
| SBu-n | H | COMe | H |

| $R_1$ | $R_2$ | $R_3$ | $R_4$ |
|---|---|---|---|
| SPen-n | H | COMe | H |
| $SO_2Me$ | H | COMe | H |
| $SO_2Et$ | H | COMe | H |
| $SO_2Pr$-n | H | COMe | H |
| COMe | H | COMe | H |
| COEt | H | COMe | H |
| COPr-n | H | COMe | H |
| $CO_2H$ | H | COMe | H |
| $CO_2Me$ | H | COMe | H |
| $CO_2Et$ | H | COMe | H |
| $CO_2Pr$-n | H | COMe | H |
| $CO_2Bu$-n | H | COMe | H |
| $CO_2Pen$-n | H | COMe | H |
| $CH_2Cl$ | H | COMe | H |
| $CH_2Br$ | H | COMe | H |
| $CH_2I$ | H | COMe | H |
| $CH_2CF_3$ | H | COMe | H |
| $CH_2CN$ | H | COMe | H |
| $CH_2OH$ | H | COMe | H |
| $CH_2OMe$ | H | COMe | H |
| $CH_2OEt$ | H | COMe | H |
| $CH_2OPr$-n | H | COMe | H |
| $CH_2SMe$ | H | COMe | H |
| $CH_2SEt$ | H | COMe | H |
| $CH_2SPr$-n | H | COMe | H |
| $CH_2SO_2Me$ | H | COMe | H |
| $CH_2SO_2Et$ | H | COMe | H |
| $CH_2SO_2Pr$-n | H | COMe | H |
| $CH_2COMe$ | H | COMe | H |
| $CH_2COEt$ | H | COMe | H |
| $CH_2COPr$-n | H | COMe | H |
| $CH_2CO_2H$ | H | COMe | H |
| $CH_2CO_2Me$ | H | COMe | H |
| $CH_2CO_2Et$ | H | COMe | H |
| $CH_2CO_2Pr$-n | H | COMe | H |
| $CH_2OCH_2CH=CH_2$ | H | COMe | H |
| $CH_2OCH_2C\equiv CH$ | H | COMe | H |
| $CH=CHCO_2Me$ | H | COMe | H |
| $CH=CHCO_2Et$ | H | COMe | H |
| $CH=CHCO_2Pr$-n | H | COMe | H |

| $R_1$ | $R_2$ | $R_3$ | $R_4$ |
|---|---|---|---|
| CH=CHCN | H | COMe | H |
| Ph | H | COMe | H |
| $CH_2Ph$ | H | COMe | H |
| OPh | H | COMe | H |
| SPh | H | COMe | H |
| $SO_2Ph$ | H | COMe | H |
| CONHMe | H | COMe | H |
| CONHEt | H | COMe | H |
| CONHPr-n | H | COMe | H |
| CONHPr-i | H | COMe | H |
| CONHBu-n | H | COMe | H |
| CONHBu-sec | H | COMe | H |
| CONHBu-t | H | COMe | H |
| $CON(Me)_2$ | H | COMe | H |
| $CON(Et)_2$ | H | COMe | H |
| $CON(Pr\text{-}n)_2$ | H | COMe | H |
| F | H | $CO_2Me$ | H |
| Cl | H | $CO_2Me$ | H |
| Br | H | $CO_2Me$ | H |
| I | H | $CO_2Me$ | H |
| $CH=CH_2$ | H | $CO_2Me$ | H |
| $CH_2CH=CH_2$ | H | $CO_2Me$ | H |
| $C\equiv CH$ | H | $CO_2Me$ | H |
| $CH_2C\equiv CH$ | H | $CO_2Me$ | H |
| $CH_2F$ | H | $CO_2Me$ | H |
| $CHF_2$ | H | $CO_2Me$ | H |
| CN | H | $CO_2Me$ | H |
| $NH_2$ | H | $CO_2Me$ | H |
| NHCOMe | H | $CO_2Me$ | H |
| $CH_2NH_2$ | H | $CO_2Me$ | H |
| $CH_2NHCOMe$ | H | $CO_2Me$ | H |
| $CH_2OCHF_2$ | H | $CO_2Me$ | H |
| $CH_2OCF_3$ | H | $CO_2Me$ | H |
| $CF_3$ | H | $CO_2Me$ | H |
| $NO_2$ | H | $CO_2Me$ | H |
| OMe | H | $CO_2Me$ | H |
| OEt | H | $CO_2Me$ | H |
| OPr-n | H | $CO_2Me$ | H |
| OBu-n | H | $CO_2Me$ | H |
| OPen-n | H | $CO_2Me$ | H |

| $R_1$ | $R_2$ | $R_3$ | $R_4$ |
|---|---|---|---|
| SMe | H | $CO_2Me$ | H |
| SEt | H | $CO_2Me$ | H |
| SPr-n | H | $CO_2Me$ | H |
| SBu-n | H | $CO_2Me$ | H |
| SPen-n | H | $CO_2Me$ | H |
| $SO_2Me$ | H | $CO_2Me$ | H |
| $SO_2Et$ | H | $CO_2Me$ | H |
| $SO_2Pr$-n | H | $CO_2Me$ | H |
| COMe | H | $CO_2Me$ | H |
| COEt | H | $CO_2Me$ | H |
| COPr-n | H | $CO_2Me$ | H |
| $CO_2H$ | H | $CO_2Me$ | H |
| $CO_2Me$ | H | $CO_2Me$ | H |
| $CO_2Et$ | H | $CO_2Me$ | H |
| $CO_2Pr$-n | H | $CO_2Me$ | H |
| $CO_2Bu$-n | H | $CO_2Me$ | H |
| $CO_2Pen$-n | H | $CO_2Me$ | H |
| $CH_2Cl$ | H | $CO_2Me$ | H |
| $CH_2Br$ | H | $CO_2Me$ | H |
| $CH_2I$ | H | $CO_2Me$ | H |
| $CH_2CF_3$ | H | $CO_2Me$ | H |
| $CH_2CN$ | H | $CO_2Me$ | H |
| $CH_2OH$ | H | $CO_2Me$ | H |
| $CH_2OMe$ | H | $CO_2Me$ | H |
| $CH_2OEt$ | H | $CO_2Me$ | H |
| $CH_2OPr$-n | H | $CO_2Me$ | H |
| $CH_2SMe$ | H | $CO_2Me$ | H |
| $CH_2SEt$ | H | $CO_2Me$ | H |
| $CH_2SPr$-n | H | $CO_2Me$ | H |
| $CH_2SO_2Me$ | H | $CO_2Me$ | H |
| $CH_2SO_2Et$ | H | $CO_2Me$ | H |
| $CH_2SO_2Pr$-n | H | $CO_2Me$ | H |
| $CH_2COMe$ | H | $CO_2Me$ | H |
| $CH_2COEt$ | H | $CO_2Me$ | H |
| $CH_2COPr$-n | H | $CO_2Me$ | H |
| $CH_2CO_2H$ | H | $CO_2Me$ | H |
| $CH_2CO_2Me$ | H | $CO_2Me$ | H |
| $CH_2CO_2Et$ | H | $CO_2Me$ | H |
| $CH_2CO_2Pr$-n | H | $CO_2Me$ | H |
| $CH_2OCH_2CH=CH_2$ | H | $CO_2Me$ | H |

| $R_1$ | $R_2$ | $R_3$ | $R_4$ |
|---|---|---|---|
| $CH_2OCH_2C\equiv CH$ | H | $CO_2Me$ | H |
| $CH=CHCO_2Me$ | H | $CO_2Me$ | H |
| $CH=CHCO_2Et$ | H | $CO_2Me$ | H |
| $CH=CHCO_2Pr$-n | H | $CO_2Me$ | H |
| CH=CHCN | H | $CO_2Me$ | H |
| Ph | H | $CO_2Me$ | H |
| $CH_2Ph$ | H | $CO_2Me$ | H |
| -OPh | H | $CO_2Me$ | H |
| SPh | H | $CO_2Me$ | H |
| $SO_2Ph$ | H | $CO_2Me$ | H |
| CONHMe | H | $CO_2Me$ | H |
| CONHEt | H | $CO_2Me$ | H |
| CONHPr-n | H | $CO_2Me$ | H |
| CONHPr-i | H | $CO_2Me$ | H |
| CONHBu-n | H | $CO_2Me$ | H |
| CONHBu-sec | H | $CO_2Me$ | H |
| CONHBu-t | H | $CO_2Me$ | H |
| $CON(Me)_2$ | H | $CO_2Me$ | H |
| $CON(Et)_2$ | H | $CO_2Me$ | H |
| $CON(Pr\text{-}n)_2$ | H | $CO_2Me$ | H |
| F | H | $CO_2H$ | H |
| Cl | H | $CO_2H$ | H |
| Br | H | $CO_2H$ | H |
| I | H | $CO_2H$ | H |
| $CH=CH_2$ | H | $CO_2H$ | H |
| $CH_2CH=CH_2$ | H | $CO_2H$ | H |
| $C\equiv CH$ | H | $CO_2H$ | H |
| $CH_2C\equiv CH$ | H | $CO_2H$ | H |
| $CH_2F$ | H | $CO_2H$ | H |
| $CHF_2$ | H | $CO_2H$ | H |
| CN | H | $CO_2H$ | H |
| $NH_2$ | H | $CO_2H$ | H |
| NHCOMe | H | $CO_2H$ | H |
| $CH_2NH_2$ | H | $CO_2H$ | H |
| $CH_2NHCOMe$ | H | $CO_2H$ | H |
| $CH_2OCHF_2$ | H | $CO_2H$ | H |
| $CH_2OCF_3$ | H | $CO_2H$ | H |
| $CF_3$ | H | $CO_2H$ | H |
| $NO_2$ | H | $CO_2H$ | H |
| OMe | H | $CO_2H$ | H |

| $R_1$ | $R_2$ | $R_3$ | $R_4$ |
|---|---|---|---|
| OEt | H | $CO_2H$ | H |
| OPr-n | H | $CO_2H$ | H |
| OBu-n | H | $CO_2H$ | H |
| OPen-n | H | $CO_2H$ | H |
| SMe | H | $CO_2H$ | H |
| SEt | H | $CO_2H$ | H |
| SPr-n | H | $CO_2H$ | H |
| SBu-n | H | $CO_2H$ | H |
| SPen-n | H | $CO_2H$ | H |
| $SO_2Me$ | H | $CO_2H$ | H |
| $SO_2Et$ | H | $CO_2H$ | H |
| $SO_2Pr$-n | H | $CO_2H$ | H |
| COMe | H | $CO_2H$ | H |
| COEt | H | $CO_2H$ | H |
| COPr-n | H | $CO_2H$ | H |
| $CO_2H$ | H | $CO_2H$ | H |
| $CO_2Me$ | H | $CO_2H$ | H |
| $CO_2Et$ | H | $CO_2H$ | H |
| $CO_2Pr$-n | H | $CO_2H$ | H |
| $CO_2Bu$-n | H | $CO_2H$ | H |
| $CO_2Pen$-n | H | $CO_2H$ | H |
| $CH_2Cl$ | H | $CO_2H$ | H |
| $CH_2Br$ | H | $CO_2H$ | H |
| $CH_2I$ | H | $CO_2H$ | H |
| $CH_2CF_3$ | H | $CO_2H$ | H |
| $CH_2CN$ | H | $CO_2H$ | H |
| $CH_2OH$ | H | $CO_2H$ | H |
| $CH_2OMe$ | H | $CO_2H$ | H |
| $CH_2OEt$ | H | $CO_2H$ | H |
| $CH_2OPr$-n | H | $CO_2H$ | H |
| $CH_2SMe$ | H | $CO_2H$ | H |
| $CH_2SEt$ | H | $CO_2H$ | H |
| $CH_2SPr$-n | H | $CO_2H$ | H |
| $CH_2SO_2Me$ | H | $CO_2H$ | H |
| $CH_2SO_2Et$ | H | $CO_2H$ | H |
| $CH_2SO_2Pr$-n | H | $CO_2H$ | H |
| $CH_2COMe$ | H | $CO_2H$ | H |
| $CH_2COEt$ | H | $CO_2H$ | H |
| $CH_2COPr$-n | H | $CO_2H$ | H |
| $CH_2CO_2H$ | H | $CO_2H$ | H |

| $R_1$ | $R_2$ | $R_3$ | $R_4$ |
|---|---|---|---|
| $CH_2CO_2Me$ | H | $CO_2H$ | H |
| $CH_2CO_2Et$ | H | $CO_2H$ | H |
| $CH_2CO_2Pr$-n | H | $CO_2H$ | H |
| $CH_2OCH_2CH=CH_2$ | H | $CO_2H$ | H |
| $CH_2OCH_2C\equiv CH$ | H | $CO_2H$ | H |
| $CH=CHCO_2Me$ | H | $CO_2H$ | H |
| $CH=CHCO_2Et$ | H | $CO_2H$ | H |
| $CH=CHCO_2Pr$-n | H | $CO_2H$ | H |
| CH=CHCN | H | $CO_2H$ | H |
| Ph | H | $CO_2H$ | H |
| $CH_2Ph$ | H | $CO_2H$ | H |
| OPh | H | $CO_2H$ | H |
| SPh | H | $CO_2H$ | H |
| $SO_2Ph$ | H | $CO_2H$ | H |
| CONHMe | H | $CO_2H$ | H |
| CONHEt | H | $CO_2H$ | H |
| CONHPr-n | H | $CO_2H$ | H |
| CONHPr-i | H | $CO_2H$ | H |
| CONHBu-n | H | $CO_2H$ | H |
| CONHBu-sec | H | $CO_2H$ | H |
| CONHBu-t | H | $CO_2H$ | H |
| $CON(Me)_2$ | H | $CO_2H$ | H |
| $CON(Et)_2$ | H | $CO_2H$ | H |
| $CON(Pr\text{-}n)_2$ | H | $CO_2H$ | H |
| F | H | $CH_2F$ | H |
| Cl | H | $CH_2F$ | H |
| Br | H | $CH_2F$ | H |
| I | H | $CH_2F$ | H |
| $CH=CH_2$ | H | $CH_2F$ | H |
| $CH_2CH=CH_2$ | H | $CH_2F$ | H |
| $C\equiv CH$ | H | $CH_2F$ | H |
| $CH_2C\equiv CH$ | H | $CH_2F$ | H |
| $CH_2F$ | H | $CH_2F$ | H |
| $CHF_2$ | H | $CH_2F$ | H |
| CN | H | $CH_2F$ | H |
| $NH_2$ | H | $CH_2F$ | H |
| NHCOMe | H | $CH_2F$ | H |
| $CH_2NH_2$ | H | $CH_2F$ | H |
| $CH_2NHCOMe$ | H | $CH_2F$ | H |
| $CH_2OCHF_2$ | H | $CH_2F$ | H |

| $R_1$ | $R_2$ | $R_3$ | $R_4$ |
|---|---|---|---|
| $CH_2OCF_3$ | H | $CH_2F$ | H |
| $CF_3$ | H | $CH_2F$ | H |
| $NO_2$ | H | $CH_2F$ | H |
| OMe | H | $CH_2F$ | H |
| OEt | H | $CH_2F$ | H |
| OPr-n | H | $CH_2F$ | H |
| OBu-n | H | $CH_2F$ | H |
| OPen-n | H | $CH_2F$ | H |
| SMe | H | $CH_2F$ | H |
| SEt | H | $CH_2F$ | H |
| SPr-n | H | $CH_2F$ | H |
| SBu-n | H | $CH_2F$ | H |
| SPen-n | H | $CH_2F$ | H |
| $SO_2Me$ | H | $CH_2F$ | H |
| $SO_2Et$ | H | $CH_2F$ | H |
| $SO_2Pr$-n | H | $CH_2F$ | H |
| COMe | H | $CH_2F$ | H |
| COEt | H | $CH_2F$ | H |
| COPr-n | H | $CH_2F$ | H |
| $CO_2H$ | H | $CH_2F$ | H |
| $CO_2Me$ | H | $CH_2F$ | H |
| $CO_2Et$ | H | $CH_2F$ | H |
| $CO_2Pr$-n | H | $CH_2F$ | H |
| $CO_2Bu$-n | H | $CH_2F$ | H |
| $CO_2Pen$-n | H | $CH_2F$ | H |
| $CH_2Cl$ | H | $CH_2F$ | H |
| $CH_2Br$ | H | $CH_2F$ | H |
| $CH_2I$ | H | $CH_2F$ | H |
| $CH_2CF_3$ | H | $CH_2F$ | H |
| $CH_2CN$ | H | $CH_2F$ | H |
| $CH_2OH$ | H | $CH_2F$ | H |
| $CH_2OMe$ | H | $CH_2F$ | H |
| $CH_2OEt$ | H | $CH_2F$ | H |
| $CH_2OPr$-n | H | $CH_2F$ | H |
| $CH_2SMe$ | H | $CH_2F$ | H |
| $CH_2SEt$ | H | $CH_2F$ | H |
| $CH_2SPr$-n | H | $CH_2F$ | H |
| $CH_2SO_2Me$ | H | $CH_2F$ | H |
| $CH_2SO_2Et$ | H | $CH_2F$ | H |
| $CH_2SO_2Pr$-n | H | $CH_2F$ | H |

| $R_1$ | $R_2$ | $R_3$ | $R_4$ |
|---|---|---|---|
| $CH_2COMe$ | H | $CH_2F$ | H |
| $CH_2COEt$ | H | $CH_2F$ | H |
| $CH_2COPr$-n | H | $CH_2F$ | H |
| $CH_2CO_2H$ | H | $CH_2F$ | H |
| $CH_2CO_2Me$ | H | $CH_2F$ | H |
| $CH_2CO_2Et$ | H | $CH_2F$ | H |
| $CH_2CO_2Pr$-n | H | $CH_2F$ | H |
| $CH_2OCH_2CH=CH_2$ | H | $CH_2F$ | H |
| $CH_2OCH_2C\equiv CH$ | H | $CH_2F$ | H |
| $CH=CHCO_2Me$ | H | $CH_2F$ | H |
| $CH=CHCO_2Et$ | H | $CH_2F$ | H |
| $CH=CHCO_2Pr$-n | H | $CH_2F$ | H |
| CH=CHCN | H | $CH_2F$ | H |
| Ph | H | $CH_2F$ | H |
| $CH_2Ph$ | H | $CH_2F$ | H |
| OPh | H | $CH_2F$ | H |
| SPh | H | $CH_2F$ | H |
| $SO_2Ph$ | H | $CH_2F$ | H |
| CONHMe | H | $CH_2F$ | H |
| CONHEt | H | $CH_2F$ | H |
| CONHPr-n | H | $CH_2F$ | H |
| CONHPr-i | H | $CH_2F$ | H |
| CONHBu-n | H | $CH_2F$ | H |
| CONHBu-sec | H | $CH_2F$ | H |
| CONHBu-t | H | $CH_2F$ | H |
| $CON(Me)_2$ | H | $CH_2F$ | H |
| $CON(Et)_2$ | H | $CH_2F$ | H |
| $CON(Pr\text{-}n)_2$ | H | $CH_2F$ | H |
| F | H | $CH_2Cl$ | H |
| Cl | H | $CH_2Cl$ | H |
| Br | H | $CH_2Cl$ | H |
| I | H | $CH_2Cl$ | H |
| $CH=CH_2$ | H | $CH_2Cl$ | H |
| $CH_2CH=CH_2$ | H | $CH_2Cl$ | H |
| $C\equiv CH$ | H | $CH_2Cl$ | H |
| $CH_2C\equiv CH$ | H | $CH_2Cl$ | H |
| $CH_2F$ | H | $CH_2Cl$ | H |
| $CHF_2$ | H | $CH_2Cl$ | H |
| CN | H | $CH_2Cl$ | H |
| $NH_2$ | H | $CH_2Cl$ | H |

| $R_1$ | $R_2$ | $R_3$ | $R_4$ |
|---|---|---|---|
| NHCOMe | H | $CH_2Cl$ | H |
| $CH_2NH_2$ | H | $CH_2Cl$ | H |
| $CH_2NHCOMe$ | H | $CH_2Cl$ | H |
| $CH_2OCHF_2$ | H | $CH_2Cl$ | H |
| $CH_2OCF_3$ | H | $CH_2Cl$ | H |
| $CF_3$ | H | $CH_2Cl$ | H |
| $NO_2$ | H | $CH_2Cl$ | H |
| OMe | H | $CH_2Cl$ | H |
| OEt | H | $CH_2Cl$ | H |
| OPr-n | H | $CH_2Cl$ | H |
| OBu-n | H | $CH_2Cl$ | H |
| OPen-n | H | $CH_2Cl$ | H |
| SMe | H | $CH_2Cl$ | H |
| SEt | H | $CH_2Cl$ | H |
| SPr-n | H | $CH_2Cl$ | H |
| SBu-n | H | $CH_2Cl$ | H |
| SPen-n | H | $CH_2Cl$ | H |
| $SO_2Me$ | H | $CH_2Cl$ | H |
| $SO_2Et$ | H | $CH_2Cl$ | H |
| $SO_2Pr$-n | H | $CH_2Cl$ | H |
| COMe | H | $CH_2Cl$ | H |
| COEt | H | $CH_2Cl$ | H |
| COPr-n | H | $CH_2Cl$ | H |
| $CO_2H$ | H | $CH_2Cl$ | H |
| $CO_2Me$ | H | $CH_2Cl$ | H |
| $CO_2Et$ | H | $CH_2Cl$ | H |
| $CO_2Pr$-n | H | $CH_2Cl$ | H |
| $CO_2Bu$-n | H | $CH_2Cl$ | H |
| $CO_2Pen$-n | H | $CH_2Cl$ | H |
| $CH_2Cl$ | H | $CH_2Cl$ | H |
| $CH_2Br$ | H | $CH_2Cl$ | H |
| $CH_2I$ | H | $CH_2Cl$ | H |
| $CH_2CF_3$ | H | $CH_2Cl$ | H |
| $CH_2CN$ | H | $CH_2Cl$ | H |
| $CH_2OH$ | H | $CH_2Cl$ | H |
| $CH_2OMe$ | H | $CH_2Cl$ | H |
| $CH_2OEt$ | H | $CH_2Cl$ | H |
| $CH_2OPr$-n | H | $CH_2Cl$ | H |
| $CH_2SMe$ | H | $CH_2Cl$ | H |
| $CH_2SEt$ | H | $CH_2Cl$ | H |

| $R_1$ | $R_2$ | $R_3$ | $R_4$ |
|---|---|---|---|
| $CH_2SPr$-n | H | $CH_2Cl$ | H |
| $CH_2SO_2Me$ | H | $CH_2Cl$ | H |
| $CH_2SO_2Et$ | H | $CH_2Cl$ | H |
| $CH_2SO_2Pr$-n | H | $CH_2Cl$ | H |
| $CH_2COMe$ | H | $CH_2Cl$ | H |
| $CH_2COEt$ | H | $CH_2Cl$ | H |
| $CH_2COPr$-n | H | $CH_2Cl$ | H |
| $CH_2CO_2H$ | H | $CH_2Cl$ | H |
| $CH_2CO_2Me$ | H | $CH_2Cl$ | H |
| $CH_2CO_2Et$ | H | $CH_2Cl$ | H |
| $CH_2CO_2Pr$-n | H | $CH_2Cl$ | H |
| $CH_2OCH_2CH=CH_2$ | H | $CH_2Cl$ | H |
| $CH_2OCH_2C\equiv CH$ | H | $CH_2Cl$ | H |
| $CH=CHCO_2Me$ | H | $CH_2Cl$ | H |
| $CH=CHCO_2Et$ | H | $CH_2Cl$ | H |
| $CH=CHCO_2Pr$-n | H | $CH_2Cl$ | H |
| CH=CHCN | H | $CH_2Cl$ | H |
| Ph | H | $CH_2Cl$ | H |
| $CH_2Ph$ | H | $CH_2Cl$ | H |
| OPh | H | $CH_2Cl$ | H |
| SPh | H | $CH_2Cl$ | H |
| $SO_2Ph$ | H | $CH_2Cl$ | H |
| CONHMe | H | $CH_2Cl$ | H |
| CONHEt | H | $CH_2Cl$ | H |
| CONHPr-n | H | $CH_2Cl$ | H |
| CONHPr-i | H | $CH_2Cl$ | H |
| CONHBu-n | H | $CH_2Cl$ | H |
| CONHBu-sec | H | $CH_2Cl$ | H |
| CONHBu-t | H | $CH_2Cl$ | H |
| $CON(Me)_2$ | H | $CH_2Cl$ | H |
| $CON(Et)_2$ | H | $CH_2Cl$ | H |
| $CON(Pr$-n$)_2$ | H | $CH_2Cl$ | H |
| F | H | $CH_2OMe$ | H |
| Cl | H | $CH_2OMe$ | H |
| Br | H | $CH_2OMe$ | H |
| I | H | $CH_2OMe$ | H |
| $CH=CH_2$ | H | $CH_2OMe$ | H |
| $CH_2CH=CH_2$ | H | $CH_2OMe$ | H |
| $C\equiv CH$ | H | $CH_2OMe$ | H |
| $CH_2C\equiv CH$ | H | $CH_2OMe$ | H |

| $R_1$ | $R_2$ | $R_3$ | $R_4$ |
|---|---|---|---|
| $CH_2F$ | H | $CH_2OMe$ | H |
| $CHF_2$ | H | $CH_2OMe$ | H |
| CN | H | $CH_2OMe$ | H |
| $NH_2$ | H | $CH_2OMe$ | H |
| NHCOMe | H | $CH_2OMe$ | H |
| $CH_2NH_2$ | H | $CH_2OMe$ | H |
| $CH_2NHCOMe$ | H | $CH_2OMe$ | H |
| $CH_2OCHF_2$ | H | $CH_2OMe$ | H |
| $CH_2OCF_3$ | H | $CH_2OMe$ | H |
| $CF_3$ | H | $CH_2OMe$ | H |
| $NO_2$ | H | $CH_2OMe$ | H |
| OMe | H | $CH_2OMe$ | H |
| OEt | H | $CH_2OMe$ | H |
| OPr-n | H | $CH_2OMe$ | H |
| OBu-n | H | $CH_2OMe$ | H |
| OPen-n | H | $CH_2OMe$ | H |
| SMe | H | $CH_2OMe$ | H |
| SEt | H | $CH_2OMe$ | H |
| SPr-n | H | $CH_2OMe$ | H |
| SBu-n | H | $CH_2OMe$ | H |
| SPen-n | H | $CH_2OMe$ | H |
| $SO_2Me$ | H | $CH_2OMe$ | H |
| $SO_2Et$ | H | $CH_2OMe$ | H |
| $SO_2Pr$-n | H | $CH_2OMe$ | H |
| COMe | H | $CH_2OMe$ | H |
| COEt | H | $CH_2OMe$ | H |
| COPr-n | H | $CH_2OMe$ | H |
| $CO_2H$ | H | $CH_2OMe$ | H |
| $CO_2Me$ | H | $CH_2OMe$ | H |
| $CO_2Et$ | H | $CH_2OMe$ | H |
| $CO_2Pr$-n | H | $CH_2OMe$ | H |
| $CO_2Bu$-n | H | $CH_2OMe$ | H |
| $CO_2Pen$-n | H | $CH_2OMe$ | H |
| $CH_2Cl$ | H | $CH_2OMe$ | H |
| $CH_2Br$ | H | $CH_2OMe$ | H |
| $CH_2I$ | H | $CH_2OMe$ | H |
| $CH_2CF_3$ | H | $CH_2OMe$ | H |
| $CH_2CN$ | H | $CH_2OMe$ | H |
| $CH_2OH$ | H | $CH_2OMe$ | H |
| $CH_2OMe$ | H | $CH_2OMe$ | H |

| $R_1$ | $R_2$ | $R_3$ | $R_4$ |
|---|---|---|---|
| $CH_2OEt$ | H | $CH_2OMe$ | H |
| $CH_2OPr$-n | H | $CH_2OMe$ | H |
| $CH_2SMe$ | H | $CH_2OMe$ | H |
| $CH_2SEt$ | H | $CH_2OMe$ | H |
| $CH_2SPr$-n | H | $CH_2OMe$ | H |
| $CH_2SO_2Me$ | H | $CH_2OMe$ | H |
| $CH_2SO_2Et$ | H | $CH_2OMe$ | H |
| $CH_2SO_2Pr$-n | H | $CH_2OMe$ | H |
| $CH_2COMe$ | H | $CH_2OMe$ | H |
| $CH_2COEt$ | H | $CH_2OMe$ | H |
| $CH_2COPr$-n | H | $CH_2OMe$ | H |
| $CH_2CO_2H$ | H | $CH_2OMe$ | H |
| $CH_2CO_2Me$ | H | $CH_2OMe$ | H |
| $CH_2CO_2Et$ | H | $CH_2OMe$ | H |
| $CH_2CO_2Pr$-n | H | $CH_2OMe$ | H |
| $CH_2OCH_2CH{=}CH_2$ | H | $CH_2OMe$ | H |
| $CH_2OCH_2C{\equiv}CH$ | H | $CH_2OMe$ | H |
| $CH{=}CHCO_2Me$ | H | $CH_2OMe$ | H |
| $CH{=}CHCO_2Et$ | H | $CH_2OMe$ | H |
| $CH{=}CHCO_2Pr$-n | H | $CH_2OMe$ | H |
| $CH{=}CHCN$ | H | $CH_2OMe$ | H |
| Ph | H | $CH_2OMe$ | H |
| $CH_2Ph$ | H | $CH_2OMe$ | H |
| OPh | H | $CH_2OMe$ | H |
| SPh | H | $CH_2OMe$ | H |
| $SO_2Ph$ | H | $CH_2OMe$ | H |
| CONHMe | H | $CH_2OMe$ | H |
| CONHEt | H | $CH_2OMe$ | H |
| CONHPr-n | H | $CH_2OMe$ | H |
| CONHPr-i | H | $CH_2OMe$ | H |
| CONHBu-n | H | $CH_2OMe$ | H |
| CONHBu-sec | H | $CH_2OMe$ | H |
| CONHBu-t | H | $CH_2OMe$ | H |
| $CON(Me)_2$ | H | $CH_2OMe$ | H |
| $CON(Et)_2$ | H | $CH_2OMe$ | H |
| $CON(Pr\text{-}n)_2$ | H | $CH_2OMe$ | H |
| F | H | $CH_2SMe$ | H |
| Cl | H | $CH_2SMe$ | H |
| Br | H | $CH_2SMe$ | H |
| I | H | $CH_2SMe$ | H |

| $R_1$ | $R_2$ | $R_3$ | $R_4$ |
|---|---|---|---|
| $CH=CH_2$ | H | $CH_2SMe$ | H |
| $CH_2CH=CH_2$ | H | $CH_2SMe$ | H |
| $C\equiv CH$ | H | $CH_2SMe$ | H |
| $CH_2C\equiv CH$ | H | $CH_2SMe$ | H |
| $CH_2F$ | H | $CH_2SMe$ | H |
| $CHF_2$ | H | $CH_2SMe$ | H |
| CN | H | $CH_2SMe$ | H |
| $NH_2$ | H | $CH_2SMe$ | H |
| NHCOMe | H | $CH_2SMe$ | H |
| $CH_2NH_2$ | H | $CH_2SMe$ | H |
| $CH_2NHCOMe$ | H | $CH_2SMe$ | H |
| $CH_2OCHF_2$ | H | $CH_2SMe$ | H |
| $CH_2OCF_3$ | H | $CH_2SMe$ | H |
| $CF_3$ | H | $CH_2SMe$ | H |
| $NO_2$ | H | $CH_2SMe$ | H |
| OMe | H | $CH_2SMe$ | H |
| OEt | H | $CH_2SMe$ | H |
| OPr-n | H | $CH_2SMe$ | H |
| OBu-n | H | $CH_2SMe$ | H |
| OPen-n | H | $CH_2SMe$ | H |
| SMe | H | $CH_2SMe$ | H |
| SEt | H | $CH_2SMe$ | H |
| SPr-n | H | $CH_2SMe$ | H |
| SBu-n | H | $CH_2SMe$ | H |
| SPen-n | H | $CH_2SMe$ | H |
| $SO_2Me$ | H | $CH_2SMe$ | H |
| $SO_2Et$ | H | $CH_2SMe$ | H |
| $SO_2Pr$-n | H | $CH_2SMe$ | H |
| COMe | H | $CH_2SMe$ | H |
| COEt | H | $CH_2SMe$ | H |
| COPr-n | H | $CH_2SMe$ | H |
| $CO_2H$ | H | $CH_2SMe$ | H |
| $CO_2Me$ | H | $CH_2SMe$ | H |
| $CO_2Et$ | H | $CH_2SMe$ | H |
| $CO_2Pr$-n | H | $CH_2SMe$ | H |
| $CO_2Bu$-n | H | $CH_2SMe$ | H |
| $CO_2Pen$-n | H | $CH_2SMe$ | H |
| $CH_2Cl$ | H | $CH_2SMe$ | H |
| $CH_2Br$ | H | $CH_2SMe$ | H |
| $CH_2I$ | H | $CH_2SMe$ | H |

| $R_1$ | $R_2$ | $R_3$ | $R_4$ |
|---|---|---|---|
| $CH_2CF_3$ | H | $CH_2SMe$ | H |
| $CH_2CN$ | H | $CH_2SMe$ | H |
| $CH_2OH$ | H | $CH_2SMe$ | H |
| $CH_2OMe$ | H | $CH_2SMe$ | H |
| $CH_2OEt$ | H | $CH_2SMe$ | H |
| $CH_2OPr$-n | H | $CH_2SMe$ | H |
| $CH_2SMe$ | H | $CH_2SMe$ | H |
| $CH_2SEt$ | H | $CH_2SMe$ | H |
| $CH_2SPr$-n | H | $CH_2SMe$ | H |
| $CH_2SO_2Me$ | H | $CH_2SMe$ | H |
| $CH_2SO_2Et$ | H | $CH_2SMe$ | H |
| $CH_2SO_2Pr$-n | H | $CH_2SMe$ | H |
| $CH_2COMe$ | H | $CH_2SMe$ | H |
| $CH_2COEt$ | H | $CH_2SMe$ | H |
| $CH_2COPr$-n | H | $CH_2SMe$ | H |
| $CH_2CO_2H$ | H | $CH_2SMe$ | H |
| $CH_2CO_2Me$ | H | $CH_2SMe$ | H |
| $CH_2CO_2Et$ | H | $CH_2SMe$ | H |
| $CH_2CO_2Pr$-n | H | $CH_2SMe$ | H |
| $CH_2OCH_2CH=CH_2$ | H | $CH_2SMe$ | H |
| $CH_2OCH_2C\equiv CH$ | H | $CH_2SMe$ | H |
| $CH=CHCO_2Me$ | H | $CH_2SMe$ | H |
| $CH=CHCO_2Et$ | H | $CH_2SMe$ | H |
| $CH=CHCO_2Pr$-n | H | $CH_2SMe$ | H |
| CH=CHCN | H | $CH_2SMe$ | H |
| Ph | H | $CH_2SMe$ | H |
| $CH_2Ph$ | H | $CH_2SMe$ | H |
| OPh | H | $CH_2SMe$ | H |
| SPh | H | $CH_2SMe$ | H |
| $SO_2Ph$ | H | $CH_2SMe$ | H |
| CONHMe | H | $CH_2SMe$ | H |
| CONHEt | H | $CH_2SMe$ | H |
| CONHPr-n | H | $CH_2SMe$ | H |
| CONHPr-i | H | $CH_2SMe$ | H |
| CONHBu-n | H | $CH_2SMe$ | H |
| CONHBu-sec | H | $CH_2SMe$ | H |
| CONHBu-t | H | $CH_2SMe$ | H |
| $CON(Me)_2$ | H | $CH_2SMe$ | H |
| $CON(Et)_2$ | H | $CH_2SMe$ | H |
| $CON(Pr\text{-}n)_2$ | H | $CH_2SMe$ | H |

| $R_1$ | $R_2$ | $R_3$ | $R_4$ |
|---|---|---|---|
| F | H | $CH_2SO_2Me$ | H |
| Cl | H | $CH_2SO_2Me$ | H |
| Br | H | $CH_2SO_2Me$ | H |
| I | H | $CH_2SO_2Me$ | H |
| $CH=CH_2$ | H | $CH_2SO_2Me$ | H |
| $CH_2CH=CH_2$ | H | $CH_2SO_2Me$ | H |
| $C\equiv CH$ | H | $CH_2SO_2Me$ | H |
| $CH_2C\equiv CH$ | H | $CH_2SO_2Me$ | H |
| $CH_2F$ | H | $CH_2SO_2Me$ | H |
| $CHF_2$ | H | $CH_2SO_2Me$ | H |
| CN | H | $CH_2SO_2Me$ | H |
| $NH_2$ | H | $CH_2SO_2Me$ | H |
| NHCOMe | H | $CH_2SO_2Me$ | H |
| $CH_2NH_2$ | H | $CH_2SO_2Me$ | H |
| $CH_2NHCOMe$ | H | $CH_2SO_2Me$ | H |
| $CH_2OCHF_2$ | H | $CH_2SO_2Me$ | H |
| $CH_2OCF_3$ | H | $CH_2SO_2Me$ | H |
| $CF_3$ | H | $CH_2SO_2Me$ | H |
| $NO_2$ | H | $CH_2SO_2Me$ | H |
| OMe | H | $CH_2SO_2Me$ | H |
| OEt | H | $CH_2SO_2Me$ | H |
| OPr-n | H | $CH_2SO_2Me$ | H |
| OBu-n | H | $CH_2SO_2Me$ | H |
| OPen-n | H | $CH_2SO_2Me$ | H |
| SMe | H | $CH_2SO_2Me$ | H |
| SEt | H | $CH_2SO_2Me$ | H |
| SPr-n | H | $CH_2SO_2Me$ | H |
| SBu-n | H | $CH_2SO_2Me$ | H |
| SPen-n | H | $CH_2SO_2Me$ | H |
| $SO_2Me$ | H | $CH_2SO_2Me$ | H |
| $SO_2Et$ | H | $CH_2SO_2Me$ | H |
| $SO_2Pr$-n | H | $CH_2SO_2Me$ | H |
| COMe | H | $CH_2SO_2Me$ | H |
| COEt | H | $CH_2SO_2Me$ | H |
| COPr-n | H | $CH_2SO_2Me$ | H |
| $CO_2H$ | H | $CH_2SO_2Me$ | H |
| $CO_2Me$ | H | $CH_2SO_2Me$ | H |
| $CO_2Et$ | H | $CH_2SO_2Me$ | H |
| $CO_2Pr$-n | H | $CH_2SO_2Me$ | H |
| $CO_2Bu$-n | H | $CH_2SO_2Me$ | H |

| $R_1$ | $R_2$ | $R_3$ | $R_4$ |
|---|---|---|---|
| $CO_2$Pen-n | H | $CH_2SO_2Me$ | H |
| $CH_2Cl$ | H | $CH_2SO_2Me$ | H |
| $CH_2Br$ | H | $CH_2SO_2Me$ | H |
| $CH_2I$ | H | $CH_2SO_2Me$ | H |
| $CH_2CF_3$ | H | $CH_2SO_2Me$ | H |
| $CH_2CN$ | H | $CH_2SO_2Me$ | H |
| $CH_2OH$ | H | $CH_2SO_2Me$ | H |
| $CH_2OMe$ | H | $CH_2SO_2Me$ | H |
| $CH_2OEt$ | H | $CH_2SO_2Me$ | H |
| $CH_2O$Pr-n | H | $CH_2SO_2Me$ | H |
| $CH_2SMe$ | H | $CH_2SO_2Me$ | H |
| $CH_2SEt$ | H | $CH_2SO_2Me$ | H |
| $CH_2S$Pr-n | H | $CH_2SO_2Me$ | H |
| $CH_2SO_2Me$ | H | $CH_2SO_2Me$ | H |
| $CH_2SO_2Et$ | H | $CH_2SO_2Me$ | H |
| $CH_2SO_2$Pr-n | H | $CH_2SO_2Me$ | H |
| $CH_2COMe$ | H | $CH_2SO_2Me$ | H |
| $CH_2COEt$ | H | $CH_2SO_2Me$ | H |
| $CH_2CO$Pr-n | H | $CH_2SO_2Me$ | H |
| $CH_2CO_2H$ | H | $CH_2SO_2Me$ | H |
| $CH_2CO_2Me$ | H | $CH_2SO_2Me$ | H |
| $CH_2CO_2Et$ | H | $CH_2SO_2Me$ | H |
| $CH_2CO_2$Pr-n | H | $CH_2SO_2Me$ | H |
| $CH_2OCH_2CH=CH_2$ | H | $CH_2SO_2Me$ | H |
| $CH_2OCH_2C\equiv CH$ | H | $CH_2SO_2Me$ | H |
| $CH=CHCO_2Me$ | H | $CH_2SO_2Me$ | H |
| $CH=CHCO_2Et$ | H | $CH_2SO_2Me$ | H |
| $CH=CHCO_2$Pr-n | H | $CH_2SO_2Me$ | H |
| CH=CHCN | H | $CH_2SO_2Me$ | H |
| Ph | H | $CH_2SO_2Me$ | H |
| $CH_2$Ph | H | $CH_2SO_2Me$ | H |
| OPh | H | $CH_2SO_2Me$ | H |
| SPh | H | $CH_2SO_2Me$ | H |
| $SO_2$Ph | H | $CH_2SO_2Me$ | H |
| CONHMe | H | $CH_2SO_2Me$ | H |
| CONHEt | H | $CH_2SO_2Me$ | H |
| CONHPr-n | H | $CH_2SO_2Me$ | H |
| CONHPr-i | H | $CH_2SO_2Me$ | H |
| CONHBu-n | H | $CH_2SO_2Me$ | H |
| CONHBu-sec | H | $CH_2SO_2Me$ | H |

| $R_1$ | $R_2$ | $R_3$ | $R_4$ |
|---|---|---|---|
| CONHBu-t | H | $CH_2SO_2Me$ | H |
| $CON(Me)_2$ | H | $CH_2SO_2Me$ | H |
| $CON(Et)_2$ | H | $CH_2SO_2Me$ | H |
| $CON(Pr\text{-}n)_2$ | H | $CH_2SO_2Me$ | H |
| F | H | $CH_2CO_2Me$ | H |
| Cl | H | $CH_2CO_2Me$ | H |
| Br | H | $CH_2CO_2Me$ | H |
| I | H | $CH_2CO_2Me$ | H |
| $CH{=}CH_2$ | H | $CH_2CO_2Me$ | H |
| $CH_2CH{=}CH_2$ | H | $CH_2CO_2Me$ | H |
| $C\equiv CH$ | H | $CH_2CO_2Me$ | H |
| $CH_2C\equiv CH$ | H | $CH_2CO_2Me$ | H |
| $CH_2F$ | H | $CH_2CO_2Me$ | H |
| $CHF_2$ | H | $CH_2CO_2Me$ | H |
| CN | H | $CH_2CO_2Me$ | H |
| $NH_2$ | H | $CH_2CO_2Me$ | H |
| NHCOMe | H | $CH_2CO_2Me$ | H |
| $CH_2NH_2$ | H | $CH_2CO_2Me$ | H |
| $CH_2NHCOMe$ | H | $CH_2CO_2Me$ | H |
| $CH_2OCHF_2$ | H | $CH_2CO_2Me$ | H |
| $CH_2OCF_3$ | H | $CH_2CO_2Me$ | H |
| $CF_3$ | H | $CH_2CO_2Me$ | H |
| $NO_2$ | H | $CH_2CO_2Me$ | H |
| OMe | H | $CH_2CO_2Me$ | H |
| OEt | H | $CH_2CO_2Me$ | H |
| OPr-n | H | $CH_2CO_2Me$ | H |
| OBu-n | H | $CH_2CO_2Me$ | H |
| OPen-n | H | $CH_2CO_2Me$ | H |
| SMe | H | $CH_2CO_2Me$ | H |
| SEt | H | $CH_2CO_2Me$ | H |
| SPr-n | H | $CH_2CO_2Me$ | H |
| SBu-n | H | $CH_2CO_2Me$ | H |
| SPen-n | H | $CH_2CO_2Me$ | H |
| $SO_2Me$ | H | $CH_2CO_2Me$ | H |
| $SO_2Et$ | H | $CH_2CO_2Me$ | H |
| $SO_2Pr\text{-}n$ | H | $CH_2CO_2Me$ | H |
| COMe | H | $CH_2CO_2Me$ | H |
| COEt | H | $CH_2CO_2Me$ | H |
| COPr-n | H | $CH_2CO_2Me$ | H |
| $CO_2H$ | H | $CH_2CO_2Me$ | H |

| $R_1$ | $R_2$ | $R_3$ | $R_4$ |
|---|---|---|---|
| $CO_2Me$ | H | $CH_2CO_2Me$ | H |
| $CO_2Et$ | H | $CH_2CO_2Me$ | H |
| $CO_2Pr$-n | H | $CH_2CO_2Me$ | H |
| $CO_2Bu$-n | H | $CH_2CO_2Me$ | H |
| $CO_2Pen$-n | H | $CH_2CO_2Me$ | H |
| $CH_2Cl$ | H | $CH_2CO_2Me$ | H |
| $CH_2Br$ | H | $CH_2CO_2Me$ | H |
| $CH_2I$ | H | $CH_2CO_2Me$ | H |
| $CH_2CF_3$ | H | $CH_2CO_2Me$ | H |
| $CH_2CN$ | H | $CH_2CO_2Me$ | H |
| $CH_2OH$ | H | $CH_2CO_2Me$ | H |
| $CH_2OMe$ | H | $CH_2CO_2Me$ | H |
| $CH_2OEt$ | H | $CH_2CO_2Me$ | H |
| $CH_2OPr$-n | H | $CH_2CO_2Me$ | H |
| $CH_2SMe$ | H | $CH_2CO_2Me$ | H |
| $CH_2SEt$ | H | $CH_2CO_2Me$ | H |
| $CH_2SPr$-n | H | $CH_2CO_2Me$ | H |
| $CH_2SO_2Me$ | H | $CH_2CO_2Me$ | H |
| $CH_2SO_2Et$ | H | $CH_2CO_2Me$ | H |
| $CH_2SO_2Pr$-n | H | $CH_2CO_2Me$ | H |
| $CH_2COMe$ | H | $CH_2CO_2Me$ | H |
| $CH_2COEt$ | H | $CH_2CO_2Me$ | H |
| $CH_2COPr$-n | H | $CH_2CO_2Me$ | H |
| $CH_2CO_2H$ | H | $CH_2CO_2Me$ | H |
| $CH_2CO_2Me$ | H | $CH_2CO_2Me$ | H |
| $CH_2CO_2Et$ | H | $CH_2CO_2Me$ | H |
| $CH_2CO_2Pr$-n | H | $CH_2CO_2Me$ | H |
| $CH_2OCH_2CH=CH_2$ | H | $CH_2CO_2Me$ | H |
| $CH_2OCH_2C\equiv CH$ | H | $CH_2CO_2Me$ | H |
| $CH=CHCO_2Me$ | H | $CH_2CO_2Me$ | H |
| $CH=CHCO_2Et$ | H | $CH_2CO_2Me$ | H |
| $CH=CHCO_2Pr$-n | H | $CH_2CO_2Me$ | H |
| $CH=CHCN$ | H | $CH_2CO_2Me$ | H |
| Ph | H | $CH_2CO_2Me$ | H |
| $CH_2Ph$ | H | $CH_2CO_2Me$ | H |
| OPh | H | $CH_2CO_2Me$ | H |
| SPh | H | $CH_2CO_2Me$ | H |
| $SO_2Ph$ | H | $CH_2CO_2Me$ | H |
| CONHMe | H | $CH_2CO_2Me$ | H |
| CONHEt | H | $CH_2CO_2Me$ | H |

| $R_1$ | $R_2$ | $R_3$ | $R_4$ |
|---|---|---|---|
| CONHPr-n | H | $CH_2CO_2Me$ | H |
| CONHPr-i | H | $CH_2CO_2Me$ | H |
| CONHBu-n | H | $CH_2CO_2Me$ | H |
| CONHBu-sec | H | $CH_2CO_2Me$ | H |
| CONHBu-t | H | $CH_2CO_2Me$ | H |
| $CON(Me)_2$ | H | $CH_2CO_2Me$ | H |
| $CON(Et)_2$ | H | $CH_2CO_2Me$ | H |
| $CON(Pr-n)_2$ | H | $CH_2CO_2Me$ | H |
| F | H | Ph | H |
| Cl | H | Ph | H |
| Br | H | Ph | H |
| I | H | Ph | H |
| $CH=CH_2$ | H | Ph | H |
| $CH_2CH=CH_2$ | H | Ph | H |
| $C\equiv CH$ | H | Ph | H |
| $CH_2C\equiv CH$ | H | Ph | H |
| $CH_2F$ | H | Ph | H |
| $CHF_2$ | H | Ph | H |
| CN | H | Ph | H |
| $NH_2$ | H | Ph | H |
| NHCOMe | H | Ph | H |
| $CH_2NH_2$ | H | Ph | H |
| $CH_2NHCOMe$ | H | Ph | H |
| $CH_2OCHF_2$ | H | Ph | H |
| $CH_2OCF_3$ | H | Ph | H |
| $CF_3$ | H | Ph | H |
| $NO_2$ | H | Ph | H |
| OMe | H | Ph | H |
| OEt | H | Ph | H |
| OPr-n | H | Ph | H |
| OBu-n | H | Ph | H |
| OPen-n | H | Ph | H |
| SMe | H | Ph | H |
| SEt | H | Ph | H |
| SPr-n | H | Ph | H |
| SBu-n | H | Ph | H |
| SPen-n | H | Ph | H |
| $SO_2Me$ | H | Ph | H |
| $SO_2Et$ | H | Ph | H |
| $SO_2Pr-n$ | H | Ph | H |

| $R_1$ | $R_2$ | $R_3$ | $R_4$ |
|---|---|---|---|
| COMe | H | Ph | H |
| COEt | H | Ph | H |
| COPr-n | H | Ph | H |
| $CO_2H$ | H | Ph | H |
| $CO_2Me$ | H | Ph | H |
| $CO_2Et$ | H | Ph | H |
| $CO_2Pr$-n | H | Ph | H |
| $CO_2Bu$-n | H | Ph | H |
| $CO_2Pen$-n | H | Ph | H |
| $CH_2Cl$ | H | Ph | H |
| $CH_2Br$ | H | Ph | H |
| $CH_2I$ | H | Ph | H |
| $CH_2CF_3$ | H | Ph | H |
| $CH_2CN$ | H | Ph | H |
| $CH_2OH$ | H | Ph | H |
| $CH_2OMe$ | H | Ph | H |
| $CH_2OEt$ | H | Ph | H |
| $CH_2OPr$-n | H | Ph | H |
| $CH_2SMe$ | H | Ph | H |
| $CH_2SEt$ | H | Ph | H |
| $CH_2SPr$-n | H | Ph | H |
| $CH_2SO_2Me$ | H | Ph | H |
| $CH_2SO_2Et$ | H | Ph | H |
| $CH_2SO_2Pr$-n | H | Ph | H |
| $CH_2COMe$ | H | Ph | H |
| $CH_2COEt$ | H | Ph | H |
| $CH_2COPr$-n | H | Ph | H |
| $CH_2CO_2H$ | H | Ph | H |
| $CH_2CO_2Me$ | H | Ph | H |
| $CH_2CO_2Et$ | H | Ph | H |
| $CH_2CO_2Pr$-n | H | Ph | H |
| $CH_2OCH_2CH=CH_2$ | H | Ph | H |
| $CH_2OCH_2C\equiv CH$ | H | Ph | H |
| $CH=CHCO_2Me$ | H | Ph | H |
| $CH=CHCO_2Et$ | H | Ph | H |
| $CH=CHCO_2Pr$-n | H | Ph | H |
| CH=CHCN | H | Ph | H |
| Ph | H | Ph | H |
| $CH_2Ph$ | H | Ph | H |
| OPh | H | Ph | H |

| $R_1$ | $R_2$ | $R_3$ | $R_4$ |
|---|---|---|---|
| SPh | H | Ph | H |
| $SO_2Ph$ | H | Ph | H |
| CONHMe | H | Ph | H |
| CONHEt | H | Ph | H |
| CONHPr-n | H | Ph | H |
| CONHPr-i | H | Ph | H |
| CONHBu-n | H | Ph | H |
| CONHBu-sec | H | Ph | H |
| CONHBu-t | H | Ph | H |
| $CON(Me)_2$ | H | Ph | H |
| $CON(Et)_2$ | H | Ph | H |
| $CON(Pr-n)_2$ | H | Ph | H |
| F | H | $CH_2Ph$ | H |
| Cl | H | $CH_2Ph$ | H |
| Br | H | $CH_2Ph$ | H |
| I | H | $CH_2Ph$ | H |
| $CH=CH_2$ | H | $CH_2Ph$ | H |
| $CH_2CH=CH_2$ | H | $CH_2Ph$ | H |
| $C\equiv CH$ | H | $CH_2Ph$ | H |
| $CH_2C\equiv CH$ | H | $CH_2Ph$ | H |
| $CH_2F$ | H | $CH_2Ph$ | H |
| $CHF_2$ | H | $CH_2Ph$ | H |
| CN | H | $CH_2Ph$ | H |
| $NH_2$ | H | $CH_2Ph$ | H |
| NHCOMe | H | $CH_2Ph$ | H |
| $CH_2NH_2$ | H | $CH_2Ph$ | H |
| $CH_2NHCOMe$ | H | $CH_2Ph$ | H |
| $CH_2OCHF_2$ | H | $CH_2Ph$ | H |
| $CH_2OCF_3$ | H | $CH_2Ph$ | H |
| $CF_3$ | H | $CH_2Ph$ | H |
| $NO_2$ | H | $CH_2Ph$ | H |
| OMe | H | $CH_2Ph$ | H |
| OEt | H | $CH_2Ph$ | H |
| OPr-n | H | $CH_2Ph$ | H |
| OBu-n | H | $CH_2Ph$ | H |
| OPen-n | H | $CH_2Ph$ | H |
| SMe | H | $CH_2Ph$ | H |
| SEt | H | $CH_2Ph$ | H |
| SPr-n | H | $CH_2Ph$ | H |
| SBu-n | H | $CH_2Ph$ | H |

| $R_1$ | $R_2$ | $R_3$ | $R_4$ |
|---|---|---|---|
| SPen-n | H | $CH_2Ph$ | H |
| $SO_2Me$ | H | $CH_2Ph$ | H |
| $SO_2Et$ | H | $CH_2Ph$ | H |
| $SO_2Pr$-n | H | $CH_2Ph$ | H |
| COMe | H | $CH_2Ph$ | H |
| COEt | H | $CH_2Ph$ | H |
| COPr-n | H | $CH_2Ph$ | H |
| $CO_2H$ | H | $CH_2Ph$ | H |
| $CO_2Me$ | H | $CH_2Ph$ | H |
| $CO_2Et$ | H | $CH_2Ph$ | H |
| $CO_2Pr$-- | H | $CH_2Ph$ | H |
| $CO_2Bu$-n | H | $CH_2Ph$ | H |
| $CO_2Pen$-n | H | $CH_2Ph$ | H |
| $CH_2Cl$ | H | $CH_2Ph$ | H |
| $CH_2Br$ | H | $CH_2Ph$ | H |
| $CH_2I$ | H | $CH_2Ph$ | H |
| $CH_2CF_3$ | H | $CH_2Ph$ | H |
| $CH_2CN$ | H | $CH_2Ph$ | H |
| $CH_2OH$ | H | $CH_2Ph$ | H |
| $CH_2OMe$ | H | $CH_2Ph$ | H |
| $CH_2OEt$ | H | $CH_2Ph$ | H |
| $CH_2OPr$-n | H | $CH_2Ph$ | H |
| $CH_2SMe$ | H | $CH_2Ph$ | H |
| $CH_2SEt$ | H | $CH_2Ph$ | H |
| $CH_2SPr$-n | H | $CH_2Ph$ | H |
| $CH_2SO_2Me$ | H | $CH_2Ph$ | H |
| $CH_2SO_2Et$ | H | $CH_2Ph$ | H |
| $CH_2SO_2Pr$-n | H | $CH_2Ph$ | H |
| $CH_2COMe$ | H | $CH_2Ph$ | H |
| $CH_2COEt$ | H | $CH_2Ph$ | H |
| $CH_2COPr$-n | H | $CH_2Ph$ | H |
| $CH_2CO_2H$ | H | $CH_2Ph$ | H |
| $CH_2CO_2Me$ | H | $CH_2Ph$ | H |
| $CH_2CO_2Et$ | H | $CH_2Ph$ | H |
| $CH_2CO_2Pr$-n | H | $CH_2Ph$ | H |
| $CH_2OCH_2CH=CH_2$ | H | $CH_2Ph$ | H |
| $CH_2OCH_2C\equiv CH$ | H | $CH_2Ph$ | H |
| $CH=CHCO_2Me$ | H | $CH_2Ph$ | H |
| $CH=CHCO_2Et$ | H | $CH_2Ph$ | H |
| $CH=CHCO_2Pr$-n | H | $CH_2Ph$ | H |

| $R_1$ | $R_2$ | $R_3$ | $R_4$ |
|---|---|---|---|
| CH=CHCN | H | $CH_2Ph$ | H |
| Ph | H | $CH_2Ph$ | H |
| $CH_2Ph$ | H | $CH_2Ph$ | H |
| OPh | H | $CH_2Ph$ | H |
| SPh | H | $CH_2Ph$ | H |
| $SO_2Ph$ | H | $CH_2Ph$ | H |
| CONHMe | H | $CH_2Ph$ | H |
| CONHEt | H | $CH_2Ph$ | H |
| CONHPr-n | H | $CH_2Ph$ | H |
| CONHPr-i | H | $CH_2Ph$ | H |
| CONHBu-n | H | $CH_2Ph$ | H |
| CONHBu-sec | H | $CH_2Ph$ | H |
| CONHBu-t | H | $CH_2Ph$ | H |
| $CON(Me)_2$ | H | $CH_2Ph$ | H |
| $CON(Et)_2$ | H | $CH_2Ph$ | H |
| $CON(Pr\text{-}n)_2$ | H | $CH_2Ph$ | H |
| F | H | OPh | H |
| Cl | H | OPh | H |
| Br | H | OPh | H |
| I | H | OPh | H |
| $CH=CH_2$ | H | OPh | H |
| $CH_2CH=CH_2$ | H | OPh | H |
| $C\equiv CH$ | H | OPh | H |
| $CH_2C\equiv CH$ | H | OPh | H |
| $CH_2F$ | H | OPh | H |
| $CHF_2$ | H | OPh | H |
| CN | H | OPh | H |
| $NH_2$ | H | OPh | H |
| NHCOMe | H | OPh | H |
| $CH_2NH_2$ | H | OPh | H |
| $CH_2NHCOMe$ | H | OPh | H |
| $CH_2OCHF_2$ | H | OPh | H |
| $CH_2OCF_3$ | H | OPh | H |
| $CF_3$ | H | OPh | H |
| $NO_2$ | H | OPh | H |
| OMe | H | OPh | H |
| OEt | H | OPh | H |
| OPr-n | H | OPh | H |
| OBu-n | H | OPh | H |
| OPen-n | H | OPh | H |

| $R_1$ | $R_2$ | $R_3$ | $R_4$ |
|---|---|---|---|
| SMe | H | OPh | H |
| SEt | H | OPh | H |
| SPr-n | H | OPh | H |
| SBu-n | H | OPh | H |
| SPen-n | H | OPh | H |
| $SO_2Me$ | H | OPh | H |
| $SO_2Et$ | H | OPh | H |
| $SO_2Pr$-n | H | OPh | H |
| COMe | H | OPh | H |
| COEt | H | OPh | H |
| COPr-n | H | OPh | H |
| $CO_2H$ | H | OPh | H |
| $CO_2Me$ | H | OPh | H |
| $CO_2Et$ | H | OPh | H |
| $CO_2Pr$-n | H | OPh | H |
| $CO_2Bu$-n | H | OPh | H |
| $CO_2Pen$-n | H | OPh | H |
| $CH_2Cl$ | H | OPh | H |
| $CH_2Br$ | H | OPh | H |
| $CH_2I$ | H | OPh | H |
| $CH_2CF_3$ | H | OPh | H |
| $CH_2CN$ | H | OPh | H |
| $CH_2OH$ | H | OPh | H |
| $CH_2OMe$ | H | OPh | H |
| $CH_2OEt$ | H | OPh | H |
| $CH_2OPr$-n | H | OPh | H |
| $CH_2SMe$ | H | OPh | H |
| $CH_2SEt$ | H | OPh | H |
| $CH_2SPr$-n | H | OPh | H |
| $CH_2SO_2Me$ | H | OPh | H |
| $CH_2SO_2Et$ | H | OPh | H |
| $CH_2SO_2Pr$-n | H | OPh | H |
| $CH_2COMe$ | H | OPh | H |
| $CH_2COEt$ | H | OPh | H |
| $CH_2COPr$-n | H | OPh | H |
| $CH_2CO_2H$ | H | OPh | H |
| $CH_2CO_2Me$ | H | OPh | H |
| $CH_2CO_2Et$ | H | OPh | H |
| $CH_2CO_2Pr$-n | H | OPh | H |
| $CH_2OCH_2CH=CH_2$ | H | OPh | H |

| $R_1$ | $R_2$ | $R_3$ | $R_4$ |
|---|---|---|---|
| $CH_2OCH_2C\equiv CH$ | H | OPh | H |
| $CH=CHCO_2Me$ | H | OPh | H |
| $CH=CHCO_2Et$ | H | OPh | H |
| $CH=CHCO_2Pr$-n | H | OPh | H |
| CH=CHCN | H | OPh | H |
| Ph | H | OPh | H |
| $CH_2Ph$ | H | OPh | H |
| OPh | H | OPh | H |
| SPh | H | OPh | H |
| $SO_2Ph$ | H | OPh | H |
| CONHMe | H | OPh | H |
| CONHEt | H | OPh | H |
| CONHPr-n | H | OPh | H |
| CONHPr-i | H | OPh | H |
| CONHBu-n | H | OPh | H |
| CONHBu-sec | H | OPh | H |
| CONHBu-t | H | OPh | H |
| $CON(Me)_2$ | H | OPh | H |
| $CON(Et)_2$ | H | OPh | H |
| $CON(Pr\text{-}n)_2$ | H | OPh | H |
| F | H | SPh | H |
| Cl | H | SPh | H |
| Br | H | SPh | H |
| I | H | SPh | H |
| $CH=CH_2$ | H | SPh | H |
| $CH_2CH=CH_2$ | H | SPh | H |
| $C\equiv CH$ | H | SPh | H |
| $CH_2C\equiv CH$ | H | SPh | H |
| $CH_2F$ | H | SPh | H |
| $CHF_2$ | H | SPh | H |
| CN | H | SPh | H |
| $NH_2$ | H | SPh | H |
| NHCOMe | H | SPh | H |
| $CH_2NH_2$ | H | SPh | H |
| $CH_2NHCOMe$ | H | SPh | H |
| $CH_2OCHF_2$ | H | SPh | H |
| $CH_2OCF_3$ | H | SPh | H |
| $CF_3$ | H | SPh | H |
| $NO_2$ | H | SPh | H |
| OMe | H | SPh | H |

| $R_1$ | $R_2$ | $R_3$ | $R_4$ |
|---|---|---|---|
| OEt | H | SPh | H |
| OPr-n | H | SPh | H |
| OBu-n | H | SPh | H |
| OPen-n | H | SPh | H |
| SMe | H | SPh | H |
| SEt | H | SPh | H |
| SPr-n | H | SPh | H |
| SBu-n | H | SPh | H |
| SPen-n | H | SPh | H |
| $SO_2Me$ | H | SPh | H |
| $SO_2Et$ | H | SPh | H |
| $SO_2Pr$-n | H | SPh | H |
| COMe | H | SPh | H |
| COEt | H | SPh | H |
| COPr-n | H | SPh | H |
| $CO_2H$ | H | SPh | H |
| $CO_2Me$ | H | SPh | H |
| $CO_2Et$ | H | SPh | H |
| $CO_2Pr$-n | H | SPh | H |
| $CO_2Bu$-n | H | SPh | H |
| $CO_2Pen$-n | H | SPh | H |
| $CH_2Cl$ | H | SPh | H |
| $CH_2Br$ | H | SPh | H |
| $CH_2I$ | H | SPh | H |
| $CH_2CF_3$ | H | SPh | H |
| $CH_2CN$ | H | SPh | H |
| $CH_2OH$ | H | SPh | H |
| $CH_2OMe$ | H | SPh | H |
| $CH_2OEt$ | H | SPh | H |
| $CH_2OPr$-n | H | SPh | H |
| $CH_2SMe$ | H | SPh | H |
| $CH_2SEt$ | H | SPh | H |
| $CH_2SPr$-n | H | SPh | H |
| $CH_2SO_2Me$ | H | SPh | H |
| $CH_2SO_2Et$ | H | SPh | H |
| $CH_2SO_2Pr$-n | H | SPh | H |
| $CH_2COMe$ | H | SPh | H |
| $CH_2COEt$ | H | SPh | H |
| $CH_2COPr$-n | H | SPh | H |
| $CH_2CO_2H$ | H | SPh | H |

| $R_1$ | $R_2$ | $R_3$ | $R_4$ |
|---|---|---|---|
| $CH_2CO_2Me$ | H | SPh | H |
| $CH_2CO_2Et$ | H | SPh | H |
| $CH_2CO_2Pr$-n | H | SPh | H |
| $CH_2OCH_2CH=CH_2$ | H | SPh | H |
| $CH_2OCH_2C\equiv CH$ | H | SPh | H |
| $CH=CHCO_2Me$ | H | SPh | H |
| $CH=CHCO_2Et$ | H | SPh | H |
| $CH=CHCO_2Pr$-n | H | SPh | H |
| CH=CHCN | H | SPh | H |
| Ph | H | SPh | H |
| $CH_2Ph$ | H | SPh | H |
| OPh | H | SPh | H |
| SPh | H | SPh | H |
| $SO_2Ph$ | H | SPh | H |
| CONHMe | H | SPh | H |
| CONHEt | H | SPh | H |
| CONHPr-n | H | SPh | H |
| CONHPr-i | H | SPh | H |
| CONHBu-n | H | SPh | H |
| CONHBu-sec | H | SPh | H |
| CONHBu-t | H | SPh | H |
| $CON(Me)_2$ | H | SPh | H |
| $CON(Et)_2$ | H | SPh | H |
| $CON(Pr\text{-}n)_2$ | H | SPh | H |
| F | H | $SO_2Ph$ | H |
| Cl | H | $SO_2Ph$ | H |
| Br | H | $SO_2Ph$ | H |
| I | H | $SO_2Ph$ | H |
| $CH=CH_2$ | H | $SO_2Ph$ | H |
| $CH_2CH=CH_2$ | H | $SO_2Ph$ | H |
| $C\equiv CH$ | H | $SO_2Ph$ | H |
| $CH_2C\equiv CH$ | H | $SO_2Ph$ | H |
| $CH_2F$ | H | $SO_2Ph$ | H |
| $CHF_2$ | H | $SO_2Ph$ | H |
| CN | H | $SO_2Ph$ | H |
| $NH_2$ | H | $SO_2Ph$ | H |
| NHCOMe | H | $SO_2Ph$ | H |
| $CH_2NH_2$ | H | $SO_2Ph$ | H |
| $CH_2NHCOMe$ | H | $SO_2Ph$ | H |
| $CH_2OCHF_2$ | H | $SO_2Ph$ | H |

| $R_1$ | $R_2$ | $R_3$ | $R_4$ |
|---|---|---|---|
| $CH_2OCF_3$ | H | $SO_2Ph$ | H |
| $CF_3$ | H | $SO_2Ph$ | H |
| $NO_2$ | H | $SO_2Ph$ | H |
| OMe | H | $SO_2Ph$ | H |
| OEt | H | $SO_2Ph$ | H |
| OPr-n | H | $SO_2Ph$ | H |
| OBu-n | H | $SO_2Ph$ | H |
| OPen-n | H | $SO_2Ph$ | H |
| SMe | H | $SO_2Ph$ | H |
| SEt | H | $SO_2Ph$ | H |
| SPr-n | H | $SO_2Ph$ | H |
| SBu-n | H | $SO_2Ph$ | H |
| SPen-n | H | $SO_2Ph$ | H |
| $SO_2Me$ | H | $SO_2Ph$ | H |
| $SO_2Et$ | H | $SO_2Ph$ | H |
| $SO_2Pr$-n | H | $SO_2Ph$ | H |
| COMe | H | $SO_2Ph$ | H |
| COEt | H | $SO_2Ph$ | H |
| COPr-n | H | $SO_2Ph$ | H |
| $CO_2H$ | H | $SO_2Ph$ | H |
| $CO_2Me$ | H | $SO_2Ph$ | H |
| $CO_2Et$ | H | $SO_2Ph$ | H |
| $CO_2Pr$-n | H | $SO_2Ph$ | H |
| $CO_2Bu$-n | H | $SO_2Ph$ | H |
| $CO_2Pen$-n | H | $SO_2Ph$ | H |
| $CH_2Cl$ | H | $SO_2Ph$ | H |
| $CH_2Br$ | H | $SO_2Ph$ | H |
| $CH_2I$ | H | $SO_2Ph$ | H |
| $CH_2CF_3$ | H | $SO_2Ph$ | H |
| $CH_2CN$ | H | $SO_2Ph$ | H |
| $CH_2OH$ | H | $SO_2Ph$ | H |
| $CH_2OMe$ | H | $SO_2Ph$ | H |
| $CH_2OEt$ | H | $SO_2Ph$ | H |
| $CH_2OPr$-n | H | $SO_2Ph$ | H |
| $CH_2SMe$ | H | $SO_2Ph$ | H |
| $CH_2SEt$ | H | $SO_2Ph$ | H |
| $CH_2SPr$-n | H | $SO_2Ph$ | H |
| $CH_2SO_2Me$ | H | $SO_2Ph$ | H |
| $CH_2SO_2Et$ | H | $SO_2Ph$ | H |
| $CH_2SO_2Pr$-n | H | $SO_2Ph$ | H |

| $R_1$ | $R_2$ | $R_3$ | $R_4$ |
|---|---|---|---|
| $CH_2COMe$ | H | $SO_2Ph$ | H |
| $CH_2COEt$ | H | $SO_2Ph$ | H |
| $CH_2COPr$-n | H | $SO_2Ph$ | H |
| $CH_2CO_2H$ | H | $SO_2Ph$ | H |
| $CH_2CO_2Me$ | H | $SO_2Ph$ | H |
| $CH_2CO_2Et$ | H | $SO_2Ph$ | H |
| $CH_2CO_2Pr$-n | H | $SO_2Ph$ | H |
| $CH_2OCH_2CH=CH_2$ | H | $SO_2Ph$ | H |
| $CH_2OCH_2C\equiv CH$ | H | $SO_2Ph$ | H |
| $CH=CHCO_2Me$ | H | $SO_2Ph$ | H |
| $CH=CHCO_2Et$ | H | $SO_2Ph$ | H |
| $CH=CHCO_2Pr$-n | H | $SO_2Ph$ | H |
| $CH=CHCN$ | H | $SO_2Ph$ | H |
| Ph | H | $SO_2Ph$ | H |
| $CH_2Ph$ | H | $SO_2Ph$ | H |
| OPh | H | $SO_2Ph$ | H |
| SPh | H | $SO_2Ph$ | H |
| $SO_2Ph$ | H | $SO_2Ph$ | H |
| CONHMe | H | $SO_2Ph$ | H |
| CONHEt | H | $SO_2Ph$ | H |
| CONHPr-n | H | $SO_2Ph$ | H |
| CONHPr-i | H | $SO_2Ph$ | H |
| CONHBu-n | H | $SO_2Ph$ | H |
| CONHBu-sec | H | $SO_2Ph$ | H |
| CONHBu-t | H | $SO_2Ph$ | H |
| $CON(Me)_2$ | H | $SO_2Ph$ | H |
| $CON(Et)_2$ | H | $SO_2Ph$ | H |
| $CON(Pr\text{-}n)_2$ | H | $SO_2Ph$ | H |
| F | H | $CON(Et)_2$ | H |
| Cl | H | $CON(Et)_2$ | H |
| Br | H | $CON(Et)_2$ | H |
| I | H | $CON(Et)_2$ | H |
| $CH=CH_2$ | H | $CON(Et)_2$ | H |
| $CH_2CH=CH_2$ | H | $CON(Et)_2$ | H |
| $C\equiv CH$ | H | $CON(Et)_2$ | H |
| $CH_2C\equiv CH$ | H | $CON(Et)_2$ | H |
| $CH_2F$ | H | $CON(Et)_2$ | H |
| $CHF_2$ | H | $CON(Et)_2$ | H |
| CN | H | $CON(Et)_2$ | H |
| $NH_2$ | H | $CON(Et)_2$ | H |

| $R_1$ | $R_2$ | $R_3$ | $R_4$ |
|---|---|---|---|
| NHCOMe | H | CON(Et)$_2$ | H |
| $CH_2NH_2$ | H | CON(Et)$_2$ | H |
| $CH_2$NHCOMe | H | CON(Et)$_2$ | H |
| $CH_2OCHF_2$ | H | CON(Et)$_2$ | H |
| $CH_2OCF_3$ | H | CON(Et)$_2$ | H |
| $CF_3$ | H | CON(Et)$_2$ | H |
| $NO_2$ | H | CON(Et)$_2$ | H |
| OMe | H | CON(Et)$_2$ | H |
| OEt | H | CON(Et)$_2$ | H |
| OPr-n | H | CON(Et)$_2$ | H |
| OBu-n | H | CON(Et)$_2$ | H |
| OPen-n | H | CON(Et)$_2$ | H |
| SMe | H | CON(Et)$_2$ | H |
| SEt | H | CON(Et)$_2$ | H |
| SPr-n | H | CON(Et)$_2$ | H |
| SBu-n | H | CON(Et)$_2$ | H |
| SPen-n | H | CON(Et)$_2$ | H |
| $SO_2$Me | H | CON(Et)$_2$ | H |
| $SO_2$Et | H | CON(Et)$_2$ | H |
| $SO_2$Pr-n | H | CON(Et)$_2$ | H |
| COMe | H | CON(Et)$_2$ | H |
| COEt | H | CON(Et)$_2$ | H |
| COPr-n | H | CON(Et)$_2$ | H |
| $CO_2H$ | H | CON(Et)$_2$ | H |
| $CO_2$Me | H | CON(Et)$_2$ | H |
| $CO_2$Et | H | CON(Et)$_2$ | H |
| $CO_2$Pr-n | H | CON(Et)$_2$ | H |
| $CO_2$Bu-n | H | CON(Et)$_2$ | H |
| $CO_2$Pen-n | H | CON(Et)$_2$ | H |
| $CH_2Cl$ | H | CON(Et)$_2$ | H |
| $CH_2Br$ | H | CON(Et)$_2$ | H |
| $CH_2I$ | H | CON(Et)$_2$ | H |
| $CH_2CF_3$ | H | CON(Et)$_2$ | H |
| $CH_2CN$ | H | CON(Et)$_2$ | H |
| $CH_2OH$ | H | CON(Et)$_2$ | H |
| $CH_2$OMe | H | CON(Et)$_2$ | H |
| $CH_2$OEt | H | CON(Et)$_2$ | H |
| $CH_2$OPr-n | H | CON(Et)$_2$ | H |
| $CH_2$SMe | H | CON(Et)$_2$ | H |
| $CH_2$SEt | H | CON(Et)$_2$ | H |

| $R_1$ | $R_2$ | $R_3$ | $R_4$ |
|---|---|---|---|
| $CH_2SPr$-n | H | $CON(Et)_2$ | H |
| $CH_2SO_2Me$ | H | $CON(Et)_2$ | H |
| $CH_2SO_2Et$ | H | $CON(Et)_2$ | H |
| $CH_2SO_2Pr$-n | H | $CON(Et)_2$ | H |
| $CH_2COMe$ | H | $CON(Et)_2$ | H |
| $CH_2COEt$ | H | $CON(Et)_2$ | H |
| $CH_2COPr$-n | H | $CON(Et)_2$ | H |
| $CH_2CO_2H$ | H | $CON(Et)_2$ | H |
| $CH_2CO_2Me$ | H | $CON(Et)_2$ | H |
| $CH_2CO_2Et$ | H | $CON(Et)_2$ | H |
| $CH_2CO_2Pr$-n | H | $CON(Et)_2$ | H |
| $CH_2OCH_2CH{=}CH_2$ | H | $CON(Et)_2$ | H |
| $CH_2OCH_2C\equiv CH$ | H | $CON(Et)_2$ | H |
| $CH{=}CHCO_2Me$ | H | $CON(Et)_2$ | H |
| $CH{=}CHCO_2Et$ | H | $CON(Et)_2$ | H |
| $CH{=}CHCO_2Pr$-n | H | $CON(Et)_2$ | H |
| $CH{=}CHCN$ | H | $CON(Et)_2$ | H |
| Ph | H | $CON(Et)_2$ | H |
| $CH_2Ph$ | H | $CON(Et)_2$ | H |
| OPh | H | $CON(Et)_2$ | H |
| SPh | H | $CON(Et)_2$ | H |
| $SO_2Ph$ | H | $CON(Et)_2$ | H |
| CONHMe | H | $CON(Et)_2$ | H |
| CONHEt | H | $CON(Et)_2$ | H |
| CONHPr-n | H | $CON(Et)_2$ | H |
| CONHPr-i | H | $CON(Et)_2$ | H |
| CONHBu-n | H | $CON(Et)_2$ | H |
| CONHBu-sec | H | $CON(Et)_2$ | H |
| CONHBu-t | H | $CON(Et)_2$ | H |
| $CON(Me)_2$ | H | $CON(Et)_2$ | H |
| $CON(Et)_2$ | H | $CON(Et)_2$ | H |
| $CON(Pr$-n$)_2$ | H | $CON(Et)_2$ | H |
| F | H | CN | H |
| Cl | H | CN | H |
| Br | H | CN | H |
| I | H | CN | H |
| $CH{=}CH_2$ | H | CN | H |
| $CH_2CH{=}CH_2$ | H | CN | H |
| $C\equiv CH$ | H | CN | H |
| $CH_2C\equiv CH$ | H | CN | H |

| $R_1$ | $R_2$ | $R_3$ | $R_4$ |
|---|---|---|---|
| $CH_2F$ | H | CN | H |
| $CHF_2$ | H | CN | H |
| CN | H | CN | H |
| $NH_2$ | H | CN | H |
| NHCOMe | H | CN | H |
| $CH_2NH_2$ | H | CN | H |
| $CH_2NHCOMe$ | H | CN | H |
| $CH_2OCHF_2$ | H | CN | H |
| $CH_2OCF_3$ | H | CN | H |
| $CF_3$ | H | CN | H |
| $NO_2$ | H | CN | H |
| OMe | H | CN | H |
| OEt | H | CN | H |
| OPr-n | H | CN | H |
| OBu-n | H | CN | H |
| OPen-n | H | CN | H |
| SMe | H | CN | H |
| SEt | H | CN | H |
| SPr-n | H | CN | H |
| SBu-n | H | CN | H |
| SPen-n | H | CN | H |
| $SO_2Me$ | H | CN | H |
| $SO_2Et$ | H | CN | H |
| $SO_2Pr$-n | H | CN | H |
| COMe | H | CN | H |
| COEt | H | CN | H |
| COPr-n | H | CN | H |
| $CO_2H$ | H | CN | H |
| $CO_2Me$ | H | CN | H |
| $CO_2Et$ | H | CN | H |
| $CO_2Pr$-n | H | CN | H |
| $CO_2Bu$-n | H | CN | H |
| $CO_2Pen$-n | H | CN | H |
| $CH_2Cl$ | H | CN | H |
| $CH_2Br$ | H | CN | H |
| $CH_2I$ | H | CN | H |
| $CH_2CF_3$ | H | CN | H |
| $CH_2CN$ | H | CN | H |
| $CH_2OH$ | H | CN | H |
| $CH_2OMe$ | H | CN | H |

| $R_1$ | $R_2$ | $R_3$ | $R_4$ |
|---|---|---|---|
| $CH_2OEt$ | H | CN | H |
| $CH_2OPr$-n | H | CN | H |
| $CH_2SMe$ | H | CN | H |
| $CH_2SEt$ | H | CN | H |
| $CH_2SPr$-n | H | CN | H |
| $CH_2SO_2Me$ | H | CN | H |
| $CH_2SO_2Et$ | H | CN | H |
| $CH_2SO_2Pr$-n | H | CN | H |
| $CH_2COMe$ | H | CN | H |
| $CH_2COEt$ | H | CN | H |
| $CH_2COPr$-n | H | CN | H |
| $CH_2CO_2H$ | H | CN | H |
| $CH_2CO_2Me$ | H | CN | H |
| $CH_2CO_2Et$ | H | CN | H |
| $CH_2CO_2Pr$-n | H | CN | H |
| $CH_2OCH_2CH{=}CH_2$ | H | CN | H |
| $CH_2OCH_2C\equiv CH$ | H | CN | H |
| $CH{=}CHCO_2Me$ | H | CN | H |
| $CH{=}CHCO_2Et$ | H | CN | H |
| $CH{=}CHCO_2Pr$-n | H | CN | H |
| CH=CHCN | H | CN | H |
| Ph | H | CN | H |
| $CH_2Ph$ | H | CN | H |
| OPh | H | CN | H |
| SPh | H | CN | H |
| $SO_2Ph$ | H | CN | H |
| CONHMe | H | CN | H |
| CONHEt | H | CN | H |
| CONHPr-n | H | CN | H |
| CONHPr-i | H | CN | H |
| CONHBu-n | H | CN | H |
| CONHBu-sec | H | CN | H |
| CONHBu-t | H | CN | H |
| $CON(Me)_2$ | H | CN | H |
| $CON(Et)_2$ | H | CN | H |
| $CON(Pr\text{-}n)_2$ | H | CN | H |

| $R_3$ | $R_2$ | $R_1$ | $R_4$ |
|---|---|---|---|
| F | H | Me | H |
| Cl | H | Me | H |
| Br | H | Me | H |
| I | H | Me | H |
| $CH=CH_2$ | H | Me | H |
| $CH_2CH=CH_2$ | H | Me | H |
| $C\equiv CH$ | H | Me | H |
| $CH_2C\equiv CH$ | H | Me | H |
| $CH_2F$ | H | Me | H |
| $CHF_2$ | H | Me | H |
| CN | H | Me | H |
| $NH_2$ | H | Me | H |
| NHCOMe | H | Me | H |
| $CH_2NH_2$ | H | Me | H |
| $CH_2NHCOMe$ | H | Me | H |
| $CH_2OCHF_2$ | H | Me | H |
| $CH_2OCF_3$ | H | Me | H |
| $CF_3$ | H | Me | H |
| $NO_2$ | H | Me | H |
| OMe | H | Me | H |
| OEt | H | Me | H |
| OPr-n | H | Me | H |
| OBu-n | H | Me | H |
| OPen-n | H | Me | H |
| SMe | H | Me | H |
| SEt | H | Me | H |
| SPr-n | H | Me | H |
| SBu-n | H | Me | H |
| SPen-n | H | Me | H |
| $SO_2Me$ | H | Me | H |
| $SO_2Et$ | H | Me | H |
| $SO_2Pr$-n | H | Me | H |
| COMe | H | Me | H |
| COEt | H | Me | H |
| COPr-n | H | Me | H |
| $CO_2H$ | H | Me | H |
| $CO_2Me$ | H | Me | H |
| $CO_2Et$ | H | Me | H |
| $CO_2Pr$-n | H | Me | H |
| $CO_2Bu$-n | H | Me | H |

| $R_3$ | $R_2$ | $R_1$ | $R_4$ |
|---|---|---|---|
| $CH_2CF_3$ | H | Me | H |
| $CO_2Pen$-n | H | Me | H |
| $CH_2Cl$ | H | Me | H |
| $CH_2Br$ | H | Me | H |
| $CH_2I$ | H | Me | H |
| $CH_2CN$ | H | Me | H |
| $CH_2OH$ | H | Me | H |
| $CH_2OMe$ | H | Me | H |
| $CH_2OEt$ | H | Me | H |
| $CH_2OPr$-n | H | Me | H |
| $CH_2SMe$ | H | Me | H |
| $CH_2SEt$ | H | Me | H |
| $CH_2SPr$-n | H | Me | H |
| $CH_2SO_2Me$ | H | Me | H |
| $CH_2SO_2Et$ | H | Me | H |
| $CH_2SO_2Pr$-n | H | Me | H |
| $CH_2COMe$ | H | Me | H |
| $CH_2COEt$ | H | Me | H |
| $CH_2COPr$-n | H | Me | H |
| $CH_2CO_2H$ | H | Me | H |
| $CH_2CO_2Me$ | H | Me | H |
| $CH_2CO_2Et$ | H | Me | H |
| $CH_2CO_2Pr$-n | H | Me | H |
| $CH_2OCH_2CH=CH_2$ | H | Me | H |
| $CH_2OCH_2C\equiv CH$ | H | Me | H |
| $CH=CHCO_2Me$ | H | Me | H |
| $CH=CHCO_2Et$ | H | Me | H |
| $CH=CHCO_2Pr$-n | H | Me | H |
| CH=CHCN | H | Me | H |
| Ph | H | Me | H |
| Ph-2-F | H | Me | H |
| Ph-3-F | H | Me | H |
| Ph-4-F | H | Me | H |
| Ph-2-Cl | H | Me | H |
| Ph-3-Cl | H | Me | H |
| Ph-4-Cl | H | Me | H |
| Ph-2-Br | H | Me | H |
| Ph-3-Br | H | Me | H |
| Ph-4-Br | H | Me | H |
| $Ph-2-CF_3$ | H | Me | H |

| $R_3$ | $R_2$ | $R_1$ | $R_4$ |
|---|---|---|---|
| Ph-4-Me | H | Me | H |
| Ph-3-$CF_3$ | H | Me | H |
| Ph-4-$CF_3$ | H | Me | H |
| Ph-2-Me | H | Me | H |
| Ph-3-Me | H | Me | H |
| Ph-2-Et | H | Me | H |
| Ph-2-OMe | H | Me | H |
| Ph-3-OMe | H | Me | H |
| Ph-4-OMe | H | Me | H |
| Ph-2-OEt | H | Me | H |
| Ph-2-$CO_2$Me | H | Me | H |
| Ph-2-$CO_2$Et | H | Me | H |
| Ph-3-$CO_2$Me | H | Me | H |
| Ph-4-$CO_2$Me | H | Me | H |
| $CH_2$Ph | H | Me | H |
| $CH_2$Ph-2-F | H | Me | H |
| $CH_2$Ph-3-F | H | Me | H |
| $CH_2$Ph-4-F | H | Me | H |
| $CH_2$Ph-2-Cl | H | Me | H |
| $CH_2$Ph-3-Cl | H | Me | H |
| $CH_2$Ph-4-Cl | H | Me | H |
| $CH_2$Ph-2-Br | H | Me | H |
| $CH_2$Ph-3-Br | H | Me | H |
| $CH_2$Ph-4-Br | H | Me | H |
| $CH_2$Ph-2-$CF_3$ | H | Me | H |
| $CH_2$Ph-3-$CF_3$ | H | Me | H |
| $CH_2$Ph-4-$CF_3$ | H | Me | H |
| $CH_2$Ph-2-Me | H | Me | H |
| $CH_2$Ph-3-Me | H | Me | H |
| $CH_2$Ph-4-Me | H | Me | H |
| $CH_2$Ph-2-Et | H | Me | H |
| $CH_2$Ph-2-OMe | H | Me | H |
| $CH_2$Ph-3-OMe | H | Me | H |
| $CH_2$Ph-4-OMe | H | Me | H |
| $CH_2$Ph-2-OEt | H | Me | H |
| $CH_2$Ph-2-$CO_2$Me | H | Me | H |
| $CH_2$Ph-2-$CO_2$Et | H | Me | H |
| $CH_2$Ph-3-$CO_2$Me | H | Me | H |
| $CH_2$Ph-4-$CO_2$Me | H | Me | H |
| OPh | H | Me | H |

| $R_3$ | $R_2$ | $R_1$ | $R_4$ |
|---|---|---|---|
| OPh-3-F | H | Me | H |
| OPh-2-Cl | H | Me | H |
| OPh-3-Cl | H | Me | H |
| OPh-4-Cl | H | Me | H |
| OPh-2-F | H | Me | H |
| OPh-4-F | H | Me | H |
| OPh-2-$CF_3$ | H | Me | H |
| OPh-3-$CF_3$ | H | Me | H |
| OPh-4-$CF_3$ | H | Me | H |
| OPh-2-Me | H | Me | H |
| OPh-3-Me | H | Me | H |
| OPh-4-Me | H | Me | H |
| OPh-2-Et | H | Me | H |
| OPh-2-OMe | H | Me | H |
| OPh-3-OMe | H | Me | H |
| OPh-4-OMe | H | Me | H |
| OPh-2-OEt | H | Me | H |
| OPh-2-$CO_2$Me | H | Me | H |
| OPh-3-$CO_2$Me | H | Me | H |
| OPh-4-$CO_2$Me | H | Me | H |
| OPh-2-$CO_2$Et | H | Me | H |
| SPh | H | Me | H |
| SPh-2-Cl | H | Me | H |
| SPh-3-Cl | H | Me | H |
| SPh-4-Cl | H | Me | H |
| SPh-2-F | H | Me | H |
| SPh-3-F | H | Me | H |
| SPh-4-F | H | Me | H |
| SPh-2-$CF_3$ | H | Me | H |
| SPh-3-$CF_3$ | H | Me | H |
| SPh-4-$CF_3$ | H | Me | H |
| SPh-2-Me | H | Me | H |
| SPh-3-Me | H | Me | H |
| SPh-4-Me | H | Me | H |
| SPh-2-Et | H | Me | H |
| SPh-2-OMe | H | Me | H |
| SPh-3-OMe | H | Me | H |
| SPh-4-OMe | H | Me | H |
| SPh-2-OEt | H | Me | H |
| SPh-2-$CO_2$Me | H | Me | H |

| $R_3$ | $R_2$ | $R_1$ | $R_4$ |
|---|---|---|---|
| $SO_2Ph$-2-Cl | H | Me | H |
| SPh-3-$CO_2Me$ | H | Me | H |
| SPh-4-$CO_2Me$ | H | Me | H |
| SPh-2-$CO_2Et$ | H | Me | H |
| $SO_2Ph$ | H | Me | H |
| $SO_2Ph$-3-Cl | H | Me | H |
| $SO_2Ph$-4-Cl | H | Me | H |
| $SO_2Ph$-2-F | H | Me | H |
| $SO_2Ph$-3-F | H | Me | H |
| $SO_2Ph$-4-F | H | Me | H |
| $SO_2Ph$-2-$CF_3$ | H | Me | H |
| $SO_2Ph$-3-$CF_3$ | H | Me | H |
| $SO_2Ph$-4-$CF_3$ | H | Me | H |
| $SO_2Ph$-2-Me | H | Me | H |
| $SO_2Ph$-3-Me | H | Me | H |
| $SO_2Ph$-4-Me | H | Me | H |
| $SO_2Ph$-2-Et | H | Me | H |
| $SO_2Ph$-2-OMe | H | Me | H |
| $SO_2Ph$-3-OMe | H | Me | H |
| $SO_2Ph$-4-OMe | H | Me | H |
| $SO_2Ph$-2-OEt | H | Me | H |
| $CH_2Ph$-2-$CO_2Me$ | H | Me | H |
| $CH_2Ph$-3-$CO_2Me$ | H | Me | H |
| $CH_2Ph$-4-$CO_2Me$ | H | Me | H |
| $CH_2Ph$-2-$CO_2Et$ | H | Me | H |
| CONHMe | H | Me | H |
| CONHEt | H | Me | H |
| CONHPr-n | H | Me | H |
| CONHPr-i | H | Me | H |
| CONHBu-n | H | Me | H |
| CONHBu-sec | H | Me | H |
| CONHBu-t | H | Me | H |
| $CON(Me)_2$ | H | Me | H |
| $CON(Et)_2$ | H | Me | H |
| $CON(Pr\text{-}n)_2$ | H | Me | H |

| $R_3$ | $R_2$ | $R_1$ | $R_4$ |
|---|---|---|---|
| F | H | Et | H |
| Cl | H | Et | H |
| Br | H | Et | H |
| I | H | Et | H |
| $CH=CH_2$ | H | Et | H |
| $CH_2CH=CH_2$ | H | Et | H |
| $C\equiv CH$ | H | Et | H |
| $CH_2C\equiv CH$ | H | Et | H |
| $CH_2F$ | H | Et | H |
| $CHF_2$ | H | Et | H |
| CN | H | Et | H |
| $NH_2$ | H | Et | H |
| NHCOMe | H | Et | H |
| $CH_2NH_2$ | H | Et | H |
| $CH_2NHCOMe$ | H | Et | H |
| $CH_2OCHF_2$ | H | Et | H |
| $CH_2OCF_3$ | H | Et | H |
| $CF_3$ | H | Et | H |
| $NO_2$ | H | Et | H |
| OMe | H | Et | H |
| OEt | H | Et | H |
| OPr-n | H | Et | H |
| OBu-n | H | Et | H |
| OPen-n | H | Et | H |
| SMe | H | Et | H |
| SEt | H | Et | H |
| SPr-n | H | Et | H |
| SBu-n | H | Et | H |
| SPen-n | H | Et | H |
| $SO_2Me$ | H | Et | H |
| $SO_2Et$ | H | Et | H |
| $SO_2Pr$-n | H | Et | H |
| COMe | H | Et | H |
| COEt | H | Et | H |
| COPr-n | H | Et | H |
| $CO_2H$ | H | Et | H |
| $CO_2Me$ | H | Et | H |
| $CO_2Et$ | H | Et | H |
| $CO_2Pr$-n | H | Et | H |
| $CO_2Bu$-n | H | Et | H |

| $R_3$ | $R_2$ | $R_1$ | $R_4$ |
|---|---|---|---|
| $CO_2$Pen-n | H | Et | H |
| $CH_2Cl$ | H | Et | H |
| $CH_2Br$ | H | Et | H |
| $CH_2I$ | H | Et | H |
| $CH_2CF_3$ | H | Et | H |
| $CH_2CN$ | H | Et | H |
| $CH_2OH$ | H | Et | H |
| $CH_2OMe$ | H | Et | H |
| $CH_2OEt$ | H | Et | H |
| $CH_2OPr$-n | H | Et | H |
| $CH_2SMe$ | H | Et | H |
| $CH_2SEt$ | H | Et | H |
| $CH_2SPr$-n | H | Et | H |
| $CH_2SO_2Me$ | H | Et | H |
| $CH_2SO_2Et$ | H | Et | H |
| $CH_2SO_2Pr$-n | H | Et | H |
| $CH_2COMe$ | H | Et | H |
| $CH_2COEt$ | H | Et | H |
| $CH_2COPr$-n | H | Et | H |
| $CH_2CO_2H$ | H | Et | H |
| $CH_2CO_2Me$ | H | Et | H |
| $CH_2CO_2Et$ | H | Et | H |
| $CH_2CO_2Pr$-n | H | Et | H |
| $CH_2OCH_2CH=CH_2$ | H | Et | H |
| $CH_2OCH_2C\equiv CH$ | H | Et | H |
| $CH=CHCO_2Me$ | H | Et | H |
| $CH=CHCO_2Et$ | H | Et | H |
| $CH=CHCO_2Pr$-n | H | Et | H |
| CH=CHCN | H | Et | H |
| Ph | H | Et | H |
| $CH_2Ph$ | H | Et | H |
| OPh | H | Et | H |
| SPh | H | Et | H |
| $SO_2Ph$ | H | Et | H |
| CONHMe | H | Et | H |
| CONHEt | H | Et | H |
| CONHPr-n | H | Et | H |
| CONHPr-i | H | Et | H |
| CONHBu-n | H | Et | H |
| CONHBu-sec | H | Et | H |

| $R_3$ | $R_2$ | $R_1$ | $R_4$ |
|---|---|---|---|
| CONHBu-t | H | Et | H |
| $CON(Me)_2$ | H | Et | H |
| $CON(Et)_2$ | H | Et | H |
| $CON(Pr-n)_2$ | H | Et | H |
| F | Me | H | H |
| Cl | Me | H | H |
| Br | Me | H | H |
| I | Me | H | H |
| $CH=CH_2$ | Me | H | H |
| $CH_2CH=CH_2$ | Me | H | H |
| $C\equiv CH$ | Me | H | H |
| $CH_2C\equiv CH$ | Me | H | H |
| $CH_2F$ | Me | H | H |
| $CHF_2$ | Me | H | H |
| CN | Me | H | H |
| $NH_2$ | Me | H | H |
| NHCOMe | Me | H | H |
| $CH_2NH_2$ | Me | H | H |
| $CH_2NHCOMe$ | Me | H | H |
| $CH_2OCHF_2$ | Me | H | H |
| $CH_2OCF_3$ | Me | H | H |
| $CF_3$ | Me | H | H |
| $NO_2$ | Me | H | H |
| OMe | Me | H | H |
| OEt | Me | H | H |
| OPr-n | Me | H | H |
| OBu-n | Me | H | H |
| OPen-n | Me | H | H |
| SMe | Me | H | H |
| SEt | Me | H | H |
| SPr-n | Me | H | H |
| SBu-n | Me | H | H |
| SPen-n | Me | H | H |
| $SO_2Me$ | Me | H | H |
| $SO_2Et$ | Me | H | H |
| $SO_2Pr-n$ | Me | H | H |
| COMe | Me | H | H |
| COEt | Me | H | H |
| COPr-n | Me | H | H |
| $CO_2H$ | Me | H | H |

| $R_3$ | $R_2$ | $R_1$ | $R_4$ |
|---|---|---|---|
| $CO_2Me$ | Me | H | H |
| $CO_2Et$ | Me | H | H |
| $CO_2Pr$-n | Me | H | H |
| $CO_2Bu$-n | Me | H | H |
| $CO_2Pen$-n | Me | H | H |
| $CH_2Cl$ | Me | H | H |
| $CH_2Br$ | Me | H | H |
| $CH_2I$ | Me | H | H |
| $CH_2CF_3$ | Me | H | H |
| $CH_2CN$ | Me | H | H |
| $CH_2OH$ | Me | H | H |
| $CH_2OMe$ | Me | H | H |
| $CH_2OEt$ | Me | H | H |
| $CH_2OPr$-n | Me | H | H |
| $CH_2SMe$ | Me | H | H |
| $CH_2SEt$ | Me | H | H |
| $CH_2SPr$-n | Me | H | H |
| $CH_2SO_2Me$ | Me | H | H |
| $CH_2SO_2Et$ | Me | H | H |
| $CH_2SO_2Pr$-n | Me | H | H |
| $CH_2COMe$ | Me | H | H |
| $CH_2COEt$ | Me | H | H |
| $CH_2COPr$-n | Me | H | H |
| $CH_2CO_2H$ | Me | H | H |
| $CH_2CO_2Me$ | Me | H | H |
| $CH_2CO_2Et$ | Me | H | H |
| $CH_2CO_2Pr$-n | Me | H | H |
| $CH_2OCH_2CH=CH_2$ | Me | H | H |
| $CH_2OCH_2C\equiv CH$ | Me | H | H |
| $CH=CHCO_2Me$ | Me | H | H |
| $CH=CHCO_2Et$ | Me | H | H |
| $CH=CHCO_2Pr$-n | Me | H | H |
| $CH=CHCN$ | Me | H | H |
| Ph | Me | H | H |
| Ph-2-F | Me | H | H |
| Ph-3-F | Me | H | H |
| Ph-4-F | Me | H | H |
| Ph-2-Cl | Me | H | H |
| Ph-3-Cl | Me | H | H |
| Ph-4-Cl | Me | H | H |

| $R_3$ | $R_2$ | $R_1$ | $R_4$ |
|---|---|---|---|
| Ph-2-Br | Me | H | H |
| Ph-3-Br | Me | H | H |
| Ph-4-Br | Me | H | H |
| Ph-2-$CF_3$ | Me | H | H |
| Ph-3-$CF_3$ | Me | H | H |
| Ph-4-$CF_3$ | Me | H | H |
| Ph-2-Me | Me | H | H |
| Ph-3-Me | Me | H | H |
| Ph-4-Me | Me | H | H |
| Ph-2-Et | Me | H | H |
| Ph-2-OMe | Me | H | H |
| Ph-3-OMe | Me | H | H |
| Ph-4-OMe | Me | H | H |
| Ph-2-OEt | Me | H | H |
| Ph-2-$CO_2$Me | Me | H | H |
| Ph-2-$CO_2$Et | Me | H | H |
| Ph-3-$CO_2$Me | Me | H | H |
| Ph-4-$CO_2$Me | Me | H | H |
| $CH_2$Ph | Me | H | H |
| $CH_2$Ph-2-F | Me | H | H |
| $CH_2$Ph-3-F | Me | H | H |
| $CH_2$Ph-4-F | Me | H | H |
| $CH_2$Ph-2-Cl | Me | H | H |
| $CH_2$Ph-3-Cl | Me | H | H |
| $CH_2$Ph-4-Cl | Me | H | H |
| $CH_2$Ph-2-Br | Me | H | H |
| $CH_2$Ph-3-Br | Me | H | H |
| $CH_2$Ph-4-Br | Me | H | H |
| $CH_2$Ph-2-$CF_3$ | Me | H | H |
| $CH_2$Ph-3-$CF_3$ | Me | H | H |
| $CH_2$Ph-4-$CF_3$ | Me | H | H |
| $CH_2$Ph-2-Me | Me | H | H |
| $CH_2$Ph-3-Me | Me | H | H |
| $CH_2$Ph-4-Me | Me | H | H |
| $CH_2$Ph-2-Et | Me | H | H |
| $CH_2$Ph-2-OMe | Me | H | H |
| $CH_2$Ph-3-OMe | Me | H | H |
| $CH_2$Ph-4-OMe | Me | H | H |
| $CH_2$Ph-2-OEt | Me | H | H |
| $CH_2$Ph-2-$CO_2$Me | Me | H | H |

| $R_3$ | $R_2$ | $R_1$ | $R_4$ |
|---|---|---|---|
| $CH_2Ph$-2-$CO_2Et$ | Me | H | H |
| $CH_2Ph$-3-$CO_2Me$ | Me | H | H |
| $CH_2Ph$-4-$CO_2Me$ | Me | H | H |
| OPh | Me | H | H |
| OPh-2-Cl | Me | H | H |
| OPh-3-Cl | Me | H | H |
| OPh-4-Cl | Me | H | H |
| OPh-2-F | Me | H | H |
| OPh-3-F | Me | H | H |
| OPh-4-F | Me | H | H |
| OPh-2-$CF_3$ | Me | H | H |
| OPh-3-$CF_3$ | Me | H | H |
| OPh-4-$CF_3$ | Me | H | H |
| OPh-2-Me | Me | H | H |
| OPh-3-Me | Me | H | H |
| OPh-4-Me | Me | H | H |
| OPh-2-Et | Me | H | H |
| OPh-2-OMe | Me | H | H |
| OPh-3-OMe | Me | H | H |
| OPh-4-OMe | Me | H | H |
| OPh-2-OEt | Me | H | H |
| OPh-2-$CO_2Me$ | Me | H | H |
| OPh-3-$CO_2Me$ | Me | H | H |
| OPh-4-$CO_2Me$ | Me | H | H |
| OPh-2-$CO_2Et$ | Me | H | H |
| SPh | Me | H | H |
| SPh-2-Cl | Me | H | H |
| SPh-3-Cl | Me | H | H |
| SPh-4-Cl | Me | H | H |
| SPh-2-F | Me | H | H |
| SPh-3-F | Me | H | H |
| SPh-4-F | Me | H | H |
| SPh-2-$CF_3$ | Me | H | H |
| SPh-3-$CF_3$ | Me | H | H |
| SPh-4-$CF_3$ | Me | H | H |
| SPh-2-Me | Me | H | H |
| SPh-3-Me | Me | H | H |
| SPh-4-Me | Me | H | H |
| SPh-2-Et | Me | H | H |
| SPh-2-OMe | Me | H | H |

| $R_3$ | $R_2$ | $R_1$ | $R_4$ |
|---|---|---|---|
| SPh-3-OMe | Me | H | H |
| SPh-4-OMe | Me | H | H |
| SPh-2-OEt | Me | H | H |
| SPh-2-$CO_2$Me | Me | H | H |
| SPh-3-$CO_2$Me | Me | H | H |
| SPh-4-$CO_2$Me | Me | H | H |
| SPh-2-$CO_2$Et | Me | H | H |
| $SO_2$Ph | Me | H | H |
| $SO_2$Ph-2-Cl | Me | H | H |
| $SO_2$Ph-3-Cl | Me | H | H |
| $SO_2$Ph-4-Cl | Me | H | H |
| $SO_2$Ph-2-F | Me | H | H |
| $SO_2$Ph-3-F | Me | H | H |
| $SO_2$Ph-4-F | Me | H | H |
| $SO_2$Ph-2-$CF_3$ | Me | H | H |
| $SO_2$Ph-3-$CF_3$ | Me | H | H |
| $SO_2$Ph-4-$CF_3$ | Me | H | H |
| $SO_2$Ph-2-Me | Me | H | H |
| $SO_2$Ph-3-Me | Me | H | H |
| $SO_2$Ph-4-Me | Me | H | H |
| $SO_2$Ph-2-Et | Me | H | H |
| $SO_2$Ph-2-OMe | Me | H | H |
| $SO_2$Ph-3-OMe | Me | H | H |
| $SO_2$Ph-4-OMe | Me | H | H |
| $SO_2$Ph-2-OEt | Me | H | H |
| $CH_2$Ph-2-$CO_2$Me | Me | H | H |
| $CH_2$Ph-3-$CO_2$Me | Me | H | H |
| $CH_2$Ph-4-$CO_2$Me | Me | H | H |
| $CH_2$Ph-2-$CO_2$Et | Me | H | H |
| CONHMe | Me | H | H |
| CONHEt | Me | H | H |
| CONHPr-n | Me | H | H |
| CONHPr-i | Me | H | H |
| CONHBu-n | Me | H | H |
| CONHBu-sec | Me | H | H |
| CONHBu-t | Me | H | H |
| CON(Me)$_2$ | Me | H | H |
| CON(Et)$_2$ | Me | H | H |
| CON(Pr-n)$_2$ | Me | H | H |
| F | Et | H | H |

| $R_3$ | $R_2$ | $R_1$ | $R_4$ |
|---|---|---|---|
| Cl | Et | H | H |
| Br | Et | H | H |
| I | Et | H | H |
| $CH=CH_2$ | Et | H | H |
| $CH_2CH=CH_2$ | Et | H | H |
| $C\equiv CH$ | Et | H | H |
| $CH_2C\equiv CH$ | Et | H | H |
| $CH_2F$ | Et | H | H |
| $CHF_2$ | Et | H | H |
| CN | Et | H | H |
| $NH_2$ | Et | H | H |
| NHCOMe | Et | H | H |
| $CH_2NH_2$ | Et | H | H |
| $CH_2NHCOMe$ | Et | H | H |
| $CH_2OCHF_2$ | Et | H | H |
| $CH_2OCF_3$ | Et | H | H |
| $CF_3$ | Et | H | H |
| $NO_2$ | Et | H | H |
| OMe | Et | H | H |
| OEt | Et | H | H |
| OPr-n | Et | H | H |
| OBu-n | Et | H | H |
| OPen-n | Et | H | H |
| SMe | Et | H | H |
| SEt | Et | H | H |
| SPr-n | Et | H | H |
| SBu-n | Et | H | H |
| SPen-n | Et | H | H |
| $SO_2Me$ | Et | H | H |
| $SO_2Et$ | Et | H | H |
| $SO_2Pr$-n | Et | H | H |
| COMe | Et | H | H |
| COEt | Et | H | H |
| COPr-n | Et | H | H |
| $CO_2H$ | Et | H | H |
| $CO_2Me$ | Et | H | H |
| $CO_2Et$ | Et | H | H |
| $CO_2Pr$-n | Et | H | H |
| $CO_2Bu$-n | Et | H | H |
| $CO_2Pen$-n | Et | H | H |

| $R_3$ | $R_2$ | $R_1$ | $R_4$ |
|---|---|---|---|
| $CH_2Cl$ | Et | H | H |
| $CH_2Br$ | Et | H | H |
| $CH_2I$ | Et | H | H |
| $CH_2CF_3$ | Et | H | H |
| $CH_2CN$ | Et | H | H |
| $CH_2OH$ | Et | H | H |
| $CH_2OMe$ | Et | H | H |
| $CH_2OEt$ | Et | H | H |
| $CH_2OPr$-n | Et | H | H |
| $CH_2SMe$ | Et | H | H |
| $CH_2SEt$ | Et | H | H |
| $CH_2SPr$-n | Et | H | H |
| $CH_2SO_2Me$ | Et | H | H |
| $CH_2SO_2Et$ | Et | H | H |
| $CH_2SO_2Pr$-n | Et | H | H |
| $CH_2COMe$ | Et | H | H |
| $CH_2COEt$ | Et | H | H |
| $CH_2COPr$-n | Et | H | H |
| $CH_2CO_2H$ | Et | H | H |
| $CH_2CO_2Me$ | Et | H | H |
| $CH_2CO_2Et$ | Et | H | H |
| $CH_2CO_2Pr$-n | Et | H | H |
| $CH_2OCH_2CH=CH_2$ | Et | H | H |
| $CH_2OCH_2C\equiv CH$ | Et | H | H |
| $CH=CHCO_2Me$ | Et | H | H |
| $CH=CHCO_2Et$ | Et | H | H |
| $CH=CHCO_2Pr$-n | Et | H | H |
| CH=CHCN | Et | H | H |
| Ph | Et | H | H |
| $CH_2Ph$ | Et | H | H |
| OPh | Et | H | H |
| SPh | Et | H | H |
| $SO_2Ph$ | Et | H | H |
| CONHMe | Et | H | H |
| CONHEt | Et | H | H |
| CONHPr-n | Et | H | H |
| CONHPr-i | Et | H | H |
| CONHBu-n | Et | H | H |
| CONHBu-sec | Et | H | H |
| CONHBu-t | Et | H | H |

| $R_3$ | $R_2$ | $R_1$ | $R_4$ |
|---|---|---|---|
| $CON(Me)_2$ | Et | H | H |
| $CON(Et)_2$ | Et | H | H |
| $CON(Pr\text{-}n)_2$ | Et | H | H |
| F | H | Pr-n | H |
| Cl | H | Pr-n | H |
| Br | H | Pr-n | H |
| I | H | Pr-n | H |
| $CH=CH_2$ | H | Pr-n | H |
| $CH_2CH=CH_2$ | H | Pr-n | H |
| $C\equiv CH$ | H | Pr-n | H |
| $CH_2C\equiv CH$ | H | Pr-n | H |
| $CH_2F$ | H | Pr-n | H |
| $CHF_2$ | H | Pr-n | H |
| CN | H | Pr-n | H |
| $NH_2$ | H | Pr-n | H |
| NHCOMe | H | Pr-n | H |
| $CH_2NH_2$ | H | Pr-n | H |
| $CH_2NHCOMe$ | H | Pr-n | H |
| $CH_2OCHF_2$ | H | Pr-n | H |
| $CH_2OCF_3$ | H | Pr-n | H |
| $CF_3$ | H | Pr-n | H |
| $NO_2$ | H | Pr-n | H |
| OMe | H | Pr-n | H |
| OEt | H | Pr-n | H |
| OPr-n | H | Pr-n | H |
| OBu-n | H | Pr-n | H |
| OPen-n | H | Pr-n | H |
| SMe | H | Pr-n | H |
| SEt | H | Pr-n | H |
| SPr-n | H | Pr-n | H |
| SBu-n | H | Pr-n | H |
| SPen-n | H | Pr-n | H |
| $SO_2Me$ | H | Pr-n | H |
| $SO_2Et$ | H | Pr-n | H |
| $SO_2Pr\text{-}n$ | H | Pr-n | H |
| COMe | H | Pr-n | H |
| COEt | H | Pr-n | H |
| COPr-n | H | Pr-n | H |
| $CO_2H$ | H | Pr-n | H |
| $CO_2Me$ | H | Pr-n | H |

| $R_3$ | $R_2$ | $R_1$ | $R_4$ |
|---|---|---|---|
| $CO_2Et$ | H | Pr-n | H |
| $CO_2Pr$-n | H | Pr-n | H |
| $CO_2Bu$-n | H | Pr-n | H |
| $CO_2Pen$-n | H | Pr-n | H |
| $CH_2Cl$ | H | Pr-n | H |
| $CH_2Br$ | H | Pr-n | H |
| $CH_2I$ | H | Pr-n | H |
| $CH_2CF_3$ | H | Pr-n | H |
| $CH_2CN$ | H | Pr-n | H |
| $CH_2OH$ | H | Pr-n | H |
| $CH_2OMe$ | H | Pr-n | H |
| $CH_2OEt$ | H | Pr-n | H |
| $CH_2OPr$-n | H | Pr-n | H |
| $CH_2SMe$ | H | Pr-n | H |
| $CH_2SEt$ | H | Pr-n | H |
| $CH_2SPr$-n | H | Pr-n | H |
| $CH_2SO_2Me$ | H | Pr-n | H |
| $CH_2SO_2Et$ | H | Pr-n | H |
| $CH_2SO_2Pr$-n | H | Pr-n | H |
| $CH_2COMe$ | H | Pr-n | H |
| $CH_2COEt$ | H | Pr-n | H |
| $CH_2COPr$-n | H | Pr-n | H |
| $CH_2CO_2H$ | H | Pr-n | H |
| $CH_2CO_2Me$ | H | Pr-n | H |
| $CH_2CO_2Et$ | H | Pr-n | H |
| $CH_2CO_2Pr$-n | H | Pr-n | H |
| $CH_2OCH_2CH=CH_2$ | H | Pr-n | H |
| $CH_2OCH_2C\equiv CH$ | H | Pr-n | H |
| $CH=CHCO_2Me$ | H | Pr-n | H |
| $CH=CHCO_2Et$ | H | Pr-n | H |
| $CH=CHCO_2Pr$-n | H | Pr-n | H |
| CH=CHCN | H | Pr-n | H |
| Ph | H | Pr-n | H |
| $CH_2Ph$ | H | Pr-n | H |
| OPh | H | Pr-n | H |
| SPh | H | Pr-n | H |
| $SO_2Ph$ | H | Pr-n | H |
| CONHMe | H | Pr-n | H |
| CONHEt | H | Pr-n | H |
| CONHPr-n | H | Pr-n | H |

| $R_3$ | $R_2$ | $R_1$ | $R_4$ |
|---|---|---|---|
| CONHPr-i | H | Pr-n | H |
| CONHBu-n | H | Pr-n | H |
| CONHBu-sec | H | Pr-n | H |
| CONHBu-t | H | Pr-n | H |
| $CON(Me)_2$ | H | Pr-n | H |
| $CON(Et)_2$ | H | Pr-n | H |
| $CON(Pr\text{-}n)_2$ | H | Pr-n | H |
| F | H | Cl | H |
| Cl | H | Cl | H |
| Br | H | Cl | H |
| I | H | Cl | H |
| $CH=CH_2$ | H | Cl | H |
| $CH_2CH=CH_2$ | H | Cl | H |
| $C\equiv CH$ | H | Cl | H |
| $CH_2C\equiv CH$ | H | Cl | H |
| $CH_2F$ | H | Cl | H |
| $CHF_2$ | H | Cl | H |
| CN | H | Cl | H |
| $NH_2$ | H | Cl | H |
| NHCOMe | H | Cl | H |
| $CH_2NH_2$ | H | Cl | H |
| $CH_2NHCOMe$ | H | Cl | H |
| $CH_2OCHF_2$ | H | Cl | H |
| $CH_2OCF_3$ | H | Cl | H |
| $CF_3$ | H | Cl | H |
| $NO_2$ | H | Cl | H |
| OMe | H | Cl | H |
| OEt | H | Cl | H |
| OPr-n | H | Cl | H |
| OBu-n | H | Cl | H |
| OPen-n | H | Cl | H |
| SMe | H | Cl | H |
| SEt | H | Cl | H |
| SPr-n | H | Cl | H |
| SBu-n | H | Cl | H |
| SPen-n | H | Cl | H |
| $SO_2Me$ | H | Cl | H |
| $SO_2Et$ | H | Cl | H |
| $SO_2Pr\text{-}n$ | H | Cl | H |
| COMe | H | Cl | H |

| $R_3$ | $R_2$ | $R_1$ | $R_4$ |
|---|---|---|---|
| COEt | H | Cl | H |
| COPr-n | H | Cl | H |
| $CO_2H$ | H | Cl | H |
| $CO_2Me$ | H | Cl | H |
| $CO_2Et$ | H | Cl | H |
| $CO_2Pr$-n | H | Cl | H |
| $CO_2Bu$-n | H | Cl | H |
| $CO_2Pen$-n | H | Cl | H |
| $CH_2Cl$ | H | Cl | H |
| $CH_2Br$ | H | Cl | H |
| $CH_2I$ | H | Cl | H |
| $CH_2CF_3$ | H | Cl | H |
| $CH_2CN$ | H | Cl | H |
| $CH_2OH$ | H | Cl | H |
| $CH_2OMe$ | H | Cl | H |
| $CH_2OEt$ | H | Cl | H |
| $CH_2OPr$-n | H | Cl | H |
| $CH_2SMe$ | H | Cl | H |
| $CH_2SEt$ | H | Cl | H |
| $CH_2SPr$-n | H | Cl | H |
| $CH_2SO_2Me$ | H | Cl | H |
| $CH_2SO_2Et$ | H | Cl | H |
| $CH_2SO_2Pr$-n | H | Cl | H |
| $CH_2COMe$ | H | Cl | H |
| $CH_2COEt$ | H | Cl | H |
| $CH_2COPr$-n | H | Cl | H |
| $CH_2CO_2H$ | H | Cl | H |
| $CH_2CO_2Me$ | H | Cl | H |
| $CH_2CO_2Et$ | H | Cl | H |
| $CH_2CO_2Pr$-n | H | Cl | H |
| $CH_2OCH_2CH=CH_2$ | H | Cl | H |
| $CH_2OCH_2C\equiv CH$ | H | Cl | H |
| $CH=CHCO_2Me$ | H | Cl | H |
| $CH=CHCO_2Et$ | H | Cl | H |
| $CH=CHCO_2Pr$-n | H | Cl | H |
| CH=CHCN | H | Cl | H |
| Ph | H | Cl | H |
| $CH_2Ph$ | H | Cl | H |
| OPh | H | Cl | H |
| SPh | H | Cl | H |

| $R_3$ | $R_2$ | $R_1$ | $R_4$ |
|---|---|---|---|
| $SO_2Ph$ | H | Cl | H |
| CONHMe | H | Cl | H |
| CONHEt | H | Cl | H |
| CONHPr-n | H | Cl | H |
| CONHPr-i | H | Cl | H |
| CONHBu-n | H | Cl | H |
| CONHBu-sec | H | Cl | H |
| CONHBu-t | H | Cl | H |
| $CON(Me)_2$ | H | Cl | H |
| $CON(Et)_2$ | H | Cl | H |
| $CON(Pr-n)_2$ | H | Cl | H |
| F | H | $NO_2$ | H |
| Cl | H | $NO_2$ | H |
| Br | H | $NO_2$ | H |
| I | H | $NO_2$ | H |
| $CH=CH_2$ | H | $NO_2$ | H |
| $CH_2CH=CH_2$ | H | $NO_2$ | H |
| $C\equiv CH$ | H | $NO_2$ | H |
| $CH_2C\equiv CH$ | H | $NO_2$ | H |
| $CH_2F$ | H | $NO_2$ | H |
| $CHF_2$ | H | $NO_2$ | H |
| CN | H | $NO_2$ | H |
| $NH_2$ | H | $NO_2$ | H |
| NHCOMe | H | $NO_2$ | H |
| $CH_2NH_2$ | H | $NO_2$ | H |
| $CH_2NHCOMe$ | H | $NO_2$ | H |
| $CH_2OCHF_2$ | H | $NO_2$ | H |
| $CH_2OCF_3$ | H | $NO_2$ | H |
| $CF_3$ | H | $NO_2$ | H |
| $NO_2$ | H | $NO_2$ | H |
| OMe | H | $NO_2$ | H |
| OEt | H | $NO_2$ | H |
| OPr-n | H | $NO_2$ | H |
| OBu-n | H | $NO_2$ | H |
| OPen-n | H | $NO_2$ | H |
| SMe | H | $NO_2$ | H |
| SEt | H | $NO_2$ | H |
| SPr-n | H | $NO_2$ | H |
| SBu-n | H | $NO_2$ | H |
| SPen-n | H | $NO_2$ | H |

| $R_3$ | $R_2$ | $R_1$ | $R_4$ |
|---|---|---|---|
| $SO_2Me$ | H | $NO_2$ | H |
| $SO_2Et$ | H | $NO_2$ | H |
| $SO_2Pr-n$ | H | $NO_2$ | H |
| COMe | H | $NO_2$ | H |
| COEt | H | $NO_2$ | H |
| COPr-n | H | $NO_2$ | H |
| $CO_2H$ | H | $NO_2$ | H |
| $CO_2Me$ | H | $NO_2$ | H |
| $CO_2Et$ | H | $NO_2$ | H |
| $CO_2Pr-n$ | H | $NO_2$ | H |
| $CO_2Bu-n$ | H | $NO_2$ | H |
| $CO_2Pen-n$ | H | $NO_2$ | H |
| $CH_2Cl$ | H | $NO_2$ | H |
| $CH_2Br$ | H | $NO_2$ | H |
| $CH_2I$ | H | $NO_2$ | H |
| $CH_2CF_3$ | H | $NO_2$ | H |
| $CH_2CN$ | H | $NO_2$ | H |
| $CH_2OH$ | H | $NO_2$ | H |
| $CH_2OMe$ | H | $NO_2$ | H |
| $CH_2OEt$ | H | $NO_2$ | H |
| $CH_2OPr-n$ | H | $NO_2$ | H |
| $CH_2SMe$ | H | $NO_2$ | H |
| $CH_2SEt$ | H | $NO_2$ | H |
| $CH_2SPr-n$ | H | $NO_2$ | H |
| $CH_2SO_2Me$ | H | $NO_2$ | H |
| $CH_2SO_2Et$ | H | $NO_2$ | H |
| $CH_2SO_2Pr-n$ | H | $NO_2$ | H |
| $CH_2COMe$ | H | $NO_2$ | H |
| $CH_2COEt$ | H | $NO_2$ | H |
| $CH_2COPr-n$ | H | $NO_2$ | H |
| $CH_2CO_2H$ | H | $NO_2$ | H |
| $CH_2CO_2Me$ | H | $NO_2$ | H |
| $CH_2CO_2Et$ | H | $NO_2$ | H |
| $CH_2CO_2Pr-n$ | H | $NO_2$ | H |
| $CH_2OCH_2CH=CH_2$ | H | $NO_2$ | H |
| $CH_2OCH_2C\equiv CH$ | H | $NO_2$ | H |
| $CH=CHCO_2Me$ | H | $NO_2$ | H |
| $CH=CHCO_2Et$ | H | $NO_2$ | H |
| $CH=CHCO_2Pr-n$ | H | $NO_2$ | H |
| CH=CHCN | H | $NO_2$ | H |

| $R_3$ | $R_2$ | $R_1$ | $R_4$ |
|---|---|---|---|
| Ph | H | $NO_2$ | H |
| $CH_2Ph$ | H | $NO_2$ | H |
| OPh | H | $NO_2$ | H |
| SPh | H | $NO_2$ | H |
| $SO_2Ph$ | H | $NO_2$ | H |
| CONHMe | H | $NO_2$ | H |
| CONHEt | H | $NO_2$ | H |
| CONHPr-n | H | $NO_2$ | H |
| CONHPr-i | H | $NO_2$ | H |
| CONHBu-n | H | $NO_2$ | H |
| CONHBu-sec | H | $NO_2$ | H |
| CONHBu-t | H | $NO_2$ | H |
| $CON(Me)_2$ | H | $NO_2$ | H |
| $CON(Et)_2$ | H | $NO_2$ | H |
| $CON(Pr\text{-}n)_2$ | H | $NO_2$ | H |
| F | H | $CF_3$ | H |
| Cl | H | $CF_3$ | H |
| Br | H | $CF_3$ | H |
| I | H | $CF_3$ | H |
| $CH=CH_2$ | H | $CF_3$ | H |
| $CH_2CH=CH_2$ | H | $CF_3$ | H |
| $C\equiv CH$ | H | $CF_3$ | H |
| $CH_2C\equiv CH$ | H | $CF_3$ | H |
| $CH_2F$ | H | $CF_3$ | H |
| $CHF_2$ | H | $CF_3$ | H |
| CN | H | $CF_3$ | H |
| $NH_2$ | H | $CF_3$ | H |
| NHCOMe | H | $CF_3$ | H |
| $CH_2NH_2$ | H | $CF_3$ | H |
| $CH_2NHCOMe$ | H | $CF_3$ | H |
| $CH_2OCHF_2$ | H | $CF_3$ | H |
| $CH_2OCF_3$ | H | $CF_3$ | H |
| $CF_3$ | H | $CF_3$ | H |
| $NO_2$ | H | $CF_3$ | H |
| OMe | H | $CF_3$ | H |
| OEt | H | $CF_3$ | H |
| OPr-n | H | $CF_3$ | H |
| OBu-n | H | $CF_3$ | H |
| OPen-n | H | $CF_3$ | H |
| SMe | H | $CF_3$ | H |

| $R_3$ | $R_2$ | $R_1$ | $R_4$ |
|---|---|---|---|
| SEt | H | $CF_3$ | H |
| SPr-n | H | $CF_3$ | H |
| SBu-n | H | $CF_3$ | H |
| SPen-n | H | $CF_3$ | H |
| $SO_2Me$ | H | $CF_3$ | H |
| $SO_2Et$ | H | $CF_3$ | H |
| $SO_2Pr$-n | H | $CF_3$ | H |
| COMe | H | $CF_3$ | H |
| COEt | H | $CF_3$ | H |
| COPr-n | H | $CF_3$ | H |
| $CO_2H$ | H | $CF_3$ | H |
| $CO_2Me$ | H | $CF_3$ | H |
| $CO_2Et$ | H | $CF_3$ | H |
| $CO_2Pr$-n | H | $CF_3$ | H |
| $CO_2Bu$-n | H | $CF_3$ | H |
| $CO_2Pen$-n | H | $CF_3$ | H |
| $CH_2Cl$ | H | $CF_3$ | H |
| $CH_2Br$ | H | $CF_3$ | H |
| $CH_2I$ | H | $CF_3$ | H |
| $CH_2CF_3$ | H | $CF_3$ | H |
| $CH_2CN$ | H | $CF_3$ | H |
| $CH_2OH$ | H | $CF_3$ | H |
| $CH_2OMe$ | H | $CF_3$ | H |
| $CH_2OEt$ | H | $CF_3$ | H |
| $CH_2OPr$-n | H | $CF_3$ | H |
| $CH_2SMe$ | H | $CF_3$ | H |
| $CH_2SEt$ | H | $CF_3$ | H |
| $CH_2SPr$-n | H | $CF_3$ | H |
| $CH_2SO_2Me$ | H | $CF_3$ | H |
| $CH_2SO_2Et$ | H | $CF_3$ | H |
| $CH_2SO_2Pr$-n | H | $CF_3$ | H |
| $CH_2COMe$ | H | $CF_3$ | H |
| $CH_2COEt$ | H | $CF_3$ | H |
| $CH_2COPr$-n | H | $CF_3$ | H |
| $CH_2CO_2H$ | H | $CF_3$ | H |
| $CH_2CO_2Me$ | H | $CF_3$ | H |
| $CH_2CO_2Et$ | H | $CF_3$ | H |
| $CH_2CO_2Pr$-n | H | $CF_3$ | H |
| $CH_2OCH_2CH=CH_2$ | H | $CF_3$ | H |
| $CH_2OCH_2C\equiv CH$ | H | $CF_3$ | H |

| $R_3$ | $R_2$ | $R_1$ | $R_4$ |
|---|---|---|---|
| $CH=CHCO_2Me$ | H | $CF_3$ | H |
| $CH=CHCO_2Et$ | H | $CF_3$ | H |
| $CH=CHCO_2Pr$-n | H | $CF_3$ | H |
| CH=CHCN | H | $CF_3$ | H |
| Ph | H | $CF_3$ | H |
| $CH_2Ph$ | H | $CF_3$ | H |
| OPh | H | $CF_3$ | H |
| SPh | H | $CF_3$ | H |
| $SO_2Ph$ | H | $CF_3$ | H |
| CONHMe | H | $CF_3$ | H |
| CONHEt | H | $CF_3$ | H |
| CONHPr-n | H | $CF_3$ | H |
| CONHPr-i | H | $CF_3$ | H |
| CONHBu-n | H | $CF_3$ | H |
| CONHBu-sec | H | $CF_3$ | H |
| CONHBu-t | H | $CF_3$ | H |
| $CON(Me)_2$ | H | $CF_3$ | H |
| $CON(Et)_2$ | H | $CF_3$ | H |
| $CON(Pr$-$n)_2$ | H | $CF_3$ | H |

| $R_3$ | $R_2$ | $R_1$ | $R_4$ |
|---|---|---|---|
| F | H | OMe | H |
| Cl | H | OMe | H |
| Br | H | OMe | H |
| I | H | OMe | H |
| $CH=CH_2$ | H | OMe | H |
| $CH_2CH=CH_2$ | H | OMe | H |
| $C\equiv CH$ | H | OMe | H |
| $CH_2C\equiv CH$ | H | OMe | H |
| $CH_2F$ | H | OMe | H |
| $CHF_2$ | H | OMe | H |
| CN | H | OMe | H |
| $NH_2$ | H | OMe | H |
| NHCOMe | H | OMe | H |
| $CH_2NH_2$ | H | OMe | H |
| $CH_2NHCOMe$ | H | OMe | H |
| $CH_2OCHF_2$ | H | OMe | H |
| $CH_2OCF_3$ | H | OMe | H |
| $CF_3$ | H | OMe | H |
| $NO_2$ | H | OMe | H |
| OMe | H | OMe | H |
| OEt | H | OMe | H |
| OPr-n | H | OMe | H |
| OBu-n | H | OMe | H |
| OPen-n | H | OMe | H |
| SMe | H | OMe | H |
| SEt | H | OMe | H |
| SPr-n | H | OMe | H |
| SBu-n | H | OMe | H |
| SPen-n | H | OMe | H |
| $SO_2Me$ | H | OMe | H |
| $SO_2Et$ | H | OMe | H |
| $SO_2Pr$-n | H | OMe | H |
| COMe | H | OMe | H |
| COEt | H | OMe | H |
| COPr-n | H | OMe | H |
| $CO_2H$ | H | OMe | H |
| $CO_2Me$ | H | OMe | H |
| $CO_2Et$ | H | OMe | H |
| $CO_2Pr$-n | H | OMe | H |
| $CO_2Bu$-n | H | OMe | H |

| $R_3$ | $R_2$ | $R_1$ | $R_4$ |
|---|---|---|---|
| $CO_2$Pen-n | H | OMe | H |
| $CH_2Cl$ | H | OMe | H |
| $CH_2Br$ | H | OMe | H |
| $CH_2I$ | H | OMe | H |
| $CH_2CF_3$ | H | OMe | H |
| $CH_2CN$ | H | OMe | H |
| $CH_2OH$ | H | OMe | H |
| $CH_2OMe$ | H | OMe | H |
| $CH_2OEt$ | H | OMe | H |
| $CH_2OPr$-n | H | OMe | H |
| $CH_2SMe$ | H | OMe | H |
| $CH_2SEt$ | H | OMe | H |
| $CH_2SPr$-n | H | OMe | H |
| $CH_2SO_2Me$ | H | OMe | H |
| $CH_2SO_2Et$ | H | OMe | H |
| $CH_2SO_2Pr$-n | H | OMe | H |
| $CH_2COMe$ | H | OMe | H |
| $CH_2COEt$ | H | OMe | H |
| $CH_2COPr$-n | H | OMe | H |
| $CH_2CO_2H$ | H | OMe | H |
| $CH_2CO_2Me$ | H | OMe | H |
| $CH_2CO_2Et$ | H | OMe | H |
| $CH_2CO_2Pr$-n | H | OMe | H |
| $CH_2OCH_2CH=CH_2$ | H | OMe | H |
| $CH_2OCH_2C\equiv CH$ | H | OMe | H |
| $CH=CHCO_2Me$ | H | OMe | H |
| $CH=CHCO_2Et$ | H | OMe | H |
| $CH=CHCO_2Pr$-n | H | OMe | H |
| CH=CHCN | H | OMe | H |
| Ph | H | OMe | H |
| $CH_2Ph$ | H | OMe | H |
| OPh | H | OMe | H |
| SPh | H | OMe | H |
| $SO_2Ph$ | H | OMe | H |
| CONHMe | H | OMe | H |
| CONHEt | H | OMe | H |
| CONHPr-n | H | OMe | H |
| CONHPr-i | H | OMe | H |
| CONHBu-n | H | OMe | H |
| CONHBu-sec | H | OMe | H |

| $R_3$ | $R_2$ | $R_1$ | $R_4$ |
|---|---|---|---|
| CONHBu-t | H | OMe | H |
| $CON(Me)_2$ | H | OMe | H |
| $CON(Et)_2$ | H | OMe | H |
| $CON(Pr\text{-}n)_2$ | H | OMe | H |
| F | H | SMe | H |
| Cl | H | SMe | H |
| Br | H | SMe | H |
| I | H | SMe | H |
| $CH=CH_2$ | H | SMe | H |
| $CH_2CH=CH_2$ | H | SMe | H |
| $C\equiv CH$ | H | SMe | H |
| $CH_2C\equiv CH$ | H | SMe | H |
| $CH_2F$ | H | SMe | H |
| $CHF_2$ | H | SMe | H |
| CN | H | SMe | H |
| $NH_2$ | H | SMe | H |
| NHCOMe | H | SMe | H |
| $CH_2NH_2$ | H | SMe | H |
| $CH_2NHCOMe$ | H | SMe | H |
| $CH_2OCHF_2$ | H | SMe | H |
| $CH_2OCF_3$ | H | SMe | H |
| $CF_3$ | H | SMe | H |
| $NO_2$ | H | SMe | H |
| OMe | H | SMe | H |
| OEt | H | SMe | H |
| OPr-n | H | SMe | H |
| OBu-n | H | SMe | H |
| OPen-n | H | SMe | H |
| SMe | H | SMe | H |
| SEt | H | SMe | H |
| SPr-n | H | SMe | H |
| SBu-n | H | SMe | H |
| SPen-n | H | SMe | H |
| $SO_2Me$ | H | SMe | H |
| $SO_2Et$ | H | SMe | H |
| $SO_2Pr\text{-}n$ | H | SMe | H |
| COMe | H | SMe | H |
| COEt | H | SMe | H |
| COPr-n | H | SMe | H |
| $CO_2H$ | H | SMe | H |

| $R_3$ | $R_2$ | $R_1$ | $R_4$ |
|---|---|---|---|
| $CO_2Me$ | H | SMe | H |
| $CO_2Et$ | H | SMe | H |
| $CO_2Pr$-n | H | SMe | H |
| $CO_2Bu$-n | H | SMe | H |
| $CO_2Pen$-n | H | SMe | H |
| $CH_2Cl$ | H | SMe | H |
| $CH_2Br$ | H | SMe | H |
| $CH_2I$ | H | SMe | H |
| $CH_2CF_3$ | H | SMe | H |
| $CH_2CN$ | H | SMe | H |
| $CH_2OH$ | H | SMe | H |
| $CH_2OMe$ | H | SMe | H |
| $CH_2OEt$ | H | SMe | H |
| $CH_2OPr$-n | H | SMe | H |
| $CH_2SMe$ | H | SMe | H |
| $CH_2SEt$ | H | SMe | H |
| $CH_2SPr$-n | H | SMe | H |
| $CH_2SO_2Me$ | H | SMe | H |
| $CH_2SO_2Et$ | H | SMe | H |
| $CH_2SO_2Pr$-n | H | SMe | H |
| $CH_2COMe$ | H | SMe | H |
| $CH_2COEt$ | H | SMe | H |
| $CH_2COPr$-n | H | SMe | H |
| $CH_2CO_2H$ | H | SMe | H |
| $CH_2CO_2Me$ | H | SMe | H |
| $CH_2CO_2Et$ | H | SMe | H |
| $CH_2CO_2Pr$-n | H | SMe | H |
| $CH_2OCH_2CH{=}CH_2$ | H | SMe | H |
| $CH_2OCH_2C\equiv CH$ | H | SMe | H |
| $CH{=}CHCO_2Me$ | H | SMe | H |
| $CH{=}CHCO_2Et$ | H | SMe | H |
| $CH{=}CHCO_2Pr$-n | H | SMe | H |
| CH=CHCN | H | SMe | H |
| Ph | H | SMe | H |
| $CH_2Ph$ | H | SMe | H |
| OPh | H | SMe | H |
| SPh | H | SMe | H |
| $SO_2Ph$ | H | SMe | H |
| CONHMe | H | SMe | H |
| CONHEt | H | SMe | H |

| $R_3$ | $R_2$ | $R_1$ | $R_4$ |
|---|---|---|---|
| CONHPr-n | H | SMe | H |
| CONHPr-i | H | SMe | H |
| CONHBu-n | H | SMe | H |
| CONHBu-sec | H | SMe | H |
| CONHBu-t | H | SMe | H |
| $CON(Me)_2$ | H | SMe | H |
| $CON(Et)_2$ | H | SMe | H |
| $CON(Pr-n)_2$ | H | SMe | H |
| F | H | $SO_2Me$ | H |
| Cl | H | $SO_2Me$ | H |
| Br | H | $SO_2Me$ | H |
| I | H | $SO_2Me$ | H |
| $CH=CH_2$ | H | $SO_2Me$ | H |
| $CH_2CH=CH_2$ | H | $SO_2Me$ | H |
| $C\equiv CH$ | H | $SO_2Me$ | H |
| $CH_2C\equiv CH$ | H | $SO_2Me$ | H |
| $CH_2F$ | H | $SO_2Me$ | H |
| $CHF_2$ | H | $SO_2Me$ | H |
| CN | H | $SO_2Me$ | H |
| $NH_2$ | H | $SO_2Me$ | H |
| NHCOMe | H | $SO_2Me$ | H |
| $CH_2NH_2$ | H | $SO_2Me$ | H |
| $CH_2NHCOMe$ | H | $SO_2Me$ | H |
| $CH_2OCHF_2$ | H | $SO_2Me$ | H |
| $CH_2OCF_3$ | H | $SO_2Me$ | H |
| $CF_3$ | H | $SO_2Me$ | H |
| $NO_2$ | H | $SO_2Me$ | H |
| OMe | H | $SO_2Me$ | H |
| OEt | H | $SO_2Me$ | H |
| OPr-n | H | $SO_2Me$ | H |
| OBu-n | H | $SO_2Me$ | H |
| OPen-n | H | $SO_2Me$ | H |
| SMe | H | $SO_2Me$ | H |
| SEt | H | $SO_2Me$ | H |
| SPr-n | H | $SO_2Me$ | H |
| SBu-n | H | $SO_2Me$ | H |
| SPen-n | H | $SO_2Me$ | H |
| $SO_2Me$ | H | $SO_2Me$ | H |
| $SO_2Et$ | H | $SO_2Me$ | H |
| $SO_2Pr-n$ | H | $SO_2Me$ | H |

| $R_3$ | $R_2$ | $R_1$ | $R_4$ |
|---|---|---|---|
| COMe | H | $SO_2Me$ | H |
| COEt | H | $SO_2Me$ | H |
| COPr-n | H | $SO_2Me$ | H |
| $CO_2H$ | H | $SO_2Me$ | H |
| $CO_2Me$ | H | $SO_2Me$ | H |
| $CO_2Et$ | H | $SO_2Me$ | H |
| $CO_2Pr$-n | H | $SO_2Me$ | H |
| $CO_2Bu$-n | H | $SO_2Me$ | H |
| $CO_2Pen$-n | H | $SO_2Me$ | H |
| $CH_2Cl$ | H | $SO_2Me$ | H |
| $CH_2Br$ | H | $SO_2Me$ | H |
| $CH_2I$ | H | $SO_2Me$ | H |
| $CH_2CF_3$ | H | $SO_2Me$ | H |
| $CH_2CN$ | H | $SO_2Me$ | H |
| $CH_2OH$ | H | $SO_2Me$ | H |
| $CH_2OMe$ | H | $SO_2Me$ | H |
| $CH_2OEt$ | H | $SO_2Me$ | H |
| $CH_2OPr$-n | H | $SO_2Me$ | H |
| $CH_2SMe$ | H | $SO_2Me$ | H |
| $CH_2SEt$ | H | $SO_2Me$ | H |
| $CH_2SPr$-n | H | $SO_2Me$ | H |
| $CH_2SO_2Me$ | H | $SO_2Me$ | H |
| $CH_2SO_2Et$ | H | $SO_2Me$ | H |
| $CH_2SO_2Pr$-n | H | $SO_2Me$ | H |
| $CH_2COMe$ | H | $SO_2Me$ | H |
| $CH_2COEt$ | H | $SO_2Me$ | H |
| $CH_2COPr$-n | H | $SO_2Me$ | H |
| $CH_2CO_2H$ | H | $SO_2Me$ | H |
| $CH_2CO_2Me$ | H | $SO_2Me$ | H |
| $CH_2CO_2Et$ | H | $SO_2Me$ | H |
| $CH_2CO_2Pr$-n | H | $SO_2Me$ | H |
| $CH_2OCH_2CH=CH_2$ | H | $SO_2Me$ | H |
| $CH_2OCH_2C\equiv CH$ | H | $SO_2Me$ | H |
| $CH=CHCO_2Me$ | H | $SO_2Me$ | H |
| $CH=CHCO_2Et$ | H | $SO_2Me$ | H |
| $CH=CHCO_2Pr$-n | H | $SO_2Me$ | H |
| CH=CHCN | H | $SO_2Me$ | H |
| Ph | H | $SO_2Me$ | H |
| $CH_2Ph$ | H | $SO_2Me$ | H |
| OPh | H | $SO_2Me$ | H |

| $R_3$ | $R_2$ | $R_1$ | $R_4$ |
|---|---|---|---|
| SPh | H | $SO_2Me$ | H |
| $SO_2Ph$ | H | $SO_2Me$ | H |
| CONHMe | H | $SO_2Me$ | H |
| CONHEt | H | $SO_2Me$ | H |
| CONHPr-n | H | $SO_2Me$ | H |
| CONHPr-i | H | $SO_2Me$ | H |
| CONHBu-n | H | $SO_2Me$ | H |
| CONHBu-sec | H | $SO_2Me$ | H |
| CONHBu-t | H | $SO_2Me$ | H |
| $CON(Me)_2$ | H | $SO_2Me$ | H |
| $CON(Et)_2$ | H | $SO_2Me$ | H |
| $CON(Pr-n)_2$ | H | $SO_2Me$ | H |
| F | H | COMe | H |
| Cl | H | COMe | H |
| Br | H | COMe | H |
| I | H | COMe | H |
| $CH=CH_2$ | H | COMe | H |
| $CH_2CH=CH_2$ | H | COMe | H |
| $C\equiv CH$ | H | COMe | H |
| $CH_2C\equiv CH$ | H | COMe | H |
| $CH_2F$ | H | COMe | H |
| $CHF_2$ | H | COMe | H |
| CN | H | COMe | H |
| $NH_2$ | H | COMe | H |
| NHCOMe | H | COMe | H |
| $CH_2NH_2$ | H | COMe | H |
| $CH_2NHCOMe$ | H | COMe | H |
| $CH_2OCHF_2$ | H | COMe | H |
| $CH_2OCF_3$ | H | COMe | H |
| $CF_3$ | H | COMe | H |
| $NO_2$ | H | COMe | H |
| OMe | H | COMe | H |
| OEt | H | COMe | H |
| OPr-n | H | COMe | H |
| OBu-n | H | COMe | H |
| OPen-n | H | COMe | H |
| SMe | H | COMe | H |
| SEt | H | COMe | H |
| SPr-n | H | COMe | H |
| SBu-n | H | COMe | H |

| $R_3$ | $R_2$ | $R_1$ | $R_4$ |
|---|---|---|---|
| SPen-n | H | COMe | H |
| $SO_2Me$ | H | COMe | H |
| $SO_2Et$ | H | COMe | H |
| $SO_2Pr$-n | H | COMe | H |
| COMe | H | COMe | H |
| COEt | H | COMe | H |
| COPr-n | H | COMe | H |
| $CO_2H$ | H | COMe | H |
| $CO_2Me$ | H | COMe | H |
| $CO_2Et$ | H | COMe | H |
| $CO_2Pr$-n | H | COMe | H |
| $CO_2Bu$-n | H | COMe | H |
| $CO_2Pen$-n | H | COMe | H |
| $CH_2Cl$ | H | COMe | H |
| $CH_2Br$ | H | COMe | H |
| $CH_2I$ | H | COMe | H |
| $CH_2CF_3$ | H | COMe | H |
| $CH_2CN$ | H | COMe | H |
| $CH_2OH$ | H | COMe | H |
| $CH_2OMe$ | H | COMe | H |
| $CH_2OEt$ | H | COMe | H |
| $CH_2OPr$-n | H | COMe | H |
| $CH_2SMe$ | H | COMe | H |
| $CH_2SEt$ | H | COMe | H |
| $CH_2SPr$-n | H | COMe | H |
| $CH_2SO_2Me$ | H | COMe | H |
| $CH_2SO_2Et$ | H | COMe | H |
| $CH_2SO_2Pr$-n | H | COMe | H |
| $CH_2COMe$ | H | COMe | H |
| $CH_2COEt$ | H | COMe | H |
| $CH_2COPr$-n | H | COMe | H |
| $CH_2CO_2H$ | H | COMe | H |
| $CH_2CO_2Me$ | H | COMe | H |
| $CH_2CO_2Et$ | H | COMe | H |
| $CH_2CO_2Pr$-n | H | COMe | H |
| $CH_2OCH_2CH=CH_2$ | H | COMe | H |
| $CH_2OCH_2C\equiv CH$ | H | COMe | H |
| $CH=CHCO_2Me$ | H | COMe | H |
| $CH=CHCO_2Et$ | H | COMe | H |
| $CH=CHCO_2Pr$-n | H | COMe | H |

| $R_3$ | $R_2$ | $R_1$ | $R_4$ |
|---|---|---|---|
| CH=CHCN | H | COMe | H |
| Ph | H | COMe | H |
| $CH_2Ph$ | H | COMe | H |
| OPh | H | COMe | H |
| SPh | H | COMe | H |
| $SO_2Ph$ | H | COMe | H |
| CONHMe | H | COMe | H |
| CONHEt | H | COMe | H |
| CONHPr-n | H | COMe | H |
| CONHPr-i | H | COMe | H |
| CONHBu-n | H | COMe | H |
| CONHBu-sec | H | COMe | H |
| CONHBu-t | H | COMe | H |
| $CON(Me)_2$ | H | COMe | H |
| $CON(Et)_2$ | H | COMe | H |
| $CON(Pr\text{-}n)_2$ | H | COMe | H |
| F | H | $CO_2Me$ | H |
| Cl | H | $CO_2Me$ | H |
| Br | H | $CO_2Me$ | H |
| I | H | $CO_2Me$ | H |
| $CH=CH_2$ | H | $CO_2Me$ | H |
| $CH_2CH=CH_2$ | H | $CO_2Me$ | H |
| C≡CH | H | $CO_2Me$ | H |
| $CH_2C≡CH$ | H | $CO_2Me$ | H |
| $CH_2F$ | H | $CO_2Me$ | H |
| $CHF_2$ | H | $CO_2Me$ | H |
| CN | H | $CO_2Me$ | H |
| $NH_2$ | H | $CO_2Me$ | H |
| NHCOMe | H | $CO_2Me$ | H |
| $CH_2NH_2$ | H | $CO_2Me$ | H |
| $CH_2NHCOMe$ | H | $CO_2Me$ | H |
| $CH_2OCHF_2$ | H | $CO_2Me$ | H |
| $CH_2OCF_3$ | H | $CO_2Me$ | H |
| $CF_3$ | H | $CO_2Me$ | H |
| $NO_2$ | H | $CO_2Me$ | H |
| OMe | H | $CO_2Me$ | H |
| OEt | H | $CO_2Me$ | H |
| OPr-n | H | $CO_2Me$ | H |
| OBu-n | H | $CO_2Me$ | H |
| OPen-n | H | $CO_2Me$ | H |

| $R_3$ | $R_2$ | $R_1$ | $R_4$ |
|---|---|---|---|
| SMe | H | $CO_2Me$ | H |
| SEt | H | $CO_2Me$ | H |
| SPr-n | H | $CO_2Me$ | H |
| SBu-n | H | $CO_2Me$ | H |
| SPen-n | H | $CO_2Me$ | H |
| $SO_2Me$ | H | $CO_2Me$ | H |
| $SO_2Et$ | H | $CO_2Me$ | H |
| $SO_2Pr$-n | H | $CO_2Me$ | H |
| COMe | H | $CO_2Me$ | H |
| COEt | H | $CO_2Me$ | H |
| COPr-n | H | $CO_2Me$ | H |
| $CO_2H$ | H | $CO_2Me$ | H |
| $CO_2Me$ | H | $CO_2Me$ | H |
| $CO_2Et$ | H | $CO_2Me$ | H |
| $CO_2Pr$-n | H | $CO_2Me$ | H |
| $CO_2Bu$-n | H | $CO_2Me$ | H |
| $CO_2Pen$-n | H | $CO_2Me$ | H |
| $CH_2Cl$ | H | $CO_2Me$ | H |
| $CH_2Br$ | H | $CO_2Me$ | H |
| $CH_2I$ | H | $CO_2Me$ | H |
| $CH_2CF_3$ | H | $CO_2Me$ | H |
| $CH_2CN$ | H | $CO_2Me$ | H |
| $CH_2OH$ | H | $CO_2Me$ | H |
| $CH_2OMe$ | H | $CO_2Me$ | H |
| $CH_2OEt$ | H | $CO_2Me$ | H |
| $CH_2OPr$-n | H | $CO_2Me$ | H |
| $CH_2SMe$ | H | $CO_2Me$ | H |
| $CH_2SEt$ | H | $CO_2Me$ | H |
| $CH_2SPr$-n | H | $CO_2Me$ | H |
| $CH_2SO_2Me$ | H | $CO_2Me$ | H |
| $CH_2SO_2Et$ | H | $CO_2Me$ | H |
| $CH_2SO_2Pr$-n | H | $CO_2Me$ | H |
| $CH_2COMe$ | H | $CO_2Me$ | H |
| $CH_2COEt$ | H | $CO_2Me$ | H |
| $CH_2COPr$-n | H | $CO_2Me$ | H |
| $CH_2CO_2H$ | H | $CO_2Me$ | H |
| $CH_2CO_2Me$ | H | $CO_2Me$ | H |
| $CH_2CO_2Et$ | H | $CO_2Me$ | H |
| $CH_2CO_2Pr$-n | H | $CO_2Me$ | H |
| $CH_2OCH_2CH=CH_2$ | H | $CO_2Me$ | H |

| $R_3$ | $R_2$ | $R_1$ | $R_4$ |
|---|---|---|---|
| $CH_2OCH_2C\equiv CH$ | H | $CO_2Me$ | H |
| $CH=CHCO_2Me$ | H | $CO_2Me$ | H |
| $CH=CHCO_2Et$ | H | $CO_2Me$ | H |
| $CH=CHCO_2Pr$-n | H | $CO_2Me$ | H |
| CH=CHCN | H | $CO_2Me$ | H |
| Ph | H | $CO_2Me$ | H |
| $CH_2Ph$ | H | $CO_2Me$ | H |
| OPh | H | $CO_2Me$ | H |
| SPh | H | $CO_2Me$ | H |
| $SO_2Ph$ | H | $CO_2Me$ | H |
| CONHMe | H | $CO_2Me$ | H |
| CONHEt | H | $CO_2Me$ | H |
| CONHPr-n | H | $CO_2Me$ | H |
| CONHPr-i | H | $CO_2Me$ | H |
| CONHBu-n | H | $CO_2Me$ | H |
| CONHBu-sec | H | $CO_2Me$ | H |
| CONHBu-t | H | $CO_2Me$ | H |
| $CON(Me)_2$ | H | $CO_2Me$ | H |
| $CON(Et)_2$ | H | $CO_2Me$ | H |
| $CON(Pr\text{-}n)_2$ | H | $CO_2Me$ | H |
| F | H | $CO_2H$ | H |
| Cl | H | $CO_2H$ | H |
| Br | H | $CO_2H$ | H |
| I | H | $CO_2H$ | H |
| $CH=CH_2$ | H | $CO_2H$ | H |
| $CH_2CH=CH_2$ | H | $CO_2H$ | H |
| $C\equiv CH$ | H | $CO_2H$ | H |
| $CH_2C\equiv CH$ | H | $CO_2H$ | H |
| $CH_2F$ | H | $CO_2H$ | H |
| $CHF_2$ | H | $CO_2H$ | H |
| CN | H | $CO_2H$ | H |
| $NH_2$ | H | $CO_2H$ | H |
| NHCOMe | H | $CO_2H$ | H |
| $CH_2NH_2$ | H | $CO_2H$ | H |
| $CH_2NHCOMe$ | H | $CO_2H$ | H |
| $CH_2OCHF_2$ | H | $CO_2H$ | H |
| $CH_2OCF_3$ | H | $CO_2H$ | H |
| $CF_3$ | H | $CO_2H$ | H |
| $NO_2$ | H | $CO_2H$ | H |
| OMe | H | $CO_2H$ | H |

| $R_3$ | $R_2$ | $R_1$ | $R_4$ |
|---|---|---|---|
| OEt | H | $CO_2H$ | H |
| OPr-n | H | $CO_2H$ | H |
| OBu-n | H | $CO_2H$ | H |
| OPen-n | H | $CO_2H$ | H |
| SMe | H | $CO_2H$ | H |
| SEt | H | $CO_2H$ | H |
| SPr-n | H | $CO_2H$ | H |
| SBu-n | H | $CO_2H$ | H |
| SPen-n | H | $CO_2H$ | H |
| $SO_2Me$ | H | $CO_2H$ | H |
| $SO_2Et$ | H | $CO_2H$ | H |
| $SO_2Pr$-n | H | $CO_2H$ | H |
| COMe | H | $CO_2H$ | H |
| COEt | H | $CO_2H$ | H |
| COPr-n | H | $CO_2H$ | H |
| $CO_2H$ | H | $CO_2H$ | H |
| $CO_2Me$ | H | $CO_2H$ | H |
| $CO_2Et$ | H | $CO_2H$ | H |
| $CO_2Pr$-n | H | $CO_2H$ | H |
| $CO_2Bu$-n | H | $CO_2H$ | H |
| $CO_2Pen$-n | H | $CO_2H$ | H |
| $CH_2Cl$ | H | $CO_2H$ | H |
| $CH_2Br$ | H | $CO_2H$ | H |
| $CH_2I$ | H | $CO_2H$ | H |
| $CH_2CF_3$ | H | $CO_2H$ | H |
| $CH_2CN$ | H | $CO_2H$ | H |
| $CH_2OH$ | H | $CO_2H$ | H |
| $CH_2OMe$ | H | $CO_2H$ | H |
| $CH_2OEt$ | H | $CO_2H$ | H |
| $CH_2OPr$-n | H | $CO_2H$ | H |
| $CH_2SMe$ | H | $CO_2H$ | H |
| $CH_2SEt$ | H | $CO_2H$ | H |
| $CH_2SPr$-n | H | $CO_2H$ | H |
| $CH_2SO_2Me$ | H | $CO_2H$ | H |
| $CH_2SO_2Et$ | H | $CO_2H$ | H |
| $CH_2SO_2Pr$-n | H | $CO_2H$ | H |
| $CH_2COMe$ | H | $CO_2H$ | H |
| $CH_2COEt$ | H | $CO_2H$ | H |
| $CH_2COPr$-n | H | $CO_2H$ | H |
| $CH_2CO_2H$ | H | $CO_2H$ | H |

| $R_3$ | $R_2$ | $R_1$ | $R_4$ |
|---|---|---|---|
| $CH_2CO_2Me$ | H | $CO_2H$ | H |
| $CH_2CO_2Et$ | H | $CO_2H$ | H |
| $CH_2CO_2Pr$-n | H | $CO_2H$ | H |
| $CH_2OCH_2CH=CH_2$ | H | $CO_2H$ | H |
| $CH_2OCH_2C\equiv CH$ | H | $CO_2H$ | H |
| $CH=CHCO_2Me$ | H | $CO_2H$ | H |
| $CH=CHCO_2Et$ | H | $CO_2H$ | H |
| $CH=CHCO_2Pr$-n | H | $CO_2H$ | H |
| CH=CHCN | H | $CO_2H$ | H |
| Ph | H | $CO_2H$ | H |
| $CH_2Ph$ | H | $CO_2H$ | H |
| OPh | H | $CO_2H$ | H |
| SPh | H | $CO_2H$ | H |
| $SO_2Ph$ | H | $CO_2H$ | H |
| CONHMe | H | $CO_2H$ | H |
| CONHEt | H | $CO_2H$ | H |
| CONHPr-n | H | $CO_2H$ | H |
| CONHPr-i | H | $CO_2H$ | H |
| CONHBu-n | H | $CO_2H$ | H |
| CONHBu-sec | H | $CO_2H$ | H |
| CONHBu-t | H | $CO_2H$ | H |
| $CON(Me)_2$ | H | $CO_2H$ | H |
| $CON(Et)_2$ | H | $CO_2H$ | H |
| $CON(Pr\text{-}n)_2$ | H | $CO_2H$ | H |
| F | H | $CH_2F$ | H |
| Cl | H | $CH_2F$ | H |
| Br | H | $CH_2F$ | H |
| I | H | $CH_2F$ | H |
| $CH=CH_2$ | H | $CH_2F$ | H |
| $CH_2CH=CH_2$ | H | $CH_2F$ | H |
| $C\equiv CH$ | H | $CH_2F$ | H |
| $CH_2C\equiv CH$ | H | $CH_2F$ | H |
| $CH_2F$ | H | $CH_2F$ | H |
| $CHF_2$ | H | $CH_2F$ | H |
| CN | H | $CH_2F$ | H |
| $NH_2$ | H | $CH_2F$ | H |
| NHCOMe | H | $CH_2F$ | H |
| $CH_2NH_2$ | H | $CH_2F$ | H |
| $CH_2NHCOMe$ | H | $CH_2F$ | H |
| $CH_2OCHF_2$ | H | $CH_2F$ | H |

| $R_3$ | $R_2$ | $R_1$ | $R_4$ |
|---|---|---|---|
| $CH_2OCF_3$ | H | $CH_2F$ | H |
| $CF_3$ | H | $CH_2F$ | H |
| $NO_2$ | H | $CH_2F$ | H |
| OMe | H | $CH_2F$ | H |
| OEt | H | $CH_2F$ | H |
| OPr-n | H | $CH_2F$ | H |
| OBu-n | H | $CH_2F$ | H |
| OPen-n | H | $CH_2F$ | H |
| SMe | H | $CH_2F$ | H |
| SEt | H | $CH_2F$ | H |
| SPr-n | H | $CH_2F$ | H |
| SBu-n | H | $CH_2F$ | H |
| SPen-n | H | $CH_2F$ | H |
| $SO_2Me$ | H | $CH_2F$ | H |
| $SO_2Et$ | H | $CH_2F$ | H |
| $SO_2Pr$-n | H | $CH_2F$ | H |
| COMe | H | $CH_2F$ | H |
| COEt | H | $CH_2F$ | H |
| COPr-n | H | $CH_2F$ | H |
| $CO_2H$ | H | $CH_2F$ | H |
| $CO_2Me$ | H | $CH_2F$ | H |
| $CO_2Et$ | H | $CH_2F$ | H |
| $CO_2Pr$-n | H | $CH_2F$ | H |
| $CO_2Bu$-n | H | $CH_2F$ | H |
| $CO_2Pen$-n | H | $CH_2F$ | H |
| $CH_2Cl$ | H | $CH_2F$ | H |
| $CH_2Br$ | H | $CH_2F$ | H |
| $CH_2I$ | H | $CH_2F$ | H |
| $CH_2CF_3$ | H | $CH_2F$ | H |
| $CH_2CN$ | H | $CH_2F$ | H |
| $CH_2OH$ | H | $CH_2F$ | H |
| $CH_2OMe$ | H | $CH_2F$ | H |
| $CH_2OEt$ | H | $CH_2F$ | H |
| $CH_2OPr$-n | H | $CH_2F$ | H |
| $CH_2SMe$ | H | $CH_2F$ | H |
| $CH_2SEt$ | H | $CH_2F$ | H |
| $CH_2SPr$-n | H | $CH_2F$ | H |
| $CH_2SO_2Me$ | H | $CH_2F$ | H |
| $CH_2SO_2Et$ | H | $CH_2F$ | H |
| $CH_2SO_2Pr$-n | H | $CH_2F$ | H |

| $R_3$ | $R_2$ | $R_1$ | $R_4$ |
|---|---|---|---|
| $CH_2COMe$ | H | $CH_2F$ | H |
| $CH_2COEt$ | H | $CH_2F$ | H |
| $CH_2COPr$-n | H | $CH_2F$ | H |
| $CH_2CO_2H$ | H | $CH_2F$ | H |
| $CH_2CO_2Me$ | H | $CH_2F$ | H |
| $CH_2CO_2Et$ | H | $CH_2F$ | H |
| $CH_2CO_2Pr$-n | H | $CH_2F$ | H |
| $CH_2OCH_2CH=CH_2$ | H | $CH_2F$ | H |
| $CH_2OCH_2C\equiv CH$ | H | $CH_2F$ | H |
| $CH=CHCO_2Me$ | H | $CH_2F$ | H |
| $CH=CHCO_2Et$ | H | $CH_2F$ | H |
| $CH=CHCO_2Pr$-n | H | $CH_2F$ | H |
| CH=CHCN | H | $CH_2F$ | H |
| Ph | H | $CH_2F$ | H |
| $CH_2Ph$ | H | $CH_2F$ | H |
| OPh | H | $CH_2F$ | H |
| SPh | H | $CH_2F$ | H |
| $SO_2Ph$ | H | $CH_2F$ | H |
| CONHMe | H | $CH_2F$ | H |
| CONHEt | H | $CH_2F$ | H |
| CONHPr-n | H | $CH_2F$ | H |
| CONHPr-i | H | $CH_2F$ | H |
| CONHBu-n | H | $CH_2F$ | H |
| CONHBu-sec | H | $CH_2F$ | H |
| CONHBu-t | H | $CH_2F$ | H |
| $CON(Me)_2$ | H | $CH_2F$ | H |
| $CON(Et)_2$ | H | $CH_2F$ | H |
| $CON(Pr\text{-}n)_2$ | H | $CH_2F$ | H |
| F | H | $CH_2Cl$ | H |
| Cl | H | $CH_2Cl$ | H |
| Br | H | $CH_2Cl$ | H |
| I | H | $CH_2Cl$ | H |
| $CH=CH_2$ | H | $CH_2Cl$ | H |
| $CH_2CH=CH_2$ | H | $CH_2Cl$ | H |
| $C\equiv CH$ | H | $CH_2Cl$ | H |
| $CH_2C\equiv CH$ | H | $CH_2Cl$ | H |
| $CH_2F$ | H | $CH_2Cl$ | H |
| $CHF_2$ | H | $CH_2Cl$ | H |
| CN | H | $CH_2Cl$ | H |
| $NH_2$ | H | $CH_2Cl$ | H |

| $R_3$ | $R_2$ | $R_1$ | $R_4$ |
|---|---|---|---|
| NHCOMe | H | $CH_2Cl$ | H |
| $CH_2NH_2$ | H | $CH_2Cl$ | H |
| $CH_2NHCOMe$ | H | $CH_2Cl$ | H |
| $CH_2OCHF_2$ | H | $CH_2Cl$ | H |
| $CH_2OCF_3$ | H | $CH_2Cl$ | H |
| $CF_3$ | H | $CH_2Cl$ | H |
| $NO_2$ | H | $CH_2Cl$ | H |
| OMe | H | $CH_2Cl$ | H |
| OEt | H | $CH_2Cl$ | H |
| OPr-n | H | $CH_2Cl$ | H |
| OBu-n | H | $CH_2Cl$ | H |
| OPen-n | H | $CH_2Cl$ | H |
| SMe | H | $CH_2Cl$ | H |
| SEt | H | $CH_2Cl$ | H |
| SPr-n | H | $CH_2Cl$ | H |
| SBu-n | H | $CH_2Cl$ | H |
| SPen-n | H | $CH_2Cl$ | H |
| $SO_2Me$ | H | $CH_2Cl$ | H |
| $SO_2Et$ | H | $CH_2Cl$ | H |
| $SO_2Pr$-n | H | $CH_2Cl$ | H |
| COMe | H | $CH_2Cl$ | H |
| COEt | H | $CH_2Cl$ | H |
| COPr-n | H | $CH_2Cl$ | H |
| $CO_2H$ | H | $CH_2Cl$ | H |
| $CO_2Me$ | H | $CH_2Cl$ | H |
| $CO_2Et$ | H | $CH_2Cl$ | H |
| $CO_2Pr$-n | H | $CH_2Cl$ | H |
| $CO_2Bu$-n | H | $CH_2Cl$ | H |
| $CO_2Pen$-n | H | $CH_2Cl$ | H |
| $CH_2Cl$ | H | $CH_2Cl$ | H |
| $CH_2Br$ | H | $CH_2Cl$ | H |
| $CH_2I$ | H | $CH_2Cl$ | H |
| $CH_2CF_3$ | H | $CH_2Cl$ | H |
| $CH_2CN$ | H | $CH_2Cl$ | H |
| $CH_2OH$ | H | $CH_2Cl$ | H |
| $CH_2OMe$ | H | $CH_2Cl$ | H |
| $CH_2OEt$ | H | $CH_2Cl$ | H |
| $CH_2OPr$-n | H | $CH_2Cl$ | H |
| $CH_2SMe$ | H | $CH_2Cl$ | H |
| $CH_2SEt$ | H | $CH_2Cl$ | H |

| $R_3$ | $R_2$ | $R_1$ | $R_4$ |
|---|---|---|---|
| $CH_2SPr$-n | H | $CH_2Cl$ | H |
| $CH_2SO_2Me$ | H | $CH_2Cl$ | H |
| $CH_2SO_2Et$ | H | $CH_2Cl$ | H |
| $CH_2SO_2Pr$-n | H | $CH_2Cl$ | H |
| $CH_2COMe$ | H | $CH_2Cl$ | H |
| $CH_2COEt$ | H | $CH_2Cl$ | H |
| $CH_2COPr$-n | H | $CH_2Cl$ | H |
| $CH_2CO_2H$ | H | $CH_2Cl$ | H |
| $CH_2CO_2Me$ | H | $CH_2Cl$ | H |
| $CH_2CO_2Et$ | H | $CH_2Cl$ | H |
| $CH_2CO_2Pr$-n | H | $CH_2Cl$ | H |
| $CH_2OCH_2CH{=}CH_2$ | H | $CH_2Cl$ | H |
| $CH_2OCH_2C\equiv CH$ | H | $CH_2Cl$ | H |
| $CH{=}CHCO_2Me$ | H | $CH_2Cl$ | H |
| $CH{=}CHCO_2Et$ | H | $CH_2Cl$ | H |
| $CH{=}CHCO_2Pr$-n | H | $CH_2Cl$ | H |
| $CH{=}CHCN$ | H | $CH_2Cl$ | H |
| Ph | H | $CH_2Cl$ | H |
| $CH_2Ph$ | H | $CH_2Cl$ | H |
| OPh | H | $CH_2Cl$ | H |
| SPh | H | $CH_2Cl$ | H |
| $SO_2Ph$ | H | $CH_2Cl$ | H |
| CONHMe | H | $CH_2Cl$ | H |
| CONHEt | H | $CH_2Cl$ | H |
| CONHPr-n | H | $CH_2Cl$ | H |
| CONHPr-i | H | $CH_2Cl$ | H |
| CONHBu-n | H | $CH_2Cl$ | H |
| CONHBu-sec | H | $CH_2Cl$ | H |
| CONHBu-t | H | $CH_2Cl$ | H |
| $CON(Me)_2$ | H | $CH_2Cl$ | H |
| $CON(Et)_2$ | H | $CH_2Cl$ | H |
| $CON(Pr\text{-}n)_2$ | H | $CH_2Cl$ | H |
| F | H | $CH_2OMe$ | H |
| Cl | H | $CH_2OMe$ | H |
| Br | H | $CH_2OMe$ | H |
| I | H | $CH_2OMe$ | H |
| $CH{=}CH_2$ | H | $CH_2OMe$ | H |
| $CH_2CH{=}CH_2$ | H | $CH_2OMe$ | H |
| $C\equiv CH$ | H | $CH_2OMe$ | H |
| $CH_2C\equiv CH$ | H | $CH_2OMe$ | H |

| $R_3$ | $R_2$ | $R_1$ | $R_4$ |
|---|---|---|---|
| $CH_2F$ | H | $CH_2OMe$ | H |
| $CHF_2$ | H | $CH_2OMe$ | H |
| CN | H | $CH_2OMe$ | H |
| $NH_2$ | H | $CH_2OMe$ | H |
| NHCOMe | H | $CH_2OMe$ | H |
| $CH_2NH_2$ | H | $CH_2OMe$ | H |
| $CH_2NHCOMe$ | H | $CH_2OMe$ | H |
| $CH_2OCHF_2$ | H | $CH_2OMe$ | H |
| $CH_2OCF_3$ | H | $CH_2OMe$ | H |
| $CF_3$ | H | $CH_2OMe$ | H |
| $NO_2$ | H | $CH_2OMe$ | H |
| OMe | H | $CH_2OMe$ | H |
| OEt | H | $CH_2OMe$ | H |
| OPr-n | H | $CH_2OMe$ | H |
| OBu-n | H | $CH_2OMe$ | H |
| OPen-n | H | $CH_2OMe$ | H |
| SMe | H | $CH_2OMe$ | H |
| SEt | H | $CH_2OMe$ | H |
| SPr-n | H | $CH_2OMe$ | H |
| SBu-n | H | $CH_2OMe$ | H |
| SPen-n | H | $CH_2OMe$ | H |
| $SO_2Me$ | H | $CH_2OMe$ | H |
| $SO_2Et$ | H | $CH_2OMe$ | H |
| $SO_2Pr$-n | H | $CH_2OMe$ | H |
| COMe | H | $CH_2OMe$ | H |
| COEt | H | $CH_2OMe$ | H |
| COPr-n | H | $CH_2OMe$ | H |
| $CO_2H$ | H | $CH_2OMe$ | H |
| $CO_2Me$ | H | $CH_2OMe$ | H |
| $CO_2Et$ | H | $CH_2OMe$ | H |
| $CO_2Pr$-n | H | $CH_2OMe$ | H |
| $CO_2Bu$-n | H | $CH_2OMe$ | H |
| $CO_2Pen$-n | H | $CH_2OMe$ | H |
| $CH_2Cl$ | H | $CH_2OMe$ | H |
| $CH_2Br$ | H | $CH_2OMe$ | H |
| $CH_2I$ | H | $CH_2OMe$ | H |
| $CH_2CF_3$ | H | $CH_2OMe$ | H |
| $CH_2CN$ | H | $CH_2OMe$ | H |
| $CH_2OH$ | H | $CH_2OMe$ | H |
| $CH_2OMe$ | H | $CH_2OMe$ | H |

| $R_3$ | $R_2$ | $R_1$ | $R_4$ |
|---|---|---|---|
| $CH_2OEt$ | H | $CH_2OMe$ | H |
| $CH_2OPr$-n | H | $CH_2OMe$ | H |
| $CH_2SMe$ | H | $CH_2OMe$ | H |
| $CH_2SEt$ | H | $CH_2OMe$ | H |
| $CH_2SPr$-n | H | $CH_2OMe$ | H |
| $CH_2SO_2Me$ | H | $CH_2OMe$ | H |
| $CH_2SO_2Et$ | H | $CH_2OMe$ | H |
| $CH_2SO_2Pr$-n | H | $CH_2OMe$ | H |
| $CH_2COMe$ | H | $CH_2OMe$ | H |
| $CH_2COEt$ | H | $CH_2OMe$ | H |
| $CH_2COPr$-n | H | $CH_2OMe$ | H |
| $CH_2CO_2H$ | H | $CH_2OMe$ | H |
| $CH_2CO_2Me$ | H | $CH_2OMe$ | H |
| $CH_2CO_2Et$ | H | $CH_2OMe$ | H |
| $CH_2CO_2Pr$-n | H | $CH_2OMe$ | H |
| $CH_2OCH_2CH=CH_2$ | H | $CH_2OMe$ | H |
| $CH_2OCH_2C\equiv CH$ | H | $CH_2OMe$ | H |
| $CH=CHCO_2Me$ | H | $CH_2OMe$ | H |
| $CH=CHCO_2Et$ | H | $CH_2OMe$ | H |
| $CH=CHCO_2Pr$-n | H | $CH_2OMe$ | H |
| CH=CHCN | H | $CH_2OMe$ | H |
| Ph | H | $CH_2OMe$ | H |
| $CH_2Ph$ | H | $CH_2OMe$ | H |
| OPh | H | $CH_2OMe$ | H |
| SPh | H | $CH_2OMe$ | H |
| $SO_2Ph$ | H | $CH_2OMe$ | H |
| CONHMe | H | $CH_2OMe$ | H |
| CONHEt | H | $CH_2OMe$ | H |
| CONHPr-n | H | $CH_2OMe$ | H |
| CONHPr-i | H | $CH_2OMe$ | H |
| CONHBu-n | H | $CH_2OMe$ | H |
| CONHBu-sec | H | $CH_2OMe$ | H |
| CONHBu-t | H | $CH_2OMe$ | H |
| $CON(Me)_2$ | H | $CH_2OMe$ | H |
| $CON(Et)_2$ | H | $CH_2OMe$ | H |
| $CON(Pr$-n$)_2$ | H | $CH_2OMe$ | H |
| F | H | $CH_2SMe$ | H |
| Cl | H | $CH_2SMe$ | H |
| Br | H | $CH_2SMe$ | H |
| I | H | $CH_2SMe$ | H |

| $R_3$ | $R_2$ | $R_1$ | $R_4$ |
|---|---|---|---|
| $CH=CH_2$ | H | $CH_2SMe$ | H |
| $CH_2CH=CH_2$ | H | $CH_2SMe$ | H |
| $C\equiv CH$ | H | $CH_2SMe$ | H |
| $CH_2C\equiv CH$ | H | $CH_2SMe$ | H |
| $CH_2F$ | H | $CH_2SMe$ | H |
| $CHF_2$ | H | $CH_2SMe$ | H |
| CN | H | $CH_2SMe$ | H |
| $NH_2$ | H | $CH_2SMe$ | H |
| NHCOMe | H | $CH_2SMe$ | H |
| $CH_2NH_2$ | H | $CH_2SMe$ | H |
| $CH_2NHCOMe$ | H | $CH_2SMe$ | H |
| $CH_2OCHF_2$ | H | $CH_2SMe$ | H |
| $CH_2OCF_3$ | H | $CH_2SMe$ | H |
| $CF_3$ | H | $CH_2SMe$ | H |
| $NO_2$ | H | $CH_2SMe$ | H |
| OMe | H | $CH_2SMe$ | H |
| OEt | H | $CH_2SMe$ | H |
| OPr-n | H | $CH_2SMe$ | H |
| OBu-n | H | $CH_2SMe$ | H |
| OPen-n | H | $CH_2SMe$ | H |
| SMe | H | $CH_2SMe$ | H |
| SEt | H | $CH_2SMe$ | H |
| SPr-n | H | $CH_2SMe$ | H |
| SBu-n | H | $CH_2SMe$ | H |
| SPen-n | H | $CH_2SMe$ | H |
| $SO_2Me$ | H | $CH_2SMe$ | H |
| $SO_2Et$ | H | $CH_2SMe$ | H |
| $SO_2Pr$-n | H | $CH_2SMe$ | H |
| COMe | H | $CH_2SMe$ | H |
| COEt | H | $CH_2SMe$ | H |
| COPr-n | H | $CH_2SMe$ | H |
| $CO_2H$ | H | $CH_2SMe$ | H |
| $CO_2Me$ | H | $CH_2SMe$ | H |
| $CO_2Et$ | H | $CH_2SMe$ | H |
| $CO_2Pr$-n | H | $CH_2SMe$ | H |
| $CO_2Bu$-n | H | $CH_2SMe$ | H |
| $CO_2Pen$-n | H | $CH_2SMe$ | H |
| $CH_2Cl$ | H | $CH_2SMe$ | H |
| $CH_2Br$ | H | $CH_2SMe$ | H |
| $CH_2I$ | H | $CH_2SMe$ | H |

| $R_3$ | $R_2$ | $R_1$ | $R_4$ |
|---|---|---|---|
| $CH_2CF_3$ | H | $CH_2SMe$ | H |
| $CH_2CN$ | H | $CH_2SMe$ | H |
| $CH_2OH$ | H | $CH_2SMe$ | H |
| $CH_2OMe$ | H | $CH_2SMe$ | H |
| $CH_2OEt$ | H | $CH_2SMe$ | H |
| $CH_2OPr$-n | H | $CH_2SMe$ | H |
| $CH_2SMe$ | H | $CH_2SMe$ | H |
| $CH_2SEt$ | H | $CH_2SMe$ | H |
| $CH_2SPr$-n | H | $CH_2SMe$ | H |
| $CH_2SO_2Me$ | H | $CH_2SMe$ | H |
| $CH_2SO_2Et$ | H | $CH_2SMe$ | H |
| $CH_2SO_2Pr$-n | H | $CH_2SMe$ | H |
| $CH_2COMe$ | H | $CH_2SMe$ | H |
| $CH_2COEt$ | H | $CH_2SMe$ | H |
| $CH_2COPr$-n | H | $CH_2SMe$ | H |
| $CH_2CO_2H$ | H | $CH_2SMe$ | H |
| $CH_2CO_2Me$ | H | $CH_2SMe$ | H |
| $CH_2CO_2Et$ | H | $CH_2SMe$ | H |
| $CH_2CO_2Pr$-n | H | $CH_2SMe$ | H |
| $CH_2OCH_2CH=CH_2$ | H | $CH_2SMe$ | H |
| $CH_2OCH_2C\equiv CH$ | H | $CH_2SMe$ | H |
| $CH=CHCO_2Me$ | H | $CH_2SMe$ | H |
| $CH=CHCO_2Et$ | H | $CH_2SMe$ | H |
| $CH=CHCO_2Pr$-n | H | $CH_2SMe$ | H |
| CH=CHCN | H | $CH_2SMe$ | H |
| Ph | H | $CH_2SMe$ | H |
| $CH_2Ph$ | H | $CH_2SMe$ | H |
| OPh | H | $CH_2SMe$ | H |
| SPh | H | $CH_2SMe$ | H |
| $SO_2Ph$ | H | $CH_2SMe$ | H |
| CONHMe | H | $CH_2SMe$ | H |
| CONHEt | H | $CH_2SMe$ | H |
| CONHPr-n | H | $CH_2SMe$ | H |
| CONHPr-i | H | $CH_2SMe$ | H |
| CONHBu-n | H | $CH_2SMe$ | H |
| CONHBu-sec | H | $CH_2SMe$ | H |
| CONHBu-t | H | $CH_2SMe$ | H |
| $CON(Me)_2$ | H | $CH_2SMe$ | H |
| $CON(Et)_2$ | H | $CH_2SMe$ | H |
| $CON(Pr\text{-}n)_2$ | H | $CH_2SMe$ | H |

| $R_3$ | $R_2$ | $R_1$ | $R_4$ |
|---|---|---|---|
| F | H | $CH_2SO_2Me$ | H |
| Cl | H | $CH_2SO_2Me$ | H |
| Br | H | $CH_2SO_2Me$ | H |
| I | H | $CH_2SO_2Me$ | H |
| $CH=CH_2$ | H | $CH_2SO_2Me$ | H |
| $CH_2CH=CH_2$ | H | $CH_2SO_2Me$ | H |
| $C\equiv CH$ | H | $CH_2SO_2Me$ | H |
| $CH_2C\equiv CH$ | H | $CH_2SO_2Me$ | H |
| $CH_2F$ | H | $CH_2SO_2Me$ | H |
| $CHF_2$ | H | $CH_2SO_2Me$ | H |
| CN | H | $CH_2SO_2Me$ | H |
| $NH_2$ | H | $CH_2SO_2Me$ | H |
| NHCOMe | H | $CH_2SO_2Me$ | H |
| $CH_2NH_2$ | H | $CH_2SO_2Me$ | H |
| $CH_2NHCOMe$ | H | $CH_2SO_2Me$ | H |
| $CH_2OCHF_2$ | H | $CH_2SO_2Me$ | H |
| $CH_2OCF_3$ | H | $CH_2SO_2Me$ | H |
| $CF_3$ | H | $CH_2SO_2Me$ | H |
| $NO_2$ | H | $CH_2SO_2Me$ | H |
| OMe | H | $CH_2SO_2Me$ | H |
| OEt | H | $CH_2SO_2Me$ | H |
| OPr-n | H | $CH_2SO_2Me$ | H |
| OBu-n | H | $CH_2SO_2Me$ | H |
| OPen-n | H | $CH_2SO_2Me$ | H |
| SMe | H | $CH_2SO_2Me$ | H |
| SEt | H | $CH_2SO_2Me$ | H |
| SPr-n | H | $CH_2SO_2Me$ | H |
| SBu-n | H | $CH_2SO_2Me$ | H |
| SPen-n | H | $CH_2SO_2Me$ | H |
| $SO_2Me$ | H | $CH_2SO_2Me$ | H |
| $SO_2Et$ | H | $CH_2SO_2Me$ | H |
| $SO_2Pr$-n | H | $CH_2SO_2Me$ | H |
| COMe | H | $CH_2SO_2Me$ | H |
| COEt | H | $CH_2SO_2Me$ | H |
| COPr-n | H | $CH_2SO_2Me$ | H |
| $CO_2H$ | H | $CH_2SO_2Me$ | H |
| $CO_2Me$ | H | $CH_2SO_2Me$ | H |
| $CO_2Et$ | H | $CH_2SO_2Me$ | H |
| $CO_2Pr$-n | H | $CH_2SO_2Me$ | H |
| $CO_2Bu$-n | H | $CH_2SO_2Me$ | H |

| $R_3$ | $R_2$ | $R_1$ | $R_4$ |
|---|---|---|---|
| $CO_2$Pen-n | H | $CH_2SO_2Me$ | H |
| $CH_2Cl$ | H | $CH_2SO_2Me$ | H |
| $CH_2Br$ | H | $CH_2SO_2Me$ | H |
| $CH_2I$ | H | $CH_2SO_2Me$ | H |
| $CH_2CF_3$ | H | $CH_2SO_2Me$ | H |
| $CH_2CN$ | H | $CH_2SO_2Me$ | H |
| $CH_2OH$ | H | $CH_2SO_2Me$ | H |
| $CH_2OMe$ | H | $CH_2SO_2Me$ | H |
| $CH_2OEt$ | H | $CH_2SO_2Me$ | H |
| $CH_2O$Pr-n | H | $CH_2SO_2Me$ | H |
| $CH_2SMe$ | H | $CH_2SO_2Me$ | H |
| $CH_2SEt$ | H | $CH_2SO_2Me$ | H |
| $CH_2S$Pr-n | H | $CH_2SO_2Me$ | H |
| $CH_2SO_2Me$ | H | $CH_2SO_2Me$ | H |
| $CH_2SO_2Et$ | H | $CH_2SO_2Me$ | H |
| $CH_2SO_2$Pr-n | H | $CH_2SO_2Me$ | H |
| $CH_2COMe$ | H | $CH_2SO_2Me$ | H |
| $CH_2COEt$ | H | $CH_2SO_2Me$ | H |
| $CH_2CO$Pr-n | H | $CH_2SO_2Me$ | H |
| $CH_2CO_2H$ | H | $CH_2SO_2Me$ | H |
| $CH_2CO_2Me$ | H | $CH_2SO_2Me$ | H |
| $CH_2CO_2Et$ | H | $CH_2SO_2Me$ | H |
| $CH_2CO_2$Pr-n | H | $CH_2SO_2Me$ | H |
| $CH_2OCH_2CH=CH_2$ | H | $CH_2SO_2Me$ | H |
| $CH_2OCH_2C\equiv CH$ | H | $CH_2SO_2Me$ | H |
| $CH=CHCO_2Me$ | H | $CH_2SO_2Me$ | H |
| $CH=CHCO_2Et$ | H | $CH_2SO_2Me$ | H |
| $CH=CHCO_2$Pr-n | H | $CH_2SO_2Me$ | H |
| CH=CHCN | H | $CH_2SO_2Me$ | H |
| Ph | H | $CH_2SO_2Me$ | H |
| $CH_2Ph$ | H | $CH_2SO_2Me$ | H |
| OPh | H | $CH_2SO_2Me$ | H |
| SPh | H | $CH_2SO_2Me$ | H |
| $SO_2Ph$ | H | $CH_2SO_2Me$ | H |
| CONHMe | H | $CH_2SO_2Me$ | H |
| CONHEt | H | $CH_2SO_2Me$ | H |
| CONHPr-n | H | $CH_2SO_2Me$ | H |
| CONHPr-i | H | $CH_2SO_2Me$ | H |
| CONHBu-n | H | $CH_2SO_2Me$ | H |
| CONHBu-sec | H | $CH_2SO_2Me$ | H |

| $R_3$ | $R_2$ | $R_1$ | $R_4$ |
|---|---|---|---|
| CONHBu-t | H | $CH_2SO_2Me$ | H |
| $CON(Me)_2$ | H | $CH_2SO_2Me$ | H |
| $CON(Et)_2$ | H | $CH_2SO_2Me$ | H |
| $CON(Pr\text{-}n)_2$ | H | $CH_2SO_2Me$ | H |
| F | H | $CH_2CO_2Me$ | H |
| Cl | H | $CH_2CO_2Me$ | H |
| Br | H | $CH_2CO_2Me$ | H |
| I | H | $CH_2CO_2Me$ | H |
| $CH=CH_2$ | H | $CH_2CO_2Me$ | H |
| $CH_2CH=CH_2$ | H | $CH_2CO_2Me$ | H |
| $C\equiv CH$ | H | $CH_2CO_2Me$ | H |
| $CH_2C\equiv CH$ | H | $CH_2CO_2Me$ | H |
| $CH_2F$ | H | $CH_2CO_2Me$ | H |
| $CHF_2$ | H | $CH_2CO_2Me$ | H |
| CN | H | $CH_2CO_2Me$ | H |
| $NH_2$ | H | $CH_2CO_2Me$ | H |
| NHCOMe | H | $CH_2CO_2Me$ | H |
| $CH_2NH_2$ | H | $CH_2CO_2Me$ | H |
| $CH_2NHCOMe$ | H | $CH_2CO_2Me$ | H |
| $CH_2OCHF_2$ | H | $CH_2CO_2Me$ | H |
| $CH_2OCF_3$ | H | $CH_2CO_2Me$ | H |
| $CF_3$ | H | $CH_2CO_2Me$ | H |
| $NO_2$ | H | $CH_2CO_2Me$ | H |
| OMe | H | $CH_2CO_2Me$ | H |
| OEt | H | $CH_2CO_2Me$ | H |
| OPr-n | H | $CH_2CO_2Me$ | H |
| OBu-n | H | $CH_2CO_2Me$ | H |
| OPen-n | H | $CH_2CO_2Me$ | H |
| SMe | H | $CH_2CO_2Me$ | H |
| SEt | H | $CH_2CO_2Me$ | H |
| SPr-n | H | $CH_2CO_2Me$ | H |
| SBu-n | H | $CH_2CO_2Me$ | H |
| SPen-n | H | $CH_2CO_2Me$ | H |
| $SO_2Me$ | H | $CH_2CO_2Me$ | H |
| $SO_2Et$ | H | $CH_2CO_2Me$ | H |
| $SO_2Pr\text{-}n$ | H | $CH_2CO_2Me$ | H |
| COMe | H | $CH_2CO_2Me$ | H |
| COEt | H | $CH_2CO_2Me$ | H |
| COPr-n | H | $CH_2CO_2Me$ | H |
| $CO_2H$ | H | $CH_2CO_2Me$ | H |

| $R_3$ | $R_2$ | $R_1$ | $R_4$ |
|---|---|---|---|
| $CO_2Me$ | H | $CH_2CO_2Me$ | H |
| $CO_2Et$ | H | $CH_2CO_2Me$ | H |
| $CO_2Pr$-n | H | $CH_2CO_2Me$ | H |
| $CO_2Bu$-n | H | $CH_2CO_2Me$ | H |
| $CO_2Pen$-n | H | $CH_2CO_2Me$ | H |
| $CH_2Cl$ | H | $CH_2CO_2Me$ | H |
| $CH_2Br$ | H | $CH_2CO_2Me$ | H |
| $CH_2I$ | H | $CH_2CO_2Me$ | H |
| $CH_2CF_3$ | H | $CH_2CO_2Me$ | H |
| $CH_2CN$ | H | $CH_2CO_2Me$ | H |
| $CH_2OH$ | H | $CH_2CO_2Me$ | H |
| $CH_2OMe$ | H | $CH_2CO_2Me$ | H |
| $CH_2OEt$ | H | $CH_2CO_2Me$ | H |
| $CH_2OPr$-n | H | $CH_2CO_2Me$ | H |
| $CH_2SMe$ | H | $CH_2CO_2Me$ | H |
| $CH_2SEt$ | H | $CH_2CO_2Me$ | H |
| $CH_2SPr$-n | H | $CH_2CO_2Me$ | H |
| $CH_2SO_2Me$ | H | $CH_2CO_2Me$ | H |
| $CH_2SO_2Et$ | H | $CH_2CO_2Me$ | H |
| $CH_2SO_2Pr$-n | H | $CH_2CO_2Me$ | H |
| $CH_2COMe$ | H | $CH_2CO_2Me$ | H |
| $CH_2COEt$ | H | $CH_2CO_2Me$ | H |
| $CH_2COPr$-n | H | $CH_2CO_2Me$ | H |
| $CH_2CO_2H$ | H | $CH_2CO_2Me$ | H |
| $CH_2CO_2Me$ | H | $CH_2CO_2Me$ | H |
| $CH_2CO_2Et$ | H | $CH_2CO_2Me$ | H |
| $CH_2CO_2Pr$-n | H | $CH_2CO_2Me$ | H |
| $CH_2OCH_2CH=CH_2$ | H | $CH_2CO_2Me$ | H |
| $CH_2OCH_2C\equiv CH$ | H | $CH_2CO_2Me$ | H |
| $CH=CHCO_2Me$ | H | $CH_2CO_2Me$ | H |
| $CH=CHCO_2Et$ | H | $CH_2CO_2Me$ | H |
| $CH=CHCO_2Pr$-n | H | $CH_2CO_2Me$ | H |
| $CH=CHCN$ | H | $CH_2CO_2Me$ | H |
| Ph | H | $CH_2CO_2Me$ | H |
| $CH_2Ph$ | H | $CH_2CO_2Me$ | H |
| OPh | H | $CH_2CO_2Me$ | H |
| SPh | H | $CH_2CO_2Me$ | H |
| $SO_2Ph$ | H | $CH_2CO_2Me$ | H |
| CONHMe | H | $CH_2CO_2Me$ | H |
| CONHEt | H | $CH_2CO_2Me$ | H |

| $R_3$ | $R_2$ | $R_1$ | $R_4$ |
|---|---|---|---|
| CONHPr-n | H | $CH_2CO_2Me$ | H |
| CONHPr-i | H | $CH_2CO_2Me$ | H |
| CONHBu-n | H | $CH_2CO_2Me$ | H |
| CONHBu-sec | H | $CH_2CO_2Me$ | H |
| CONHBu-t | H | $CH_2CO_2Me$ | H |
| $CON(Me)_2$ | H | $CH_2CO_2Me$ | H |
| $CON(Et)_2$ | H | $CH_2CO_2Me$ | H |
| $CON(Pr\text{-}n)_2$ | H | $CH_2CO_2Me$ | H |
| F | H | Ph | H |
| Cl | H | Ph | H |
| Br | H | Ph | H |
| I | H | Ph | H |
| $CH=CH_2$ | H | Ph | H |
| $CH_2CH=CH_2$ | H | Ph | H |
| $C\equiv CH$ | H | Ph | H |
| $CH_2C\equiv CH$ | H | Ph | H |
| $CH_2F$ | H | Ph | H |
| $CHF_2$ | H | Ph | H |
| CN | H | Ph | H |
| $NH_2$ | H | Ph | H |
| NHCOMe | H | Ph | H |
| $CH_2NH_2$ | H | Ph | H |
| $CH_2NHCOMe$ | H | Ph | H |
| $CH_2OCHF_2$ | H | Ph | H |
| $CH_2OCF_3$ | H | Ph | H |
| $CF_3$ | H | Ph | H |
| $NO_2$ | H | Ph | H |
| OMe | H | Ph | H |
| OEt | H | Ph | H |
| OPr-n | H | Ph | H |
| OBu-n | H | Ph | H |
| OPen-n | H | Ph | H |
| SMe | H | Ph | H |
| SEt | H | Ph | H |
| SPr-n | H | Ph | H |
| SBu-n | H | Ph | H |
| SPen-n | H | Ph | H |
| $SO_2Me$ | H | Ph | H |
| $SO_2Et$ | H | Ph | H |
| $SO_2Pr\text{-}n$ | H | Ph | H |

| $R_3$ | $R_2$ | $R_1$ | $R_4$ |
|---|---|---|---|
| COMe | H | Ph | H |
| COEt | H | Ph | H |
| COPr-n | H | Ph | H |
| $CO_2H$ | H | Ph | H |
| $CO_2Me$ | H | Ph | H |
| $CO_2Et$ | H | Ph | H |
| $CO_2Pr$-n | H | Ph | H |
| $CO_2Bu$-n | H | Ph | H |
| $CO_2Pen$-n | H | Ph | H |
| $CH_2Cl$ | H | Ph | H |
| $CH_2Br$ | H | Ph | H |
| $CH_2I$ | H | Ph | H |
| $CH_2CF_3$ | H | Ph | H |
| $CH_2CN$ | H | Ph | H |
| $CH_2OH$ | H | Ph | H |
| $CH_2OMe$ | H | Ph | H |
| $CH_2OEt$ | H | Ph | H |
| $CH_2OPr$-n | H | Ph | H |
| $CH_2SMe$ | H | Ph | H |
| $CH_2SEt$ | H | Ph | H |
| $CH_2SPr$-n | H | Ph | H |
| $CH_2SO_2Me$ | H | Ph | H |
| $CH_2SO_2Et$ | H | Ph | H |
| $CH_2SO_2Pr$-n | H | Ph | H |
| $CH_2COMe$ | H | Ph | H |
| $CH_2COEt$ | H | Ph | H |
| $CH_2COPr$-n | H | Ph | H |
| $CH_2CO_2H$ | H | Ph | H |
| $CH_2CO_2Me$ | H | Ph | H |
| $CH_2CO_2Et$ | H | Ph | H |
| $CH_2CO_2Pr$-n | H | Ph | H |
| $CH_2OCH_2CH=CH_2$ | H | Ph | H |
| $CH_2OCH_2C\equiv CH$ | H | Ph | H |
| $CH=CHCO_2Me$ | H | Ph | H |
| $CH=CHCO_2Et$ | H | Ph | H |
| $CH=CHCO_2Pr$-n | H | Ph | H |
| CH=CHCN | H | Ph | H |
| Ph | H | Ph | H |
| $CH_2Ph$ | H | Ph | H |
| OPh | H | Ph | H |

| $R_3$ | $R_2$ | $R_1$ | $R_4$ |
|---|---|---|---|
| SPh | H | Ph | H |
| $SO_2Ph$ | H | Ph | H |
| CONHMe | H | Ph | H |
| CONHEt | H | Ph | H |
| CONHPr-n | H | Ph | H |
| CONHPr-i | H | Ph | H |
| CONHBu-n | H | Ph | H |
| CONHBu-sec | H | Ph | H |
| CONHBu-t | H | Ph | H |
| $CON(Me)_2$ | H | Ph | H |
| $CON(Et)_2$ | H | Ph | H |
| $CON(Pr\text{-}n)_2$ | H | Ph | H |
| F | H | $CH_2Ph$ | H |
| Cl | H | $CH_2Ph$ | H |
| Br | H | $CH_2Ph$ | H |
| I | H | $CH_2Ph$ | H |
| $CH=CH_2$ | H | $CH_2Ph$ | H |
| $CH_2CH=CH_2$ | H | $CH_2Ph$ | H |
| $C\equiv CH$ | H | $CH_2Ph$ | H |
| $CH_2C\equiv CH$ | H | $CH_2Ph$ | H |
| $CH_2F$ | H | $CH_2Ph$ | H |
| $CHF_2$ | H | $CH_2Ph$ | H |
| CN | H | $CH_2Ph$ | H |
| $NH_2$ | H | $CH_2Ph$ | H |
| NHCOMe | H | $CH_2Ph$ | H |
| $CH_2NH_2$ | H | $CH_2Ph$ | H |
| $CH_2NHCOMe$ | H | $CH_2Ph$ | H |
| $CH_2OCHF_2$ | H | $CH_2Ph$ | H |
| $CH_2OCF_3$ | H | $CH_2Ph$ | H |
| $CF_3$ | H | $CH_2Ph$ | H |
| $NO_2$ | H | $CH_2Ph$ | H |
| OMe | H | $CH_2Ph$ | H |
| OEt | H | $CH_2Ph$ | H |
| OPr-n | H | $CH_2Ph$ | H |
| OBu-n | H | $CH_2Ph$ | H |
| OPen-n | H | $CH_2Ph$ | H |
| SMe | H | $CH_2Ph$ | H |
| SEt | H | $CH_2Ph$ | H |
| SPr-n | H | $CH_2Ph$ | H |
| SBu-n | H | $CH_2Ph$ | H |

| $R_3$ | $R_2$ | $R_1$ | $R_4$ |
|---|---|---|---|
| SPen-n | H | $CH_2Ph$ | H |
| $SO_2Me$ | H | $CH_2Ph$ | H |
| $SO_2Et$ | H | $CH_2Ph$ | H |
| $SO_2Pr$-n | H | $CH_2Ph$ | H |
| COMe | H | $CH_2Ph$ | H |
| COEt | H | $CH_2Ph$ | H |
| COPr-n | H | $CH_2Ph$ | H |
| $CO_2H$ | H | $CH_2Ph$ | H |
| $CO_2Me$ | H | $CH_2Ph$ | H |
| $CO_2Et$ | H | $CH_2Ph$ | H |
| $CO_2Pr$-n | H | $CH_2Ph$ | H |
| $CO_2Bu$-n | H | $CH_2Ph$ | H |
| $CO_2Pen$-n | H | $CH_2Ph$ | H |
| $CH_2Cl$ | H | $CH_2Ph$ | H |
| $CH_2Br$ | H | $CH_2Ph$ | H |
| $CH_2I$ | H | $CH_2Ph$ | H |
| $CH_2CF_3$ | H | $CH_2Ph$ | H |
| $CH_2CN$ | H | $CH_2Ph$ | H |
| $CH_2OH$ | H | $CH_2Ph$ | H |
| $CH_2OMe$ | H | $CH_2Ph$ | H |
| $CH_2OEt$ | H | $CH_2Ph$ | H |
| $CH_2OPr$-n | H | $CH_2Ph$ | H |
| $CH_2SMe$ | H | $CH_2Ph$ | H |
| $CH_2SEt$ | H | $CH_2Ph$ | H |
| $CH_2SPr$-n | H | $CH_2Ph$ | H |
| $CH_2SO_2Me$ | H | $CH_2Ph$ | H |
| $CH_2SO_2Et$ | H | $CH_2Ph$ | H |
| $CH_2SO_2Pr$-n | H | $CH_2Ph$ | H |
| $CH_2COMe$ | H | $CH_2Ph$ | H |
| $CH_2COEt$ | H | $CH_2Ph$ | H |
| $CH_2COPr$-n | H | $CH_2Ph$ | H |
| $CH_2CO_2H$ | H | $CH_2Ph$ | H |
| $CH_2CO_2Me$ | H | $CH_2Ph$ | H |
| $CH_2CO_2Et$ | H | $CH_2Ph$ | H |
| $CH_2CO_2Pr$-n | H | $CH_2Ph$ | H |
| $CH_2OCH_2CH{=}CH_2$ | H | $CH_2Ph$ | H |
| $CH_2OCH_2C{\equiv}CH$ | H | $CH_2Ph$ | H |
| $CH{=}CHCO_2Me$ | H | $CH_2Ph$ | H |
| $CH{=}CHCO_2Et$ | H | $CH_2Ph$ | H |
| $CH{=}CHCO_2Pr$-n | H | $CH_2Ph$ | H |

| $R_3$ | $R_2$ | $R_1$ | $R_4$ |
|---|---|---|---|
| CH=CHCN | H | $CH_2Ph$ | H |
| Ph | H | $CH_2Ph$ | H |
| $CH_2Ph$ | H | $CH_2Ph$ | H |
| OPh | H | $CH_2Ph$ | H |
| SPh | H | $CH_2Ph$ | H |
| $SO_2Ph$ | H | $CH_2Ph$ | H |
| CONHMe | H | $CH_2Ph$ | H |
| CONHEt | H | $CH_2Ph$ | H |
| CONHPr-n | H | $CH_2Ph$ | H |
| CONHPr-i | H | $CH_2Ph$ | H |
| CONHBu-n | H | $CH_2Ph$ | H |
| CONHBu-sec | H | $CH_2Ph$ | H |
| CONHBu-t | H | $CH_2Ph$ | H |
| $CON(Me)_2$ | H | $CH_2Ph$ | H |
| $CON(Et)_2$ | H | $CH_2Ph$ | H |
| $CON(Pr\text{-}n)_2$ | H | $CH_2Ph$ | H |
| F | H | OPh | H |
| Cl | H | OPh | H |
| Br | H | OPh | H |
| I | H | OPh | H |
| $CH=CH_2$ | H | OPh | H |
| $CH_2CH=CH_2$ | H | OPh | H |
| $C\equiv CH$ | H | OPh | H |
| $CH_2C\equiv CH$ | H | OPh | H |
| $CH_2F$ | H | OPh | H |
| $CHF_2$ | H | OPh | H |
| CN | H | OPh | H |
| $NH_2$ | H | OPh | H |
| NHCOMe | H | OPh | H |
| $CH_2NH_2$ | H | OPh | H |
| $CH_2NHCOMe$ | H | OPh | H |
| $CH_2OCHF_2$ | H | OPh | H |
| $CH_2OCF_3$ | H | OPh | H |
| $CF_3$ | H | OPh | H |
| $NO_2$ | H | OPh | H |
| OMe | H | OPh | H |
| OEt | H | OPh | H |
| OPr-n | H | OPh | H |
| OBu-n | H | OPh | H |
| OPen-n | H | OPh | H |

| $R_3$ | $R_2$ | $R_1$ | $R_4$ |
|---|---|---|---|
| SMe | H | OPh | H |
| SEt | H | OPh | H |
| SPr-n | H | OPh | H |
| SBu-n | H | OPh | H |
| SPen-n | H | OPh | H |
| $SO_2Me$ | H | OPh | H |
| $SO_2Et$ | H | OPh | H |
| $SO_2Pr$-n | H | OPh | H |
| COMe | H | OPh | H |
| COEt | H | OPh | H |
| COPr-n | H | OPh | H |
| $CO_2H$ | H | OPh | H |
| $CO_2Me$ | H | OPh | H |
| $CO_2Et$ | H | OPh | H |
| $CO_2Pr$-n | H | OPh | H |
| $CO_2Bu$-n | H | OPh | H |
| $CO_2Pen$-n | H | OPh | H |
| $CH_2Cl$ | H | OPh | H |
| $CH_2Br$ | H | OPh | H |
| $CH_2I$ | H | OPh | H |
| $CH_2CF_3$ | H | OPh | H |
| $CH_2CN$ | H | OPh | H |
| $CH_2OH$ | H | OPh | H |
| $CH_2OMe$ | H | OPh | H |
| $CH_2OEt$ | H | OPh | H |
| $CH_2OPr$-n | H | OPh | H |
| $CH_2SMe$ | H | OPh | H |
| $CH_2SEt$ | H | OPh | H |
| $CH_2SPr$-n | H | OPh | H |
| $CH_2SO_2Me$ | H | OPh | H |
| $CH_2SO_2Et$ | H | OPh | H |
| $CH_2SO_2Pr$-n | H | OPh | H |
| $CH_2COMe$ | H | OPh | H |
| $CH_2COEt$ | H | OPh | H |
| $CH_2COPr$-n | H | OPh | H |
| $CH_2CO_2H$ | H | OPh | H |
| $CH_2CO_2Me$ | H | OPh | H |
| $CH_2CO_2Et$ | H | OPh | H |
| $CH_2CO_2Pr$-n | H | OPh | H |
| $CH_2OCH_2CH=CH_2$ | H | OPh | H |

| $R_3$ | $R_2$ | $R_1$ | $R_4$ |
|---|---|---|---|
| $CH_2OCH_2C\equiv CH$ | H | OPh | H |
| $CH=CHCO_2Me$ | H | OPh | H |
| $CH=CHCO_2Et$ | H | OPh | H |
| $CH=CHCO_2Pr$-n | H | OPh | H |
| CH=CHCN | H | OPh | H |
| Ph | H | OPh | H |
| $CH_2Ph$ | H | OPh | H |
| OPh | H | OPh | H |
| SPh | H | OPh | H |
| $SO_2Ph$ | H | OPh | H |
| CONHMe | H | OPh | H |
| CONHEt | H | OPh | H |
| CONHPr-n | H | OPh | H |
| CONHPr-i | H | OPh | H |
| CONHBu-n | H | OPh | H |
| CONHBu-sec | H | OPh | H |
| CONHBu-t | H | OPh | H |
| $CON(Me)_2$ | H | OPh | H |
| $CON(Et)_2$ | H | OPh | H |
| $CON(Pr\text{-}n)_2$ | H | OPh | H |
| F | H | SPh | H |
| Cl | H | SPh | H |
| Br | H | SPh | H |
| I | H | SPh | H |
| $CH=CH_2$ | H | SPh | H |
| $CH_2CH=CH_2$ | H | SPh | H |
| $C\equiv CH$ | H | SPh | H |
| $CH_2C\equiv CH$ | H | SPh | H |
| $CH_2F$ | H | SPh | H |
| $CHF_2$ | H | SPh | H |
| CN | H | SPh | H |
| $NH_2$ | H | SPh | H |
| NHCOMe | H | SPh | H |
| $CH_2NH_2$ | H | SPh | H |
| $CH_2NHCOMe$ | H | SPh | H |
| $CH_2OCHF_2$ | H | SPh | H |
| $CH_2OCF_3$ | H | SPh | H |
| $CF_3$ | H | SPh | H |
| $NO_2$ | H | SPh | H |
| OMe | H | SPh | H |

| $R_3$ | $R_2$ | $R_1$ | $R_4$ |
|---|---|---|---|
| OEt | H | SPh | H |
| OPr-n | H | SPh | H |
| OBu-n | H | SPh | H |
| OPen-n | H | SPh | H |
| SMe | H | SPh | H |
| SEt | H | SPh | H |
| SPr-n | H | SPh | H |
| SBu-n | H | SPh | H |
| SPen-n | H | SPh | H |
| $SO_2Me$ | H | SPh | H |
| $SO_2Et$ | H | SPh | H |
| $SO_2Pr$-n | H | SPh | H |
| COMe | H | SPh | H |
| COEt | H | SPh | H |
| COPr-n | H | SPh | H |
| $CO_2H$ | H | SPh | H |
| $CO_2Me$ | H | SPh | H |
| $CO_2Et$ | H | SPh | H |
| $CO_2Pr$-n | H | SPh | H |
| $CO_2Bu$-n | H | SPh | H |
| $CO_2Pen$-n | H | SPh | H |
| $CH_2Cl$ | H | SPh | H |
| $CH_2Br$ | H | SPh | H |
| $CH_2I$ | H | SPh | H |
| $CH_2CF_3$ | H | SPh | H |
| $CH_2CN$ | H | SPh | H |
| $CH_2OH$ | H | SPh | H |
| $CH_2OMe$ | H | SPh | H |
| $CH_2OEt$ | H | SPh | H |
| $CH_2OPr$-n | H | SPh | H |
| $CH_2SMe$ | H | SPh | H |
| $CH_2SEt$ | H | SPh | H |
| $CH_2SPr$-n | H | SPh | H |
| $CH_2SO_2Me$ | H | SPh | H |
| $CH_2SO_2Et$ | H | SPh | H |
| $CH_2SO_2Pr$-n | H | SPh | H |
| $CH_2COMe$ | H | SPh | H |
| $CH_2COEt$ | H | SPh | H |
| $CH_2COPr$-n | H | SPh | H |
| $CH_2CO_2H$ | H | SPh | H |

| $R_3$ | $R_2$ | $R_1$ | $R_4$ |
|---|---|---|---|
| $CH_2CO_2Me$ | H | SPh | H |
| $CH_2CO_2Et$ | H | SPh | H |
| $CH_2CO_2Pr$-n | H | SPh | H |
| $CH_2OCH_2CH=CH_2$ | H | SPh | H |
| $CH_2OCH_2C\equiv CH$ | H | SPh | H |
| $CH=CHCO_2Me$ | H | SPh | H |
| $CH=CHCO_2Et$ | H | SPh | H |
| $CH=CHCO_2Pr$-n | H | SPh | H |
| CH=CHCN | H | SPh | H |
| Ph | H | SPh | H |
| $CH_2Ph$ | H | SPh | H |
| OPh | H | SPh | H |
| SPh | H | SPh | H |
| $SO_2Ph$ | H | SPh | H |
| CONHMe | H | SPh | H |
| CONHEt | H | SPh | H |
| CONHPr-n | H | SPh | H |
| CONHPr-i | H | SPh | H |
| CONHBu-n | H | SPh | H |
| CONHBu-sec | H | SPh | H |
| CONHBu-t | H | SPh | H |
| $CON(Me)_2$ | H | SPh | H |
| $CON(Et)_2$ | H | SPh | H |
| $CON(Pr\text{-}n)_2$ | H | SPh | H |
| F | H | $SO_2Ph$ | H |
| Cl | H | $SO_2Ph$ | H |
| Br | H | $SO_2Ph$ | H |
| I | H | $SO_2Ph$ | H |
| $CH=CH_2$ | H | $SO_2Ph$ | H |
| $CH_2CH=CH_2$ | H | $SO_2Ph$ | H |
| $C\equiv CH$ | H | $SO_2Ph$ | H |
| $CH_2C\equiv CH$ | H | $SO_2Ph$ | H |
| $CH_2F$ | H | $SO_2Ph$ | H |
| $CHF_2$ | H | $SO_2Ph$ | H |
| CN | H | $SO_2Ph$ | H |
| $NH_2$ | H | $SO_2Ph$ | H |
| NHCOMe | H | $SO_2Ph$ | H |
| $CH_2NH_2$ | H | $SO_2Ph$ | H |
| $CH_2NHCOMe$ | H | $SO_2Ph$ | H |
| $CH_2OCHF_2$ | H | $SO_2Ph$ | H |

| $R_3$ | $R_2$ | $R_1$ | $R_4$ |
|---|---|---|---|
| $CH_2OCF_3$ | H | $SO_2Ph$ | H |
| $CF_3$ | H | $SO_2Ph$ | H |
| $NO_2$ | H | $SO_2Ph$ | H |
| OMe | H | $SO_2Ph$ | H |
| OEt | H | $SO_2Ph$ | H |
| OPr-n | H | $SO_2Ph$ | H |
| OBu-n | H | $SO_2Ph$ | H |
| OPen-n | H | $SO_2Ph$ | H |
| SMe | H | $SO_2Ph$ | H |
| SEt | H | $SO_2Ph$ | H |
| SPr-n | H | $SO_2Ph$ | H |
| SBu-n | H | $SO_2Ph$ | H |
| SPen-n | H | $SO_2Ph$ | H |
| $SO_2Me$ | H | $SO_2Ph$ | H |
| $SO_2Et$ | H | $SO_2Ph$ | H |
| $SO_2Pr$-n | H | $SO_2Ph$ | H |
| COMe | H | $SO_2Ph$ | H |
| COEt | H | $SO_2Ph$ | H |
| COPr-n | H | $SO_2Ph$ | H |
| $CO_2H$ | H | $SO_2Ph$ | H |
| $CO_2Me$ | H | $SO_2Ph$ | H |
| $CO_2Et$ | H | $SO_2Ph$ | H |
| $CO_2Pr$-n | H | $SO_2Ph$ | H |
| $CO_2Bu$-n | H | $SO_2Ph$ | H |
| $CO_2Pen$-n | H | $SO_2Ph$ | H |
| $CH_2Cl$ | H | $SO_2Ph$ | H |
| $CH_2Br$ | H | $SO_2Ph$ | H |
| $CH_2I$ | H | $SO_2Ph$ | H |
| $CH_2CF_3$ | H | $SO_2Ph$ | H |
| $CH_2CN$ | H | $SO_2Ph$ | H |
| $CH_2OH$ | H | $SO_2Ph$ | H |
| $CH_2OMe$ | H | $SO_2Ph$ | H |
| $CH_2OEt$ | H | $SO_2Ph$ | H |
| $CH_2OPr$-n | H | $SO_2Ph$ | H |
| $CH_2SMe$ | H | $SO_2Ph$ | H |
| $CH_2SEt$ | H | $SO_2Ph$ | H |
| $CH_2SPr$-n | H | $SO_2Ph$ | H |
| $CH_2SO_2Me$ | H | $SO_2Ph$ | H |
| $CH_2SO_2Et$ | H | $SO_2Ph$ | H |
| $CH_2SO_2Pr$-n | H | $SO_2Ph$ | H |

| $R_3$ | $R_2$ | $R_1$ | $R_4$ |
|---|---|---|---|
| $CH_2COMe$ | H | $SO_2Ph$ | H |
| $CH_2COEt$ | H | $SO_2Ph$ | H |
| $CH_2COPr$-n | H | $SO_2Ph$ | H |
| $CH_2CO_2H$ | H | $SO_2Ph$ | H |
| $CH_2CO_2Me$ | H | $SO_2Ph$ | H |
| $CH_2CO_2Et$ | H | $SO_2Ph$ | H |
| $CH_2CO_2Pr$-n | H | $SO_2Ph$ | H |
| $CH_2OCH_2CH=CH_2$ | H | $SO_2Ph$ | H |
| $CH_2OCH_2C\equiv CH$ | H | $SO_2Ph$ | H |
| $CH=CHCO_2Me$ | H | $SO_2Ph$ | H |
| $CH=CHCO_2Et$ | H | $SO_2Ph$ | H |
| $CH=CHCO_2Pr$-n | H | $SO_2Ph$ | H |
| CH=CHCN | H | $SO_2Ph$ | H |
| Ph | H | $SO_2Ph$ | H |
| $CH_2Ph$ | H | $SO_2Ph$ | H |
| OPh | H | $SO_2Ph$ | H |
| SPh | H | $SO_2Ph$ | H |
| $SO_2Ph$ | H | $SO_2Ph$ | H |
| CONHMe | H | $SO_2Ph$ | H |
| CONHEt | H | $SO_2Ph$ | H |
| CONHPr-n | H | $SO_2Ph$ | H |
| CONHPr-i | H | $SO_2Ph$ | H |
| CONHBu-n | H | $SO_2Ph$ | H |
| CONHBu-sec | H | $SO_2Ph$ | H |
| CONHBu-t | H | $SO_2Ph$ | H |
| $CON(Me)_2$ | H | $SO_2Ph$ | H |
| $CON(Et)_2$ | H | $SO_2Ph$ | H |
| $CON(Pr\text{-}n)_2$ | H | $SO_2Ph$ | H |
| F | H | $CON(Et)_2$ | H |
| Cl | H | $CON(Et)_2$ | H |
| Br | H | $CON(Et)_2$ | H |
| I | H | $CON(Et)_2$ | H |
| $CH=CH_2$ | H | $CON(Et)_2$ | H |
| $CH_2CH=CH_2$ | H | $CON(Et)_2$ | H |
| $C\equiv CH$ | H | $CON(Et)_2$ | H |
| $CH_2C\equiv CH$ | H | $CON(Et)_2$ | H |
| $CH_2F$ | H | $CON(Et)_2$ | H |
| $CHF_2$ | H | $CON(Et)_2$ | H |
| CN | H | $CON(Et)_2$ | H |
| $NH_2$ | H | $CON(Et)_2$ | H |

| $R_3$ | $R_2$ | $R_1$ | $R_4$ |
|---|---|---|---|
| NHCOMe | H | $CON(Et)_2$ | H |
| $CH_2NH_2$ | H | $CON(Et)_2$ | H |
| $CH_2NHCOMe$ | H | $CON(Et)_2$ | H |
| $CH_2OCHF_2$ | H | $CON(Et)_2$ | H |
| $CH_2OCF_3$ | H | $CON(Et)_2$ | H |
| $CF_3$ | H | $CON(Et)_2$ | H |
| $NO_2$ | H | $CON(Et)_2$ | H |
| OMe | H | $CON(Et)_2$ | H |
| OEt | H | $CON(Et)_2$ | H |
| OPr-n | H | $CON(Et)_2$ | H |
| OBu-n | H | $CON(Et)_2$ | H |
| OPen-n | H | $CON(Et)_2$ | H |
| SMe | H | $CON(Et)_2$ | H |
| SEt | H | $CON(Et)_2$ | H |
| SPr-n | H | $CON(Et)_2$ | H |
| SBu-n | H | $CON(Et)_2$ | H |
| SPen-n | H | $CON(Et)_2$ | H |
| $SO_2Me$ | H | $CON(Et)_2$ | H |
| $SO_2Et$ | H | $CON(Et)_2$ | H |
| $SO_2Pr$-n | H | $CON(Et)_2$ | H |
| COMe | H | $CON(Et)_2$ | H |
| COEt | H | $CON(Et)_2$ | H |
| COPr-n | H | $CON(Et)_2$ | H |
| $CO_2H$ | H | $CON(Et)_2$ | H |
| $CO_2Me$ | H | $CON(Et)_2$ | H |
| $CO_2Et$ | H | $CON(Et)_2$ | H |
| $CO_2Pr$-n | H | $CON(Et)_2$ | H |
| $CO_2Bu$-n | H | $CON(Et)_2$ | H |
| $CO_2Pen$-n | H | $CON(Et)_2$ | H |
| $CH_2Cl$ | H | $CON(Et)_2$ | H |
| $CH_2Br$ | H | $CON(Et)_2$ | H |
| $CH_2I$ | H | $CON(Et)_2$ | H |
| $CH_2CF_3$ | H | $CON(Et)_2$ | H |
| $CH_2CN$ | H | $CON(Et)_2$ | H |
| $CH_2OH$ | H | $CON(Et)_2$ | H |
| $CH_2OMe$ | H | $CON(Et)_2$ | H |
| $CH_2OEt$ | H | $CON(Et)_2$ | H |
| $CH_2OPr$-n | H | $CON(Et)_2$ | H |
| $CH_2SMe$ | H | $CON(Et)_2$ | H |
| $CH_2SEt$ | H | $CON(Et)_2$ | H |

| $R_3$ | $R_2$ | $R_1$ | $R_4$ |
|---|---|---|---|
| $CH_2SPr$-n | H | $CON(Et)_2$ | H |
| $CH_2SO_2Me$ | H | $CON(Et)_2$ | H |
| $CH_2SO_2Et$ | H | $CON(Et)_2$ | H |
| $CH_2SO_2Pr$-n | H | $CON(Et)_2$ | H |
| $CH_2COMe$ | H | $CON(Et)_2$ | H |
| $CH_2COEt$ | H | $CON(Et)_2$ | H |
| $CH_2COPr$-n | H | $CON(Et)_2$ | H |
| $CH_2CO_2H$ | H | $CON(Et)_2$ | H |
| $CH_2CO_2Me$ | H | $CON(Et)_2$ | H |
| $CH_2CO_2Et$ | H | $CON(Et)_2$ | H |
| $CH_2CO_2Pr$-n | H | $CON(Et)_2$ | H |
| $CH_2OCH_2CH=CH_2$ | H | $CON(Et)_2$ | H |
| $CH_2OCH_2C\equiv CH$ | H | $CON(Et)_2$ | H |
| $CH=CHCO_2Me$ | H | $CON(Et)_2$ | H |
| $CH=CHCO_2Et$ | H | $CON(Et)_2$ | H |
| $CH=CHCO_2Pr$-n | H | $CON(Et)_2$ | H |
| CH=CHCN | H | $CON(Et)_2$ | H |
| Ph | H | $CON(Et)_2$ | H |
| $CH_2Ph$ | H | $CON(Et)_2$ | H |
| OPh | H | $CON(Et)_2$ | H |
| SPh | H | $CON(Et)_2$ | H |
| $SO_2Ph$ | H | $CON(Et)_2$ | H |
| CONHMe | H | $CON(Et)_2$ | H |
| CONHEt | H | $CON(Et)_2$ | H |
| CONHPr-n | H | $CON(Et)_2$ | H |
| CONHPr-i | H | $CON(Et)_2$ | H |
| CONHBu-n | H | $CON(Et)_2$ | H |
| CONHBu-sec | H | $CON(Et)_2$ | H |
| CONHBu-t | H | $CON(Et)_2$ | H |
| $CON(Me)_2$ | H | $CON(Et)_2$ | H |
| $CON(Et)_2$ | H | $CON(Et)_2$ | H |
| $CON(Pr\text{-}n)_2$ | H | $CON(Et)_2$ | H |
| F | H | CN | H |
| Cl | H | CN | H |
| Br | H | CN | H |
| I | H | CN | H |
| $CH=CH_2$ | H | CN | H |
| $CH_2CH=CH_2$ | H | CN | H |
| $C\equiv CH$ | H | CN | H |
| $CH_2C\equiv CH$ | H | CN | H |

| $R_3$ | $R_2$ | $R_1$ | $R_4$ |
|---|---|---|---|
| $CH_2F$ | H | CN | H |
| $CHF_2$ | H | CN | H |
| CN | H | CN | H |
| $NH_2$ | H | CN | H |
| NHCOMe | H | CN | H |
| $CH_2NH_2$ | H | CN | H |
| $CH_2NHCOMe$ | H | CN | H |
| $CH_2OCHF_2$ | H | CN | H |
| $CH_2OCF_3$ | H | CN | H |
| $CF_3$ | H | CN | H |
| $NO_2$ | H | CN | H |
| OMe | H | CN | H |
| OEt | H | CN | H |
| OPr-n | H | CN | H |
| OBu-n | H | CN | H |
| OPen-n | H | CN | H |
| SMe | H | CN | H |
| SEt | H | CN | H |
| SPr-n | H | CN | H |
| SBu-n | H | CN | H |
| SPen-n | H | CN | H |
| $SO_2Me$ | H | CN | H |
| $SO_2Et$ | H | CN | H |
| $SO_2Pr$-n | H | CN | H |
| COMe | H | CN | H |
| COEt | H | CN | H |
| COPr-n | H | CN | H |
| $CO_2H$ | H | CN | H |
| $CO_2Me$ | H | CN | H |
| $CO_2Et$ | H | CN | H |
| $CO_2Pr$-n | H | CN | H |
| $CO_2Bu$-n | H | CN | H |
| $CO_2Pen$-n | H | CN | H |
| $CH_2Cl$ | H | CN | H |
| $CH_2Br$ | H | CN | H |
| $CH_2I$ | H | CN | H |
| $CH_2CF_3$ | H | CN | H |
| $CH_2CN$ | H | CN | H |
| $CH_2OH$ | H | CN | H |
| $CH_2OMe$ | H | CN | H |
| $CH_2OEt$ | H | CN | H |
| $CH_2OPr$-n | H | CN | H |

| $R_3$ | $R_2$ | $R_1$ | $R_4$ |
|---|---|---|---|
| $CH_2SMe$ | H | CN | H |
| $CH_2SEt$ | H | CN | H |
| $CH_2SPr$-n | H | CN | H |
| $CH_2SO_2Me$ | H | CN | H |
| $CH_2SO_2Et$ | H | CN | H |
| $CH_2SO_2Pr$-n | H | CN | H |
| $CH_2COMe$ | H | CN | H |
| $CH_2COEt$ | H | CN | H |
| $CH_2COPr$-n | H | CN | H |
| $CH_2CO_2H$ | H | CN | H |
| $CH_2CO_2Me$ | H | CN | H |
| $CH_2CO_2Et$ | H | CN | H |
| $CH_2CO_2Pr$-n | H | CN | H |
| $CH_2OCH_2CH=CH_2$ | H | CN | H |
| $CH_2OCH_2C\equiv CH$ | H | CN | H |
| $CH=CHCO_2Me$ | H | CN | H |
| $CH=CHCO_2Et$ | H | CN | H |
| $CH=CHCO_2Pr$-n | H | CN | H |
| CH=CHCN | H | CN | H |
| Ph | H | CN | H |
| $CH_2Ph$ | H | CN | H |
| OPh | H | CN | H |
| SPh | H | CN | H |
| H | Br | Me | H |
| Me | Br | H | H |
| $SO_2Ph$ | H | CN | H |
| CONHMe | H | CN | H |
| CONHEt | H | CN | H |
| CONHPr-n | H | CN | H |
| CONHPr-i | H | CN | H |
| CONHBu-n | H | CN | H |
| CONHBu-sec | H | CN | H |
| CONHBu-t | H | CN | H |
| $CON(Me)_2$ | H | CN | H |
| $CON(Et)_2$ | H | CN | H |
| $CON(Pr\text{-}n)_2$ | H | CN | H |
| Cl | Cl | H | H |
| H | Cl | Cl | H |
| Br | Br | H | H |
| Br | H | Br | H |
| H | Br | Br | H |
| H | Cl | Me | H |
| Me | Cl | H | H |

Table 3

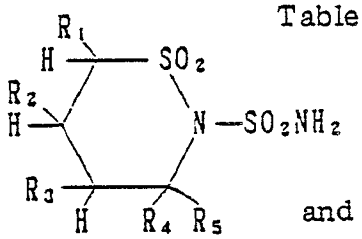

and

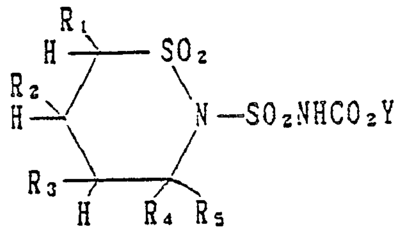

(Y=Me, Et, Pr-n, Bu-n or Ph)

| $R_1$ | $R_2$ | $R_3$ | $R_4$ | $R_5$ |
|---|---|---|---|---|
| H | H | H | H | H |
| Me | H | H | H | H |
| H | Me | H | H | H |
| H | H | Me | H | H |
| H | H | H | Me | H |
| Et | H | H | H | H |
| H | Et | H | H | H |
| H | H | Et | H | H |
| H | H | H | Et | H |
| Pr-n | H | H | H | H |
| H | Pr-n | H | H | H |
| H | H | Pr-n | H | H |
| H | H | H | Pr-n | H |
| Pr-i | H | H | H | H |
| H | Pr-i | H | H | H |
| H | H | Pr-i | H | H |
| H | H | H | Pr-i | H |
| Bu-n | H | H | H | H |
| H | Bu-n | H | H | H |
| H | H | Bu-n | H | H |
| H | H | H | Bu-n | H |
| H | H | H | Me | Me |
| Me | H | H | Me | Me |
| H | Me | H | Me | Me |
| H | H | Me | Me | Me |
| Et | H | H | Me | Me |
| H | Et | H | Me | Me |
| H | H | Et | Me | Me |
| Pr-n | H | H | Me | Me |
| H | Pr-n | H | Me | Me |
| H | H | Pr-n | Me | Me |

| $R_1$ | $R_2$ | $R_3$ | $R_4$ | $R_5$ |
|---|---|---|---|---|
| Pr-i | H | H | Me | Me |
| H | Pr-i | H | Me | Me |
| H | H | Pr-i | Me | Me |
| Bu-n | H | H | Me | Me |
| H | Bu-n | H | Me | Me |
| H | H | Bu-n | Me | Me |
| Me | Me | H | Me | Me |
| Me | H | Me | Me | Me |
| H | Me | Me | Me | Me |
| Me | Me | Me | Me | Me |
| Et | Et | H | Me | Me |
| Et | H | Et | Me | Me |
| H | Et | Et | Me | Me |
| Et | Et | Et | Me | Me |
| Me | H | Et | Me | Me |
| Et | Me | H | Me | Me |
| Et | H | Me | Me | Me |
| Me | Et | H | Me | Me |
| H | Me | Et | Me | Me |
| H | Et | Me | Me | Me |
| H | H | H | Me | Et |
| Me | H | H | Me | Et |
| H | Me | H | Me | Et |
| H | H | Me | Me | Et |
| Et | H | H | Me | Et |
| H | Et | H | Me | Et |
| H | H | Et | Me | Et |
| Pr-n | H | H | Me | Et |
| H | Pr-n | H | Me | Et |
| H | H | Pr-n | Me | Et |
| Pr-i | H | H | Me | Et |
| H | Pr-i | H | Me | Et |
| H | H | Pr-i | Me | Et |
| Bu-n | H | H | Me | Et |
| H | Bu-n | H | Me | Et |
| H | H | Bu-n | Me | Et |
| Me | Me | H | Me | Et |
| Me | H | Me | Me | Et |
| H | Me | Me | Me | Et |
| Me | Me | Me | Me | Et |

| $R_1$ | $R_2$ | $R_3$ | $R_4$ | $R_5$ |
|---|---|---|---|---|
| Et | Et | H | Me | Et |
| Et | H | Et | Me | Et |
| H | Et | Et | Me | Et |
| Et | Et | Et | Me | Et |
| Me | H | Et | Me | Et |
| Et | Me | H | Me | Et |
| Et | H | Me | Me | Et |
| Me | Et | H | Me | Et |
| H | Me | Et | Me | Et |
| H | Et | Me | Me | Et |
| H | H | H | Me | Pr-n |
| Me | H | H | Me | Pr-n |
| H | Me | H | Me | Pr-n |
| H | H | Me | Me | Pr-n |
| Et | H | H | Me | Pr-n |
| H | Et | H | Me | Pr-n |
| H | H | Et | Me | Pr-n |
| Pr-n | H | H | Me | Pr-n |
| H | Pr-n | H | Me | Pr-n |
| H | H | Pr-n | Me | Pr-n |
| Pr-i | H | H | Me | Pr-n |
| H | Pr-i | H | Me | Pr-n |
| H | H | Pr-i | Me | Pr-n |
| Bu-n | H | H | Me | Pr-n |
| H | Bu-n | H | Me | Pr-n |
| H | H | Bu-n | Me | Pr-n |
| Me | Me | H | Me | Pr-n |
| Me | H | Me | Me | Pr-n |
| H | Me | Me | Me | Pr-n |
| Me | Me | Me | Me | Pr-n |
| Et | Et | H | Me | Pr-n |
| Et | H | Et | Me | Pr-n |
| H | Et | Et | Me | Pr-n |
| Et | Et | Et | Me | Pr-n |
| Me | H | Et | Me | Pr-n |
| Et | Me | H | Me | Pr-n |
| Et | H | Me | Me | Pr-n |
| Me | Et | H | Me | Pr-n |
| H | Me | Et | Me | Pr-n |
| H | Et | Me | Me | Pr-n |

| $R_1$ | $R_2$ | $R_3$ | $R_4$ | $R_5$ |
|---|---|---|---|---|
| H | H | H | Me | Pr-i |
| Me | H | H | Me | Pr-i |
| H | Me | H | Me | Pr-i |
| H | H | Me | Me | Pr-i |
| Et | H | H | Me | Pr-i |
| H | Et | H | Me | Pr-i |
| H | H | Et | Me | Pr-i |
| Pr-n | H | H | Me | Pr-i |
| H | Pr-n | H | Me | Pr-i |
| H | H | Pr-n | Me | Pr-i |
| Pr-i | H | H | Me | Pr-i |
| H | Pr-i | H | Me | Pr-i |
| H | H | Pr-i | Me | Pr-i |
| Bu-n | H | H | Me | Pr-i |
| H | Bu-n | H | Me | Pr-i |
| H | H | Bu-n | Me | Pr-i |
| Me | Me | H | Me | Pr-i |
| Me | H | Me | Me | Pr-i |
| H | Me | Me | Me | Pr-i |
| Me | Me | Me | Me | Pr-i |
| Et | Et | H | Me | Pr-i |
| Et | H | Et | Me | Pr-i |
| H | Et | Et | Me | Pr-i |
| Et | Et | Et | Me | Pr-i |
| Me | H | Et | Me | Pr-i |
| Et | Me | H | Me | Pr-i |
| Et | H | Me | Me | Pr-i |
| Me | Et | H | Me | Pr-i |
| H | Me | Et | Me | Pr-i |
| H | Et | Me | Me | Pr-i |
| H | H | H | Et | Et |
| Me | H | H | Et | Et |
| H | Me | H | Et | Et |
| H | H | Me | Et | Et |
| Et | H | H | Et | Et |
| H | Et | H | Et | Et |
| H | H | Et | Et | Et |
| Pr-n | H | H | Et | Et |
| H | Pr-n | H | Et | Et |
| H | H | Pr-n | Et | Et |

| $R_1$ | $R_2$ | $R_3$ | $R_4$ | $R_5$ |
|---|---|---|---|---|
| Pr-i | H | H | Et | Et |
| H | Pr-i | H | Et | Et |
| H | H | Pr-i | Et | Et |
| Bu-n | H | H | Et | Et |
| H | Bu-n | H | Et | Et |
| H | H | Bu-n | Et | Et |
| Me | Me | H | Et | Et |
| Me | H | Me | Et | Et |
| H | Me | Me | Et | Et |
| Me | Me | Me | Et | Et |
| Et | Et | H | Et | Et |
| Et | H | Et | Et | Et |
| H | Et | Et | Et | Et |
| Et | Et | Et | Et | Et |
| Me | H | Et | Et | Et |
| Et | Me | H | Et | Et |
| Et | H | Me | Et | Et |
| Me | Et | H | Et | Et |
| H | Me | Et | Et | Et |
| H | Et | Me | Et | Et |
| H | H | H | Et | Et |
| F | H | H | Me | Me |
| Cl | H | H | Me | Me |
| Br | H | H | Me | Me |
| I | H | H | Me | Me |
| $CH=CH_2$ | H | H | Me | Me |
| $CH_2CH=CH_2$ | H | H | Me | Me |
| $C\equiv CH$ | H | H | Me | Me |
| $CH_2C\equiv CH$ | H | H | Me | Me |
| $CH_2F$ | H | H | Me | Me |
| $CHF_2$ | H | H | Me | Me |
| CN | H | H | Me | Me |
| $NH_2$ | H | H | Me | Me |
| NHCOMe | H | H | Me | Me |
| $CH_2NH_2$ | H | H | Me | Me |
| $CH_2NHCOMe$ | H | H | Me | Me |
| $CH_2OCHF_2$ | H | H | Me | Me |
| $CH_2OCF_3$ | H | H | Me | Me |
| $CF_3$ | H | H | Me | Me |
| $NO_2$ | H | H | Me | Me |

| $R_1$ | $R_2$ | $R_3$ | $R_4$ | $R_5$ |
|---|---|---|---|---|
| OMe | H | H | Me | Me |
| OEt | H | H | Me | Me |
| OPr-n | H | H | Me | Me |
| SMe | H | H | Me | Me |
| SEt | H | H | Me | Me |
| SPr-n | H | H | Me | Me |
| SBu-n | H | H | Me | Me |
| SPen-n | H | H | Me | Me |
| $SO_2Me$ | H | H | Me | Me |
| $SO_2Et$ | H | H | Me | Me |
| $SO_2Pr$-n | H | H | Me | Me |
| COMe | H | H | Me | Me |
| COEt | H | H | Me | Me |
| COPr-n | H | H | Me | Me |
| $CO_2H$ | H | H | Me | Me |
| $CO_2Me$ | H | H | Me | Me |
| $CO_2Et$ | H | H | Me | Me |
| $CO_2Pr$-n | H | H | Me | Me |
| $CO_2Pr$-i | H | H | Me | Me |
| $CO_2Bu$-n | H | H | Me | Me |
| $CO_2Bu$-t | H | H | Me | Me |
| $CO_2Bu$-sec | H | H | Me | Me |
| $CO_2Pen$-n | H | H | Me | Me |
| $CH_2Cl$ | H | H | Me | Me |
| $CH_2Br$ | H | H | Me | Me |
| $CH_2I$ | H | H | Me | Me |
| $CH_2CF_3$ | H | H | Me | Me |
| $CH_2CN$ | H | H | Me | Me |
| $CH_2OH$ | H | H | Me | Me |
| $CH_2OMe$ | H | H | Me | Me |
| $CH_2OEt$ | H | H | Me | Me |
| $CH_2OPr$-n | H | H | Me | Me |
| $CH_2OPr$-i | H | H | Me | Me |
| $CH_2SMe$ | H | H | Me | Me |
| $CH_2SEt$ | H | H | Me | Me |
| $CH_2SPr$-n | H | H | Me | Me |
| $CH_2SPr$-i | H | H | Me | Me |
| $CH_2SO_2Me$ | H | H | Me | Me |
| $CH_2SO_2Et$ | H | H | Me | Me |
| $CH_2SO_2Pr$-n | H | H | Me | Me |

| $R_1$ | $R_2$ | $R_3$ | $R_4$ | $R_5$ |
|---|---|---|---|---|
| $CH_2SO_2Pr$-i | H | H | Me | Me |
| $CH_2COMe$ | H | H | Me | Me |
| $CH_2COEt$ | H | H | Me | Me |
| $CH_2COPr$-n | H | H | Me | Me |
| $CH_2COPr$-i | H | H | Me | Me |
| $CH_2CO_2H$ | H | H | Me | Me |
| $CH_2CO_2Me$ | H | H | Me | Me |
| $CH_2CO_2Et$ | H | H | Me | Me |
| $CH_2CO_2Pr$-n | H | H | Me | Me |
| $CH_2CO_2Pr$-i | H | H | Me | Me |
| $CH_2OCH_2CH=CH_2$ | H | H | Me | Me |
| $CH_2OCH_2C\equiv CH$ | H | H | Me | Me |
| $CH=CHCO_2Me$ | H | H | Me | Me |
| $CH=CHCO_2Et$ | H | H | Me | Me |
| $CH=CHCO_2Pr$-n | H | H | Me | Me |
| $CH=CHCO_2Pr$-i | H | H | Me | Me |
| CH=CHCN | H | H | Me | Me |
| Ph | H | H | Me | Me |
| $CH_2Ph$ | H | H | Me | Me |
| OPh | H | H | Me | Me |
| SPh | H | H | Me | Me |
| $SO_2Ph$ | H | H | Me | Me |
| CONHMe | H | H | Me | Me |
| CONHEt | H | H | Me | Me |
| CONHPr-n | H | H | Me | Me |
| CONHPr-i | H | H | Me | Me |
| CONHBu-n | H | H | Me | Me |
| CONHBu-sec | H | H | Me | Me |
| CONHBu-t | H | H | Me | Me |
| $CON(Me)_2$ | H | H | Me | Me |
| $CON(Et)_2$ | H | H | Me | Me |
| $CON(Pr\text{-}n)_2$ | H | H | Me | Me |
| F | H | H | Me | Et |
| Cl | H | H | Me | Et |
| Br | H | H | Me | Et |
| I | H | H | Me | Et |
| $CH=CH_2$ | H | H | Me | Et |
| $CH_2CH=CH_2$ | H | H | Me | Et |
| $C\equiv CH$ | H | H | Me | Et |
| $CH_2C\equiv CH$ | H | H | Me | Et |

| $R_1$ | $R_2$ | $R_3$ | $R_4$ | $R_5$ |
|---|---|---|---|---|
| $CH_2F$ | H | H | Me | Et |
| $CHF_2$ | H | H | Me | Et |
| CN | H | H | Me | Et |
| $NH_2$ | H | H | Me | Et |
| NHCOMe | H | H | Me | Et |
| $CH_2NH_2$ | H | H | Me | Et |
| $CH_2NHCOMe$ | H | H | Me | Et |
| $CH_2OCHF_2$ | H | H | Me | Et |
| $CH_2OCF_3$ | H | H | Me | Et |
| $CF_3$ | H | H | Me | Et |
| $NO_2$ | H | H | Me | Et |
| OMe | H | H | Me | Et |
| OEt | H | H | Me | Et |
| OPr-n | H | H | Me | Et |
| SMe | H | H | Me | Et |
| SEt | H | H | Me | Et |
| SPr-n | H | H | Me | Et |
| SBu-n | H | H | Me | Et |
| SPen-n | H | H | Me | Et |
| $SO_2Me$ | H | H | Me | Et |
| $SO_2Et$ | H | H | Me | Et |
| $SO_2Pr$-n | H | H | Me | Et |
| COMe | H | H | Me | Et |
| COEt | H | H | Me | Et |
| COPr-n | H | H | Me | Et |
| $CO_2H$ | H | H | Me | Et |
| $CO_2Me$ | H | H | Me | Et |
| $CO_2Et$ | H | H | Me | Et |
| $CO_2Pr$-n | H | H | Me | Et |
| $CO_2Pr$-i | H | H | Me | Et |
| $CO_2Bu$-n | H | H | Me | Et |
| $CO_2Bu$-t | H | H | Me | Et |
| $CO_2Bu$-sec | H | H | Me | Et |
| $CO_2Pen$-n | H | H | Me | Et |
| $CH_2Cl$ | H | H | Me | Et |
| $CH_2Br$ | H | H | Me | Et |
| $CH_2I$ | H | H | Me | Et |
| $CH_2CF_3$ | H | H | Me | Et |
| $CH_2CN$ | H | H | Me | Et |
| $CH_2OH$ | H | H | Me | Et |

| $R_1$ | $R_2$ | $R_3$ | $R_4$ | $R_5$ |
|---|---|---|---|---|
| $CH_2OMe$ | H | H | Me | Et |
| $CH_2OEt$ | H | H | Me | Et |
| $CH_2OPr-n$ | H | H | Me | Et |
| $CH_2OPr-i$ | H | H | Me | Et |
| $CH_2SMe$ | H | H | Me | Et |
| $CH_2SEt$ | H | H | Me | Et |
| $CH_2SPr-n$ | H | H | Me | Et |
| $CH_2SPr-i$ | H | H | Me | Et |
| $CH_2SO_2Me$ | H | H | Me | Et |
| $CH_2SO_2Et$ | H | H | Me | Et |
| $CH_2SO_2Pr-n$ | H | H | Me | Et |
| $CH_2SO_2Pr-i$ | H | H | Me | Et |
| $CH_2COMe$ | H | H | Me | Et |
| $CH_2COEt$ | H | H | Me | Et |
| $CH_2COPr-n$ | H | H | Me | Et |
| $CH_2COPr-i$ | H | H | Me | Et |
| $CH_2CO_2H$ | H | H | Me | Et |
| $CH_2CO_2Me$ | H | H | Me | Et |
| $CH_2CO_2Et$ | H | H | Me | Et |
| $CH_2CO_2Pr-n$ | H | H | Me | Et |
| $CH_2CO_2Pr-i$ | H | H | Me | Et |
| $CH_2OCH_2CH=CH_2$ | H | H | Me | Et |
| $CH_2OCH_2C\equiv CH$ | H | H | Me | Et |
| $CH=CHCO_2Me$ | H | H | Me | Et |
| $CH=CHCO_2Et$ | H | H | Me | Et |
| $CH=CHCO_2Pr-n$ | H | H | Me | Et |
| $CH=CHCO_2Pr-i$ | H | H | Me | Et |
| CH=CHCN | H | H | Me | Et |
| Ph | H | H | Me | Et |
| $CH_2Ph$ | H | H | Me | Et |
| OPh | H | H | Me | Et |
| SPh | H | H | Me | Et |
| $SO_2Ph$ | H | H | Me | Et |
| CONHMe | H | H | Me | Et |
| CONHEt | H | H | Me | Et |
| CONHPr-n | H | H | Me | Et |
| CONHPr-i | H | H | Me | Et |
| CONHBu-n | H | H | Me | Et |
| CONHBu-sec | H | H | Me | Et |
| CONHBu-t | H | H | Me | Et |

| $R_1$ | $R_2$ | $R_3$ | $R_4$ | $R_5$ |
|---|---|---|---|---|
| $CON(Me)_2$ | H | H | Me | Et |
| $CON(Et)_2$ | H | H | Me | Et |
| $CON(Pr\text{-}n)_2$ | H | H | Me | Et |
| H | F | H | Me | Me |
| H | Cl | H | Me | Me |
| H | Br | H | Me | Me |
| H | I | H | Me | Me |
| H | $CH=CH_2$ | H | Me | Me |
| H | $CH_2CH=CH_2$ | H | Me | Me |
| H | $C\equiv CH$ | H | Me | Me |
| H | $CH_2C\equiv CH$ | H | Me | Me |
| H | $CH_2F$ | H | Me | Me |
| H | $CHF_2$ | H | Me | Me |
| H | CN | H | Me | Me |
| H | $NH_2$ | H | Me | Me |
| H | NHCOMe | H | Me | Me |
| H | $CH_2NH_2$ | H | Me | Me |
| H | $CH_2NHCOMe$ | H | Me | Me |
| H | $CH_2OCHF_2$ | H | Me | Me |
| H | $CH_2OCF_3$ | H | Me | Me |
| H | $CF_3$ | H | Me | Me |
| H | $NO_2$ | H | Me | Me |
| H | OMe | H | Me | Me |
| H | OEt | H | Me | Me |
| H | OPr-n | H | Me | Me |
| H | SMe | H | Me | Me |
| H | SEt | H | Me | Me |
| H | SPr-n | H | Me | Me |
| H | SBu-n | H | Me | Me |
| H | SPen-n | H | Me | Me |
| H | $SO_2Me$ | H | Me | Me |
| H | $SO_2Et$ | H | Me | Me |
| H | $SO_2Pr\text{-}n$ | H | Me | Me |
| H | COMe | H | Me | Me |
| H | COEt | H | Me | Me |
| H | COPr-n | H | Me | Me |
| H | $CO_2H$ | H | Me | Me |
| H | $CO_2Me$ | H | Me | Me |
| H | $CO_2Et$ | H | Me | Me |
| H | $CO_2Pr\text{-}n$ | H | Me | Me |

| $R_1$ | $R_2$ | $R_3$ | $R_4$ | $R_5$ |
|---|---|---|---|---|
| H | $CO_2Pr$-i | H | Me | Me |
| H | $CO_2Bu$-n | H | Me | Me |
| H | $CO_2Bu$-t | H | Me | Me |
| H | $CO_2Bu$-sec | H | Me | Me |
| H | $CO_2Pen$-n | H | Me | Me |
| H | $CH_2Cl$ | H | Me | Me |
| H | $CH_2Br$ | H | Me | Me |
| H | $CH_2I$ | H | Me | Me |
| H | $CH_2CF_3$ | H | Me | Me |
| H | $CH_2CN$ | H | Me | Me |
| H | $CH_2OH$ | H | Me | Me |
| H | $CH_2OMe$ | H | Me | Me |
| H | $CH_2OEt$ | H | Me | Me |
| H | $CH_2OPr$-n | H | Me | Me |
| H | $CH_2OPr$-i | H | Me | Me |
| H | $CH_2SMe$ | H | Me | Me |
| H | $CH_2SEt$ | H | Me | Me |
| H | $CH_2SPr$-n | H | Me | Me |
| H | $CH_2SPr$-i | H | Me | Me |
| H | $CH_2SO_2Me$ | H | Me | Me |
| H | $CH_2SO_2Et$ | H | Me | Me |
| H | $CH_2SO_2Pr$-n | H | Me | Me |
| H | $CH_2SO_2Pr$-i | H | Me | Me |
| H | $CH_2COMe$ | H | Me | Me |
| H | $CH_2COEt$ | H | Me | Me |
| H | $CH_2COPr$-n | H | Me | Me |
| H | $CH_2COPr$-i | H | Me | Me |
| H | $CH_2CO_2H$ | H | Me | Me |
| H | $CH_2CO_2Me$ | H | Me | Me |
| H | $CH_2CO_2Et$ | H | Me | Me |
| H | $CH_2CO_2Pr$-n | H | Me | Me |
| H | $CH_2CO_2Pr$-i | H | Me | Me |
| H | $CH_2OCH_2CH=CH_2$ | H | Me | Me |
| H | $CH_2OCH_2C\equiv CH$ | H | Me | Me |
| H | $CH=CHCO_2Me$ | H | Me | Me |
| H | $CH=CHCO_2Et$ | H | Me | Me |
| H | $CH=CHCO_2Pr$-n | H | Me | Me |
| H | $CH=CHCO_2Pr$-i | H | Me | Me |
| H | $CH=CHCN$ | H | Me | Me |
| H | Ph | H | Me | Me |

| $R_1$ | $R_2$ | $R_3$ | $R_4$ | $R_5$ |
|---|---|---|---|---|
| H | $CH_2Ph$ | H | Me | Me |
| H | OPh | H | Me | Me |
| H | SPh | H | Me | Me |
| H | $SO_2Ph$ | H | Me | Me |
| H | CONHMe | H | Me | Me |
| H | CONHEt | H | Me | Me |
| H | CONHPr-n | H | Me | Me |
| H | CONHPr-i | H | Me | Me |
| H | CONHBu-n | H | Me | Me |
| H | CONHBu-sec | H | Me | Me |
| H | CONHBu-t | H | Me | Me |
| H | $CON(Me)_2$ | H | Me | Me |
| H | $CON(Et)_2$ | H | Me | Me |
| H | $CON(Pr\text{-}n)_2$ | H | Me | Me |
| H | H | F | Me | Me |
| H | H | Cl | Me | Me |
| H | H | Br | Me | Me |
| H | H | I | Me | Me |
| H | H | $CH{=}CH_2$ | Me | Me |
| H | H | $CH_2CH{=}CH_2$ | Me | Me |
| H | H | $C\equiv CH$ | Me | Me |
| H | H | $CH_2C\equiv CH$ | Me | Me |
| H | H | $CH_2F$ | Me | Me |
| H | H | $CHF_2$ | Me | Me |
| H | H | CN | Me | Me |
| H | H | $NH_2$ | Me | Me |
| H | H | NHCOMe | Me | Me |
| H | H | $CH_2NH_2$ | Me | Me |
| H | H | $CH_2NHCOMe$ | Me | Me |
| H | H | $CH_2OCHF_2$ | Me | Me |
| H | H | $CH_2OCF_3$ | Me | Me |
| H | H | $CF_3$ | Me | Me |
| H | H | $NO_2$ | Me | Me |
| H | H | OMe | Me | Me |
| H | H | OEt | Me | Me |
| H | H | OPr-n | Me | Me |
| H | H | SMe | Me | Me |
| H | H | SEt | Me | Me |
| H | H | SPr-n | Me | Me |
| H | H | SBu-n | Me | Me |

| $R_1$ | $R_2$ | $R_3$ | $R_4$ | $R_5$ |
|---|---|---|---|---|
| H | H | SPen-n | Me | Me |
| H | H | $SO_2Me$ | Me | Me |
| H | H | $SO_2Et$ | Me | Me |
| H | H | $SO_2Pr$-n | Me | Me |
| H | H | COMe | Me | Me |
| H | H | COEt | Me | Me |
| H | H | COPr-n | Me | Me |
| H | H | $CO_2H$ | Me | Me |
| H | H | $CO_2Me$ | Me | Me |
| H | H | $CO_2Et$ | Me | Me |
| H | H | $CO_2Pr$-n | Me | Me |
| H | H | $CO_2Pr$-i | Me | Me |
| H | H | $CO_2Bu$-n | Me | Me |
| H | H | $CO_2Bu$-t | Me | Me |
| H | H | $CO_2Bu$-sec | Me | Me |
| H | H | $CO_2Pen$-n | Me | Me |
| H | H | $CH_2Cl$ | Me | Me |
| H | H | $CH_2Br$ | Me | Me |
| H | H | $CH_2I$ | Me | Me |
| H | H | $CH_2CF_3$ | Me | Me |
| H | H | $CH_2CN$ | Me | Me |
| H | H | $CH_2OH$ | Me | Me |
| H | H | $CH_2OMe$ | Me | Me |
| H | H | $CH_2OEt$ | Me | Me |
| H | H | $CH_2OPr$-n | Me | Me |
| H | H | $CH_2OPr$-i | Me | Me |
| H | H | $CH_2SMe$ | Me | Me |
| H | H | $CH_2SEt$ | Me | Me |
| H | H | $CH_2SPr$-n | Me | Me |
| H | H | $CH_2SPr$-i | Me | Me |
| H | H | $CH_2SO_2Me$ | Me | Me |
| H | H | $CH_2SO_2Et$ | Me | Me |
| H | H | $CH_2SO_2Pr$-n | Me | Me |
| H | H | $CH_2SO_2Pr$-i | Me | Me |
| H | H | $CH_2COMe$ | Me | Me |
| H | H | $CH_2COEt$ | Me | Me |
| H | H | $CH_2COPr$-n | Me | Me |
| H | H | $CH_2COPr$-i | Me | Me |
| H | H | $CH_2CO_2H$ | Me | Me |
| H | H | $CH_2CO_2Me$ | Me | Me |

| $R_1$ | $R_2$ | $R_3$ | $R_4$ | $R_5$ |
|---|---|---|---|---|
| H | H | $CH_2CO_2Et$ | Me | Me |
| H | H | $CH_2CO_2Pr$-n | Me | Me |
| H | H | $CH_2CO_2Pr$-i | Me | Me |
| H | H | $CH_2OCH_2CH=CH_2$ | Me | Me |
| H | H | $CH_2OCH_2C\equiv CH$ | Me | Me |
| H | H | $CH=CHCO_2Me$ | Me | Me |
| H | H | $CH=CHCO_2Et$ | Me | Me |
| H | H | $CH=CHCO_2Pr$-n | Me | Me |
| H | H | $CH=CHCO_2Pr$-i | Me | Me |
| H | H | CH=CHCN | Me | Me |
| H | H | Ph | Me | Me |
| H | H | $CH_2Ph$ | Me | Me |
| H | H | OPh | Me | Me |
| H | H | SPh | Me | Me |
| H | H | $SO_2Ph$ | Me | Me |
| H | H | CONHMe | Me | Me |
| H | H | CONHEt | Me | Me |
| H | H | CONHPr-n | Me | Me |
| H | H | CONHPr-i | Me | Me |
| H | H | CONHBu-n | Me | Me |
| H | H | CONHBu-sec | Me | Me |
| H | H | CONHBu-t | Me | Me |
| H | H | $CON(Me)_2$ | Me | Me |
| H | H | $CON(Et)_2$ | Me | Me |
| H | H | $CON(Pr\text{-}n)_2$ | Me | Me |
| H | H | H | F | H |
| H | H | H | Cl | H |
| H | H | H | Br | H |
| H | H | H | I | H |
| H | H | H | $CH=CH_2$ | H |
| H | H | H | $CH_2CH=CH_2$ | H |
| H | H | H | $C\equiv CH$ | H |
| H | H | H | $CH_2C\equiv CH$ | H |
| H | H | H | $CH_2F$ | H |
| H | H | H | $CHF_2$ | H |
| H | H | H | CN | H |
| H | H | H | $NH_2$ | H |
| H | H | H | NHCOMe | H |
| H | H | H | $CH_2NH_2$ | H |
| H | H | H | $CH_2NHCOMe$ | H |

| $R_1$ | $R_2$ | $R_3$ | $R_4$ | $R_5$ |
|---|---|---|---|---|
| H | H | H | $CH_2OCHF_2$ | H |
| H | H | H | $CH_2OCF_3$ | H |
| H | H | H | $CF_3$ | H |
| H | H | H | $NO_2$ | H |
| H | H | H | OMe | H |
| H | H | H | OEt | H |
| H | H | H | OPr-n | H |
| H | H | H | SMe | H |
| H | H | H | SEt | H |
| H | H | H | SPr-n | H |
| H | H | H | SBu-n | H |
| H | H | H | SPen-n | H |
| H | H | H | $SO_2Me$ | H |
| H | H | H | $SO_2Et$ | H |
| H | H | H | $SO_2Pr$-n | H |
| H | H | H | COMe | H |
| H | H | H | COEt | H |
| H | H | H | COPr-n | H |
| H | H | H | $CO_2H$ | H |
| H | H | H | $CO_2Me$ | H |
| H | H | H | $CO_2Et$ | H |
| H | H | H | $CO_2Pr$-n | H |
| H | H | H | $CO_2Pr$-i | H |
| H | H | H | $CO_2Bu$-n | H |
| H | H | H | $CO_2Bu$-t | H |
| H | H | H | $CO_2Bu$-sec | H |
| H | H | H | $CO_2Pen$-n | H |
| H | H | H | $CH_2Cl$ | H |
| H | H | H | $CH_2Br$ | H |
| H | H | H | $CH_2I$ | H |
| H | H | H | $CH_2CF_3$ | H |
| H | H | H | $CH_2CN$ | H |
| H | H | H | $CH_2OH$ | H |
| H | H | H | $CH_2OMe$ | H |
| H | H | H | $CH_2OEt$ | H |
| H | H | H | $CH_2OPr$-n | H |

| $R_1$ | $R_2$ | $R_3$ | $R_4$ | $R_5$ |
|---|---|---|---|---|
| H | H | H | $CH_2OPr$-i | H |
| H | H | H | $CH_2SMe$ | H |
| H | H | H | $CH_2SEt$ | H |
| H | H | H | $CH_2SPr$-n | H |
| H | H | H | $CH_2SPr$-i | H |
| H | H | H | $CH_2SO_2Me$ | H |
| H | H | H | $CH_2SO_2Et$ | H |
| H | H | H | $CH_2SO_2Pr$-n | H |
| H | H | H | $CH_2SO_2Pr$-i | H |
| H | H | H | $CH_2COMe$ | H |
| H | H | H | $CH_2COEt$ | H |
| H | H | H | $CH_2COPr$-n | H |
| H | H | H | $CH_2COPr$-i | H |
| H | H | H | $CH_2CO_2H$ | H |
| H | H | H | $CH_2CO_2Me$ | H |
| H | H | H | $CH_2CO_2Et$ | H |
| H | H | H | $CH_2CO_2Pr$-n | H |
| H | H | H | $CH_2CO_2Pr$-i | H |
| H | H | H | $CH_2OCH_2CH=CH_2$ | H |
| H | H | H | $CH_2OCH_2C\equiv CH$ | H |
| H | H | H | $CH=CHCO_2Me$ | H |
| H | H | H | $CH=CHCO_2Et$ | H |
| H | H | H | $CH=CHCO_2Pr$-n | H |
| H | H | H | $CH=CHCO_2Pr$-i | H |
| H | H | H | CH=CHCN | H |
| H | H | H | Ph | H |
| H | H | H | Ph-2-F | H |
| H | H | H | Ph-3-F | H |
| H | H | H | Ph-4-F | H |
| H | H | H | Ph-2-Cl | H |
| H | H | H | Ph-3-Cl | H |
| H | H | H | Ph-4-Cl | H |
| H | H | H | Ph-2-Br | H |
| H | H | H | Ph-3-Br | H |
| H | H | H | Ph-4-Br | H |
| H | H | H | Ph-2-$CF_3$ | H |
| H | H | H | Ph-3-$CF_3$ | H |
| H | H | H | Ph-4-$CF_3$ | H |
| H | H | H | Ph-2-Me | H |
| H | H | H | Ph-3-Me | H |

| $R_1$ | $R_2$ | $R_3$ | $R_4$ | $R_5$ |
|---|---|---|---|---|
| H | H | H | Ph-4-Me | H |
| H | H | H | Ph-2-Et | H |
| H | H | H | Ph-2-OMe | H |
| H | H | H | Ph-3-OMe | H |
| H | H | H | Ph-4-OMe | H |
| H | H | H | Ph-2-OEt | H |
| H | H | H | Ph-2-$CO_2$Me | H |
| H | H | H | Ph-2-$CO_2$Et | H |
| H | H | H | Ph-3-$CO_2$Me | H |
| H | H | H | Ph-4-$CO_2$Me | H |
| H | H | H | $CH_2$Ph | H |
| H | H | H | $CH_2$Ph-2-F | H |
| H | H | H | $CH_2$Ph-3-F | H |
| H | H | H | $CH_2$Ph-4-F | H |
| H | H | H | $CH_2$Ph-2-Cl | H |
| H | H | H | $CH_2$Ph-3-Cl | H |
| H | H | H | $CH_2$Ph-4-Cl | H |
| H | H | H | $CH_2$Ph-2-Br | H |
| H | H | H | $CH_2$Ph-3-Br | H |
| H | H | H | $CH_2$Ph-4-Br | H |
| H | H | H | $CH_2$Ph-2-$CF_3$ | H |
| H | H | H | $CH_2$Ph-3-$CF_3$ | H |
| H | H | H | $CH_2$Ph-4-$CF_3$ | H |
| H | H | H | $CH_2$Ph-2-Me | H |
| H | H | H | $CH_2$Ph-3-Me | H |
| H | H | H | $CH_2$Ph-4-Me | H |
| H | H | H | $CH_2$Ph-2-Et | H |
| H | H | H | $CH_2$Ph-2-OMe | H |
| H | H | H | $CH_2$Ph-3-OMe | H |
| H | H | H | $CH_2$Ph-4-OMe | H |
| H | H | H | $CH_2$Ph-2-OEt | H |
| H | H | H | $CH_2$Ph-2-$CO_2$Me | H |
| H | H | H | $CH_2$Ph-2-$CO_2$Et | H |
| H | H | H | $CH_2$Ph-3-$CO_2$Me | H |
| H | H | H | $CH_2$Ph-4-$CO_2$Me | H |
| H | H | H | OPh | H |
| H | H | H | OPh-2-Cl | H |
| H | H | H | OPh-3-Cl | H |
| H | H | H | OPh-4-Cl | H |
| H | H | H | OPh-2-F | H |

| $R_1$ | $R_2$ | $R_3$ | $R_4$ | $R_5$ |
|---|---|---|---|---|
| H | H | H | OPh-3-F | H |
| H | H | H | OPh-4-F | H |
| H | H | H | OPh-2-$CF_3$ | H |
| H | H | H | OPh-3-$CF_3$ | H |
| H | H | H | OPh-4-$CF_3$ | H |
| H | H | H | OPh-2-Me | H |
| H | H | H | OPh-3-Me | H |
| H | H | H | OPh-4-Me | H |
| H | H | H | OPh-2-Et | H |
| H | H | H | OPh-2-OMe | H |
| H | H | H | OPh-3-OMe | H |
| H | H | H | OPh-4-OMe | H |
| H | H | H | OPh-2-OEt | H |
| H | H | H | OPh-2-$CO_2$Me | H |
| H | H | H | OPh-3-$CO_2$Me | H |
| H | H | H | OPh-4-$CO_2$Me | H |
| H | H | H | OPh-2-$CO_2$Et | H |
| H | H | H | SPh | H |
| H | H | H | SPh-2-Cl | H |
| H | H | H | SPh-3-Cl | H |
| H | H | H | SPh-4-Cl | H |
| H | H | H | SPh-2-F | H |
| H | H | H | SPh-3-F | H |
| H | H | H | SPh-4-F | H |
| H | H | H | SPh-2-$CF_3$ | H |
| H | H | H | SPh-3-$CF_3$ | H |
| H | H | H | SPh-4-$CF_3$ | H |
| H | H | H | SPh-2-Me | H |
| H | H | H | SPh-3-Me | H |
| H | H | H | SPh-4-Me | H |
| H | H | H | SPh-2-Et | H |
| H | H | H | SPh-2-OMe | H |
| H | H | H | SPh-3-OMe | H |
| H | H | H | SPh-4-OMe | H |
| H | H | H | SPh-2-OEt | H |
| H | H | H | SPh-2-$CO_2$Me | H |
| H | H | H | SPh-3-$CO_2$Me | H |
| H | H | H | SPh-4-$CO_2$Me | H |
| H | H | H | SPh-4-$CO_2$Et | H |
| H | H | H | $SO_2$Ph | H |

| $R_1$ | $R_2$ | $R_3$ | $R_4$ | $R_5$ |
|---|---|---|---|---|
| H | H | H | $SO_2Ph$-2-Cl | H |
| H | H | H | $SO_2Ph$-3-Cl | H |
| H | H | H | $SO_2Ph$-4-Cl | H |
| H | H | H | $SO_2Ph$-2-F | H |
| H | H | H | $SO_2Ph$-3-F | H |
| H | H | H | $SO_2Ph$-4-F | H |
| H | H | H | $SO_2Ph$-2-$CF_3$ | H |
| H | H | H | $SO_2Ph$-3-$CF_3$ | H |
| H | H | H | $SO_2Ph$-4-$CF_3$ | H |
| H | H | H | $SO_2Ph$-2-Me | H |
| H | H | H | $SO_2Ph$-3-Me | H |
| H | H | H | $SO_2Ph$-4-Me | H |
| H | H | H | $SO_2Ph$-2-Et | H |
| H | H | H | $SO_2Ph$-2-OMe | H |
| H | H | H | $SO_2Ph$-3-OMe | H |
| H | H | H | $SO_2Ph$-4-OMe | H |
| H | H | H | $SO_2Ph$-2-OEt | H |
| H | H | H | $SO_2Ph$-2-$CO_2Me$ | H |
| H | H | H | $SO_2Ph$-3-$CO_2Me$ | H |
| H | H | H | $SO_2Ph$-4-$CO_2Me$ | H |
| H | H | H | $SO_2Ph$-2-$CO_2Et$ | H |
| H | H | H | CONHMe | H |
| H | H | H | CONHEt | H |
| H | H | H | CONHPr-n | H |
| H | H | H | CONHPr-i | H |
| H | H | H | CONHBu-n | H |
| H | H | H | CONHBu-sec | H |
| H | H | H | CONHBu-t | H |
| H | H | H | $CON(Me)_2$ | H |
| H | H | H | $CON(Et)_2$ | H |
| H | H | H | $CON(Pr\text{-}n)_2$ | H |
| F | H | H | H | H |
| Cl | H | H | H | H |
| Br | H | H | H | H |
| I | H | H | H | H |
| $CH=CH_2$ | H | H | H | H |
| $CH_2CH=CH_2$ | H | H | H | H |
| $C\equiv CH$ | H | H | H | H |
| $CH_2C\equiv CH$ | H | H | H | H |
| $CH_2F$ | H | H | H | H |

| $R_1$ | $R_2$ | $R_3$ | $R_4$ | $R_5$ |
|---|---|---|---|---|
| $CHF_2$ | H | H | H | H |
| CN | H | H | H | H |
| $NH_2$ | H | H | H | H |
| NHCOMe | H | H | H | H |
| $CH_2NH_2$ | H | H | H | H |
| $CH_2NHCOMe$ | H | H | H | H |
| $CH_2OCHF_2$ | H | H | H | H |
| $CH_2OCF_3$ | H | H | H | H |
| $CF_3$ | H | H | H | H |
| $NO_2$ | H | H | H | H |
| OMe | H | H | H | H |
| OEt | H | H | H | H |
| OPr-n | H | H | H | H |
| SMe | H | H | H | H |
| SEt | H | H | H | H |
| SPr-n | H | H | H | H |
| SBu-n | H | H | H | H |
| SPen-n | H | H | H | H |
| $SO_2Me$ | H | H | H | H |
| $SO_2Et$ | H | H | H | H |
| $SO_2Pr$-n | H | H | H | H |
| COMe | H | H | H | H |
| COEt | H | H | H | H |
| COPr-n | H | H | H | H |
| $CO_2H$ | H | H | H | H |
| $CO_2Me$ | H | H | H | H |
| $CO_2Et$ | H | H | H | H |
| $CO_2Pr$-n | H | H | H | H |
| $CO_2Pr$-i | H | H | H | H |
| $CO_2Bu$-n | H | H | H | H |
| $CO_2Bu$-t | H | H | H | H |
| $CO_2Bu$-sec | H | H | H | H |
| $CO_2Pen$-n | H | H | H | H |
| $CH_2Cl$ | H | H | H | H |
| $CH_2Br$ | H | H | H | H |
| $CH_2I$ | H | H | H | H |
| $CH_2CF_3$ | H | H | H | H |
| $CH_2CN$ | H | H | H | H |
| $CH_2OH$ | H | H | H | H |
| $CH_2OMe$ | H | H | H | H |

| $R_1$ | $R_2$ | $R_3$ | $R_4$ | $R_5$ |
|---|---|---|---|---|
| $CH_2OEt$ | H | H | H | H |
| $CH_2OPr$-n | H | H | H | H |
| $CH_2OPr$-i | H | H | H | H |
| $CH_2SMe$ | H | H | H | H |
| $CH_2SEt$ | H | H | H | H |
| $CH_2SPr$-n | H | H | H | H |
| $CH_2SPr$-i | H | H | H | H |
| $CH_2SO_2Me$ | H | H | H | H |
| $CH_2SO_2Et$ | H | H | H | H |
| $CH_2SO_2Pr$-n | H | H | H | H |
| $CH_2SO_2Pr$-i | H | H | H | H |
| $CH_2COMe$ | H | H | H | H |
| $CH_2COEt$ | H | H | H | H |
| $CH_2COPr$-n | H | H | H | H |
| $CH_2COPr$-i | H | H | H | H |
| $CH_2CO_2H$ | H | H | H | H |
| $CH_2CO_2Me$ | H | H | H | H |
| $CH_2CO_2Et$ | H | H | H | H |
| $CH_2CO_2Pr$-n | H | H | H | H |
| $CH_2CO_2Pr$-i | H | H | H | H |
| $CH_2OCH_2CH=CH_2$ | H | H | H | H |
| $CH_2OCH_2C\equiv CH$ | H | H | H | H |
| $CH=CHCO_2Me$ | H | H | H | H |
| $CH=CHCO_2Et$ | H | H | H | H |
| $CH=CHCO_2Pr$-n | H | H | H | H |
| $CH=CHCO_2Pr$-i | H | H | H | H |
| CH=CHCN | H | H | H | H |
| Ph | H | H | H | H |
| Ph-2-F | H | H | H | H |
| Ph-3-F | H | H | H | H |
| Ph-4-F | H | H | H | H |
| Ph-2-Cl | H | H | H | H |
| Ph-3-Cl | H | H | H | H |
| Ph-4-Cl | H | H | H | H |
| Ph-2-Br | H | H | H | H |
| Ph-3-Br | H | H | H | H |
| Ph-4-Br | H | H | H | H |
| Ph-2-$CF_3$ | H | H | H | H |
| Ph-3-$CF_3$ | H | H | H | H |
| Ph-4-$CF_3$ | H | H | H | H |

| $R_1$ | $R_2$ | $R_3$ | $R_4$ | $R_5$ |
|---|---|---|---|---|
| Ph-2-Me | H | H | H | H |
| Ph-3-Me | H | H | H | H |
| Ph-4-Me | H | H | H | H |
| Ph-2-Et | H | H | H | H |
| Ph-2-OMe | H | H | H | H |
| Ph-3-OMe | H | H | H | H |
| Ph-4-OMe | H | H | H | H |
| Ph-2-OEt | H | H | H | H |
| Ph-2-$CO_2$Me | H | H | H | H |
| Ph-2-$CO_2$Et | H | H | H | H |
| Ph-3-$CO_2$Me | H | H | H | H |
| Ph-4-$CO_2$Me | H | H | H | H |
| $CH_2$Ph | H | H | H | H |
| $CH_2$Ph-2-F | H | H | H | H |
| $CH_2$Ph-3-F | H | H | H | H |
| $CH_2$Ph-4-F | H | H | H | H |
| $CH_2$Ph-2-Cl | H | H | H | H |
| $CH_2$Ph-3-Cl | H | H | H | H |
| $CH_2$Ph-4-Cl | H | H | H | H |
| $CH_2$Ph-2-Br | H | H | H | H |
| $CH_2$Ph-3-Br | H | H | H | H |
| $CH_2$Ph-4-Br | H | H | H | H |
| $CH_2$Ph-2-$CF_3$ | H | H | H | H |
| $CH_2$Ph-3-$CF_3$ | H | H | H | H |
| $CH_2$Ph-4-$CF_3$ | H | H | H | H |
| $CH_2$Ph-2-Me | H | H | H | H |
| $CH_2$Ph-3-Me | H | H | H | H |
| $CH_2$Ph-4-Me | H | H | H | H |
| $CH_2$Ph-2-Et | H | H | H | H |
| $CH_2$Ph-2-OMe | H | H | H | H |
| $CH_2$Ph-3-OMe | H | H | H | H |
| $CH_2$Ph-4-OMe | H | H | H | H |
| $CH_2$Ph-2-OEt | H | H | H | H |
| $CH_2$Ph-2-$CO_2$Me | H | H | H | H |
| $CH_2$Ph-2-$CO_2$Et | H | H | H | H |
| $CH_2$Ph-3-$CO_2$Me | H | H | H | H |
| $CH_2$Ph-4-$CO_2$Me | H | H | H | H |
| OPh | H | H | H | H |
| OPh-2-Cl | H | H | H | H |
| OPh-3-Cl | H | H | H | H |

| $R_1$ | $R_2$ | $R_3$ | $R_4$ | $R_5$ |
|---|---|---|---|---|
| OPh-4-Cl | H | H | H | H |
| OPh-2-F | H | H | H | H |
| OPh-3-F | H | H | H | H |
| OPh-4-F | H | H | H | H |
| OPh-2-$CF_3$ | H | H | H | H |
| OPh-3-$CF_3$ | H | H | H | H |
| OPh-4-$CF_3$ | H | H | H | H |
| OPh-2-Me | H | H | H | H |
| OPh-3-Me | H | H | H | H |
| OPh-4-Me | H | H | H | H |
| OPh-2-Et | H | H | H | H |
| OPh-2-OMe | H | H | H | H |
| OPh-3-OMe | H | H | H | H |
| OPh-4-OMe | H | H | H | H |
| OPh-2-OEt | H | H | H | H |
| OPh-2-$CO_2$Me | H | H | H | H |
| OPh-3-$CO_2$Me | H | H | H | H |
| OPh-4-$CO_2$Me | H | H | H | H |
| OPh-2-$CO_2$Et | H | H | H | H |
| SPh | H | H | H | H |
| SPh-2-Cl | H | H | H | H |
| SPh-3-Cl | H | H | H | H |
| SPh-4-Cl | H | H | H | H |
| SPh-2-F | H | H | H | H |
| SPh-3-F | H | H | H | H |
| SPh-4-F | H | H | H | H |
| SPh-2-$CF_3$ | H | H | H | H |
| SPh-3-$CF_3$ | H | H | H | H |
| SPh-4-$CF_3$ | H | H | H | H |
| SPh-2-Me | H | H | H | H |
| SPh-3-Me | H | H | H | H |
| SPh-4-Me | H | H | H | H |
| SPh-2-Et | H | H | H | H |
| SPh-2-OMe | H | H | H | H |
| SPh-3-OMe | H | H | H | H |
| SPh-4-OMe | H | H | H | H |
| SPh-2-OEt | H | H | H | H |
| SPh-2-$CO_2$Me | H | H | H | H |
| SPh-3-$CO_2$Me | H | H | H | H |
| SPh-4-$CO_2$Me | H | H | H | H |

| $R_1$ | $R_2$ | $R_3$ | $R_4$ | $R_5$ |
|---|---|---|---|---|
| SPh-4-$CO_2$Et | H | H | H | H |
| $SO_2$Ph | H | H | H | H |
| $SO_2$Ph-2-Cl | H | H | H | H |
| $SO_2$Ph-3-Cl | H | H | H | H |
| $SO_2$Ph-4-Cl | H | H | H | H |
| $SO_2$Ph-2-F | H | H | H | H |
| $SO_2$Ph-3-F | H | H | H | H |
| $SO_2$Ph-4-F | H | H | H | H |
| $SO_2$Ph-2-$CF_3$ | H | H | H | H |
| $SO_2$Ph-3-$CF_3$ | H | H | H | H |
| $SO_2$Ph-4-$CF_3$ | H | H | H | H |
| $SO_2$Ph-2-Me | H | H | H | H |
| $SO_2$Ph-3-Me | H | H | H | H |
| $SO_2$Ph-4-Me | H | H | H | H |
| $SO_2$Ph-2-Et | H | H | H | H |
| $SO_2$Ph-2-OMe | H | H | H | H |
| $SO_2$Ph-3-OMe | H | H | H | H |
| $SO_2$Ph-4-OMe | H | H | H | H |
| $SO_2$Ph-2-OEt | H | H | H | H |
| $SO_2$Ph-2-$CO_2$Me | H | H | H | H |
| $SO_2$Ph-3-$CO_2$Me | H | H | H | H |
| $SO_2$Ph-4-$CO_2$Me | H | H | H | H |
| $SO_2$Ph-2-$CO_2$Et | H | H | H | H |
| CONHMe | H | H | H | H |
| CONHEt | H | H | H | H |
| CONHPr-n | H | H | H | H |
| CONHPr-i | H | H | H | H |
| CONHBu-n | H | H | H | H |
| CONHBu-sec | H | H | H | H |
| CONHBu-t | H | H | H | H |
| CON(Me)$_2$ | H | H | H | H |
| CON(Et)$_2$ | H | H | H | H |
| CON(Pr-n)$_2$ | H | H | H | H |
| H | F | H | H | H |
| H | Cl | H | H | H |
| H | Br | H | H | H |
| H | I | H | H | H |
| H | CH=$CH_2$ | H | H | H |
| H | $CH_2$CH=$CH_2$ | H | H | H |
| H | C≡CH | H | H | H |

| $R_1$ | $R_2$ | $R_3$ | $R_4$ | $R_5$ |
|---|---|---|---|---|
| H | $CH_2C{\equiv}CH$ | H | H | H |
| H | $CH_2F$ | H | H | H |
| H | $CHF_2$ | H | H | H |
| H | CN | H | H | H |
| H | $NH_2$ | H | H | H |
| H | NHCOMe | H | H | H |
| H | $CH_2NH_2$ | H | H | H |
| H | $CH_2NHCOMe$ | H | H | H |
| H | $CH_2OCHF_2$ | H | H | H |
| H | $CH_2OCF_3$ | H | H | H |
| H | $CF_3$ | H | H | H |
| H | $NO_2$ | H | H | H |
| H | OMe | H | H | H |
| H | OEt | H | H | H |
| H | OPr-n | H | H | H |
| H | SMe | H | H | H |
| H | SEt | H | H | H |
| H | SPr-n | H | H | H |
| H | SBu-n | H | H | H |
| H | SPen-n | H | H | H |
| H | $SO_2Me$ | H | H | H |
| H | $SO_2Et$ | H | H | H |
| H | $SO_2Pr$-n | H | H | H |
| H | COMe | H | H | H |
| H | COEt | H | H | H |
| H | COPr-n | H | H | H |
| H | $CO_2H$ | H | H | H |
| H | $CO_2Me$ | H | H | H |
| H | $CO_2Et$ | H | H | H |
| H | $CO_2Pr$-n | H | H | H |
| H | $CO_2Pr$-i | H | H | H |
| H | $CO_2Bu$-n | H | H | H |
| H | $CO_2Bu$-t | H | H | H |
| H | $CO_2Bu$-sec | H | H | H |
| H | $CO_2Pen$-n | H | H | H |
| H | $CH_2Cl$ | H | H | H |
| H | $CH_2Br$ | H | H | H |
| H | $CH_2I$ | H | H | H |
| H | $CH_2CF_3$ | H | H | H |
| H | $CH_2CN$ | H | H | H |

| $R_1$ | $R_2$ | $R_3$ | $R_4$ | $R_5$ |
|---|---|---|---|---|
| H | $CH_2OH$ | H | H | H |
| H | $CH_2OMe$ | H | H | H |
| H | $CH_2OEt$ | H | H | H |
| H | $CH_2OPr$-n | H | H | H |
| H | $CH_2OPr$-i | H | H | H |
| H | $CH_2SMe$ | H | H | H |
| H | $CH_2SEt$ | H | H | H |
| H | $CH_2SPr$-n | H | H | H |
| H | $CH_2SPr$-i | H | H | H |
| H | $CH_2SO_2Me$ | H | H | H |
| H | $CH_2SO_2Et$ | H | H | H |
| H | $CH_2SO_2Pr$-n | H | H | H |
| H | $CH_2SO_2Pr$-i | H | H | H |
| H | $CH_2COMe$ | H | H | H |
| H | $CH_2COEt$ | H | H | H |
| H | $CH_2COPr$-n | H | H | H |
| H | $CH_2COPr$-i | H | H | H |
| H | $CH_2CO_2H$ | H | H | H |
| H | $CH_2CO_2Me$ | H | H | H |
| H | $CH_2CO_2Et$ | H | H | H |
| H | $CH_2CO_2Pr$-n | H | H | H |
| H | $CH_2CO_2Pr$-i | H | H | H |
| H | $CH_2OCH_2CH=CH_2$ | H | H | H |
| H | $CH_2OCH_2C\equiv CH$ | H | H | H |
| H | $CH=CHCO_2Me$ | H | H | H |
| H | $CH=CHCO_2Et$ | H | H | H |
| H | $CH=CHCO_2Pr$-n | H | H | H |
| H | $CH=CHCO_2Pr$-i | H | H | H |
| H | CH=CHCN | H | H | H |
| H | Ph | H | H | H |
| H | Ph-2-F | H | H | H |
| H | Ph-3-F | H | H | H |
| H | Ph-4-F | H | H | H |
| H | Ph-2-Cl | H | H | H |
| H | Ph-3-Cl | H | H | H |
| H | Ph-4-Cl | H | H | H |
| H | Ph-2-Br | H | H | H |
| H | Ph-3-Br | H | H | H |
| H | Ph-4-Br | H | H | H |
| H | $Ph-2-CF_3$ | H | H | H |

| $R_1$ | $R_2$ | $R_3$ | $R_4$ | $R_5$ |
|---|---|---|---|---|
| H | Ph-3-$CF_3$ | H | H | H |
| H | Ph-4-$CF_3$ | H | H | H |
| H | Ph-2-Me | H | H | H |
| H | Ph-3-Me | H | H | H |
| H | Ph-4-Me | H | H | H |
| H | Ph-2-Et | H | H | H |
| H | Ph-2-OMe | H | H | H |
| H | Ph-3-OMe | H | H | H |
| H | Ph-4-OMe | H | H | H |
| H | Ph-2-OEt | H | H | H |
| H | Ph-2-$CO_2$Me | H | H | H |
| H | Ph-2-$CO_2$Et | H | H | H |
| H | Ph-3-$CO_2$Me | H | H | H |
| H | Ph-4-$CO_2$Me | H | H | H |
| H | $CH_2$Ph | H | H | H |
| H | $CH_2$Ph-2-F | H | H | H |
| H | $CH_2$Ph-3-F | H | H | H |
| H | $CH_2$Ph-4-F | H | H | H |
| H | $CH_2$Ph-2-Cl | H | H | H |
| H | $CH_2$Ph-3-Cl | H | H | H |
| H | $CH_2$Ph-4-Cl | H | H | H |
| H | $CH_2$Ph-2-Br | H | H | H |
| H | $CH_2$Ph-3-Br | H | H | H |
| H | $CH_2$Ph-4-Br | H | H | H |
| H | $CH_2$Ph-2-$CF_3$ | H | H | H |
| H | $CH_2$Ph-3-$CF_3$ | H | H | H |
| H | $CH_2$Ph-4-$CF_3$ | H | H | H |
| H | $CH_2$Ph-2-Me | H | H | H |
| H | $CH_2$Ph-3-Me | H | H | H |
| H | $CH_2$Ph-4-Me | H | H | H |
| H | $CH_2$Ph-2-Et | H | H | H |
| H | $CH_2$Ph-2-OMe | H | H | H |
| H | $CH_2$Ph-3-OMe | H | H | H |
| H | $CH_2$Ph-4-OMe | H | H | H |
| H | $CH_2$Ph-2-OEt | H | H | H |
| H | $CH_2$Ph-2-$CO_2$Me | H | H | H |
| H | $CH_2$Ph-2-$CO_2$Et | H | H | H |
| H | $CH_2$Ph-3-$CO_2$Me | H | H | H |
| H | $CH_2$Ph-4-$CO_2$Me | H | H | H |
| H | OPh | H | H | H |

| $R_1$ | $R_2$ | $R_3$ | $R_4$ | $R_5$ |
|---|---|---|---|---|
| H | OPh-2-Cl | H | H | H |
| H | OPh-3-Cl | H | H | H |
| H | OPh-4-Cl | H | H | H |
| H | OPh-2-F | H | H | H |
| H | OPh-3-F | H | H | H |
| H | OPh-4-F | H | H | H |
| H | OPh-2-$CF_3$ | H | H | H |
| H | OPh-3-$CF_3$ | H | H | H |
| H | OPh-4-$CF_3$ | H | H | H |
| H | OPh-2-Me | H | H | H |
| H | OPh-3-Me | H | H | H |
| H | OPh-4-Me | H | H | H |
| H | OPh-2-Et | H | H | H |
| H | OPh-2-OMe | H | H | H |
| H | OPh-3-OMe | H | H | H |
| H | OPh-4-OMe | H | H | H |
| H | OPh-2-OEt | H | H | H |
| H | OPh-2-$CO_2$Me | H | H | H |
| H | OPh-3-$CO_2$Me | H | H | H |
| H | OPh-4-$CO_2$Me | H | H | H |
| H | OPh-2-$CO_2$Et | H | H | H |
| H | SPh | H | H | H |
| H | SPh-2-Cl | H | H | H |
| H | SPh-3-Cl | H | H | H |
| H | SPh-4-Cl | H | H | H |
| H | SPh-2-F | H | H | H |
| H | SPh-3-F | H | H | H |
| H | SPh-4-F | H | H | H |
| H | SPh-2-$CF_3$ | H | H | H |
| H | SPh-3-$CF_3$ | H | H | H |
| H | SPh-4-$CF_3$ | H | H | H |
| H | SPh-2-Me | H | H | H |
| H | SPh-3-Me | H | H | H |
| H | SPh-4-Me | H | H | H |
| H | SPh-2-Et | H | H | H |
| H | SPh-2-OMe | H | H | H |
| H | SPh-3-OMe | H | H | H |
| H | SPh-4-OMe | H | H | H |
| H | SPh-2-OEt | H | H | H |
| H | SPh-2-$CO_2$Me | H | H | H |

| $R_1$ | $R_2$ | $R_3$ | $R_4$ | $R_5$ |
|---|---|---|---|---|
| H | SPh-3-$CO_2$Me | H | H | H |
| H | SPh-4-$CO_2$Me | H | H | H |
| H | SPh-4-$CO_2$Et | H | H | H |
| H | $SO_2$Ph | H | H | H |
| H | $SO_2$Ph-2-Cl | H | H | H |
| H | $SO_2$Ph-3-Cl | H | H | H |
| H | $SO_2$Ph-4-Cl | H | H | H |
| H | $SO_2$Ph-2-F | H | H | H |
| H | $SO_2$Ph-3-F | H | H | H |
| H | $SO_2$Ph-4-F | H | H | H |
| H | $SO_2$Ph-2-$CF_3$ | H | H | H |
| H | $SO_2$Ph-3-$CF_3$ | H | H | H |
| H | $SO_2$Ph-4-$CF_3$ | H | H | H |
| H | $SO_2$Ph-2-Me | H | H | H |
| H | $SO_2$Ph-3-Me | H | H | H |
| H | $SO_2$Ph-4-Me | H | H | H |
| H | $SO_2$Ph-2-Et | H | H | H |
| H | $SO_2$Ph-2-OMe | H | H | H |
| H | $SO_2$Ph-3-OMe | H | H | H |
| H | $SO_2$Ph-4-OMe | H | H | H |
| H | $SO_2$Ph-2-OEt | H | H | H |
| H | $SO_2$Ph-2-$CO_2$Me | H | H | H |
| H | $SO_2$Ph-3-$CO_2$Me | H | H | H |
| H | $SO_2$Ph-4-$CO_2$Me | H | H | H |
| H | $SO_2$Ph-2-$CO_2$Et | H | H | H |
| H | CONHMe | H | H | H |
| H | CONHEt | H | H | H |
| H | CONHPr-n | H | H | H |
| H | CONHPr-i | H | H | H |
| H | CONHBu-n | H | H | H |
| H | CONHBu-sec | H | H | H |
| H | CONHBu-t | H | H | H |
| H | CON(Me)$_2$ | H | H | H |
| H | CON(Et)$_2$ | H | H | H |
| H | CON(Pr-n)$_2$ | H | H | H |
| H | H | F | H | H |
| H | H | Cl | H | H |
| H | H | Br | H | H |
| H | H | I | H | H |
| H | H | CH=$CH_2$ | H | H |

| $R_1$ | $R_2$ | $R_3$ | $R_4$ | $R_5$ |
|---|---|---|---|---|
| H | H | $CH_2CH=CH_2$ | H | H |
| H | H | $C\equiv CH$ | H | H |
| H | H | $CH_2C\equiv CH$ | H | H |
| H | H | $CH_2F$ | H | H |
| H | H | $CHF_2$ | H | H |
| H | H | CN | H | H |
| H | H | $NH_2$ | H | H |
| H | H | NHCOMe | H | H |
| H | H | $CH_2NH_2$ | H | H |
| H | H | $CH_2NHCOMe$ | H | H |
| H | H | $CH_2OCHF_2$ | H | H |
| H | H | $CH_2OCF_3$ | H | H |
| H | H | $CF_3$ | H | H |
| H | H | $NO_2$ | H | H |
| H | H | OMe | H | H |
| H | H | OEt | H | H |
| H | H | OPr-n | H | H |
| H | H | SMe | H | H |
| H | H | SEt | H | H |
| H | H | SPr-n | H | H |
| H | H | SBu-n | H | H |
| H | H | SPen-n | H | H |
| H | H | $SO_2Me$ | H | H |
| H | H | $SO_2Et$ | H | H |
| H | H | $SO_2Pr$-n | H | H |
| H | H | COMe | H | H |
| H | H | COEt | H | H |
| H | H | COPr-n | H | H |
| H | H | $CO_2H$ | H | H |
| H | H | $CO_2Me$ | H | H |
| H | H | $CO_2Et$ | H | H |
| H | H | $CO_2Pr$-n | H | H |
| H | H | $CO_2Pr$-i | H | H |
| H | H | $CO_2Bu$-n | H | H |
| H | H | $CO_2Bu$-t | H | H |
| H | H | $CO_2Bu$-sec | H | H |
| H | H | $CO_2Pen$-n | H | H |
| H | H | $CH_2Cl$ | H | H |
| H | H | $CH_2Br$ | H | H |
| H | H | $CH_2I$ | H | H |

| $R_1$ | $R_2$ | $R_3$ | $R_4$ | $R_5$ |
|---|---|---|---|---|
| H | H | $CH_2CF_3$ | H | H |
| H | H | $CH_2CN$ | H | H |
| H | H | $CH_2OH$ | H | H |
| H | H | $CH_2OMe$ | H | H |
| H | H | $CH_2OEt$ | H | H |
| H | H | $CH_2OPr$-n | H | H |
| H | H | $CH_2OPr$-i | H | H |
| H | H | $CH_2SMe$ | H | H |
| H | H | $CH_2SEt$ | H | H |
| H | H | $CH_2SPr$-n | H | H |
| H | H | $CH_2SPr$-i | H | H |
| H | H | $CH_2SO_2Me$ | H | H |
| H | H | $CH_2SO_2Et$ | H | H |
| H | H | $CH_2SO_2Pr$-n | H | H |
| H | H | $CH_2SO_2Pr$-i | H | H |
| H | H | $CH_2COMe$ | H | H |
| H | H | $CH_2COEt$ | H | H |
| H | H | $CH_2COPr$-n | H | H |
| H | H | $CH_2COPr$-i | H | H |
| H | H | $CH_2CO_2H$ | H | H |
| H | H | $CH_2CO_2Me$ | H | H |
| H | H | $CH_2CO_2Et$ | H | H |
| H | H | $CH_2CO_2Pr$-n | H | H |
| H | H | $CH_2CO_2Pr$-i | H | H |
| H | H | $CH_2OCH_2CH=CH_2$ | H | H |
| H | H | $CH_2OCH_2C\equiv CH$ | H | H |
| H | H | $CH=CHCO_2Me$ | H | H |
| H | H | $CH=CHCO_2Et$ | H | H |
| H | H | $CH=CHCO_2Pr$-n | H | H |
| H | H | $CH=CHCO_2Pr$-i | H | H |
| H | H | CH=CHCN | H | H |
| H | H | Ph | H | H |
| H | H | Ph-2-F | H | H |
| H | H | Ph-3-F | H | H |
| H | H | Ph-4-F | H | H |
| H | H | Ph-2-Cl | H | H |
| H | H | Ph-3-Cl | H | H |
| H | H | Ph-4-Cl | H | H |
| H | H | Ph-2-Br | H | H |
| H | H | Ph-3-Br | H | H |

| $R_1$ | $R_2$ | $R_3$ | $R_4$ | $R_5$ |
|---|---|---|---|---|
| H | H | Ph-4-Br | H | H |
| H | H | Ph-2-$CF_3$ | H | H |
| H | H | Ph-3-$CF_3$ | H | H |
| H | H | Ph-4-$CF_3$ | H | H |
| H | H | Ph-2-Me | H | H |
| H | H | Ph-3-Me | H | H |
| H | H | Ph-4-Me | H | H |
| H | H | Ph-2-Et | H | H |
| H | H | Ph-2-OMe | H | H |
| H | H | Ph-3-OMe | H | H |
| H | H | Ph-4-OMe | H | H |
| H | H | Ph-2-OEt | H | H |
| H | H | Ph-2-$CO_2$Me | H | H |
| H | H | Ph-2-$CO_2$Et | H | H |
| H | H | Ph-3-$CO_2$Me | H | H |
| H | H | Ph-4-$CO_2$Me | H | H |
| H | H | $CH_2$Ph | H | H |
| H | H | $CH_2$Ph-2-F | H | H |
| H | H | $CH_2$Ph-3-F | H | H |
| H | H | $CH_2$Ph-4-F | H | H |
| H | H | $CH_2$Ph-2-Cl | H | H |
| H | H | $CH_2$Ph-3-Cl | H | H |
| H | H | $CH_2$Ph-4-Cl | H | H |
| H | H | $CH_2$Ph-2-Br | H | H |
| H | H | $CH_2$Ph-3-Br | H | H |
| H | H | $CH_2$Ph-4-Br | H | H |
| H | H | $CH_2$Ph-2-$CF_3$ | H | H |
| H | H | $CH_2$Ph-3-$CF_3$ | H | H |
| H | H | $CH_2$Ph-4-$CF_3$ | H | H |
| H | H | $CH_2$Ph-2-Me | H | H |
| H | H | $CH_2$Ph-3-Me | H | H |
| H | H | $CH_2$Ph-4-Me | H | H |
| H | H | $CH_2$Ph-2-Et | H | H |
| H | H | $CH_2$Ph-2-OMe | H | H |
| H | H | $CH_2$Ph-3-OMe | H | H |
| H | H | $CH_2$Ph-4-OMe | H | H |
| H | H | $CH_2$Ph-2-OEt | H | H |
| H | H | $CH_2$Ph-2-$CO_2$Me | H | H |
| H | H | $CH_2$Ph-2-$CO_2$Et | H | H |
| H | H | $CH_2$Ph-3-$CO_2$Me | H | H |

| $R_1$ | $R_2$ | $R_3$ | $R_4$ | $R_5$ |
|---|---|---|---|---|
| H | H | $CH_2Ph$-4-$CO_2Me$ | H | H |
| H | H | OPh | H | H |
| H | H | OPh-2-Cl | H | H |
| H | H | OPh-3-Cl | H | H |
| H | H | OPh-4-Cl | H | H |
| H | H | OPh-2-F | H | H |
| H | H | OPh-3-F | H | H |
| H | H | OPh-4-F | H | H |
| H | H | OPh-2-$CF_3$ | H | H |
| H | H | OPh-3-$CF_3$ | H | H |
| H | H | OPh-4-$CF_3$ | H | H |
| H | H | OPh-2-Me | H | H |
| H | H | OPh-3-Me | H | H |
| H | H | OPh-4-Me | H | H |
| H | H | OPh-2-Et | H | H |
| H | H | OPh-2-OMe | H | H |
| H | H | OPh-3-OMe | H | H |
| H | H | OPh-4-OMe | H | H |
| H | H | OPh-2-OEt | H | H |
| H | H | OPh-2-$CO_2Me$ | H | H |
| H | H | OPh-3-$CO_2Me$ | H | H |
| H | H | OPh-4-$CO_2Me$ | H | H |
| H | H | OPh-2-$CO_2Et$ | H | H |
| H | H | SPh | H | H |
| H | H | SPh-2-Cl | H | H |
| H | H | SPh-3-Cl | H | H |
| H | H | SPh-4-Cl | H | H |
| H | H | SPh-2-F | H | H |
| H | H | SPh-3-F | H | H |
| H | H | SPh-4-F | H | H |
| H | H | SPh-2-$CF_3$ | H | H |
| H | H | SPh-3-$CF_3$ | H | H |
| H | H | SPh-4-$CF_3$ | H | H |
| H | H | SPh-2-Me | H | H |
| H | H | SPh-3-Me | H | H |
| H | H | SPh-4-Me | H | H |
| H | H | SPh-2-Et | H | H |
| H | H | SPh-2-OMe | H | H |
| H | H | SPh-3-OMe | H | H |
| H | H | SPh-4-OMe | H | H |

| $R_1$ | $R_2$ | $R_3$ | $R_4$ | $R_5$ |
|---|---|---|---|---|
| H | H | SPh-2-OEt | H | H |
| H | H | SPh-2-$CO_2$Me | H | H |
| H | H | SPh-3-$CO_2$Me | H | H |
| H | H | SPh-4-$CO_2$Me | H | H |
| H | H | SPh-4-$CO_2$Et | H | H |
| H | H | $SO_2$Ph | H | H |
| H | H | $SO_2$Ph-2-Cl | H | H |
| H | H | $SO_2$Ph-3-Cl | H | H |
| H | H | $SO_2$Ph-4-Cl | H | H |
| H | H | $SO_2$Ph-2-F | H | H |
| H | H | $SO_2$Ph-3-F | H | H |
| H | H | $SO_2$Ph-4-F | H | H |
| H | H | $SO_2$Ph-2-$CF_3$ | H | H |
| H | H | $SO_2$Ph-3-$CF_3$ | H | H |
| H | H | $SO_2$Ph-4-$CF_3$ | H | H |
| H | H | $SO_2$Ph-2-Me | H | H |
| H | H | $SO_2$Ph-3-Me | H | H |
| H | H | $SO_2$Ph-4-Me | H | H |
| H | H | $SO_2$Ph-2-Et | H | H |
| H | H | $SO_2$Ph-2-OMe | H | H |
| H | H | $SO_2$Ph-3-OMe | H | H |
| H | H | $SO_2$Ph-4-OMe | H | H |
| H | H | $SO_2$Ph-2-OEt | H | H |
| H | H | $SO_2$Ph-2-$CO_2$Me | H | H |
| H | H | $SO_2$Ph-3-$CO_2$Me | H | H |
| H | H | $SO_2$Ph-4-$CO_2$Me | H | H |
| H | H | $SO_2$Ph-2-$CO_2$Et | H | H |
| H | H | CONHMe | H | H |
| H | H | CONHEt | H | H |
| H | H | CONHPr-n | H | H |
| H | H | CONHPr-i | H | H |
| H | H | CONHBu-n | H | H |
| H | H | CONHBu-sec | H | H |
| H | H | CONHBu-t | H | H |
| H | H | CON(Me)$_2$ | H | H |
| H | H | CON(Et)$_2$ | H | H |
| H | H | CON(Pr-n)$_2$ | H | H |

| $R_1$ | $R_2$ | $R_3$ | $R_4$ | $R_5$ |
|---|---|---|---|---|
| Me | Me | H | H | H |
| Me | H | Me | H | H |
| Me | H | H | Me | H |
| Me | Et | H | H | H |
| Me | H | Et | H | H |
| Me | H | H | Et | H |
| Me | Pr-n | H | H | H |
| Me | H | Pr-n | H | H |
| Me | H | H | Pr-n | H |
| Me | Pr-i | H | H | H |
| Me | H | Pr-i | H | H |
| Me | H | H | Pr-i | H |
| Me | Bu-n | H | H | H |
| Me | H | Bu-n | H | H |
| Me | H | H | Bu-n | H |
| Et | Me | H | H | H |
| Et | H | Me | H | H |
| Et | H | H | Me | H |
| Et | Et | H | H | H |
| Et | H | Et | H | H |
| Et | H | H | Et | H |
| Et | Pr-n | H | H | H |
| Et | H | Pr-n | H | H |
| Et | H | H | Pr-n | H |
| Et | Pr-i | H | H | H |
| Et | H | Pr-i | H | H |
| Et | H | H | Pr-i | H |
| Et | Bu-n | H | H | H |
| Et | H | Bu-n | H | H |
| Et | H | H | Bu-n | H |
| Pr-n | Me | H | H | H |
| Pr-n | H | Me | H | H |
| Pr-n | H | H | Me | H |
| Pr-n | Et | H | H | H |
| Pr-n | H | Et | H | H |
| Pr-n | H | H | Et | H |
| Pr-n | Pr-n | H | H | H |
| Pr-n | H | Pr-n | H | H |
| Pr-n | H | H | Pr-n | H |
| Pr-n | Pr-i | H | H | H |

| $R_1$ | $R_2$ | $R_3$ | $R_4$ | $R_5$ |
|---|---|---|---|---|
| Pr-n | H | Pr-i | H | H |
| Pr-n | H | H | Pr-i | H |
| Pr-n | Bu-n | H | H | H |
| Pr-n | H | Bu-n | H | H |
| Pr-n | H | H | Bu-n | H |
| Pr-i | Me | H | H | H |
| Pr-i | H | Me | H | H |
| Pr-i | H | H | Me | H |
| Pr-i | Et | H | H | H |
| Pr-i | H | Et | H | H |
| Pr-i | H | H | Et. | H |
| Pr-i | Pr-n | H | H | H |
| Pr-i | H | Pr-n | H | H |
| Pr-i | H | H | Pr-n | H |
| Pr-i | Pr-i | H | H | H |
| Pr-i | H | Pr-i | H | H |
| Pr-i | H | H | Pr-i | H |
| Pr-i | Bu-n | H | H | H |
| Pr-i | H | Bu-n | H | H |
| Pr-i | H | H | Bu-n | H |
| Bu-n | Me | H | H | H |
| Bu-n | H | Me | H | H |
| Bu-n | H | H | Me | H |
| Bu-n | Et | H | H | H |
| Bu-n | H | Et | H | H |
| Bu-n | H | H | Et | H |
| Bu-n | Pr-n | H | H | H |
| Bu-n | H | Pr-n | H | H |
| Bu-n | H | H | Pr-n | H |
| Bu-n | Pr-i | H | H | H |
| Bu-n | H | Pr-i | H | H |
| Bu-n | H | H | Pr-i | H |
| Bu-n | Bu-n | H | H | H |
| Bu-n | H | Bu-n | H | H |
| Bu-n | H | H | Bu-n | H |
| H | Me | Me | H | H |
| H | Me | H | Me | H |
| H | Me | Et | H | H |
| H | Me | H | Et | H |
| H | Me | Pr-n | H | H |

| $R_1$ | $R_2$ | $R_3$ | $R_4$ | $R_5$ |
|---|---|---|---|---|
| H | Me | H | Pr-n | H |
| H | Me | Pr-i | H | H |
| H | Me | H | Pr-i | H |
| H | Et | Me | H | H |
| H | Et | H | Me | H |
| H | Et | Et | H | H |
| H | Et | H | Et | H |
| H | Et | Pr-n | H | H |
| H | Et | H | Pr-n | H |
| H | Et | Pr-i | H | H |
| H | Et | H | Pr-i | H |
| H | Pr-n | Me | H | H |
| H | Pr-n | H | Me | H |
| H | Pr-n | Et | H | H |
| H | Pr-n | H | Et | H |
| H | Pr-n | Pr-n | H | H |
| H | Pr-n | H | Pr-n | H |
| H | Pr-n | Pr-i | H | H |
| H | Pr-n | H | Pr-i | H |
| H | Pr-i | Me | H | H |
| H | Pr-i | H | Me | H |
| H | Pr-i | Et | H | H |
| H | Pr-i | H | Et | H |
| H | Pr-i | Pr-n | H | H |
| H | Pr-i | H | Pr-n | H |
| H | Pr-i | Pr-i | H | H |
| H | Pr-i | H | Pr-i | H |
| H | H | Me | Me | H |
| H | H | Me | Et | H |
| H | H | Me | Pr-n | H |
| H | H | Me | Pr-i | H |
| H | H | Et | Me | H |
| H | H | Et | Et | H |
| H | H | Et | Pr-n | H |
| H | H | Et | Pr-i | H |
| H | H | Pr-n | Me | H |
| H | H | Pr-n | Et | H |
| H | H | Pr-n | Pr-n | H |
| H | H | Pr-n | Pr-i | H |
| H | H | Pr-i | Me | H |

| $R_1$ | $R_2$ | $R_3$ | $R_4$ | $R_5$ |
|---|---|---|---|---|
| H | H | Pr-i | Et | H |
| H | H | Pr-i | Pr-n | H |
| H | H | Pr-i | Pr-i | H |
| Me | Me | Me | H | H |
| Me | Me | Et | H | H |
| Me | Me | Pr-n | H | H |
| Me | Me | Pr-i | H | H |
| Me | Et | Me | H | H |
| Me | Et | Et | H | H |
| Me | Et | Pr-n | H | H |
| Me | Et | Pr-i | H | H |
| Me | Pr-n | Me | H | H |
| Me | Pr-n | Et | H | H |
| Me | Pr-n | Pr-n | H | H |
| Me | Pr-n | Pr-i | H | H |
| Me | Pr-i | Me | H | H |
| Me | Pr-i | Et | H | H |
| Me | Pr-i | Pr-n | H | H |
| Me | Pr-i | Pr-i | H | H |
| Et | Me | Me | H | H |
| Et | Me | Et | H | H |
| Et | Me | Pr-n | H | H |
| Et | Me | Pr-i | H | H |
| Et | Et | Me | H | H |
| Et | Et | Et | H | H |
| Et | Et | Pr-n | H | H |
| Et | Et | Pr-i | H | H |
| Et | Pr-n | Me | H | H |
| Et | Pr-n | Et | H | H |
| Et | Pr-n | Pr-n | H | H |
| Et | Pr-n | Pr-i | H | H |
| Et | Pr-i | Me | H | H |
| Et | Pr-i | Et | H | H |
| Et | Pr-i | Pr-n | H | H |
| Et | Pr-i | Pr-i | H | H |
| Pr-n | Me | Me | H | H |
| Pr-n | Me | Et | H | H |
| Pr-n | Me | Pr-n | H | H |
| Pr-n | Me | Pr-i | H | H |
| Pr-n | Et | Me | H | H |

| $R_1$ | $R_2$ | $R_3$ | $R_4$ | $R_5$ |
|---|---|---|---|---|
| Pr-n | Et | Et | H | H |
| Pr-n | Et | Pr-n | H | H |
| Pr-n | Et | Pr-i | H | H |
| Pr-n | Pr-n | Me | H | H |
| Pr-n | Pr-n | Et | H | H |
| Pr-n | Pr-n | Pr-n | H | H |
| Pr-n | Pr-n | Pr-i | H | H |
| Pr-n | Pr-i | Me | H | H |
| Pr-n | Pr-i | Et | H | H |
| Pr-n | Pr-i | Pr-n | H | H |
| Pr-n | Pr-i | Pr-i | H | H |
| Pr-i | Me | Me | H | H |
| Pr-i | Me | Et | H | H |
| Pr-i | Me | Pr-n | H | H |
| Pr-i | Me | Pr-i | H | H |
| Pr-i | Et | Me | H | H |
| Pr-i | Et | Et | H | H |
| Pr-i | Et | Pr-n | H | H |
| Pr-i | Et | Pr-i | H | H |
| Pr-i | Pr-n | Me | H | H |
| Pr-i | Pr-n | Et | H | H |
| Pr-i | Pr-n | Pr-n | H | H |
| Pr-i | Pr-n | Pr-i | H | H |
| Pr-i | Pr-i | Me | H | H |
| Pr-i | Pr-i | Et | H | H |
| Pr-i | Pr-i | Pr-n | H | H |
| Pr-i | Pr-i | Pr-i | H | H |
| Me | H | Me | Me | H |
| Me | H | Me | Et | H |
| Me | H | Me | Pr-n | H |
| Me | H | Me | Pr-i | H |
| Me | H | Et | Me | H |
| Me | H | Et | Et | H |
| Me | H | Et | Pr-n | H |
| Me | H | Et | Pr-i | H |
| Me | H | Pr-n | Me | H |
| Me | H | Pr-n | Et | H |
| Me | H | Pr-n | Pr-n | H |
| Me | H | Pr-n | Pr-i | H |
| Me | H | Pr-i | Me | H |

| $R_1$ | $R_2$ | $R_3$ | $R_4$ | $R_5$ |
|---|---|---|---|---|
| Me | H | Pr-i | Et | H |
| Me | H | Pr-i | Pr-n | H |
| Me | H | Pr-i | Pr-i | H |
| Et | H | Me | Me | H |
| Et | H | Me | Et | H |
| Et | H | Me | Pr-n | H |
| Et | H | Me | Pr-i | H |
| Et | H | Et | Me | H |
| Et | H | Et | Et | H |
| Et | H | Et | Pr-n | H |
| Et | H | Et | Pr-i | H |
| Et | H | Pr-n | Me | H |
| Et | H | Pr-n | Et | H |
| Et | H | Pr-n | Pr-n | H |
| Et | H | Pr-n | Pr-i | H |
| Et | H | Pr-i | Me | H |
| Et | H | Pr-i | Et | H |
| Et | H | Pr-i | Pr-n | H |
| Et | H | Pr-i | Pr-i | H |
| Pr-n | H | Me | Me | H |
| Pr-n | H | Me | Et | H |
| Pr-n | H | Me | Pr-n | H |
| Pr-n | H | Me | Pr-i | H |
| Pr-n | H | Et | Me | H |
| Pr-n | H | Et | Et | H |
| Pr-n | H | Et | Pr-n | H |
| Pr-n | H | Et | Pr-i | H |
| Pr-n | H | Pr-n | Me | H |
| Pr-n | H | Pr-n | Et | H |
| Pr-n | H | Pr-n | Pr-n | H |
| Pr-n | H | Pr-n | Pr-i | H |
| Pr-n | H | Pr-i | Me | H |
| Pr-n | H | Pr-i | Et | H |
| Pr-n | H | Pr-i | Pr-n | H |
| Pr-n | H | Pr-i | Pr-i | H |
| Pr-i | H | Me | Me | H |
| Pr-i | H | Me | Et | H |
| Pr-i | H | Me | Pr-n | H |
| Pr-i | H | Me | Pr-i | H |
| Pr-i | H | Et | Me | H |

| $R_1$ | $R_2$ | $R_3$ | $R_4$ | $R_5$ |
|---|---|---|---|---|
| Pr-i | H | Et | Et | H |
| Pr-i | H | Et | Pr-n | H |
| Pr-i | H | Et | Pr-i | H |
| Pr-i | H | Pr-n | Me | H |
| Pr-i | H | Pr-n | Et | H |
| Pr-i | H | Pr-n | Pr-n | H |
| Pr-i | H | Pr-n | Pr-i | H |
| Pr-i | H | Pr-i | Me | H |
| Pr-i | H | Pr-i | Et | H |
| Pr-i | H | Pr-i | Pr-n | H |
| Pr-i | H | Pr-i | Pr-i | H |
| Me | Me | H | Me | H |
| Me | Me | H | Et | H |
| Me | Me | H | Pr-n | H |
| Me | Me | H | Pr-i | H |
| Me | Et | H | Me | H |
| Me | Et | H | Et | H |
| Me | Et | H | Pr-n | H |
| Me | Et | H | Pr-i | H |
| Me | Pr-n | H | Me | H |
| Me | Pr-n | H | Et | H |
| Me | Pr-n | H | Pr-n | H |
| Me | Pr-n | H | Pr-i | H |
| Me | Pr-i | H | Me | H |
| Me | Pr-i | H | Et | H |
| Me | Pr-i | H | Pr-n | H |
| Me | Pr-i | H | Pr-i | H |
| Et | Me | H | Me | H |
| Et | Me | H | Et | H |
| Et | Me | H | Pr-n | H |
| Et | Me | H | Pr-i | H |
| Et | Et | H | Me | H |
| Et | Et | H | Et | H |
| Et | Et | H | Pr-n | H |
| Et | Et | H | Pr-i | H |
| Et | Pr-n | H | Me | H |
| Et | Pr-n | H | Et | H |
| Et | Pr-n | H | Pr-n | H |
| Et | Pr-n | H | Pr-i | H |
| Et | Pr-i | H | Me | H |

| $R_1$ | $R_2$ | $R_3$ | $R_4$ | $R_5$ |
|---|---|---|---|---|
| Et | Pr-i | H | Et | H |
| Et | Pr-i | H | Pr-n | H |
| Et | Pr-i | H | Pr-i | H |
| Pr-n | Me | H | Me | H |
| Pr-n | Me | H | Et | H |
| Pr-n | Me | H | Pr-n | H |
| Pr-n | Me | H | Pr-i | H |
| Pr-n | Et | H | Me | H |
| Pr-n | Et | H | Et | H |
| Pr-n | Et | H | Pr-n | H |
| Pr-n | Et | H | Pr-i | H |
| Pr-n | Pr-n | H | Me | H |
| Pr-n | Pr-n | H | Et | H |
| Pr-n | Pr-n | H | Pr-n | H |
| Pr-n | Pr-n | H | Pr-i | H |
| Pr-n | Pr-i | H | Me | H |
| Pr-n | Pr-i | H | Et | H |
| Pr-n | Pr-i | H | Pr-n | H |
| Pr-n | Pr-i | H | Pr-i | H |
| Pr-i | Me | H | Me | H |
| Pr-i | Me | H | Et | H |
| Pr-i | Me | H | Pr-n | H |
| Pr-i | Me | H | Pr-i | H |
| Pr-i | Et | H | Me | H |
| Pr-i | Et | H | Et | H |
| Pr-i | Et | H | Pr-n | H |
| Pr-i | Et | H | Pr-i | H |
| Pr-i | Pr-n | H | Me | H |
| Pr-i | Pr-n | H | Et | H |
| Pr-i | Pr-n | H | Pr-n | H |
| Pr-i | Pr-n | H | Pr-i | H |
| Pr-i | Pr-i | H | Me | H |
| Pr-i | Pr-i | H | Et | H |
| Pr-i | Pr-i | H | Pr-n | H |
| Pr-i | Pr-i | H | Pr-i | H |
| H | Me | Me | Me | H |
| H | Me | Me | Et | H |
| H | Me | Me | Pr-n | H |
| H | Me | Me | Pr-i | H |
| H | Me | Et | Me | H |

| $R_1$ | $R_2$ | $R_3$ | $R_4$ | $R_5$ |
|---|---|---|---|---|
| H | Me | Et | Et | H |
| H | Me | Et | Pr-n | H |
| H | Me | Et | Pr-i | H |
| H | Me | Pr-n | Me | H |
| H | Me | Pr-n | Et | H |
| H | Me | Pr-n | Pr-n | H |
| H | Me | Pr-n | Pr-i | H |
| H | Me | Pr-i | Me | H |
| H | Me | Pr-i | Et | H |
| H | Me | Pr-i | Pr-n | H |
| H | Me | Pr-i | Pr-i | H |
| H | Et | Me | Me | H |
| H | Et | Me | Et | H |
| H | Et | Me | Pr-n | H |
| H | Et | Me | Pr-i | H |
| H | Et | Et | Me | H |
| H | Et | Et | Et | H |
| H | Et | Et | Pr-n | H |
| H | Et | Et | Pr-i | H |
| H | Et | Pr-n | Me | H |
| H | Et | Pr-n | Et | H |
| H | Et | Pr-n | Pr-n | H |
| H | Et | Pr-n | Pr-i | H |
| H | Et | Pr-i | Me | H |
| H | Et | Pr-i | Et | H |
| H | Et | Pr-i | Pr-n | H |
| H | Et | Pr-i | Pr-i | H |
| H | Pr-n | Me | Me | H |
| H | Pr-n | Me | Et | H |
| H | Pr-n | Me | Pr-n | H |
| H | Pr-n | Me | Pr-i | H |
| H | Pr-n | Et | Me | H |
| H | Pr-n | Et | Et | H |
| H | Pr-n | Et | Pr-n | H |
| H | Pr-n | Et | Pr-i | H |
| H | Pr-n | Pr-n | Me | H |
| H | Pr-n | Pr-n | Et | H |
| H | Pr-n | Pr-n | Pr-n | H |
| H | Pr-n | Pr-n | Pr-i | H |
| H | Pr-n | Pr-i | Me | H |

| $R_1$ | $R_2$ | $R_3$ | $R_4$ | $R_5$ |
|---|---|---|---|---|
| H | Pr-n | Pr-i | Et | H |
| H | Pr-n | Pr-i | Pr-n | H |
| H | Pr-n | Pr-i | Pr-i | H |
| H | Pr-i | Me | Me | H |
| H | Pr-i | Me | Et | H |
| H | Pr-i | Me | Pr-n | H |
| H | Pr-i | Me | Pr-i | H |
| H | Pr-i | Et | Me | H |
| H | Pr-i | Et | Et | H |
| H | Pr-i | Et | Pr-n | H |
| H | Pr-i | Et | Pr-i | H |
| H | Pr-i | Pr-n | Me | H |
| H | Pr-i | Pr-n | Et | H |
| H | Pr-i | Pr-n | Pr-n | H |
| H | Pr-i | Pr-n | Pr-i | H |
| H | Pr-i | Pr-i | Me | H |
| H | Pr-i | Pr-i | Et | H |
| H | Pr-i | Pr-i | Pr-n | H |
| H | Pr-i | Pr-i | Pr-i | H |
| F | H | H | Me | H |
| Cl | H | H | Me | H |
| Br | H | H | Me | H |
| I | H | H | Me | H |
| $CH=CH_2$ | H | H | Me | H |
| $CH_2CH=CH_2$ | H | H | Me | H |
| $C\equiv CH$ | H | H | Me | H |
| $CH_2C\equiv CH$ | H | H | Me | H |
| $CH_2F$ | H | H | Me | H |
| $CHF_2$ | H | H | Me | H |
| CN | H | H | Me | H |
| $NH_2$ | H | H | Me | H |
| NHCOMe | H | H | Me | H |
| $CH_2NH_2$ | H | H | Me | H |
| $CH_2NHCOMe$ | H | H | Me | H |
| $CH_2OCHF_2$ | H | H | Me | H |
| $CH_2OCF_3$ | H | H | Me | H |
| $CF_3$ | H | H | Me | H |
| $NO_2$ | H | H | Me | H |
| OMe | H | H | Me | H |
| OEt | H | H | Me | H |

| $R_1$ | $R_2$ | $R_3$ | $R_4$ | $R_5$ |
|---|---|---|---|---|
| OPr-n | H | H | Me | H |
| SMe | H | H | Me | H |
| SEt | H | H | Me | H |
| SPr-n | H | H | Me | H |
| SBu-n | H | H | Me | H |
| SPen-n | H | H | Me | H |
| $SO_2Me$ | H | H | Me | H |
| $SO_2Et$ | H | H | Me | H |
| $SO_2Pr$-n | H | H | Me | H |
| COMe | H | H | Me | H |
| COEt | H | H | Me | H |
| COPr-n | H | H | Me | H |
| $CO_2H$ | H | H | Me | H |
| $CO_2Me$ | H | H | Me | H |
| $CO_2Et$ | H | H | Me | H |
| $CO_2Pr$-n | H | H | Me | H |
| $CO_2Pr$-i | H | H | Me | H |
| $CO_2Bu$-n | H | H | Me | H |
| $CO_2Bu$-t | H | H | Me | H |
| $CO_2Bu$-sec | H | H | Me | H |
| $CO_2Pen$-n | H | H | Me | H |
| $CH_2Cl$ | H | H | Me | H |
| $CH_2Br$ | H | H | Me | H |
| $CH_2I$ | H | H | Me | H |
| $CH_2CF_3$ | H | H | Me | H |
| $CH_2CN$ | H | H | Me | H |
| $CH_2OH$ | H | H | Me | H |
| $CH_2OMe$ | H | H | Me | H |
| $CH_2OEt$ | H | H | Me | H |
| $CH_2OPr$-n | H | H | Me | H |
| $CH_2OPr$-i | H | H | Me | H |
| $CH_2SMe$ | H | H | Me | H |
| $CH_2SEt$ | H | H | Me | H |
| $CH_2SPr$-n | H | H | Me | H |
| $CH_2SPr$-i | H | H | Me | H |
| $CH_2SO_2Me$ | H | H | Me | H |
| $CH_2SO_2Et$ | H | H | Me | H |
| $CH_2SO_2Pr$-n | H | H | Me | H |
| $CH_2SO_2Pr$-i | H | H | Me | H |
| $CH_2COMe$ | H | H | Me | H |

| $R_1$ | $R_2$ | $R_3$ | $R_4$ | $R_5$ |
|---|---|---|---|---|
| $CH_2COEt$ | H | H | Me | H |
| $CH_2COPr$-n | H | H | Me | H |
| $CH_2COPr$-i | H | H | Me | H |
| $CH_2CO_2H$ | H | H | Me | H |
| $CH_2CO_2Me$ | H | H | Me | H |
| $CH_2CO_2Et$ | H | H | Me | H |
| $CH_2CO_2Pr$-n | H | H | Me | H |
| $CH_2CO_2Pr$-i | H | H | Me | H |
| $CH_2OCH_2CH=CH_2$ | H | H | Me | H |
| $CH_2OCH_2C\equiv CH$ | H | H | Me | H |
| $CH=CHCO_2Me$ | H | H | Me | H |
| $CH=CHCO_2Et$ | H | H | Me | H |
| $CH=CHCO_2Pr$-n | H | H | Me | H |
| $CH=CHCO_2Pr$-i | H | H | Me | H |
| CH=CHCN | H | H | Me | H |
| Ph | H | H | Me | H |
| Ph-2-F | H | H | Me | H |
| Ph-3-F | H | H | Me | H |
| Ph-4-F | H | H | Me | H |
| Ph-2-Cl | H | H | Me | H |
| Ph-3-Cl | H | H | Me | H |
| Ph-4-Cl | H | H | Me | H |
| Ph-2-Br | H | H | Me | H |
| Ph-3-Br | H | H | Me | H |
| Ph-4-Br | H | H | Me | H |
| Ph-2-$CF_3$ | H | H | Me | H |
| Ph-3-$CF_3$ | H | H | Me | H |
| Ph-4-$CF_3$ | H | H | Me | H |
| Ph-2-Me | H | H | Me | H |
| Ph-3-Me | H | H | Me | H |
| Ph-4-Me | H | H | Me | H |
| Ph-2-Et | H | H | Me | H |
| Ph-2-OMe | H | H | Me | H |
| Ph-3-OMe | H | H | Me | H |
| Ph-4-OMe | H | H | Me | H |
| Ph-2-OEt | H | H | Me | H |
| Ph-2-$CO_2Me$ | H | H | Me | H |
| Ph-2-$CO_2Et$ | H | H | Me | H |
| Ph-3-$CO_2Me$ | H | H | Me | H |
| Ph-4-$CO_2Me$ | H | H | Me | H |

| $R_1$ | $R_2$ | $R_3$ | $R_4$ | $R_5$ |
|---|---|---|---|---|
| $CH_2Ph$ | H | H | Me | H |
| $CH_2Ph$-2-F | H | H | Me | H |
| $CH_2Ph$-3-F | H | H | Me | H |
| $CH_2Ph$-4-F | H | H | Me | H |
| $CH_2Ph$-2-Cl | H | H | Me | H |
| $CH_2Ph$-3-Cl | H | H | Me | H |
| $CH_2Ph$-4-Cl | H | H | Me | H |
| $CH_2Ph$-2-Br | H | H | Me | H |
| $CH_2Ph$-3-Br | H | H | Me | H |
| $CH_2Ph$-4-Br | H | H | Me | H |
| $CH_2Ph$-2-$CF_3$ | H | H | Me | H |
| $CH_2Ph$-3-$CF_3$ | H | H | Me | H |
| $CH_2Ph$-4-$CF_3$ | H | H | Me | H |
| $CH_2Ph$-2-Me | H | H | Me | H |
| $CH_2Ph$-3-Me | H | H | Me | H |
| $CH_2Ph$-4-Me | H | H | Me | H |
| $CH_2Ph$-2-Et | H | H | Me | H |
| $CH_2Ph$-2-OMe | H | H | Me | H |
| $CH_2Ph$-3-OMe | H | H | Me | H |
| $CH_2Ph$-4-OMe | H | H | Me | H |
| $CH_2Ph$-2-OEt | H | H | Me | H |
| $CH_2Ph$-2-$CO_2Me$ | H | H | Me | H |
| $CH_2Ph$-2-$CO_2Et$ | H | H | Me | H |
| $CH_2Ph$-3-$CO_2Me$ | H | H | Me | H |
| $CH_2Ph$-4-$CO_2Me$ | H | H | Me | H |
| OPh | H | H | Me | H |
| OPh-2-Cl | H | H | Me | H |
| OPh-3-Cl | H | H | Me | H |
| OPh-4-Cl | H | H | Me | H |
| OPh-2-F | H | H | Me | H |
| OPh-3-F | H | H | Me | H |
| OPh-4-F | H | H | Me | H |
| OPh-2-$CF_3$ | H | H | Me | H |
| OPh-3-$CF_3$ | H | H | Me | H |
| OPh-4-$CF_3$ | H | H | Me | H |
| OPh-2-Me | H | H | Me | H |
| OPh-3-Me | H | H | Me | H |
| OPh-4-Me | H | H | Me | H |
| OPh-2-Et | H | H | Me | H |
| OPh-2-OMe | H | H | Me | H |

| $R_1$ | $R_2$ | $R_3$ | $R_4$ | $R_5$ |
|---|---|---|---|---|
| OPh-3-OMe | H | H | Me | H |
| OPh-4-OMe | H | H | Me | H |
| OPh-2-OEt | H | H | Me | H |
| OPh-2-$CO_2$Me | H | H | Me | H |
| OPh-3-$CO_2$Me | H | H | Me | H |
| OPh-4-$CO_2$Me | H | H | Me | H |
| OPh-2-$CO_2$Et | H | H | Me | H |
| SPh | H | H | Me | H |
| SPh-2-Cl | H | H | Me | H |
| SPh-3-Cl | H | H | Me | H |
| SPh-4-Cl | H | H | Me | H |
| SPh-2-F | H | H | Me | H |
| SPh-3-F | H | H | Me | H |
| SPh-4-F | H | H | Me | H |
| SPh-2-$CF_3$ | H | H | Me | H |
| SPh-3-$CF_3$ | H | H | Me | H |
| SPh-4-$CF_3$ | H | H | Me | H |
| SPh-2-Me | H | H | Me | H |
| SPh-3-Me | H | H | Me | H |
| SPh-4-Me | H | H | Me | H |
| SPh-2-Et | H | H | Me | H |
| SPh-2-OMe | H | H. | Me | H |
| SPh-3-OMe | H | H | Me | H |
| SPh-4-OMe | H | H | Me | H |
| SPh-2-OEt | H | H | Me | H |
| SPh-2-$CO_2$Me | H | H | Me | H |
| SPh-3-$CO_2$Me | H | H | Me | H |
| SPh-4-$CO_2$Me | H | H | Me | H |
| SPh-4-$CO_2$Et | H | H | Me | H |
| $SO_2$Ph | H | H | Me | H |
| $SO_2$Ph-2-Cl | H | H | Me | H |
| $SO_2$Ph-3-Cl | H | H | Me | H |
| $SO_2$Ph-4-Cl | H | H | Me | H |
| $SO_2$Ph-2-F | H | H | Me | H |
| $SO_2$Ph-3-F | H | H | Me | H |
| $SO_2$Ph-4-F | H | H | Me | H |
| $SO_2$Ph-2-$CF_3$ | H | H | Me | H |
| $SO_2$Ph-3-$CF_3$ | H | H | Me | H |
| $SO_2$Ph-4-$CF_3$ | H | H | Me | H |
| $SO_2$Ph-2-Me | H | H | Me | H |

| $R_1$ | $R_2$ | $R_3$ | $R_4$ | $R_5$ |
|---|---|---|---|---|
| $SO_2Ph$-3-Me | H | H | Me | H |
| $SO_2Ph$-4-Me | H | H | Me | H |
| $SO_2Ph$-2-Et | H | H | Me | H |
| $SO_2Ph$-2-OMe | H | H | Me | H |
| $SO_2Ph$-3-OMe | H | H | Me | H |
| $SO_2Ph$-4-OMe | H | H | Me | H |
| $SO_2Ph$-2-OEt | H | H | Me | H |
| $SO_2Ph$-2-$CO_2Me$ | H | H | Me | H |
| $SO_2Ph$-3-$CO_2Me$ | H | H | Me | H |
| $SO_2Ph$-4-$CO_2Me$ | H | H | Me | H |
| $SO_2Ph$-2-$CO_2Et$ | H | H | Me | H |
| CONHMe | H | H | Me | H |
| CONHEt | H | H | Me | H |
| CONHPr-n | H | H | Me | H |
| CONHPr-i | H | H | Me | H |
| CONHBu-n | H | H | Me | H |
| CONHBu-sec | H | H | Me | H |
| CONHBu-t | H | H | Me | H |
| $CON(Me)_2$ | H | H | Me | H |
| $CON(Et)_2$ | H | H | Me | H |
| $CON(Pr\text{-}n)_2$ | H | H | Me | H |
| F | H | H | Et | H |
| Cl | H | H | Et | H |
| Br | H | H | Et | H |
| I | H | H | Et | H |
| $CH=CH_2$ | H | H | Et | H |
| $CH_2CH=CH_2$ | H | H | Et | H |
| $C\equiv CH$ | H | H | Et | H |
| $CH_2C\equiv CH$ | H | H | Et | H |
| $CH_2F$ | H | H | Et | H |
| $CHF_2$ | H | H | Et | H |
| CN | H | H | Et | H |
| $NH_2$ | H | H | Et | H |
| NHCOMe | H | H | Et | H |
| $CH_2NH_2$ | H | H | Et | H |
| $CH_2NHCOMe$ | H | H | Et | H |
| $CH_2OCHF_2$ | H | H | Et | H |
| $CH_2OCF_3$ | H | H | Et | H |
| $CF_3$ | H | H | Et | H |
| $NO_2$ | H | H | Et | H |

| $R_1$ | $R_2$ | $R_3$ | $R_4$ | $R_5$ |
|---|---|---|---|---|
| OMe | H | H | Et | H |
| OEt | H | H | Et | H |
| OPr-n | H | H | Et | H |
| SMe | H | H | Et | H |
| SEt | H | H | Et | H |
| SPr-n | H | H | Et | H |
| SBu-n | H | H | Et | H |
| SPen-n | H | H | Et | H |
| $SO_2Me$ | H | H | Et | H |
| $SO_2Et$ | H | H | Et | H |
| $SO_2Pr$-n | H | H | Et | H |
| COMe | H | H | Et | H |
| COEt | H | H | Et | H |
| COPr-n | H | H | Et | H |
| $CO_2H$ | H | H | Et | H |
| $CO_2Me$ | H | H | Et | H |
| $CO_2Et$ | H | H | Et | H |
| $CO_2Pr$-n | H | H | Et | H |
| $CO_2Pr$-i | H | H | Et | H |
| $CO_2Bu$-n | H | H | Et | H |
| $CO_2Bu$-t | H | H | Et | H |
| $CO_2Bu$-sec | H | H | Et | H |
| $CO_2Pen$-n | H | H | Et | H |
| $CH_2Cl$ | H | H | Et | H |
| $CH_2Br$ | H | H | Et | H |
| $CH_2I$ | H | H | Et | H |
| $CH_2CF_3$ | H | H | Et | H |
| $CH_2CN$ | H | H | Et | H |
| $CH_2OH$ | H | H | Et | H |
| $CH_2OMe$ | H | H | Et | H |
| $CH_2OEt$ | H | H | Et | H |
| $CH_2OPr$-n | H | H | Et | H |
| $CH_2OPr$-i | H | H | Et | H |
| $CH_2SMe$ | H | H | Et | H |
| $CH_2SEt$ | H | H | Et | H |
| $CH_2SPr$-n | H | H | Et | H |
| $CH_2SPr$-i | H | H | Et | H |
| $CH_2SO_2Me$ | H | H | Et | H |
| $CH_2SO_2Et$ | H | H | Et | H |
| $CH_2SO_2Pr$-n | H | H | Et | H |

| $R_1$ | $R_2$ | $R_3$ | $R_4$ | $R_5$ |
|---|---|---|---|---|
| $CH_2SO_2Pr$-i | H | H | Et | H |
| $CH_2COMe$ | H | H | Et | H |
| $CH_2COEt$ | H | H | Et | H |
| $CH_2COPr$-n | H | H | Et | H |
| $CH_2COPr$-i | H | H | Et | H |
| $CH_2CO_2H$ | H | H | Et | H |
| $CH_2CO_2Me$ | H | H | Et | H |
| $CH_2CO_2Et$ | H | H | Et | H |
| $CH_2CO_2Pr$-n | H | H | Et | H |
| $CH_2CO_2Pr$-i | H | H | Et | H |
| $CH_2OCH_2CH=CH_2$ | H | H | Et | H |
| $CH_2OCH_2C\equiv CH$ | H | H | Et | H |
| $CH=CHCO_2Me$ | H | H | Et | H |
| $CH=CHCO_2Et$ | H | H | Et | H |
| $CH=CHCO_2Pr$-n | H | H | Et | H |
| $CH=CHCO_2Pr$-i | H | H | Et | H |
| CH=CHCN | H | H | Et | H |
| Ph | H | H | Et | H |
| $CH_2Ph$ | H | H | Et | H |
| OPh | H | H | Et | H |
| SPh | H | H | Et | H |
| $SO_2Ph$ | H | H | Et | H |
| CONHMe | H | H | Et | H |
| CONHEt | H | H | Et | H |
| CONHPr-n | H | H | Et | H |
| CONHPr-i | H | H | Et | H |
| CONHBu-n | H | H | Et | H |
| CONHBu-sec | H | H | Et | H |
| CONHBu-t | H | H | Et | H |
| $CON(Me)_2$ | H | H | Et | H |
| $CON(Et)_2$ | H | H | Et | H |
| $CON(Pr$-$n)_2$ | H | H | Et | H |
| F | H | H | Cl | H |
| Cl | H | H | Cl | H |
| Br | H | H | Cl | H |
| I | H | H | Cl | H |
| $CH=CH_2$ | H | H | Cl | H |
| $CH_2CH=CH_2$ | H | H | Cl | H |
| $C\equiv CH$ | H | H | Cl | H |
| $CH_2C\equiv CH$ | H | H | Cl | H |

| $R_1$ | $R_2$ | $R_3$ | $R_4$ | $R_5$ |
|---|---|---|---|---|
| $CH_2F$ | H | H | Cl | H |
| $CHF_2$ | H | H | Cl | H |
| CN | H | H | Cl | H |
| $NH_2$ | H | H | Cl | H |
| NHCOMe | H | H | Cl | H |
| $CH_2NH_2$ | H | H | Cl | H |
| $CH_2NHCOMe$ | H | H | Cl | H |
| $CH_2OCHF_2$ | H | H | Cl | H |
| $CH_2OCF_3$ | H | H | Cl | H |
| $CF_3$ | H | H | Cl | H |
| $NO_2$ | H | H | Cl | H |
| OMe | H | H | Cl | H |
| OEt | H | H | Cl | H |
| OPr-n | H | H | Cl | H |
| SMe | H | H | Cl | H |
| SEt | H | H | Cl | H |
| SPr-n | H | H | Cl | H |
| SBu-n | H | H | Cl | H |
| SPen-n | H | H | Cl | H |
| $SO_2Me$ | H | H | Cl | H |
| $SO_2Et$ | H | H | Cl | H |
| $SO_2Pr$-n | H | H | Cl | H |
| COMe | H | H | Cl | H |
| COEt | H | H | Cl | H |
| COPr-n | H | H | Cl | H |
| $CO_2H$ | H | H | Cl | H |
| $CO_2Me$ | H | H | Cl | H |
| $CO_2Et$ | H | H | Cl | H |
| $CO_2Pr$-n | H | H | Cl | H |
| $CO_2Pr$-i | H | H | Cl | H |
| $CO_2Bu$-n | H | H | Cl | H |
| $CO_2Bu$-t | H | H | Cl | H |
| $CO_2Bu$-sec | H | H | Cl | H |
| $CO_2Pen$-n | H | H | Cl | H |
| $CH_2Cl$ | H | H | Cl | H |
| $CH_2Br$ | H | H | Cl | H |
| $CH_2I$ | H | H | Cl | H |
| $CH_2CF_3$ | H | H | Cl | H |
| $CH_2CN$ | H | H | Cl | H |
| $CH_2OH$ | H | H | Cl | H |

| $R_1$ | $R_2$ | $R_3$ | $R_4$ | $R_5$ |
|---|---|---|---|---|
| $CH_2OMe$ | H | H | Cl | H |
| $CH_2OEt$ | H | H | Cl | H |
| $CH_2OPr$-n | H | H | Cl | H |
| $CH_2OPr$-i | H | H | Cl | H |
| $CH_2SMe$ | H | H | Cl | H |
| $CH_2SEt$ | H | H | Cl | H |
| $CH_2SPr$-n | H | H | Cl | H |
| $CH_2SPr$-i | H | H | Cl | H |
| $CH_2SO_2Me$ | H | H | Cl | H |
| $CH_2SO_2Et$ | H | H | Cl | H |
| $CH_2SO_2Pr$-n | H | H | Cl | H |
| $CH_2SO_2Pr$-i | H | H | Cl | H |
| $CH_2COMe$ | H | H | Cl | H |
| $CH_2COEt$ | H | H | Cl | H |
| $CH_2COPr$-n | H | H | Cl | H |
| $CH_2COPr$-i | H | H | Cl | H |
| $CH_2CO_2H$ | H | H | Cl | H |
| $CH_2CO_2Me$ | H | H | Cl | H |
| $CH_2CO_2Et$ | H | H | Cl | H |
| $CH_2CO_2Pr$-n | H | H | Cl | H |
| $CH_2CO_2Pr$-i | H | H | Cl | H |
| $CH_2OCH_2CH=CH_2$ | H | H | Cl | H |
| $CH_2OCH_2C\equiv CH$ | H | H | Cl | H |
| $CH=CHCO_2Me$ | H | H | Cl | H |
| $CH=CHCO_2Et$ | H | H | Cl | H |
| $CH=CHCO_2Pr$-n | H | H | Cl | H |
| $CH=CHCO_2Pr$-i | H | H | Cl | H |
| CH=CHCN | H | H | Cl | H |
| Ph | H | H | Cl | H |
| $CH_2Ph$ | H | H | Cl | H |
| OPh | H | H | Cl | H |
| SPh | H | H | Cl | H |
| $SO_2Ph$ | H | H | Cl | H |
| CONHMe | H | H | Cl | H |
| CONHEt | H | H | Cl | H |
| CONHPr-n | H | H | Cl | H |
| CONHPr-i | H | H | Cl | H |
| CONHBu-n | H | H | Cl | H |
| CONHBu-sec | H | H | Cl | H |
| CONHBu-t | H | H | Cl | H |

| $R_1$ | $R_2$ | $R_3$ | $R_4$ | $R_5$ |
|---|---|---|---|---|
| $CON(Me)_2$ | H | H | Cl | H |
| $CON(Et)_2$ | H | H | Cl | H |
| $CON(Pr-n)_2$ | H | H | Cl | H |
| F | H | H | $CO_2Me$ | H |
| Cl | H | H | $CO_2Me$ | H |
| Br | H | H | $CO_2Me$ | H |
| I | H | H | $CO_2Me$ | H |
| $CH=CH_2$ | H | H | $CO_2Me$ | H |
| $CH_2CH=CH_2$ | H | H | $CO_2Me$ | H |
| $C\equiv CH$ | H | H | $CO_2Me$ | H |
| $CH_2C\equiv CH$ | H | H | $CO_2Me$ | H |
| $CH_2F$ | H | H | $CO_2Me$ | H |
| $CHF_2$ | H | H | $CO_2Me$ | H |
| CN | H | H | $CO_2Me$ | H |
| $NH_2$ | H | H | $CO_2Me$ | H |
| NHCOMe | H | H | $CO_2Me$ | H |
| $CH_2NH_2$ | H | H | $CO_2Me$ | H |
| $CH_2NHCOMe$ | H | H | $CO_2Me$ | H |
| $CH_2OCHF_2$ | H | H | $CO_2Me$ | H |
| $CH_2OCF_3$ | H | H | $CO_2Me$ | H |
| $CF_3$ | H | H | $CO_2Me$ | H |
| $NO_2$ | H | H | $CO_2Me$ | H |
| OMe | H | H | $CO_2Me$ | H |
| OEt | H | H | $CO_2Me$ | H |
| OPr-n | H | H | $CO_2Me$ | H |
| SMe | H | H | $CO_2Me$ | H |
| SEt | H | H | $CO_2Me$ | H |
| SPr-n | H | H | $CO_2Me$ | H |
| SBu-n | H | H | $CO_2Me$ | H |
| SPen-n | H | H | $CO_2Me$ | H |
| $SO_2Me$ | H | H | $CO_2Me$ | H |
| $SO_2Et$ | H | H | $CO_2Me$ | H |
| $SO_2Pr-n$ | H | H | $CO_2Me$ | H |
| COMe | H | H | $CO_2Me$ | H |
| COEt | H | H | $CO_2Me$ | H |
| COPr-n | H | H | $CO_2Me$ | H |
| $CO_2H$ | H | H | $CO_2Me$ | H |
| $CO_2Me$ | H | H | $CO_2Me$ | H |
| $CO_2Et$ | H | H | $CO_2Me$ | H |
| $CO_2Pr-n$ | H | H | $CO_2Me$ | H |

| $R_1$ | $R_2$ | $R_3$ | $R_4$ | $R_5$ |
|---|---|---|---|---|
| $CO_2Pr$-i | H | H | $CO_2Me$ | H |
| $CO_2Bu$-n | H | H | $CO_2Me$ | H |
| $CO_2Bu$-t | H | H | $CO_2Me$ | H |
| $CO_2Bu$-sec | H | H | $CO_2Me$ | H |
| $CO_2Pen$-n | H | H | $CO_2Me$ | H |
| $CH_2Cl$ | H | H | $CO_2Me$ | H |
| $CH_2Br$ | H | H | $CO_2Me$ | H |
| $CH_2I$ | H | H | $CO_2Me$ | H |
| $CH_2CF_3$ | H | H | $CO_2Me$ | H |
| $CH_2CN$ | H | H | $CO_2Me$ | H |
| $CH_2OH$ | H | H | $CO_2Me$ | H |
| $CH_2OMe$ | H | H | $CO_2Me$ | H |
| $CH_2OEt$ | H | H | $CO_2Me$ | H |
| $CH_2OPr$-n | H | H | $CO_2Me$ | H |
| $CH_2OPr$-i | H | H | $CO_2Me$ | H |
| $CH_2SMe$ | H | H | $CO_2Me$ | H |
| $CH_2SEt$ | H | H | $CO_2Me$ | H |
| $CH_2SPr$-n | H | H | $CO_2Me$ | H |
| $CH_2SPr$-i | H | H | $CO_2Me$ | H |
| $CH_2SO_2Me$ | H | H | $CO_2Me$ | H |
| $CH_2SO_2Et$ | H | H | $CO_2Me$ | H |
| $CH_2SO_2Pr$-n | H | H | $CO_2Me$ | H |
| $CH_2SO_2Pr$-i | H | H | $CO_2Me$ | H |
| $CH_2COMe$ | H | H | $CO_2Me$ | H |
| $CH_2COEt$ | H | H | $CO_2Me$ | H |
| $CH_2COPr$-n | H | H | $CO_2Me$ | H |
| $CH_2COPr$-i | H | H | $CO_2Me$ | H |
| $CH_2CO_2H$ | H | H | $CO_2Me$ | H |
| $CH_2CO_2Me$ | H | H | $CO_2Me$ | H |
| $CH_2CO_2Et$ | H | H | $CO_2Me$ | H |
| $CH_2CO_2Pr$-n | H | H | $CO_2Me$ | H |
| $CH_2CO_2Pr$-i | H | H | $CO_2Me$ | H |
| $CH_2OCH_2CH=CH_2$ | H | H | $CO_2Me$ | H |
| $CH_2OCH_2C\equiv CH$ | H | H | $CO_2Me$ | H |
| $CH=CHCO_2Me$ | H | H | $CO_2Me$ | H |
| $CH=CHCO_2Et$ | H | H | $CO_2Me$ | H |
| $CH=CHCO_2Pr$-n | H | H | $CO_2Me$ | H |
| $CH=CHCO_2Pr$-i | H | H | $CO_2Me$ | H |
| $CH=CHCN$ | H | H | $CO_2Me$ | H |
| Ph | H | H | $CO_2Me$ | H |

| $R_1$ | $R_2$ | $R_3$ | $R_4$ | $R_5$ |
|---|---|---|---|---|
| $CH_2Ph$ | H | H | $CO_2Me$ | H |
| OPh | H | H | $CO_2Me$ | H |
| SPh | H | H | $CO_2Me$ | H |
| $SO_2Ph$ | H | H | $CO_2Me$ | H |
| CONHMe | H | H | $CO_2Me$ | H |
| CONHEt | H | H | $CO_2Me$ | H |
| CONHPr-n | H | H | $CO_2Me$ | H |
| CONHPr-i | H | H | $CO_2Me$ | H |
| CONHBu-n | H | H | $CO_2Me$ | H |
| CONHBu-sec | H | H | $CO_2Me$ | H |
| CONHBu-t | H | H | $CO_2Me$ | H |
| $CON(Me)_2$ | H | H | $CO_2Me$ | H |
| $CON(Et)_2$ | H | H | $CO_2Me$ | H |
| $CON(Pr\text{-}n)_2$ | H | H | $CO_2Me$ | H |
| F | H | H | $CH_2Cl$ | H |
| Cl | H | H | $CH_2Cl$ | H |
| Br | H | H | $CH_2Cl$ | H |
| I | H | H | $CH_2Cl$ | H |
| $CH=CH_2$ | H | H | $CH_2Cl$ | H |
| $CH_2CH=CH_2$ | H | H | $CH_2Cl$ | H |
| $C\equiv CH$ | H | H | $CH_2Cl$ | H |
| $CH_2C\equiv CH$ | H | H | $CH_2Cl$ | H |
| $CH_2F$ | H | H | $CH_2Cl$ | H |
| $CHF_2$ | H | H | $CH_2Cl$ | H |
| CN | H | H | $CH_2Cl$ | H |
| $NH_2$ | H | H | $CH_2Cl$ | H |
| NHCOMe | H | H | $CH_2Cl$ | H |
| $CH_2NH_2$ | H | H | $CH_2Cl$ | H |
| $CH_2NHCOMe$ | H | H | $CH_2Cl$ | H |
| $CH_2OCHF_2$ | H | H | $CH_2Cl$ | H |
| $CH_2OCF_3$ | H | H | $CH_2Cl$ | H |
| $CF_3$ | H | H | $CH_2Cl$ | H |
| $NO_2$ | H | H | $CH_2Cl$ | H |
| OMe | H | H | $CH_2Cl$ | H |
| OEt | H | H | $CH_2Cl$ | H |
| OPr-n | H | H | $CH_2Cl$ | H |
| SMe | H | H | $CH_2Cl$ | H |
| SEt | H | H | $CH_2Cl$ | H |
| SPr-n | H | H | $CH_2Cl$ | H |
| SBu-n | H | H | $CH_2Cl$ | H |

| $R_1$ | $R_2$ | $R_3$ | $R_4$ | $R_5$ |
|---|---|---|---|---|
| SPen-n | H | H | $CH_2Cl$ | H |
| $SO_2Me$ | H | H | $CH_2Cl$ | H |
| $SO_2Et$ | H | H | $CH_2Cl$ | H |
| $SO_2Pr$-n | H | H | $CH_2Cl$ | H |
| COMe | H | H | $CH_2Cl$ | H |
| COEt | H | H | $CH_2Cl$ | H |
| COPr-n | H | H | $CH_2Cl$ | H |
| $CO_2H$ | H | H | $CH_2Cl$ | H |
| $CO_2Me$ | H | H | $CH_2Cl$ | H |
| $CO_2Et$ | H | H | $CH_2Cl$ | H |
| $CO_2Pr$-n | H | H | $CH_2Cl$ | H |
| $CO_2Pr$-i | H | H | $CH_2Cl$ | H |
| $CO_2Bu$-n | H | H | $CH_2Cl$ | H |
| $CO_2Bu$-t | H | H | $CH_2Cl$ | H |
| $CO_2Bu$-sec | H | H | $CH_2Cl$ | H |
| $CO_2Pen$-n | H | H | $CH_2Cl$ | H |
| $CH_2Cl$ | H | H | $CH_2Cl$ | H |
| $CH_2Br$ | H | H | $CH_2Cl$ | H |
| $CH_2I$ | H | H | $CH_2Cl$ | H |
| $CH_2CF_3$ | H | H | $CH_2Cl$ | H |
| $CH_2CN$ | H | H | $CH_2Cl$ | H |
| $CH_2OH$ | H | H | $CH_2Cl$ | H |
| $CH_2OMe$ | H | H | $CH_2Cl$ | H |
| $CH_2OEt$ | H | H | $CH_2Cl$ | H |
| $CH_2OPr$-n | H | H | $CH_2Cl$ | H |
| $CH_2OPr$-i | H | H | $CH_2Cl$ | H |
| $CH_2SMe$ | H | H | $CH_2Cl$ | H |
| $CH_2SEt$ | H | H | $CH_2Cl$ | H |
| $CH_2SPr$-n | H | H | $CH_2Cl$ | H |
| $CH_2SPr$-i | H | H | $CH_2Cl$ | H |
| $CH_2SO_2Me$ | H | H | $CH_2Cl$ | H |
| $CH_2SO_2Et$ | H | H | $CH_2Cl$ | H |
| $CH_2SO_2Pr$-n | H | H | $CH_2Cl$ | H |
| $CH_2SO_2Pr$-i | H | H | $CH_2Cl$ | H |
| $CH_2COMe$ | H | H | $CH_2Cl$ | H |
| $CH_2COEt$ | H | H | $CH_2Cl$ | H |
| $CH_2COPr$-n | H | H | $CH_2Cl$ | H |
| $CH_2COPr$-i | H | H | $CH_2Cl$ | H |
| $CH_2CO_2H$ | H | H | $CH_2Cl$ | H |
| $CH_2CO_2Me$ | H | H | $CH_2Cl$ | H |

| $R_1$ | $R_2$ | $R_3$ | $R_4$ | $R_5$ |
|---|---|---|---|---|
| $CH_2CO_2Et$ | H | H | $CH_2Cl$ | H |
| $CH_2CO_2Pr$-n | H | H | $CH_2Cl$ | H |
| $CH_2CO_2Pr$-i | H | H | $CH_2Cl$ | H |
| $CH_2OCH_2CH=CH_2$ | H | H | $CH_2Cl$ | H |
| $CH_2OCH_2C\equiv CH$ | H | H | $CH_2Cl$ | H |
| $CH=CHCO_2Me$ | H | H | $CH_2Cl$ | H |
| $CH=CHCO_2Et$ | H | H | $CH_2Cl$ | H |
| $CH=CHCO_2Pr$-n | H | H | $CH_2Cl$ | H |
| $CH=CHCO_2Pr$-i | H | H | $CH_2Cl$ | H |
| CH=CHCN | H | H | $CH_2Cl$ | H |
| Ph | H | H | $CH_2Cl$ | H |
| $CH_2Ph$ | H | H | $CH_2Cl$ | H |
| OPh | H | H | $CH_2Cl$ | H |
| SPh | H | H | $CH_2Cl$ | H |
| $SO_2Ph$ | H | H | $CH_2Cl$ | H |
| CONHMe | H | H | $CH_2Cl$ | H |
| CONHEt | H | H | $CH_2Cl$ | H |
| CONHPr-n | H | H | $CH_2Cl$ | H |
| CONHPr-i | H | H | $CH_2Cl$ | H |
| CONHBu-n | H | H | $CH_2Cl$ | H |
| CONHBu-sec | H | H | $CH_2Cl$ | H |
| CONHBu-t | H | H | $CH_2Cl$ | H |
| $CON(Me)_2$ | H | H | $CH_2Cl$ | H |
| $CON(Et)_2$ | H | H | $CH_2Cl$ | H |
| $CON(Pr\text{-}n)_2$ | H | H | $CH_2Cl$ | H |
| F | H | H | $CH_2OMe$ | H |
| Cl | H | H | $CH_2OMe$ | H |
| Br | H | H | $CH_2OMe$ | H |
| I | H | H | $CH_2OMe$ | H |
| $CH=CH_2$ | H | H | $CH_2OMe$ | H |
| $CH_2CH=CH_2$ | H | H | $CH_2OMe$ | H |
| $C\equiv CH$ | H | H | $CH_2OMe$ | H |
| $CH_2C\equiv CH$ | H | H | $CH_2OMe$ | H |
| $CH_2F$ | H | H | $CH_2OMe$ | H |
| $CHF_2$ | H | H | $CH_2OMe$ | H |
| CN | H | H | $CH_2OMe$ | H |
| $NH_2$ | H | H | $CH_2OMe$ | H |
| NHCOMe | H | H | $CH_2OMe$ | H |
| $CH_2NH_2$ | H | H | $CH_2OMe$ | H |
| $CH_2NHCOMe$ | H | H | $CH_2OMe$ | H |

| $R_1$ | $R_2$ | $R_3$ | $R_4$ | $R_5$ |
|---|---|---|---|---|
| $CH_2OCHF_2$ | H | H | $CH_2OMe$ | H |
| $CH_2OCF_3$ | H | H | $CH_2OMe$ | H |
| $CF_3$ | H | H | $CH_2OMe$ | H |
| $NO_2$ | H | H | $CH_2OMe$ | H |
| OMe | H | H | $CH_2OMe$ | H |
| OEt | H | H | $CH_2OMe$ | H |
| OPr-n | H | H | $CH_2OMe$ | H |
| SMe | H | H | $CH_2OMe$ | H |
| SEt | H | H | $CH_2OMe$ | H |
| SPr-n | H | H | $CH_2OMe$ | H |
| SBu-n | H | H | $CH_2OMe$ | H |
| SPen-n | H | H | $CH_2OMe$ | H |
| $SO_2Me$ | H | H | $CH_2OMe$ | H |
| $SO_2Et$ | H | H | $CH_2OMe$ | H |
| $SO_2Pr$-n | H | H | $CH_2OMe$ | H |
| COMe | H | H | $CH_2OMe$ | H |
| COEt | H | H | $CH_2OMe$ | H |
| COPr-n | H | H | $CH_2OMe$ | H |
| $CO_2H$ | H | H | $CH_2OMe$ | H |
| $CO_2Me$ | H | H | $CH_2OMe$ | H |
| $CO_2Et$ | H | H | $CH_2OMe$ | H |
| $CO_2Pr$-n | H | H | $CH_2OMe$ | H |
| $CO_2Pr$-i | H | H | $CH_2OMe$ | H |
| $CO_2Bu$-n | H | H | $CH_2OMe$ | H |
| $CO_2Bu$-t | H | H | $CH_2OMe$ | H |
| $CO_2Bu$-sec | H | H | $CH_2OMe$ | H |
| $CO_2Pen$-n | H | H | $CH_2OMe$ | H |
| $CH_2Cl$ | H | H | $CH_2OMe$ | H |
| $CH_2Br$ | H | H | $CH_2OMe$ | H |
| $CH_2I$ | H | H | $CH_2OMe$ | H |
| $CH_2CF_3$ | H | H | $CH_2OMe$ | H |
| $CH_2CN$ | H | H | $CH_2OMe$ | H |
| $CH_2OH$ | H | H | $CH_2OMe$ | H |
| $CH_2OMe$ | H | H | $CH_2OMe$ | H |
| $CH_2OEt$ | H | H | $CH_2OMe$ | H |
| $CH_2OPr$-n | H | H | $CH_2OMe$ | H |
| $CH_2OPr$-i | H | H | $CH_2OMe$ | H |
| $CH_2SMe$ | H | H | $CH_2OMe$ | H |
| $CH_2SEt$ | H | H | $CH_2OMe$ | H |
| $CH_2SPr$-n | H | H | $CH_2OMe$ | H |

| $R_1$ | $R_2$ | $R_3$ | $R_4$ | $R_5$ |
|---|---|---|---|---|
| $CH_2SPr$-i | H | H | $CH_2OMe$ | H |
| $CH_2SO_2Me$ | H | H | $CH_2OMe$ | H |
| $CH_2SO_2Et$ | H | H | $CH_2OMe$ | H |
| $CH_2SO_2Pr$-n | H | H | $CH_2OMe$ | H |
| $CH_2SO_2Pr$-i | H | H | $CH_2OMe$ | H |
| $CH_2COMe$ | H | H | $CH_2OMe$ | H |
| $CH_2COEt$ | H | H | $CH_2OMe$ | H |
| $CH_2COPr$-n | H | H | $CH_2OMe$ | H |
| $CH_2COPr$-i | H | H | $CH_2OMe$ | H |
| $CH_2CO_2H$ | H | H | $CH_2OMe$ | H |
| $CH_2CO_2Me$ | H | H | $CH_2OMe$ | H |
| $CH_2CO_2Et$ | H | H | $CH_2OMe$ | H |
| $CH_2CO_2Pr$-n | H | H | $CH_2OMe$ | H |
| $CH_2CO_2Pr$-i | H | H | $CH_2OMe$ | H |
| $CH_2OCH_2CH=CH_2$ | H | H | $CH_2OMe$ | H |
| $CH_2OCH_2C\equiv CH$ | H | H | $CH_2OMe$ | H |
| $CH=CHCO_2Me$ | H | H | $CH_2OMe$ | H |
| $CH=CHCO_2Et$ | H | H | $CH_2OMe$ | H |
| $CH=CHCO_2Pr$-n | H | H | $CH_2OMe$ | H |
| $CH=CHCO_2Pr$-i | H | H | $CH_2OMe$ | H |
| $CH=CHCN$ | H | H | $CH_2OMe$ | H |
| Ph | H | H | $CH_2OMe$ | H |
| $CH_2Ph$ | H | H | $CH_2OMe$ | H |
| OPh | H | H | $CH_2OMe$ | H |
| SPh | H | H | $CH_2OMe$ | H |
| $SO_2Ph$ | H | H | $CH_2OMe$ | H |
| CONHMe | H | H | $CH_2OMe$ | H |
| CONHEt | H | H | $CH_2OMe$ | H |
| CONHPr-n | H | H | $CH_2OMe$ | H |
| CONHPr-i | H | H | $CH_2OMe$ | H |
| CONHBu-n | H | H | $CH_2OMe$ | H |
| CONHBu-sec | H | H | $CH_2OMe$ | H |
| CONHBu-t | H | H | $CH_2OMe$ | H |
| $CON(Me)_2$ | H | H | $CH_2OMe$ | H |
| $CON(Et)_2$ | H | H | $CH_2OMe$ | H |
| $CON(Pr\text{-}n)_2$ | H | H | $CH_2OMe$ | H |
| F | H | H | $CH_2SMe$ | H |
| Cl | H | H | $CH_2SMe$ | H |
| Br | H | H | $CH_2SMe$ | H |
| I | H | H | $CH_2SMe$ | H |

| $R_1$ | $R_2$ | $R_3$ | $R_4$ | $R_5$ |
|---|---|---|---|---|
| $CH=CH_2$ | H | H | $CH_2SMe$ | H |
| $CH_2CH=CH_2$ | H | H | $CH_2SMe$ | H |
| $C\equiv CH$ | H | H | $CH_2SMe$ | H |
| $CH_2C\equiv CH$ | H | H | $CH_2SMe$ | H |
| $CH_2F$ | H | H | $CH_2SMe$ | H |
| $CHF_2$ | H | H | $CH_2SMe$ | H |
| CN | H | H | $CH_2SMe$ | H |
| $NH_2$ | H | H | $CH_2SMe$ | H |
| NHCOMe | H | H | $CH_2SMe$ | H |
| $CH_2NH_2$ | H | H | $CH_2SMe$ | H |
| $CH_2NHCOMe$ | H | H | $CH_2SMe$ | H |
| $CH_2OCHF_2$ | H | H | $CH_2SMe$ | H |
| $CH_2OCF_3$ | H | H | $CH_2SMe$ | H |
| $CF_3$ | H | H | $CH_2SMe$ | H |
| $NO_2$ | H | H | $CH_2SMe$ | H |
| OMe | H | H | $CH_2SMe$ | H |
| OEt | H | H | $CH_2SMe$ | H |
| OPr-n | H | H | $CH_2SMe$ | H |
| SMe | H | H | $CH_2SMe$ | H |
| SEt | H | H | $CH_2SMe$ | H |
| SPr-n | H | H | $CH_2SMe$ | H |
| SBu-n | H | H | $CH_2SMe$ | H |
| SPen-n | H | H | $CH_2SMe$ | H |
| $SO_2Me$ | H | H | $CH_2SMe$ | H |
| $SO_2Et$ | H | H | $CH_2SMe$ | H |
| $SO_2Pr$-n | H | H | $CH_2SMe$ | H |
| COMe | H | H | $CH_2SMe$ | H |
| COEt | H | H | $CH_2SMe$ | H |
| COPr-n | H | H | $CH_2SMe$ | H |
| $CO_2H$ | H | H | $CH_2SMe$ | H |
| $CO_2Me$ | H | H | $CH_2SMe$ | H |
| $CO_2Et$ | H | H | $CH_2SMe$ | H |
| $CO_2Pr$-n | H | H | $CH_2SMe$ | H |
| $CO_2Pr$-i | H | H | $CH_2SMe$ | H |
| $CO_2Bu$-n | H | H | $CH_2SMe$ | H |
| $CO_2Bu$-t | H | H | $CH_2SMe$ | H |
| $CO_2Bu$-sec | H | H | $CH_2SMe$ | H |
| $CO_2Pen$-n | H | H | $CH_2SMe$ | H |
| $CH_2Cl$ | H | H | $CH_2SMe$ | H |
| $CH_2Br$ | H | H | $CH_2SMe$ | H |

| $R_1$ | $R_2$ | $R_3$ | $R_4$ | $R_5$ |
|---|---|---|---|---|
| $CH_2I$ | H | H | $CH_2SMe$ | H |
| $CH_2CF_3$ | H | H | $CH_2SMe$ | H |
| $CH_2CN$ | H | H | $CH_2SMe$ | H |
| $CH_2OH$ | H | H | $CH_2SMe$ | H |
| $CH_2OMe$ | H | H | $CH_2SMe$ | H |
| $CH_2OEt$ | H | H | $CH_2SMe$ | H |
| $CH_2OPr$-n | H | H | $CH_2SMe$ | H |
| $CH_2OPr$-i | H | H | $CH_2SMe$ | H |
| $CH_2SMe$ | H | H | $CH_2SMe$ | H |
| $CH_2SEt$ | H | H | $CH_2SMe$ | H |
| $CH_2SPr$-n | H | H | $CH_2SMe$ | H |
| $CH_2SPr$-i | H | H | $CH_2SMe$ | H |
| $CH_2SO_2Me$ | H | H | $CH_2SMe$ | H |
| $CH_2SO_2Et$ | H | H | $CH_2SMe$ | H |
| $CH_2SO_2Pr$-n | H | H | $CH_2SMe$ | H |
| $CH_2SO_2Pr$-i | H | H | $CH_2SMe$ | H |
| $CH_2COMe$ | H | H | $CH_2SMe$ | H |
| $CH_2COEt$ | H | H | $CH_2SMe$ | H |
| $CH_2COPr$-n | H | H | $CH_2SMe$ | H |
| $CH_2COPr$-i | H | H | $CH_2SMe$ | H |
| $CH_2CO_2H$ | H | H | $CH_2SMe$ | H |
| $CH_2CO_2Me$ | H | H | $CH_2SMe$ | H |
| $CH_2CO_2Et$ | H | H | $CH_2SMe$ | H |
| $CH_2CO_2Pr$-n | H | H | $CH_2SMe$ | H |
| $CH_2CO_2Pr$-i | H | H | $CH_2SMe$ | H |
| $CH_2OCH_2CH=CH_2$ | H | H | $CH_2SMe$ | H |
| $CH_2OCH_2C \equiv CH$ | H | H | $CH_2SMe$ | H |
| $CH=CHCO_2Me$ | H | H | $CH_2SMe$ | H |
| $CH=CHCO_2Et$ | H | H | $CH_2SMe$ | H |
| $CH=CHCO_2Pr$-n | H | H | $CH_2SMe$ | H |
| $CH=CHCO_2Pr$-i | H | H | $CH_2SMe$ | H |
| CH=CHCN | H | H | $CH_2SMe$ | H |
| Ph | H | H | $CH_2SMe$ | H |
| $CH_2Ph$ | H | H | $CH_2SMe$ | H |
| OPh | H | H | $CH_2SMe$ | H |
| SPh | H | H | $CH_2SMe$ | H |
| $SO_2Ph$ | H | H | $CH_2SMe$ | H |
| CONHMe | H | H | $CH_2SMe$ | H |
| CONHEt | H | H | $CH_2SMe$ | H |
| CONHPr-n | H | H | $CH_2SMe$ | H |

| $R_1$ | $R_2$ | $R_3$ | $R_4$ | $R_5$ |
|---|---|---|---|---|
| CONHPr-i | H | H | $CH_2SMe$ | H |
| CONHBu-n | H | H | $CH_2SMe$ | H |
| CONHBu-sec | H | H | $CH_2SMe$ | H |
| CONHBu-t | H | H | $CH_2SMe$ | H |
| $CON(Me)_2$ | H | H | $CH_2SMe$ | H |
| $CON(Et)_2$ | H | H | $CH_2SMe$ | H |
| $CON(Pr-n)_2$ | H | H | $CH_2SMe$ | H |
| F | H | H | CN | H |
| Cl | H | H | CN | H |
| Br | H | H | CN | H |
| I | H | H | CN | H |
| $CH=CH_2$ | H | H | CN | H |
| $CH_2CH=CH_2$ | H | H | CN | H |
| $C\equiv CH$ | H | H | CN | H |
| $CH_2C\equiv CH$ | H | H | CN | H |
| $CH_2F$ | H | H | CN | H |
| $CHF_2$ | H | H | CN | H |
| CN | H | H | CN | H |
| $NH_2$ | H | H | CN | H |
| NHCOMe | H | H | CN | H |
| $CH_2NH_2$ | H | H | CN | H |
| $CH_2NHCOMe$ | H | H | CN | H |
| $CH_2OCHF_2$ | H | H | CN | H |
| $CH_2OCF_3$ | H | H | CN | H |
| $CF_3$ | H | H | CN | H |
| $NO_2$ | H | H | CN | H |
| OMe | H | H | CN | H |
| OEt | H | H | CN | H |
| OPr-n | H | H | CN | H |
| SMe | H | H | CN | H |
| SEt | H | H | CN | H |
| SPr-n | H | H | CN | H |
| SBu-n | H | H | CN | H |
| SPen-n | H | H | CN | H |
| $SO_2Me$ | H | H | CN | H |
| $SO_2Et$ | H | H | CN | H |
| $SO_2Pr-n$ | H | H | CN | H |
| COMe | H | H | CN | H |
| COEt | H | H | CN | H |
| COPr-n | H | H | CN | H |

| $R_1$ | $R_2$ | $R_3$ | $R_4$ | $R_5$ |
|---|---|---|---|---|
| $CO_2H$ | H | H | CN | H |
| $CO_2Me$ | H | H | CN | H |
| $CO_2Et$ | H | H | CN | H |
| $CO_2Pr$-n | H | H | CN | H |
| $CO_2Pr$-i | H | H | CN | H |
| $CO_2Bu$-n | H | H | CN | H |
| $CO_2Bu$-t | H | H | CN | H |
| $CO_2Bu$-sec | H | H | CN | H |
| $CO_2Pen$-n | H | H | CN | H |
| $CH_2Cl$ | H | H | CN | H |
| $CH_2Br$ | H | H | CN | H |
| $CH_2I$ | H | H | CN | H |
| $CH_2CF_3$ | H | H | CN | H |
| $CH_2CN$ | H | H | CN | H |
| $CH_2OH$ | H | H | CN | H |
| $CH_2OMe$ | H | H | CN | H |
| $CH_2OEt$ | H | H | CN | H |
| $CH_2OPr$-n | H | H | CN | H |
| $CH_2OPr$-i | H | H | CN | H |
| $CH_2SMe$ | H | H | CN | H |
| $CH_2SEt$ | H | H | CN | H |
| $CH_2SPr$-n | H | H | CN | H |
| $CH_2SPr$-i | H | H | CN | H |
| $CH_2SO_2Me$ | H | H | CN | H |
| $CH_2SO_2Et$ | H | H | CN | H |
| $CH_2SO_2Pr$-n | H | H | CN | H |
| $CH_2SO_2Pr$-i | H | H | CN | H |
| $CH_2COMe$ | H | H | CN | H |
| $CH_2COEt$ | H | H | CN | H |
| $CH_2COPr$-n | H | H | CN | H |
| $CH_2COPr$-i | H | H | CN | H |
| $CH_2CO_2H$ | H | H | CN | H |
| $CH_2CO_2Me$ | H | H | CN | H |
| $CH_2CO_2Et$ | H | H | CN | H |
| $CH_2CO_2Pr$-n | H | H | CN | H |
| $CH_2CO_2Pr$-i | H | H | CN | H |
| $CH_2OCH_2CH=CH_2$ | H | H | CN | H |
| $CH_2OCH_2C\equiv CH$ | H | H | CN | H |
| $CH=CHCO_2Me$ | H | H | CN | H |
| $CH=CHCO_2Et$ | H | H | CN | H |

| $R_1$ | $R_2$ | $R_3$ | $R_4$ | $R_5$ |
|---|---|---|---|---|
| $CH=CHCO_2Pr$-n | H | H | CN | H |
| $CH=CHCO_2Pr$-i | H | H | CN | H |
| CH=CHCN | H | H | CN | H |
| Ph | H | H | CN | H |
| $CH_2Ph$ | H | H | CN | H |
| OPh | H | H | CN | H |
| SPh | H | H | CN | H |
| $SO_2Ph$ | H | H | CN | H |
| CONHMe | H | H | CN | H |
| CONHEt | H | H | CN | H |
| CONHPr-n | H | H | CN | H |
| CONHPr-i | H | H | CN | H |
| CONHBu-n | H | H | CN | H |
| CONHBu-sec | H | H | CN | H |
| CONHBu-t | H | H | CN | H |
| $CON(Me)_2$ | H | H | CN | H |
| $CON(Et)_2$ | H | H | CN | H |
| $CON(Pr\text{-}n)_2$ | H | H | CN | H |

| $R_4$ | $R_3$ | $R_2$ | $R_1$ | $R_5$ |
|---|---|---|---|---|
| F | H | H | Me | H |
| Cl | H | H | Me | H |
| Br | H | H | Me | H |
| I | H | H | Me | H |
| $CH=CH_2$ | H | H | Me | H |
| $CH_2CH=CH_2$ | H | H | Me | H |
| $C\equiv CH$ | H | H | Me | H |
| $CH_2C\equiv CH$ | H | H | Me | H |
| $CH_2F$ | H | H | Me | H |
| $CHF_2$ | H | H | Me | H |
| CN | H | H | Me | H |
| $NH_2$ | H | H | Me | H |
| NHCOMe | H | H | Me | H |
| $CH_2NH_2$ | H | H | Me | H |
| $CH_2NHCOMe$ | H | H | Me | H |
| $CH_2OCHF_2$ | H | H | Me | H |
| $CH_2OCF_3$ | H | H | Me | H |
| $CF_3$ | H | H | Me | H |
| $NO_2$ | H | H | Me | H |
| OMe | H | H | Me | H |
| OEt | H | H | Me | H |
| OPr-n | H | H | Me | H |
| SMe | H | H | Me | H |
| SEt | H | H | Me | H |
| SPr-n | H | H | Me | H |
| SBu-n | H | H | Me | H |
| SPen-n | H | H | Me | H |
| $SO_2Me$ | H | H | Me | H |
| $SO_2Et$ | H | H | Me | H |
| $SO_2Pr$-n | H | H | Me | H |
| COMe | H | H | Me | H |
| COEt | H | H | Me | H |
| COPr-n | H | H | Me | H |
| $CO_2H$ | H | H | Me | H |
| $CO_2Me$ | H | H | Me | H |
| $CO_2Et$ | H | H | Me | H |
| $CO_2Pr$-n | H | H | Me | H |
| $CO_2Pr$-i | H | H | Me | H |
| $CO_2Bu$-n | H | H | Me | H |
| $CO_2Bu$-t | H | H | Me | H |

| $R_4$ | $R_3$ | $R_2$ | $R_1$ | $R_5$ |
|---|---|---|---|---|
| $CO_2$Bu-sec | H | H | Me | H |
| $CO_2$Pen-n | H | H | Me | H |
| $CH_2Cl$ | H | H | Me | H |
| $CH_2Br$ | H | H | Me | H |
| $CH_2I$ | H | H | Me | H |
| $CH_2CF_3$ | H | H | Me | H |
| $CH_2CN$ | H | H | Me | H |
| $CH_2OH$ | H | H | Me | H |
| $CH_2OMe$ | H | H | Me | H |
| $CH_2OEt$ | H | H | Me | H |
| $CH_2OPr$-n | H | H | Me | H |
| $CH_2OPr$-i | H | H | Me | H |
| $CH_2SMe$ | H | H | Me | H |
| $CH_2SEt$ | H | H | Me | H |
| $CH_2SPr$-n | H | H | Me | H |
| $CH_2SPr$-i | H | H | Me | H |
| $CH_2SO_2Me$ | H | H | Me | H |
| $CH_2SO_2Et$ | H | H | Me | H |
| $CH_2SO_2Pr$-n | H | H | Me | H |
| $CH_2SO_2Pr$-i | H | H | Me | H |
| $CH_2COMe$ | H | H | Me | H |
| $CH_2COEt$ | H | H | Me | H |
| $CH_2COPr$-n | H | H | Me | H |
| $CH_2COPr$-i | H | H | Me | H |
| $CH_2CO_2H$ | H | H | Me | H |
| $CH_2CO_2Me$ | H | H | Me | H |
| $CH_2CO_2Et$ | H | H | Me | H |
| $CH_2CO_2Pr$-n | H | H | Me | H |
| $CH_2CO_2Pr$-i | H | H | Me | H |
| $CH_2OCH_2CH=CH_2$ | H | H | Me | H |
| $CH_2OCH_2C\equiv CH$ | H | H | Me | H |
| $CH=CHCO_2Me$ | H | H | Me | H |
| $CH=CHCO_2Et$ | H | H | Me | H |
| $CH=CHCO_2Pr$-n | H | H | Me | H |
| $CH=CHCO_2Pr$-i | H | H | Me | H |
| CH=CHCN | H | H | Me | H |
| Ph | H | H | Me | H |
| Ph-2-F | H | H | Me | H |
| Ph-3-F | H | H | Me | H |
| Ph-4-F | H | H | Me | H |

| $R_4$ | $R_3$ | $R_2$ | $R_1$ | $R_5$ |
|---|---|---|---|---|
| Ph-2-Cl | H | H | Me | H |
| Ph-3-Cl | H | H | Me | H |
| Ph-4-Cl | H | H | Me | H |
| Ph-2-Br | H | H | Me | H |
| Ph-3-Br | H | H | Me | H |
| Ph-4-Br | H | H | Me | H |
| Ph-2-$CF_3$ | H | H | Me | H |
| Ph-3-$CF_3$ | H | H | Me | H |
| Ph-4-$CF_3$ | H | H | Me | H |
| Ph-2-Me | H | H | Me | H |
| Ph-3-Me | H | H | Me | H |
| Ph-4-Me | H | H | Me | H |
| Ph-2-Et | H | H | Me | H |
| Ph-2-OMe | H | H | Me | H |
| Ph-3-OMe | H | H | Me | H |
| Ph-4-OMe | H | H | Me | H |
| Ph-2-OEt | H | H | Me | H |
| Ph-2-$CO_2$Me | H | H | Me | H |
| Ph-2-$CO_2$Et | H | H | Me | H |
| Ph-3-$CO_2$Me | H | H | Me | H |
| Ph-4-$CO_2$Me | H | H | Me | H |
| $CH_2$Ph | H | H | Me- | H |
| $CH_2$Ph-2-F | H | H | Me | H |
| $CH_2$Ph-3-F | H | H | Me | H |
| $CH_2$Ph-4-F | H | H | Me | H |
| $CH_2$Ph-2-Cl | H | H | Me | H |
| $CH_2$Ph-3-Cl | H | H | Me | H |
| $CH_2$Ph-4-Cl | H | H | Me | H |
| $CH_2$Ph-2-Br | H | H | Me | H |
| $CH_2$Ph-3-Br | H | H | Me | H |
| $CH_2$Ph-4-Br | H | H | Me | H |
| $CH_2$Ph-2-$CF_3$ | H | H | Me | H |
| $CH_2$Ph-3-$CF_3$ | H | H | Me | H |
| $CH_2$Ph-4-$CF_3$ | H | H | Me | H |
| $CH_2$Ph-2-Me | H | H | Me | H |
| $CH_2$Ph-3-Me | H | H | Me | H |
| $CH_2$Ph-4-Me | H | H | Me | H |
| $CH_2$Ph-2-Et | H | H | Me | H |
| $CH_2$Ph-2-OMe | H | H | Me | H |
| $CH_2$Ph-3-OMe | H | H | Me | H |

| $R_4$ | $R_3$ | $R_2$ | $R_1$ | $R_5$ |
|---|---|---|---|---|
| $CH_2Ph$-4-OMe | H | H | Me | H |
| $CH_2Ph$-2-OEt | H | H | Me | H |
| $CH_2Ph$-2-$CO_2Me$ | H | H | Me | H |
| $CH_2Ph$-2-$CO_2Et$ | H | H | Me | H |
| $CH_2Ph$-3-$CO_2Me$ | H | H | Me | H |
| $CH_2Ph$-4-$CO_2Me$ | H | H | Me | H |
| OPh | H | H | Me | H |
| OPh-2-Cl | H | H | Me | H |
| OPh-3-Cl | H | H | Me | H |
| OPh-4-Cl | H | H | Me | H |
| OPh-2-F | H | H | Me | H |
| OPh-3-F | H | H | Me | H |
| OPh-4-F | H | H | Me | H |
| OPh-2-$CF_3$ | H | H | Me | H |
| OPh-3-$CF_3$ | H | H | Me | H |
| OPh-4-$CF_3$ | H | H | Me | H |
| OPh-2-Me | H | H | Me | H |
| OPh-3-Me | H | H | Me | H |
| OPh-4-Me | H | H | Me | H |
| OPh-2-Et | H | H | Me | H |
| OPh-2-OMe | H | H | Me | H |
| OPh-3-OMe | H | H | Me | H |
| OPh-4-OMe | H | H | Me | H |
| OPh-2-OEt | H | H | Me | H |
| OPh-2-$CO_2Me$ | H | H | Me | H |
| OPh-3-$CO_2Me$ | H | H | Me | H |
| OPh-4-$CO_2Me$ | H | H | Me | H |
| OPh-2-$CO_2Et$ | H | H | Me | H |
| SPh | H | H | Me | H |
| SPh-2-Cl | H | H | Me | H |
| SPh-3-Cl | H | H | Me | H |
| SPh-4-Cl | H | H | Me | H |
| SPh-2-F | H | H | Me | H |
| SPh-3-F | H | H | Me | H |
| SPh-4-F | H | H | Me | H |
| SPh-2-$CF_3$ | H | H | Me | H |
| SPh-3-$CF_3$ | H | H | Me | H |
| SPh-4-$CF_3$ | H | H | Me | H |
| SPh-2-Me | H | H | Me | H |
| SPh-3-Me | H | H | Me | H |

| $R_4$ | $R_3$ | $R_2$ | $R_1$ | $R_5$ |
|---|---|---|---|---|
| SPh-4-Me | H | H | Me | H |
| SPh-2-Et | H | H | Me | H |
| SPh-2-OMe | H | H | Me | H |
| SPh-3-OMe | H | H | Me | H |
| SPh-4-OMe | H | H | Me | H |
| SPh-2-OEt | H | H | Me | H |
| SPh-2-$CO_2$Me | H | H | Me | H |
| SPh-3-$CO_2$Me | H | H | Me | H |
| SPh-4-$CO_2$Me | H | H | Me | H |
| SPh-4-$CO_2$Et | H | H | Me | H |
| $SO_2$Ph | H | H | Me | H |
| $SO_2$Ph-2-Cl | H | H | Me | H |
| $SO_2$Ph-3-Cl | H | H | Me | H |
| $SO_2$Ph-4-Cl | H | H | Me | H |
| $SO_2$Ph-2-F | H | H | Me | H |
| $SO_2$Ph-3-F | H | H | Me | H |
| $SO_2$Ph-4-F | H | H | Me | H |
| $SO_2$Ph-2-$CF_3$ | H | H | Me | H |
| $SO_2$Ph-3-$CF_3$ | H | H | Me | H |
| $SO_2$Ph-4-$CF_3$ | H | H | Me | H |
| $SO_2$Ph-2-Me | H | H | Me | H |
| $SO_2$Ph-3-Me | H | H | Me | H |
| $SO_2$Ph-4-Me | H | H | Me | H |
| $SO_2$Ph-2-Et | H | H | Me | H |
| $SO_2$Ph-2-OMe | H | H | Me | H |
| $SO_2$Ph-3-OMe | H | H | Me | H |
| $SO_2$Ph-4-OMe | H | H | Me | H |
| $SO_2$Ph-2-OEt | H | H | Me | H |
| $SO_2$Ph-2-$CO_2$Me | H | H | Me | H |
| $SO_2$Ph-3-$CO_2$Me | H | H | Me | H |
| $SO_2$Ph-4-$CO_2$Me | H | H | Me | H |
| $SO_2$Ph-2-$CO_2$Et | H | H | Me | H |
| CONHMe | H | H | Me | H |
| CONHEt | H | H | Me | H |
| CONHPr-n | H | H | Me | H |
| CONHPr-i | H | H | Me | H |
| CONHBu-n | H | H | Me | H |
| CONHBu-sec | H | H | Me | H |
| CONHBu-t | H | H | Me | H |
| CON$(Me)_2$ | H | H | Me | H |

| $R_4$ | $R_3$ | $R_2$ | $R_1$ | $R_5$ |
|---|---|---|---|---|
| $CON(Et)_2$ | H | H | Me | H |
| $CON(Pr\text{-}n)_2$ | H | H | Me | H |
| F | H | H | Et | H |
| Cl | H | H | Et | H |
| Br | H | H | Et | H |
| I | H | H | Et | H |
| $CH=CH_2$ | H | H | Et | H |
| $CH_2CH=CH_2$ | H | H | Et | H |
| $C\equiv CH$ | H | H | Et | H |
| $CH_2C\equiv CH$ | H | H | Et | H |
| $CH_2F$ | H | H | Et | H |
| $CHF_2$ | H | H | Et | H |
| CN | H | H | Et | H |
| $NH_2$ | H | H | Et | H |
| NHCOMe | H | H | Et | H |
| $CH_2NH_2$ | H | H | Et | H |
| $CH_2NHCOMe$ | H | H | Et | H |
| $CH_2OCHF_2$ | H | H | Et | H |
| $CH_2OCF_3$ | H | H | Et | H |
| $CF_3$ | H | H | Et | H |
| $NO_2$ | H | H | Et | H |
| OMe | H | H | Et | H |
| OEt | H | H | Et | H |
| OPr-n | H | H | Et | H |
| SMe | H | H | Et | H |
| SEt | H | H | Et | H |
| SPr-n | H | H | Et | H |
| SBu-n | H | H | Et | H |
| SPen-n | H | H | Et | H |
| $SO_2Me$ | H | H | Et | H |
| $SO_2Et$ | H | H | Et | H |
| $SO_2Pr\text{-}n$ | H | H | Et | H |
| COMe | H | H | Et | H |
| COEt | H | H | Et | H |
| COPr-n | H | H | Et | H |
| $CO_2H$ | H | H | Et | H |
| $CO_2Me$ | H | H | Et | H |
| $CO_2Et$ | H | H | Et | H |
| $CO_2Pr\text{-}n$ | H | H | Et | H |
| $CO_2Pr\text{-}i$ | H | H | Et | H |

| $R_4$ | $R_3$ | $R_2$ | $R_1$ | $R_5$ |
|---|---|---|---|---|
| $CO_2Bu$-n | H | H | Et | H |
| $CO_2Bu$-t | H | H | Et | H |
| $CO_2Bu$-sec | H | H | Et | H |
| $CO_2Pen$-n | H | H | Et | H |
| $CH_2Cl$ | H | H | Et | H |
| $CH_2Br$ | H | H | Et | H |
| $CH_2I$ | H | H | Et | H |
| $CH_2CF_3$ | H | H | Et | H |
| $CH_2CN$ | H | H | Et | H |
| $CH_2OH$ | H | H | Et | H |
| $CH_2OMe$ | H | H | Et | H |
| $CH_2OEt$ | H | H | Et | H |
| $CH_2OPr$-n | H | H | Et | H |
| $CH_2OPr$-i | H | H | Et | H |
| $CH_2SMe$ | H | H | Et | H |
| $CH_2SEt$ | H | H | Et | H |
| $CH_2SPr$-n | H | H | Et | H |
| $CH_2SPr$-i | H | H | Et | H |
| $CH_2SO_2Me$ | H | H | Et | H |
| $CH_2SO_2Et$ | H | H | Et | H |
| $CH_2SO_2Pr$-n | H | H | Et | H |
| $CH_2SO_2Pr$-i | H | H | Et | H |
| $CH_2COMe$ | H | H | Et | H |
| $CH_2COEt$ | H | H | Et | H |
| $CH_2COPr$-n | H | H | Et | H |
| $CH_2COPr$-i | H | H | Et | H |
| $CH_2CO_2H$ | H | H | Et | H |
| $CH_2CO_2Me$ | H | H | Et | H |
| $CH_2CO_2Et$ | H | H | Et | H |
| $CH_2CO_2Pr$-n | H | H | Et | H |
| $CH_2CO_2Pr$-i | H | H | Et | H |
| $CH_2OCH_2CH=CH_2$ | H | H | Et | H |
| $CH_2OCH_2C\equiv CH$ | H | H | Et | H |
| $CH=CHCO_2Me$ | H | H | Et | H |
| $CH=CHCO_2Et$ | H | H | Et | H |
| $CH=CHCO_2Pr$-n | H | H | Et | H |
| $CH=CHCO_2Pr$-i | H | H | Et | H |
| CH=CHCN | H | H | Et | H |
| Ph | H | H | Et | H |
| $CH_2Ph$ | H | H | Et | H |

| $R_4$ | $R_3$ | $R_2$ | $R_1$ | $R_5$ |
|---|---|---|---|---|
| OPh | H | H | Et | H |
| SPh | H | H | Et | H |
| $SO_2Ph$ | H | H | Et | H |
| CONHMe | H | H | Et | H |
| CONHEt | H | H | Et | H |
| CONHPr-n | H | H | Et | H |
| CONHPr-i | H | H | Et | H |
| CONHBu-n | H | H | Et | H |
| CONHBu-sec | H | H | Et | H |
| CONHBu-t | H | H | Et | H |
| $CON(Me)_2$ | H | H | Et | H |
| $CON(Et)_2$ | H | H | Et | H |
| $CON(Pr\text{-}n)_2$ | H | H | Et | H |
| F | H | H | Cl | H |
| Cl | H | H | Cl | H |
| Br | H | H | Cl | H |
| I | H | H | Cl | H |
| $CH=CH_2$ | H | H | Cl | H |
| $CH_2CH=CH_2$ | H | H | Cl | H |
| $C\equiv CH$ | H | H | Cl | H |
| $CH_2C\equiv CH$ | H | H | Cl | H |
| $CH_2F$ | H | H | Cl | H |
| $CHF_2$ | H | H | Cl | H |
| CN | H | H | Cl | H |
| $NH_2$ | H | H | Cl | H |
| NHCOMe | H | H | Cl | H |
| $CH_2NH_2$ | H | H | Cl | H |
| $CH_2NHCOMe$ | H | H | Cl | H |
| $CH_2OCHF_2$ | H | H | Cl | H |
| $CH_2OCF_3$ | H | H | Cl | H |
| $CF_3$ | H | H | Cl | H |
| $NO_2$ | H | H | Cl | H |
| OMe | H | H | Cl | H |
| OEt | H | H | Cl | H |
| OPr-n | H | H | Cl | H |
| SMe | H | H | Cl | H |
| SEt | H | H | Cl | H |
| SPr-n | H | H | Cl | H |
| SBu-n | H | H | Cl | H |
| SPen-n | H | H | Cl | H |

| $R_4$ | $R_3$ | $R_2$ | $R_1$ | $R_5$ |
|---|---|---|---|---|
| $SO_2Me$ | H | H | Cl | H |
| $SO_2Et$ | H | H | Cl | H |
| $SO_2Pr$-n | H | H | Cl | H |
| COMe | H | H | Cl | H |
| COEt | H | H | Cl | H |
| COPr-n | H | H | Cl | H |
| $CO_2H$ | H | H | Cl | H |
| $CO_2Me$ | H | H | Cl | H |
| $CO_2Et$ | H | H | Cl | H |
| $CO_2Pr$-n | H | H | Cl | H |
| $CO_2Pr$-i | H | H | Cl | H |
| $CO_2Bu$-n | H | H | Cl | H |
| $CO_2Bu$-t | H | H | Cl | H |
| $CO_2Bu$-sec | H | H | Cl | H |
| $CO_2Pen$-n | H | H | Cl | H |
| $CH_2Cl$ | H | H | Cl | H |
| $CH_2Br$ | H | H | Cl | H |
| $CH_2I$ | H | H | Cl | H |
| $CH_2CF_3$ | H | H | Cl | H |
| $CH_2CN$ | H | H | Cl | H |
| $CH_2OH$ | H | H | Cl | H |
| $CH_2OMe$ | H | H | Cl- | H |
| $CH_2OEt$ | H | H | Cl | H |
| $CH_2OPr$-n | H | H | Cl | H |
| $CH_2OPr$-i | H | H | Cl | H |
| $CH_2SMe$ | H | H | Cl | H |
| $CH_2SEt$ | H | H | Cl | H |
| $CH_2SPr$-n | H | H | Cl | H |
| $CH_2SPr$-i | H | H | Cl | H |
| $CH_2SO_2Me$ | H | H | Cl | H |
| $CH_2SO_2Et$ | H | H | Cl | H |
| $CH_2SO_2Pr$-n | H | H | Cl | H |
| $CH_2SO_2Pr$-i | H | H | Cl | H |
| $CH_2COMe$ | H | H | Cl | H |
| $CH_2COEt$ | H | H | Cl | H |
| $CH_2COPr$-n | H | H | Cl | H |
| $CH_2COPr$-i | H | H | Cl | H |
| $CH_2CO_2H$ | H | H | Cl | H |
| $CH_2CO_2Me$ | H | H | Cl | H |
| $CH_2CO_2Et$ | H | H | Cl | H |

| $R_4$ | $R_3$ | $R_2$ | $R_1$ | $R_5$ |
|---|---|---|---|---|
| $CH_2CO_2Pr$-n | H | H | Cl | H |
| $CH_2CO_2Pr$-i | H | H | Cl | H |
| $CH_2OCH_2CH=CH_2$ | H | H | Cl | H |
| $CH_2OCH_2C\equiv CH$ | H | H | Cl | H |
| $CH=CHCO_2Me$ | H | H | Cl | H |
| $CH=CHCO_2Et$ | H | H | Cl | H |
| $CH=CHCO_2Pr$-n | H | H | Cl | H |
| $CH=CHCO_2Pr$-i | H | H | Cl | H |
| CH=CHCN | H | H | Cl | H |
| Ph | H | H | Cl | H |
| $CH_2Ph$ | H | H | Cl | H |
| OPh | H | H | Cl | H |
| SPh | H | H | Cl | H |
| $SO_2Ph$ | H | H | Cl | H |
| CONHMe | H | H | Cl | H |
| CONHEt | H | H | Cl | H |
| CONHPr-n | H | H | Cl | H |
| CONHPr-i | H | H | Cl | H |
| CONHBu-n | H | H | Cl | H |
| CONHBu-sec | H | H | Cl | H |
| CONHBu-t | H | H | Cl | H |
| $CON(Me)_2$ | H | H | Cl | H |
| $CON(Et)_2$ | H | H | Cl | H |
| $CON(Pr\text{-}n)_2$ | H | H | Cl | H |
| F | H | H | $CO_2Me$ | H |
| Cl | H | H | $CO_2Me$ | H |
| Br | H | H | $CO_2Me$ | H |
| I | H | H | $CO_2Me$ | H |
| $CH=CH_2$ | H | H | $CO_2Me$ | H |
| $CH_2CH=CH_2$ | H | H | $CO_2Me$ | H |
| $C\equiv CH$ | H | H | $CO_2Me$ | H |
| $CH_2C\equiv CH$ | H | H | $CO_2Me$ | H |
| $CH_2F$ | H | H | $CO_2Me$ | H |
| $CHF_2$ | H | H | $CO_2Me$ | H |
| CN | H | H | $CO_2Me$ | H |
| $NH_2$ | H | H | $CO_2Me$ | H |
| NHCOMe | H | H | $CO_2Me$ | H |
| $CH_2NH_2$ | H | H | $CO_2Me$ | H |
| $CH_2NHCOMe$ | H | H | $CO_2Me$ | H |
| $CH_2OCHF_2$ | H | H | $CO_2Me$ | H |

| $R_4$ | $R_3$ | $R_2$ | $R_1$ | $R_5$ |
|---|---|---|---|---|
| $CH_2OCF_3$ | H | H | $CO_2Me$ | H |
| $CF_3$ | H | H | $CO_2Me$ | H |
| $NO_2$ | H | H | $CO_2Me$ | H |
| OMe | H | H | $CO_2Me$ | H |
| OEt | H | H | $CO_2Me$ | H |
| OPr-n | H | H | $CO_2Me$ | H |
| SMe | H | H | $CO_2Me$ | H |
| SEt | H | H | $CO_2Me$ | H |
| SPr-n | H | H | $CO_2Me$ | H |
| SBu-n | H | H | $CO_2Me$ | H |
| SPen-n | H | H | $CO_2Me$ | H |
| $SO_2Me$ | H | H | $CO_2Me$ | H |
| $SO_2Et$ | H | H | $CO_2Me$ | H |
| $SO_2Pr$-n | H | H | $CO_2Me$ | H |
| COMe | H | H | $CO_2Me$ | H |
| COEt | H | H | $CO_2Me$ | H |
| COPr-n | H | H | $CO_2Me$ | H |
| $CO_2H$ | H | H | $CO_2Me$ | H |
| $CO_2Me$ | H | H | $CO_2Me$ | H |
| $CO_2Et$ | H | H | $CO_2Me$ | H |
| $CO_2Pr$-n | H | H | $CO_2Me$ | H |
| $CO_2Pr$-i | H | H | $CO_2Me$ | H |
| $CO_2Bu$-n | H | H | $CO_2Me$ | H |
| $CO_2Bu$-t | H | H | $CO_2Me$ | H |
| $CO_2Bu$-sec | H | H | $CO_2Me$ | H |
| $CO_2Pen$-n | H | H | $CO_2Me$ | H |
| $CH_2Cl$ | H | H | $CO_2Me$ | H |
| $CH_2Br$ | H | H | $CO_2Me$ | H |
| $CH_2I$ | H | H | $CO_2Me$ | H |
| $CH_2CF_3$ | H | H | $CO_2Me$ | H |
| $CH_2CN$ | H | H | $CO_2Me$ | H |
| $CH_2OH$ | H | H | $CO_2Me$ | H |
| $CH_2OMe$ | H | H | $CO_2Me$ | H |
| $CH_2OEt$ | H | H | $CO_2Me$ | H |
| $CH_2OPr$-n | H | H | $CO_2Me$ | H |
| $CH_2OPr$-i | H | H | $CO_2Me$ | H |
| $CH_2SMe$ | H | H | $CO_2Me$ | H |
| $CH_2SEt$ | H | H | $CO_2Me$ | H |
| $CH_2SPr$-n | H | H | $CO_2Me$ | H |
| $CH_2SPr$-i | H | H | $CO_2Me$ | H |

| $R_4$ | $R_3$ | $R_2$ | $R_1$ | $R_5$ |
|---|---|---|---|---|
| $CH_2SO_2Me$ | H | H | $CO_2Me$ | H |
| $CH_2SO_2Et$ | H | H | $CO_2Me$ | H |
| $CH_2SO_2Pr$-n | H | H | $CO_2Me$ | H |
| $CH_2SO_2Pr$-i | H | H | $CO_2Me$ | H |
| $CH_2COMe$ | H | H | $CO_2Me$ | H |
| $CH_2COEt$ | H | H | $CO_2Me$ | H |
| $CH_2COPr$-n | H | H | $CO_2Me$ | H |
| $CH_2COPr$-i | H | H | $CO_2Me$ | H |
| $CH_2CO_2H$ | H | H | $CO_2Me$ | H |
| $CH_2CO_2Me$ | H | H | $CO_2Me$ | H |
| $CH_2CO_2Et$ | H | H | $CO_2Me$ | H |
| $CH_2CO_2Pr$-n | H | H | $CO_2Me$ | H |
| $CH_2CO_2Pr$-i | H | H | $CO_2Me$ | H |
| $CH_2OCH_2CH=CH_2$ | H | H | $CO_2Me$ | H |
| $CH_2OCH_2C\equiv CH$ | H | H | $CO_2Me$ | H |
| $CH=CHCO_2Me$ | H | H | $CO_2Me$ | H |
| $CH=CHCO_2Et$ | H | H | $CO_2Me$ | H |
| $CH=CHCO_2Pr$-n | H | H | $CO_2Me$ | H |
| $CH=CHCO_2Pr$-i | H | H | $CO_2Me$ | H |
| CH=CHCN | H | H | $CO_2Me$ | H |
| Ph | H | H | $CO_2Me$ | H |
| $CH_2Ph$ | H | H | $CO_2Me$ | H |
| OPh | H | H | $CO_2Me$ | H |
| SPh | H | H | $CO_2Me$ | H |
| $SO_2Ph$ | H | H | $CO_2Me$ | H |
| CONHMe | H | H | $CO_2Me$ | H |
| CONHEt | H | H | $CO_2Me$ | H |
| CONHPr-n | H | H | $CO_2Me$ | H |
| CONHPr-i | H | H | $CO_2Me$ | H |
| CONHBu-n | H | H | $CO_2Me$ | H |
| CONHBu-sec | H | H | $CO_2Me$ | H |
| CONHBu-t | H | H | $CO_2Me$ | H |
| $CON(Me)_2$ | H | H | $CO_2Me$ | H |
| $CON(Et)_2$ | H | H | $CO_2Me$ | H |
| $CON(Pr\text{-}n)_2$ | H | H | $CO_2Me$ | H |
| F | H | H | $CH_2Cl$ | H |
| Cl | H | H | $CH_2Cl$ | H |
| Br | H | H | $CH_2Cl$ | H |
| I | H | H | $CH_2Cl$ | H |
| $CH=CH_2$ | H | H | $CH_2Cl$ | H |

| $R_4$ | $R_3$ | $R_2$ | $R_1$ | $R_5$ |
|---|---|---|---|---|
| $CH_2CH=CH_2$ | H | H | $CH_2Cl$ | H |
| $C\equiv CH$ | H | H | $CH_2Cl$ | H |
| $CH_2C\equiv CH$ | H | H | $CH_2Cl$ | H |
| $CH_2F$ | H | H | $CH_2Cl$ | H |
| $CHF_2$ | H | H | $CH_2Cl$ | H |
| CN | H | H | $CH_2Cl$ | H |
| $NH_2$ | H | H | $CH_2Cl$ | H |
| NHCOMe | H | H | $CH_2Cl$ | H |
| $CH_2NH_2$ | H | H | $CH_2Cl$ | H |
| $CH_2NHCOMe$ | H | H | $CH_2Cl$ | H |
| $CH_2OCHF_2$ | H | H | $CH_2Cl$ | H |
| $CH_2OCF_3$ | H | H | $CH_2Cl$ | H |
| $CF_3$ | H | H | $CH_2Cl$ | H |
| $NO_2$ | H | H | $CH_2Cl$ | H |
| OMe | H | H | $CH_2Cl$ | H |
| OEt | H | H | $CH_2Cl$ | H |
| OPr-n | H | H | $CH_2Cl$ | H |
| SMe | H | H | $CH_2Cl$ | H |
| SEt | H | H | $CH_2Cl$ | H |
| SPr-n | H | H | $CH_2Cl$ | H |
| SBu-n | H | H | $CH_2Cl$ | H |
| SPen-n | H | H | $CH_2Cl$ | H |
| $SO_2Me$ | H | H | $CH_2Cl$ | H |
| $SO_2Et$ | H | H | $CH_2Cl$ | H |
| $SO_2Pr$-n | H | H | $CH_2Cl$ | H |
| COMe | H | H | $CH_2Cl$ | H |
| COEt | H | H | $CH_2Cl$ | H |
| COPr-n | H | H | $CH_2Cl$ | H |
| $CO_2H$ | H | H | $CH_2Cl$ | H |
| $CO_2Me$ | H | H | $CH_2Cl$ | H |
| $CO_2Et$ | H | H | $CH_2Cl$ | H |
| $CO_2Pr$-n | H | H | $CH_2Cl$ | H |
| $CO_2Pr$-i | H | H | $CH_2Cl$ | H |
| $CO_2Bu$-n | H | H | $CH_2Cl$ | H |
| $CO_2Bu$-t | H | H | $CH_2Cl$ | H |
| $CO_2Bu$-sec | H | H | $CH_2Cl$ | H |
| $CO_2Pen$-n | H | H | $CH_2Cl$ | H |
| $CH_2Cl$ | H | H | $CH_2Cl$ | H |
| $CH_2Br$ | H | H | $CH_2Cl$ | H |
| $CH_2I$ | H | H | $CH_2Cl$ | H |

| $R_4$ | $R_3$ | $R_2$ | $R_1$ | $R_5$ |
|---|---|---|---|---|
| $CH_2CF_3$ | H | H | $CH_2Cl$ | H |
| $CH_2CN$ | H | H | $CH_2Cl$ | H |
| $CH_2OH$ | H | H | $CH_2Cl$ | H |
| $CH_2OMe$ | H | H | $CH_2Cl$ | H |
| $CH_2OEt$ | H | H | $CH_2Cl$ | H |
| $CH_2OPr$-n | H | H | $CH_2Cl$ | H |
| $CH_2OPr$-i | H | H | $CH_2Cl$ | H |
| $CH_2SMe$ | H | H | $CH_2Cl$ | H |
| $CH_2SEt$ | H | H | $CH_2Cl$ | H |
| $CH_2SPr$-n | H | H | $CH_2Cl$ | H |
| $CH_2SPr$-i | H | H | $CH_2Cl$ | H |
| $CH_2SO_2Me$ | H | H | $CH_2Cl$ | H |
| $CH_2SO_2Et$ | H | H | $CH_2Cl$ | H |
| $CH_2SO_2Pr$-n | H | H | $CH_2Cl$ | H |
| $CH_2SO_2Pr$-i | H | H | $CH_2Cl$ | H |
| $CH_2COMe$ | H | H | $CH_2Cl$ | H |
| $CH_2COEt$ | H | H | $CH_2Cl$ | H |
| $CH_2COPr$-n | H | H | $CH_2Cl$ | H |
| $CH_2COPr$-i | H | H | $CH_2Cl$ | H |
| $CH_2CO_2H$ | H | H | $CH_2Cl$ | H |
| $CH_2CO_2Me$ | H | H | $CH_2Cl$ | H |
| $CH_2CO_2Et$ | H | H | $CH_2Cl$ | H |
| $CH_2CO_2Pr$-n | H | H | $CH_2Cl$ | H |
| $CH_2CO_2Pr$-i | H | H | $CH_2Cl$ | H |
| $CH_2OCH_2CH=CH_2$ | H | H | $CH_2Cl$ | H |
| $CH_2OCH_2C\equiv CH$ | H | H | $CH_2Cl$ | H |
| $CH=CHCO_2Me$ | H | H | $CH_2Cl$ | H |
| $CH=CHCO_2Et$ | H | H | $CH_2Cl$ | H |
| $CH=CHCO_2Pr$-n | H | H | $CH_2Cl$ | H |
| $CH=CHCO_2Pr$-i | H | H | $CH_2Cl$ | H |
| CH=CHCN | H | H | $CH_2Cl$ | H |
| Ph | H | H | $CH_2Cl$ | H |
| $CH_2Ph$ | H | H | $CH_2Cl$ | H |
| OPh | H | H | $CH_2Cl$ | H |
| SPh | H | H | $CH_2Cl$ | H |
| $SO_2Ph$ | H | H | $CH_2Cl$ | H |
| CONHMe | H | H | $CH_2Cl$ | H |
| CONHEt | H | H | $CH_2Cl$ | H |
| CONHPr-n | H | H | $CH_2Cl$ | H |
| CONHPr-i | H | H | $CH_2Cl$ | H |

| $R_4$ | $R_3$ | $R_2$ | $R_1$ | $R_5$ |
|---|---|---|---|---|
| CONHBu-n | H | H | $CH_2Cl$ | H |
| CONHBu-sec | H | H | $CH_2Cl$ | H |
| CONHBu-t | H | H | $CH_2Cl$ | H |
| $CON(Me)_2$ | H | H | $CH_2Cl$ | H |
| $CON(Et)_2$ | H | H | $CH_2Cl$ | H |
| $CON(Pr\text{-}n)_2$ | H | H | $CH_2Cl$ | H |
| F | H | H | $CH_2OMe$ | H |
| Cl | H | H | $CH_2OMe$ | H |
| Br | H | H | $CH_2OMe$ | H |
| I | H | H | $CH_2OMe$ | H |
| $CH=CH_2$ | H | H | $CH_2OMe$ | H |
| $CH_2CH=CH_2$ | H | H | $CH_2OMe$ | H |
| $C\equiv CH$ | H | H | $CH_2OMe$ | H |
| $CH_2C\equiv CH$ | H | H | $CH_2OMe$ | H |
| $CH_2F$ | H | H | $CH_2OMe$ | H |
| $CHF_2$ | H | H | $CH_2OMe$ | H |
| CN | H | H | $CH_2OMe$ | H |
| $NH_2$ | H | H | $CH_2OMe$ | H |
| NHCOMe | H | H | $CH_2OMe$ | H |
| $CH_2NH_2$ | H | H | $CH_2OMe$ | H |
| $CH_2NHCOMe$ | H | H | $CH_2OMe$ | H |
| $CH_2OCHF_2$ | H | H | $CH_2OMe$ | H |
| $CH_2OCF_3$ | H | H | $CH_2OMe$ | H |
| $CF_3$ | H | H | $CH_2OMe$ | H |
| $NO_2$ | H | H | $CH_2OMe$ | H |
| OMe | H | H | $CH_2OMe$ | H |
| OEt | H | H | $CH_2OMe$ | H |
| OPr-n | H | H | $CH_2OMe$ | H |
| SMe | H | H | $CH_2OMe$ | H |
| SEt | H | H | $CH_2OMe$ | H |
| SPr-n | H | H | $CH_2OMe$ | H |
| SBu-n | H | H | $CH_2OMe$ | H |
| SPen-n | H | H | $CH_2OMe$ | H |
| $SO_2Me$ | H | H | $CH_2OMe$ | H |
| $SO_2Et$ | H | H | $CH_2OMe$ | H |
| $SO_2Pr\text{-}n$ | H | H | $CH_2OMe$ | H |
| COMe | H | H | $CH_2OMe$ | H |
| COEt | H | H | $CH_2OMe$ | H |
| COPr-n | H | H | $CH_2OMe$ | H |
| $CO_2H$ | H | H | $CH_2OMe$ | H |

| $R_4$ | $R_3$ | $R_2$ | $R_1$ | $R_5$ |
|---|---|---|---|---|
| $CO_2Me$ | H | H | $CH_2OMe$ | H |
| $CO_2Et$ | H | H | $CH_2OMe$ | H |
| $CO_2Pr$-n | H | H | $CH_2OMe$ | H |
| $CO_2Pr$-i | H | H | $CH_2OMe$ | H |
| $CO_2Bu$-n | H | H | $CH_2OMe$ | H |
| $CO_2Bu$-t | H | H | $CH_2OMe$ | H |
| $CO_2Bu$-sec | H | H | $CH_2OMe$ | H |
| $CO_2Pen$-n | H | H | $CH_2OMe$ | H |
| $CH_2Cl$ | H | H | $CH_2OMe$ | H |
| $CH_2Br$ | H | H | $CH_2OMe$ | H |
| $CH_2I$ | H | H | $CH_2OMe$ | H |
| $CH_2CF_3$ | H | H | $CH_2OMe$ | H |
| $CH_2CN$ | H | H | $CH_2OMe$ | H |
| $CH_2OH$ | H | H | $CH_2OMe$ | H |
| $CH_2OMe$ | H | H | $CH_2OMe$ | H |
| $CH_2OEt$ | H | H | $CH_2OMe$ | H |
| $CH_2OPr$-n | H | H | $CH_2OMe$ | H |
| $CH_2OPr$-i | H | H | $CH_2OMe$ | H |
| $CH_2SMe$ | H | H | $CH_2OMe$ | H |
| $CH_2SEt$ | H | H | $CH_2OMe$ | H |
| $CH_2SPr$-n | H | H | $CH_2OMe$ | H |
| $CH_2SPr$-i | H | H | $CH_2OMe$ | H |
| $CH_2SO_2Me$ | H | H | $CH_2OMe$ | H |
| $CH_2SO_2Et$ | H | H | $CH_2OMe$ | H |
| $CH_2SO_2Pr$-n | H | H | $CH_2OMe$ | H |
| $CH_2SO_2Pr$-i | H | H | $CH_2OMe$ | H |
| $CH_2COMe$ | H | H | $CH_2OMe$ | H |
| $CH_2COEt$ | H | H | $CH_2OMe$ | H |
| $CH_2COPr$-n | H | H | $CH_2OMe$ | H |
| $CH_2COPr$-i | H | H | $CH_2OMe$ | H |
| $CH_2CO_2H$ | H | H | $CH_2OMe$ | H |
| $CH_2CO_2Me$ | H | H | $CH_2OMe$ | H |
| $CH_2CO_2Et$ | H | H | $CH_2OMe$ | H |
| $CH_2CO_2Pr$-n | H | H | $CH_2OMe$ | H |
| $CH_2CO_2Pr$-i | H | H | $CH_2OMe$ | H |
| $CH_2OCH_2CH{=}CH_2$ | H | H | $CH_2OMe$ | H |
| $CH_2OCH_2C{\equiv}CH$ | H | H | $CH_2OMe$ | H |
| $CH{=}CHCO_2Me$ | H | H | $CH_2OMe$ | H |
| $CH{=}CHCO_2Et$ | H | H | $CH_2OMe$ | H |
| $CH{=}CHCO_2Pr$-n | H | H | $CH_2OMe$ | H |

| $R_4$ | $R_3$ | $R_2$ | $R_1$ | $R_5$ |
|---|---|---|---|---|
| $CH=CHCO_2Pr$-i | H | H | $CH_2OMe$ | H |
| CH=CHCN | H | H | $CH_2OMe$ | H |
| Ph | H | H | $CH_2OMe$ | H |
| $CH_2Ph$ | H | H | $CH_2OMe$ | H |
| OPh | H | H | $CH_2OMe$ | H |
| SPh | H | H | $CH_2OMe$ | H |
| $SO_2Ph$ | H | H | $CH_2OMe$ | H |
| CONHMe | H | H | $CH_2OMe$ | H |
| CONHEt | H | H | $CH_2OMe$ | H |
| CONHPr-n | H | H | $CH_2OMe$ | H |
| CONHPr-i | H | H | $CH_2OMe$ | H |
| CONHBu-n | H | H | $CH_2OMe$ | H |
| CONHBu-sec | H | H | $CH_2OMe$ | H |
| CONHBu-t | H | H | $CH_2OMe$ | H |
| $CON(Me)_2$ | H | H | $CH_2OMe$ | H |
| $CON(Et)_2$ | H | H | $CH_2OMe$ | H |
| $CON(Pr\text{-}n)_2$ | H | H | $CH_2OMe$ | H |
| F | H | H | $CH_2SMe$ | H |
| Cl | H | H | $CH_2SMe$ | H |
| Br | H | H | $CH_2SMe$ | H |
| I | H | H | $CH_2SMe$ | H |
| $CH=CH_2$ | H | H | $CH_2SMe$ | H |
| $CH_2CH=CH_2$ | H | H | $CH_2SMe$ | H |
| $C\equiv CH$ | H | H | $CH_2SMe$ | H |
| $CH_2C\equiv CH$ | H | H | $CH_2SMe$ | H |
| $CH_2F$ | H | H | $CH_2SMe$ | H |
| $CHF_2$ | H | H | $CH_2SMe$ | H |
| CN | H | H | $CH_2SMe$ | H |
| $NH_2$ | H | H | $CH_2SMe$ | H |
| NHCOMe | H | H | $CH_2SMe$ | H |
| $CH_2NH_2$ | H | H | $CH_2SMe$ | H |
| $CH_2NHCOMe$ | H | H | $CH_2SMe$ | H |
| $CH_2OCHF_2$ | H | H | $CH_2SMe$ | H |
| $CH_2OCF_3$ | H | H | $CH_2SMe$ | H |
| $CF_3$ | H | H | $CH_2SMe$ | H |
| $NO_2$ | H | H | $CH_2SMe$ | H |
| OMe | H | H | $CH_2SMe$ | H |
| OEt | H | H | $CH_2SMe$ | H |
| OPr-n | H | H | $CH_2SMe$ | H |
| SMe | H | H | $CH_2SMe$ | H |

| $R_4$ | $R_3$ | $R_2$ | $R_1$ | $R_5$ |
|---|---|---|---|---|
| SEt | H | H | $CH_2SMe$ | H |
| SPr-n | H | H | $CH_2SMe$ | H |
| SBu-n | H | H | $CH_2SMe$ | H |
| SPen-n | H | H | $CH_2SMe$ | H |
| $SO_2Me$ | H | H | $CH_2SMe$ | H |
| $SO_2Et$ | H | H | $CH_2SMe$ | H |
| $SO_2Pr$-n | H | H | $CH_2SMe$ | H |
| COMe | H | H | $CH_2SMe$ | H |
| COEt | H | H | $CH_2SMe$ | H |
| COPr-n | H | H | $CH_2SMe$ | H |
| $CO_2H$ | H | H | $CH_2SMe$ | H |
| $CO_2Me$ | H | H | $CH_2SMe$ | H |
| $CO_2Et$ | H | H | $CH_2SMe$ | H |
| $CO_2Pr$-n | H | H | $CH_2SMe$ | H |
| $CO_2Pr$-i | H | H | $CH_2SMe$ | H |
| $CO_2Bu$-n | H | H | $CH_2SMe$ | H |
| $CO_2Bu$-t | H | H | $CH_2SMe$ | H |
| $CO_2Bu$-sec | H | H | $CH_2SMe$ | H |
| $CO_2Pen$-n | H | H | $CH_2SMe$ | H |
| $CH_2Cl$ | H | H | $CH_2SMe$ | H |
| $CH_2Br$ | H | H | $CH_2SMe$ | H |
| $CH_2I$ | H | H | $CH_2SMe$ | H |
| $CH_2CF_3$ | H | H | $CH_2SMe$ | H |
| $CH_2CN$ | H | H | $CH_2SMe$ | H |
| $CH_2OH$ | H | H | $CH_2SMe$ | H |
| $CH_2OMe$ | H | H | $CH_2SMe$ | H |
| $CH_2OEt$ | H | H | $CH_2SMe$ | H |
| $CH_2OPr$-n | H | H | $CH_2SMe$ | H |
| $CH_2OPr$-i | H | H | $CH_2SMe$ | H |
| $CH_2SMe$ | H | H | $CH_2SMe$ | H |
| $CH_2SEt$ | H | H | $CH_2SMe$ | H |
| $CH_2SPr$-n | H | H | $CH_2SMe$ | H |
| $CH_2SPr$-i | H | H | $CH_2SMe$ | H |
| $CH_2SO_2Me$ | H | H | $CH_2SMe$ | H |
| $CH_2SO_2Et$ | H | H | $CH_2SMe$ | H |
| $CH_2SO_2Pr$-n | H | H | $CH_2SMe$ | H |
| $CH_2SO_2Pr$-i | H | H | $CH_2SMe$ | H |
| $CH_2COMe$ | H | H | $CH_2SMe$ | H |
| $CH_2COEt$ | H | H | $CH_2SMe$ | H |
| $CH_2COPr$-n | H | H | $CH_2SMe$ | H |

| $R_4$ | $R_3$ | $R_2$ | $R_1$ | $R_5$ |
|---|---|---|---|---|
| $CH_2COPr$-i | H | H | $CH_2SMe$ | H |
| $CH_2CO_2H$ | H | H | $CH_2SMe$ | H |
| $CH_2CO_2Me$ | H | H | $CH_2SMe$ | H |
| $CH_2CO_2Et$ | H | H | $CH_2SMe$ | H |
| $CH_2CO_2Pr$-n | H | H | $CH_2SMe$ | H |
| $CH_2CO_2Pr$-i | H | H | $CH_2SMe$ | H |
| $CH_2OCH_2CH=CH_2$ | H | H | $CH_2SMe$ | H |
| $CH_2OCH_2C\equiv CH$ | H | H | $CH_2SMe$ | H |
| $CH=CHCO_2Me$ | H | H | $CH_2SMe$ | H |
| $CH=CHCO_2Et$ | H | H | $CH_2SMe$ | H |
| $CH=CHCO_2Pr$-n | H | H | $CH_2SMe$ | H |
| $CH=CHCO_2Pr$-i | H | H | $CH_2SMe$ | H |
| CH=CHCN | H | H | $CH_2SMe$ | H |
| Ph | H | H | $CH_2SMe$ | H |
| $CH_2Ph$ | H | H | $CH_2SMe$ | H |
| OPh | H | H | $CH_2SMe$ | H |
| SPh | H | H | $CH_2SMe$ | H |
| $SO_2Ph$ | H | H | $CH_2SMe$ | H |
| CONHMe | H | H | $CH_2SMe$ | H |
| CONHEt | H | H | $CH_2SMe$ | H |
| CONHPr-n | H | H | $CH_2SMe$ | H |
| CONHPr-i | H | H | $CH_2SMe$ | H |
| CONHBu-n | H | H | $CH_2SMe$ | H |
| CONHBu-sec | H | H | $CH_2SMe$ | H |
| CONHBu-t | H | H | $CH_2SMe$ | H |
| $CON(Me)_2$ | H | H | $CH_2SMe$ | H |
| $CON(Et)_2$ | H | H | $CH_2SMe$ | H |
| $CON(Pr\text{-}n)_2$ | H | H | $CH_2SMe$ | H |
| F | H | H | CN | H |
| Cl | H | H | CN | H |
| Br | H | H | CN | H |
| I | H | H | CN | H |
| $CH=CH_2$ | H | H | CN | H |
| $CH_2CH=CH_2$ | H | H | CN | H |
| $C\equiv CH$ | H | H | CN | H |
| $CH_2C\equiv CH$ | H | H | CN | H |
| $CH_2F$ | H | H | CN | H |
| $CHF_2$ | H | H | CN | H |
| CN | H | H | CN | H |
| $NH_2$ | H | H | CN | H |

| $R_4$ | $R_3$ | $R_2$ | $R_1$ | $R_5$ |
|---|---|---|---|---|
| NHCOMe | H | H | CN | H |
| $CH_2NH_2$ | H | H | CN | H |
| $CH_2NHCOMe$ | H | H | CN | H |
| $CH_2OCHF_2$ | H | H | CN | H |
| $CH_2OCF_3$ | H | H | CN | H |
| $CF_3$ | H | H | CN | H |
| $NO_2$ | H | H | CN | H |
| OMe | H | H | CN | H |
| OEt | H | H | CN | H |
| OPr-n | H | H | CN | H |
| SMe | H | H | CN | H |
| SEt | H | H | CN | H |
| SPr-n | H | H | CN | H |
| SBu-n | H | H | CN | H |
| SPen-n | H | H | CN | H |
| $SO_2Me$ | H | H | CN | H |
| $SO_2Et$ | H | H | CN | H |
| $SO_2Pr$-n | H | H | CN | H |
| COMe | H | H | CN | H |
| COEt | H | H | CN | H |
| COPr-n | H | H | CN | H |
| $CO_2H$ | H | H | CN | H |
| $CO_2Me$ | H | H | CN | H |
| $CO_2Et$ | H | H | CN | H |
| $CO_2Pr$-n | H | H | CN | H |
| $CO_2Pr$-i | H | H | CN | H |
| $CO_2Bu$-n | H | H | CN | H |
| $CO_2Bu$-t | H | H | CN | H |
| $CO_2Bu$-sec | H | H | CN | H |
| $CO_2Pen$-n | H | H | CN | H |
| $CH_2Cl$ | H | H | CN | H |
| $CH_2Br$ | H | H | CN | H |
| $CH_2I$ | H | H | CN | H |
| $CH_2CF_3$ | H | H | CN | H |
| $CH_2CN$ | H | H | CN | H |
| $CH_2OH$ | H | H | CN | H |
| $CH_2OMe$ | H | H | CN | H |
| $CH_2OEt$ | H | H | CN | H |
| $CH_2OPr$-n | H | H | CN | H |
| $CH_2OPr$-i | H | H | CN | H |

| $R_4$ | $R_3$ | $R_2$ | $R_1$ | $R_5$ |
|---|---|---|---|---|
| $CH_2SMe$ | H | H | CN | H |
| $CH_2SEt$ | H | H | CN | H |
| $CH_2SPr$-n | H | H | CN | H |
| $CH_2SPr$-i | H | H | CN | H |
| $CH_2SO_2Me$ | H | H | CN | H |
| $CH_2SO_2Et$ | H | H | CN | H |
| $CH_2SO_2Pr$-n | H | H | CN | H |
| $CH_2SO_2Pr$-i | H | H | CN | H |
| $CH_2COMe$ | H | H | CN | H |
| $CH_2COEt$ | H | H | CN | H |
| $CH_2COPr$-n | H | H | CN | H |
| $CH_2COPr$-i | H | H | CN | H |
| $CH_2CO_2H$ | H | H | CN | H |
| $CH_2CO_2Me$ | H | H | CN | H |
| $CH_2CO_2Et$ | H | H | CN | H |
| $CH_2CO_2Pr$-n | H | H | CN | H |
| $CH_2CO_2Pr$-i | H | H | CN | H |
| $CH_2OCH_2CH=CH_2$ | H | H | CN | H |
| $CH_2OCH_2C\equiv CH$ | H | H | CN | H |
| $CH=CHCO_2Me$ | H | H | CN | H |
| $CH=CHCO_2Et$ | H | H | CN | H |
| $CH=CHCO_2Pr$-n | H | H | CN | H |
| $CH=CHCO_2Pr$-i | H | H | CN | H |
| CH=CHCN | H | H | CN | H |
| Ph | H | H | CN | H |
| $CH_2Ph$ | H | H | CN | H |
| OPh | H | H | CN | H |
| SPh | H | H | CN | H |
| $SO_2Ph$ | H | H | CN | H |
| CONHMe | H | H | CN | H |
| CONHEt | H | H | CN | H |
| CONHPr-n | H | H | CN | H |
| CONHPr-i | H | H | CN | H |
| CONHBu-n | H | H | CN | H |
| CONHBu-sec | H | H | CN | H |
| CONHBu-t | H | H | CN | H |
| $CON(Me)_2$ | H | H | CN | H |
| $CON(Et)_2$ | H | H | CN | H |
| $CON(Pr\text{-}n)_2$ | H | H | CN | H |
| H | H | Cl | Cl | H |

| $R_1$ | $R_2$ | $R_3$ | $R_4$ | $R_5$ |
|---|---|---|---|---|
| Cl | H | Cl | H | H |
| Cl | H | H | Cl | H |
| H | Cl | Cl | H | H |
| H | Cl | H | Cl | H |
| H | H | Cl | Cl | H |
| Me | Cl | H | H | H |
| Me | H | Cl | H | H |
| H | Cl | H | Me | H |
| H | H | Cl | Me | H |
| Et | Cl | H | H | H |
| Et | H | Cl | H | H |
| H | Cl | H | Et | H |
| H | H | Cl | Et | H |
| Br | Br | H | H | H |
| Br | H | Br | H | H |
| Br | H | H | Br | H |
| H | Br | Br | H | H |
| H | Br | H | Br | H |
| H | H | Br | Br | H |

Table 4

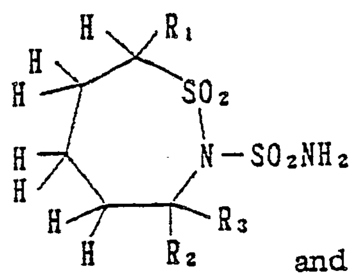

and

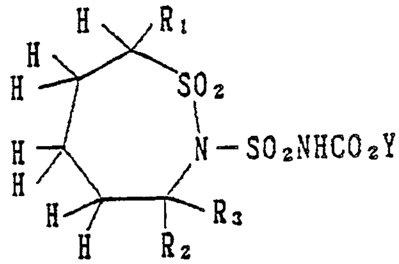

(Y=Me, Et, Pr-n, Bu-n or Ph)

| $R_1$ | $R_2$ | $R_3$ |
|---|---|---|
| H | H | H |
| Me | H | H |
| H | Me | H |
| Et | H | H |
| H | Et | H |
| Pr-n | H | H |
| H | Pr-n | H |
| Pr-i | H | H |
| H | Pr-i | H |
| Bu-n | H | H |
| H | Bu-n | H |
| Me | Me | H |
| Me | Et | H |
| Me | Pr-n | H |
| Me | Pr-i | H |
| Et | Me | H |
| Et | Et | H |
| Et | Pr-n | H |
| Et | Pr-i | H |
| Pr-n | Me | H |
| Pr-n | Et | H |
| Pr-n | Pr-n | H |
| Pr-n | Pr-i | H |
| Pr-i | Me | H |
| Pr-i | Et | H |
| Pr-i | Pr-n | H |
| Pr-i | Pr-i | H |
| H | Me | Me |
| H | Me | Et |
| H | Me | Pr-n |

| $R_1$ | $R_2$ | $R_3$ |
|---|---|---|
| H | Me | Pr-i |
| H | Et | Et |
| H | Et | Pr-n |
| Me | Me | Me |
| Et | Me | Me |
| Pr-n | Me | Me |
| Pr-i | Me | Me |
| H | Cl | H |
| H | Br | H |
| H | I | H |
| H | $CF_3$ | H |
| H | $NO_2$ | H |
| H | OMe | H |
| H | SMe | H |
| H | $SO_2Me$ | H |
| H | COMe | H |
| H | $CO_2H$ | H |
| H | $CO_2Me$ | H |
| H | $CO_2Et$ | H |
| H | $CH_2Cl$ | H |
| H | $CH_2Br$ | H |
| H | $CH_2I$ | H |
| H | $CH_2CF_3$ | H |
| H | $CH_2CN$ | H |
| H | $CH_2OH$ | H |
| H | $CH_2OMe$ | H |
| H | $CH_2OEt$ | H |
| H | $CH_2SMe$ | H |
| H | $CH_2SEt$ | H |
| H | $CH_2SO_2Me$ | H |
| H | $CH_2SO_2Et$ | H |
| H | $CH_2COMe$ | H |
| H | $CH_2COEt$ | H |
| H | $CH_2CO_2H$ | H |
| H | $CH_2CO_2Me$ | H |
| H | $CH_2CO_2Et$ | H |
| H | $CH_2OCH_2CH=CH_2$ | H |
| H | $CH_2OCH_2C\equiv CH$ | H |
| H | $CH=CHCO_2Me$ | H |
| H | $CH=CHCO_2Et$ | H |

| $R_1$ | $R_2$ | $R_3$ |
|---|---|---|
| H | CH=CHCN | H |
| H | Ph | H |
| H | Ph-2-Cl | H |
| H | Ph-3-Cl | H |
| H | Ph-4-Cl | H |
| H | $CH_2Ph$ | H |
| H | $CH_2Ph$-2-Cl | H |
| H | $CH_2Ph$-3-Cl | H |
| H | $CH_2Ph$-4-Cl | H |
| Me | Cl | H |
| Me | Br | H |
| Me | I | H |
| Me | $CF_3$ | H |
| Me | $NO_2$ | H |
| Me | OMe | H |
| Me | SMe | H |
| Me | $SO_2Me$ | H |
| Me | COMe | H |
| Me | $CO_2H$ | H |
| Me | $CO_2Me$ | H |
| Me | $CO_2Et$ | H |
| Me | $CH_2Cl$ | H |
| Me | $CH_2Br$ | H |
| Me | $CH_2I$ | H |
| Me | $CH_2CF_3$ | H |
| Me | $CH_2CN$ | H |
| Me | $CH_2OH$ | H |
| Me | $CH_2OMe$ | H |
| Me | $CH_2OEt$ | H |
| Me | $CH_2SMe$ | H |
| Me | $CH_2SEt$ | H |
| Me | $CH_2SO_2Me$ | H |
| Me | $CH_2SO_2Et$ | H |
| Me | $CH_2COMe$ | H |
| Me | $CH_2COEt$ | H |
| Me | $CH_2CO_2H$ | H |
| Me | $CH_2CO_2Me$ | H |
| Me | $CH_2CO_2Et$ | H |
| Me | $CH_2OCH_2CH=CH_2$ | H |
| Me | $CH_2OCH_2C\equiv CH$ | H |

| $R_1$ | $R_2$ | $R_3$ |
|---|---|---|
| Me | $CH=CHCO_2Me$ | H |
| Me | $CH=CHCO_2Et$ | H |
| Me | CH=CHCN | H |
| Me | Ph | H |
| Me | Ph-2-Cl | H |
| Me | Ph-3-Cl | H |
| Me | Ph-4-Cl | H |
| Me | $CH_2Ph$ | H |
| Me | $CH_2Ph$-2-Cl | H |
| Me | $CH_2Ph$-3-Cl | H |
| Me | $CH_2Ph$-4-Cl | H |
| Et | Cl | H |
| Et | Br | H |
| Et | I | H |
| Et | $CF_3$ | H |
| Et | $NO_2$ | H |
| Et | OMe | H |
| Et | SMe | H |
| Et | $SO_2Me$ | H |
| Et | COMe | H |
| Et | $CO_2H$ | H |
| Et | $CO_2Me$ | H |
| Et | $CO_2Et$ | H |
| Et | $CH_2Cl$ | H |
| Et | $CH_2Br$ | H |
| Et | $CH_2I$ | H |
| Et | $CH_2CF_3$ | H |
| Et | $CH_2CN$ | H |
| Et | $CH_2OH$ | H |
| Et | $CH_2OMe$ | H |
| Et | $CH_2OEt$ | H |
| Et | $CH_2SMe$ | H |
| Et | $CH_2SEt$ | H |
| Et | $CH_2SO_2Me$ | H |
| Et | $CH_2SO_2Et$ | H |
| Et | $CH_2COMe$ | H |
| Et | $CH_2COEt$ | H |
| Et | $CH_2CO_2H$ | H |
| Et | $CH_2CO_2Me$ | H |
| Et | $CH_2CO_2Et$ | H |

| $R_1$ | $R_2$ | $R_3$ |
|---|---|---|
| Et | $CH_2OCH_2CH=CH_2$ | H |
| Et | $CH_2OCH_2C\equiv CH$ | H |
| Et | $CH=CHCO_2Me$ | H |
| Et | $CH=CHCO_2Et$ | H |
| Et | CH=CHCN | H |
| Et | Ph | H |
| Et | Ph-2-Cl | H |
| Et | Ph-3-Cl | H |
| Et | Ph-4-Cl | H |
| Et | $CH_2Ph$ | H |
| Et | $CH_2Ph$-2-Cl | H |
| Et | $CH_2Ph$-3-Cl | H |
| Et | $CH_2Ph$-4-Cl | H |
| Cl | Me | H |
| Br | Me | H |
| I | Me | H |
| $CF_3$ | Me | H |
| $NO_2$ | Me | H |
| OMe | Me | H |
| SMe | Me | H |
| $SO_2Me$ | Me | H |
| COMe | Me | H |
| $CO_2H$ | Me | H |
| $CO_2Me$ | Me | H |
| $CO_2Et$ | Me | H |
| $CH_2Cl$ | Me | H |
| $CH_2Br$ | Me | H |
| $CH_2I$ | Me | H |
| $CH_2CF_3$ | Me | H |
| $CH_2CN$ | Me | H |
| $CH_2OH$ | Me | H |
| $CH_2OMe$ | Me | H |
| $CH_2OEt$ | Me | H |
| $CH_2SMe$ | Me | H |
| $CH_2SEt$ | Me | H |
| $CH_2SO_2Me$ | Me | H |
| $CH_2SO_2Et$ | Me | H |
| $CH_2COMe$ | Me | H |
| $CH_2COEt$ | Me | H |
| $CH_2CO_2H$ | Me | H |

| $R_1$ | $R_2$ | $R_3$ |
|---|---|---|
| $CH_2CO_2Me$ | Me | H |
| $CH_2CO_2Et$ | Me | H |
| $CH_2OCH_2CH=CH_2$ | Me | H |
| $CH_2OCH_2C\equiv CH$ | Me | H |
| $CH=CHCO_2Me$ | Me | H |
| $CH=CHCO_2Et$ | Me | H |
| CH=CHCN | Me | H |
| Ph | Me | H |
| Ph-2-Cl | Me | H |
| Ph-3-Cl | Me | H |
| Ph-4-Cl | Me | H |
| $CH_2Ph$ | Me | H |
| $CH_2Ph$-2-Cl | Me | H |
| $CH_2Ph$-3-Cl | Me | H |
| $CH_2Ph$-4-Cl | Me | H |
| Cl | Et | H |
| Br | Et | H |
| I | Et | H |
| $CF_3$ | Et | H |
| $NO_2$ | Et | H |
| OMe | Et | H |
| SMe | Et | H |
| $SO_2Me$ | Et | H |
| COMe | Et | H |
| $CO_2H$ | Et | H |
| $CO_2Me$ | Et | H |
| $CO_2Et$ | Et | H |
| $CH_2Cl$ | Et | H |
| $CH_2Br$ | Et | H |
| $CH_2I$ | Et | H |
| $CH_2CF_3$ | Et | H |
| $CH_2CN$ | Et | H |
| $CH_2OH$ | Et | H |
| $CH_2OMe$ | Et | H |
| $CH_2OEt$ | Et | H |
| $CH_2SMe$ | Et | H |
| $CH_2SEt$ | Et | H |
| $CH_2SO_2Me$ | Et | H |
| $CH_2SO_2Et$ | Et | H |
| $CH_2COMe$ | Et | H |

| $R_1$ | $R_2$ | $R_3$ |
|---|---|---|
| $CH_2COEt$ | Et | H |
| $CH_2CO_2H$ | Et | H |
| $CH_2CO_2Me$ | Et | H |
| $CH_2CO_2Et$ | Et | H |
| $CH_2OCH_2CH=CH_2$ | Et | H |
| $CH_2OCH_2C\equiv CH$ | Et | H |
| $CH=CHCO_2Me$ | Et | H |
| $CH=CHCO_2Et$ | Et | H |
| $CH=CHCN$ | Et | H |
| Ph | Et | H |
| Ph-2-Cl | Et | H |
| Ph-3-Cl | Et | H |
| Ph-4-Cl | Et | H |
| $CH_2Ph$ | Et | H |
| $CH_2Ph$-2-Cl | Et | H |
| $CH_2Ph$-3-Cl | Et | H |
| $CH_2Ph$-4-Cl | Et | H |

Table 5

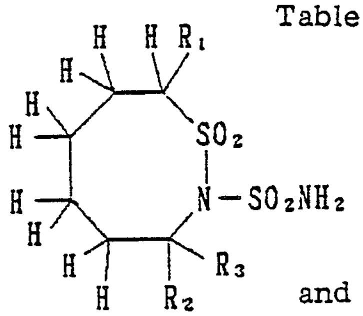

and

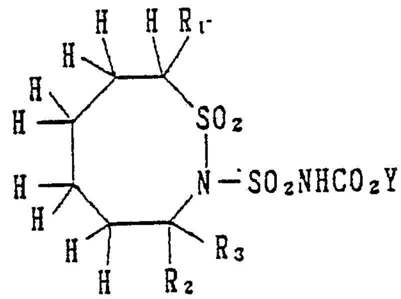

(Y=Me, Et, Pr-n, Bu-n or Ph)

| $R_1$ | $R_2$ | $R_3$ |
|---|---|---|
| H | H | H |
| Me | H | H |
| H | Me | H |
| Et | H | H |
| H | Et | H |
| Pr-n | H | H |

| $R_1$ | $R_2$ | $R_3$ |
|---|---|---|
| H | Pr-n | H |
| Me | Me | H |
| Me | Et | H |
| Me | Pr-n | H |
| Me | Pr-i | H |
| Et | Me | H |
| Et | Et | H |
| Et | Pr-n | H |
| Et | Pr-i | H |
| Pr-n | Me | H |
| Pr-n | Et | H |
| Pr-n | Pr-n | H |
| Pr-n | Pr-i | H |
| Pr-i | Me | H |
| Pr-i | Et | H |
| Pr-i | Pr-n | H |
| Pr-i | Pr-i | H |
| H | Me | Me |
| H | Me | Et |
| H | Me | Pr-n |
| H | Me | Pr-i |
| H | Et | Et |
| H | Et | Pr-n |
| Me | Me | Me |
| Et | Me | Me |
| Pr-n | Me | Me |
| Pr-i | Me | Me |
| H | Ph | H |
| H | $CH_2Ph$ | H |
| Me | Ph | H |
| Me | $CH_2Ph$ | H |
| Et | Ph | H |
| Et | $CH_2Ph$ | H |
| Ph | Me | H |
| $CH_2Ph$ | Me | H |
| Ph | Et | H |
| $CH_2Ph$ | Et | H |

Table 6

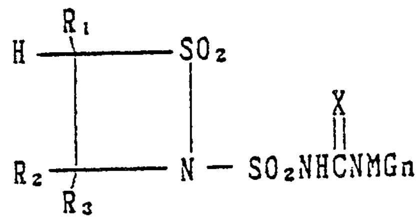

| $R_1$ | $R_2$ | $R_3$ | X | M | Gn |
|---|---|---|---|---|---|
| H | H | H | O | H | Ga |
| H | H | H | S | H | G3 |
| H | H | H | O | Me | Gb |
| H | H | H | O | Et | Gb |
| H | Me | H | O | H | Gb |
| H | Me | H | S | H | G3 |
| H | Et | H | O | H | Gb |
| H | Et | H | S | H | G3 |
| H | Pr-n | H | O | H | Gb |
| H | Pr-n | H | S | H | G3 |
| H | Pr-i | H | O | H | Gb |
| H | Pr-i | H | S | H | G3 |
| H | Bu-n | H | O | H | Gb |
| H | Bu-n | H | S | H | G3 |
| Me | H | H | O | H | Gb |
| Me | H | H | S | H | G3 |
| Et | H | H | O | H | Gb |
| Et | H | H | S | H | G3 |
| Pr-n | H | H | O | H | Gb |
| Pr-n | H | H | S | H | G3 |
| Pr-i | H | H | O | H | Gb |
| Pr-i | H | H | S | H | G3 |
| Bu-n | H | H | O | H | Gb |
| Bu-n | H | H | S | H | G3 |
| H | Me | Me | O | H | Ga |
| H | Me | Me | S | H | G3 |
| H | Me | Et | O | H | Gb |
| H | Me | Et | S | H | G3 |
| H | Me | Pr-n | O | H | Gb |
| H | Me | Pr-n | S | H | G3 |

| $R_1$ | $R_2$ | $R_3$ | X | M | Gn |
|---|---|---|---|---|---|
| H | Me | Pr-i | O | H | Gb |
| H | Me | Pr-i | S | H | G3 |
| H | Me | Bu-n | O | H | Gb |
| H | Me | Bu-n | S | H | G3 |
| H | Et | Et | O | H | Gb |
| H | Et | Et | S | H | G3 |
| H | Et | Pr-n | O | H | Gb |
| H | Et | Pr-n | S | H | G3 |
| H | Et | Pr-i | O | H | Gb |
| H | Et | Pr-i | S | H | G3 |
| H | Pr-n | Pr-n | O | H | Gb |
| H | Pr-n | Pr-n | S | H | G3 |
| Me | Me | H | O | H | Gb |
| Me | Me | H | S | H | G3 |
| Me | Et | H | O | H | Gb |
| Me | Et | H | S | H | G3 |
| Me | Pr-n | H | O | H | Gb |
| Me | Pr-n | H | S | H | G3 |
| Me | Pr-i | H | O | H | Gb |
| Me | Pr-i | H | S | H | G3 |
| Me | Bu-n | H | O | H | Gb |
| Me | Bu-n | H | S | H | G3 |
| Et | Me | H | O | H | Gb |
| Et | Me | H | S | H | G3 |
| Et | Et | H | O | H | Gb |
| Et | Et | H | S | H | G3 |
| Et | Pr-n | H | O | H | Gb |
| Et | Pr-n | H | S | H | G3 |
| Et | Pr-i | H | O | H | Gb |
| Et | Pr-i | H | S | H | G3 |
| Et | Bu-n | H | O | H | Gb |
| Et | Bu-n | H | S | H | G3 |
| Pr-n | Me | H | O | H | Gb |
| Pr-n | Me | H | S | H | G3 |

| $R_1$ | $R_2$ | $R_3$ | X | M | Gn |
|---|---|---|---|---|---|
| Pr-n | Et | H | O | H | Gb |
| Pr-n | Et | H | S | H | G3 |
| Pr-n | Pr-n | H | O | H | Gb |
| Pr-n | Pr-n | H | S | H | G3 |
| Pr-n | Pr-i | H | O | H | Gb |
| Pr-n | Pr-i | H | S | H | G3 |
| Pr-n | Bu-n | H | O | H | Gb |
| Pr-n | Bu-n | H | S | H | G3 |
| Pr-i | Me | H | O | H | Gb |
| Pr-i | Me | H | S | H | G3 |
| Pr-i | Et | H | O | H | Gb |
| Pr-i | Et | H | S | H | G3 |
| Pr-i | Pr-n | H | O | H | Gb |
| Pr-i | Pr-n | H | S | H | G3 |
| Pr-i | Pr-i | H | O | H | Gb |
| Pr-i | Pr-i | H | S | H | G3 |
| Pr-i | Bu-n | H | O | H | Gb |
| Pr-i | Bu-n | H | S | H | G3 |
| Bu-n | Me | H | O | H | Gb |
| Bu-n | Me | H | S | H | G3 |
| Bu-n | Et | H | O | H | Gb |
| Bu-n | Et | H | S | H | G3 |
| Bu-n | Pr-n | H | O | H | Gb |
| Bu-n | Pr-n | H | S | H | G3 |
| Bu-n | Pr-i | H | O | H | Gb |
| Bu-n | Pr-i | H | S | H | G3 |
| Bu-n | Bu-n | H | O | H | Gb |
| Bu-n | Bu-n | H | S | H | G3 |
| Me | Me | Me | O | H | Gb |
| Me | Me | Me | S | H | G3 |
| Me | Me | Et | O | H | Gb |
| Me | Me | Et | S | H | G3 |
| Me | Me | Pr-n | O | H | Gb |
| Me | Me | Pr-n | S | H | G3 |
| Me | Me | Pr-i | O | H | Gb |
| Me | Me | Pr-i | S | H | G3 |

| $R_1$ | $R_2$ | $R_3$ | X | M | Gn |
|---|---|---|---|---|---|
| Me | Et | Et | O | H | Gb |
| Me | Et | Et | S | H | G3 |
| Me | Et | Pr-n | O | H | Gb |
| Me | Et | Pr-n | S | H | G3 |
| Me | Et | Pr-i | O | H | Gb |
| Me | Et | Pr-i | S | H | G3 |
| Et | Me | Me | O | H | Gb |
| Et | Me | Me | S | H | G3 |
| Et | Me | Et | O | H | Gb |
| Et | Me | Et | S | H | G3 |
| Et | Me | Pr-n | O | H | Gb |
| Et | Me | Pr-n | S | H | G3 |
| Et | Me | Pr-i | O | H | Gb |
| Et | Me | Pr-i | S | H | G3 |
| Et | Et | Et | O | H | Gb |
| Et | Et | Et | S | H | G3 |
| Et | Et | Pr-n | O | H | Gb |
| Et | Et | Pr-n | S | H | G3 |
| Et | Et | Pr-i | O | H | Gb |
| Et | Et | Pr-i | S | H | G3 |
| Pr-n | Me | Me | O | H | Gb |
| Pr-n | Me | Me | S | H | G3 |
| Pr-n | Me | Et | O | H | Gb |
| Pr-n | Me | Et | S | H | G3 |
| Pr-n | Me | Pr-n | O | H | Gb |
| Pr-n | Me | Pr-n | S | H | G3 |
| Pr-n | Me | Pr-i | O | H | Gb |
| Pr-n | Me | Pr-i | S | H | G3 |
| Pr-n | Et | Et | O | H | Gb |
| Pr-n | Et | Et | S | H | G3 |
| Pr-n | Et | Pr-n | O | H | Gb |
| Pr-n | Et | Pr-n | S | H | G3 |
| Pr-n | Et | Pr-i | O | H | Gb |
| Pr-n | Et | Pr-i | S | H | G3 |

| $R_1$ | $R_2$ | $R_3$ | X | M | Gn |
|---|---|---|---|---|---|
| Pr-i | Me | Me | O | H | Gb |
| Pr-i | Me | Me | S | H | G3 |
| Pr-i | Me | Et | O | H | Gb |
| Pr-i | Me | Et | S | H | G3 |
| Pr-i | Me | Pr-n | O | H | Gb |
| Pr-i | Me | Pr-n | S | H | G3 |
| Pr-i | Me | Pr-i | O | H | Gb |
| Pr-i | Me | Pr-i | S | H | G3 |
| Pr-i | Et | Et | O | H | Gb |
| Pr-i | Et | Et | S | H | G3 |
| Pr-i | Et | Pr-n | O | H | Gb |
| Pr-i | Et | Pr-n | S | H | G3 |
| Pr-i | Et | Pr-i | O | H | Gb |
| Pr-i | Et | Pr-i | S | H | G3 |
| H | Ph | H | O | H | Ga |
| H | Ph | H | S | H | G3 |
| H | Ph-2-Cl | H | O | H | Ga |
| H | Ph-2-Cl | H | S | H | G3 |
| H | Ph-3-Cl | H | O | H | Gb |
| H | Ph-4-Cl | H | O | H | Gb |
| H | $CH_2Ph$ | H | O | H | Ga |
| H | $CH_2Ph$ | H | S | H | G3 |
| Ph | H | H | O | H | Ga |
| Ph | H | H | S | H | G3 |
| Ph-2-Cl | H | H | O | H | Ga |
| Ph-2-Cl | H | H | S | H | G3 |
| Ph-3-Cl | H | H | O | H | Gb |
| Ph-4-Cl | H | H | O | H | Gb |
| $CH_2Ph$ | H | H | O | H | Ga |
| $CH_2Ph$ | H | H | S | H | G3 |
| Me | Ph | H | O | H | Ga |
| Me | Ph | H | S | H | G3 |
| Me | Ph-2-Cl | H | O | H | Ga |
| Me | Ph-2-Cl | H | S | H | G3 |
| Me | Ph-3-Cl | H | O | H | Gb |
| Me | Ph-4-Cl | H | O | H | Gb |
| Me | $CH_2Ph$ | H | O | H | Ga |
| Me | $CH_2Ph$ | H | S | H | G3 |

| $R_1$ | $R_2$ | $R_3$ | X | M | Gn |
|---|---|---|---|---|---|
| Et | Ph | H | O | H | Ga |
| Et | Ph | H | S | H | G3 |
| Et | Ph-2-Cl | H | O | H | Ga |
| Et | Ph-2-Cl | H | S | H | G3 |
| Et | Ph-3-Cl | H | O | H | Gb |
| Et | Ph-4-Cl | H | O | H | Gb |
| Et | $CH_2Ph$ | H | O | H | Ga |
| Et | $CH_2Ph$ | H | S | H | G3 |
| Pr-n | Ph | H | O | H | Ga |
| Pr-n | Ph | H | S | H | G3 |
| Pr-n | Ph-2-Cl | H | O | H | Ga |
| Pr-n | Ph-2-Cl | H | S | H | G3 |
| Pr-n | Ph-3-Cl | H | O | H | Gb |
| Pr-n | Ph-4-Cl | H | O | H | Gb |
| Pr-n | $CH_2Ph$ | H | O | H | Ga |
| Pr-n | $CH_2Ph$ | H | S | H | G3 |
| Ph | Me | H | O | H | Ga |
| Ph | Me | H | S | H | G3 |
| Ph-2-Cl | Me | H | O | H | Ga |
| Ph-2-Cl | Me | H | S | H | G3 |
| Ph-3-Cl | Me | H | O | H | Gb |
| Ph-4-Cl | Me | H | O | H | Gb |
| $CH_2Ph$ | Me | H | O | H | Ga |
| $CH_2Ph$ | Me | H | S | H | G3 |
| Ph | Et | H | O | H | Ga |
| Ph | Et | H | S | H | G3 |
| Ph-2-Cl | Et | H | O | H | Ga |
| Ph-2-Cl | Et | H | S | H | G3 |
| Ph-3-Cl | Et | H | O | H | Gb |
| Ph-4-Cl | Et | H | O | H | Gb |
| $CH_2Ph$ | Et | H | O | H | Ga |
| $CH_2Ph$ | Et | H | S | H | G3 |
| Ph | Pr-n | H | O | H | Ga |
| Ph | Pr-n | H | S | H | G3 |
| Ph-2-Cl | Pr-n | H | O | H | Ga |
| Ph-2-Cl | Pr-n | H | S | H | G3 |
| Ph-3-Cl | Pr-n | H | O | H | Gb |
| Ph-4-Cl | Pr-n | H | O | H | Gb |
| $CH_2Ph$ | Pr-n | H | O | H | Ga |
| $CH_2Ph$ | Pr-n | H | S | H | G3 |

Table 7

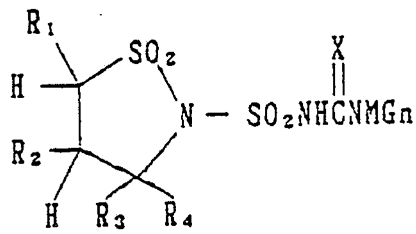

| R1 | R2 | R3 | R4 | X | M | Gn |
|---|---|---|---|---|---|---|
| H | H | H | H | O | H | Ga |
| H | H | H | H | S | H | G3 |
| H | H | H | H | O | Me | Gb |
| H | H | H | H | O | Et | Gb |
| H | H | H | H | O | $CH_2CH=CH_2$ | Gb |
| H | H | H | H | O | $CH_2C\equiv CH$ | Gb |
| Me | H | H | H | O | H | Ga |
| Me | H | H | H | S | H | G3 |
| H | Me | H | H | O | H | Gb |
| H | Me | H | H | S | H | G3 |
| H | H | Me | H | O | H | Ga |
| H | H | Me | H | S | H | G3 |
| H | H | Me | H | O | Me | Gb |
| H | H | Me | H | O | Et | Gb |
| H | H | Me | H | O | $CH_2CH=CH_2$ | Gb |
| H | H | Me | H | O | $CH_2C\equiv CH$ | Gb |
| Et | H | H | H | O | H | Ga |
| Et | H | H | H | S | H | G3 |
| H | Et | H | H | O | H | Ga |
| H | Et | H | H | S | H | G3 |
| H | H | Et | H | O | H | Ga |
| H | H | Et | H | S | H | G3 |
| H | H | Et | H | O | Me | Gb |
| H | H | Et | H | O | Et | Gb |
| H | H | Et | H | O | $CH_2CH=CH_2$ | Gb |
| H | H | Et | H | O | $CH_2C\equiv CH$ | Gb |
| Pr-n | H | H | H | O | H | Gb |
| Pr-n | H | H | H | S | H | G3 |
| H | Pr-n | H | H | O | H | Gb |
| H | Pr-n | H | H | S | H | G3 |

| $R_1$ | $R_2$ | $R_3$ | $R_4$ | X | M | Gn |
|---|---|---|---|---|---|---|
| H | H | Pr-n | H | O | H | Gb |
| H | H | Pr-n | H | S | H | G3 |
| Pr-i | H | H | H | O | H | Gb |
| Pr-i | H | H | H | S | H | G3 |
| H | Pr-i | H | H | O | H | Gb |
| H | Pr-i | H | H | S | H | G3 |
| H | H | Pr-i | H | O | H | Gb |
| H | H | Pr-i | H | S | H | G3 |
| Bu-n | H | H | H | O | H | Gb |
| H | Bu-n | H | H | O | H | Gb |
| H | H | Bu-n | H | O | H | Gb |
| H | H | Me | Me | O | H | Ga |
| H | H | Me | Me | S | H | G3 |
| H | H | Me | Me | O | Me | Gb |
| H | H | Me | Me | O | Et | Gb |
| H | H | Me | Me | O | $CH_2CH=CH_2$ | Gb |
| H | H | Me | Me | O | $CH_2C\equiv CH$ | Gb |
| Me | H | Me | Me | O | H | Ga |
| Me | H | Me | Me | S | H | G3 |
| H | Me | Me | Me | O | H | Ga |
| H | Me | Me | Me | S | H | G3 |
| Et | H | Me | Me | O | H | Gb |
| H | Et | Me | Me | O | H | Gb |
| Pr-n | H | Me | Me | O | H | Gb |
| H | Pr-n | Me | Me | O | H | Gb |
| Pr-i | H | Me | Me | O | H | Gb |
| H | Pr-i | Me | Me | O | H | Gb |
| Bu-n | H | Me | Me | O | H | Gb |
| H | Bu-n | Me | Me | O | H | Gb |
| Me | Me | Me | Me | O | H | Ga |
| Me | Me | Me | Me | S | H | G3 |
| Me | Et | Me | Me | O | H | Gb |
| Me | Pr-n | Me | Me | O | H | Gb |
| Me | Bu-n | Me | Me | O | H | Gb |
| Et | Me | Me | Me | O | H | Gb |
| Et | Et | Me | Me | O | H | Gb |
| Et | Pr-n | Me | Me | O | H | Gb |
| Et | Bu-n | Me | Me | O | H | Gb |
| Pr-n | Me | Me | Me | O | H | Gb |
| Pr-n | Et | Me | Me | O | H | Gb |

| $R_1$ | $R_2$ | $R_3$ | $R_4$ | X | M | Gn |
|---|---|---|---|---|---|---|
| Pr-n | Pr-n | Me | Me | O | H | Gb |
| Pr-n | Bu-n | Me | Me | O | H | Gb |
| Bu-n | Me | Me | Me | O | H | Gb |
| Bu-n | Et | Me | Me | O | H | Gb |
| Bu-n | Pr-n | Me | Me | O | H | Gb |
| Bu-n | Bu-n | Me | Me | O | H | Gb |
| F | H | Me | Me | O | H | Ga |
| Cl | H | Me | Me | O | H | Ga |
| Br | H | Me | Me | O | H | Ga |
| I | H | Me | Me | O | H | Ga |
| $CH=CH_2$ | H | Me | Me | O | H | Ga |
| $CH_2CH=CH_2$ | H | Me | Me | O | H | Ga |
| $C\equiv CH$ | H | Me | Me | O | H | Ga |
| $CH_2C\equiv CH$ | H | Me | Me | O | H | Ga |
| $CH_2F$ | H | Me | Me | O | H | Ga |
| $CHF_2$ | H | Me | Me | O | H | Ga |
| CN | H | Me | Me | O | H | Ga |
| $NH_2$ | H | Me | Me | O | H | Ga |
| NHCOMe | H | Me | Me | O | H | Ga |
| $CH_2NH_2$ | H | Me | Me | O | H | Ga |
| $CH_2NHCOMe$ | H | Me | Me | O | H | Ga |
| $CH_2OCHF_2$ | H | Me | Me | O | H | Ga |
| $CF_3$ | H | Me | Me | O | H | Ga |
| $NO_2$ | H | Me | Me | O | H | Gb |
| OMe | H | Me | Me | O | H | Ga |
| OEt | H | Me | Me | O | H | Ga |
| OPr-n | H | Me | Me | O | H | Gb |
| SMe | H | Me | Me | O | H | Ga |
| SEt | H | Me | Me | O | H | Ga |
| $SO_2Me$ | H | Me | Me | O | H | Ga |
| $SO_2Et$ | H | Me | Me | O | H | Ga |
| COMe | H | Me | Me | O | H | Ga |
| COEt | H | Me | Me | O | H | Ga |
| $CO_2H$ | H | Me | Me | O | H | Gb |
| $CO_2Me$ | H | Me | Me | O | H | Ga |
| $CO_2Et$ | H | Me | Me | O | H | Ga |
| $CO_2Pr$-n | H | Me | Me | O | H | Gb |
| $CO_2Bu$-n | H | Me | Me | O | H | Gc |
| $CH_2Cl$ | H | Me | Me | O | H | Ga |
| $CH_2Br$ | H | Me | Me | O | H | Gb |

| $R_1$ | $R_2$ | $R_3$ | $R_4$ | X | M | Gn |
|---|---|---|---|---|---|---|
| $CH_2I$ | H | Me | Me | O | H | Gb |
| $CH_2CF_3$ | H | Me | Me | O | H | Ga |
| $CH_2CN$ | H | Me | Me | O | H | Ga |
| $CH_2OH$ | H | Me | Me | O | H | Gb |
| $CH_2OMe$ | H | Me | Me | O | H | Ga |
| $CH_2OEt$ | H | Me | Me | O | H | Ga |
| $CH_2OPr$-n | H | Me | Me | O | H | Gb |
| $CH_2SMe$ | H | Me | Me | O | H | Ga |
| $CH_2SEt$ | H | Me | Me | O | H | Ga |
| $CH_2SPr$-n | H | Me | Me | O | H | Gb |
| $CH_2SO_2Me$ | H | Me | Me | O | H | Ga |
| $CH_2SO_2Et$ | H | Me | Me | O | H | Ga |
| $CH_2COMe$ | H | Me | Me | O | H | Ga |
| $CH_2COEt$ | H | Me | Me | O | H | Ga |
| $CH_2COPr$-n | H | Me | Me | O | H | Gb |
| $CH_2CO_2H$ | H | Me | Me | O | H | Gb |
| $CH_2CO_2Me$ | H | Me | Me | O | H | Ga |
| $CH_2CO_2Et$ | H | Me | Me | O | H | Ga |
| $CH_2CO_2Pr$-n | H | Me | Me | O | H | Gb |
| $CH_2OCH_2CH=CH_2$ | H | Me | Me | O | H | Gb |
| $CH_2OCH_2C\equiv CH$ | H | Me | Me | O | H | Gb |
| $CH=CHCO_2Me$ | H | Me | Me | O | H | Ga |
| $CH=CHCO_2Et$ | H | Me | Me | O | H | Ga |
| $CH=CHCO_2Pr$-n | H | Me | Me | O | H | Gb |
| CH=CHCN | H | Me | Me | O | H | Ga |
| Ph | H | Me | Me | O | H | Ga |
| $CH_2Ph$ | H | Me | Me | O | H | Ga |
| OPh | H | Me | Me | O | H | Ga |
| SPh | H | Me | Me | O | H | Ga |
| $SO_2Ph$ | H | Me | Me | O | H | Ga |
| CONHMe | H | Me | Me | O | H | Ga |
| CONHEt | H | Me | Me | O | H | Ga |
| $CON(Me)_2$ | H | Me | Me | O | H | Ga |
| $CON(Et)_2$ | H | Me | Me | O | H | Ga |
| F | Me | Me | Me | O | H | Ga |
| Cl | Me | Me | Me | O | H | Ga |
| Br | Me | Me | Me | O | H | Ga |
| I | Me | Me | Me | O | H | Ga |
| $CH=CH_2$ | Me | Me | Me | O | H | Ga |
| $CH_2CH=CH_2$ | Me | Me | Me | O | H | Ga |

| $R_1$ | $R_2$ | $R_3$ | $R_4$ | X | M | Gn |
|---|---|---|---|---|---|---|
| C ≡CH | Me | Me | Me | O | H | Ga |
| $CH_2C≡CH$ | Me | Me | Me | O | H | Ga |
| $CH_2F$ | Me | Me | Me | O | H | Ga |
| $CHF_2$ | Me | Me | Me | O | H | Ga |
| CN | Me | Me | Me | O | H | Ga |
| $NH_2$ | Me | Me | Me | O | H | Ga |
| NHCOMe | Me | Me | Me | O | H | Ga |
| $CH_2NH_2$ | Me | Me | Me | O | H | Ga |
| $CH_2NHCOMe$ | Me | Me | Me | O | H | Ga |
| $CH_2OCHF_2$ | Me | Me | Me | O | H | Ga |
| $CF_3$ | Me | Me | Me | O | H | Ga |
| $NO_2$ | Me | Me | Me | O | H | Gb |
| OMe | Me | Me | Me | O | H | Ga |
| OEt | Me | Me | Me | O | H | Ga |
| OPr-n | Me | Me | Me | O | H | Gb |
| SMe | Me | Me | Me | O | H | Ga |
| SEt | Me | Me | Me | O | H | Ga |
| $SO_2Me$ | Me | Me | Me | O | H | Ga |
| $SO_2Et$ | Me | Me | Me | O | H | Ga |
| COMe | Me | Me | Me | O | H | Ga |
| COEt | Me | Me | Me | O | H | Ga |
| $CO_2H$ | Me | Me | Me | O | H | Gb |
| $CO_2Me$ | Me | Me | Me | O | H | Ga |
| $CO_2Et$ | Me | Me | Me | O | H | Ga |
| $CO_2Pr$-n | Me | Me | Me | O | H | Gb |
| $CO_2Bu$-n | Me | Me | Me | O | H | Gc |
| $CH_2Cl$ | Me | Me | Me | O | H | Ga |
| $CH_2Br$ | Me | Me | Me | O | H | Gb |
| $CH_2I$ | Me | Me | Me | O | H | Gb |
| $CH_2CF_3$ | Me | Me | Me | O | H | Ga |
| $CH_2CN$ | Me | Me | Me | O | H | Ga |
| $CH_2OH$ | Me | Me | Me | O | H | Gb |
| $CH_2OMe$ | Me | Me | Me | O | H | Ga |
| $CH_2OEt$ | Me | Me | Me | O | H | Ga |
| $CH_2OPr$-n | Me | Me | Me | O | H | Gb |
| $CH_2SMe$ | Me | Me | Me | O | H | Ga |
| $CH_2SEt$ | Me | Me | Me | O | H | Ga |
| $CH_2SPr$-n | Me | Me | Me | O | H | Gb |
| $CH_2SO_2Me$ | Me | Me | Me | O | H | Ga |
| $CH_2SO_2Et$ | Me | Me | Me | O | H | Ga |

| $R_1$ | $R_2$ | $R_3$ | $R_4$ | X | M | Gn |
|---|---|---|---|---|---|---|
| $CH_2COMe$ | Me | Me | Me | O | H | Ga |
| $CH_2COEt$ | Me | Me | Me | O | H | Ga |
| $CH_2COPr\text{-}n$ | Me | Me | Me | O | H | Gb |
| $CH_2CO_2H$ | Me | Me | Me | O | H | Gb |
| $CH_2CO_2Me$ | Me | Me | Me | O | H | Ga |
| $CH_2CO_2Et$ | Me | Me | Me | O | H | Ga |
| $CH_2CO_2Pr\text{-}n$ | Me | Me | Me | O | H | Gb |
| $CH_2OCH_2CH{=}CH_2$ | Me | Me | Me | O | H | Gb |
| $CH_2OCH_2C{\equiv}CH$ | Me | Me | Me | O | H | Gb |
| $CH{=}CHCO_2Me$ | Me | Me | Me | O | H | Ga |
| $CH{=}CHCO_2Et$ | Me | Me | Me | O | H | Ga |
| $CH{=}CHCO_2Pr\text{-}n$ | Me | Me | Me | O | H | Gb |
| $CH{=}CHCN$ | Me | Me | Me | O | H | Ga |
| Ph | Me | Me | Me | O | H | Ga |
| $CH_2Ph$ | Me | Me | Me | O | H | Ga |
| OPh | Me | Me | Me | O | H | Ga |
| SPh | Me | Me | Me | O | H | Ga |
| $SO_2Ph$ | Me | Me | Me | O | H | Ga |
| CONHMe | Me | Me | Me | O | H | Ga |
| CONHEt | Me | Me | Me | O | H | Ga |
| $CON(Me)_2$ | Me | Me | Me | O | H | Ga |
| $CON(Et)_2$ | Me | Me | Me | O | H | Ga |
| F | Cl | Me | Me | O | H | Ga |
| Cl | Cl | Me | Me | O | H | Ga |
| Br | Cl | Me | Me | O | H | Ga |
| I | Cl | Me | Me | O | H | Ga |
| $CH{=}CH_2$ | Cl | Me | Me | O | H | Ga |
| $CH_2CH{=}CH_2$ | Cl | Me | Me | O | H | Ga |
| $C{\equiv}CH$ | Cl | Me | Me | O | H | Ga |
| $CH_2C{\equiv}CH$ | Cl | Me | Me | O | H | Ga |
| $CH_2F$ | Cl | Me | Me | O | H | Ga |
| $CHF_2$ | Cl | Me | Me | O | H | Ga |
| CN | Cl | Me | Me | O | H | Ga |
| $NH_2$ | Cl | Me | Me | O | H | Ga |
| NHCOMe | Cl | Me | Me | O | H | Ga |
| $CH_2NH_2$ | Cl | Me | Me | O | H | Ga |
| $CH_2NHCOMe$ | Cl | Me | Me | O | H | Ga |
| $CH_2OCHF_2$ | Cl | Me | Me | O | H | Ga |
| $CF_3$ | Cl | Me | Me | O | H | Ga |
| $NO_2$ | Cl | Me | Me | O | H | Gb |

| $R_1$ | $R_2$ | $R_3$ | $R_4$ | X | M | Gn |
|---|---|---|---|---|---|---|
| OMe | Cl | Me | Me | O | H | Ga |
| OEt | Cl | Me | Me | O | H | Ga |
| OPr-n | Cl | Me | Me | O | H | Gb |
| SMe | Cl | Me | Me | O | H | Ga |
| SEt | Cl | Me | Me | O | H | Ga |
| $SO_2Me$ | Cl | Me | Me | O | H | Ga |
| $SO_2Et$ | Cl | Me | Me | O | H | Ga |
| COMe | Cl | Me | Me | O | H | Ga |
| COEt | Cl | Me | Me | O | H | Ga |
| $CO_2H$ | Cl | Me | Me | O | H | Gb |
| $CO_2Me$ | Cl | Me | Me | O | H | Ga |
| $CO_2Et$ | Cl | Me | Me | O | H | Ga |
| $CO_2Pr$-n | Cl | Me | Me | O | H | Gb |
| $CO_2Bu$-n | Cl | Me | Me | O | H | Gc |
| $CH_2Cl$ | Cl | Me | Me | O | H | Ga |
| $CH_2Br$ | Cl | Me | Me | O | H | Gb |
| $CH_2I$ | Cl | Me | Me | O | H | Gb |
| $CH_2CF_3$ | Cl | Me | Me | O | H | Ga |
| $CH_2CN$ | Cl | Me | Me | O | H | Ga |
| $CH_2OH$ | Cl | Me | Me | O | H | Gb |
| $CH_2OMe$ | Cl | Me | Me | O | H | Ga |
| $CH_2OEt$ | Cl | Me | Me | O | H | Ga |
| $CH_2OPr$-n | Cl | Me | Me | O | H | Gb |
| $CH_2SMe$ | Cl | Me | Me | O | H | Ga |
| $CH_2SEt$ | Cl | Me | Me | O | H | Ga |
| $CH_2SPr$-n | Cl | Me | Me | O | H | Gb |
| $CH_2SO_2Me$ | Cl | Me | Me | O | H | Ga |
| $CH_2SO_2Et$ | Cl | Me | Me | O | H | Ga |
| $CH_2COMe$ | Cl | Me | Me | O | H | Ga |
| $CH_2COEt$ | Cl | Me | Me | O | H | Ga |
| $CH_2COPr$-n | Cl | Me | Me | O | H | Gb |
| $CH_2CO_2H$ | Cl | Me | Me | O | H | Gb |
| $CH_2CO_2Me$ | Cl | Me | Me | O | H | Ga |
| $CH_2CO_2Et$ | Cl | Me | Me | O | H | Ga |
| $CH_2CO_2Pr$-n | Cl | Me | Me | O | H | Gb |
| $CH_2OCH_2CH=CH_2$ | Cl | Me | Me | O | H | Gb |
| $CH_2OCH_2C\equiv CH$ | Cl | Me | Me | O | H | Gb |
| $CH=CHCO_2Me$ | Cl | Me | Me | O | H | Ga |
| $CH=CHCO_2Et$ | Cl | Me | Me | O | H | Ga |
| $CH=CHCO_2Pr$-n | Cl | Me | Me | O | H | Gb |

| $R_1$ | $R_2$ | $R_3$ | $R_4$ | X | M | Gn |
|---|---|---|---|---|---|---|
| CH=CHCN | Cl | Me | Me | O | H | Ga |
| Ph | Cl | Me | Me | O | H | Ga |
| $CH_2Ph$ | Cl | Me | Me | O | H | Ga |
| OPh | Cl | Me | Me | O | H | Ga |
| SPh | Cl | Me | Me | O | H | Ga |
| $SO_2Ph$ | Cl | Me | Me | O | H | Ga |
| CONHMe | Cl | Me | Me | O | H | Ga |
| CONHEt | Cl | Me | Me | O | H | Ga |
| $CON(Me)_2$ | Cl | Me | Me | O | H | Ga |
| $CON(Et)_2$ | Cl | Me | Me | O | H | Ga |
| H | H | Me | Et | O | H | Ga |
| F | H | Me | Et | O | H | Ga |
| Cl | H | Me | Et | O | H | Ga |
| Br | H | Me | Et | O | H | Ga |
| I | H | Me | Et | O | H | Ga |
| $CH=CH_2$ | H | Me | Et | O | H | Ga |
| $CH_2CH=CH_2$ | H | Me | Et | O | H | Ga |
| C≡CH | H | Me | Et | O | H | Ga |
| $CH_2C≡CH$ | H | Me | Et | O | H | Ga |
| $CH_2F$ | H | Me | Et | O | H | Ga |
| $CHF_2$ | H | Me | Et | O | H | Ga |
| CN | H | Me | Et | O | H | Ga |
| $NH_2$ | H | Me | Et | O | H | Ga |
| NHCOMe | H | Me | Et | O | H | Ga |
| $CH_2NH_2$ | H | Me | Et | O | H | Ga |
| $CH_2NHCOMe$ | H | Me | Et | O | H | Ga |
| $CH_2OCHF_2$ | H | Me | Et | O | H | Ga |
| $CF_3$ | H | Me | Et | O | H | Ga |
| $NO_2$ | H | Me | Et | O | H | Gb |
| OMe | H | Me | Et | O | H | Ga |
| OEt | H | Me | Et | O | H | Ga |
| OPr-n | H | Me | Et | O | H | Gb |
| SMe | H | Me | Et | O | H | Ga |
| SEt | H | Me | Et | O | H | Ga |
| $SO_2Me$ | H | Me | Et | O | H | Ga |
| $SO_2Et$ | H | Me | Et | O | H | Ga |
| COMe | H | Me | Et | O | H | Ga |
| COEt | H | Me | Et | O | H | Ga |
| $CO_2H$ | H | Me | Et | O | H | Gb |
| $CO_2Me$ | H | Me | Et | O | H | Ga |

| $R_1$ | $R_2$ | $R_3$ | $R_4$ | X | M | Gn |
|---|---|---|---|---|---|---|
| $CO_2Et$ | H | Me | Et | O | H | Ga |
| $CO_2Pr$-n | H | Me | Et | O | H | Gb |
| $CO_2Bu$-n | H | Me | Et | O | H | Gc |
| $CH_2Cl$ | H | Me | Et | O | H | Ga |
| $CH_2Br$ | H | Me | Et | O | H | Gb |
| $CH_2I$ | H | Me | Et | O | H | Gb |
| $CH_2CF_3$ | H | Me | Et | O | H | Ga |
| $CH_2CN$ | H | Me | Et | O | H | Ga |
| $CH_2OH$ | H | Me | Et | O | H | Gb |
| $CH_2OMe$ | H | Me | Et | O | H | Ga |
| $CH_2OEt$ | H | Me | Et | O | H | Ga |
| $CH_2OPr$-n | H | Me | Et | O | H | Gb |
| $CH_2SMe$ | H | Me | Et | O | H | Ga |
| $CH_2SEt$ | H | Me | Et | O | H | Ga |
| $CH_2SPr$-n | H | Me | Et | O | H | Gb |
| $CH_2SO_2Me$ | H | Me | Et | O | H | Ga |
| $CH_2SO_2Et$ | H | Me | Et | O | H | Ga |
| $CH_2COMe$ | H | Me | Et | O | H | Ga |
| $CH_2COEt$ | H | Me | Et | O | H | Ga |
| $CH_2COPr$-n | H | Me | Et | O | H | Gb |
| $CH_2CO_2H$ | H | Me | Et | O | H | Gb |
| $CH_2CO_2Me$ | H | Me | Et | O | H | Ga |
| $CH_2CO_2Et$ | H | Me | Et | O | H | Ga |
| $CH_2CO_2Pr$-n | H | Me | Et | O | H | Gb |
| $CH_2OCH_2CH{=}CH_2$ | H | Me | Et | O | H | Gb |
| $CH_2OCH_2C{\equiv}CH$ | H | Me | Et | O | H | Gb |
| $CH{=}CHCO_2Me$ | H | Me | Et | O | H | Ga |
| $CH{=}CHCO_2Et$ | H | Me | Et | O | H | Ga |
| $CH{=}CHCO_2Pr$-n | H | Me | Et | O | H | Gb |
| $CH{=}CHCN$ | H | Me | Et | O | H | Ga |
| Ph | H | Me | Et | O | H | Ga |
| $CH_2Ph$ | H | Me | Et | O | H | Ga |
| OPh | H | Me | Et | O | H | Ga |
| SPh | H | Me | Et | O | H | Ga |
| $SO_2Ph$ | H | Me | Et | O | H | Ga |
| CONHMe | H | Me | Et | O | H | Ga |
| CONHEt | H | Me | Et | O | H | Ga |
| $CON(Me)_2$ | H | Me | Et | O | H | Ga |
| $CON(Et)_2$ | H | Me | Et | O | H | Ga |
| H | H | Me | Pr-n | O | H | Ga |

| $R_1$ | $R_2$ | $R_3$ | $R_4$ | X | M | Gn |
|---|---|---|---|---|---|---|
| F | H | Me | Pr-n | O | H | Ga |
| Cl | H | Me | Pr-n | O | H | Ga |
| Br | H | Me | Pr-n | O | H | Ga |
| I | H | Me | Pr-n | O | H | Ga |
| $CH=CH_2$ | H | Me | Pr-n | O | H | Ga |
| $CH_2CH=CH_2$ | H | Me | Pr-n | O | H | Ga |
| $C\equiv CH$ | H | Me | Pr-n | O | H | Ga |
| $CH_2C\equiv CH$ | H | Me | Pr-n | O | H | Ga |
| $CH_2F$ | H | Me | Pr-n | O | H | Ga |
| $CHF_2$ | H | Me | Pr-n | O | H | Ga |
| CN | H | Me | Pr-n | O | H | Ga |
| $NH_2$ | H | Me | Pr-n | O | H | Ga |
| NHCOMe | H | Me | Pr-n | O | H | Ga |
| $CH_2NH_2$ | H | Me | Pr-n | O | H | Ga |
| $CH_2NHCOMe$ | H | Me | Pr-n | O | H | Ga |
| $CH_2OCHF_2$ | H | Me | Pr-n | O | H | Ga |
| $CF_3$ | H | Me | Pr-n | O | H | Ga |
| $NO_2$ | H | Me | Pr-n | O | H | Gb |
| OMe | H | Me | Pr-n | O | H | Ga |
| OEt | H | Me | Pr-n | O | H | Ga |
| OPr-n | H | Me | Pr-n | O | H | Gb |
| SMe | H | Me | Pr-n | O | H | Ga |
| SEt | H | Me | Pr-n | O | H | Ga |
| $SO_2Me$ | H | Me | Pr-n | O | H | Ga |
| $SO_2Et$ | H | Me | Pr-n | O | H | Ga |
| COMe | H | Me | Pr-n | O | H | Ga |
| COEt | H | Me | Pr-n | O | H | Ga |
| $CO_2H$ | H | Me | Pr-n | O | H | Gb |
| $CO_2Me$ | H | Me | Pr-n | O | H | Ga |
| $CO_2Et$ | H | Me | Pr-n | O | H | Ga |
| $CO_2Pr$-n | H | Me | Pr-n | O | H | Gb |
| $CO_2Bu$-n | H | Me | Pr-n | O | H | Gc |
| $CH_2Cl$ | H | Me | Pr-n | O | H | Ga |
| $CH_2Br$ | H | Me | Pr-n | O | H | Gb |
| $CH_2I$ | H | Me | Pr-n | O | H | Gb |
| $CH_2CF_3$ | H | Me | Pr-n | O | H | Ga |
| $CH_2CN$ | H | Me | Pr-n | O | H | Ga |
| $CH_2OH$ | H | Me | Pr-n | O | H | Gb |
| $CH_2OMe$ | H | Me | Pr-n | O | H | Ga |
| $CH_2OEt$ | H | Me | Pr-n | O | H | Ga |

| $R_1$ | $R_2$ | $R_3$ | $R_4$ | X | M | Gn |
|---|---|---|---|---|---|---|
| $CH_2OPr$-n | H | Me | Pr-n | O | H | Gb |
| $CH_2SMe$ | H | Me | Pr-n | O | H | Ga |
| $CH_2SEt$ | H | Me | Pr-n | O | H | Ga |
| $CH_2SPr$-n | H | Me | Pr-n | O | H | Gb |
| $CH_2SO_2Me$ | H | Me | Pr-n | O | H | Ga |
| $CH_2SO_2Et$ | H | Me | Pr-n | O | H | Ga |
| $CH_2COMe$ | H | Me | Pr-n | O | H | Ga |
| $CH_2COEt$ | H | Me | Pr-n | O | H | Ga |
| $CH_2COPr$-n | H | Me | Pr-n | O | H | Gb |
| $CH_2CO_2H$ | H | Me | Pr-n | O | H | Gb |
| $CH_2CO_2Me$ | H | Me | Pr-n | O | H | Ga |
| $CH_2CO_2Et$ | H | Me | Pr-n | O | H | Ga |
| $CH_2CO_2Pr$-n | H | Me | Pr-n | O | H | Gb |
| $CH_2OCH_2CH=CH_2$ | H | Me | Pr-n | O | H | Gb |
| $CH_2OCH_2C\equiv CH$ | H | Me | Pr-n | O | H | Gb |
| $CH=CHCO_2Me$ | H | Me | Pr-n | O | H | Ga |
| $CH=CHCO_2Et$ | H | Me | Pr-n | O | H | Ga |
| $CH=CHCO_2Pr$-n | H | Me | Pr-n | O | H | Gb |
| CH=CHCN | H | Me | Pr-n | O | H | Ga |
| Ph | H | Me | Pr-n | O | H | Ga |
| $CH_2Ph$ | H | Me | Pr-n | O | H | Ga |
| OPh | H | Me | Pr-n | O | H | Ga |
| SPh | H | Me | Pr-n | O | H | Ga |
| $SO_2Ph$ | H | Me | Pr-n | O | H | Ga |
| CONHMe | H | Me | Pr-n | O | H | Ga |
| CONHEt | H | Me | Pr-n | O | H | Ga |
| $CON(Me)_2$ | H | Me | Pr-n | O | H | Ga |
| $CON(Et)_2$ | H | Me | Pr-n | O | H | Ga |
| H | H | Me | Pr-i | O | H | Ga |
| F | H | Me | Pr-i | O | H | Ga |
| Cl | H | Me | Pr-i | O | H | Ga |
| Br | H | Me | Pr-i | O | H | Ga |
| I | H | Me | Pr-i | O | H | Ga |
| $CH=CH_2$ | H | Me | Pr-i | O | H | Ga |
| $CH_2CH=CH_2$ | H | Me | Pr-i | O | H | Ga |
| $C\equiv CH$ | H | Me | Pr-i | O | H | Ga |
| $CH_2C\equiv CH$ | H | Me | Pr-i | O | H | Ga |
| $CH_2F$ | H | Me | Pr-i | O | H | Ga |
| $CHF_2$ | H | Me | Pr-i | O | H | Ga |
| CN | H | Me | Pr-i | O | H | Ga |

| $R_1$ | $R_2$ | $R_3$ | $R_4$ | X | M | Gn |
|---|---|---|---|---|---|---|
| $NH_2$ | H | Me | Pr-i | O | H | Ga |
| NHCOMe | H | Me | Pr-i | O | H | Ga |
| $CH_2NH_2$ | H | Me | Pr-i | O | H | Ga |
| $CH_2NHCOMe$ | H | Me | Pr-i | O | H | Ga |
| $CH_2OCHF_2$ | H | Me | Pr-i | O | H | Ga |
| $CF_3$ | H | Me | Pr-i | O | H | Ga |
| $NO_2$ | H | Me | Pr-i | O | H | Gb |
| OMe | H | Me | Pr-i | O | H | Ga |
| OEt | H | Me | Pr-i | O | H | Ga |
| OPr-n | H | Me | Pr-i | O | H | Gb |
| SMe | H | Me | Pr-i | O | H | Ga |
| SEt | H | Me | Pr-i | O | H | Ga |
| $SO_2Me$ | H | Me | Pr-i | O | H | Ga |
| $SO_2Et$ | H | Me | Pr-i | O | H | Ga |
| COMe | H | Me | Pr-i | O | H | Ga |
| COEt | H | Me | Pr-i | O | H | Ga |
| $CO_2H$ | H | Me | Pr-i | O | H | Gb |
| $CO_2Me$ | H | Me | Pr-i | O | H | Ga |
| $CO_2Et$ | H | Me | Pr-i | O | H | Ga |
| $CO_2Pr$-n | H | Me | Pr-i | O | H | Gb |
| $CO_2Bu$-n | H | Me | Pr-i | O | H | Gc |
| $CH_2Cl$ | H | Me | Pr-i | O | H | Ga |
| $CH_2Br$ | H | Me | Pr-i | O | H | Gb |
| $CH_2I$ | H | Me | Pr-i | O | H | Gb |
| $CH_2CF_3$ | H | Me | Pr-i | O | H | Ga |
| $CH_2CN$ | H | Me | Pr-i | O | H | Ga |
| $CH_2OH$ | H | Me | Pr-i | O | H | Gb |
| $CH_2OMe$ | H | Me | Pr-i | O | H | Ga |
| $CH_2OEt$ | H | Me | Pr-i | O | H | Ga |
| $CH_2OPr$-n | H | Me | Pr-i | O | H | Gb |
| $CH_2SMe$ | H | Me | Pr-i | O | H | Ga |
| $CH_2SEt$ | H | Me | Pr-i | O | H | Ga |
| $CH_2SPr$-n | H | Me | Pr-i | O | H | Gb |
| $CH_2SO_2Me$ | H | Me | Pr-i | O | H | Ga |
| $CH_2SO_2Et$ | H | Me | Pr-i | O | H | Ga |
| $CH_2COMe$ | H | Me | Pr-i | O | H | Ga |
| $CH_2COEt$ | H | Me | Pr-i | O | H | Ga |
| $CH_2COPr$-n | H | Me | Pr-i | O | H | Gb |
| $CH_2CO_2H$ | H | Me | Pr-i | O | H | Gb |
| $CH_2CO_2Me$ | H | Me | Pr-i | O | H | Ga |

| $R_1$ | $R_2$ | $R_3$ | $R_4$ | X | M | Gn |
|---|---|---|---|---|---|---|
| $CH_2CO_2Et$ | H | Me | Pr-i | O | H | Ga |
| $CH_2CO_2Pr\text{-}n$ | H | Me | Pr-i | O | H | Gb |
| $CH_2OCH_2CH=CH_2$ | H | Me | Pr-i | O | H | Gb |
| $CH_2OCH_2C\equiv CH$ | H | Me | Pr-i | O | H | Gb |
| $CH=CHCO_2Me$ | H | Me | Pr-i | O | H | Ga |
| $CH=CHCO_2Et$ | H | Me | Pr-i | O | H | Ga |
| $CH=CHCO_2Pr\text{-}n$ | H | Me | Pr-i | O | H | Gb |
| CH=CHCN | H | Me | Pr-i | O | H | Ga |
| Ph | H | Me | Pr-i | O | H | Ga |
| $CH_2Ph$ | H | Me | Pr-i | O | H | Ga |
| OPh | H | Me | Pr-i | O | H | Ga |
| SPh | H | Me | Pr-i | O | H | Ga |
| $SO_2Ph$ | H | Me | Pr-i | O | H | Ga |
| CONHMe | H | Me | Pr-i | O | H | Ga |
| CONHEt | H | Me | Pr-i | O | H | Ga |
| $CON(Me)_2$ | H | Me | Pr-i | O | H | Ga |
| $CON(Et)_2$ | H | Me | Pr-i | O | H | Ga |
| H | H | Et | Et | O | H | Ga |
| F | H | Et | Et | O | H | Ga |
| Cl | H | Et | Et | O | H | Ga |
| Br | H | Et | Et | O | H | Ga |
| I | H | Et | Et | O | H | Ga |
| $CH=CH_2$ | H | Et | Et | O | H | Ga |
| $CH_2CH=CH_2$ | H | Et | Et | O | H | Ga |
| $C\equiv CH$ | H | Et | Et | O | H | Ga |
| $CH_2C\equiv CH$ | H | Et | Et | O | H | Ga |
| $CH_2F$ | H | Et | Et | O | H | Ga |
| $CHF_2$ | H | Et | Et | O | H | Ga |
| CN | H | Et | Et | O | H | Ga |
| $NH_2$ | H | Et | Et | O | H | Ga |
| NHCOMe | H | Et | Et | O | H | Ga |
| $CH_2NH_2$ | H | Et | Et | O | H | Ga |
| $CH_2NHCOMe$ | H | Et | Et | O | H | Ga |
| $CH_2OCHF_2$ | H | Et | Et | O | H | Ga |
| $CF_3$ | H | Et | Et | O | H | Ga |
| $NO_2$ | H | Et | Et | O | H | Gb |
| OMe | H | Et | Et | O | H | Ga |
| OEt | H | Et | Et | O | H | Ga |
| OPr-n | H | Et | Et | O | H | Gb |
| SMe | H | Et | Et | O | H | Ga |

| $R_1$ | $R_2$ | $R_3$ | $R_4$ | X | M | Gn |
|---|---|---|---|---|---|---|
| SEt | H | Et | Et | O | H | Ga |
| $SO_2Me$ | H | Et | Et | O | H | Ga |
| $SO_2Et$ | H | Et | Et | O | H | Ga |
| COMe | H | Et | Et | O | H | Ga |
| COEt | H | Et | Et | O | H | Ga |
| $CO_2H$ | H | Et | Et | O | H | Gb |
| $CO_2Me$ | H | Et | Et | O | H | Ga |
| $CO_2Et$ | H | Et | Et | O | H | Ga |
| $CO_2Pr$-n | H | Et | Et | O | H | Gb |
| $CO_2Bu$-n | H | Et | Et | O | H | Gc |
| $CH_2Cl$ | H | Et | Et | O | H | Ga |
| $CH_2Br$ | H | Et | Et | O | H | Gb |
| $CH_2I$ | H | Et | Et | O | H | Gb |
| $CH_2CF_3$ | H | Et | Et | O | H | Ga |
| $CH_2CN$ | H | Et | Et | O | H | Ga |
| $CH_2OH$ | H | Et | Et | O | H | Gb |
| $CH_2OMe$ | H | Et | Et | O | H | Ga |
| $CH_2OEt$ | H | Et | Et | O | H | Ga |
| $CH_2OPr$-n | H | Et | Et | O | H | Gb |
| $CH_2SMe$ | H | Et | Et | O | H | Ga |
| $CH_2SEt$ | H | Et | Et | O | H | Ga |
| $CH_2SPr$-n | H | Et | Et | O | H | Gb |
| $CH_2SO_2Me$ | H | Et | Et | O | H | Ga |
| $CH_2SO_2Et$ | H | Et | Et | O | H | Ga |
| $CH_2COMe$ | H | Et | Et | O | H | Ga |
| $CH_2COEt$ | H | Et | Et | O | H | Ga |
| $CH_2COPr$-n | H | Et | Et | O | H | Gb |
| $CH_2CO_2H$ | H | Et | Et | O | H | Gb |
| $CH_2CO_2Me$ | H | Et | Et | O | H | Ga |
| $CH_2CO_2Et$ | H | Et | Et | O | H | Ga |
| $CH_2CO_2Pr$-n | H | Et | Et | O | H | Gb |
| $CH_2OCH_2CH=CH_2$ | H | Et | Et | O | H | Gb |
| $CH_2OCH_2C\equiv CH$ | H | Et | Et | O | H | Gb |
| $CH=CHCO_2Me$ | H | Et | Et | O | H | Ga |
| $CH=CHCO_2Et$ | H | Et | Et | O | H | Ga |
| $CH=CHCO_2Pr$-n | H | Et | Et | O | H | Gb |
| CH=CHCN | H | Et | Et | O | H | Ga |
| Ph | H | Et | Et | O | H | Ga |
| $CH_2Ph$ | H | Et | Et | O | H | Ga |
| OPh | H | Et | Et | O | H | Ga |

| $R_1$ | $R_2$ | $R_3$ | $R_4$ | X | M | Gn |
|---|---|---|---|---|---|---|
| SPh | H | Et | Et | O | H | Ga |
| $SO_2Ph$ | H | Et | Et | O | H | Ga |
| CONHMe | H | Et | Et | O | H | Ga |
| CONHEt | H | Et | Et | O | H | Ga |
| $CON(Me)_2$ | H | Et | Et | O | H | Ga |
| $CON(Et)_2$ | H | Et | Et | O | H | Ga |
| H | H | F | Me | O | H | Ga |
| H | H | Cl | Me | O | H | Ga |
| H | H | Br | Me | O | H | Ga |
| H | H | I | Me | O | H | Ga |
| H | H | $CH=CH_2$ | Me | O | H | Ga |
| H | H | $CH_2CH=CH_2$ | Me | O | H | Ga |
| H | H | $C\equiv CH$ | Me | O | H | Ga |
| H | H | $CH_2C\equiv CH$ | Me | O | H | Ga |
| H | H | $CH_2F$ | Me | O | H | Ga |
| H | H | $CHF_2$ | Me | O | H | Ga |
| H | H | CN | Me | O | H | Ga |
| H | H | $NH_2$ | Me | O | H | Ga |
| H | H | NHCOMe | Me | O | H | Ga |
| H | H | $CH_2NH_2$ | Me | O | H | Ga |
| H | H | $CH_2NHCOMe$ | Me | O | H | Ga |
| H | H | $CH_2OCHF_2$ | Me | O | H | Ga |
| H | H | $CF_3$ | Me | O | H | Ga |
| H | H | $NO_2$ | Me | O | H | Gb |
| H | H | OMe | Me | O | H | Ga |
| H | H | OEt | Me | O | H | Ga |
| H | H | OPr-n | Me | O | H | Gb |
| H | H | SMe | Me | O | H | Ga |
| H | H | SEt | Me | O | H | Ga |
| H | H | $SO_2Me$ | Me | O | H | Ga |
| H | H | $SO_2Et$ | Me | O | H | Ga |
| H | H | COMe | Me | O | H | Ga |
| H | H | COEt | Me | O | H | Ga |
| H | H | $CO_2H$ | Me | O | H | Gb |
| H | H | $CO_2Me$ | Me | O | H | Ga |
| H | H | $CO_2Et$ | Me | O | H | Ga |
| H | H | $CO_2Pr$-n | Me | O | H | Gb |
| H | H | $CO_2Bu$-n | Me | O | H | Gc |
| H | H | $CH_2Cl$ | Me | O | H | Ga |
| H | H | $CH_2Br$ | Me | O | H | Gb |

| $R_1$ | $R_2$ | $R_3$ | $R_4$ | X | M | Gn |
|---|---|---|---|---|---|---|
| H | H | $CH_2I$ | Me | O | H | Gb |
| H | H | $CH_2CF_3$ | Me | O | H | Ga |
| H | H | $CH_2CN$ | Me | O | H | Ga |
| H | H | $CH_2OH$ | Me | O | H | Gb |
| H | H | $CH_2OMe$ | Me | O | H | Ga |
| H | H | $CH_2OEt$ | Me | O | H | Ga |
| H | H | $CH_2OPr$-n | Me | O | H | Gb |
| H | H | $CH_2SMe$ | Me | O | H | Ga |
| H | H | $CH_2SEt$ | Me | O | H | Ga |
| H | H | $CH_2SPr$-n | Me | O | H | Gb |
| H | H | $CH_2SO_2Me$ | Me | O | H | Ga |
| H | H | $CH_2SO_2Et$ | Me | O | H | Ga |
| H | H | $CH_2COMe$ | Me | O | H | Ga |
| H | H | $CH_2COEt$ | Me | O | H | Ga |
| H | H | $CH_2COPr$-n | Me | O | H | Gb |
| H | H | $CH_2CO_2H$ | Me | O | H | Gb |
| H | H | $CH_2CO_2Me$ | Me | O | H | Ga |
| H | H | $CH_2CO_2Et$ | Me | O | H | Ga |
| H | H | $CH_2CO_2Pr$-n | Me | O | H | Gb |
| H | H | $CH_2OCH_2CH{=}CH_2$ | Me | O | H | Gb |
| H | H | $CH_2OCH_2C{\equiv}CH$ | Me | O | H | Gb |
| H | H | $CH{=}CHCO_2Me$ | Me | O | H | Ga |
| H | H | $CH{=}CHCO_2Et$ | Me | O | H | Ga |
| H | H | $CH{=}CHCO_2Pr$-n | Me | O | H | Gb |
| H | H | $CH{=}CHCN$ | Me | O | H | Ga |
| H | H | Ph | Me | O | H | Ga |
| H | H | $CH_2Ph$ | Me | O | H | Ga |
| H | H | OPh | Me | O | H | Ga |
| H | H | SPh | Me | O | H | Ga |
| H | H | $SO_2Ph$ | Me | O | H | Ga |
| H | H | CONHMe | Me | O | H | Ga |
| H | H | CONHEt | Me | O | H | Ga |
| H | H | $CON(Me)_2$ | Me | O | H | Ga |
| H | H | $CON(Et)_2$ | Me | O | H | Ga |
| Me | H | F | Me | O | H | Ga |
| Me | H | Cl | Me | O | H | Ga |
| Me | H | Br | Me | O | H | Ga |
| Me | H | I | Me | O | H | Ga |
| Me | H | $CH{=}CH_2$ | Me | O | H | Ga |
| Me | H | $CH_2CH{=}CH_2$ | Me | O | H | Ga |

| $R_1$ | $R_2$ | $R_3$ | $R_4$ | X | M | Gn |
|---|---|---|---|---|---|---|
| Me | H | C ≡CH | Me | O | H | Ga |
| Me | H | $CH_2C≡CH$ | Me | O | H | Ga |
| Me | H | $CH_2F$ | Me | O | H | Ga |
| Me | H | $CHF_2$ | Me | O | H | Ga |
| Me | H | CN | Me | O | H | Ga |
| Me | H | $NH_2$ | Me | O | H | Ga |
| Me | H | NHCOMe | Me | O | H | Ga |
| Me | H | $CH_2NH_2$ | Me | O | H | Ga |
| Me | H | $CH_2NHCOMe$ | Me | O | H | Ga |
| Me | H | $CH_2OCHF_2$ | Me | O | H | Ga |
| Me | H | $CF_3$ | Me | O | H | Ga |
| Me | H | $NO_2$ | Me | O | H | Gb |
| Me | H | OMe | Me | O | H | Ga |
| Me | H | OEt | Me | O | H | Ga |
| Me | H | OPr-n | Me | O | H | Gb |
| Me | H | SMe | Me | O | H | Ga |
| Me | H | SEt | Me | O | H | Ga |
| Me | H | $SO_2Me$ | Me | O | H | Ga |
| Me | H | $SO_2Et$ | Me | O | H | Ga |
| Me | H | COMe | Me | O | H | Ga |
| Me | H | COEt | Me | O | H | Ga |
| Me | H | $CO_2H$ | Me | O | H | Gb |
| Me | H | $CO_2Me$ | Me | O | H | Ga |
| Me | H | $CO_2Et$ | Me | O | H | Ga |
| Me | H | $CO_2Pr$-n | Me | O | H | Gb |
| Me | H | $CO_2Bu$-n | Me | O | H | Gc |
| Me | H | $CH_2Cl$ | Me | O | H | Ga |
| Me | H | $CH_2Br$ | Me | O | H | Gb |
| Me | H | $CH_2I$ | Me | O | H | Gb |
| Me | H | $CH_2CF_3$ | Me | O | H | Ga |
| Me | H | $CH_2CN$ | Me | O | H | Ga |
| Me | H | $CH_2OH$ | Me | O | H | Gb |
| Me | H | $CH_2OMe$ | Me | O | H | Ga |
| Me | H | $CH_2OEt$ | Me | O | H | Ga |
| Me | H | $CH_2OPr$-n | Me | O | H | Gb |
| Me | H | $CH_2SMe$ | Me | O | H | Ga |
| Me | H | $CH_2SEt$ | Me | O | H | Ga |
| Me | H | $CH_2SPr$-n | Me | O | H | Gb |
| Me | H | $CH_2SO_2Me$ | Me | O | H | Ga |
| Me | H | $CH_2SO_2Et$ | Me | O | H | Ga |

| $R_1$ | $R_2$ | $R_3$ | $R_4$ | X | M | Gn |
|---|---|---|---|---|---|---|
| Me | H | $CH_2COMe$ | Me | O | H | Ga |
| Me | H | $CH_2COEt$ | Me | O | H | Ga |
| Me | H | $CH_2COPr\text{-}n$ | Me | O | H | Gb |
| Me | H | $CH_2CO_2H$ | Me | O | H | Gb |
| Me | H | $CH_2CO_2Me$ | Me | O | H | Ga |
| Me | H | $CH_2CO_2Et$ | Me | O | H | Ga |
| Me | H | $CH_2CO_2Pr\text{-}n$ | Me | O | H | Gb |
| Me | H | $CH_2OCH_2CH=CH_2$ | Me | O | H | Gb |
| Me | H | $CH_2OCH_2C\equiv CH$ | Me | O | H | Gb |
| Me | H | $CH=CHCO_2Me$ | Me | O | H | Ga |
| Me | H | $CH=CHCO_2Et$ | Me | O | H | Ga |
| Me | H | $CH=CHCO_2Pr\text{-}n$ | Me | O | H | Gb |
| Me | H | $CH=CHCN$ | Me | O | H | Ga |
| Me | H | Ph | Me | O | H | Ga |
| Me | H | $CH_2Ph$ | Me | O | H | Ga |
| Me | H | OPh | Me | O | H | Ga |
| Me | H | SPh | Me | O | H | Ga |
| Me | H | $SO_2Ph$ | Me | O | H | Ga |
| Me | H | CONHMe | Me | O | H | Ga |
| Me | H | CONHEt | Me | O | H | Ga |
| Me | H | $CON(Me)_2$ | Me | O | H | Ga |
| Me | H | $CON(Et)_2$ | Me | O | H | Ga |
| Me | F | Me | H | O | H | Ga |
| Me | Cl | Me | H | O | H | Ga |
| Me | Br | Me | H | O | H | Ga |
| Me | I | Me | H | O | H | Ga |
| Me | $CH=CH_2$ | Me | H | O | H | Ga |
| Me | $CH_2CH=CH_2$ | Me | H | O | H | Ga |
| Me | $C\equiv CH$ | Me | H | O | H | Ga |
| Me | $CH_2C\equiv CH$ | Me | H | O | H | Ga |
| Me | $CH_2F$ | Me | H | O | H | Ga |
| Me | $CHF_2$ | Me | H | O | H | Ga |
| Me | CN | Me | H | O | H | Ga |
| Me | $NH_2$ | Me | H | O | H | Ga |
| Me | NHCOMe | Me | H | O | H | Ga |
| Me | $CH_2NH_2$ | Me | H | O | H | Ga |
| Me | $CH_2NHCOMe$ | Me | H | O | H | Ga |
| Me | $CH_2OCHF_2$ | Me | H | O | H | Ga |
| Me | $CF_3$ | Me | H | O | H | Ga |
| Me | $NO_2$ | Me | H | O | H | Gb |

| $R_1$ | $R_2$ | $R_3$ | $R_4$ | X | M | Gn |
|---|---|---|---|---|---|---|
| Me | OMe | Me | H | O | H | Ga |
| Me | OEt | Me | H | O | H | Ga |
| Me | OPr-n | Me | H | O | H | Gb |
| Me | SMe | Me | H | O | H | Ga |
| Me | SEt | Me | H | O | H | Ga |
| Me | $SO_2Me$ | Me | H | O | H | Ga |
| Me | $SO_2Et$ | Me | H | O | H | Ga |
| Me | COMe | Me | H | O | H | Ga |
| Me | COEt | Me | H | O | H | Ga |
| Me | $CO_2H$ | Me | H | O | H | Gb |
| Me | $CO_2Me$ | Me | H | O | H | Ga |
| Me | $CO_2Et$ | Me | H | O | H | Ga |
| Me | $CO_2Pr$-n | Me | H | O | H | Gb |
| Me | $CO_2Bu$-n | Me | H | O | H | Gc |
| Me | $CH_2Cl$ | Me | H | O | H | Ga |
| Me | $CH_2Br$ | Me | H | O | H | Gb |
| Me | $CH_2I$ | Me | H | O | H | Gb |
| Me | $CH_2CF_3$ | Me | H | O | H | Ga |
| Me | $CH_2CN$ | Me | H | O | H | Ga |
| Me | $CH_2OH$ | Me | H | O | H | Gb |
| Me | $CH_2OMe$ | Me | H | O | H | Ga |
| Me | $CH_2OEt$ | Me | H | O | H | Ga |
| Me | $CH_2OPr$-n | Me | H | O | H | Gb |
| Me | $CH_2SMe$ | Me | H | O | H | Ga |
| Me | $CH_2SEt$ | Me | H | O | H | Ga |
| Me | $CH_2SPr$-n | Me | H | O | H | Gb |
| Me | $CH_2SO_2Me$ | Me | H | O | H | Ga |
| Me | $CH_2SO_2Et$ | Me | H | O | H | Ga |
| Me | $CH_2COMe$ | Me | H | O | H | Ga |
| Me | $CH_2COEt$ | Me | H | O | H | Ga |
| Me | $CH_2COPr$-n | Me | H | O | H | Gb |
| Me | $CH_2CO_2H$ | Me | H | O | H | Gb |
| Me | $CH_2CO_2Me$ | Me | H | O | H | Ga |
| Me | $CH_2CO_2Et$ | Me | H | O | H | Ga |
| Me | $CH_2CO_2Pr$-n | Me | H | O | H | Gb |
| Me | $CH_2OCH_2CH=CH_2$ | Me | H | O | H | Gb |
| Me | $CH_2OCH_2C \equiv CH$ | Me | H | O | H | Gb |
| Me | $CH=CHCO_2Me$ | Me | H | O | H | Ga |
| Me | $CH=CHCO_2Et$ | Me | H | O | H | Ga |
| Me | $CH=CHCO_2Pr$-n | Me | H | O | H | Gb |

| $R_1$ | $R_2$ | $R_3$ | $R_4$ | X | M | Gn |
|---|---|---|---|---|---|---|
| Me | CH=CHCN | Me | H | O | H | Ga |
| Me | Ph | Me | H | O | H | Ga |
| Me | $CH_2Ph$ | Me | H | O | H | Ga |
| Me | OPh | Me | H | O | H | Ga |
| Me | SPh | Me | H | O | H | Ga |
| Me | $SO_2Ph$ | Me | H | O | H | Ga |
| Me | CONHMe | Me | H | O | H | Ga |
| Me | CONHEt | Me | H | O | H | Ga |
| Me | $CON(Me)_2$ | Me | H | O | H | Ga |
| Me | $CON(Et)_2$ | Me | H | O | H | Ga |

| $R_1$ | $R_2$ | $R_3$ | $R_4$ | X | M | Gn |
|---|---|---|---|---|---|---|
| H | H | F | H | O | H | Ga |
| H | H | F | H | S | H | G3 |
| H | H | Cl | H | O | H | Ga |
| H | H | Cl | H | S | H | G3 |
| H | H | Br | H | O | H | Ga |
| H | H | Br | H | S | H | G3 |
| H | H | I | H | O | H | Ga |
| H | H | I | H | S | H | G3 |
| H | H | $CH=CH_2$ | H | O | H | Ga |
| H | H | $CH=CH_2$ | H | S | H | G3 |
| H | H | $CH_2CH=CH_2$ | H | O | H | Ga |
| H | H | $CH_2CH=CH_2$ | H | S | H | G3 |
| H | H | $C\equiv CH$ | H | O | H | Ga |
| H | H | $C\equiv CH$ | H | S | H | G3 |
| H | H | $CH_2C\equiv CH$ | H | O | H | Ga |
| H | H | $CH_2C\equiv CH$ | H | S | H | G3 |
| H | H | $CH_2OSO_2Me$ | H | O | H | Ga |
| H | H | $CH_2OSO_2Me$ | H | S | H | G3 |
| H | H | $CHF_2$ | H | O | H | Ga |
| H | H | $CHF_2$ | H | S | H | G3 |
| H | H | CN | H | O | H | Ga |
| H | H | CN | H | S | H | G3 |
| H | H | $NH_2$ | H | O | H | Ga |
| H | H | $NH_2$ | H | S | H | G3 |
| H | H | NHCOMe | H | O | H | Ga |
| H | H | NHCOMe | H | S | H | G3 |
| H | H | $CH_2NH_2$ | H | O | H | Ga |
| H | H | $CH_2NH_2$ | H | S | H | G3 |
| H | H | $CH_2NHCOMe$ | H | O | H | Ga |
| H | H | $CH_2NHCOMe$ | H | S | H | G3 |
| H | H | $CH_2OCHF_2$ | H | O | H | Ga |
| H | H | $CH_2OCHF_2$ | H | S | H | G3 |
| H | H | $CF_3$ | H | O | H | Ga |
| H | H | $CF_3$ | H | S | H | G3 |
| H | H | $CF_3$ | H | O | Me | Gb |
| H | H | $CF_3$ | H | O | Et | Gb |
| H | H | $CF_3$ | H | O | $CH_2CH=CH_2$ | Gb |
| H | H | $CF_3$ | H | O | $CH_2C\equiv CH$ | Gb |
| H | H | $NO_2$ | H | O | H | Gb |
| H | H | $NO_2$ | H | S | H | G3 |
| H | H | $NO_2$ | H | O | Me | Gb |

| $R_1$ | $R_2$ | $R_3$ | $R_4$ | X | M | Gn |
|---|---|---|---|---|---|---|
| H | H | $NO_2$ | H | O | Et | Gb |
| H | H | $NO_2$ | H | O | $CH_2CH=CH_2$ | Gb |
| H | H | $NO_2$ | H | O | $CH_2C\equiv CH$ | Gb |
| H | H | OMe | H | O | H | Ga |
| H | H | OMe | H | S | H | G3 |
| H | H | OMe | H | O | Me | Gb |
| H | H | OMe | H | O | Et | Gb |
| H | H | OMe | H | O | $CH_2CH=CH_2$ | Gb |
| H | H | OMe | H | O | $CH_2C\equiv CH$ | Gb |
| H | H | OEt | H | O | H | Ga |
| H | H | OEt | H | S | H | G3 |
| H | H | OEt | H | O | Me | Gb |
| H | H | OEt | H | O | Et | Gb |
| H | H | OEt | H | O | $CH_2CH=CH_2$ | Gb |
| H | H | OEt | H | O | $CH_2C\equiv CH$ | Gb |
| H | H | OPr-n | H | O | H | Gb |
| H | H | OPr-n | H | S | H | G3 |
| H | H | OBu-n | H | O | H | Gc |
| H | H | OPen-n | H | O | H | Gc |
| H | H | SMe | H | O | H | Ga |
| H | H | SMe | H | S | H | G3 |
| H | H | SMe | H | O | Me | Gb |
| H | H | SMe | H | O | Et | Gb |
| H | H | SMe | H | O | $CH_2CH=CH_2$ | Gb |
| H | H | SMe | H | O | $CH_2C\equiv CH$ | Gb |
| H | H | SEt | H | O | H | Ga |
| H | H | SEt | H | S | H | G3 |
| H | H | SEt | H | O | Me | Gb |
| H | H | SEt | H | O | Et | Gb |
| H | H | SEt | H | O | $CH_2CH=CH_2$ | Gb |
| H | H | SEt | H | O | $CH_2C\equiv CH$ | Gb |
| H | H | SPr-n | H | O | H | Gb |
| H | H | SPr-n | H | S | H | G3 |
| H | H | SBu-n | H | O | H | Gc |
| H | H | SPen-n | H | O | H | Gc |
| H | H | $SO_2Me$ | H | O | H | Ga |
| H | H | $SO_2Me$ | H | S | H | G3 |
| H | H | $SO_2Me$ | H | O | Me | Gb |
| H | H | $SO_2Me$ | H | O | Et | Gb |
| H | H | $SO_2Me$ | H | O | $CH_2CH=CH_2$ | Gb |

| $R_1$ | $R_2$ | $R_3$ | $R_4$ | X | M | Gn |
|---|---|---|---|---|---|---|
| H | H | $SO_2Me$ | H | O | $CH_2C\equiv CH$ | Gb |
| H | H | $SO_2Et$ | H | O | H | Ga |
| H | H | $SO_2Et$ | H | S | H | G3 |
| H | H | $SO_2Et$ | H | O | Me | Gb |
| H | H | $SO_2Et$ | H | O | Et | Gb |
| H | H | $SO_2Et$ | H | O | $CH_2CH=CH_2$ | Gb |
| H | H | $SO_2Et$ | H | O | $CH_2C\equiv CH$ | Gb |
| H | H | $SO_2Pr$-n | H | O | H | Gb |
| H | H | $SO_2Pr$-n | H | S | H | G3 |
| H | H | COMe | H | O | H | Ga |
| H | H | COMe | H | S | H | G3 |
| H | H | COMe | H | O | Me | Gb |
| H | H | COMe | H | O | Et | Gb |
| H | H | COMe | H | O | $CH_2CH=CH_2$ | Gb |
| H | H | COMe | H | O | $CH_2C\equiv CH$ | Gb |
| H | H | COEt | H | O | H | Ga |
| H | H | COEt | H | S | H | G3 |
| H | H | COEt | H | O | Me | Gb |
| H | H | COEt | H | O | Et | Gb |
| H | H | COEt | H | O | $CH_2CH=CH_2$ | Gb |
| H | H | COEt | H | O | $CH_2C\equiv CH$ | Gb |
| H | H | COPr-n | H | O | H | Gb |
| H | H | COPr-n | H | S | H | G3 |
| H | H | $CO_2H$ | H | O | H | Gb |
| H | H | $CO_2H$ | H | S | H | G3 |
| H | H | $CO_2Me$ | H | O | H | Ga |
| H | H | $CO_2Me$ | H | S | H | G3 |
| H | H | $CO_2Me$ | H | O | Me | Gb |
| H | H | $CO_2Me$ | H | O | Et | Gb |
| H | H | $CO_2Me$ | H | O | $CH_2CH=CH_2$ | Gb |
| H | H | $CO_2Me$ | H | O | $CH_2C\equiv CH$ | Gb |
| H | H | $CO_2Et$ | H | O | H | Ga |
| H | H | $CO_2Et$ | H | S | H | G3 |
| H | H | $CO_2Et$ | H | O | Me | Gb |
| H | H | $CO_2Et$ | H | O | Et | Gb |
| H | H | $CO_2Et$ | H | O | $CH_2CH=CH_2$ | Gb |
| H | H | $CO_2Et$ | H | O | $CH_2C\equiv CH$ | Gb |
| H | H | $CO_2Pr$-n | H | O | H | Gb |
| H | H | $CO_2Pr$-n | H | S | H | G3 |
| H | H | $CO_2Bu$-n | H | O | H | Gc |

| $R_1$ | $R_2$ | $R_3$ | $R_4$ | X | M | Gn |
|---|---|---|---|---|---|---|
| H | H | $CO_2Pen$-n | H | O | H | Gc |
| H | H | $CH_2F$ | H | O | H | Ga |
| H | H | $CH_2F$ | H | S | H | G3 |
| H | H | $CH_2F$ | H | O | Me | Gb |
| H | H | $CH_2F$ | H | O | Et | Gb |
| H | H | $CH_2F$ | H | O | $CH_2CH=CH_2$ | Gb |
| H | H | $CH_2F$ | H | O | $CH_2C\equiv CH$ | Gb |
| H | H | $CH_2Cl$ | H | O | H | Ga |
| H | H | $CH_2Cl$ | H | S | H | G3 |
| H | H | $CH_2Cl$ | H | O | Me | Gb |
| H | H | $CH_2Cl$ | H | O | Et | Gb |
| H | H | $CH_2Cl$ | H | O | $CH_2CH=CH_2$ | Gb |
| H | H | $CH_2Cl$ | H | O | $CH_2C\equiv CH$ | Gb |
| H | H | $CH_2Br$ | H | O | H | Gb |
| H | H | $CH_2Br$ | H | S | H | G3 |
| H | H | $CH_2I$ | H | O | H | Gb |
| H | H | $CH_2I$ | H | S | H | G3 |
| H | H | $CH_2CF_3$ | H | O | H | Ga |
| H | H | $CH_2CF_3$ | H | S | H | G3 |
| H | H | $CH_2CF_3$ | H | O | Me | Gb |
| H | H | $CH_2CF_3$ | H | O | Et | Gb |
| H | H | $CH_2CF_3$ | H | O | $CH_2CH=CH_2$ | Gb |
| H | H | $CH_2CF_3$ | H | O | $CH_2C\equiv CH$ | Gb |
| H | H | $CH_2CN$ | H | O | H | Ga |
| H | H | $CH_2CN$ | H | S | H | G3 |
| H | H | $CH_2CN$ | H | O | Me | Gb |
| H | H | $CH_2CN$ | H | O | Et | Gb |
| H | H | $CH_2CN$ | H | O | $CH_2CH=CH_2$ | Gb |
| H | H | $CH_2CN$ | H | O | $CH_2C\equiv CH$ | Gb |
| H | H | $CH_2OH$ | H | O | H | Gb |
| H | H | $CH_2OMe$ | H | O | H | Ga |
| H | H | $CH_2OMe$ | H | S | H | G3 |
| H | H | $CH_2OMe$ | H | O | Me | Gb |
| H | H | $CH_2OMe$ | H | O | Et | Gb |
| H | H | $CH_2OMe$ | H | O | $CH_2CH=CH_2$ | Gb |
| H | H | $CH_2OMe$ | H | O | $CH_2C\equiv CH$ | Gb |
| H | H | $CH_2OEt$ | H | O | H | Ga |
| H | H | $CH_2OEt$ | H | S | H | G3 |
| H | H | $CH_2OEt$ | H | O | Me | Gb |
| H | H | $CH_2OEt$ | H | O | Et | Gb |

| $R_1$ | $R_2$ | $R_3$ | $R_4$ | X | M | Gn |
|---|---|---|---|---|---|---|
| H | H | $CH_2OEt$ | H | O | $CH_2CH=CH_2$ | Gb |
| H | H | $CH_2OEt$ | H | O | $CH_2C\equiv CH$ | Gb |
| H | H | $CH_2OPr$-n | H | O | H | Gb |
| H | H | $CH_2OPr$-n | H | S | H | G3 |
| H | H | $CH_2SMe$ | H | O | H | Ga |
| H | H | $CH_2SMe$ | H | S | H | G3 |
| H | H | $CH_2SMe$ | H | O | Me | Gb |
| H | H | $CH_2SMe$ | H | O | Et | Gb |
| H | H | $CH_2SMe$ | H | O | $CH_2CH=CH_2$ | Gb |
| H | H | $CH_2SMe$ | H | O | $CH_2C\equiv CH$ | Gb |
| H | H | $CH_2SEt$ | H | O | H | Ga |
| H | H | $CH_2SEt$ | H | S | H | G3 |
| H | H | $CH_2SEt$ | H | O | Me | Gb |
| H | H | $CH_2SEt$ | H | O | Et | Gb |
| H | H | $CH_2SEt$ | H | O | $CH_2CH=CH_2$ | Gb |
| H | H | $CH_2SEt$ | H | O | $CH_2C\equiv CH$ | Gb |
| H | H | $CH_2SPr$-n | H | O | H | Gb |
| H | H | $CH_2SPr$-n | H | S | H | G3 |
| H | H | $CH_2SO_2Me$ | H | O | H | Ga |
| H | H | $CH_2SO_2Et$ | H | O | H | Ga |
| H | H | $CH_2SO_2Me$ | H | S | H | G3 |
| H | H | $CH_2SO_2Et$ | H | S | H | G3 |
| H | H | $CH_2COMe$ | H | O | H | Ga |
| H | H | $CH_2COMe$ | H | S | H | G3 |
| H | H | $CH_2COMe$ | H | O | Me | Gb |
| H | H | $CH_2COMe$ | H | O | Et | Gb |
| H | H | $CH_2COMe$ | H | O | $CH_2CH=CH_2$ | Gb |
| H | H | $CH_2COMe$ | H | O | $CH_2C\equiv CH$ | Gb |
| H | H | $CH_2COEt$ | H | O | H | Ga |
| H | H | $CH_2COEt$ | H | S | H | G3 |
| H | H | $CH_2COEt$ | H | O | Me | Gb |
| H | H | $CH_2COEt$ | H | O | Et | Gb |
| H | H | $CH_2COEt$ | H | O | $CH_2CH=CH_2$ | Gb |
| H | H | $CH_2COEt$ | H | O | $CH_2C\equiv CH$ | Gb |
| H | H | $CH_2COPr$-n | H | O | H | Gb |
| H | H | $CH_2COPr$-n | H | S | H | G3 |
| H | H | $CH_2CO_2H$ | H | O | H | Gb |
| H | H | $CH_2CO_2H$ | H | S | H | G3 |
| H | H | $CH_2CO_2Me$ | H | O | H | Ga |
| H | H | $CH_2CO_2Me$ | H | S | H | G3 |

| $R_1$ | $R_2$ | $R_3$ | $R_4$ | X | M | Gn |
|---|---|---|---|---|---|---|
| H | H | $CH_2CO_2Me$ | H | O | Me | Gb |
| H | H | $CH_2CO_2Me$ | H | O | Et | Gb |
| H | H | $CH_2CO_2Me$ | H | O | $CH_2CH=CH_2$ | Gb |
| H | H | $CH_2CO_2Me$ | H | O | $CH_2C\equiv CH$ | Gb |
| H | H | $CH_2CO_2Et$ | H | O | H | Ga |
| H | H | $CH_2CO_2Et$ | H | S | H | G3 |
| H | H | $CH_2CO_2Et$ | H | O | Me | Gb |
| H | H | $CH_2CO_2Et$ | H | O | Et | Gb |
| H | H | $CH_2CO_2Et$ | H | O | $CH_2CH=CH_2$ | Gb |
| H | H | $CH_2CO_2Et$ | H | O | $CH_2C\equiv CH$ | Gb |
| H | H | $CH_2CO_2Pr\text{-}n$ | H | O | H | Gb |
| H | H | $CH_2CO_2Pr\text{-}n$ | H | S | H | G3 |
| H | H | $CH_2OCH_2CH=CH_2$ | H | O | H | Gb |
| H | H | $CH_2OCH_2CH=CH_2$ | H | O | Me | Gb |
| H | H | $CH_2OCH_2CH=CH_2$ | H | O | Et | Gb |
| H | H | $CH_2OCH_2CH=CH_2$ | H | O | $CH_2CH=CH_2$ | Gb |
| H | H | $CH_2OCH_2CH=CH_2$ | H | O | $CH_2C\equiv CH$ | Gb |
| H | H | $CH_2OCH_2C\equiv CH$ | H | O | H | Gb |
| H | H | $CH_2OCH_2C\equiv CH$ | H | O | Me | Gb |
| H | H | $CH_2OCH_2C\equiv CH$ | H | O | Et | Gb |
| H | H | $CH_2OCH_2C\equiv CH$ | H | O | $CH_2CH=CH_2$ | Gb |
| H | H | $CH_2OCH_2C\equiv CH$ | H | O | $CH_2C\equiv CH$ | Gb |
| H | H | $CH=CHCO_2Me$ | H | O | H | Ga |
| H | H | $CH=CHCO_2Me$ | H | S | H | G3 |
| H | H | $CH=CHCO_2Me$ | H | O | Me | Gb |
| H | H | $CH=CHCO_2Me$ | H | O | Et | Gb |
| H | H | $CH=CHCO_2Me$ | H | O | $CH_2CH=CH_2$ | Gb |
| H | H | $CH=CHCO_2Me$ | H | O | $CH_2C\equiv CH$ | Gb |
| H | H | $CH=CHCO_2Et$ | H | O | H | Ga |
| H | H | $CH=CHCO_2Et$ | H | S | H | G3 |
| H | H | $CH=CHCO_2Et$ | H | O | Me | Gb |
| H | H | $CH=CHCO_2Et$ | H | O | Et | Gb |
| H | H | $CH=CHCO_2Et$ | H | O | $CH_2CH=CH_2$ | Gb |
| H | H | $CH=CHCO_2Et$ | H | O | $CH_2C\equiv CH$ | Gb |
| H | H | $CH=CHCO_2Pr\text{-}n$ | H | O | H | Gb |
| H | H | $CH=CHCO_2Pr\text{-}n$ | H | S | H | G3 |
| H | H | $CH=CHCN$ | H | O | H | Ga |
| H | H | $CH=CHCN$ | H | S | H | G3 |
| H | H | Ph | H | O | H | Ga |
| H | H | Ph | H | S | H | G3 |

| $R_1$ | $R_2$ | $R_3$ | $R_4$ | X | M | Gn |
|---|---|---|---|---|---|---|
| H | H | Ph | H | O | Me | Gb |
| H | H | Ph | H | O | Et | Gb |
| H | H | Ph | H | O | $CH_2CH=CH_2$ | Gb |
| H | H | Ph | H | O | $CH_2C\equiv CH$ | Gb |
| H | H | Ph-2-F | H | O | H | Ga |
| H | H | Ph-2-F | H | S | H | G3 |
| H | H | Ph-3-F | H | O | H | Gb |
| H | H | Ph-4-F | H | O | H | Gb |
| H | H | Ph-2-Cl | H | O | H | Ga |
| H | H | Ph-2-Cl | H | S | H | G3 |
| H | H | Ph-3-Cl | H | O | H | Gb |
| H | H | Ph-4-Cl | H | O | H | Gb |
| H | H | Ph-2-Br | H | O | H | Ga |
| H | H | Ph-2-Br | H | S | H | G3 |
| H | H | Ph-3-Br | H | O | H | Gb |
| H | H | Ph-4-Br | H | O | H | Gb |
| H | H | Ph-2-$CF_3$ | H | O | H | Ga |
| H | H | Ph-2-$CF_3$ | H | S | H | G3 |
| H | H | Ph-3-$CF_3$ | H | O | H | Gb |
| H | H | Ph-4-$CF_3$ | H | O | H | Gb |
| H | H | Ph-2-Me | H | O | H | Ga |
| H | H | Ph-2-Me | H | S | H | G3 |
| H | H | Ph-3-Me | H | O | H | Gb |
| H | H | Ph-4-Me | H | O | H | Gb |
| H | H | Ph-2-Et | H | O | H | Ga |
| H | H | Ph-2-Et | H | S | H | G3 |
| H | H | Ph-2-OMe | H | O | H | Ga |
| H | H | Ph-2-OMe | H | S | H | G3 |
| H | H | Ph-3-OMe | H | O | H | Gb |
| H | H | Ph-4-OMe | H | O | H | Gb |
| H | H | Ph-2-OEt | H | O | H | Ga |
| H | H | Ph-2-OEt | H | S | H | G3 |
| H | H | Ph-2-$CO_2$Me | H | O | H | Ga |
| H | H | Ph-2-$CO_2$Me | H | S | H | G3 |
| H | H | Ph-2-$CO_2$Et | H | O | H | Ga |
| H | H | Ph-2-$CO_2$Et | H | S | H | G3 |
| H | H | Ph-3-$CO_2$Me | H | O | H | Gb |
| H | H | Ph-4-$CO_2$Me | H | O | H | Gb |
| H | H | $CH_2$Ph | H | O | H | Ga |
| H | H | $CH_2$Ph | H | S | H | G3 |

| $R_1$ | $R_2$ | $R_3$ | $R_4$ | X | M | Gn |
|---|---|---|---|---|---|---|
| H | H | $CH_2Ph$ | H | O | Me | Gb |
| H | H | $CH_2Ph$ | H | O | Et | Gb |
| H | H | $CH_2Ph$ | H | O | $CH_2CH{=}CH_2$ | Gb |
| H | H | $CH_2Ph$ | H | O | $CH_2C{\equiv}CH$ | Gb |
| H | H | $CH_2Ph$-2-F | H | O | H | Ga |
| H | H | $CH_2Ph$-2-F | H | S | H | G3 |
| H | H | $CH_2Ph$-3-F | H | O | H | Gb |
| H | H | $CH_2Ph$-4-F | H | O | H | Gb |
| H | H | $CH_2Ph$-2-Cl | H | O | H | Ga |
| H | H | $CH_2Ph$-2-Cl | H | S | H | G3 |
| H | H | $CH_2Ph$-3-Cl | H | O | H | Gb |
| H | H | $CH_2Ph$-4-Cl | H | O | H | Gb |
| H | H | $CH_2Ph$-2-Br | H | O | H | Ga |
| H | H | $CH_2Ph$-2-Br | H | S | H | G3 |
| H | H | $CH_2Ph$-3-Br | H | O | H | Gb |
| H | H | $CH_2Ph$-4-Br | H | O | H | Gb |
| H | H | $CH_2Ph$-2-$CF_3$ | H | O | H | Ga |
| H | H | $CH_2Ph$-2-$CF_3$ | H | S | H | G3 |
| H | H | $CH_2Ph$-3-$CF_3$ | H | O | H | Gb |
| H | H | $CH_2Ph$-4-$CF_3$ | H | O | H | Gb |
| H | H | $CH_2Ph$-2-Me | H | O | H | Ga |
| H | H | $CH_2Ph$-2-Me | H | S | H | G3 |
| H | H | $CH_2Ph$-3-Me | H | O | H | Gb |
| H | H | $CH_2Ph$-4-Me | H | O | H | Gb |
| H | H | $CH_2Ph$-2-Et | H | O | H | Ga |
| H | H | $CH_2Ph$-2-Et | H | S | H | G3 |
| H | H | $CH_2Ph$-2-OMe | H | O | H | Ga |
| H | H | $CH_2Ph$-2-OMe | H | S | H | G3 |
| H | H | $CH_2Ph$-3-OMe | H | O | H | Gb |
| H | H | $CH_2Ph$-4-OMe | H | O | H | Gb |
| H | H | $CH_2Ph$-2-OEt | H | O | H | Ga |
| H | H | $CH_2Ph$-2-OEt | H | S | H | G3 |
| H | H | $CH_2Ph$-2-$CO_2Me$ | H | O | H | Ga |
| H | H | $CH_2Ph$-2-$CO_2Me$ | H | S | H | G3 |
| H | H | $CH_2Ph$-2-$CO_2Et$ | H | O | H | Ga |
| H | H | $CH_2Ph$-2-$CO_2Et$ | H | S | H | G3 |
| H | H | $CH_2Ph$-3-$CO_2Me$ | H | O | H | Gb |
| H | H | $CH_2Ph$-4-$CO_2Me$ | H | O | H | Gb |
| H | H | OPh | H | O | H | Ga |
| H | H | OPh | H | S | H | G3 |

| $R_1$ | $R_2$ | $R_3$ | $R_4$ | X | M | Gn |
|---|---|---|---|---|---|---|
| H | H | OPh | H | O | Me | Gb |
| H | H | OPh | H | O | Et | Gb |
| H | H | OPh | H | O | $CH_2CH=CH_2$ | Gb |
| H | H | OPh | H | O | $CH_2C\equiv CH$ | Gb |
| H | H | OPh-2-Cl | H | O | H | Ga |
| H | H | OPh-2-Cl | H | S | H | G3 |
| H | H | OPh-3-Cl | H | O | H | Gb |
| H | H | OPh-4-Cl | H | O | H | Gb |
| H | H | OPh-2-F | H | O | H | Ga |
| H | H | OPh-2-F | H | S | H | G3 |
| H | H | OPh-3-F | H | O | H | Gb |
| H | H | OPh-4-F | H | O | H | Gb |
| H | H | $OPh-2-CF_3$ | H | O | H | Ga |
| H | H | $OPh-2-CF_3$ | H | S | H | G3 |
| H | H | $OPh-3-CF_3$ | H | O | H | Gb |
| H | H | $OPh-4-CF_3$ | H | O | H | Gb |
| H | H | OPh-2-Me | H | O | H | Ga |
| H | H | OPh-2-Me | H | S | H | G3 |
| H | H | OPh-3-Me | H | O | H | Gb |
| H | H | OPh-4-Me | H | O | H | Gb |
| H | H | OPh-2-Et | H | O | H | Ga |
| H | H | OPh-2-Et | H | S | H | G3 |
| H | H | OPh-2-OMe | H | O | H | Ga |
| H | H | OPh-2-OMe | H | S | H | G3 |
| H | H | OPh-3-OMe | H | O | H | Gb |
| H | H | OPh-4-OMe | H | O | H | Gb |
| H | H | OPh-2-OEt | H | O | H | Ga |
| H | H | OPh-2-OEt | H | S | H | G3 |
| H | H | $OPh-2-CO_2Me$ | H | O | H | Ga |
| H | H | $OPh-2-CO_2Me$ | H | S | H | G3 |
| H | H | $OPh-3-CO_2Me$ | H | O | H | Gb |
| H | H | $OPh-4-CO_2Me$ | H | O | H | Gb |
| H | H | $OPh-2-CO_2Et$ | H | O | H | Ga |
| H | H | $OPh-2-CO_2Et$ | H | S | H | G3 |
| H | H | SPh | H | O | H | Ga |
| H | H | SPh | H | S | H | G3 |
| H | H | SPh | H | O | Me | Gb |
| H | H | SPh | H | O | Et | Gb |
| H | H | SPh | H | O | $CH_2CH=CH_2$ | Gb |
| H | H | SPh | H | O | $CH_2C\equiv CH$ | Gb |
| H | H | $CON(Et)_2$ | H | S | H | G3 |
| H | H | $CON(Et)_2$ | H | O | Me | Gb |
| H | H | $CON(Et)_2$ | H | O | Et | Gb |

| $R_1$ | $R_2$ | $R_3$ | $R_4$ | X | M | Gn |
|---|---|---|---|---|---|---|
| H | H | SPh-2-Cl | H | O | H | Ga |
| H | H | SPh-2-Cl | H | S | H | G3 |
| H | H | SPh-3-Cl | H | O | H | Gb |
| H | H | SPh-4-Cl | H | O | H | Gb |
| H | H | SPh-2-F | H | O | H | Ga |
| H | H | SPh-2-F | H | S | H | G3 |
| H | H | SPh-3-F | H | O | H | Gb |
| H | H | SPh-4-F | H | O | H | Gb |
| H | H | SPh-2-$CF_3$ | H | O | H | Ga |
| H | H | SPh-2-$CF_3$ | H | S | H | G3 |
| H | H | SPh-3-$CF_3$ | H | O | H | Gb |
| H | H | SPh-4-$CF_3$ | H | O | H | Gb |
| H | H | SPh-2-Me | H | O | H | Ga |
| H | H | SPh-2-Me | H | S | H | G3 |
| H | H | SPh-3-Me | H | O | H | Gb |
| H | H | SPh-4-Me | H | O | H | Gb |
| H | H | SPh-2-Et | H | O | H | Ga |
| H | H | SPh-2-Et | H | S | H | G3 |
| H | H | SPh-2-OMe | H | O | H | Ga |
| H | H | SPh-2-OMe | H | S | H | G3 |
| H | H | SPh-3-OMe | H | O | H | Gb |
| H | H | SPh-4-OMe | H | O | H | Gb |
| H | H | SPh-2-OEt | H | O | H | Ga |
| H | H | SPh-2-OEt | H | S | H | G3 |
| H | H | SPh-2-$CO_2$Me | H | O | H | Ga |
| H | H | SPh-2-$CO_2$Me | H | S | H | G3 |
| H | H | SPh-3-$CO_2$Me | H | O | H | Gb |
| H | H | SPh-4-$CO_2$Me | H | O | H | Gb |
| H | H | SPh-2-$CO_2$Et | H | O | H | Ga |
| H | H | SPh-2-$CO_2$Et | H | S | H | G3 |
| H | H | $SO_2$Ph | H | O | H | Ga |
| H | H | $SO_2$Ph | H | S | H | G3 |
| H | H | $SO_2$Ph | H | O | Me | Gb |
| H | H | $SO_2$Ph | H | O | Et | Gb |
| H | H | $SO_2$Ph | H | O | $CH_2CH=CH_2$ | Gb |
| H | H | $SO_2$Ph | H | O | $CH_2C \equiv CH$ | Gb |
| H | H | $SO_2$Ph-2-Cl | H | O | H | Ga |
| H | H | $SO_2$Ph-2-Cl | H | S | H | G3 |
| H | H | $SO_2$Ph-3-Cl | H | O | H | Gb |
| H | H | CON$(Et)_2$ | H | O | $CH_2CH=CH_2$ | Gb |
| H | H | CON$(Et)_2$ | H | O | $CH_2C \equiv CH$ | Gb |
| H | H | $SO_2$Ph-4-Cl | H | O | H | Gb |

| $R_1$ | $R_2$ | $R_3$ | $R_4$ | X | M | Gn |
|---|---|---|---|---|---|---|
| H | H | $SO_2Ph$-2-F | H | O | H | Ga |
| H | H | $SO_2Ph$-2-F | H | S | H | G3 |
| H | H | $SO_2Ph$-3-F | H | O | H | Gb |
| H | H | $SO_2Ph$-4-F | H | O | H | Gb |
| H | H | $SO_2Ph$-2-$CF_3$ | H | O | H | Ga |
| H | H | $SO_2Ph$-2-$CF_3$ | H | S | H | G3 |
| H | H | $SO_2Ph$-3-$CF_3$ | H | O | H | Gb |
| H | H | $SO_2Ph$-4-$CF_3$ | H | O | H | Gb |
| H | H | $SO_2Ph$-2-Me | H | O | H | Ga |
| H | H | $SO_2Ph$-2-Me | H | S | H | G3 |
| H | H | $SO_2Ph$-3-Me | H | O | H | Gb |
| H | H | $SO_2Ph$-4-Me | H | O | H | Gb |
| H | H | $SO_2Ph$-2-Et | H | O | H | Ga |
| H | H | $SO_2Ph$-2-Et | H | S | H | G3 |
| H | H | $SO_2Ph$-2-OMe | H | O | H | Ga |
| H | H | $SO_2Ph$-2-OMe | H | S | H | G3 |
| H | H | $SO_2Ph$-3-OMe | H | O | H | Gb |
| H | H | $SO_2Ph$-4-OMe | H | O | H | Gb |
| H | H | $SO_2Ph$-2-OEt | H | O | H | Ga |
| H | H | $SO_2Ph$-2-OEt | H | S | H | G3 |
| H | H | $SO_2Ph$-2-$CO_2Me$ | H | O | H | Ga |
| H | H | $SO_2Ph$-2-$CO_2Me$ | H | S | H | G3 |
| H | H | $SO_2Ph$-3-$CO_2Me$ | H | O | H | Gb |
| H | H | $SO_2Ph$-4-$CO_2Me$ | H | O | H | Gb |
| H | H | $SO_2Ph$-2-$CO_2Et$ | H | O | H | Ga |
| H | H | $SO_2Ph$-2-$CO_2Et$ | H | S | H | G3 |
| H | H | CONHMe | H | O | H | Ga |
| H | H | CONHMe | H | S | H | G3 |
| H | H | CONHEt | H | O | H | Ga |
| H | H | CONHEt | H | S | H | G3 |
| H | H | CONHPr-n | H | O | H | Gb |
| H | H | CONHPr-n | H | S | H | G3 |
| H | H | CONHPr-i | H | O | H | Gb |
| H | H | CONHPr-i | H | S | H | G3 |
| H | H | CONHBu-n | H | O | H | Gb |
| H | H | CONHBu-sec | H | O | H | Gb |
| H | H | CONHBu-t | H | O | H | Gb |
| H | H | $CON(Me)_2$ | H | O | H | Ga |
| H | H | $CON(Me)_2$ | H | S | H | G3 |
| H | H | $CON(Pr\text{-}n)_2$ | H | O | H | Gb |
| H | H | $CON(Pr\text{-}n)_2$ | H | S | H | G3 |
| H | H | $CON(Et)_2$ | H | O | H | Ga |

| $R_3$ | $R_2$ | $R_1$ | $R_4$ | X | M | Gn |
|---|---|---|---|---|---|---|
| H | H | F | H | O | H | Ga |
| H | H | F | H | S | H | G3 |
| H | H | Cl | H | O | H | Ga |
| H | H | Cl | H | S | H | G3 |
| H | H | Br | H | O | H | Ga |
| H | H | Br | H | S | H | G3 |
| H | H | I | H | O | H | Ga |
| H | H | I | H | S | H | G3 |
| H | H | $CH=CH_2$ | H | O | H | Ga |
| H | H | $CH=CH_2$ | H | S | H | G3 |
| H | H | $CH_2CH=CH_2$ | H | O | H | Ga |
| H | H | $CH_2CH=CH_2$ | H | S | H | G3 |
| H | H | $C\equiv CH$ | H | O | H | Ga |
| H | H | $C\equiv CH$ | H | S | H | G3 |
| H | H | $CH_2C\equiv CH$ | H | O | H | Ga |
| H | H | $CH_2C\equiv CH$ | H | S | H | G3 |
| H | H | $CH_2OSO_2Me$ | H | O | H | Ga |
| H | H | $CH_2OSO_2Me$ | H | S | H | G3 |
| H | H | $CHF_2$ | H | O | H | Ga |
| H | H | $CHF_2$ | H | S | H | G3 |
| H | H | CN | H | O | H | Ga |
| H | H | CN | H | S | H | G3 |
| H | H | $NH_2$ | H | O | H | Ga |
| H | H | $NH_2$ | H | S | H | G3 |
| H | H | NHCOMe | H | O | H | Ga |
| H | H | NHCOMe | H | S | H | G3 |
| H | H | $CH_2NH_2$ | H | O | H | Ga |
| H | H | $CH_2NH_2$ | H | S | H | G3 |
| H | H | $CH_2NHCOMe$ | H | O | H | Ga |
| H | H | $CH_2NHCOMe$ | H | S | H | G3 |
| H | H | $CH_2OCHF_2$ | H | O | H | Ga |
| H | H | $CH_2OCHF_2$ | H | S | H | G3 |
| H | H | $CF_3$ | H | O | H | Ga |
| H | H | $CF_3$ | H | S | H | G3 |
| H | H | $CF_3$ | H | O | Me | Gb |
| H | H | $CF_3$ | H | O | Et | Gb |
| H | H | $CF_3$ | H | O | $CH_2CH=CH_2$ | Gb |
| H | H | $CF_3$ | H | O | $CH_2C\equiv CH$ | Gb |
| H | H | $NO_2$ | H | O | H | Gb |
| H | H | $NO_2$ | H | S | H | G3 |
| H | H | $NO_2$ | H | O | Me | Gb |

| $R_3$ | $R_2$ | $R_1$ | $R_4$ | X | M | Gn |
|---|---|---|---|---|---|---|
| H | H | $NO_2$ | H | O | Et | Gb |
| H | H | $NO_2$ | H | O | $CH_2CH=CH_2$ | Gb |
| H | H | $NO_2$ | H | O | $CH_2C\equiv CH$ | Gb |
| H | H | OMe | H | O | H | Ga |
| H | H | OMe | H | S | H | G3 |
| H | H | OMe | H | O | Me | Gb |
| H | H | OMe | H | O | Et | Gb |
| H | H | OMe | H | O | $CH_2CH=CH_2$ | Gb |
| H | H | OMe | H | O | $CH_2C\equiv CH$ | Gb |
| H | H | OEt | H | O | H | Ga |
| H | H | OEt | H | S | H | G3 |
| H | H | OEt | H | O | Me | Gb |
| H | H | OEt | H | O | Et | Gb |
| H | H | OEt | H | O | $CH_2CH=CH_2$ | Gb |
| H | H | OEt | H | O | $CH_2C\equiv CH$ | Gb |
| H | H | OPr-n | H | O | H | Gb |
| H | H | OPr-n | H | S | H | G3 |
| H | H | OBu-n | H | O | H | Gc |
| H | H | OPen-n | H | O | H | Gc |
| H | H | SMe | H | O | H | Ga |
| H | H | SMe | H | S | H | G3 |
| H | H | SMe | H | O | Me | Gb |
| H | H | SMe | H | O | Et | Gb |
| H | H | SMe | H | O | $CH_2CH=CH_2$ | Gb |
| H | H | SMe | H | O | $CH_2C\equiv CH$ | Gb |
| H | H | SEt | H | O | H | Ga |
| H | H | SEt | H | S | H | G3 |
| H | H | SEt | H | O | Me | Gb |
| H | H | SEt | H | O | Et | Gb |
| H | H | SEt | H | O | $CH_2CH=CH_2$ | Gb |
| H | H | SEt | H | O | $CH_2C\equiv CH$ | Gb |
| H | H | SPr-n | H | O | H | Gb |
| H | H | SPr-n | H | S | H | G3 |
| H | H | SBu-n | H | O | H | Gc |
| H | H | SPen-n | H | O | H | Gc |
| H | H | $SO_2Me$ | H | O | H | Ga |
| H | H | $SO_2Me$ | H | S | H | G3 |
| H | H | $SO_2Me$ | H | O | Me | Gb |
| H | H | $SO_2Me$ | H | O | Et | Gb |
| H | H | $SO_2Me$ | H | O | $CH_2CH=CH_2$ | Gb |

| $R_3$ | $R_2$ | $R_1$ | $R_4$ | X | M | Gn |
|---|---|---|---|---|---|---|
| H | H | $SO_2Me$ | H | O | $CH_2C\equiv CH$ | Gb |
| H | H | $SO_2Et$ | H | O | H | Ga |
| H | H | $SO_2Et$ | H | S | H | G3 |
| H | H | $SO_2Et$ | H | O | Me | Gb |
| H | H | $SO_2Et$ | H | O | Et | Gb |
| H | H | $SO_2Et$ | H | O | $CH_2CH=CH_2$ | Gb |
| H | H | $SO_2Et$ | H | O | $CH_2C\equiv CH$ | Gb |
| H | H | $SO_2Pr$-n | H | O | H | Gb |
| H | H | $SO_2Pr$-n | H | S | H | G3 |
| H | H | COMe | H | O | H | Ga |
| H | H | COMe | H | S | H | G3 |
| H | H | COMe | H | O | Me | Gb |
| H | H | COMe | H | O | Et | Gb |
| H | H | COMe | H | O | $CH_2CH=CH_2$ | Gb |
| H | H | COMe | H | O | $CH_2C\equiv CH$ | Gb |
| H | H | COEt | H | O | H | Ga |
| H | H | COEt | H | S | H | G3 |
| H | H | COEt | H | O | Me | Gb |
| H | H | COEt | H | O | Et | Gb |
| H | H | COEt | H | O | $CH_2CH=CH_2$ | Gb |
| H | H | COEt | H | O | $CH_2C\equiv CH$ | Gb |
| H | H | COPr-n | H | O | H | Gb |
| H | H | COPr-n | H | S | H | G3 |
| H | H | $CO_2H$ | H | O | H | Gb |
| H | H | $CO_2H$ | H | S | H | G3 |
| H | H | $CO_2Me$ | H | O | H | Ga |
| H | H | $CO_2Me$ | H | S | H | G3 |
| H | H | $CO_2Me$ | H | O | Me | Gb |
| H | H | $CO_2Me$ | H | O | Et | Gb |
| H | H | $CO_2Me$ | H | O | $CH_2CH=CH_2$ | Gb |
| H | H | $CO_2Me$ | H | O | $CH_2C\equiv CH$ | Gb |
| H | H | $CO_2Et$ | H | O | H | Ga |
| H | H | $CO_2Et$ | H | S | H | G3 |
| H | H | $CO_2Et$ | H | O | Me | Gb |
| H | H | $CO_2Et$ | H | O | Et | Gb |
| H | H | $CO_2Et$ | H | O | $CH_2CH=CH_2$ | Gb |
| H | H | $CO_2Et$ | H | O | $CH_2C\equiv CH$ | Gb |
| H | H | $CO_2Pr$-n | H | O | H | Gb |
| H | H | $CO_2Pr$-n | H | S | H | G3 |
| H | H | $CO_2Bu$-n | H | O | H | Gc |

| $R_3$ | $R_2$ | $R_1$ | $R_4$ | X | M | Gn |
|---|---|---|---|---|---|---|
| H | H | $CO_2Pen$-n | H | O | H | Gc |
| H | H | $CH_2F$ | H | O | H | Ga |
| H | H | $CH_2F$ | H | S | H | G3 |
| H | H | $CH_2F$ | H | O | Me | Gb |
| H | H | $CH_2F$ | H | O | Et | Gb |
| H | H | $CH_2F$ | H | O | $CH_2CH=CH_2$ | Gb |
| H | H | $CH_2F$ | H | O | $CH_2C\equiv CH$ | Gb |
| H | H | $CH_2Cl$ | H | O | H | Ga |
| H | H | $CH_2Cl$ | H | S | H | G3 |
| H | H | $CH_2Cl$ | H | O | Me | Gb |
| H | H | $CH_2Cl$ | H | O | Et | Gb |
| H | H | $CH_2Cl$ | H | O | $CH_2CH=CH_2$ | Gb |
| H | H | $CH_2Cl$ | H | O | $CH_2C\equiv CH$ | Gb |
| H | H | $CH_2Br$ | H | O | H | Gb |
| H | H | $CH_2Br$ | H | S | H | G3 |
| H | H | $CH_2I$ | H | O | H | Gb |
| H | H | $CH_2I$ | H | S | H | G3 |
| H | H | $CH_2CF_3$ | H | O | H | Ga |
| H | H | $CH_2CF_3$ | H | S | H | G3 |
| H | H | $CH_2CF_3$ | H | O | Me | Gb |
| H | H | $CH_2CF_3$ | H | O | Et | Gb |
| H | H | $CH_2CF_3$ | H | O | $CH_2CH=CH_2$ | Gb |
| H | H | $CH_2CF_3$ | H | O | $CH_2C\equiv CH$ | Gb |
| H | H | $CH_2CN$ | H | O | H | Ga |
| H | H | $CH_2CN$ | H | S | H | G3 |
| H | H | $CH_2CN$ | H | O | Me | Gb |
| H | H | $CH_2CN$ | H | O | Et | Gb |
| H | H | $CH_2CN$ | H | O | $CH_2CH=CH_2$ | Gb |
| H | H | $CH_2CN$ | H | O | $CH_2C\equiv CH$ | Gb |
| H | H | $CH_2OH$ | H | O | H | Gb |
| H | H | $CH_2OMe$ | H | O | H | Ga |
| H | H | $CH_2OMe$ | H | S | H | G3 |
| H | H | $CH_2OMe$ | H | O | Me | Gb |
| H | H | $CH_2OMe$ | H | O | Et | Gb |
| H | H | $CH_2OMe$ | H | O | $CH_2CH=CH_2$ | Gb |
| H | H | $CH_2OMe$ | H | O | $CH_2C\equiv CH$ | Gb |
| H | H | $CH_2OEt$ | H | O | H | Ga |
| H | H | $CH_2OEt$ | H | S | H | G3 |
| H | H | $CH_2OEt$ | H | O | Me | Gb |
| H | H | $CH_2OEt$ | H | O | Et | Gb |

| $R_3$ | $R_2$ | $R_1$ | $R_4$ | X | M | Gn |
|---|---|---|---|---|---|---|
| H | H | $CH_2OEt$ | H | O | $CH_2CH=CH_2$ | Gb |
| H | H | $CH_2OEt$ | H | O | $CH_2C\equiv CH$ | Gb |
| H | H | $CH_2OPr$-n | H | O | H | Gb |
| H | H | $CH_2OPr$-n | H | S | H | G3 |
| H | H | $CH_2SMe$ | H | O | H | Ga |
| H | H | $CH_2SMe$ | H | S | H | G3 |
| H | H | $CH_2SMe$ | H | O | Me | Gb |
| H | H | $CH_2SMe$ | H | O | Et | Gb |
| H | H | $CH_2SMe$ | H | O | $CH_2CH=CH_2$ | Gb |
| H | H | $CH_2SMe$ | H | O | $CH_2C\equiv CH$ | Gb |
| H | H | $CH_2SEt$ | H | O | H | Ga |
| H | H | $CH_2SEt$ | H | S | H | G3 |
| H | H | $CH_2SEt$ | H | O | Me | Gb |
| H | H | $CH_2SEt$ | H | O | Et | Gb |
| H | H | $CH_2SEt$ | H | O | $CH_2CH=CH_2$ | Gb |
| H | H | $CH_2SEt$ | H | O | $CH_2C\equiv CH$ | Gb |
| H | H | $CH_2SPr$-n | H | O | H | Gb |
| H | H | $CH_2SPr$-n | H | S | H | G3 |
| H | H | $CH_2SO_2Me$ | H | O | H | Ga |
| H | H | $CH_2SO_2Et$ | H | O | H | Ga |
| H | H | $CH_2SO_2Me$ | H | S | H | G3 |
| H | H | $CH_2SO_2Et$ | H | S | H | G3 |
| H | H | $CH_2COMe$ | H | O | H | Ga |
| H | H | $CH_2COMe$ | H | S | H | G3 |
| H | H | $CH_2COMe$ | H | O | Me | Gb |
| H | H | $CH_2COMe$ | H | O | Et | Gb |
| H | H | $CH_2COMe$ | H | O | $CH_2CH=CH_2$ | Gb |
| H | H | $CH_2COMe$ | H | O | $CH_2C\equiv CH$ | Gb |
| H | H | $CH_2COEt$ | H | O | H | Ga |
| H | H | $CH_2COEt$ | H | S | H | G3 |
| H | H | $CH_2COEt$ | H | O | Me | Gb |
| H | H | $CH_2COEt$ | H | O | Et | Gb |
| H | H | $CH_2COEt$ | H | O | $CH_2CH=CH_2$ | Gb |
| H | H | $CH_2COEt$ | H | O | $CH_2C\equiv CH$ | Gb |
| H | H | $CH_2COPr$-n | H | O | H | Gb |
| H | H | $CH_2COPr$-n | H | S | H | G3 |
| H | H | $CH_2CO_2H$ | H | O | H | Gb |
| H | H | $CH_2CO_2H$ | H | S | H | G3 |
| H | H | $CH_2CO_2Me$ | H | O | H | Ga |
| H | H | $CH_2CO_2Me$ | H | S | H | G3 |

| $R_3$ | $R_2$ | $R_1$ | $R_4$ | X | M | Gn |
|---|---|---|---|---|---|---|
| H | H | $CH_2CO_2Me$ | H | O | Me | Gb |
| H | H | $CH_2CO_2Me$ | H | O | Et | Gb |
| H | H | $CH_2CO_2Me$ | H | O | $CH_2CH=CH_2$ | Gb |
| H | H | $CH_2CO_2Me$ | H | O | $CH_2C\equiv CH$ | Gb |
| H | H | $CH_2CO_2Et$ | H | O | H | Ga |
| H | H | $CH_2CO_2Et$ | H | S | H | G3 |
| H | H | $CH_2CO_2Et$ | H | O | Me | Gb |
| H | H | $CH_2CO_2Et$ | H | O | Et | Gb |
| H | H | $CH_2CO_2Et$ | H | O | $CH_2CH=CH_2$ | Gb |
| H | H | $CH_2CO_2Et$ | H | O | $CH_2C\equiv CH$ | Gb |
| H | H | $CH_2CO_2Pr$-n | H | O | H | Gb |
| H | H | $CH_2CO_2Pr$-n | H | S | H | G3 |
| H | H | $CH_2OCH_2CH=CH_2$ | H | O | H | Gb |
| H | H | $CH_2OCH_2CH=CH_2$ | H | O | Me | Gb |
| H | H | $CH_2OCH_2CH=CH_2$ | H | O | Et | Gb |
| H | H | $CH_2OCH_2CH=CH_2$ | H | O | $CH_2CH=CH_2$ | Gb |
| H | H | $CH_2OCH_2CH=CH_2$ | H | O | $CH_2C\equiv CH$ | Gb |
| H | H | $CH_2OCH_2C\equiv CH$ | H | O | H | Gb |
| H | H | $CH_2OCH_2C\equiv CH$ | H | O | Me | Gb |
| H | H | $CH_2OCH_2C\equiv CH$ | H | O | Et | Gb |
| H | H | $CH_2OCH_2C\equiv CH$ | H | O | $CH_2CH=CH_2$ | Gb |
| H | H | $CH_2OCH_2C\equiv CH$ | H | O | $CH_2C\equiv CH$ | Gb |
| H | H | $CH=CHCO_2Me$ | H | O | H | Ga |
| H | H | $CH=CHCO_2Me$ | H | S | H | G3 |
| H | H | $CH=CHCO_2Me$ | H | O | Me | Gb |
| H | H | $CH=CHCO_2Me$ | H | O | Et | Gb |
| H | H | $CH=CHCO_2Me$ | H | O | $CH_2CH=CH_2$ | Gb |
| H | H | $CH=CHCO_2Me$ | H | O | $CH_2C\equiv CH$ | Gb |
| H | H | $CH=CHCO_2Et$ | H | O | H | Ga |
| H | H | $CH=CHCO_2Et$ | H | S | H | G3 |
| H | H | $CH=CHCO_2Et$ | H | O | Me | Gb |
| H | H | $CH=CHCO_2Et$ | H | O | Et | Gb |
| H | H | $CH=CHCO_2Et$ | H | O | $CH_2CH=CH_2$ | Gb |
| H | H | $CH=CHCO_2Et$ | H | O | $CH_2C\equiv CH$ | Gb |
| H | H | $CH=CHCO_2Pr$-n | H | O | H | Gb |
| H | H | $CH=CHCO_2Pr$-n | H | S | H | G3 |
| H | H | CH=CHCN | H | O | H | Ga |
| H | H | CH=CHCN | H | S | H | G3 |
| H | H | Ph | H | O | H | Ga |
| H | H | Ph | H | S | H | G3 |

| $R_3$ | $R_2$ | $R_1$ | $R_4$ | X | M | Gn |
|---|---|---|---|---|---|---|
| H | H | Ph | H | O | Me | Gb |
| H | H | Ph | H | O | Et | Gb |
| H | H | Ph | H | O | $CH_2CH{=}CH_2$ | Gb |
| H | H | Ph | H | O | $CH_2C{\equiv}CH$ | Gb |
| H | H | Ph-2-F | H | O | H | Ga |
| H | H | Ph-2-F | H | S | H | G3 |
| H | H | Ph-3-F | H | O | H | Gb |
| H | H | Ph-4-F | H | O | H | Gb |
| H | H | Ph-2-Cl | H | O | H | Ga |
| H | H | Ph-2-Cl | H | S | H | G3 |
| H | H | Ph-3-Cl | H | O | H | Gb |
| H | H | Ph-4-Cl | H | O | H | Gb |
| H | H | Ph-2-Br | H | O | H | Ga |
| H | H | Ph-2-Br | H | S | H | G3 |
| H | H | Ph-3-Br | H | O | H | Gb |
| H | H | Ph-4-Br | H | O | H | Gb |
| H | H | Ph-2-$CF_3$ | H | O | H | Ga |
| H | H | Ph-2-$CF_3$ | H | S | H | G3 |
| H | H | Ph-3-$CF_3$ | H | O | H | Gb |
| H | H | Ph-4-$CF_3$ | H | O | H | Gb |
| H | H | Ph-2-Me | H | O | H | Ga |
| H | H | Ph-2-Me | H | S | H | G3 |
| H | H | Ph-3-Me | H | O | H | Gb |
| H | H | Ph-4-Me | H | O | H | Gb |
| H | H | Ph-2-Et | H | O | H | Ga |
| H | H | Ph-2-Et | H | S | H | G3 |
| H | H | Ph-2-OMe | H | O | H | Ga |
| H | H | Ph-2-OMe | H | S | H | G3 |
| H | H | Ph-3-OMe | H | O | H | Gb |
| H | H | Ph-4-OMe | H | O | H | Gb |
| H | H | Ph-2-OEt | H | O | H | Ga |
| H | H | Ph-2-OEt | H | S | H | G3 |
| H | H | Ph-2-$CO_2$Me | H | O | H | Ga |
| H | H | Ph-2-$CO_2$Me | H | S | H | G3 |
| H | H | Ph-2-$CO_2$Et | H | O | H | Ga |
| H | H | Ph-2-$CO_2$Et | H | S | H | G3 |
| H | H | Ph-3-$CO_2$Me | H | O | H | Gb |
| H | H | Ph-4-$CO_2$Me | H | O | H | Gb |
| H | H | $CH_2$Ph | H | O | H | Ga |
| H | H | $CH_2$Ph | H | S | H | G3 |

| $R_3$ | $R_2$ | $R_1$ | $R_4$ | X | M | Gn |
|---|---|---|---|---|---|---|
| H | H | $CH_2Ph$ | H | O | Me | Gb |
| H | H | $CH_2Ph$ | H | O | Et | Gb |
| H | H | $CH_2Ph$ | H | O | $CH_2CH{=}CH_2$ | Gb |
| H | H | $CH_2Ph$ | H | O | $CH_2C{\equiv}CH$ | Gb |
| H | H | $CH_2Ph$-2-F | H | O | H | Ga |
| H | H | $CH_2Ph$-2-F | H | S | H | G3 |
| H | H | $CH_2Ph$-3-F | H | O | H | Gb |
| H | H | $CH_2Ph$-4-F | H | O | H | Gb |
| H | H | $CH_2Ph$-2-Cl | H | O | H | Ga |
| H | H | $CH_2Ph$-2-Cl | H | S | H | G3 |
| H | H | $CH_2Ph$-3-Cl | H | O | H | Gb |
| H | H | $CH_2Ph$-4-Cl | H | O | H | Gb |
| H | H | $CH_2Ph$-2-Br | H | O | H | Ga |
| H | H | $CH_2Ph$-2-Br | H | S | H | G3 |
| H | H | $CH_2Ph$-3-Br | H | O | H | Gb |
| H | H | $CH_2Ph$-4-Br | H | O | H | Gb |
| H | H | $CH_2Ph$-2-$CF_3$ | H | O | H | Ga |
| H | H | $CH_2Ph$-2-$CF_3$ | H | S | H | G3 |
| H | H | $CH_2Ph$-3-$CF_3$ | H | O | H | Gb |
| H | H | $CH_2Ph$-4-$CF_3$ | H | O | H | Gb |
| H | H | $CH_2Ph$-2-Me | H | O | H | Ga |
| H | H | $CH_2Ph$-2-Me | H | S | H | G3 |
| H | H | $CH_2Ph$-3-Me | H | O | H | Gb |
| H | H | $CH_2Ph$-4-Me | H | O | H | Gb |
| H | H | $CH_2Ph$-2-Et | H | O | H | Ga |
| H | H | $CH_2Ph$-2-Et | H | S | H | G3 |
| H | H | $CH_2Ph$-2-OMe | H | O | H | Ga |
| H | H | $CH_2Ph$-2-OMe | H | S | H | G3 |
| H | H | $CH_2Ph$-3-OMe | H | O | H | Gb |
| H | H | $CH_2Ph$-4-OMe | H | O | H | Gb |
| H | H | $CH_2Ph$-2-OEt | H | O | H | Ga |
| H | H | $CH_2Ph$-2-OEt | H | S | H | G3 |
| H | H | $CH_2Ph$-2-$CO_2Me$ | H | O | H | Ga |
| H | H | $CH_2Ph$-2-$CO_2Me$ | H | S | H | G3 |
| H | H | $CH_2Ph$-2-$CO_2Et$ | H | O | H | Ga |
| H | H | $CH_2Ph$-2-$CO_2Et$ | H | S | H | G3 |
| H | H | $CH_2Ph$-3-$CO_2Me$ | H | O | H | Gb |
| H | H | $CH_2Ph$-4-$CO_2Me$ | H | O | H | Gb |
| H | H | OPh | H | O | H | Ga |
| H | H | OPh | H | S | H | G3 |

| $R_3$ | $R_2$ | $R_1$ | $R_4$ | X | M | Gn |
|---|---|---|---|---|---|---|
| H | H | OPh | H | O | Me | Gb |
| H | H | OPh | H | O | Et | Gb |
| H | H | OPh | H | O | $CH_2CH=CH_2$ | Gb |
| H | H | OPh | H | O | $CH_2C\equiv CH$ | Gb |
| H | H | OPh-2-Cl | H | O | H | Ga |
| H | H | OPh-2-Cl | H | S | H | G3 |
| H | H | OPh-3-Cl | H | O | H | Gb |
| H | H | OPh-4-Cl | H | O | H | Gb |
| H | H | OPh-2-F | H | O | H | Ga |
| H | H | OPh-2-F | H | S | H | G3 |
| H | H | OPh-3-F | H | O | H | Gb |
| H | H | OPh-4-F | H | O | H | Gb |
| H | H | OPh-2-$CF_3$ | H | O | H | Ga |
| H | H | OPh-2-$CF_3$ | H | S | H | G3 |
| H | H | OPh-3-$CF_3$ | H | O | H | Gb |
| H | H | OPh-4-$CF_3$ | H | O | H | Gb |
| H | H | OPh-2-Me | H | O | H | Ga |
| H | H | OPh-2-Me | H | S | H | G3 |
| H | H | OPh-3-Me | H | O | H | Gb |
| H | H | OPh-4-Me | H | O | H | Gb |
| H | H | OPh-2-Et | H | O | H | Ga |
| H | H | OPh-2-Et | H | S | H | G3 |
| H | H | OPh-2-OMe | H | O | H | Ga |
| H | H | OPh-2-OMe | H | S | H | G3 |
| H | H | OPh-3-OMe | H | O | H | Gb |
| H | H | OPh-4-OMe | H | O | H | Gb |
| H | H | OPh-2-OEt | H | O | H | Ga |
| H | H | OPh-2-OEt | H | S | H | G3 |
| H | H | OPh-2-$CO_2$Me | H | O | H | Ga |
| H | H | OPh-2-$CO_2$Me | H | S | H | G3 |
| H | H | OPh-3-$CO_2$Me | H | O | H | Gb |
| H | H | OPh-4-$CO_2$Me | H | O | H | Gb |
| H | H | OPh-2-$CO_2$Et | H | O | H | Ga |
| H | H | OPh-2-$CO_2$Et | H | S | H | G3 |
| H | H | SPh | H | O | H | Ga |
| H | H | SPh | H | S | H | G3 |
| H | H | SPh | H | O | Me | Gb |
| H | H | SPh | H | O | Et | Gb |
| H | H | SPh | H | O | $CH_2CH=CH_2$ | Gb |
| H | H | SPh | H | O | $CH_2C\equiv CH$ | Gb |
| H | H | CON(Et)$_2$ | H | S | H | G3 |
| H | H | CON(Et)$_2$ | H | O | Me | Gb |
| H | H | CON(Et)$_2$ | H | O | Et | Gb |

| $R_3$ | $R_2$ | $R_1$ | $R_4$ | X | M | Gn |
|---|---|---|---|---|---|---|
| H | H | SPh-2-Cl | H | O | H | Ga |
| H | H | SPh-2-Cl | H | S | H | G3 |
| H | H | SPh-3-Cl | H | O | H | Gb |
| H | H | SPh-4-Cl | H | O | H | Gb |
| H | H | SPh-2-F | H | O | H | Ga |
| H | H | SPh-2-F | H | S | H | G3 |
| H | H | SPh-3-F | H | O | H | Gb |
| H | H | SPh-4-F | H | O | H | Gb |
| H | H | SPh-2-$CF_3$ | H | O | H | Ga |
| H | H | SPh-2-$CF_3$ | H | S | H | G3 |
| H | H | SPh-3-$CF_3$ | H | O | H | Gb |
| H | H | SPh-4-$CF_3$ | H | O | H | Gb |
| H | H | SPh-2-Me | H | O | H | Ga |
| H | H | SPh-2-Me | H | S | H | G3 |
| H | H | SPh-3-Me | H | O | H | Gb |
| H | H | SPh-4-Me | H | O | H | Gb |
| H | H | SPh-2-Et | H | O | H | Ga |
| H | H | SPh-2-Et | H | S | H | G3 |
| H | H | SPh-2-OMe | H | O | H | Ga |
| H | H | SPh-2-OMe | H | S | H | G3 |
| H | H | SPh-3-OMe | H | O | H | Gb |
| H | H | SPh-4-OMe | H | O | H | Gb |
| H | H | SPh-2-OEt | H | O | H | Ga |
| H | H | SPh-2-OEt | H | S | H | G3 |
| H | H | SPh-2-$CO_2$Me | H | O | H | Ga |
| H | H | SPh-2-$CO_2$Me | H | S | H | G3 |
| H | H | SPh-3-$CO_2$Me | H | O | H | Gb |
| H | H | SPh-4-$CO_2$Me | H | O | H | Gb |
| H | H | SPh-2-$CO_2$Et | H | O | H | Ga |
| H | H | SPh-2-$CO_2$Et | H | S | H | G3 |
| H | H | $SO_2$Ph | H | O | H | Ga |
| H | H | $SO_2$Ph | H | S | H | G3 |
| H | H | $SO_2$Ph | H | O | Me | Gb |
| H | H | $SO_2$Ph | H | O | Et | Gb |
| H | H | $SO_2$Ph | H | O | $CH_2CH=CH_2$ | Gb |
| H | H | $SO_2$Ph | H | O | $CH_2C\equiv CH$ | Gb |
| H | H | $SO_2$Ph-2-Cl | H | O | H | Ga |
| H | H | $SO_2$Ph-2-Cl | H | S | H | G3 |
| H | H | $SO_2$Ph-3-Cl | H | O | H | Gb |
| H | H | CON(Et)$_2$ | H | O | $CH_2CH=CH_2$ | Gb |
| H | H | CON(Et)$_2$ | H | O | $CH_2C\equiv CH$ | Gb |
| H | H | $SO_2$Ph-4-Cl | H | O | H | Gb |

| $R_3$ | $R_2$ | $R_1$ | $R_4$ | X | M | Gn |
|---|---|---|---|---|---|---|
| H | H | $SO_2Ph$-2-F | H | O | H | Ga |
| H | H | $SO_2Ph$-2-F | H | S | H | G3 |
| H | H | $SO_2Ph$-3-F | H | O | H | Gb |
| H | H | $SO_2Ph$-4-F | H | O | H | Gb |
| H | H | $SO_2Ph$-2-$CF_3$ | H | O | H | Ga |
| H | H | $SO_2Ph$-2-$CF_3$ | H | S | H | G3 |
| H | H | $SO_2Ph$-3-$CF_3$ | H | O | H | Gb |
| H | H | $SO_2Ph$-4-$CF_3$ | H | O | H | Gb |
| H | H | $SO_2Ph$-2-Me | H | O | H | Ga |
| H | H | $SO_2Ph$-2-Me | H | S | H | G3 |
| H | H | $SO_2Ph$-3-Me | H | O | H | Gb |
| H | H | $SO_2Ph$-4-Me | H | O | H | Gb |
| H | H | $SO_2Ph$-2-Et | H | O | H | Ga |
| H | H | $SO_2Ph$-2-Et | H | S | H | G3 |
| H | H | $SO_2Ph$-2-OMe | H | O | H | Ga |
| H | H | $SO_2Ph$-2-OMe | H | S | H | G3 |
| H | H | $SO_2Ph$-3-OMe | H | O | H | Gb |
| H | H | $SO_2Ph$-4-OMe | H | O | H | Gb |
| H | H | $SO_2Ph$-2-OEt | H | O | H | Ga |
| H | H | $SO_2Ph$-2-OEt | H | S | H | G3 |
| H | H | $SO_2Ph$-2-$CO_2Me$ | H | O | H | Ga |
| H | H | $SO_2Ph$-2-$CO_2Me$ | H | S | H | G3 |
| H | H | $SO_2Ph$-3-$CO_2Me$ | H | O | H | Gb |
| H | H | $SO_2Ph$-4-$CO_2Me$ | H | O | H | Gb |
| H | H | $SO_2Ph$-2-$CO_2Et$ | H | O | H | Ga |
| H | H | $SO_2Ph$-2-$CO_2Et$ | H | S | H | G3 |
| H | H | CONHMe | H | O | H | Ga |
| H | H | CONHMe | H | S | H | G3 |
| H | H | CONHEt | H | O | H | Ga |
| H | H | CONHEt | H | S | H | G3 |
| H | H | CONHPr-n | H | O | H | Gb |
| H | H | CONHPr-n | H | S | H | G3 |
| H | H | CONHPr-i | H | O | H | Gb |
| H | H | CONHPr-i | H | S | H | G3 |
| H | H | CONHBu-n | H | O | H | Gb |
| H | H | CONHBu-sec | H | O | H | Gb |
| H | H | CONHBu-t | H | O | H | Gb |
| H | H | CON(Me)$_2$ | H | O | H | Ga |
| H | H | CON(Me)$_2$ | H | S | H | G3 |
| H | H | CON(Pr-n)$_2$ | H | O | H | Gb |
| H | H | CON(Pr-n)$_2$ | H | S | H | G3 |
| H | H | CON(Et)$_2$ | H | O | H | Ga |

| $R_4$ | $R_3$ | $R_2$ | $R_1$ | X | M | Gn |
|---|---|---|---|---|---|---|
| H | H | F | H | O | H | Ga |
| H | H | F | H | S | H | G3 |
| H | H | Cl | H | O | H | Ga |
| H | H | Cl | H | S | H | G3 |
| H | H | Br | H | O | H | Ga |
| H | H | Br | H | S | H | G3 |
| H | H | I | H | O | H | Ga |
| H | H | I | H | S | H | G3 |
| H | H | $CH=CH_2$ | H | O | H | Ga |
| H | H | $CH=CH_2$ | H | S | H | G3 |
| H | H | $CH_2CH=CH_2$ | H | O | H | Ga |
| H | H | $CH_2CH=CH_2$ | H | S | H | G3 |
| H | H | $C\equiv CH$ | H | O | H | Ga |
| H | H | $C\equiv CH$ | H | S | H | G3 |
| H | H | $CH_2C\equiv CH$ | H | O | H | Ga |
| H | H | $CH_2C\equiv CH$ | H | S | H | G3 |
| H | H | $CH_2OSO_2Me$ | H | O | H | Ga |
| H | H | $CH_2OSO_2Me$ | H | S | H | G3 |
| H | H | $CHF_2$ | H | O | H | Ga |
| H | H | $CHF_2$ | H | S | H | G3 |
| H | H | CN | H | O | H | Ga |
| H | H | CN | H | S | H | G3 |
| H | H | $NH_2$ | H | O | H | Ga |
| H | H | $NH_2$ | H | S | H | G3 |
| H | H | NHCOMe | H | O | H | Ga |
| H | H | NHCOMe | H | S | H | G3 |
| H | H | $CH_2NH_2$ | H | O | H | Ga |
| H | H | $CH_2NH_2$ | H | S | H | G3 |
| H | H | $CH_2NHCOMe$ | H | O | H | Ga |
| H | H | $CH_2NHCOMe$ | H | S | H | G3 |
| H | H | $CH_2OCHF_2$ | H | O | H | Ga |
| H | H | $CH_2OCHF_2$ | H | S | H | G3 |
| H | H | $CF_3$ | H | O | H | Ga |
| H | H | $CF_3$ | H | S | H | G3 |
| H | H | $CF_3$ | H | O | Me | Gb |
| H | H | $CF_3$ | H | O | Et | Gb |
| H | H | $CF_3$ | H | O | $CH_2CH=CH_2$ | Gb |
| H | H | $CF_3$ | H | O | $CH_2C\equiv CH$ | Gb |
| H | H | $NO_2$ | H | O | H | Gb |
| H | H | $NO_2$ | H | S | H | G3 |
| H | H | $NO_2$ | H | O | Me | Gb |

| $R_4$ | $R_3$ | $R_2$ | $R_1$ | X | M | Gn |
|---|---|---|---|---|---|---|
| H | H | $NO_2$ | H | O | Et | Gb |
| H | H | $NO_2$ | H | O | $CH_2CH=CH_2$ | Gb |
| H | H | $NO_2$ | H | O | $CH_2C\equiv CH$ | Gb |
| H | H | OMe | H | O | H | Ga |
| H | H | OMe | H | S | H | G3 |
| H | H | OMe | H | O | Me | Gb |
| H | H | OMe | H | O | Et | Gb |
| H | H | OMe | H | O | $CH_2CH=CH_2$ | Gb |
| H | H | OMe | H | O | $CH_2C\equiv CH$ | Gb |
| H | H | OEt | H | O | H | Ga |
| H | H | OEt | H | S | H | G3 |
| H | H | OEt | H | O | Me | Gb |
| H | H | OEt | H | O | Et | Gb |
| H | H | OEt | H | O | $CH_2CH=CH_2$ | Gb |
| H | H | OEt | H | O | $CH_2C\equiv CH$ | Gb |
| H | H | OPr-n | H | O | H | Gb |
| H | H | OPr-n | H | S | H | G3 |
| H | H | OBu-n | H | O | H | Gc |
| H | H | OPen-n | H | O | H | Gc |
| H | H | SMe | H | O | H | Ga |
| H | H | SMe | H | S | H | G3 |
| H | H | SMe | H | O | Me | Gb |
| H | H | SMe | H | O | Et | Gb |
| H | H | SMe | H | O | $CH_2CH=CH_2$ | Gb |
| H | H | SMe | H | O | $CH_2C\equiv CH$ | Gb |
| H | H | SEt | H | O | H | Ga |
| H | H | SEt | H | S | H | G3 |
| H | H | SEt | H | O | Me | Gb |
| H | H | SEt | H | O | Et | Gb |
| H | H | SEt | H | O | $CH_2CH=CH_2$ | Gb |
| H | H | SEt | H | O | $CH_2C\equiv CH$ | Gb |
| H | H | SPr-n | H | O | H | Gb |
| H | H | SPr-n | H | S | H | G3 |
| H | H | SBu-n | H | O | H | Gc |
| H | H | SPen-n | H | O | H | Gc |
| H | H | $SO_2Me$ | H | O | H | Ga |
| H | H | $SO_2Me$ | H | S | H | G3 |
| H | H | $SO_2Me$ | H | O | Me | Gb |
| H | H | $SO_2Me$ | H | O | Et | Gb |
| H | H | $SO_2Me$ | H | O | $CH_2CH=CH_2$ | Gb |

| $R_4$ | $R_3$ | $R_2$ | $R_1$ | X | M | Gn |
|---|---|---|---|---|---|---|
| H | H | $SO_2Me$ | H | O | $CH_2C\equiv CH$ | Gb |
| H | H | $SO_2Et$ | H | O | H | Ga |
| H | H | $SO_2Et$ | H | S | H | G3 |
| H | H | $SO_2Et$ | H | O | Me | Gb |
| H | H | $SO_2Et$ | H | O | Et | Gb |
| H | H | $SO_2Et$ | H | O | $CH_2CH=CH_2$ | Gb |
| H | H | $SO_2Et$ | H | O | $CH_2C\equiv CH$ | Gb |
| H | H | $SO_2Pr$-n | H | O | H | Gb |
| H | H | $SO_2Pr$-n | H | S | H | G3 |
| H | H | COMe | H | O | H | Ga |
| H | H | COMe | H | S | H | G3 |
| H | H | COMe | H | O | Me | Gb |
| H | H | COMe | H | O | Et | Gb |
| H | H | COMe | H | O | $CH_2CH=CH_2$ | Gb |
| H | H | COMe | H | O | $CH_2C\equiv CH$ | Gb |
| H | H | COEt | H | O | H | Ga |
| H | H | COEt | H | S | H | G3 |
| H | H | COEt | H | O | Me | Gb |
| H | H | COEt | H | O | Et | Gb |
| H | H | COEt | H | O | $CH_2CH=CH_2$ | Gb |
| H | H | COEt | H | O | $CH_2C\equiv CH$ | Gb |
| H | H | COPr-n | H | O | H | Gb |
| H | H | COPr-n | H | S | H | G3 |
| H | H | $CO_2H$ | H | O | H | Gb |
| H | H | $CO_2H$ | H | S | H | G3 |
| H | H | $CO_2Me$ | H | O | H | Ga |
| H | H | $CO_2Me$ | H | S | H | G3 |
| H | H | $CO_2Me$ | H | O | Me | Gb |
| H | H | $CO_2Me$ | H | O | Et | Gb |
| H | H | $CO_2Me$ | H | O | $CH_2CH=CH_2$ | Gb |
| H | H | $CO_2Me$ | H | O | $CH_2C\equiv CH$ | Gb |
| H | H | $CO_2Et$ | H | O | H | Ga |
| H | H | $CO_2Et$ | H | S | H | G3 |
| H | H | $CO_2Et$ | H | O | Me | Gb |
| H | H | $CO_2Et$ | H | O | Et | Gb |
| H | H | $CO_2Et$ | H | O | $CH_2CH=CH_2$ | Gb |
| H | H | $CO_2Et$ | H | O | $CH_2C\equiv CH$ | Gb |
| H | H | $CO_2Pr$-n | H | O | H | Gb |
| H | H | $CO_2Pr$-n | H | S | H | G3 |
| H | H | $CO_2Bu$-n | H | O | H | Gc |

| $R_4$ | $R_3$ | $R_2$ | $R_1$ | X | M | Gn |
|---|---|---|---|---|---|---|
| H | H | $CO_2Pen$-n | H | O | H | Gc |
| H | H | $CH_2F$ | H | O | H | Ga |
| H | H | $CH_2F$ | H | S | H | G3 |
| H | H | $CH_2F$ | H | O | Me | Gb |
| H | H | $CH_2F$ | H | O | Et | Gb |
| H | H | $CH_2F$ | H | O | $CH_2CH{=}CH_2$ | Gb |
| H | H | $CH_2F$ | H | O | $CH_2C{\equiv}CH$ | Gb |
| H | H | $CH_2Cl$ | H | O | H | Ga |
| H | H | $CH_2Cl$ | H | S | H | G3 |
| H | H | $CH_2Cl$ | H | O | Me | Gb |
| H | H | $CH_2Cl$ | H | O | Et | Gb |
| H | H | $CH_2Cl$ | H | O | $CH_2CH{=}CH_2$ | Gb |
| H | H | $CH_2Cl$ | H | O | $CH_2C{\equiv}CH$ | Gb |
| H | H | $CH_2Br$ | H | O | H | Gb |
| H | H | $CH_2Br$ | H | S | H | G3 |
| H | H | $CH_2I$ | H | O | H | Gb |
| H | H | $CH_2I$ | H | S | H | G3 |
| H | H | $CH_2CF_3$ | H | O | H | Ga |
| H | H | $CH_2CF_3$ | H | S | H | G3 |
| H | H | $CH_2CF_3$ | H | O | Me | Gb |
| H | H | $CH_2CF_3$ | H | O | Et | Gb |
| H | H | $CH_2CF_3$ | H | O | $CH_2CH{=}CH_2$ | Gb |
| H | H | $CH_2CF_3$ | H | O | $CH_2C{\equiv}CH$ | Gb |
| H | H | $CH_2CN$ | H | O | H | Ga |
| H | H | $CH_2CN$ | H | S | H | G3 |
| H | H | $CH_2CN$ | H | O | Me | Gb |
| H | H | $CH_2CN$ | H | O | Et | Gb |
| H | H | $CH_2CN$ | H | O | $CH_2CH{=}CH_2$ | Gb |
| H | H | $CH_2CN$ | H | O | $CH_2C{\equiv}CH$ | Gb |
| H | H | $CH_2OH$ | H | O | H | Gb |
| H | H | $CH_2OMe$ | H | O | H | Ga |
| H | H | $CH_2OMe$ | H | S | H | G3 |
| H | H | $CH_2OMe$ | H | O | Me | Gb |
| H | H | $CH_2OMe$ | H | O | Et | Gb |
| H | H | $CH_2OMe$ | H | O | $CH_2CH{=}CH_2$ | Gb |
| H | H | $CH_2OMe$ | H | O | $CH_2C{\equiv}CH$ | Gb |
| H | H | $CH_2OEt$ | H | O | H | Ga |
| H | H | $CH_2OEt$ | H | S | H | G3 |
| H | H | $CH_2OEt$ | H | O | Me | Gb |
| H | H | $CH_2OEt$ | H | O | Et | Gb |

| $R_4$ | $R_3$ | $R_2$ | $R_1$ | X | M | Gn |
|---|---|---|---|---|---|---|
| H | H | $CH_2OEt$ | H | O | $CH_2CH=CH_2$ | Gb |
| H | H | $CH_2OEt$ | H | O | $CH_2C\equiv CH$ | Gb |
| H | H | $CH_2OPr$-n | H | O | H | Gb |
| H | H | $CH_2OPr$-n | H | S | H | G3 |
| H | H | $CH_2SMe$ | H | O | H | Ga |
| H | H | $CH_2SMe$ | H | S | H | G3 |
| H | H | $CH_2SMe$ | H | O | Me | Gb |
| H | H | $CH_2SMe$ | H | O | Et | Gb |
| H | H | $CH_2SMe$ | H | O | $CH_2CH=CH_2$ | Gb |
| H | H | $CH_2SMe$ | H | O | $CH_2C\equiv CH$ | Gb |
| H | H | $CH_2SEt$ | H | O | H | Ga |
| H | H | $CH_2SEt$ | H | S | H | G3 |
| H | H | $CH_2SEt$ | H | O | Me | Gb |
| H | H | $CH_2SEt$ | H | O | Et | Gb |
| H | H | $CH_2SEt$ | H | O | $CH_2CH=CH_2$ | Gb |
| H | H | $CH_2SEt$ | H | O | $CH_2C\equiv CH$ | Gb |
| H | H | $CH_2SPr$-n | H | O | H | Gb |
| H | H | $CH_2SPr$-n | H | S | H | G3 |
| H | H | $CH_2SO_2Me$ | H | O | H | Ga |
| H | H | $CH_2SO_2Et$ | H | O | H | Ga |
| H | H | $CH_2SO_2Me$ | H | S | H | G3 |
| H | H | $CH_2SO_2Et$ | H | S | H | G3 |
| H | H | $CH_2COMe$ | H | O | H | Ga |
| H | H | $CH_2COMe$ | H | S | H | G3 |
| H | H | $CH_2COMe$ | H | O | Me | Gb |
| H | H | $CH_2COMe$ | H | O | Et | Gb |
| H | H | $CH_2COMe$ | H | O | $CH_2CH=CH_2$ | Gb |
| H | H | $CH_2COMe$ | H | O | $CH_2C\equiv CH$ | Gb |
| H | H | $CH_2COEt$ | H | O | H | Ga |
| H | H | $CH_2COEt$ | H | S | H | G3 |
| H | H | $CH_2COEt$ | H | O | Me | Gb |
| H | H | $CH_2COEt$ | H | O | Et | Gb |
| H | H | $CH_2COEt$ | H | O | $CH_2CH=CH_2$ | Gb |
| H | H | $CH_2COEt$ | H | O | $CH_2C\equiv CH$ | Gb |
| H | H | $CH_2COPr$-n | H | O | H | Gb |
| H | H | $CH_2COPr$-n | H | S | H | G3 |
| H | H | $CH_2CO_2H$ | H | O | H | Gb |
| H | H | $CH_2CO_2H$ | H | S | H | G3 |
| H | H | $CH_2CO_2Me$ | H | O | H | Ga |
| H | H | $CH_2CO_2Me$ | H | S | H | G3 |

| $R_4$ | $R_3$ | $R_2$ | $R_1$ | X | M | Gn |
|---|---|---|---|---|---|---|
| H | H | $CH_2CO_2Me$ | H | O | Me | Gb |
| H | H | $CH_2CO_2Me$ | H | O | Et | Gb |
| H | H | $CH_2CO_2Me$ | H | O | $CH_2CH=CH_2$ | Gb |
| H | H | $CH_2CO_2Me$ | H | O | $CH_2C\equiv CH$ | Gb |
| H | H | $CH_2CO_2Et$ | H | O | H | Ga |
| H | H | $CH_2CO_2Et$ | H | S | H | G3 |
| H | H | $CH_2CO_2Et$ | H | O | Me | Gb |
| H | H | $CH_2CO_2Et$ | H | O | Et | Gb |
| H | H | $CH_2CO_2Et$ | H | O | $CH_2CH=CH_2$ | Gb |
| H | H | $CH_2CO_2Et$ | H | O | $CH_2C\equiv CH$ | Gb |
| H | H | $CH_2CO_2Pr-n$ | H | O | H | Gb |
| H | H | $CH_2CO_2Pr-n$ | H | S | H | G3 |
| H | H | $CH_2OCH_2CH=CH_2$ | H | O | H | Gb |
| H | H | $CH_2OCH_2CH=CH_2$ | H | O | Me | Gb |
| H | H | $CH_2OCH_2CH=CH_2$ | H | O | Et | Gb |
| H | H | $CH_2OCH_2CH=CH_2$ | H | O | $CH_2CH=CH_2$ | Gb |
| H | H | $CH_2OCH_2CH=CH_2$ | H | O | $CH_2C\equiv CH$ | Gb |
| H | H | $CH_2OCH_2C\equiv CH$ | H | O | H | Gb |
| H | H | $CH_2OCH_2C\equiv CH$ | H | O | Me | Gb |
| H | H | $CH_2OCH_2C\equiv CH$ | H | O | Et | Gb |
| H | H | $CH_2OCH_2C\equiv CH$ | H | O | $CH_2CH=CH_2$ | Gb |
| H | H | $CH_2OCH_2C\equiv CH$ | H | O | $CH_2C\equiv CH$ | Gb |
| H | H | $CH=CHCO_2Me$ | H | O | H | Ga |
| H | H | $CH=CHCO_2Me$ | H | S | H | G3 |
| H | H | $CH=CHCO_2Me$ | H | O | Me | Gb |
| H | H | $CH=CHCO_2Me$ | H | O | Et | Gb |
| H | H | $CH=CHCO_2Me$ | H | O | $CH_2CH=CH_2$ | Gb |
| H | H | $CH=CHCO_2Me$ | H | O | $CH_2C\equiv CH$ | Gb |
| H | H | $CH=CHCO_2Et$ | H | O | H | Ga |
| H | H | $CH=CHCO_2Et$ | H | S | H | G3 |
| H | H | $CH=CHCO_2Et$ | H | O | Me | Gb |
| H | H | $CH=CHCO_2Et$ | H | O | Et | Gb |
| H | H | $CH=CHCO_2Et$ | H | O | $CH_2CH=CH_2$ | Gb |
| H | H | $CH=CHCO_2Et$ | H | O | $CH_2C\equiv CH$ | Gb |
| H | H | $CH=CHCO_2Pr-n$ | H | O | H | Gb |
| H | H | $CH=CHCO_2Pr-n$ | H | S | H | G3 |
| H | H | $CH=CHCN$ | H | O | H | Ga |
| H | H | $CH=CHCN$ | H | S | H | G3 |
| H | H | Ph | H | O | H | Ga |
| H | H | Ph | H | S | H | G3 |

| $R_4$ | $R_3$ | $R_2$ | $R_1$ | X | M | Gn |
|---|---|---|---|---|---|---|
| H | H | Ph | H | O | Me | Gb |
| H | H | Ph | H | O | Et | Gb |
| H | H | Ph | H | O | $CH_2CH=CH_2$ | Gb |
| H | H | Ph | H | O | $CH_2C\equiv CH$ | Gb |
| H | H | Ph-2-F | H | O | H | Ga |
| H | H | Ph-2-F | H | S | H | G3 |
| H | H | Ph-3-F | H | O | H | Gb |
| H | H | Ph-4-F | H | O | H | Gb |
| H | H | Ph-2-Cl | H | O | H | Ga |
| H | H | Ph-2-Cl | H | S | H | G3 |
| H | H | Ph-3-Cl | H | O | H | Gb |
| H | H | Ph-4-Cl | H | O | H | Gb |
| H | H | Ph-2-Br | H | O | H | Ga |
| H | H | Ph-2-Br | H | S | H | G3 |
| H | H | Ph-3-Br | H | O | H | Gb |
| H | H | Ph-4-Br | H | O | H | Gb |
| H | H | Ph-2-$CF_3$ | H | O | H | Ga |
| H | H | Ph-2-$CF_3$ | H | S | H | G3 |
| H | H | Ph-3-$CF_3$ | H | O | H | Gb |
| H | H | Ph-4-$CF_3$ | H | O | H | Gb |
| H | H | Ph-2-Me | H | O | H | Ga |
| H | H | Ph-2-Me | H | S | H | G3 |
| H | H | Ph-3-Me | H | O | H | Gb |
| H | H | Ph-4-Me | H | O | H | Gb |
| H | H | Ph-2-Et | H | O | H | Ga |
| H | H | Ph-2-Et | H | S | H | G3 |
| H | H | Ph-2-OMe | H | O | H | Ga |
| H | H | Ph-2-OMe | H | S | H | G3 |
| H | H | Ph-3-OMe | H | O | H | Gb |
| H | H | Ph-4-OMe | H | O | H | Gb |
| H | H | Ph-2-OEt | H | O | H | Ga |
| H | H | Ph-2-OEt | H | S | H | G3 |
| H | H | Ph-2-$CO_2$Me | H | O | H | Ga |
| H | H | Ph-2-$CO_2$Me | H | S | H | G3 |
| H | H | Ph-2-$CO_2$Et | H | O | H | Ga |
| H | H | Ph-2-$CO_2$Et | H | S | H | G3 |
| H | H | Ph-3-$CO_2$Me | H | O | H | Gb |
| H | H | Ph-4-$CO_2$Me | H | O | H | Gb |
| H | H | $CH_2$Ph | H | O | H | Ga |
| H | H | $CH_2$Ph | H | S | H | G3 |

| $R_4$ | $R_3$ | $R_2$ | $R_1$ | X | M | Gn |
|---|---|---|---|---|---|---|
| H | H | $CH_2Ph$ | H | O | Me | Gb |
| H | H | $CH_2Ph$ | H | O | Et | Gb |
| H | H | $CH_2Ph$ | H | O | $CH_2CH=CH_2$ | Gb |
| H | H | $CH_2Ph$ | H | O | $CH_2C\equiv CH$ | Gb |
| H | H | $CH_2Ph$-2-F | H | O | H | Ga |
| H | H | $CH_2Ph$-2-F | H | S | H | G3 |
| H | H | $CH_2Ph$-3-F | H | O | H | Gb |
| H | H | $CH_2Ph$-4-F | H | O | H | Gb |
| H | H | $CH_2Ph$-2-Cl | H | O | H | Ga |
| H | H | $CH_2Ph$-2-Cl | H | S | H | G3 |
| H | H | $CH_2Ph$-3-Cl | H | O | H | Gb |
| H | H | $CH_2Ph$-4-Cl | H | O | H | Gb |
| H | H | $CH_2Ph$-2-Br | H | O | H | Ga |
| H | H | $CH_2Ph$-2-Br | H | S | H | G3 |
| H | H | $CH_2Ph$-3-Br | H | O | H | Gb |
| H | H | $CH_2Ph$-4-Br | H | O | H | Gb |
| H | H | $CH_2Ph$-2-$CF_3$ | H | O | H | Ga |
| H | H | $CH_2Ph$-2-$CF_3$ | H | S | H | G3 |
| H | H | $CH_2Ph$-3-$CF_3$ | H | O | H | Gb |
| H | H | $CH_2Ph$-4-$CF_3$ | H | O | H | Gb |
| H | H | $CH_2Ph$-2-Me | H | O | H | Ga |
| H | H | $CH_2Ph$-2-Me | H | S | H | G3 |
| H | H | $CH_2Ph$-3-Me | H | O | H | Gb |
| H | H | $CH_2Ph$-4-Me | H | O | H | Gb |
| H | H | $CH_2Ph$-2-Et | H | O | H | Ga |
| H | H | $CH_2Ph$-2-Et | H | S | H | G3 |
| H | H | $CH_2Ph$-2-OMe | H | O | H | Ga |
| H | H | $CH_2Ph$-2-OMe | H | S | H | G3 |
| H | H | $CH_2Ph$-3-OMe | H | O | H | Gb |
| H | H | $CH_2Ph$-4-OMe | H | O | H | Gb |
| H | H | $CH_2Ph$-2-OEt | H | O | H | Ga |
| H | H | $CH_2Ph$-2-OEt | H | S | H | G3 |
| H | H | $CH_2Ph$-2-$CO_2Me$ | H | O | H | Ga |
| H | H | $CH_2Ph$-2-$CO_2Me$ | H | S | H | G3 |
| H | H | $CH_2Ph$-2-$CO_2Et$ | H | O | H | Ga |
| H | H | $CH_2Ph$-2-$CO_2Et$ | H | S | H | G3 |
| H | H | $CH_2Ph$-3-$CO_2Me$ | H | O | H | Gb |
| H | H | $CH_2Ph$-4-$CO_2Me$ | H | O | H | Gb |
| H | H | OPh | H | O | H | Ga |
| H | H | OPh | H | S | H | G3 |

| $R_4$ | $R_3$ | $R_2$ | $R_1$ | X | M | Gn |
|---|---|---|---|---|---|---|
| H | H | OPh | H | O | Me | Gb |
| H | H | OPh | H | O | Et | Gb |
| H | H | OPh | H | O | $CH_2CH=CH_2$ | Gb |
| H | H | OPh | H | O | $CH_2C\equiv CH$ | Gb |
| H | H | OPh-2-Cl | H | O | H | Ga |
| H | H | OPh-2-Cl | H | S | H | G3 |
| H | H | OPh-3-Cl | H | O | H | Gb |
| H | H | OPh-4-Cl | H | O | H | Gb |
| H | H | OPh-2-F | H | O | H | Ga |
| H | H | OPh-2-F | H | S | H | G3 |
| H | H | OPh-3-F | H | O | H | Gb |
| H | H | OPh-4-F | H | O | H | Gb |
| H | H | $OPh-2-CF_3$ | H | O | H | Ga |
| H | H | $OPh-2-CF_3$ | H | S | H | G3 |
| H | H | $OPh-3-CF_3$ | H | O | H | Gb |
| H | H | $OPh-4-CF_3$ | H | O | H | Gb |
| H | H | OPh-2-Me | H | O | H | Ga |
| H | H | OPh-2-Me | H | S | H | G3 |
| H | H | OPh-3-Me | H | O | H | Gb |
| H | H | OPh-4-Me | H | O | H | Gb |
| H | H | OPh-2-Et | H | O | H | Ga |
| H | H | OPh-2-Et | H | S | H | G3 |
| H | H | OPh-2-OMe | H | O | H | Ga |
| H | H | OPh-2-OMe | H | S | H | G3 |
| H | H | OPh-3-OMe | H | O | H | Gb |
| H | H | OPh-4-OMe | H | O | H | Gb |
| H | H | OPh-2-OEt | H | O | H | Ga |
| H | H | OPh-2-OEt | H | S | H | G3 |
| H | H | $OPh-2-CO_2Me$ | H | O | H | Ga |
| H | H | $OPh-2-CO_2Me$ | H | S | H | G3 |
| H | H | $OPh-3-CO_2Me$ | H | O | H | Gb |
| H | H | $OPh-4-CO_2Me$ | H | O | H | Gb |
| H | H | $OPh-2-CO_2Et$ | H | O | H | Ga |
| H | H | $OPh-2-CO_2Et$ | H | S | H | G3 |
| H | H | SPh | H | O | H | Ga |
| H | H | SPh | H | S | H | G3 |
| H | H | SPh | H | O | Me | Gb |
| H | H | SPh | H | O | Et | Gb |
| H | H | SPh | H | O | $CH_2CH=CH_2$ | Gb |
| H | H | SPh | H | O | $CH_2C\equiv CH$ | Gb |
| H | H | $CON(Et)_2$ | H | S | H | G3 |
| H | H | $CON(Et)_2$ | H | O | Me | Gb |
| H | H | $CON(Et)_2$ | H | O | Et | Gb |

| $R_4$ | $R_3$ | $R_2$ | $R_1$ | X | M | Gn |
|---|---|---|---|---|---|---|
| H | H | SPh-2-Cl | H | O | H | Ga |
| H | H | SPh-2-Cl | H | S | H | G3 |
| H | H | SPh-3-Cl | H | O | H | Gb |
| H | H | SPh-4-Cl | H | O | H | Gb |
| H | H | SPh-2-F | H | O | H | Ga |
| H | H | SPh-2-F | H | S | H | G3 |
| H | H | SPh-3-F | H | O | H | Gb |
| H | H | SPh-4-F | H | O | H | Gb |
| H | H | SPh-2-$CF_3$ | H | O | H | Ga |
| H | H | SPh-2-$CF_3$ | H | S | H | G3 |
| H | H | SPh-3-$CF_3$ | H | O | H | Gb |
| H | H | SPh-4-$CF_3$ | H | O | H | Gb |
| H | H | SPh-2-Me | H | O | H | Ga |
| H | H | SPh-2-Me | H | S | H | G3 |
| H | H | SPh-3-Me | H | O | H | Gb |
| H | H | SPh-4-Me | H | O | H | Gb |
| H | H | SPh-2-Et | H | O | H | Ga |
| H | H | SPh-2-Et | H | S | H | G3 |
| H | H | SPh-2-OMe | H | O | H | Ga |
| H | H | SPh-2-OMe | H | S | H | G3 |
| H | H | SPh-3-OMe | H | O | H | Gb |
| H | H | SPh-4-OMe | H | O | H | Gb |
| H | H | SPh-2-OEt | H | O | H | Ga |
| H | H | SPh-2-OEt | H | S | H | G3 |
| H | H | SPh-2-$CO_2$Me | H | O | H | Ga |
| H | H | SPh-2-$CO_2$Me | H | S | H | G3 |
| H | H | SPh-3-$CO_2$Me | H | O | H | Gb |
| H | H | SPh-4-$CO_2$Me | H | O | H | Gb |
| H | H | SPh-2-$CO_2$Et | H | O | H | Ga |
| H | H | SPh-2-$CO_2$Et | H | S | H | G3 |
| H | H | $SO_2$Ph | H | O | H | Ga |
| H | H | $SO_2$Ph | H | S | H | G3 |
| H | H | $SO_2$Ph | H | O | Me | Gb |
| H | H | $SO_2$Ph | H | O | Et | Gb |
| H | H | $SO_2$Ph | H | O | $CH_2CH=CH_2$ | Gb |
| H | H | $SO_2$Ph | H | O | $CH_2C\equiv CH$ | Gb |
| H | H | $SO_2$Ph-2-Cl | H | O | H | Ga |
| H | H | $SO_2$Ph-2-Cl | H | S | H | G3 |
| H | H | $SO_2$Ph-3-Cl | H | O | H | Gb |
| H | H | CON(Et)$_2$ | H | O | $CH_2CH=CH_2$ | Gb |
| H | H | CON(Et)$_2$ | H | O | $CH_2C\equiv CH$ | Gb |
| H | H | $SO_2$Ph-4-Cl | H | O | H | Gb |

| $R_4$ | $R_3$ | $R_2$ | $R_1$ | X | M | Gn |
|---|---|---|---|---|---|---|
| H | H | $SO_2Ph$-2-F | H | O | H | Ga |
| H | H | $SO_2Ph$-2-F | H | S | H | G3 |
| H | H | $SO_2Ph$-3-F | H | O | H | Gb |
| H | H | $SO_2Ph$-4-F | H | O | H | Gb |
| H | H | $SO_2Ph$-2-$CF_3$ | H | O | H | Ga |
| H | H | $SO_2Ph$-2-$CF_3$ | H | S | H | G3 |
| H | H | $SO_2Ph$-3-$CF_3$ | H | O | H | Gb |
| H | H | $SO_2Ph$-4-$CF_3$ | H | O | H | Gb |
| H | H | $SO_2Ph$-2-Me | H | O | H | Ga |
| H | H | $SO_2Ph$-2-Me | H | S | H | G3 |
| H | H | $SO_2Ph$-3-Me | H | O | H | Gb |
| H | H | $SO_2Ph$-4-Me | H | O | H | Gb |
| H | H | $SO_2Ph$-2-Et | H | O | H | Ga |
| H | H | $SO_2Ph$-2-Et | H | S | H | G3 |
| H | H | $SO_2Ph$-2-OMe | H | O | H | Ga |
| H | H | $SO_2Ph$-2-OMe | H | S | H | G3 |
| H | H | $SO_2Ph$-3-OMe | H | O | H | Gb |
| H | H | $SO_2Ph$-4-OMe | H | O | H | Gb |
| H | H | $SO_2Ph$-2-OEt | H | O | H | Ga |
| H | H | $SO_2Ph$-2-OEt | H | S | H | G3 |
| H | H | $SO_2Ph$-2-$CO_2Me$ | H | O | H | Ga |
| H | H | $SO_2Ph$-2-$CO_2Me$ | H | S | H | G3 |
| H | H | $SO_2Ph$-3-$CO_2Me$ | H | O | H | Gb |
| H | H | $SO_2Ph$-4-$CO_2Me$ | H | O | H | Gb |
| H | H | $SO_2Ph$-2-$CO_2Et$ | H | O | H | Ga |
| H | H | $SO_2Ph$-2-$CO_2Et$ | H | S | H | G3 |
| H | H | CONHMe | H | O | H | Ga |
| H | H | CONHMe | H | S | H | G3 |
| H | H | CONHEt | H | O | H | Ga |
| H | H | CONHEt | H | S | H | G3 |
| H | H | CONHPr-n | H | O | H | Gb |
| H | H | CONHPr-n | H | S | H | G3 |
| H | H | CONHPr-i | H | O | H | Gb |
| H | H | CONHPr-i | H | S | H | G3 |
| H | H | CONHBu-n | H | O | H | Gb |
| H | H | CONHBu-sec | H | O | H | Gb |
| H | H | CONHBu-t | H | O | H | Gb |
| H | H | $CON(Me)_2$ | H | O | H | Ga |
| H | H | $CON(Me)_2$ | H | S | H | G3 |
| H | H | $CON(Pr\text{-}n)_2$ | H | O | H | Gb |
| H | H | $CON(Pr\text{-}n)_2$ | H | S | H | G3 |
| H | H | $CON(Et)_2$ | H | O | H | Ga |

| $R_1$ | $R_2$ | $R_3$ | $R_4$ | X | M | Gn |
|---|---|---|---|---|---|---|
| Me | Me | H | H | O | H | Ga |
| Me | Me | H | H | S | H | G3 |
| Me | H | Me | H | O | H | Ga |
| Me | H | Me | H | S | H | G3 |
| Me | Et | H | H | O | H | Ga |
| Me | Et | H | H | S | H | G3 |
| Me | H | Et | H | O | H | Ga |
| Me | H | Et | H | S | H | G3 |
| Me | Pr-n | H | H | O | H | Ga |
| Me | Pr-n | H | H | S | H | G3 |
| Me | H | Pr-n | H | O | H | Ga |
| Me | H | Pr-n | H | S | H | G3 |
| Me | Pr-i | H | H | O | H | Ga |
| Me | Pr-i | H | H | S | H | G3 |
| Me | H | Pr-i | H | O | H | Ga |
| Me | H | Pr-i | H | S | H | G3 |
| Me | Bu-n | H | H | O | H | Ga |
| Me | Bu-n | H | H | S | H | G3 |
| Me | H | Bu-n | H | O | H | Ga |
| Me | H | Bu-n | H | S | H | G3 |
| Et | Me | H | H | O | H | Ga |
| Et | Me | H | H | S | H | G3 |
| Et | H | Me | H | O | H | Ga |
| Et | H | Me | H | S | H | G3 |
| Et | Et | H | H | O | H | Ga |
| Et | Et | H | H | S | H | G3 |
| Et | H | Et | H | O | H | Ga |
| Et | H | Et | H | S | H | G3 |
| Et | Pr-n | H | H | O | H | Ga |
| Et | Pr-n | H | H | S | H | G3 |
| Et | H | Pr-n | H | O | H | Ga |
| Et | H | Pr-n | H | S | H | G3 |
| Et | Pr-i | H | H | O | H | Ga |
| Et | Pr-i | H | H | S | H | G3 |
| Et | H | Pr-i | H | O | H | Ga |
| Et | H | Pr-i | H | S | H | G3 |
| Et | Bu-n | H | H | O | H | Ga |
| Et | Bu-n | H | H | S | H | G3 |
| Et | H | Bu-n | H | O | H | Ga |
| Et | H | Bu-n | H | S | H | G3 |

| $R_1$ | $R_2$ | $R_3$ | $R_4$ | X | M | Gn |
|---|---|---|---|---|---|---|
| Pr-n | Me | H | H | O | H | Ga |
| Pr-n | Me | H | H | S | H | G3 |
| Pr-n | H | Me | H | O | H | Ga |
| Pr-n | H | Me | H | S | H | G3 |
| Pr-n | Et | H | H | O | H | Ga |
| Pr-n | Et | H | H | S | H | G3 |
| Pr-n | H | Et | H | O | H | Ga |
| Pr-n | H | Et | H | S | H | G3 |
| Pr-n | Pr-n | H | H | O | H | Ga |
| Pr-n | Pr-n | H | H | S | H | G3 |
| Pr-n | H | Pr-n | H | O | H | Ga |
| Pr-n | H | Pr-n | H | S | H | G3 |
| Pr-n | Pr-i | H | H | O | H | Ga |
| Pr-n | Pr-i | H | H | S | H | G3 |
| Pr-n | H | Pr-i | H | O | H | Ga |
| Pr-n | H | Pr-i | H | S | H | G3 |
| Pr-n | Bu-n | H | H | O | H | Ga |
| Pr-n | Bu-n | H | H | S | H | G3 |
| Pr-n | H | Bu-n | H | O | H | Ga |
| Pr-n | H | Bu-n | H | S | H | G3 |
| Pr-i | Me | H | H | O | H | Ga |
| Pr-i | Me | H | H | S | H | G3 |
| Pr-i | H | Me | H | O | H | Ga |
| Pr-i | H | Me | H | S | H | G3 |
| Pr-i | Et | H | H | O | H | Ga |
| Pr-i | Et | H | H | S | H | G3 |
| Pr-i | H | Et | H | O | H | Ga |
| Pr-i | H | Et | H | S | H | G3 |
| Pr-i | Pr-n | H | H | O | H | Ga |
| Pr-i | Pr-n | H | H | S | H | G3 |
| Pr-i | H | Pr-n | H | O | H | Ga |
| Pr-i | H | Pr-n | H | S | H | G3 |
| Pr-i | Pr-i | H | H | O | H | Ga |
| Pr-i | Pr-i | H | H | S | H | G3 |
| Pr-i | H | Pr-i | H | O | H | Ga |
| Pr-i | H | Pr-i | H | S | H | G3 |
| Pr-i | Bu-n | H | H | O | H | Ga |
| Pr-i | Bu-n | H | H | S | H | G3 |
| Pr-i | H | Bu-n | H | O | H | Ga |
| Pr-i | H | Bu-n | H | S | H | G3 |

| $R_1$ | $R_2$ | $R_3$ | $R_4$ | X | M | Gn |
|---|---|---|---|---|---|---|
| Bu-n | Me | H | H | O | H | Ga |
| Bu-n | Me | H | H | S | H | G3 |
| Bu-n | H | Me | H | O | H | Ga |
| Bu-n | H | Me | H | S | H | G3 |
| Bu-n | Et | H | H | O | H | Ga |
| Bu-n | Et | H | H | S | H | G3 |
| Bu-n | H | Et | H | O | H | Ga |
| Bu-n | H | Et | H | S | H | G3 |
| Bu-n | Pr-n | H | H | O | H | Ga |
| Bu-n | Pr-n | H | H | S | H | G3 |
| Bu-n | H | Pr-n | H | O | H | Ga |
| Bu-n | H | Pr-n | H | S | H | G3 |
| Bu-n | Pr-i | H | H | O | H | Ga |
| Bu-n | Pr-i | H | H | S | H | G3 |
| Bu-n | H | Pr-i | H | O | H | Ga |
| Bu-n | H | Pr-i | H | S | H | G3 |
| Bu-n | Bu-n | H | H | O | H | Ga |
| Bu-n | Bu-n | H | H | S | H | G3 |
| Bu-n | H | Bu-n | H | O | H | Ga |
| Bu-n | H | Bu-n | H | S | H | G3 |
| H | Me | Me | H | O | H | Ga |
| H | Me | Me | H | S | H | G3 |
| H | Me | Et | H | O | H | Ga |
| H | Me | Et | H | S | H | G3 |
| H | Me | Pr-n | H | O | H | Ga |
| H | Me | Pr-n | H | S | H | G3 |
| H | Me | Pr-i | H | O | H | Ga |
| H | Me | Pr-i | H | S | H | G3 |
| H | Me | Bu-n | H | O | H | Ga |
| H | Me | Bu-n | H | S | H | G3 |
| H | Et | Me | H | O | H | Ga |
| H | Et | Me | H | S | H | G3 |
| H | Et | Et | H | O | H | Ga |
| H | Et | Et | H | S | H | G3 |
| H | Et | Pr-n | H | O | H | Ga |
| H | Et | Pr-n | H | S | H | G3 |
| H | Et | Pr-i | H | O | H | Ga |
| H | Et | Pr-i | H | S | H | G3 |
| H | Et | Bu-n | H | O | H | Ga |
| H | Et | Bu-n | H | S | H | G3 |

| $R_1$ | $R_2$ | $R_3$ | $R_4$ | X | M | Gn |
|---|---|---|---|---|---|---|
| H | Pr-n | Me | H | O | H | Ga |
| H | Pr-n | Me | H | S | H | G3 |
| H | Pr-n | Et | H | O | H | Ga |
| H | Pr-n | Et | H | S | H | G3 |
| H | Pr-n | Pr-n | H | O | H | Ga |
| H | Pr-n | Pr-n | H | S | H | G3 |
| H | Pr-n | Pr-i | H | O | H | Ga |
| H | Pr-n | Pr-i | H | S | H | G3 |
| H | Pr-n | Bu-n | H | O | H | Ga |
| H | Pr-n | Bu-n | H | S | H | G3 |
| H | Pr-i | Me | H | O | H | Ga |
| H | Pr-i | Me | H | S | H | G3 |
| H | Pr-i | Et | H | O | H | Ga |
| H | Pr-i | Et | H | S | H | G3 |
| H | Pr-i | Pr-n | H | O | H | Ga |
| H | Pr-i | Pr-n | H | S | H | G3 |
| H | Pr-i | Pr-i | H | O | H | Ga |
| H | Pr-i | Pr-i | H | S | H | G3 |
| H | Pr-i | Bu-n | H | O | H | Ga |
| H | Pr-i | Bu-n | H | S | H | G3 |
| H | Bu-n | Me | H | O | H | Ga |
| H | Bu-n | Me | H | S | H | G3 |
| H | Bu-n | Et | H | O | H | Ga |
| H | Bu-n | Et | H | S | H | G3 |
| H | Bu-n | Pr-n | H | O | H | Ga |
| H | Bu-n | Pr-n | H | S | H | G3 |
| H | Bu-n | Pr-i | H | O | H | Ga |
| H | Bu-n | Pr-i | H | S | H | G3 |
| H | Bu-n | Bu-n | H | O | H | Ga |
| H | Bu-n | Bu-n | H | S | H | G3 |
| Me | Me | Me | H | O | H | Ga |
| Me | Me | Me | H | S | H | G3 |
| Me | Me | Et | H | O | H | Ga |
| Me | Me | Et | H | S | H | G3 |
| Me | Me | Pr-n | H | O | H | Ga |
| Me | Me | Pr-n | H | S | H | G3 |
| Me | Me | Pr-i | H | O | H | Ga |
| Me | Me | Pr-i | H | S | H | G3 |
| Me | Me | Bu-n | H | O | H | Ga |
| Me | Me | Bu-n | H | S | H | G3 |

| $R_1$ | $R_2$ | $R_3$ | $R_4$ | X | M | Gn |
|---|---|---|---|---|---|---|
| Me | Et | Me | H | O | H | Ga |
| Me | Et | Me | H | S | H | G3 |
| Me | Et | Et | H | O | H | Ga |
| Me | Et | Et | H | S | H | G3 |
| Me | Et | Pr-n | H | O | H | Ga |
| Me | Et | Pr-n | H | S | H | G3 |
| Me | Et | Pr-i | H | O | H | Ga |
| Me | Et | Pr-i | H | S | H | G3 |
| Me | Et | Bu-n | H | O | H | Ga |
| Me | Et | Bu-n | H | S | H | G3 |
| Me | Pr-n | Me | H | O | H | Ga |
| Me | Pr-n | Me | H | S | H | G3 |
| Me | Pr-n | Et | H | O | H | Ga |
| Me | Pr-n | Et | H | S | H | G3 |
| Me | Pr-n | Pr-n | H | O | H | Ga |
| Me | Pr-n | Pr-n | H | S | H | G3 |
| Me | Pr-n | Pr-i | H | O | H | Ga |
| Me | Pr-n | Pr-i | H | S | H | G3 |
| Me | Pr-n | Bu-n | H | O | H | Ga |
| Me | Pr-n | Bu-n | H | S | H | G3 |
| Me | Pr-i | Me | H | O | H | Ga |
| Me | Pr-i | Me | H | S | H | G3 |
| Me | Pr-i | Et | H | O | H | Ga |
| Me | Pr-i | Et | H | S | H | G3 |
| Me | Pr-i | Pr-n | H | O | H | Ga |
| Me | Pr-i | Pr-n | H | S | H | G3 |
| Me | Pr-i | Pr-i | H | O | H | Ga |
| Me | Pr-i | Pr-i | H | S | H | G3 |
| Me | Pr-i | Bu-n | H | O | H | Ga |
| Me | Pr-i | Bu-n | H | S | H | G3 |
| Me | Bu-n | Me | H | O | H | Ga |
| Me | Bu-n | Me | H | S | H | G3 |
| Me | Bu-n | Et | H | O | H | Ga |
| Me | Bu-n | Et | H | S | H | G3 |
| Me | Bu-n | Pr-n | H | O | H | Ga |
| Me | Bu-n | Pr-n | H | S | H | G3 |
| Me | Bu-n | Pr-i | H | O | H | Ga |
| Me | Bu-n | Pr-i | H | S | H | G3 |
| Me | Bu-n | Bu-n | H | O | H | Ga |
| Me | Bu-n | Bu-n | H | S | H | G3 |

| $R_1$ | $R_2$ | $R_3$ | $R_4$ | X | M | Gn |
|---|---|---|---|---|---|---|
| Et | Me | Me | H | O | H | Ga |
| Et | Me | Me | H | S | H | G3 |
| Et | Me | Et | H | O | H | Ga |
| Et | Me | Et | H | S | H | G3 |
| Et | Me | Pr-n | H | O | H | Ga |
| Et | Me | Pr-n | H | S | H | G3 |
| Et | Me | Pr-i | H | O | H | Ga |
| Et | Me | Pr-i | H | S | H | G3 |
| Et | Me | Bu-n | H | O | H | Ga |
| Et | Me | Bu-n | H | S | H | G3 |
| Et | Et | Me | H | O | H | Ga |
| Et | Et | Me | H | S | H | G3 |
| Et | Et | Et | H | O | H | Ga |
| Et | Et | Et | H | S | H | G3 |
| Et | Et | Pr-n | H | O | H | Ga |
| Et | Et | Pr-n | H | S | H | G3 |
| Et | Et | Pr-i | H | O | H | Ga |
| Et | Et | Pr-i | H | S | H | G3 |
| Et | Et | Bu-n | H | O | H | Ga |
| Et | Et | Bu-n | H | S | H | G3 |
| Et | Pr-n | Me | H | O | H | Ga |
| Et | Pr-n | Me | H | S | H | G3 |
| Et | Pr-n | Et | H | O | H | Ga |
| Et | Pr-n | Et | H | S | H | G3 |
| Et | Pr-n | Pr-n | H | O | H | Ga |
| Et | Pr-n | Pr-n | H | S | H | G3 |
| Et | Pr-n | Pr-i | H | O | H | Ga |
| Et | Pr-n | Pr-i | H | S | H | G3 |
| Et | Pr-n | Bu-n | H | O | H | Ga |
| Et | Pr-n | Bu-n | H | S | H | G3 |
| Et | Pr-i | Me | H | O | H | Ga |
| Et | Pr-i | Me | H | S | H | G3 |
| Et | Pr-i | Et | H | O | H | Ga |
| Et | Pr-i | Et | H | S | H | G3 |
| Et | Pr-i | Pr-n | H | O | H | Ga |
| Et | Pr-i | Pr-n | H | S | H | G3 |
| Et | Pr-i | Pr-i | H | O | H | Ga |
| Et | Pr-i | Pr-i | H | S | H | G3 |
| Et | Pr-i | Bu-n | H | O | H | Ga |
| Et | Pr-i | Bu-n | H | S | H | G3 |

| $R_1$ | $R_2$ | $R_3$ | $R_4$ | X | M | Gn |
|---|---|---|---|---|---|---|
| Et | Bu-n | Me | H | O | H | Ga |
| Et | Bu-n | Me | H | S | H | G3 |
| Et | Bu-n | Et | H | O | H | Ga |
| Et | Bu-n | Et | H | S | H | G3 |
| Et | Bu-n | Pr-n | H | O | H | Ga |
| Et | Bu-n | Pr-n | H | S | H | G3 |
| Et | Bu-n | Pr-i | H | O | H | Ga |
| Et | Bu-n | Pr-i | H | S | H | G3 |
| Et | Bu-n | Bu-n | H | O | H | Ga |
| Et | Bu-n | Bu-n | H | S | H | G3 |
| Pr-n | Me | Me | H | O | H | Ga |
| Pr-n | Me | Me | H | S | H | G3 |
| Pr-n | Me | Et | H | O | H | Ga |
| Pr-n | Me | Et | H | S | H | G3 |
| Pr-n | Me | Pr-n | H | O | H | Ga |
| Pr-n | Me | Pr-n | H | S | H | G3 |
| Pr-n | Me | Pr-i | H | O | H | Ga |
| Pr-n | Me | Pr-i | H | S | H | G3 |
| Pr-n | Me | Bu-n | H | O | H | Ga |
| Pr-n | Me | Bu-n | H | S | H | G3 |
| Pr-n | Et | Me | H | O | H | Ga |
| Pr-n | Et | Me | H | S | H | G3 |
| Pr-n | Et | Et | H | O | H | Ga |
| Pr-n | Et | Et | H | S | H | G3 |
| Pr-n | Et | Pr-n | H | O | H | Ga |
| Pr-n | Et | Pr-n | H | S | H | G3 |
| Pr-n | Et | Pr-i | H | O | H | Ga |
| Pr-n | Et | Pr-i | H | S | H | G3 |
| Pr-n | Et | Bu-n | H | O | H | Ga |
| Pr-n | Et | Bu-n | H | S | H | G3 |
| Pr-n | Pr-n | Me | H | O | H | Ga |
| Pr-n | Pr-n | Me | H | S | H | G3 |
| Pr-n | Pr-n | Et | H | O | H | Ga |
| Pr-n | Pr-n | Et | H | S | H | G3 |
| Pr-n | Pr-n | Pr-n | H | O | H | Ga |
| Pr-n | Pr-n | Pr-n | H | S | H | G3 |
| Pr-n | Pr-n | Pr-i | H | O | H | Ga |
| Pr-n | Pr-n | Pr-i | H | S | H | G3 |
| Pr-n | Pr-n | Bu-n | H | O | H | Ga |
| Pr-n | Pr-n | Bu-n | H | S | H | G3 |

| $R_1$ | $R_2$ | $R_3$ | $R_4$ | X | M | Gn |
|---|---|---|---|---|---|---|
| Pr-n | Pr-i | Me | H | O | H | Ga |
| Pr-n | Pr-i | Me | H | S | H | G3 |
| Pr-n | Pr-i | Et | H | O | H | Ga |
| Pr-n | Pr-i | Et | H | S | H | G3 |
| Pr-n | Pr-i | Pr-n | H | O | H | Ga |
| Pr-n | Pr-i | Pr-n | H | S | H | G3 |
| Pr-n | Pr-i | Pr-i | H | O | H | Ga |
| Pr-n | Pr-i | Pr-i | H | S | H | G3 |
| Pr-n | Pr-i | Bu-n | H | O | H | Ga |
| Pr-n | Pr-i | Bu-n | H | S | H | G3 |
| Pr-n | Bu-n | Me | H | O | H | Ga |
| Pr-n | Bu-n | Me | H | S | H | G3 |
| Pr-n | Bu-n | Et | H | O | H | Ga |
| Pr-n | Bu-n | Et | H | S | H | G3 |
| Pr-n | Bu-n | Pr-n | H | O | H | Ga |
| Pr-n | Bu-n | Pr-n | H | S | H | G3 |
| Pr-n | Bu-n | Pr-i | H | O | H | Ga |
| Pr-n | Bu-n | Pr-i | H | S | H | G3 |
| Pr-n | Bu-n | Bu-n | H | O | H | Ga |
| Pr-n | Bu-n | Bu-n | H | S | H | G3 |
| Pr-i | Me | Me | H | O | H | Ga |
| Pr-i | Me | Me | H | S | H | G3 |
| Pr-i | Me | Et | H | O | H | Ga |
| Pr-i | Me | Et | H | S | H | G3 |
| Pr-i | Me | Pr-n | H | O | H | Ga |
| Pr-i | Me | Pr-n | H | S | H | G3 |
| Pr-i | Me | Pr-i | H | O | H | Ga |
| Pr-i | Me | Pr-i | H | S | H | G3 |
| Pr-i | Me | Bu-n | H | O | H | Ga |
| Pr-i | Me | Bu-n | H | S | H | G3 |
| Pr-i | Et | Me | H | O | H | Ga |
| Pr-i | Et | Me | H | S | H | G3 |
| Pr-i | Et | Et | H | O | H | Ga |
| Pr-i | Et | Et | H | S | H | G3 |
| Pr-i | Et | Pr-n | H | O | H | Ga |
| Pr-i | Et | Pr-n | H | S | H | G3 |
| Pr-i | Et | Pr-i | H | O | H | Ga |
| Pr-i | Et | Pr-i | H | S | H | G3 |
| Pr-i | Et | Bu-n | H | O | H | Ga |
| Pr-i | Et | Bu-n | H | S | H | G3 |

| $R_1$ | $R_2$ | $R_3$ | $R_4$ | X | M | Gn |
|---|---|---|---|---|---|---|
| Pr-i | Pr-n | Me | H | O | H | Ga |
| Pr-i | Pr-n | Me | H | S | H | G3 |
| Pr-i | Pr-n | Et | H | O | H | Ga |
| Pr-i | Pr-n | Et | H | S | H | G3 |
| Pr-i | Pr-n | Pr-n | H | O | H | Ga |
| Pr-i | Pr-n | Pr-n | H | S | H | G3 |
| Pr-i | Pr-n | Pr-i | H | O | H | Ga |
| Pr-i | Pr-n | Pr-i | H | S | H | G3 |
| Pr-i | Pr-n | Bu-n | H | O | H | Ga |
| Pr-i | Pr-n | Bu-n | H | S | H | G3 |
| Pr-i | Pr-i | Me | H | O | H | Ga |
| Pr-i | Pr-i | Me | H | S | H | G3 |
| Pr-i | Pr-i | Et | H | O | H | Ga |
| Pr-i | Pr-i | Et | H | S | H | G3 |
| Pr-i | Pr-i | Pr-n | H | O | H | Ga |
| Pr-i | Pr-i | Pr-n | H | S | H | G3 |
| Pr-i | Pr-i | Pr-i | H | O | H | Ga |
| Pr-i | Pr-i | Pr-i | H | S | H | G3 |
| Pr-i | Pr-i | Bu-n | H | O | H | Ga |
| Pr-i | Pr-i | Bu-n | H | S | H | G3 |
| Pr-i | Bu-n | Me | H | O | H | Ga |
| Pr-i | Bu-n | Me | H | S | H | G3 |
| Pr-i | Bu-n | Et | H | O | H | Ga |
| Pr-i | Bu-n | Et | H | S | H | G3 |
| Pr-i | Bu-n | Pr-n | H | O | H | Ga |
| Pr-i | Bu-n | Pr-n | H | S | H | G3 |
| Pr-i | Bu-n | Pr-i | H | O | H | Ga |
| Pr-i | Bu-n | Pr-i | H | S | H | G3 |
| Pr-i | Bu-n | Bu-n | H | O | H | Ga |
| Pr-i | Bu-n | Bu-n | H | S | H | G3 |
| Bu-n | Me | Me | H | O | H | Ga |
| Bu-n | Me | Me | H | S | H | G3 |
| Bu-n | Me | Et | H | O | H | Ga |
| Bu-n | Me | Et | H | S | H | G3 |
| Bu-n | Me | Pr-n | H | O | H | Ga |
| Bu-n | Me | Pr-n | H | S | H | G3 |
| Bu-n | Me | Pr-i | H | O | H | Ga |
| Bu-n | Me | Pr-i | H | S | H | G3 |
| Bu-n | Me | Bu-n | H | O | H | Ga |
| Bu-n | Me | Bu-n | H | S | H | G3 |

| $R_1$ | $R_2$ | $R_3$ | $R_4$ | X | M | Gn |
|---|---|---|---|---|---|---|
| Bu-n | Et | Me | H | O | H | Ga |
| Bu-n | Et | Me | H | S | H | G3 |
| Bu-n | Et | Et | H | O | H | Ga |
| Bu-n | Et | Et | H | S | H | G3 |
| Bu-n | Et | Pr-n | H | O | H | Ga |
| Bu-n | Et | Pr-n | H | S | H | G3 |
| Bu-n | Et | Pr-i | H | O | H | Ga |
| Bu-n | Et | Pr-i | H | S | H | G3 |
| Bu-n | Et | Bu-n | H | O | H | Ga |
| Bu-n | Et | Bu-n | H | S | H | G3 |
| Bu-n | Pr-n | Me | H | O | H | Ga |
| Bu-n | Pr-n | Me | H | S | H | G3 |
| Bu-n | Pr-n | Et | H | O | H | Ga |
| Bu-n | Pr-n | Et | H | S | H | G3 |
| Bu-n | Pr-n | Pr-n | H | O | H | Ga |
| Bu-n | Pr-n | Pr-n | H | S | H | G3 |
| Bu-n | Pr-n | Pr-i | H | O | H | Ga |
| Bu-n | Pr-n | Pr-i | H | S | H | G3 |
| Bu-n | Pr-n | Bu-n | H | O | H | Ga |
| Bu-n | Pr-n | Bu-n | H | S | H | G3 |
| Bu-n | Pr-i | Me | H | O | H | Ga |
| Bu-n | Pr-i | Me | H | S | H | G3 |
| Bu-n | Pr-i | Et | H | O | H | Ga |
| Bu-n | Pr-i | Et | H | S | H | G3 |
| Bu-n | Pr-i | Pr-n | H | O | H | Ga |
| Bu-n | Pr-i | Pr-n | H | S | H | G3 |
| Bu-n | Pr-i | Pr-i | H | O | H | Ga |
| Bu-n | Pr-i | Pr-i | H | S | H | G3 |
| Bu-n | Pr-i | Bu-n | H | O | H | Ga |
| Bu-n | Pr-i | Bu-n | H | S | H | G3 |
| Bu-n | Bu-n | Me | H | O | H | Ga |
| Bu-n | Bu-n | Me | H | S | H | G3 |
| Bu-n | Bu-n | Et | H | O | H | Ga |
| Bu-n | Bu-n | Et | H | S | H | G3 |
| Bu-n | Bu-n | Pr-n | H | O | H | Ga |
| Bu-n | Bu-n | Pr-n | H | S | H | G3 |
| Bu-n | Bu-n | Pr-i | H | O | H | Ga |
| Bu-n | Bu-n | Pr-i | H | S | H | G3 |
| Bu-n | Bu-n | Bu-n | H | O | H | Ga |
| Bu-n | Bu-n | Bu-n | H | S | H | G3 |

| $R_1$ | $R_2$ | $R_3$ | $R_4$ | X | M | Gn |
|---|---|---|---|---|---|---|
| F | H | Me | H | O | H | Ga |
| Cl | H | Me | H | O | H | Ga |
| Br | H | Me | H | O | H | Ga |
| I | H | Me | H | O | H | Ga |
| $CH=CH_2$ | H | Me | H | O | H | Ga |
| $CH_2CH=CH_2$ | H | Me | H | O | H | Ga |
| $C\equiv CH$ | H | Me | H | O | H | Ga |
| $CH_2C\equiv CH$ | H | Me | H | O | H | Ga |
| $CH_2F$ | H | Me | H | O | H | Ga |
| $CHF_2$ | H | Me | H | O | H | Ga |
| CN | H | Me | H | O | H | Ga |
| $NH_2$ | H | Me | H | O | H | Ga |
| NHCOMe | H | Me | H | O | H | Ga |
| $CH_2NH_2$ | H | Me | H | O | H | Ga |
| $CH_2NHCOMe$ | H | Me | H | O | H | Ga |
| $CH_2OCHF_2$ | H | Me | H | O | H | Ga |
| $CF_3$ | H | Me | H | O | H | Ga |
| $NO_2$ | H | Me | H | O | H | Gb |
| OMe | H | Me | H | O | H | Ga |
| OEt | H | Me | H | O | H | Ga |
| OPr-n | H | Me | H | O | H | Gb |
| SMe | H | Me | H | O | H | Ga |
| SEt | H | Me | H | O | H | Ga |
| $SO_2Me$ | H | Me | H | O | H | Ga |
| $SO_2Et$ | H | Me | H | O | H | Ga |
| COMe | H | Me | H | O | H | Ga |
| COEt | H | Me | H | O | H | Ga |
| $CO_2H$ | H | Me | H | O | H | Gb |
| $CO_2Me$ | H | Me | H | O | H | Ga |
| $CO_2Et$ | H | Me | H | O | H | Ga |
| $CO_2Pr$-n | H | Me | H | O | H | Gb |
| $CO_2Bu$-n | H | Me | H | O | H | Gc |
| $CH_2Cl$ | H | Me | H | O | H | Ga |
| $CH_2Br$ | H | Me | H | O | H | Gb |
| $CH_2I$ | H | Me | H | O | H | Gb |
| $CH_2CF_3$ | H | Me | H | O | H | Ga |
| $CH_2CN$ | H | Me | H | O | H | Ga |
| $CH_2OH$ | H | Me | H | O | H | Gb |
| $CH_2OMe$ | H | Me | H | O | H | Ga |
| $CH_2OEt$ | H | Me | H | O | H | Ga |

| $R_1$ | $R_2$ | $R_3$ | $R_4$ | X | M | Gn |
|---|---|---|---|---|---|---|
| $CH_2OPr$-n | H | Me | H | O | H | Gb |
| $CH_2SMe$ | H | Me | H | O | H | Ga |
| $CH_2SEt$ | H | Me | H | O | H | Ga |
| $CH_2SPr$-n | H | Me | H | O | H | Gb |
| $CH_2SO_2Me$ | H | Me | H | O | H | Ga |
| $CH_2SO_2Et$ | H | Me | H | O | H | Ga |
| $CH_2COMe$ | H | Me | H | O | H | Ga |
| $CH_2COEt$ | H | Me | H | O | H | Ga |
| $CH_2COPr$-n | H | Me | H | O | H | Gb |
| $CH_2CO_2H$ | H | Me | H | O | H | Gb |
| $CH_2CO_2Me$ | H | Me | H | O | H | Ga |
| $CH_2CO_2Et$ | H | Me | H | O | H | Ga |
| $CH_2CO_2Pr$-n | H | Me | H | O | H | Gb |
| $CH_2OCH_2CH=CH_2$ | H | Me | H | O | H | Gb |
| $CH_2OCH_2C\equiv CH$ | H | Me | H | O | H | Gb |
| $CH=CHCO_2Me$ | H | Me | H | O | H | Ga |
| $CH=CHCO_2Et$ | H | Me | H | O | H | Ga |
| $CH=CHCO_2Pr$-n | H | Me | H | O | H | Gb |
| CH=CHCN | H | Me | H | O | H | Ga |
| Ph | H | Me | H | O | H | Ga |
| $CH_2Ph$ | H | Me | H | O | H | Ga |
| OPh | H | Me | H | O | H | Ga |
| SPh | H | Me | H | O | H | Ga |
| $SO_2Ph$ | H | Me | H | O | H | Ga |
| CONHMe | H | Me | H | O | H | Ga |
| CONHEt | H | Me | H | O | H | Ga |
| $CON(Me)_2$ | H | Me | H | O | H | Ga |
| $CON(Et)_2$ | H | Me | H | O | H | Ga |
| F | Me | H | H | O | H | Ga |
| Cl | Me | H | H | O | H | Ga |
| Br | Me | H | H | O | H | Ga |
| I | Me | H | H | O | H | Ga |
| $CH=CH_2$ | Me | H | H | O | H | Ga |
| $CH_2CH=CH_2$ | Me | H | H | O | H | Ga |
| $C\equiv CH$ | Me | H | H | O | H | Ga |
| $CH_2C\equiv CH$ | Me | H | H | O | H | Ga |
| $CH_2F$ | Me | H | H | O | H | Ga |
| $CHF_2$ | Me | H | H | O | H | Ga |
| CN | Me | H | H | O | H | Ga |
| $NH_2$ | Me | H | H | O | H | Ga |

| $R_1$ | $R_2$ | $R_3$ | $R_4$ | X | M | Gn |
|---|---|---|---|---|---|---|
| NHCOMe | Me | H | H | O | H | Ga |
| $CH_2NH_2$ | Me | H | H | O | H | Ga |
| $CH_2NHCOMe$ | Me | H | H | O | H | Ga |
| $CH_2OCHF_2$ | Me | H | H | O | H | Ga |
| $CF_3$ | Me | H | H | O | H | Ga |
| $NO_2$ | Me | H | H | O | H | Gb |
| OMe | Me | H | H | O | H | Ga |
| OEt | Me | H | H | O | H | Ga |
| OPr-n | Me | H | H | O | H | Gb |
| SMe | Me | H | H | O | H | Ga |
| SEt | Me | H | H | O | H | Ga |
| $SO_2Me$ | Me | H | H | O | H | Ga |
| $SO_2Et$ | Me | H | H | O | H | Ga |
| COMe | Me | H | H | O | H | Ga |
| COEt | Me | H | H | O | H | Ga |
| $CO_2H$ | Me | H | H | O | H | Gb |
| $CO_2Me$ | Me | H | H | O | H | Ga |
| $CO_2Et$ | Me | H | H | O | H | Ga |
| $CO_2Pr$-n | Me | H | H | O | H | Gb |
| $CO_2Bu$-n | Me | H | H | O | H | Gc |
| $CH_2Cl$ | Me | H | H | O | H | Ga |
| $CH_2Br$ | Me | H | H | O | H | Gb |
| $CH_2I$ | Me | H | H | O | H | Gb |
| $CH_2CF_3$ | Me | H | H | O | H | Ga |
| $CH_2CN$ | Me | H | H | O | H | Ga |
| $CH_2OH$ | Me | H | H | O | H | Gb |
| $CH_2OMe$ | Me | H | H | O | H | Ga |
| $CH_2OEt$ | Me | H | H | O | H | Ga |
| $CH_2OPr$-n | Me | H | H | O | H | Gb |
| $CH_2SMe$ | Me | H | H | O | H | Ga |
| $CH_2SEt$ | Me | H | H | O | H | Ga |
| $CH_2SPr$-n | Me | H | H | O | H | Gb |
| $CH_2SO_2Me$ | Me | H | H | O | H | Ga |
| $CH_2SO_2Et$ | Me | H | H | O | H | Ga |
| $CH_2COMe$ | Me | H | H | O | H | Ga |
| $CH_2COEt$ | Me | H | H | O | H | Ga |
| $CH_2COPr$-n | Me | H | H | O | H | Gb |
| $CH_2CO_2H$ | Me | H | H | O | H | Gb |
| $CH_2CO_2Me$ | Me | H | H | O | H | Ga |
| $CH_2CO_2Et$ | Me | H | H | O | H | Ga |

| $R_1$ | $R_2$ | $R_3$ | $R_4$ | X | M | Gn |
|---|---|---|---|---|---|---|
| $CH_2CO_2Pr$-n | Me | H | H | O | H | Gb |
| $CH_2OCH_2CH=CH_2$ | Me | H | H | O | H | Gb |
| $CH_2OCH_2C\equiv CH$ | Me | H | H | O | H | Gb |
| $CH=CHCO_2Me$ | Me | H | H | O | H | Ga |
| $CH=CHCO_2Et$ | Me | H | H | O | H | Ga |
| $CH=CHCO_2Pr$-n | Me | H | H | O | H | Gb |
| CH=CHCN | Me | H | H | O | H | Ga |
| Ph | Me | H | H | O | H | Ga |
| $CH_2Ph$ | Me | H | H | O | H | Ga |
| OPh | Me | H | H | O | H | Ga |
| SPh | Me | H | H | O | H | Ga |
| $SO_2Ph$ | Me | H | H | O | H | Ga |
| CONHMe | Me | H | H | O | H | Ga |
| CONHEt | Me | H | H | O | H | Ga |
| $CON(Me)_2$ | Me | H | H | O | H | Ga |
| $CON(Et)_2$ | Me | H | H | O | H | Ga |
| F | Et | H | H | O | H | Ga |
| Cl | Et | H | H | O | H | Ga |
| Br | Et | H | H | O | H | Ga |
| I | Et | H | H | O | H | Ga |
| $CH=CH_2$ | Et | H | H | O | H | Ga |
| $CH_2CH=CH_2$ | Et | H | H | O | H | Ga |
| $C\equiv CH$ | Et | H | H | O | H | Ga |
| $CH_2C\equiv CH$ | Et | H | H | O | H | Ga |
| $CH_2F$ | Et | H | H | O | H | Ga |
| $CHF_2$ | Et | H | H | O | H | Ga |
| CN | Et | H | H | O | H | Ga |
| $NH_2$ | Et | H | H | O | H | Ga |
| NHCOMe | Et | H | H | O | H | Ga |
| $CH_2NH_2$ | Et | H | H | O | H | Ga |
| $CH_2NHCOMe$ | Et | H | H | O | H | Ga |
| $CH_2OCHF_2$ | Et | H | H | O | H | Ga |
| $CF_3$ | Et | H | H | O | H | Ga |
| $NO_2$ | Et | H | H | O | H | Gb |
| OMe | Et | H | H | O | H | Ga |
| OEt | Et | H | H | O | H | Ga |
| OPr-n | Et | H | H | O | H | Gb |
| SMe | Et | H | H | O | H | Ga |
| SEt | Et | H | H | O | H | Ga |
| $SO_2Me$ | Et | H | H | O | H | Ga |

| $R_1$ | $R_2$ | $R_3$ | $R_4$ | X | M | Gn |
|---|---|---|---|---|---|---|
| $SO_2Et$ | Et | H | H | O | H | Ga |
| COMe | Et | H | H | O | H | Ga |
| COEt | Et | H | H | O | H | Ga |
| $CO_2H$ | Et | H | H | O | H | Gb |
| $CO_2Me$ | Et | H | H | O | H | Ga |
| $CO_2Et$ | Et | H | H | O | H | Ga |
| $CO_2Pr$-n | Et | H | H | O | H | Gb |
| $CO_2Bu$-n | Et | H | H | O | H | Gc |
| $CH_2Cl$ | Et | H | H | O | H | Ga |
| $CH_2Br$ | Et | H | H | O | H | Gb |
| $CH_2I$ | Et | H | H | O | H | Gb |
| $CH_2CF_3$ | Et | H | H | O | H | Ga |
| $CH_2CN$ | Et | H | H | O | H | Ga |
| $CH_2OH$ | Et | H | H | O | H | Gb |
| $CH_2OMe$ | Et | H | H | O | H | Ga |
| $CH_2OEt$ | Et | H | H | O | H | Ga |
| $CH_2OPr$-n | Et | H | H | O | H | Gb |
| $CH_2SMe$ | Et | H | H | O | H | Ga |
| $CH_2SEt$ | Et | H | H | O | H | Ga |
| $CH_2SPr$-n | Et | H | H | O | H | Gb |
| $CH_2SO_2Me$ | Et | H | H | O | H | Ga |
| $CH_2SO_2Et$ | Et | H | H | O | H | Ga |
| $CH_2COMe$ | Et | H | H | O | H | Ga |
| $CH_2COEt$ | Et | H | H | O | H | Ga |
| $CH_2COPr$-n | Et | H | H | O | H | Gb |
| $CH_2CO_2H$ | Et | H | H | O | H | Gb |
| $CH_2CO_2Me$ | Et | H | H | O | H | Ga |
| $CH_2CO_2Et$ | Et | H | H | O | H | Ga |
| $CH_2CO_2Pr$-n | Et | H | H | O | H | Gb |
| $CH_2OCH_2CH{=}CH_2$ | Et | H | H | O | H | Gb |
| $CH_2OCH_2C{\equiv}CH$ | Et | H | H | O | H | Gb |
| $CH{=}CHCO_2Me$ | Et | H | H | O | H | Ga |
| $CH{=}CHCO_2Et$ | Et | H | H | O | H | Ga |
| $CH{=}CHCO_2Pr$-n | Et | H | H | O | H | Gb |
| CH=CHCN | Et | H | H | O | H | Ga |
| Ph | Et | H | H | O | H | Ga |
| $CH_2Ph$ | Et | H | H | O | H | Ga |
| OPh | Et | H | H | O | H | Ga |
| SPh | Et | H | H | O | H | Ga |
| $SO_2Ph$ | Et | H | H | O | H | Ga |

| $R_1$ | $R_2$ | $R_3$ | $R_4$ | X | M | Gn |
|---|---|---|---|---|---|---|
| CONHMe | Et | H | H | O | H | Ga |
| CONHEt | Et | H | H | O | H | Ga |
| $CON(Me)_2$ | Et | H | H | O | H | Ga |
| $CON(Et)_2$ | Et | H | H | O | H | Ga |
| F | H | Pr-n | H | O | H | Ga |
| Cl | H | Pr-n | H | O | H | Ga |
| Br | H | Pr-n | H | O | H | Ga |
| I | H | Pr-n | H | O | H | Ga |
| $CH=CH_2$ | H | Pr-n | H | O | H | Ga |
| $CH_2CH=CH_2$ | H | Pr-n | H | O | H | Ga |
| $C\equiv CH$ | H | Pr-n | H | O | H | Ga |
| $CH_2C\equiv CH$ | H | Pr-n | H | O | H | Ga |
| $CH_2F$ | H | Pr-n | H | O | H | Ga |
| $CHF_2$ | H | Pr-n | H | O | H | Ga |
| CN | H | Pr-n | H | O | H | Ga |
| $NH_2$ | H | Pr-n | H | O | H | Ga |
| NHCOMe | H | Pr-n | H | O | H | Ga |
| $CH_2NH_2$ | H | Pr-n | H | O | H | Ga |
| $CH_2NHCOMe$ | H | Pr-n | H | O | H | Ga |
| $CH_2OCHF_2$ | H | Pr-n | H | O | H | Ga |
| $CF_3$ | H | Pr-n | H | O | H | Ga |
| $NO_2$ | H | Pr-n | H | O | H | Gb |
| OMe | H | Pr-n | H | O | H | Ga |
| OEt | H | Pr-n | H | O | H | Ga |
| OPr-n | H | Pr-n | H | O | H | Gb |
| SMe | H | Pr-n | H | O | H | Ga |
| SEt | H | Pr-n | H | O | H | Ga |
| $SO_2Me$ | H | Pr-n | H | O | H | Ga |
| $SO_2Et$ | H | Pr-n | H | O | H | Ga |
| COMe | H | Pr-n | H | O | H | Ga |
| COEt | H | Pr-n | H | O | H | Ga |
| $CO_2H$ | H | Pr-n | H | O | H | Gb |
| $CO_2Me$ | H | Pr-n | H | O | H | Ga |
| $CO_2Et$ | H | Pr-n | H | O | H | Ga |
| $CO_2Pr$-n | H | Pr-n | H | O | H | Gb |
| $CO_2Bu$-n | H | Pr-n | H | O | H | Gc |
| $CH_2Cl$ | H | Pr-n | H | O | H | Ga |
| $CH_2Br$ | H | Pr-n | H | O | H | Gb |
| $CH_2I$ | H | Pr-n | H | O | H | Gb |
| $CH_2CF_3$ | H | Pr-n | H | O | H | Ga |

| $R_1$ | $R_2$ | $R_3$ | $R_4$ | X | M | Gn |
|---|---|---|---|---|---|---|
| $CH_2CN$ | H | Pr-n | H | O | H | Ga |
| $CH_2OH$ | H | Pr-n | H | O | H | Gb |
| $CH_2OMe$ | H | Pr-n | H | O | H | Ga |
| $CH_2OEt$ | H | Pr-n | H | O | H | Ga |
| $CH_2OPr$-n | H | Pr-n | H | O | H | Gb |
| $CH_2SMe$ | H | Pr-n | H | O | H | Ga |
| $CH_2SEt$ | H | Pr-n | H | O | H | Ga |
| $CH_2SPr$-n | H | Pr-n | H | O | H | Gb |
| $CH_2SO_2Me$ | H | Pr-n | H | O | H | Ga |
| $CH_2SO_2Et$ | H | Pr-n | H | O | H | Ga |
| $CH_2COMe$ | H | Pr-n | H | O | H | Ga |
| $CH_2COEt$ | H | Pr-n | H | O | H | Ga |
| $CH_2COPr$-n | H | Pr-n | H | O | H | Gb |
| $CH_2CO_2H$ | H | Pr-n | H | O | H | Gb |
| $CH_2CO_2Me$ | H | Pr-n | H | O | H | Ga |
| $CH_2CO_2Et$ | H | Pr-n | H | O | H | Ga |
| $CH_2CO_2Pr$-n | H | Pr-n | H | O | H | Gb |
| $CH_2OCH_2CH{=}CH_2$ | H | Pr-n | H | O | H | Gb |
| $CH_2OCH_2C{\equiv}CH$ | H | Pr-n | H | O | H | Gb |
| $CH{=}CHCO_2Me$ | H | Pr-n | H | O | H | Ga |
| $CH{=}CHCO_2Et$ | H | Pr-n | H | O | H | Ga |
| $CH{=}CHCO_2Pr$-n | H | Pr-n | H | O | H | Gb |
| $CH{=}CHCN$ | H | Pr-n | H | O | H | Ga |
| Ph | H | Pr-n | H | O | H | Ga |
| $CH_2Ph$ | H | Pr-n | H | O | H | Ga |
| OPh | H | Pr-n | H | O | H | Ga |
| SPh | H | Pr-n | H | O | H | Ga |
| $SO_2Ph$ | H | Pr-n | H | O | H | Ga |
| CONHMe | H | Pr-n | H | O | H | Ga |
| CONHEt | H | Pr-n | H | O | H | Ga |
| $CON(Me)_2$ | H | Pr-n | H | O | H | Ga |
| $CON(Et)_2$ | H | Pr-n | H | O | H | Ga |
| F | H | Cl | H | O | H | Ga |
| Cl | H | Cl | H | O | H | Ga |
| Br | H | Cl | H | O | H | Ga |
| I | H | Cl | H | O | H | Ga |
| $CH{=}CH_2$ | H | Cl | H | O | H | Ga |
| $CH_2CH{=}CH_2$ | H | Cl | H | O | H | Ga |
| $C{\equiv}CH$ | H | Cl | H | O | H | Ga |
| $CH_2C{\equiv}CH$ | H | Cl | H | O | H | Ga |

| $R_1$ | $R_2$ | $R_3$ | $R_4$ | X | M | Gn |
|---|---|---|---|---|---|---|
| $CH_2F$ | H | Cl | H | O | H | Ga |
| $CHF_2$ | H | Cl | H | O | H | Ga |
| CN | H | Cl | H | O | H | Ga |
| $NH_2$ | H | Cl | H | O | H | Ga |
| NHCOMe | H | Cl | H | O | H | Ga |
| $CH_2NH_2$ | H | Cl | H | O | H | Ga |
| $CH_2NHCOMe$ | H | Cl | H | O | H | Ga |
| $CH_2OCHF_2$ | H | Cl | H | O | H | Ga |
| $CF_3$- | H | Cl | H | O | H | Ga |
| $NO_2$ | H | Cl | H | O | H | Gb |
| OMe | H | Cl | H | O | H | Ga |
| OEt | H | Cl | H | O | H | Ga |
| OPr-n | H | Cl | H | O | H | Gb |
| SMe | H | Cl | H | O | H | Ga |
| SEt | H | Cl | H | O | H | Ga |
| $SO_2Me$ | H | Cl | H | O | H | Ga |
| $SO_2Et$ | H | Cl | H | O | H | Ga |
| COMe | H | Cl | H | O | H | Ga |
| COEt | H | Cl | H | O | H | Ga |
| $CO_2H$ | H | Cl | H | O | H | Gb |
| $CO_2Me$ | H | Cl | H | O | H | Ga |
| $CO_2Et$ | H | Cl | H | O | H | Ga |
| $CO_2Pr$-n | H | Cl | H | O | H | Gb |
| $CO_2Bu$-n | H | Cl | H | O | H | Gc |
| $CH_2Cl$ | H | Cl | H | O | H | Ga |
| $CH_2Br$ | H | Cl | H | O | H | Gb |
| $CH_2I$ | H | Cl | H | O | H | Gb |
| $CH_2CF_3$ | H | Cl | H | O | H | Ga |
| $CH_2CN$ | H | Cl | H | O | H | Ga |
| $CH_2OH$ | H | Cl | H | O | H | Gb |
| $CH_2OMe$ | H | Cl | H | O | H | Ga |
| $CH_2OEt$ | H | Cl | H | O | H | Ga |
| $CH_2OPr$-n | H | Cl | H | O | H | Gb |
| $CH_2SMe$ | H | Cl | H | O | H | Ga |
| $CH_2SEt$ | H | Cl | H | O | H | Ga |
| $CH_2SPr$-n | H | Cl | H | O | H | Gb |
| $CH_2SO_2Me$ | H | Cl | H | O | H | Ga |
| $CH_2SO_2Et$ | H | Cl | H | O | H | Ga |
| $CH_2COMe$ | H | Cl | H | O | H | Ga |
| $CH_2COEt$ | H | Cl | H | O | H | Ga |

| $R_1$ | $R_2$ | $R_3$ | $R_4$ | X | M | Gn |
|---|---|---|---|---|---|---|
| $CH_2COPr$-n | H | Cl | H | O | H | Gb |
| $CH_2CO_2H$ | H | Cl | H | O | H | Gb |
| $CH_2CO_2Me$ | H | Cl | H | O | H | Ga |
| $CH_2CO_2Et$ | H | Cl | H | O | H | Ga |
| $CH_2CO_2Pr$-n | H | Cl | H | O | H | Gb |
| $CH_2OCH_2CH=CH_2$ | H | Cl | H | O | H | Gb |
| $CH_2OCH_2C\equiv CH$ | H | Cl | H | O | H | Gb |
| $CH=CHCO_2Me$ | H | Cl | H | O | H | Ga |
| $CH=CHCO_2Et$ | H | Cl | H | O | H | Ga |
| $CH=CHCO_2Pr$-n | H | Cl | H | O | H | Gb |
| CH=CHCN | H | Cl | H | O | H | Ga |
| Ph | H | Cl | H | O | H | Ga |
| $CH_2Ph$ | H | Cl | H | O | H | Ga |
| OPh | H | Cl | H | O | H | Ga |
| SPh | H | Cl | H | O | H | Ga |
| $SO_2Ph$ | H | Cl | H | O | H | Ga |
| CONHMe | H | Cl | H | O | H | Ga |
| CONHEt | H | Cl | H | O | H | Ga |
| $CON(Me)_2$ | H | Cl | H | O | H | Ga |
| $CON(Et)_2$ | H | Cl | H | O | H | Ga |
| F | H | $NO_2$ | H | O | H | Ga |
| Cl | H | $NO_2$ | H | O | H | Ga |
| Br | H | $NO_2$ | H | O | H | Ga |
| I | H | $NO_2$ | H | O | H | Ga |
| $CH=CH_2$ | H | $NO_2$ | H | O | H | Ga |
| $CH_2CH=CH_2$ | H | $NO_2$ | H | O | H | Ga |
| $C\equiv CH$ | H | $NO_2$ | H | O | H | Ga |
| $CH_2C\equiv CH$ | H | $NO_2$ | H | O | H | Ga |
| $CH_2F$ | H | $NO_2$ | H | O | H | Ga |
| $CHF_2$ | H | $NO_2$ | H | O | H | Ga |
| CN | H | $NO_2$ | H | O | H | Ga |
| $NH_2$ | H | $NO_2$ | H | O | H | Ga |
| NHCOMe | H | $NO_2$ | H | O | H | Ga |
| $CH_2NH_2$ | H | $NO_2$ | H | O | H | Ga |
| $CH_2NHCOMe$ | H | $NO_2$ | H | O | H | Ga |
| $CH_2OCHF_2$ | H | $NO_2$ | H | O | H | Ga |
| $CF_3$ | H | $NO_2$ | H | O | H | Ga |
| $NO_2$ | H | $NO_2$ | H | O | H | Gb |
| OMe | H | $NO_2$ | H | O | H | Ga |
| OEt | H | $NO_2$ | H | O | H | Ga |

| $R_1$ | $R_2$ | $R_3$ | $R_4$ | X | M | Gn |
|---|---|---|---|---|---|---|
| OPr-n | H | $NO_2$ | H | O | H | Gb |
| SMe | H | $NO_2$ | H | O | H | Ga |
| SEt | H | $NO_2$ | H | O | H | Ga |
| $SO_2Me$ | H | $NO_2$ | H | O | H | Ga |
| $SO_2Et$ | H | $NO_2$ | H | O | H | Ga |
| COMe | H | $NO_2$ | H | O | H | Ga |
| COEt | H | $NO_2$ | H | O | H | Ga |
| $CO_2H$ | H | $NO_2$ | H | O | H | Gb |
| $CO_2Me$ | H | $NO_2$ | H | O | H | Ga |
| $CO_2Et$ | H | $NO_2$ | H | O | H | Ga |
| $CO_2Pr$-n | H | $NO_2$ | H | O | H | Gb |
| $CO_2Bu$-n | H | $NO_2$ | H | O | H | Gc |
| $CH_2Cl$ | H | $NO_2$ | H | O | H | Ga |
| $CH_2Br$ | H | $NO_2$ | H | O | H | Gb |
| $CH_2I$ | H | $NO_2$ | H | O | H | Gb |
| $CH_2CF_3$ | H | $NO_2$ | H | O | H | Ga |
| $CH_2CN$ | H | $NO_2$ | H | O | H | Ga |
| $CH_2OH$ | H | $NO_2$ | H | O | H | Gb |
| $CH_2OMe$ | H | $NO_2$ | H | O | H | Ga |
| $CH_2OEt$ | H | $NO_2$ | H | O | H | Ga |
| $CH_2OPr$-n | H | $NO_2$ | H | O | H | Gb |
| $CH_2SMe$ | H | $NO_2$ | H | O | H | Ga |
| $CH_2SEt$ | H | $NO_2$ | H | O | H | Ga |
| $CH_2SPr$-n | H | $NO_2$ | H | O | H | Gb |
| $CH_2SO_2Me$ | H | $NO_2$ | H | O | H | Ga |
| $CH_2SO_2Et$ | H | $NO_2$ | H | O | H | Ga |
| $CH_2COMe$ | H | $NO_2$ | H | O | H | Ga |
| $CH_2COEt$ | H | $NO_2$ | H | O | H | Ga |
| $CH_2COPr$-n | H | $NO_2$ | H | O | H | Gb |
| $CH_2CO_2H$ | H | $NO_2$ | H | O | H | Gb |
| $CH_2CO_2Me$ | H | $NO_2$ | H | O | H | Ga |
| $CH_2CO_2Et$ | H | $NO_2$ | H | O | H | Ga |
| $CH_2CO_2Pr$-n | H | $NO_2$ | H | O | H | Gb |
| $CH_2OCH_2CH=CH_2$ | H | $NO_2$ | H | O | H | Gb |
| $CH_2OCH_2C\equiv CH$ | H | $NO_2$ | H | O | H | Gb |
| $CH=CHCO_2Me$ | H | $NO_2$ | H | O | H | Ga |
| $CH=CHCO_2Et$ | H | $NO_2$ | H | O | H | Ga |
| $CH=CHCO_2Pr$-n | H | $NO_2$ | H | O | H | Gb |
| CH=CHCN | H | $NO_2$ | H | O | H | Ga |
| Ph | H | $NO_2$ | H | O | H | Ga |

| $R_1$ | $R_2$ | $R_3$ | $R_4$ | X | M | Gn |
|---|---|---|---|---|---|---|
| $CH_2Ph$ | H | $NO_2$ | H | O | H | Ga |
| OPh | H | $NO_2$ | H | O | H | Ga |
| SPh | H | $NO_2$ | H | O | H | Ga |
| $SO_2Ph$ | H | $NO_2$ | H | O | H | Ga |
| CONHMe | H | $NO_2$ | H | O | H | Ga |
| CONHEt | H | $NO_2$ | H | O | H | Ga |
| $CON(Me)_2$ | H | $NO_2$ | H | O | H | Ga |
| $CON(Et)_2$ | H | $NO_2$ | H | O | H | Ga |
| F | H | $CF_3$ | H | O | H | Ga |
| Cl | H | $CF_3$ | H | O | H | Ga |
| Br | H | $CF_3$ | H | O | H | Ga |
| I | H | $CF_3$ | H | O | H | Ga |
| $CH=CH_2$ | H | $CF_3$ | H | O | H | Ga |
| $CH_2CH=CH_2$ | H | $CF_3$ | H | O | H | Ga |
| $C\equiv CH$ | H | $CF_3$ | H | O | H | Ga |
| $CH_2C\equiv CH$ | H | $CF_3$ | H | O | H | Ga |
| $CH_2F$ | H | $CF_3$ | H | O | H | Ga |
| $CHF_2$ | H | $CF_3$ | H | O | H | Ga |
| CN | H | $CF_3$ | H | O | H | Ga |
| $NH_2$ | H | $CF_3$ | H | O | H | Ga |
| NHCOMe | H | $CF_3$ | H | O | H | Ga |
| $CH_2NH_2$ | H | $CF_3$ | H | O | H | Ga |
| $CH_2NHCOMe$ | H | $CF_3$ | H | O | H | Ga |
| $CH_2OCHF_2$ | H | $CF_3$ | H | O | H | Ga |
| $CF_3$ | H | $CF_3$ | H | O | H | Ga |
| $NO_2$ | H | $CF_3$ | H | O | H | Gb |
| OMe | H | $CF_3$ | H | O | H | Ga |
| OEt | H | $CF_3$ | H | O | H | Ga |
| OPr-n | H | $CF_3$ | H | O | H | Gb |
| SMe | H | $CF_3$ | H | O | H | Ga |
| SEt | H | $CF_3$ | H | O | H | Ga |
| $SO_2Me$ | H | $CF_3$ | H | O | H | Ga |
| $SO_2Et$ | H | $CF_3$ | H | O | H | Ga |
| COMe | H | $CF_3$ | H | O | H | Ga |
| COEt | H | $CF_3$ | H | O | H | Ga |
| $CO_2H$ | H | $CF_3$ | H | O | H | Gb |
| $CO_2Me$ | H | $CF_3$ | H | O | H | Ga |
| $CO_2Et$ | H | $CF_3$ | H | O | H | Ga |
| $CO_2Pr$-n | H | $CF_3$ | H | O | H | Gb |
| $CO_2Bu$-n | H | $CF_3$ | H | O | H | Gc |

| $R_1$ | $R_2$ | $R_3$ | $R_4$ | X | M | Gn |
|---|---|---|---|---|---|---|
| $CH_2Cl$ | H | $CF_3$ | H | O | H | Ga |
| $CH_2Br$ | H | $CF_3$ | H | O | H | Gb |
| $CH_2I$ | H | $CF_3$ | H | O | H | Gb |
| $CH_2CF_3$ | H | $CF_3$ | H | O | H | Ga |
| $CH_2CN$ | H | $CF_3$ | H | O | H | Ga |
| $CH_2OH$ | H | $CF_3$ | H | O | H | Gb |
| $CH_2OMe$ | H | $CF_3$ | H | O | H | Ga |
| $CH_2OEt$ | H | $CF_3$ | H | O | H | Ga |
| $CH_2OPr$-n | H | $CF_3$ | H | O | H | Gb |
| $CH_2SMe$ | H | $CF_3$ | H | O | H | Ga |
| $CH_2SEt$ | H | $CF_3$ | H | O | H | Ga |
| $CH_2SPr$-n | H | $CF_3$ | H | O | H | Gb |
| $CH_2SO_2Me$ | H | $CF_3$ | H | O | H | Ga |
| $CH_2SO_2Et$ | H | $CF_3$ | H | O | H | Ga |
| $CH_2COMe$ | H | $CF_3$ | H | O | H | Ga |
| $CH_2COEt$ | H | $CF_3$ | H | O | H | Ga |
| $CH_2COPr$-n | H | $CF_3$ | H | O | H | Gb |
| $CH_2CO_2H$ | H | $CF_3$ | H | O | H | Gb |
| $CH_2CO_2Me$ | H | $CF_3$ | H | O | H | Ga |
| $CH_2CO_2Et$ | H | $CF_3$ | H | O | H | Ga |
| $CH_2CO_2Pr$-n | H | $CF_3$ | H | O | H | Gb |
| $CH_2OCH_2CH=CH_2$ | H | $CF_3$ | H | O | H | Gb |
| $CH_2OCH_2C\equiv CH$ | H | $CF_3$ | H | O | H | Gb |
| $CH=CHCO_2Me$ | H | $CF_3$ | H | O | H | Ga |
| $CH=CHCO_2Et$ | H | $CF_3$ | H | O | H | Ga |
| $CH=CHCO_2Pr$-n | H | $CF_3$ | H | O | H | Gb |
| $CH=CHCN$ | H | $CF_3$ | H | O | H | Ga |
| Ph | H | $CF_3$ | H | O | H | Ga |
| $CH_2Ph$ | H | $CF_3$ | H | O | H | Ga |
| OPh | H | $CF_3$ | H | O | H | Ga |
| SPh | H | $CF_3$ | H | O | H | Ga |
| $SO_2Ph$ | H | $CF_3$ | H | O | H | Ga |
| CONHMe | H | $CF_3$ | H | O | H | Ga |
| CONHEt | H | $CF_3$ | H | O | H | Ga |
| $CON(Me)_2$ | H | $CF_3$ | H | O | H | Ga |
| $CON(Et)_2$ | H | $CF_3$ | H | O | H | Ga |
| F | H | OMe | H | O | H | Ga |
| Cl | H | OMe | H | O | H | Ga |
| Br | H | OMe | H | O | H | Ga |
| I | H | OMe | H | O | H | Ga |

| $R_1$ | $R_2$ | $R_3$ | $R_4$ | X | M | Gn |
|---|---|---|---|---|---|---|
| $CH=CH_2$ | H | OMe | H | O | H | Ga |
| $CH_2CH=CH_2$ | H | OMe | H | O | H | Ga |
| $C\equiv CH$ | H | OMe | H | O | H | Ga |
| $CH_2C\equiv CH$ | H | OMe | H | O | H | Ga |
| $CH_2F$ | H | OMe | H | O | H | Ga |
| $CHF_2$ | H | OMe | H | O | H | Ga |
| CN | H | OMe | H | O | H | Ga |
| $NH_2$ | H | OMe | H | O | H | Ga |
| NHCOMe | H | OMe | H | O | H | Ga |
| $CH_2NH_2$ | H | OMe | H | O | H | Ga |
| $CH_2NHCOMe$ | H | OMe | H | O | H | Ga |
| $CH_2OCHF_2$ | H | OMe | H | O | H | Ga |
| $CF_3$ | H | OMe | H | O | H | Ga |
| $NO_2$ | H | OMe | H | O | H | Gb |
| OMe | H | OMe | H | O | H | Ga |
| OEt | H | OMe | H | O | H | Ga |
| OPr-n | H | OMe | H | O | H | Gb |
| SMe | H | OMe | H | O | H | Ga |
| SEt | H | OMe | H | O | H | Ga |
| $SO_2Me$ | H | OMe | H | O | H | Ga |
| $SO_2Et$ | H | OMe | H | O | H | Ga |
| COMe | H | OMe | H | O | H | Ga |
| COEt | H | OMe | H | O | H | Ga |
| $CO_2H$ | H | OMe | H | O | H | Gb |
| $CO_2Me$ | H | OMe | H | O | H | Ga |
| $CO_2Et$ | H | OMe | H | O | H | Ga |
| $CO_2Pr$-n | H | OMe | H | O | H | Gb |
| $CO_2Bu$-n | H | OMe | H | O | H | Gc |
| $CH_2Cl$ | H | OMe | H | O | H | Ga |
| $CH_2Br$ | H | OMe | H | O | H | Gb |
| $CH_2I$ | H | OMe | H | O | H | Gb |
| $CH_2CF_3$ | H | OMe | H | O | H | Ga |
| $CH_2CN$ | H | OMe | H | O | H | Ga |
| $CH_2OH$ | H | OMe | H | O | H | Gb |
| $CH_2OMe$ | H | OMe | H | O | H | Ga |
| $CH_2OEt$ | H | OMe | H | O | H | Ga |
| $CH_2OPr$-n | H | OMe | H | O | H | Gb |
| $CH_2SMe$ | H | OMe | H | O | H | Ga |
| $CH_2SEt$ | H | OMe | H | O | H | Ga |
| $CH_2SPr$-n | H | OMe | H | O | H | Gb |

| $R_1$ | $R_2$ | $R_3$ | $R_4$ | X | M | Gn |
|---|---|---|---|---|---|---|
| $CH_2SO_2Me$ | H | OMe | H | O | H | Ga |
| $CH_2SO_2Et$ | H | OMe | H | O | H | Ga |
| $CH_2COMe$ | H | OMe | H | O | H | Ga |
| $CH_2COEt$ | H | OMe | H | O | H | Ga |
| $CH_2COPr$-n | H | OMe | H | O | H | Gb |
| $CH_2CO_2H$ | H | OMe | H | O | H | Gb |
| $CH_2CO_2Me$ | H | OMe | H | O | H | Ga |
| $CH_2CO_2Et$ | H | OMe | H | O | H | Ga |
| $CH_2CO_2Pr$-n | H | OMe | H | O | H | Gb |
| $CH_2OCH_2CH=CH_2$ | H | OMe | H | O | H | Gb |
| $CH_2OCH_2C\equiv CH$ | H | OMe | H | O | H | Gb |
| $CH=CHCO_2Me$ | H | OMe | H | O | H | Ga |
| $CH=CHCO_2Et$ | H | OMe | H | O | H | Ga |
| $CH=CHCO_2Pr$-n | H | OMe | H | O | H | Gb |
| CH=CHCN | H | OMe | H | O | H | Ga |
| Ph | H | OMe | H | O | H | Ga |
| $CH_2Ph$ | H | OMe | H | O | H | Ga |
| OPh | H | OMe | H | O | H | Ga |
| SPh | H | OMe | H | O | H | Ga |
| $SO_2Ph$ | H | OMe | H | O | H | Ga |
| CONHMe | H | OMe | H | O | H | Ga |
| CONHEt | H | OMe | H | O | H | Ga |
| $CON(Me)_2$ | H | OMe | H | O | H | Ga |
| $CON(Et)_2$ | H | OMe | H | O | H | Ga |
| F | H | SMe | H | O | H | Ga |
| Cl | H | SMe | H | O | H | Ga |
| Br | H | SMe | H | O | H | Ga |
| I | H | SMe | H | O | H | Ga |
| $CH=CH_2$ | H | SMe | H | O | H | Ga |
| $CH_2CH=CH_2$ | H | SMe | H | O | H | Ga |
| $C\equiv CH$ | H | SMe | H | O | H | Ga |
| $CH_2C\equiv CH$ | H | SMe | H | O | H | Ga |
| $CH_2F$ | H | SMe | H | O | H | Ga |
| $CHF_2$ | H | SMe | H | O | H | Ga |
| CN | H | SMe | H | O | H | Ga |
| $NH_2$ | H | SMe | H | O | H | Ga |
| NHCOMe | H | SMe | H | O | H | Ga |
| $CH_2NH_2$ | H | SMe | H | O | H | Ga |
| $CH_2NHCOMe$ | H | SMe | H | O | H | Ga |
| $CH_2OCHF_2$ | H | SMe | H | O | H | Ga |

| $R_1$ | $R_2$ | $R_3$ | $R_4$ | X | M | Gn |
|---|---|---|---|---|---|---|
| $CF_3$ | H | SMe | H | O | H | Ga |
| $NO_2$ | H | SMe | H | O | H | Gb |
| OMe | H | SMe | H | O | H | Ga |
| OEt | H | SMe | H | O | H | Ga |
| OPr-n | H | SMe | H | O | H | Gb |
| SMe | H | SMe | H | O | H | Ga |
| SEt | H | SMe | H | O | H | Ga |
| $SO_2Me$ | H | SMe | H | O | H | Ga |
| $SO_2Et$ | H | SMe | H | O | H | Ga |
| COMe | H | SMe | H | O | H | Ga |
| COEt | H | SMe | H | O | H | Ga |
| $CO_2H$ | H | SMe | H | O | H | Gb |
| $CO_2Me$ | H | SMe | H | O | H | Ga |
| $CO_2Et$ | H | SMe | H | O | H | Ga |
| $CO_2Pr$-n | H | SMe | H | O | H | Gb |
| $CO_2Bu$-n | H | SMe | H | O | H | Gc |
| $CH_2Cl$ | H | SMe | H | O | H | Ga |
| $CH_2Br$ | H | SMe | H | O | H | Gb |
| $CH_2I$ | H | SMe | H | O | H | Gb |
| $CH_2CF_3$ | H | SMe | H | O | H | Ga |
| $CH_2CN$ | H | SMe | H | O | H | Ga |
| $CH_2OH$ | H | SMe | H | O | H | Gb |
| $CH_2OMe$ | H | SMe | H | O | H | Ga |
| $CH_2OEt$ | H | SMe | H | O | H | Ga |
| $CH_2OPr$-n | H | SMe | H | O | H | Gb |
| $CH_2SMe$ | H | SMe | H | O | H | Ga |
| $CH_2SEt$ | H | SMe | H | O | H | Ga |
| $CH_2SPr$-n | H | SMe | H | O | H | Gb |
| $CH_2SO_2Me$ | H | SMe | H | O | H | Ga |
| $CH_2SO_2Et$ | H | SMe | H | O | H | Ga |
| $CH_2COMe$ | H | SMe | H | O | H | Ga |
| $CH_2COEt$ | H | SMe | H | O | H | Ga |
| $CH_2COPr$-n | H | SMe | H | O | H | Gb |
| $CH_2CO_2H$ | H | SMe | H | O | H | Gb |
| $CH_2CO_2Me$ | H | SMe | H | O | H | Ga |
| $CH_2CO_2Et$ | H | SMe | H | O | H | Ga |
| $CH_2CO_2Pr$-n | H | SMe | H | O | H | Gb |
| $CH_2OCH_2CH=CH_2$ | H | SMe | H | O | H | Gb |
| $CH_2OCH_2C\equiv CH$ | H | SMe | H | O | H | Gb |
| $CH=CHCO_2Me$ | H | SMe | H | O | H | Ga |

| $R_1$ | $R_2$ | $R_3$ | $R_4$ | X | M | Gn |
|---|---|---|---|---|---|---|
| $CH=CHCO_2Et$ | H | SMe | H | O | H | Ga |
| $CH=CHCO_2Pr$-n | H | SMe | H | O | H | Gb |
| CH=CHCN | H | SMe | H | O | H | Ga |
| Ph | H | SMe | H | O | H | Ga |
| $CH_2Ph$ | H | SMe | H | O | H | Ga |
| OPh | H | SMe | H | O | H | Ga |
| SPh | H | SMe | H | O | H | Ga |
| $SO_2Ph$ | H | SMe | H | O | H | Ga |
| CONHMe | H | SMe | H | O | H | Ga |
| CONHEt | H | SMe | H | O | H | Ga |
| $CON(Me)_2$ | H | SMe | H | O | H | Ga |
| $CON(Et)_2$ | H | SMe | H | O | H | Ga |
| F | H | $SO_2Me$ | H | O | H | Ga |
| Cl | H | $SO_2Me$ | H | O | H | Ga |
| Br | H | $SO_2Me$ | H | O | H | Ga |
| I | H | $SO_2Me$ | H | O | H | Ga |
| $CH=CH_2$ | H | $SO_2Me$ | H | O | H | Ga |
| $CH_2CH=CH_2$ | H | $SO_2Me$ | H | O | H | Ga |
| $C\equiv CH$ | H | $SO_2Me$ | H | O | H | Ga |
| $CH_2C\equiv CH$ | H | $SO_2Me$ | H | O | H | Ga |
| $CH_2F$ | H | $SO_2Me$ | H | O | H | Ga |
| $CHF_2$ | H | $SO_2Me$ | H | O | H | Ga |
| CN | H | $SO_2Me$ | H | O | H | Ga |
| $NH_2$ | H | $SO_2Me$ | H | O | H | Ga |
| NHCOMe | H | $SO_2Me$ | H | O | H | Ga |
| $CH_2NH_2$ | H | $SO_2Me$ | H | O | H | Ga |
| $CH_2NHCOMe$ | H | $SO_2Me$ | H | O | H | Ga |
| $CH_2OCHF_2$ | H | $SO_2Me$ | H | O | H | Ga |
| $CF_3$ | H | $SO_2Me$ | H | O | H | Ga |
| $NO_2$ | H | $SO_2Me$ | H | O | H | Gb |
| OMe | H | $SO_2Me$ | H | O | H | Ga |
| OEt | H | $SO_2Me$ | H | O | H | Ga |
| OPr-n | H | $SO_2Me$ | H | O | H | Gb |
| SMe | H | $SO_2Me$ | H | O | H | Ga |
| SEt | H | $SO_2Me$ | H | O | H | Ga |
| $SO_2Me$ | H | $SO_2Me$ | H | O | H | Ga |
| $SO_2Et$ | H | $SO_2Me$ | H | O | H | Ga |
| COMe | H | $SO_2Me$ | H | O | H | Ga |
| COEt | H | $SO_2Me$ | H | O | H | Ga |
| $CO_2H$ | H | $SO_2Me$ | H | O | H | Gb |

| $R_1$ | $R_2$ | $R_3$ | $R_4$ | X | M | Gn |
|---|---|---|---|---|---|---|
| $CO_2Me$ | H | $SO_2Me$ | H | O | H | Ga |
| $CO_2Et$ | H | $SO_2Me$ | H | O | H | Ga |
| $CO_2Pr$-n | H | $SO_2Me$ | H | O | H | Gb |
| $CO_2Bu$-n | H | $SO_2Me$ | H | O | H | Gc |
| $CH_2Cl$ | H | $SO_2Me$ | H | O | H | Ga |
| $CH_2Br$ | H | $SO_2Me$ | H | O | H | Gb |
| $CH_2I$ | H | $SO_2Me$ | H | O | H | Gb |
| $CH_2CF_3$ | H | $SO_2Me$ | H | O | H | Ga |
| $CH_2CN$ | H | $SO_2Me$ | H | O | H | Ga |
| $CH_2OH$ | H | $SO_2Me$ | H | O | H | Gb |
| $CH_2OMe$ | H | $SO_2Me$ | H | O | H | Ga |
| $CH_2OEt$ | H | $SO_2Me$ | H | O | H | Ga |
| $CH_2OPr$-n | H | $SO_2Me$ | H | O | H | Gb |
| $CH_2SMe$ | H | $SO_2Me$ | H | O | H | Ga |
| $CH_2SEt$ | H | $SO_2Me$ | H | O | H | Ga |
| $CH_2SPr$-n | H | $SO_2Me$ | H | O | H | Gb |
| $CH_2SO_2Me$ | H | $SO_2Me$ | H | O | H | Ga |
| $CH_2SO_2Et$ | H | $SO_2Me$ | H | O | H | Ga |
| $CH_2COMe$ | H | $SO_2Me$ | H | O | H | Ga |
| $CH_2COEt$ | H | $SO_2Me$ | H | O | H | Ga |
| $CH_2COPr$-n | H | $SO_2Me$ | H | O | H | Gb |
| $CH_2CO_2H$ | H | $SO_2Me$ | H | O | H | Gb |
| $CH_2CO_2Me$ | H | $SO_2Me$ | H | O | H | Ga |
| $CH_2CO_2Et$ | H | $SO_2Me$ | H | O | H | Ga |
| $CH_2CO_2Pr$-n | H | $SO_2Me$ | H | O | H | Gb |
| $CH_2OCH_2CH=CH_2$ | H | $SO_2Me$ | H | O | H | Gb |
| $CH_2OCH_2C \equiv CH$ | H | $SO_2Me$ | H | O | H | Gb |
| $CH=CHCO_2Me$ | H | $SO_2Me$ | H | O | H | Ga |
| $CH=CHCO_2Et$ | H | $SO_2Me$ | H | O | H | Ga |
| $CH=CHCO_2Pr$-n | H | $SO_2Me$ | H | O | H | Gb |
| CH=CHCN | H | $SO_2Me$ | H | O | H | Ga |
| Ph | H | $SO_2Me$ | H | O | H | Ga |
| $CH_2Ph$ | H | $SO_2Me$ | H | O | H | Ga |
| OPh | H | $SO_2Me$ | H | O | H | Ga |
| SPh | H | $SO_2Me$ | H | O | H | Ga |
| $SO_2Ph$ | H | $SO_2Me$ | H | O | H | Ga |
| CONHMe | H | $SO_2Me$ | H | O | H | Ga |
| CONHEt | H | $SO_2Me$ | H | O | H | Ga |
| $CON(Me)_2$ | H | $SO_2Me$ | H | O | H | Ga |
| $CON(Et)_2$ | H | $SO_2Me$ | H | O | H | Ga |

| $R_1$ | $R_2$ | $R_3$ | $R_4$ | X | M | Gn |
|---|---|---|---|---|---|---|
| F | H | COMe | H | O | H | Ga |
| Cl | H | COMe | H | O | H | Ga |
| Br | H | COMe | H | O | H | Ga |
| I | H | COMe | H | O | H | Ga |
| $CH=CH_2$ | H | COMe | H | O | H | Ga |
| $CH_2CH=CH_2$ | H | COMe | H | O | H | Ga |
| $C\equiv CH$ | H | COMe | H | O | H | Ga |
| $CH_2C\equiv CH$ | H | COMe | H | O | H | Ga |
| $CH_2F$ | H | COMe | H | O | H | Ga |
| $CHF_2$ | H | COMe | H | O | H | Ga |
| CN | H | COMe | H | O | H | Ga |
| $NH_2$ | H | COMe | H | O | H | Ga |
| NHCOMe | H | COMe | H | O | H | Ga |
| $CH_2NH_2$ | H | COMe | H | O | H | Ga |
| $CH_2NHCOMe$ | H | COMe | H | O | H | Ga |
| $CH_2OCHF_2$ | H | COMe | H | O | H | Ga |
| $CF_3$ | H | COMe | H | O | H | Ga |
| $NO_2$ | H | COMe | H | O | H | Gb |
| OMe | H | COMe | H | O | H | Ga |
| OEt | H | COMe | H | O | H | Ga |
| OPr-n | H | COMe | H | O | H | Gb |
| SMe | H | COMe | H | O | H | Ga |
| SEt | H | COMe | H | O | H | Ga |
| $SO_2Me$ | H | COMe | H | O | H | Ga |
| $SO_2Et$ | H | COMe | H | O | H | Ga |
| COMe | H | COMe | H | O | H | Ga |
| COEt | H | COMe | H | O | H | Ga |
| $CO_2H$ | H | COMe | H | O | H | Gb |
| $CO_2Me$ | H | COMe | H | O | H | Ga |
| $CO_2Et$ | H | COMe | H | O | H | Ga |
| $CO_2Pr\text{-}n$ | H | COMe | H | O | H | Gb |
| $CO_2Bu\text{-}n$ | H | COMe | H | O | H | Gc |
| $CH_2Cl$ | H | COMe | H | O | H | Ga |
| $CH_2Br$ | H | COMe | H | O | H | Gb |
| $CH_2I$ | H | COMe | H | O | H | Gb |
| $CH_2CF_3$ | H | COMe | H | O | H | Ga |
| $CH_2CN$ | H | COMe | H | O | H | Ga |
| $CH_2OH$ | H | COMe | H | O | H | Gb |
| $CH_2OMe$ | H | COMe | H | O | H | Ga |
| $CH_2OEt$ | H | COMe | H | O | H | Ga |

| $R_1$ | $R_2$ | $R_3$ | $R_4$ | X | M | Gn |
|---|---|---|---|---|---|---|
| $CH_2OPr$-n | H | COMe | H | O | H | Gb |
| $CH_2SMe$ | H | COMe | H | O | H | Ga |
| $CH_2SEt$ | H | COMe | H | O | H | Ga |
| $CH_2SPr$-n | H | COMe | H | O | H | Gb |
| $CH_2SO_2Me$ | H | COMe | H | O | H | Ga |
| $CH_2SO_2Et$ | H | COMe | H | O | H | Ga |
| $CH_2COMe$ | H | COMe | H | O | H | Ga |
| $CH_2COEt$ | H | COMe | H | O | H | Ga |
| $CH_2COPr$-n | H | COMe | H | O | H | Gb |
| $CH_2CO_2H$ | H | COMe | H | O | H | Gb |
| $CH_2CO_2Me$ | H | COMe | H | O | H | Ga |
| $CH_2CO_2Et$ | H | COMe | H | O | H | Ga |
| $CH_2CO_2Pr$-n | H | COMe | H | O | H | Gb |
| $CH_2OCH_2CH=CH_2$ | H | COMe | H | O | H | Gb |
| $CH_2OCH_2C\equiv CH$ | H | COMe | H | O | H | Gb |
| $CH=CHCO_2Me$ | H | COMe | H | O | H | Ga |
| $CH=CHCO_2Et$ | H | COMe | H | O | H | Ga |
| $CH=CHCO_2Pr$-n | H | COMe | H | O | H | Gb |
| CH=CHCN | H | COMe | H | O | H | Ga |
| Ph | H | COMe | H | O | H | Ga |
| $CH_2Ph$ | H | COMe | H | O | H | Ga |
| OPh | H | COMe | H | O | H | Ga |
| SPh | H | COMe | H | O | H | Ga |
| $SO_2Ph$ | H | COMe | H | O | H | Ga |
| CONHMe | H | COMe | H | O | H | Ga |
| CONHEt | H | COMe | H | O | H | Ga |
| $CON(Me)_2$ | H | COMe | H | O | H | Ga |
| $CON(Et)_2$ | H | COMe | H | O | H | Ga |
| F | H | $CO_2Me$ | H | O | H | Ga |
| Cl | H | $CO_2Me$ | H | O | H | Ga |
| Br | H | $CO_2Me$ | H | O | H | Ga |
| I | H | $CO_2Me$ | H | O | H | Ga |
| $CH=CH_2$ | H | $CO_2Me$ | H | O | H | Ga |
| $CH_2CH=CH_2$ | H | $CO_2Me$ | H | O | H | Ga |
| $C\equiv CH$ | H | $CO_2Me$ | H | O | H | Ga |
| $CH_2C\equiv CH$ | H | $CO_2Me$ | H | O | H | Ga |
| $CH_2F$ | H | $CO_2Me$ | H | O | H | Ga |
| $CHF_2$ | H | $CO_2Me$ | H | O | H | Ga |
| CN | H | $CO_2Me$ | H | O | H | Ga |
| $NH_2$ | H | $CO_2Me$ | H | O | H | Ga |

| $R_1$ | $R_2$ | $R_3$ | $R_4$ | X | M | Gn |
|---|---|---|---|---|---|---|
| NHCOMe | H | $CO_2Me$ | H | O | H | Ga |
| $CH_2NH_2$ | H | $CO_2Me$ | H | O | H | Ga |
| $CH_2NHCOMe$ | H | $CO_2Me$ | H | O | H | Ga |
| $CH_2OCHF_2$ | H | $CO_2Me$ | H | O | H | Ga |
| $CF_3$ | H | $CO_2Me$ | H | O | H | Ga |
| $NO_2$ | H | $CO_2Me$ | H | O | H | Gb |
| OMe | H | $CO_2Me$ | H | O | H | Ga |
| OEt | H | $CO_2Me$ | H | O | H | Ga |
| OPr-n | H | $CO_2Me$ | H | O | H | Gb |
| SMe | H | $CO_2Me$ | H | O | H | Ga |
| SEt | H | $CO_2Me$ | H | O | H | Ga |
| $SO_2Me$ | H | $CO_2Me$ | H | O | H | Ga |
| $SO_2Et$ | H | $CO_2Me$ | H | O | H | Ga |
| COMe | H | $CO_2Me$ | H | O | H | Ga |
| COEt | H | $CO_2Me$ | H | O | H | Ga |
| $CO_2H$ | H | $CO_2Me$ | H | O | H | Gb |
| $CO_2Me$ | H | $CO_2Me$ | H | O | H | Ga |
| $CO_2Et$ | H | $CO_2Me$ | H | O | H | Ga |
| $CO_2Pr$-n | H | $CO_2Me$ | H | O | H | Gb |
| $CO_2Bu$-n | H | $CO_2Me$ | H | O | H | Gc |
| $CH_2Cl$ | H | $CO_2Me$ | H | O | H | Ga |
| $CH_2Br$ | H | $CO_2Me$ | H | O | H | Gb |
| $CH_2I$ | H | $CO_2Me$ | H | O | H | Gb |
| $CH_2CF_3$ | H | $CO_2Me$ | H | O | H | Ga |
| $CH_2CN$ | H | $CO_2Me$ | H | O | H | Ga |
| $CH_2OH$ | H | $CO_2Me$ | H | O | H | Gb |
| $CH_2OMe$ | H | $CO_2Me$ | H | O | H | Ga |
| $CH_2OEt$ | H | $CO_2Me$ | H | O | H | Ga |
| $CH_2OPr$-n | H | $CO_2Me$ | H | O | H | Gb |
| $CH_2SMe$ | H | $CO_2Me$ | H | O | H | Ga |
| $CH_2SEt$ | H | $CO_2Me$ | H | O | H | Ga |
| $CH_2SPr$-n | H | $CO_2Me$ | H | O | H | Gb |
| $CH_2SO_2Me$ | H | $CO_2Me$ | H | O | H | Ga |
| $CH_2SO_2Et$ | H | $CO_2Me$ | H | O | H | Ga |
| $CH_2COMe$ | H | $CO_2Me$ | H | O | H | Ga |
| $CH_2COEt$ | H | $CO_2Me$ | H | O | H | Ga |
| $CH_2COPr$-n | H | $CO_2Me$ | H | O | H | Gb |
| $CH_2CO_2H$ | H | $CO_2Me$ | H | O | H | Gb |
| $CH_2CO_2Me$ | H | $CO_2Me$ | H | O | H | Ga |
| $CH_2CO_2Et$ | H | $CO_2Me$ | H | O | H | Ga |

| $R_1$ | $R_2$ | $R_3$ | $R_4$ | X | M | Gn |
|---|---|---|---|---|---|---|
| $CH_2CO_2Pr$-n | H | $CO_2Me$ | H | O | H | Gb |
| $CH_2OCH_2CH=CH_2$ | H | $CO_2Me$ | H | O | H | Gb |
| $CH_2OCH_2C\equiv CH$ | H | $CO_2Me$ | H | O | H | Gb |
| $CH=CHCO_2Me$ | H | $CO_2Me$ | H | O | H | Ga |
| $CH=CHCO_2Et$ | H | $CO_2Me$ | H | O | H | Ga |
| $CH=CHCO_2Pr$-n | H | $CO_2Me$ | H | O | H | Gb |
| CH=CHCN | H | $CO_2Me$ | H | O | H | Ga |
| Ph | H | $CO_2Me$ | H | O | H | Ga |
| $CH_2Ph$ | H | $CO_2Me$ | H | O | H | Ga |
| OPh | H | $CO_2Me$ | H | O | H | Ga |
| SPh | H | $CO_2Me$ | H | O | H | Ga |
| $SO_2Ph$ | H | $CO_2Me$ | H | O | H | Ga |
| CONHMe | H | $CO_2Me$ | H | O | H | Ga |
| CONHEt | H | $CO_2Me$ | H | O | H | Ga |
| $CON(Me)_2$ | H | $CO_2Me$ | H | O | H | Ga |
| $CON(Et)_2$ | H | $CO_2Me$ | H | O | H | Ga |
| F | H | $CO_2H$ | H | O | H | Ga |
| Cl | H | $CO_2H$ | H | O | H | Ga |
| Br | H | $CO_2H$ | H | O | H | Ga |
| I | H | $CO_2H$ | H | O | H | Ga |
| $CH=CH_2$ | H | $CO_2H$ | H | O | H | Ga |
| $CH_2CH=CH_2$ | H | $CO_2H$ | H | O | H | Ga |
| $C\equiv CH$ | H | $CO_2H$ | H | O | H | Ga |
| $CH_2C\equiv CH$ | H | $CO_2H$ | H | O | H | Ga |
| $CH_2F$ | H | $CO_2H$ | H | O | H | Ga |
| $CHF_2$ | H | $CO_2H$ | H | O | H | Ga |
| CN | H | $CO_2H$ | H | O | H | Ga |
| $NH_2$ | H | $CO_2H$ | H | O | H | Ga |
| NHCOMe | H | $CO_2H$ | H | O | H | Ga |
| $CH_2NH_2$ | H | $CO_2H$ | H | O | H | Ga |
| $CH_2NHCOMe$ | H | $CO_2H$ | H | O | H | Ga |
| $CH_2OCHF_2$ | H | $CO_2H$ | H | O | H | Ga |
| $CF_3$ | H | $CO_2H$ | H | O | H | Ga |
| $NO_2$ | H | $CO_2H$ | H | O | H | Gb |
| OMe | H | $CO_2H$ | H | O | H | Ga |
| OEt | H | $CO_2H$ | H | O | H | Ga |
| OPr-n | H | $CO_2H$ | H | O | H | Gb |
| SMe | H | $CO_2H$ | H | O | H | Ga |
| SEt | H | $CO_2H$ | H | O | H | Ga |
| $SO_2Me$ | H | $CO_2H$ | H | O | H | Ga |

| $R_1$ | $R_2$ | $R_3$ | $R_4$ | X | M | Gn |
|---|---|---|---|---|---|---|
| $SO_2Et$ | H | $CO_2H$ | H | O | H | Ga |
| COMe | H | $CO_2H$ | H | O | H | Ga |
| COEt | H | $CO_2H$ | H | O | H | Ga |
| $CO_2H$ | H | $CO_2H$ | H | O | H | Gb |
| $CO_2Me$ | H | $CO_2H$ | H | O | H | Ga |
| $CO_2Et$ | H | $CO_2H$ | H | O | H | Ga |
| $CO_2Pr$-n | H | $CO_2H$ | H | O | H | Gb |
| $CO_2Bu$-n | H | $CO_2H$ | H | O | H | Gc |
| $CH_2Cl$ | H | $CO_2H$ | H | O | H | Ga |
| $CH_2Br$ | H | $CO_2H$ | H | O | H | Gb |
| $CH_2I$ | H | $CO_2H$ | H | O | H | Gb |
| $CH_2CF_3$ | H | $CO_2H$ | H | O | H | Ga |
| $CH_2CN$ | H | $CO_2H$ | H | O | H | Ga |
| $CH_2OH$ | H | $CO_2H$ | H | O | H | Gb |
| $CH_2OMe$ | H | $CO_2H$ | H | O | H | Ga |
| $CH_2OEt$ | H | $CO_2H$ | H | O | H | Ga |
| $CH_2OPr$-n | H | $CO_2H$ | H | O | H | Gb |
| $CH_2SMe$ | H | $CO_2H$ | H | O | H | Ga |
| $CH_2SEt$ | H | $CO_2H$ | H | O | H | Ga |
| $CH_2SPr$-n | H | $CO_2H$ | H | O | H | Gb |
| $CH_2SO_2Me$ | H | $CO_2H$ | H | O | H | Ga |
| $CH_2SO_2Et$ | H | $CO_2H$ | H | O | H | Ga |
| $CH_2COMe$ | H | $CO_2H$ | H | O | H | Ga |
| $CH_2COEt$ | H | $CO_2H$ | H | O | H | Ga |
| $CH_2COPr$-n | H | $CO_2H$ | H | O | H | Gb |
| $CH_2CO_2H$ | H | $CO_2H$ | H | O | H | Gb |
| $CH_2CO_2Me$ | H | $CO_2H$ | H | O | H | Ga |
| $CH_2CO_2Et$ | H | $CO_2H$ | H | O | H | Ga |
| $CH_2CO_2Pr$-n | H | $CO_2H$ | H | O | H | Gb |
| $CH_2OCH_2CH{=}CH_2$ | H | $CO_2H$ | H | O | H | Gb |
| $CH_2OCH_2C{\equiv}CH$ | H | $CO_2H$ | H | O | H | Gb |
| $CH{=}CHCO_2Me$ | H | $CO_2H$ | H | O | H | Ga |
| $CH{=}CHCO_2Et$ | H | $CO_2H$ | H | O | H | Ga |
| $CH{=}CHCO_2Pr$-n | H | $CO_2H$ | H | O | H | Gb |
| CH=CHCN | H | $CO_2H$ | H | O | H | Ga |
| Ph | H | $CO_2H$ | H | O | H | Ga |
| $CH_2Ph$ | H | $CO_2H$ | H | O | H | Ga |
| OPh | H | $CO_2H$ | H | O | H | Ga |
| SPh | H | $CO_2H$ | H | O | H | Ga |
| $SO_2Ph$ | H | $CO_2H$ | H | O | H | Ga |

| $R_1$ | $R_2$ | $R_3$ | $R_4$ | X | M | Gn |
|---|---|---|---|---|---|---|
| CONHMe | H | $CO_2H$ | H | O | H | Ga |
| CONHEt | H | $CO_2H$ | H | O | H | Ga |
| $CON(Me)_2$ | H | $CO_2H$ | H | O | H | Ga |
| $CON(Et)_2$ | H | $CO_2H$ | H | O | H | Ga |
| F | H | $CH_2F$ | H | O | H | Ga |
| Cl | H | $CH_2F$ | H | O | H | Ga |
| Br | H | $CH_2F$ | H | O | H | Ga |
| I | H | $CH_2F$ | H | O | H | Ga |
| $CH=CH_2$ | H | $CH_2F$ | H | O | H | Ga |
| $CH_2CH=CH_2$ | H | $CH_2F$ | H | O | H | Ga |
| $C\equiv CH$ | H | $CH_2F$ | H | O | H | Ga |
| $CH_2C\equiv CH$ | H | $CH_2F$ | H | O | H | Ga |
| $CH_2F$ | H | $CH_2F$ | H | O | H | Ga |
| $CHF_2$ | H | $CH_2F$ | H | O | H | Ga |
| CN | H | $CH_2F$ | H | O | H | Ga |
| $NH_2$ | H | $CH_2F$ | H | O | H | Ga |
| NHCOMe | H | $CH_2F$ | H | O | H | Ga |
| $CH_2NH_2$ | H | $CH_2F$ | H | O | H | Ga |
| $CH_2NHCOMe$ | H | $CH_2F$ | H | O | H | Ga |
| $CH_2OCHF_2$ | H | $CH_2F$ | H | O | H | Ga |
| $CF_3$ | H | $CH_2F$ | H | O | H | Ga |
| $NO_2$ | H | $CH_2F$ | H | O | H | Gb |
| OMe | H | $CH_2F$ | H | O | H | Ga |
| OEt | H | $CH_2F$ | H | O | H | Ga |
| OPr-n | H | $CH_2F$ | H | O | H | Gb |
| SMe | H | $CH_2F$ | H | O | H | Ga |
| SEt | H | $CH_2F$ | H | O | H | Ga |
| $SO_2Me$ | H | $CH_2F$ | H | O | H | Ga |
| $SO_2Et$ | H | $CH_2F$ | H | O | H | Ga |
| COMe | H | $CH_2F$ | H | O | H | Ga |
| COEt | H | $CH_2F$ | H | O | H | Ga |
| $CO_2H$ | H | $CH_2F$ | H | O | H | Gb |
| $CO_2Me$ | H | $CH_2F$ | H | O | H | Ga |
| $CO_2Et$ | H | $CH_2F$ | H | O | H | Ga |
| $CO_2Pr$-n | H | $CH_2F$ | H | O | H | Gb |
| $CO_2Bu$-n | H | $CH_2F$ | H | O | H | Gc |
| $CH_2Cl$ | H | $CH_2F$ | H | O | H | Ga |
| $CH_2Br$ | H | $CH_2F$ | H | O | H | Gb |
| $CH_2I$ | H | $CH_2F$ | H | O | H | Gb |
| $CH_2CF_3$ | H | $CH_2F$ | H | O | H | Ga |

| $R_1$ | $R_2$ | $R_3$ | $R_4$ | X | M | Gn |
|---|---|---|---|---|---|---|
| $CH_2CN$ | H | $CH_2F$ | H | O | H | Ga |
| $CH_2OH$ | H | $CH_2F$ | H | O | H | Gb |
| $CH_2OMe$ | H | $CH_2F$ | H | O | H | Ga |
| $CH_2OEt$ | H | $CH_2F$ | H | O | H | Ga |
| $CH_2OPr$-n | H | $CH_2F$ | H | O | H | Gb |
| $CH_2SMe$ | H | $CH_2F$ | H | O | H | Ga |
| $CH_2SEt$ | H | $CH_2F$ | H | O | H | Ga |
| $CH_2SPr$-n | H | $CH_2F$ | H | O | H | Gb |
| $CH_2SO_2Me$ | H | $CH_2F$ | H | O | H | Ga |
| $CH_2SO_2Et$ | H | $CH_2F$ | H | O | H | Ga |
| $CH_2COMe$ | H | $CH_2F$ | H | O | H | Ga |
| $CH_2COEt$ | H | $CH_2F$ | H | O | H | Ga |
| $CH_2COPr$-n | H | $CH_2F$ | H | O | H | Gb |
| $CH_2CO_2H$ | H | $CH_2F$ | H | O | H | Gb |
| $CH_2CO_2Me$ | H | $CH_2F$ | H | O | H | Ga |
| $CH_2CO_2Et$ | H | $CH_2F$ | H | O | H | Ga |
| $CH_2CO_2Pr$-n | H | $CH_2F$ | H | O | H | Gb |
| $CH_2OCH_2CH=CH_2$ | H | $CH_2F$ | H | O | H | Gb |
| $CH_2OCH_2C\equiv CH$ | H | $CH_2F$ | H | O | H | Gb |
| $CH=CHCO_2Me$ | H | $CH_2F$ | H | O | H | Ga |
| $CH=CHCO_2Et$ | H | $CH_2F$ | H | O | H | Ga |
| $CH=CHCO_2Pr$-n | H | $CH_2F$ | H | O | H | Gb |
| CH=CHCN | H | $CH_2F$ | H | O | H | Ga |
| Ph | H | $CH_2F$ | H | O | H | Ga |
| $CH_2Ph$ | H | $CH_2F$ | H | O | H | Ga |
| OPh | H | $CH_2F$ | H | O | H | Ga |
| SPh | H | $CH_2F$ | H | O | H | Ga |
| $SO_2Ph$ | H | $CH_2F$ | H | O | H | Ga |
| CONHMe | H | $CH_2F$ | H | O | H | Ga |
| CONHEt | H | $CH_2F$ | H | O | H | Ga |
| $CON(Me)_2$ | H | $CH_2F$ | H | O | H | Ga |
| $CON(Et)_2$ | H | $CH_2F$ | H | O | H | Ga |
| F | H | $CH_2Cl$ | H | O | H | Ga |
| Cl | H | $CH_2Cl$ | H | O | H | Ga |
| Br | H | $CH_2Cl$ | H | O | H | Ga |
| I | H | $CH_2Cl$ | H | O | H | Ga |
| $CH=CH_2$ | H | $CH_2Cl$ | H | O | H | Ga |
| $CH_2CH=CH_2$ | H | $CH_2Cl$ | H | O | H | Ga |
| $C\equiv CH$ | H | $CH_2Cl$ | H | O | H | Ga |
| $CH_2C\equiv CH$ | H | $CH_2Cl$ | H | O | H | Ga |

| $R_1$ | $R_2$ | $R_3$ | $R_4$ | X | M | Gn |
|---|---|---|---|---|---|---|
| $CH_2F$ | H | $CH_2Cl$ | H | O | H | Ga |
| $CHF_2$ | H | $CH_2Cl$ | H | O | H | Ga |
| CN | H | $CH_2Cl$ | H | O | H | Ga |
| $NH_2$ | H | $CH_2Cl$ | H | O | H | Ga |
| NHCOMe | H | $CH_2Cl$ | H | O | H | Ga |
| $CH_2NH_2$ | H | $CH_2Cl$ | H | O | H | Ga |
| $CH_2NHCOMe$ | H | $CH_2Cl$ | H | O | H | Ga |
| $CH_2OCHF_2$ | H | $CH_2Cl$ | H | O | H | Ga |
| $CF_3$- | H | $CH_2Cl$ | H | O | H | Ga |
| $NO_2$ | H | $CH_2Cl$ | H | O | H | Gb |
| OMe | H | $CH_2Cl$ | H | O | H | Ga |
| OEt | H | $CH_2Cl$ | H | O | H | Ga |
| OPr-n | H | $CH_2Cl$ | H | O | H | Gb |
| SMe | H | $CH_2Cl$ | H | O | H | Ga |
| SEt | H | $CH_2Cl$ | H | O | H | Ga |
| $SO_2Me$ | H | $CH_2Cl$ | H | O | H | Ga |
| $SO_2Et$ | H | $CH_2Cl$ | H | O | H | Ga |
| COMe | H | $CH_2Cl$ | H | O | H | Ga |
| COEt | H | $CH_2Cl$ | H | O | H | Ga |
| $CO_2H$ | H | $CH_2Cl$ | H | O | H | Gb |
| $CO_2Me$ | H | $CH_2Cl$ | H | O | H | Ga |
| $CO_2Et$ | H | $CH_2Cl$ | H | O | H | Ga |
| $CO_2Pr$-n | H | $CH_2Cl$ | H | O | H | Gb |
| $CO_2Bu$-n | H | $CH_2Cl$ | H | O | H | Gc |
| $CH_2Cl$ | H | $CH_2Cl$ | H | O | H | Ga |
| $CH_2Br$ | H | $CH_2Cl$ | H | O | H | Gb |
| $CH_2I$ | H | $CH_2Cl$ | H | O | H | Gb |
| $CH_2CF_3$ | H | $CH_2Cl$ | H | O | H | Ga |
| $CH_2CN$ | H | $CH_2Cl$ | H | O | H | Ga |
| $CH_2OH$ | H | $CH_2Cl$ | H | O | H | Gb |
| $CH_2OMe$ | H | $CH_2Cl$ | H | O | H | Ga |
| $CH_2OEt$ | H | $CH_2Cl$ | H | O | H | Ga |
| $CH_2OPr$-n | H | $CH_2Cl$ | H | O | H | Gb |
| $CH_2SMe$ | H | $CH_2Cl$ | H | O | H | Ga |
| $CH_2SEt$ | H | $CH_2Cl$ | H | O | H | Ga |
| $CH_2SPr$-n | H | $CH_2Cl$ | H | O | H | Gb |
| $CH_2SO_2Me$ | H | $CH_2Cl$ | H | O | H | Ga |
| $CH_2SO_2Et$ | H | $CH_2Cl$ | H | O | H | Ga |
| $CH_2COMe$ | H | $CH_2Cl$ | H | O | H | Ga |
| $CH_2COEt$ | H | $CH_2Cl$ | H | O | H | Ga |

| $R_1$ | $R_2$ | $R_3$ | $R_4$ | X | M | Gn |
|---|---|---|---|---|---|---|
| $CH_2COPr$-n | H | $CH_2Cl$ | H | O | H | Gb |
| $CH_2CO_2H$ | H | $CH_2Cl$ | H | O | H | Gb |
| $CH_2CO_2Me$ | H | $CH_2Cl$ | H | O | H | Ga |
| $CH_2CO_2Et$ | H | $CH_2Cl$ | H | O | H | Ga |
| $CH_2CO_2Pr$-n | H | $CH_2Cl$ | H | O | H | Gb |
| $CH_2OCH_2CH=CH_2$ | H | $CH_2Cl$ | H | O | H | Gb |
| $CH_2OCH_2C\equiv CH$ | H | $CH_2Cl$ | H | O | H | Gb |
| $CH=CHCO_2Me$ | H | $CH_2Cl$ | H | O | H | Ga |
| $CH=CHCO_2Et$ | H | $CH_2Cl$ | H | O | H | Ga |
| $CH=CHCO_2Pr$-n | H | $CH_2Cl$ | H | O | H | Gb |
| CH=CHCN | H | $CH_2Cl$ | H | O | H | Ga |
| Ph | H | $CH_2Cl$ | H | O | H | Ga |
| $CH_2Ph$ | H | $CH_2Cl$ | H | O | H | Ga |
| OPh | H | $CH_2Cl$ | H | O | H | Ga |
| SPh | H | $CH_2Cl$ | H | O | H | Ga |
| $SO_2Ph$ | H | $CH_2Cl$ | H | O | H | Ga |
| CONHMe | H | $CH_2Cl$ | H | O | H | Ga |
| CONHEt | H | $CH_2Cl$ | H | O | H | Ga |
| $CON(Me)_2$ | H | $CH_2Cl$ | H | O | H | Ga |
| $CON(Et)_2$ | H | $CH_2Cl$ | H | O | H | Ga |
| F | H | $CH_2OMe$ | H | O | H | Ga |
| Cl | H | $CH_2OMe$ | H | O | H | Ga |
| Br | H | $CH_2OMe$ | H | O | H | Ga |
| I | H | $CH_2OMe$ | H | O | H | Ga |
| $CH=CH_2$ | H | $CH_2OMe$ | H | O | H | Ga |
| $CH_2CH=CH_2$ | H | $CH_2OMe$ | H | O | H | Ga |
| $C\equiv CH$ | H | $CH_2OMe$ | H | O | H | Ga |
| $CH_2C\equiv CH$ | H | $CH_2OMe$ | H | O | H | Ga |
| $CH_2F$ | H | $CH_2OMe$ | H | O | H | Ga |
| $CHF_2$ | H | $CH_2OMe$ | H | O | H | Ga |
| CN | H | $CH_2OMe$ | H | O | H | Ga |
| $NH_2$ | H | $CH_2OMe$ | H | O | H | Ga |
| NHCOMe | H | $CH_2OMe$ | H | O | H | Ga |
| $CH_2NH_2$ | H | $CH_2OMe$ | H | O | H | Ga |
| $CH_2NHCOMe$ | H | $CH_2OMe$ | H | O | H | Ga |
| $CH_2OCHF_2$ | H | $CH_2OMe$ | H | O | H | Ga |
| $CF_3$ | H | $CH_2OMe$ | H | O | H | Ga |
| $NO_2$ | H | $CH_2OMe$ | H | O | H | Gb |
| OMe | H | $CH_2OMe$ | H | O | H | Ga |
| OEt | H | $CH_2OMe$ | H | O | H | Ga |

| $R_1$ | $R_2$ | $R_3$ | $R_4$ | X | M | Gn |
|---|---|---|---|---|---|---|
| OPr-n | H | $CH_2OMe$ | H | O | H | Gb |
| SMe | H | $CH_2OMe$ | H | O | H | Ga |
| SEt | H | $CH_2OMe$ | H | O | H | Ga |
| $SO_2Me$ | H | $CH_2OMe$ | H | O | H | Ga |
| $SO_2Et$ | H | $CH_2OMe$ | H | O | H | Ga |
| COMe | H | $CH_2OMe$ | H | O | H | Ga |
| COEt | H | $CH_2OMe$ | H | O | H | Ga |
| $CO_2H$ | H | $CH_2OMe$ | H | O | H | Gb |
| $CO_2Me$ | H | $CH_2OMe$ | H | O | H | Ga |
| $CO_2Et$ | H | $CH_2OMe$ | H | O | H | Ga |
| $CO_2Pr$-n | H | $CH_2OMe$ | H | O | H | Gb |
| $CO_2Bu$-n | H | $CH_2OMe$ | H | O | H | Gc |
| $CH_2Cl$ | H | $CH_2OMe$ | H | O | H | Ga |
| $CH_2Br$ | H | $CH_2OMe$ | H | O | H | Gb |
| $CH_2I$ | H | $CH_2OMe$ | H | O | H | Gb |
| $CH_2CF_3$ | H | $CH_2OMe$ | H | O | H | Ga |
| $CH_2CN$ | H | $CH_2OMe$ | H | O | H | Ga |
| $CH_2OH$ | H | $CH_2OMe$ | H | O | H | Gb |
| $CH_2OMe$ | H | $CH_2OMe$ | H | O | H | Ga |
| $CH_2OEt$ | H | $CH_2OMe$ | H | O | H | Ga |
| $CH_2OPr$-n | H | $CH_2OMe$ | H | O | H | Gb |
| $CH_2SMe$ | H | $CH_2OMe$ | H | O | H | Ga |
| $CH_2SEt$ | H | $CH_2OMe$ | H | O | H | Ga |
| $CH_2SPr$-n | H | $CH_2OMe$ | H | O | H | Gb |
| $CH_2SO_2Me$ | H | $CH_2OMe$ | H | O | H | Ga |
| $CH_2SO_2Et$ | H | $CH_2OMe$ | H | O | H | Ga |
| $CH_2COMe$ | H | $CH_2OMe$ | H | O | H | Ga |
| $CH_2COEt$ | H | $CH_2OMe$ | H | O | H | Ga |
| $CH_2COPr$-n | H | $CH_2OMe$ | H | O | H | Gb |
| $CH_2CO_2H$ | H | $CH_2OMe$ | H | O | H | Gb |
| $CH_2CO_2Me$ | H | $CH_2OMe$ | H | O | H | Ga |
| $CH_2CO_2Et$ | H | $CH_2OMe$ | H | O | H | Ga |
| $CH_2CO_2Pr$-n | H | $CH_2OMe$ | H | O | H | Gb |
| $CH_2OCH_2CH=CH_2$ | H | $CH_2OMe$ | H | O | H | Gb |
| $CH_2OCH_2C\equiv CH$ | H | $CH_2OMe$ | H | O | H | Gb |
| $CH=CHCO_2Me$ | H | $CH_2OMe$ | H | O | H | Ga |
| $CH=CHCO_2Et$ | H | $CH_2OMe$ | H | O | H | Ga |
| $CH=CHCO_2Pr$-n | H | $CH_2OMe$ | H | O | H | Gb |
| CH=CHCN | H | $CH_2OMe$ | H | O | H | Ga |
| Ph | H | $CH_2OMe$ | H | O | H | Ga |

| $R_1$ | $R_2$ | $R_3$ | $R_4$ | X | M | Gn |
|---|---|---|---|---|---|---|
| $CH_2Ph$ | H | $CH_2OMe$ | H | O | H | Ga |
| OPh | H | $CH_2OMe$ | H | O | H | Ga |
| SPh | H | $CH_2OMe$ | H | O | H | Ga |
| $SO_2Ph$ | H | $CH_2OMe$ | H | O | H | Ga |
| CONHMe | H | $CH_2OMe$ | H | O | H | Ga |
| CONHEt | H | $CH_2OMe$ | H | O | H | Ga |
| $CON(Me)_2$ | H | $CH_2OMe$ | H | O | H | Ga |
| $CON(Et)_2$ | H | $CH_2OMe$ | H | O | H | Ga |
| F | H | $CH_2SMe$ | H | O | H | Ga |
| Cl | H | $CH_2SMe$ | H | O | H | Ga |
| Br | H | $CH_2SMe$ | H | O | H | Ga |
| I | H | $CH_2SMe$ | H | O | H | Ga |
| $CH=CH_2$ | H | $CH_2SMe$ | H | O | H | Ga |
| $CH_2CH=CH_2$ | H | $CH_2SMe$ | H | O | H | Ga |
| $C\equiv CH$ | H | $CH_2SMe$ | H | O | H | Ga |
| $CH_2C\equiv CH$ | H | $CH_2SMe$ | H | O | H | Ga |
| $CH_2F$ | H | $CH_2SMe$ | H | O | H | Ga |
| $CHF_2$ | H | $CH_2SMe$ | H | O | H | Ga |
| CN | H | $CH_2SMe$ | H | O | H | Ga |
| $NH_2$ | H | $CH_2SMe$ | H | O | H | Ga |
| NHCOMe | H | $CH_2SMe$ | H | O | H | Ga |
| $CH_2NH_2$ | H | $CH_2SMe$ | H | O | H | Ga |
| $CH_2NHCOMe$ | H | $CH_2SMe$ | H | O | H | Ga |
| $CH_2OCHF_2$ | H | $CH_2SMe$ | H | O | H | Ga |
| $CF_3$ | H | $CH_2SMe$ | H | O | H | Ga |
| $NO_2$ | H | $CH_2SMe$ | H | O | H | Gb |
| OMe | H | $CH_2SMe$ | H | O | H | Ga |
| OEt | H | $CH_2SMe$ | H | O | H | Ga |
| OPr-n | H | $CH_2SMe$ | H | O | H | Gb |
| SMe | H | $CH_2SMe$ | H | O | H | Ga |
| SEt | H | $CH_2SMe$ | H | O | H | Ga |
| $SO_2Me$ | H | $CH_2SMe$ | H | O | H | Ga |
| $SO_2Et$ | H | $CH_2SMe$ | H | O | H | Ga |
| COMe | H | $CH_2SMe$ | H | O | H | Ga |
| COEt | H | $CH_2SMe$ | H | O | H | Ga |
| $CO_2H$ | H | $CH_2SMe$ | H | O | H | Gb |
| $CO_2Me$ | H | $CH_2SMe$ | H | O | H | Ga |
| $CO_2Et$ | H | $CH_2SMe$ | H | O | H | Ga |
| $CO_2Pr$-n | H | $CH_2SMe$ | H | O | H | Gb |
| $CO_2Bu$-n | H | $CH_2SMe$ | H | O | H | Gc |

| $R_1$ | $R_2$ | $R_3$ | $R_4$ | X | M | Gn |
|---|---|---|---|---|---|---|
| $CH_2Cl$ | H | $CH_2SMe$ | H | O | H | Ga |
| $CH_2Br$ | H | $CH_2SMe$ | H | O | H | Gb |
| $CH_2I$ | H | $CH_2SMe$ | H | O | H | Gb |
| $CH_2CF_3$ | H | $CH_2SMe$ | H | O | H | Ga |
| $CH_2CN$ | H | $CH_2SMe$ | H | O | H | Ga |
| $CH_2OH$ | H | $CH_2SMe$ | H | O | H | Gb |
| $CH_2OMe$ | H | $CH_2SMe$ | H | O | H | Ga |
| $CH_2OEt$ | H | $CH_2SMe$ | H | O | H | Ga |
| $CH_2OPr$-n | H | $CH_2SMe$ | H | O | H | Gb |
| $CH_2SMe$ | H | $CH_2SMe$ | H | O | H | Ga |
| $CH_2SEt$ | H | $CH_2SMe$ | H | O | H | Ga |
| $CH_2SPr$-n | H | $CH_2SMe$ | H | O | H | Gb |
| $CH_2SO_2Me$ | H | $CH_2SMe$ | H | O | H | Ga |
| $CH_2SO_2Et$ | H | $CH_2SMe$ | H | O | H | Ga |
| $CH_2COMe$ | H | $CH_2SMe$ | H | O | H | Ga |
| $CH_2COEt$ | H | $CH_2SMe$ | H | O | H | Ga |
| $CH_2COPr$-n | H | $CH_2SMe$ | H | O | H | Gb |
| $CH_2CO_2H$ | H | $CH_2SMe$ | H | O | H | Gb |
| $CH_2CO_2Me$ | H | $CH_2SMe$ | H | O | H | Ga |
| $CH_2CO_2Et$ | H | $CH_2SMe$ | H | O | H | Ga |
| $CH_2CO_2Pr$-n | H | $CH_2SMe$ | H | O | H | Gb |
| $CH_2OCH_2CH=CH_2$ | H | $CH_2SMe$ | H | O | H | Gb |
| $CH_2OCH_2C\equiv CH$ | H | $CH_2SMe$ | H | O | H | Gb |
| $CH=CHCO_2Me$ | H | $CH_2SMe$ | H | O | H | Ga |
| $CH=CHCO_2Et$ | H | $CH_2SMe$ | H | O | H | Ga |
| $CH=CHCO_2Pr$-n | H | $CH_2SMe$ | H | O | H | Gb |
| $CH=CHCN$ | H | $CH_2SMe$ | H | O | H | Ga |
| Ph | H | $CH_2SMe$ | H | O | H | Ga |
| $CH_2Ph$ | H | $CH_2SMe$ | H | O | H | Ga |
| OPh | H | $CH_2SMe$ | H | O | H | Ga |
| SPh | H | $CH_2SMe$ | H | O | H | Ga |
| $SO_2Ph$ | H | $CH_2SMe$ | H | O | H | Ga |
| CONHMe | H | $CH_2SMe$ | H | O | H | Ga |
| CONHEt | H | $CH_2SMe$ | H | O | H | Ga |
| $CON(Me)_2$ | H | $CH_2SMe$ | H | O | H | Ga |
| $CON(Et)_2$ | H | $CH_2SMe$ | H | O | H | Ga |
| F | H | $CH_2SO_2Me$ | H | O | H | Ga |
| Cl | H | $CH_2SO_2Me$ | H | O | H | Ga |
| Br | H | $CH_2SO_2Me$ | H | O | H | Ga |
| I | H | $CH_2SO_2Me$ | H | O | H | Ga |

| $R_1$ | $R_2$ | $R_3$ | $R_4$ | X | M | Gn |
|---|---|---|---|---|---|---|
| $CH=CH_2$ | H | $CH_2SO_2Me$ | H | O | H | Ga |
| $CH_2CH=CH_2$ | H | $CH_2SO_2Me$ | H | O | H | Ga |
| $C\equiv CH$ | H | $CH_2SO_2Me$ | H | O | H | Ga |
| $CH_2C\equiv CH$ | H | $CH_2SO_2Me$ | H | O | H | Ga |
| $CH_2F$ | H | $CH_2SO_2Me$ | H | O | H | Ga |
| $CHF_2$ | H | $CH_2SO_2Me$ | H | O | H | Ga |
| CN | H | $CH_2SO_2Me$ | H | O | H | Ga |
| $NH_2$ | H | $CH_2SO_2Me$ | H | O | H | Ga |
| NHCOMe | H | $CH_2SO_2Me$ | H | O | H | Ga |
| $CH_2NH_2$ | H | $CH_2SO_2Me$ | H | O | H | Ga |
| $CH_2NHCOMe$ | H | $CH_2SO_2Me$ | H | O | H | Ga |
| $CH_2OCHF_2$ | H | $CH_2SO_2Me$ | H | O | H | Ga |
| $CF_3$ | H | $CH_2SO_2Me$ | H | O | H | Ga |
| $NO_2$ | H | $CH_2SO_2Me$ | H | O | H | Gb |
| OMe | H | $CH_2SO_2Me$ | H | O | H | Ga |
| OEt | H | $CH_2SO_2Me$ | H | O | H | Ga |
| OPr-n | H | $CH_2SO_2Me$ | H | O | H | Gb |
| SMe | H | $CH_2SO_2Me$ | H | O | H | Ga |
| SEt | H | $CH_2SO_2Me$ | H | O | H | Ga |
| $SO_2Me$ | H | $CH_2SO_2Me$ | H | O | H | Ga |
| $SO_2Et$ | H | $CH_2SO_2Me$ | H | O | H | Ga |
| COMe | H | $CH_2SO_2Me$ | H | O | H | Ga |
| COEt | H | $CH_2SO_2Me$ | H | O | H | Ga |
| $CO_2H$ | H | $CH_2SO_2Me$ | H | O | H | Gb |
| $CO_2Me$ | H | $CH_2SO_2Me$ | H | O | H | Ga |
| $CO_2Et$ | H | $CH_2SO_2Me$ | H | O | H | Ga |
| $CO_2Pr$-n | H | $CH_2SO_2Me$ | H | O | H | Gb |
| $CO_2Bu$-n | H | $CH_2SO_2Me$ | H | O | H | Gc |
| $CH_2Cl$ | H | $CH_2SO_2Me$ | H | O | H | Ga |
| $CH_2Br$ | H | $CH_2SO_2Me$ | H | O | H | Gb |
| $CH_2I$ | H | $CH_2SO_2Me$ | H | O | H | Gb |
| $CH_2CF_3$ | H | $CH_2SO_2Me$ | H | O | H | Ga |
| $CH_2CN$ | H | $CH_2SO_2Me$ | H | O | H | Ga |
| $CH_2OH$ | H | $CH_2SO_2Me$ | H | O | H | Gb |
| $CH_2OMe$ | H | $CH_2SO_2Me$ | H | O | H | Ga |
| $CH_2OEt$ | H | $CH_2SO_2Me$ | H | O | H | Ga |
| $CH_2OPr$-n | H | $CH_2SO_2Me$ | H | O | H | Gb |
| $CH_2SMe$ | H | $CH_2SO_2Me$ | H | O | H | Ga |
| $CH_2SEt$ | H | $CH_2SO_2Me$ | H | O | H | Ga |
| $CH_2SPr$-n | H | $CH_2SO_2Me$ | H | O | H | Gb |

| $R_1$ | $R_2$ | $R_3$ | $R_4$ | X | M | Gn |
|---|---|---|---|---|---|---|
| $CH_2SO_2Me$ | H | $CH_2SO_2Me$ | H | O | H | Ga |
| $CH_2SO_2Et$ | H | $CH_2SO_2Me$ | H | O | H | Ga |
| $CH_2COMe$ | H | $CH_2SO_2Me$ | H | O | H | Ga |
| $CH_2COEt$ | H | $CH_2SO_2Me$ | H | O | H | Ga |
| $CH_2COPr$-n | H | $CH_2SO_2Me$ | H | O | H | Gb |
| $CH_2CO_2H$ | H | $CH_2SO_2Me$ | H | O | H | Gb |
| $CH_2CO_2Me$ | H | $CH_2SO_2Me$ | H | O | H | Ga |
| $CH_2CO_2Et$ | H | $CH_2SO_2Me$ | H | O | H | Ga |
| $CH_2CO_2Pr$-n | H | $CH_2SO_2Me$ | H | O | H | Gb |
| $CH_2OCH_2CH=CH_2$ | H | $CH_2SO_2Me$ | H | O | H | Gb |
| $CH_2OCH_2C\equiv CH$ | H | $CH_2SO_2Me$ | H | O | H | Gb |
| $CH=CHCO_2Me$ | H | $CH_2SO_2Me$ | H | O | H | Ga |
| $CH=CHCO_2Et$ | H | $CH_2SO_2Me$ | H | O | H | Ga |
| $CH=CHCO_2Pr$-n | H | $CH_2SO_2Me$ | H | O | H | Gb |
| CH=CHCN | H | $CH_2SO_2Me$ | H | O | H | Ga |
| Ph | H | $CH_2SO_2Me$ | H | O | H | Ga |
| $CH_2Ph$ | H | $CH_2SO_2Me$ | H | O | H | Ga |
| OPh | H | $CH_2SO_2Me$ | H | O | H | Ga |
| SPh | H | $CH_2SO_2Me$ | H | O | H | Ga |
| $SO_2Ph$ | H | $CH_2SO_2Me$ | H | O | H | Ga |
| CONHMe | H | $CH_2SO_2Me$ | H | O | H | Ga |
| CONHEt | H | $CH_2SO_2Me$ | H | O | H | Ga |
| $CON(Me)_2$ | H | $CH_2SO_2Me$ | H | O | H | Ga |
| $CON(Et)_2$ | H | $CH_2SO_2Me$ | H | O | H | Ga |
| F | H | Ph | H | O | H | Ga |
| Cl | H | Ph | H | O | H | Ga |
| Br | H | Ph | H | O | H | Ga |
| I | H | Ph | H | O | H | Ga |
| $CH=CH_2$ | H | Ph | H | O | H | Ga |
| $CH_2CH=CH_2$ | H | Ph | H | O | H | Ga |
| $C\equiv CH$ | H | Ph | H | O | H | Ga |
| $CH_2C\equiv CH$ | H | Ph | H | O | H | Ga |
| $CH_2F$ | H | Ph | H | O | H | Ga |
| $CHF_2$ | H | Ph | H | O | H | Ga |
| CN | H | Ph | H | O | H | Ga |
| $NH_2$ | H | Ph | H | O | H | Ga |
| NHCOMe | H | Ph | H | O | H | Ga |
| $CH_2NH_2$ | H | Ph | H | O | H | Ga |
| $CH_2NHCOMe$ | H | Ph | H | O | H | Ga |
| $CH_2OCHF_2$ | H | Ph | H | O | H | Ga |

| $R_1$ | $R_2$ | $R_3$ | $R_4$ | X | M | Gn |
|---|---|---|---|---|---|---|
| $CF_3$ | H | Ph | H | O | H | Ga |
| $NO_2$ | H | Ph | H | O | H | Gb |
| OMe | H | Ph | H | O | H | Ga |
| OEt | H | Ph | H | O | H | Ga |
| OPr-n | H | Ph | H | O | H | Gb |
| SMe | H | Ph | H | O | H | Ga |
| SEt | H | Ph | H | O | H | Ga |
| $SO_2Me$ | H | Ph | H | O | H | Ga |
| $SO_2Et$ | H | Ph | H | O | H | Ga |
| COMe | H | Ph | H | O | H | Ga |
| COEt | H | Ph | H | O | H | Ga |
| $CO_2H$ | H | Ph | H | O | H | Gb |
| $CO_2Me$ | H | Ph | H | O | H | Ga |
| $CO_2Et$ | H | Ph | H | O | H | Ga |
| $CO_2Pr$-n | H | Ph | H | O | H | Gb |
| $CO_2Bu$-n | H | Ph | H | O | H | Gc |
| $CH_2Cl$ | H | Ph | H | O | H | Ga |
| $CH_2Br$ | H | Ph | H | O | H | Gb |
| $CH_2I$ | H | Ph | H | O | H | Gb |
| $CH_2CF_3$ | H | Ph | H | O | H | Ga |
| $CH_2CN$ | H | Ph | H | O | H | Ga |
| $CH_2OH$ | H | Ph | H | O | H | Gb |
| $CH_2OMe$ | H | Ph | H | O | H | Ga |
| $CH_2OEt$ | H | Ph | H | O | H | Ga |
| $CH_2OPr$-n | H | Ph | H | O | H | Gb |
| $CH_2SMe$ | H | Ph | H | O | H | Ga |
| $CH_2SEt$ | H | Ph | H | O | H | Ga |
| $CH_2SPr$-n | H | Ph | H | O | H | Gb |
| $CH_2SO_2Me$ | H | Ph | H | O | H | Ga |
| $CH_2SO_2Et$ | H | Ph | H | O | H | Ga |
| $CH_2COMe$ | H | Ph | H | O | H | Ga |
| $CH_2COEt$ | H | Ph | H | O | H | Ga |
| $CH_2COPr$-n | H | Ph | H | O | H | Gb |
| $CH_2CO_2H$ | H | Ph | H | O | H | Gb |
| $CH_2CO_2Me$ | H | Ph | H | O | H | Ga |
| $CH_2CO_2Et$ | H | Ph | H | O | H | Ga |
| $CH_2CO_2Pr$-n | H | Ph | H | O | H | Gb |
| $CH_2OCH_2CH=CH_2$ | H | Ph | H | O | H | Gb |
| $CH_2OCH_2C\equiv CH$ | H | Ph | H | O | H | Gb |
| $CH=CHCO_2Me$ | H | Ph | H | O | H | Ga |

| $R_1$ | $R_2$ | $R_3$ | $R_4$ | X | M | Gn |
|---|---|---|---|---|---|---|
| $CH=CHCO_2Et$ | H | Ph | H | O | H | Ga |
| $CH=CHCO_2Pr$-n | H | Ph | H | O | H | Gb |
| CH=CHCN | H | Ph | H | O | H | Ga |
| Ph | H | Ph | H | O | H | Ga |
| $CH_2Ph$ | H | Ph | H | O | H | Ga |
| OPh | H | Ph | H | O | H | Ga |
| SPh | H | Ph | H | O | H | Ga |
| $SO_2Ph$ | H | Ph | H | O | H | Ga |
| CONHMe | H | Ph | H | O | H | Ga |
| CONHEt | H | Ph | H | O | H | Ga |
| $CON(Me)_2$ | H | Ph | H | O | H | Ga |
| $CON(Et)_2$ | H | Ph | H | O | H | Ga |
| F | H | $CH_2Ph$ | H | O | H | Ga |
| Cl | H | $CH_2Ph$ | H | O | H | Ga |
| Br | H | $CH_2Ph$ | H | O | H | Ga |
| I | H | $CH_2Ph$ | H | O | H | Ga |
| $CH=CH_2$ | H | $CH_2Ph$ | H | O | H | Ga |
| $CH_2CH=CH_2$ | H | $CH_2Ph$ | H | O | H | Ga |
| $C\equiv CH$ | H | $CH_2Ph$ | H | O | H | Ga |
| $CH_2C\equiv CH$ | H | $CH_2Ph$ | H | O | H | Ga |
| $CH_2F$ | H | $CH_2Ph$ | H | O | H | Ga |
| $CHF_2$ | H | $CH_2Ph$ | H | O | H | Ga |
| CN | H | $CH_2Ph$ | H | O | H | Ga |
| $NH_2$ | H | $CH_2Ph$ | H | O | H | Ga |
| NHCOMe | H | $CH_2Ph$ | H | O | H | Ga |
| $CH_2NH_2$ | H | $CH_2Ph$ | H | O | H | Ga |
| $CH_2NHCOMe$ | H | $CH_2Ph$ | H | O | H | Ga |
| $CH_2OCHF_2$ | H | $CH_2Ph$ | H | O | H | Ga |
| $CF_3$ | H | $CH_2Ph$ | H | O | H | Ga |
| $NO_2$ | H | $CH_2Ph$ | H | O | H | Gb |
| OMe | H | $CH_2Ph$ | H | O | H | Ga |
| OEt | H | $CH_2Ph$ | H | O | H | Ga |
| OPr-n | H | $CH_2Ph$ | H | O | H | Gb |
| SMe | H | $CH_2Ph$ | H | O | H | Ga |
| SEt | H | $CH_2Ph$ | H | O | H | Ga |
| $SO_2Me$ | H | $CH_2Ph$ | H | O | H | Ga |
| $SO_2Et$ | H | $CH_2Ph$ | H | O | H | Ga |
| COMe | H | $CH_2Ph$ | H | O | H | Ga |
| COEt | H | $CH_2Ph$ | H | O | H | Ga |
| $CO_2H$ | H | $CH_2Ph$ | H | O | H | Gb |

| $R_1$ | $R_2$ | $R_3$ | $R_4$ | X | M | Gn |
|---|---|---|---|---|---|---|
| $CO_2Me$ | H | $CH_2Ph$ | H | O | H | Ga |
| $CO_2Et$ | H | $CH_2Ph$ | H | O | H | Ga |
| $CO_2Pr$-n | H | $CH_2Ph$ | H | O | H | Gb |
| $CO_2Bu$-n | H | $CH_2Ph$ | H | O | H | Gc |
| $CH_2Cl$ | H | $CH_2Ph$ | H | O | H | Ga |
| $CH_2Br$ | H | $CH_2Ph$ | H | O | H | Gb |
| $CH_2I$ | H | $CH_2Ph$ | H | O | H | Gb |
| $CH_2CF_3$ | H | $CH_2Ph$ | H | O | H | Ga |
| $CH_2CN$ | H | $CH_2Ph$ | H | O | H | Ga |
| $CH_2OH$ | H | $CH_2Ph$ | H | O | H | Gb |
| $CH_2OMe$ | H | $CH_2Ph$ | H | O | H | Ga |
| $CH_2OEt$ | H | $CH_2Ph$ | H | O | H | Ga |
| $CH_2OPr$-n | H | $CH_2Ph$ | H | O | H | Gb |
| $CH_2SMe$ | H | $CH_2Ph$ | H | O | H | Ga |
| $CH_2SEt$ | H | $CH_2Ph$ | H | O | H | Ga |
| $CH_2SPr$-n | H | $CH_2Ph$ | H | O | H | Gb |
| $CH_2SO_2Me$ | H | $CH_2Ph$ | H | O | H | Ga |
| $CH_2SO_2Et$ | H | $CH_2Ph$ | H | O | H | Ga |
| $CH_2COMe$ | H | $CH_2Ph$ | H | O | H | Ga |
| $CH_2COEt$ | H | $CH_2Ph$ | H | O | H | Ga |
| $CH_2COPr$-n | H | $CH_2Ph$ | H | O | H | Gb |
| $CH_2CO_2H$ | H | $CH_2Ph$ | H | O | H | Gb |
| $CH_2CO_2Me$ | H | $CH_2Ph$ | H | O | H | Ga |
| $CH_2CO_2Et$ | H | $CH_2Ph$ | H | O | H | Ga |
| $CH_2CO_2Pr$-n | H | $CH_2Ph$ | H | O | H | Gb |
| $CH_2OCH_2CH=CH_2$ | H | $CH_2Ph$ | H | O | H | Gb |
| $CH_2OCH_2C\equiv CH$ | H | $CH_2Ph$ | H | O | H | Gb |
| $CH=CHCO_2Me$ | H | $CH_2Ph$ | H | O | H | Ga |
| $CH=CHCO_2Et$ | H | $CH_2Ph$ | H | O | H | Ga |
| $CH=CHCO_2Pr$-n | H | $CH_2Ph$ | H | O | H | Gb |
| $CH=CHCN$ | H | $CH_2Ph$ | H | O | H | Ga |
| Ph | H | $CH_2Ph$ | H | O | H | Ga |
| $CH_2Ph$ | H | $CH_2Ph$ | H | O | H | Ga |
| OPh | H | $CH_2Ph$ | H | O | H | Ga |
| SPh | H | $CH_2Ph$ | H | O | H | Ga |
| $SO_2Ph$ | H | $CH_2Ph$ | H | O | H | Ga |
| CONHMe | H | $CH_2Ph$ | H | O | H | Ga |
| CONHEt | H | $CH_2Ph$ | H | O | H | Ga |
| $CON(Me)_2$ | H | $CH_2Ph$ | H | O | H | Ga |
| $CON(Et)_2$ | H | $CH_2Ph$ | H | O | H | Ga |

| $R_1$ | $R_2$ | $R_3$ | $R_4$ | X | M | Gn |
|---|---|---|---|---|---|---|
| F | H | OPh | H | O | H | Ga |
| Cl | H | OPh | H | O | H | Ga |
| Br | H | OPh | H | O | H | Ga |
| I | H | OPh | H | O | H | Ga |
| $CH=CH_2$ | H | OPh | H | O | H | Ga |
| $CH_2CH=CH_2$ | H | OPh | H | O | H | Ga |
| $C\equiv CH$ | H | OPh | H | O | H | Ga |
| $CH_2C\equiv CH$ | H | OPh | H | O | H | Ga |
| $CH_2F$ | H | OPh | H | O | H | Ga |
| $CHF_2$ | H | OPh | H | O | H | Ga |
| CN | H | OPh | H | O | H | Ga |
| $NH_2$ | H | OPh | H | O | H | Ga |
| NHCOMe | H | OPh | H | O | H | Ga |
| $CH_2NH_2$ | H | OPh | H | O | H | Ga |
| $CH_2NHCOMe$ | H | OPh | H | O | H | Ga |
| $CH_2OCHF_2$ | H | OPh | H | O | H | Ga |
| $CF_3$ | H | OPh | H | O | H | Ga |
| $NO_2$ | H | OPh | H | O | H | Gb |
| OMe | H | OPh | H | O | H | Ga |
| OEt | H | OPh | H | O | H | Ga |
| OPr-n | H | OPh | H | O | H | Gb |
| SMe | H | OPh | H | O | H | Ga |
| SEt | H | OPh | H | O | H | Ga |
| $SO_2Me$ | H | OPh | H | O | H | Ga |
| $SO_2Et$ | H | OPh | H | O | H | Ga |
| COMe | H | OPh | H | O | H | Ga |
| COEt | H | OPh | H | O | H | Ga |
| $CO_2H$ | H | OPh | H | O | H | Gb |
| $CO_2Me$ | H | OPh | H | O | H | Ga |
| $CO_2Et$ | H | OPh | H | O | H | Ga |
| $CO_2Pr$-n | H | OPh | H | O | H | Gb |
| $CO_2Bu$-n | H | OPh | H | O | H | Gc |
| $CH_2Cl$ | H | OPh | H | O | H | Ga |
| $CH_2Br$ | H | OPh | H | O | H | Gb |
| $CH_2I$ | H | OPh | H | O | H | Gb |
| $CH_2CF_3$ | H | OPh | H | O | H | Ga |
| $CH_2CN$ | H | OPh | H | O | H | Ga |
| $CH_2OH$ | H | OPh | H | O | H | Gb |
| $CH_2OMe$ | H | OPh | H | O | H | Ga |
| $CH_2OEt$ | H | OPh | H | O | H | Ga |

| $R_1$ | $R_2$ | $R_3$ | $R_4$ | X | M | Gn |
|---|---|---|---|---|---|---|
| $CH_2OPr$-n | H | OPh | H | O | H | Gb |
| $CH_2SMe$ | H | OPh | H | O | H | Ga |
| $CH_2SEt$ | H | OPh | H | O | H | Ga |
| $CH_2SPr$-n | H | OPh | H | O | H | Gb |
| $CH_2SO_2Me$ | H | OPh | H | O | H | Ga |
| $CH_2SO_2Et$ | H | OPh | H | O | H | Ga |
| $CH_2COMe$ | H | OPh | H | O | H | Ga |
| $CH_2COEt$ | H | OPh | H | O | H | Ga |
| $CH_2COPr$-n | H | OPh | H | O | H | Gb |
| $CH_2CO_2H$ | H | OPh | H | O | H | Gb |
| $CH_2CO_2Me$ | H | OPh | H | O | H | Ga |
| $CH_2CO_2Et$ | H | OPh | H | O | H | Ga |
| $CH_2CO_2Pr$-n | H | OPh | H | O | H | Gb |
| $CH_2OCH_2CH=CH_2$ | H | OPh | H | O | H | Gb |
| $CH_2OCH_2C\equiv CH$ | H | OPh | H | O | H | Gb |
| $CH=CHCO_2Me$ | H | OPh | H | O | H | Ga |
| $CH=CHCO_2Et$ | H | OPh | H | O | H | Ga |
| $CH=CHCO_2Pr$-n | H | OPh | H | O | H | Gb |
| CH=CHCN | H | OPh | H | O | H | Ga |
| Ph | H | OPh | H | O | H | Ga |
| $CH_2Ph$ | H | OPh | H | O | H | Ga |
| OPh | H | OPh | H | O | H | Ga |
| SPh | H | OPh | H | O | H | Ga |
| $SO_2Ph$ | H | OPh | H | O | H | Ga |
| CONHMe | H | OPh | H | O | H | Ga |
| CONHEt | H | OPh | H | O | H | Ga |
| $CON(Me)_2$ | H | OPh | H | O | H | Ga |
| $CON(Et)_2$ | H | OPh | H | O | H | Ga |
| F | H | SPh | H | O | H | Ga |
| Cl | H | SPh | H | O | H | Ga |
| Br | H | SPh | H | O | H | Ga |
| I | H | SPh | H | O | H | Ga |
| $CH=CH_2$ | H | SPh | H | O | H | Ga |
| $CH_2CH=CH_2$ | H | SPh | H | O | H | Ga |
| $C\equiv CH$ | H | SPh | H | O | H | Ga |
| $CH_2C\equiv CH$ | H | SPh | H | O | H | Ga |
| $CH_2F$ | H | SPh | H | O | H | Ga |
| $CHF_2$ | H | SPh | H | O | H | Ga |
| CN | H | SPh | H | O | H | Ga |
| $NH_2$ | H | SPh | H | O | H | Ga |

| $R_1$ | $R_2$ | $R_3$ | $R_4$ | X | M | Gn |
|---|---|---|---|---|---|---|
| NHCOMe | H | SPh | H | O | H | Ga |
| $CH_2NH_2$ | H | SPh | H | O | H | Ga |
| $CH_2NHCOMe$ | H | SPh | H | O | H | Ga |
| $CH_2OCHF_2$ | H | SPh | H | O | H | Ga |
| $CF_3$ | H | SPh | H | O | H | Ga |
| $NO_2$ | H | SPh | H | O | H | Gb |
| OMe | H | SPh | H | O | H | Ga |
| OEt | H | SPh | H | O | H | Ga |
| OPr-n | H | SPh | H | O | H | Gb |
| SMe | H | SPh | H | O | H | Ga |
| SEt | H | SPh | H | O | H | Ga |
| $SO_2Me$ | H | SPh | H | O | H | Ga |
| $SO_2Et$ | H | SPh | H | O | H | Ga |
| COMe | H | SPh | H | O | H | Ga |
| COEt | H | SPh | H | O | H | Ga |
| $CO_2H$ | H | SPh | H | O | H | Gb |
| $CO_2Me$ | H | SPh | H | O | H | Ga |
| $CO_2Et$ | H | SPh | H | O | H | Ga |
| $CO_2Pr$-n | H | SPh | H | O | H | Gb |
| $CO_2Bu$-n | H | SPh | H | O | H | Gc |
| $CH_2Cl$ | H | SPh | H | O | H | Ga |
| $CH_2Br$ | H | SPh | H | O | H | Gb |
| $CH_2I$ | H | SPh | H | O | H | Gb |
| $CH_2CF_3$ | H | SPh | H | O | H | Ga |
| $CH_2CN$ | H | SPh | H | O | H | Ga |
| $CH_2OH$ | H | SPh | H | O | H | Gb |
| $CH_2OMe$ | H | SPh | H | O | H | Ga |
| $CH_2OEt$ | H | SPh | H | O | H | Ga |
| $CH_2OPr$-n | H | SPh | H | O | H | Gb |
| $CH_2SMe$ | H | SPh | H | O | H | Ga |
| $CH_2SEt$ | H | SPh | H | O | H | Ga |
| $CH_2SPr$-n | H | SPh | H | O | H | Gb |
| $CH_2SO_2Me$ | H | SPh | H | O | H | Ga |
| $CH_2SO_2Et$ | H | SPh | H | O | H | Ga |
| $CH_2COMe$ | H | SPh | H | O | H | Ga |
| $CH_2COEt$ | H | SPh | H | O | H | Ga |
| $CH_2COPr$-n | H | SPh | H | O | H | Gb |
| $CH_2CO_2H$ | H | SPh | H | O | H | Gb |
| $CH_2CO_2Me$ | H | SPh | H | O | H | Ga |
| $CH_2CO_2Et$ | H | SPh | H | O | H | Ga |

| $R_1$ | $R_2$ | $R_3$ | $R_4$ | X | M | Gn |
|---|---|---|---|---|---|---|
| $CH_2CO_2Pr$-n | H | SPh | H | O | H | Gb |
| $CH_2OCH_2CH=CH_2$ | H | SPh | H | O | H | Gb |
| $CH_2OCH_2C \equiv CH$ | H | SPh | H | O | H | Gb |
| $CH=CHCO_2Me$ | H | SPh | H | O | H | Ga |
| $CH=CHCO_2Et$ | H | SPh | H | O | H | Ga |
| $CH=CHCO_2Pr$-n | H | SPh | H | O | H | Gb |
| CH=CHCN | H | SPh | H | O | H | Ga |
| Ph | H | SPh | H | O | H | Ga |
| $CH_2Ph$ | H | SPh | H | O | H | Ga |
| OPh | H | SPh | H | O | H | Ga |
| SPh | H | SPh | H | O | H | Ga |
| $SO_2Ph$ | H | SPh | H | O | H | Ga |
| CONHMe | H | SPh | H | O | H | Ga |
| CONHEt | H | SPh | H | O | H | Ga |
| $CON(Me)_2$ | H | SPh | H | O | H | Ga |
| $CON(Et)_2$ | H | SPh | H | O | H | Ga |
| F | H | $SO_2Ph$ | H | O | H | Ga |
| Cl | H | $SO_2Ph$ | H | O | H | Ga |
| Br | H | $SO_2Ph$ | H | O | H | Ga |
| I | H | $SO_2Ph$ | H | O | H | Ga |
| $CH=CH_2$ | H | $SO_2Ph$ | H | O | H | Ga |
| $CH_2CH=CH_2$ | H | $SO_2Ph$ | H | O | H | Ga |
| $C \equiv CH$ | H | $SO_2Ph$ | H | O | H | Ga |
| $CH_2C \equiv CH$ | H | $SO_2Ph$ | H | O | H | Ga |
| $CH_2F$ | H | $SO_2Ph$ | H | O | H | Ga |
| $CHF_2$ | H | $SO_2Ph$ | H | O | H | Ga |
| CN | H | $SO_2Ph$ | H | O | H | Ga |
| $NH_2$ | H | $SO_2Ph$ | H | O | H | Ga |
| NHCOMe | H | $SO_2Ph$ | H | O | H | Ga |
| $CH_2NH_2$ | H | $SO_2Ph$ | H | O | H | Ga |
| $CH_2NHCOMe$ | H | $SO_2Ph$ | H | O | H | Ga |
| $CH_2OCHF_2$ | H | $SO_2Ph$ | H | O | H | Ga |
| $CF_3$ | H | $SO_2Ph$ | H | O | H | Ga |
| $NO_2$ | H | $SO_2Ph$ | H | O | H | Gb |
| OMe | H | $SO_2Ph$ | H | O | H | Ga |
| OEt | H | $SO_2Ph$ | H | O | H | Ga |
| OPr-n | H | $SO_2Ph$ | H | O | H | Gb |
| SMe | H | $SO_2Ph$ | H | O | H | Ga |
| SEt | H | $SO_2Ph$ | H | O | H | Ga |
| $SO_2Me$ | H | $SO_2Ph$ | H | O | H | Ga |

| $R_1$ | $R_2$ | $R_3$ | $R_4$ | X | M | Gn |
|---|---|---|---|---|---|---|
| $SO_2Et$ | H | $SO_2Ph$ | H | O | H | Ga |
| COMe | H | $SO_2Ph$ | H | O | H | Ga |
| COEt | H | $SO_2Ph$ | H | O | H | Ga |
| $CO_2H$ | H | $SO_2Ph$ | H | O | H | Gb |
| $CO_2Me$ | H | $SO_2Ph$ | H | O | H | Ga |
| $CO_2Et$ | H | $SO_2Ph$ | H | O | H | Ga |
| $CO_2Pr$-n | H | $SO_2Ph$ | H | O | H | Gb |
| $CO_2Bu$-n | H | $SO_2Ph$ | H | O | H | Gc |
| $CH_2Cl$ | H | $SO_2Ph$ | H | O | H | Ga |
| $CH_2Br$ | H | $SO_2Ph$ | H | O | H | Gb |
| $CH_2I$ | H | $SO_2Ph$ | H | O | H | Gb |
| $CH_2CF_3$ | H | $SO_2Ph$ | H | O | H | Ga |
| $CH_2CN$ | H | $SO_2Ph$ | H | O | H | Ga |
| $CH_2OH$ | H | $SO_2Ph$ | H | O | H | Gb |
| $CH_2OMe$ | H | $SO_2Ph$ | H | O | H | Ga |
| $CH_2OEt$ | H | $SO_2Ph$ | H | O | H | Ga |
| $CH_2OPr$-n | H | $SO_2Ph$ | H | O | H | Gb |
| $CH_2SMe$ | H | $SO_2Ph$ | H | O | H | Ga |
| $CH_2SEt$ | H | $SO_2Ph$ | H | O | H | Ga |
| $CH_2SPr$-n | H | $SO_2Ph$ | H | O | H | Gb |
| $CH_2SO_2Me$ | H | $SO_2Ph$ | H | O | H | Ga |
| $CH_2SO_2Et$ | H | $SO_2Ph$ | H | O | H | Ga |
| $CH_2COMe$ | H | $SO_2Ph$ | H | O | H | Ga |
| $CH_2COEt$ | H | $SO_2Ph$ | H | O | H | Ga |
| $CH_2COPr$-n | H | $SO_2Ph$ | H | O | H | Gb |
| $CH_2CO_2H$ | H | $SO_2Ph$ | H | O | H | Gb |
| $CH_2CO_2Me$ | H | $SO_2Ph$ | H | O | H | Ga |
| $CH_2CO_2Et$ | H | $SO_2Ph$ | H | O | H | Ga |
| $CH_2CO_2Pr$-n | H | $SO_2Ph$ | H | O | H | Gb |
| $CH_2OCH_2CH{=}CH_2$ | H | $SO_2Ph$ | H | O | H | Gb |
| $CH_2OCH_2C{\equiv}CH$ | H | $SO_2Ph$ | H | O | H | Gb |
| $CH{=}CHCO_2Me$ | H | $SO_2Ph$ | H | O | H | Ga |
| $CH{=}CHCO_2Et$ | H | $SO_2Ph$ | H | O | H | Ga |
| $CH{=}CHCO_2Pr$-n | H | $SO_2Ph$ | H | O | H | Gb |
| $CH{=}CHCN$ | H | $SO_2Ph$ | H | O | H | Ga |
| Ph | H | $SO_2Ph$ | H | O | H | Ga |
| $CH_2Ph$ | H | $SO_2Ph$ | H | O | H | Ga |
| OPh | H | $SO_2Ph$ | H | O | H | Ga |
| SPh | H | $SO_2Ph$ | H | O | H | Ga |
| $SO_2Ph$ | H | $SO_2Ph$ | H | O | H | Ga |

| $R_1$ | $R_2$ | $R_3$ | $R_4$ | X | M | Gn |
|---|---|---|---|---|---|---|
| CONHMe | H | $SO_2Ph$ | H | O | H | Ga |
| CONHEt | H | $SO_2Ph$ | H | O | H | Ga |
| $CON(Me)_2$ | H | $SO_2Ph$ | H | O | H | Ga |
| $CON(Et)_2$ | H | $SO_2Ph$ | H | O | H | Ga |
| F | H | $CON(Et)_2$ | H | O | H | Ga |
| Cl | H | $CON(Et)_2$ | H | O | H | Ga |
| Br | H | $CON(Et)_2$ | H | O | H | Ga |
| I | H | $CON(Et)_2$ | H | O | H | Ga |
| $CH=CH_2$ | H | $CON(Et)_2$ | H | O | H | Ga |
| $CH_2CH=CH_2$ | H | $CON(Et)_2$ | H | O | H | Ga |
| $C\equiv CH$ | H | $CON(Et)_2$ | H | O | H | Ga |
| $CH_2C\equiv CH$ | H | $CON(Et)_2$ | H | O | H | Ga |
| $CH_2F$ | H | $CON(Et)_2$ | H | O | H | Ga |
| $CHF_2$ | H | $CON(Et)_2$ | H | O | H | Ga |
| CN | H | $CON(Et)_2$ | H | O | H | Ga |
| $NH_2$ | H | $CON(Et)_2$ | H | O | H | Ga |
| NHCOMe | H | $CON(Et)_2$ | H | O | H | Ga |
| $CH_2NH_2$ | H | $CON(Et)_2$ | H | O | H | Ga |
| $CH_2NHCOMe$ | H | $CON(Et)_2$ | H | O | H | Ga |
| $CH_2OCHF_2$ | H | $CON(Et)_2$ | H | O | H | Ga |
| $CF_3$ | H | $CON(Et)_2$ | H | O | H | Ga |
| $NO_2$ | H | $CON(Et)_2$ | H | O | H | Gb |
| OMe | H | $CON(Et)_2$ | H | O | H | Ga |
| OEt | H | $CON(Et)_2$ | H | O | H | Ga |
| OPr-n | H | $CON(Et)_2$ | H | O | H | Gb |
| SMe | H | $CON(Et)_2$ | H | O | H | Ga |
| SEt | H | $CON(Et)_2$ | H | O | H | Ga |
| $SO_2Me$ | H | $CON(Et)_2$ | H | O | H | Ga |
| $SO_2Et$ | H | $CON(Et)_2$ | H | O | H | Ga |
| COMe | H | $CON(Et)_2$ | H | O | H | Ga |
| COEt | H | $CON(Et)_2$ | H | O | H | Ga |
| $CO_2H$ | H | $CON(Et)_2$ | H | O | H | Gb |
| $CO_2Me$ | H | $CON(Et)_2$ | H | O | H | Ga |
| $CO_2Et$ | H | $CON(Et)_2$ | H | O | H | Ga |
| $CO_2Pr$-n | H | $CON(Et)_2$ | H | O | H | Gb |
| $CO_2Bu$-n | H | $CON(Et)_2$ | H | O | H | Gc |
| $CH_2Cl$ | H | $CON(Et)_2$ | H | O | H | Ga |
| $CH_2Br$ | H | $CON(Et)_2$ | H | O | H | Gb |
| $CH_2I$ | H | $CON(Et)_2$ | H | O | H | Gb |
| $CH_2CF_3$ | H | $CON(Et)_2$ | H | O | H | Ga |

| $R_1$ | $R_2$ | $R_3$ | $R_4$ | X | M | Gn |
|---|---|---|---|---|---|---|
| $CH_2CN$ | H | $CON(Et)_2$ | H | O | H | Ga |
| $CH_2OH$ | H | $CON(Et)_2$ | H | O | H | Gb |
| $CH_2OMe$ | H | $CON(Et)_2$ | H | O | H | Ga |
| $CH_2OEt$ | H | $CON(Et)_2$ | H | O | H | Ga |
| $CH_2OPr$-n | H | $CON(Et)_2$ | H | O | H | Gb |
| $CH_2SMe$ | H | $CON(Et)_2$ | H | O | H | Ga |
| $CH_2SEt$ | H | $CON(Et)_2$ | H | O | H | Ga |
| $CH_2SPr$-n | H | $CON(Et)_2$ | H | O | H | Gb |
| $CH_2SO_2Me$ | H | $CON(Et)_2$ | H | O | H | Ga |
| $CH_2SO_2Et$ | H | $CON(Et)_2$ | H | O | H | Ga |
| $CH_2COMe$ | H | $CON(Et)_2$ | H | O | H | Ga |
| $CH_2COEt$ | H | $CON(Et)_2$ | H | O | H | Ga |
| $CH_2COPr$-n | H | $CON(Et)_2$ | H | O | H | Gb |
| $CH_2CO_2H$ | H | $CON(Et)_2$ | H | O | H | Gb |
| $CH_2CO_2Me$ | H | $CON(Et)_2$ | H | O | H | Ga |
| $CH_2CO_2Et$ | H | $CON(Et)_2$ | H | O | H | Ga |
| $CH_2CO_2Pr$-n | H | $CON(Et)_2$ | H | O | H | Gb |
| $CH_2OCH_2CH=CH_2$ | H | $CON(Et)_2$ | H | O | H | Gb |
| $CH_2OCH_2C\equiv CH$ | H | $CON(Et)_2$ | H | O | H | Gb |
| $CH=CHCO_2Me$ | H | $CON(Et)_2$ | H | O | H | Ga |
| $CH=CHCO_2Et$ | H | $CON(Et)_2$ | H | O | H | Ga |
| $CH=CHCO_2Pr$-n | H | $CON(Et)_2$ | H | O | H | Gb |
| $CH=CHCN$ | H | $CON(Et)_2$ | H | O | H | Ga |
| Ph | H | $CON(Et)_2$ | H | O | H | Ga |
| $CH_2Ph$ | H | $CON(Et)_2$ | H | O | H | Ga |
| OPh | H | $CON(Et)_2$ | H | O | H | Ga |
| SPh | H | $CON(Et)_2$ | H | O | H | Ga |
| $SO_2Ph$ | H | $CON(Et)_2$ | H | O | H | Ga |
| CONHMe | H | $CON(Et)_2$ | H | O | H | Ga |
| CONHEt | H | $CON(Et)_2$ | H | O | H | Ga |
| $CON(Me)_2$ | H | $CON(Et)_2$ | H | O | H | Ga |
| $CON(Et)_2$ | H | $CON(Et)_2$ | H | O | H | Ga |
| F | H | CN | H | O | H | Ga |
| Cl | H | CN | H | O | H | Ga |
| Br | H | CN | H | O | H | Ga |
| I | H | CN | H | O | H | Ga |
| $CH=CH_2$ | H | CN | H | O | H | Ga |
| $CH_2CH=CH_2$ | H | CN | H | O | H | Ga |
| $C\equiv CH$ | H | CN | H | O | H | Ga |
| $CH_2C\equiv CH$ | H | CN | H | O | H | Ga |

| $R_1$ | $R_2$ | $R_3$ | $R_4$ | X | M | Gn |
|---|---|---|---|---|---|---|
| $CH_2F$ | H | CN | H | O | H | Ga |
| $CHF_2$ | H | CN | H | O | H | Ga |
| CN | H | CN | H | O | H | Ga |
| $NH_2$ | H | CN | H | O | H | Ga |
| NHCOMe | H | CN | H | O | H | Ga |
| $CH_2NH_2$ | H | CN | H | O | H | Ga |
| $CH_2NHCOMe$ | H | CN | H | O | H | Ga |
| $CH_2OCHF_2$ | H | CN | H | O | H | Ga |
| $CF_3$ | H | CN | H | O | H | Ga |
| $NO_2$ | H | CN | H | O | H | Gb |
| OMe | H | CN | H | O | H | Ga |
| OEt | H | CN | H | O | H | Ga |
| OPr-n | H | CN | H | O | H | Gb |
| SMe | H | CN | H | O | H | Ga |
| SEt | H | CN | H | O | H | Ga |
| $SO_2Me$ | H | CN | H | O | H | Ga |
| $SO_2Et$ | H | CN | H | O | H | Ga |
| COMe | H | CN | H | O | H | Ga |
| COEt | H | CN | H | O | H | Ga |
| $CO_2H$ | H | CN | H | O | H | Gb |
| $CO_2Me$ | H | CN | H | O | H | Ga |
| $CO_2Et$ | H | CN | H | O | H | Ga |
| $CO_2Pr$-n | H | CN | H | O | H | Gb |
| $CO_2Bu$-n | H | CN | H | O | H | Gc |
| $CH_2Cl$ | H | CN | H | O | H | Ga |
| $CH_2Br$ | H | CN | H | O | H | Gb |
| $CH_2I$ | H | CN | H | O | H | Gb |
| $CH_2CF_3$ | H | CN | H | O | H | Ga |
| $CH_2CN$ | H | CN | H | O | H | Ga |
| $CH_2OH$ | H | CN | H | O | H | Gb |
| $CH_2OMe$ | H | CN | H | O | H | Ga |
| $CH_2OEt$ | H | CN | H | O | H | Ga |
| $CH_2OPr$-n | H | CN | H | O | H | Gb |
| $CH_2SMe$ | H | CN | H | O | H | Ga |
| $CH_2SEt$ | H | CN | H | O | H | Ga |
| $CH_2SPr$-n | H | CN | H | O | H | Gb |
| $CH_2SO_2Me$ | H | CN | H | O | H | Ga |
| $CH_2SO_2Et$ | H | CN | H | O | H | Ga |
| $CH_2COMe$ | H | CN | H | O | H | Ga |
| $CH_2COEt$ | H | CN | H | O | H | Ga |

| $R_1$ | $R_2$ | $R_3$ | $R_4$ | X | M | Gn |
|---|---|---|---|---|---|---|
| $CH_2COPr$-n | H | CN | H | O | H | Gb |
| $CH_2CO_2H$ | H | CN | H | O | H | Gb |
| $CH_2CO_2Me$ | H | CN | H | O | H | Ga |
| $CH_2CO_2Et$ | H | CN | H | O | H | Ga |
| $CH_2CO_2Pr$-n | H | CN | H | O | H | Gb |
| $CH_2OCH_2CH=CH_2$ | H | CN | H | O | H | Gb |
| $CH_2OCH_2C\equiv CH$ | H | CN | H | O | H | Gb |
| $CH=CHCO_2Me$ | H | CN | H | O | H | Ga |
| $CH=CHCO_2Et$ | H | CN | H | O | H | Ga |
| $CH=CHCO_2Pr$-n | H | CN | H | O | H | Gb |
| CH=CHCN | H | CN | H | O | H | Ga |
| Ph | H | CN | H | O | H | Ga |
| $CH_2Ph$ | H | CN | H | O | H | Ga |
| OPh | H | CN | H | O | H | Ga |
| SPh | H | CN | H | O | H | Ga |
| $SO_2Ph$ | H | CN | H | O | H | Ga |
| CONHMe | H | CN | H | O | H | Ga |
| CONHEt | H | CN | H | O | H | Ga |
| $CON(Me)_2$ | H | CN | H | O | H | Ga |
| $CON(Et)_2$ | H | CN | H | O | H | Ga |
| F | H | Et | H | O | H | Ga |
| Cl | H | Et | H | O | H | Ga |
| Br | H | Et | H | O | H | Ga |
| I | H | Et | H | O | H | Ga |
| $CH=CH_2$ | H | Et | H | O | H | Ga |
| $CH_2CH=CH_2$ | H | Et | H | O | H | Ga |
| $C\equiv CH$ | H | Et | H | O | H | Ga |
| $CH_2C\equiv CH$ | H | Et | H | O | H | Ga |
| $CH_2F$ | H | Et | H | O | H | Ga |
| $CHF_2$ | H | Et | H | O | H | Ga |
| CN | H | Et | H | O | H | Ga |
| $NH_2$ | H | Et | H | O | H | Ga |
| NHCOMe | H | Et | H | O | H | Ga |
| $CH_2NH_2$ | H | Et | H | O | H | Ga |
| $CH_2NHCOMe$ | H | Et | H | O | H | Ga |
| $CH_2OCHF_2$ | H | Et | H | O | H | Ga |
| $CF_3$ | H | Et | H | O | H | Ga |
| $NO_2$ | H | Et | H | O | H | Gb |
| OMe | H | Et | H | O | H | Ga |
| OEt | H | Et | H | O | H | Ga |

| $R_1$ | $R_2$ | $R_3$ | $R_4$ | X | M | Gn |
|---|---|---|---|---|---|---|
| OPr-n | H | Et | H | O | H | Gb |
| SMe | H | Et | H | O | H | Ga |
| SEt | H | Et | H | O | H | Ga |
| $SO_2Me$ | H | Et | H | O | H | Ga |
| $SO_2Et$ | H | Et | H | O | H | Ga |
| COMe | H | Et | H | O | H | Ga |
| COEt | H | Et | H | O | H | Ga |
| $CO_2H$ | H | Et | H | O | H | Gb |
| $CO_2Me$ | H | Et | H | O | H | Ga |
| $CO_2Et$ | H | Et | H | O | H | Ga |
| $CO_2Pr$-n | H | Et | H | O | H | Gb |
| $CO_2Bu$-n | H | Et | H | O | H | Gc |
| $CH_2Cl$ | H | Et | H | O | H | Ga |
| $CH_2Br$ | H | Et | H | O | H | Gb |
| $CH_2I$ | H | Et | H | O | H | Gb |
| $CH_2CF_3$ | H | Et | H | O | H | Ga |
| $CH_2CN$ | H | Et | H | O | H | Ga |
| $CH_2OH$ | H | Et | H | O | H | Gb |
| $CH_2OMe$ | H | Et | H | O | H | Ga |
| $CH_2OEt$ | H | Et | H | O | H | Ga |
| $CH_2OPr$-n | H | Et | H | O | H | Gb |
| $CH_2SMe$ | H | Et | H | O | H | Ga |
| $CH_2SEt$ | H | Et | H | O | H | Ga |
| $CH_2SPr$-n | H | Et | H | O | H | Gb |
| $CH_2SO_2Me$ | H | Et | H | O | H | Ga |
| $CH_2SO_2Et$ | H | Et | H | O | H | Ga |
| $CH_2COMe$ | H | Et | H | O | H | Ga |
| $CH_2COEt$ | H | Et | H | O | H | Ga |
| $CH_2COPr$-n | H | Et | H | O | H | Gb |
| $CH_2CO_2H$ | H | Et | H | O | H | Gb |
| $CH_2CO_2Me$ | H | Et | H | O | H | Ga |
| $CH_2CO_2Et$ | H | Et | H | O | H | Ga |
| $CH_2CO_2Pr$-n | H | Et | H | O | H | Gb |
| $CH_2OCH_2CH=CH_2$ | H | Et | H | O | H | Gb |
| $CH_2OCH_2C\equiv CH$ | H | Et | H | O | H | Gb |
| $CH=CHCO_2Me$ | H | Et | H | O | H | Ga |
| $CH=CHCO_2Et$ | H | Et | H | O | H | Ga |
| $CH=CHCO_2Pr$-n | H | Et | H | O | H | Gb |
| CH=CHCN | H | Et | H | O | H | Ga |
| Ph | H | Et | H | O | H | Ga |

| $R_1$ | $R_2$ | $R_3$ | $R_4$ | X | M | Gn |
|---|---|---|---|---|---|---|
| $CH_2Ph$ | H | Et | H | O | H | Ga |
| OPh | H | Et | H | O | H | Ga |
| SPh | H | Et | H | O | H | Ga |
| $SO_2Ph$ | H | Et | H | O | H | Ga |
| CONHMe | H | Et | H | O | H | Ga |
| CONHEt | H | Et | H | O | H | Ga |
| $CON(Me)_2$ | H | Et | H | O | H | Ga |
| $CON(Et)_2$ | H | Et | H | O | H | Ga |
| Cl | Cl | H | H | O | H | Ga |
| Cl | Cl | H | H | S | H | G3 |
| H | Cl | Cl | H | O | H | Ga |
| H | Cl | Cl | H | S | H | G3 |
| Br | Br | H | H | O | H | Ga |
| Br | Br | H | H | S | H | G3 |
| Br | H | Br | H | O | H | Ga |
| Br | H | Br | H | S | H | G3 |
| H | Br | Br | H | O | H | Ga |
| H | Br | Br | H | S | H | G3 |
| H | Cl | Me | H | O | H | Ga |
| H | Cl | Me | H | S | H | G3 |
| Me | Cl | H | H | O | H | Ga |
| Me | Cl | H | H | S | H | G3 |
| H | Br | Me | H | O | H | Ga |
| H | Br | Me | H | S | H | G3 |
| Me | Br | H | H | O | H | Ga |
| Me | Br | H | H | S | H | G3 |

| $R_3$ | $R_2$ | $R_1$ | $R_4$ | X | M | Gn |
|---|---|---|---|---|---|---|
| F | H | Me | H | O | H | Ga |
| Cl | H | Me | H | O | H | Ga |
| Br | H | Me | H | O | H | Ga |
| I | H | Me | H | O | H | Ga |
| $CH=CH_2$ | H | Me | H | O | H | Ga |
| $CH_2CH=CH_2$ | H | Me | H | O | H | Ga |
| $C\equiv CH$ | H | Me | H | O | H | Ga |
| $CH_2C\equiv CH$ | H | Me | H | O | H | Ga |
| $CH_2F$ | H | Me | H | O | H | Ga |
| $CHF_2$ | H | Me | H | O | H | Ga |
| CN | H | Me | H | O | H | Ga |
| $NH_2$ | H | Me | H | O | H | Ga |
| NHCOMe | H | Me | H | O | H | Ga |
| $CH_2NH_2$ | H | Me | H | O | H | Ga |
| $CH_2NHCOMe$ | H | Me | H | O | H | Ga |
| $CH_2OCHF_2$ | H | Me | H | O | H | Ga |
| $CF_3$ | H | Me | H | O | H | Ga |
| $NO_2$ | H | Me | H | O | H | Gb |
| OMe | H | Me | H | O | H | Ga |
| OEt | H | Me | H | O | H | Ga |
| OPr-n | H | Me | H | O | H | Gb |
| SMe | H | Me | H | O | H | Ga |
| SEt | H | Me | H | O | H | Ga |
| $SO_2Me$ | H | Me | H | O | H | Ga |
| $SO_2Et$ | H | Me | H | O | H | Ga |
| COMe | H | Me | H | O | H | Ga |
| COEt | H | Me | H | O | H | Ga |
| $CO_2H$ | H | Me | H | O | H | Gb |
| $CO_2Me$ | H | Me | H | O | H | Ga |
| $CO_2Et$ | H | Me | H | O | H | Ga |
| $CO_2Pr$-n | H | Me | H | O | H | Gb |
| $CO_2Bu$-n | H | Me | H | O | H | Gc |
| $CH_2Cl$ | H | Me | H | O | H | Ga |
| $CH_2Br$ | H | Me | H | O | H | Gb |
| $CH_2I$ | H | Me | H | O | H | Gb |
| $CH_2CF_3$ | H | Me | H | O | H | Ga |
| $CH_2CN$ | H | Me | H | O | H | Ga |
| $CH_2OH$ | H | Me | H | O | H | Gb |
| $CH_2OMe$ | H | Me | H | O | H | Ga |
| $CH_2OEt$ | H | Me | H | O | H | Ga |

| $R_3$ | $R_2$ | $R_1$ | $R_4$ | X | M | Gn |
|---|---|---|---|---|---|---|
| $CH_2OPr$-n | H | Me | H | O | H | Gb |
| $CH_2SMe$ | H | Me | H | O | H | Ga |
| $CH_2SEt$ | H | Me | H | O | H | Ga |
| $CH_2SPr$-n | H | Me | H | O | H | Gb |
| $CH_2SO_2Me$ | H | Me | H | O | H | Ga |
| $CH_2SO_2Et$ | H | Me | H | O | H | Ga |
| $CH_2COMe$ | H | Me | H | O | H | Ga |
| $CH_2COEt$ | H | Me | H | O | H | Ga |
| $CH_2COPr$-n | H | Me | H | O | H | Gb |
| $CH_2CO_2H$ | H | Me | H | O | H | Gb |
| $CH_2CO_2Me$ | H | Me | H | O | H | Ga |
| $CH_2CO_2Et$ | H | Me | H | O | H | Ga |
| $CH_2CO_2Pr$-n | H | Me | H | O | H | Gb |
| $CH_2OCH_2CH=CH_2$ | H | Me | H | O | H | Gb |
| $CH_2OCH_2C\equiv CH$ | H | Me | H | O | H | Gb |
| $CH=CHCO_2Me$ | H | Me | H | O | H | Ga |
| $CH=CHCO_2Et$ | H | Me | H | O | H | Ga |
| $CH=CHCO_2Pr$-n | H | Me | H | O | H | Gb |
| CH=CHCN | H | Me | H | O | H | Ga |
| Ph | H | Me | H | O | H | Ga |
| $CH_2Ph$ | H | Me | H | O | H | Ga |
| OPh | H | Me | H | O | H | Ga |
| SPh | H | Me | H | O | H | Ga |
| $SO_2Ph$ | H | Me | H | O | H | Ga |
| CONHMe | H | Me | H | O | H | Ga |
| CONHEt | H | Me | H | O | H | Ga |
| $CON(Me)_2$ | H | Me | H | O | H | Ga |
| $CON(Et)_2$ | H | Me | H | O | H | Ga |
| F | Me | H | H | O | H | Ga |
| Cl | Me | H | H | O | H | Ga |
| Br | Me | H | H | O | H | Ga |
| I | Me | H | H | O | H | Ga |
| $CH=CH_2$ | Me | H | H | O | H | Ga |
| $CH_2CH=CH_2$ | Me | H | H | O | H | Ga |
| $C\equiv CH$ | Me | H | H | O | H | Ga |
| $CH_2C\equiv CH$ | Me | H | H | O | H | Ga |
| $CH_2F$ | Me | H | H | O | H | Ga |
| $CHF_2$ | Me | H | H | O | H | Ga |
| CN | Me | H | H | O | H | Ga |
| $NH_2$ | Me | H | H | O | H | Ga |

| $R_3$ | $R_2$ | $R_1$ | $R_4$ | X | M | Gn |
|---|---|---|---|---|---|---|
| NHCOMe | Me | H | H | O | H | Ga |
| $CH_2NH_2$ | Me | H | H | O | H | Ga |
| $CH_2NHCOMe$ | Me | H | H | O | H | Ga |
| $CH_2OCHF_2$ | Me | H | H | O | H | Ga |
| $CF_3$ | Me | H | H | O | H | Ga |
| $NO_2$ | Me | H | H | O | H | Gb |
| OMe | Me | H | H | O | H | Ga |
| OEt | Me | H | H | O | H | Ga |
| OPr-n | Me | H | H | O | H | Gb |
| SMe | Me | H | H | O | H | Ga |
| SEt | Me | H | H | O | H | Ga |
| $SO_2Me$ | Me | H | H | O | H | Ga |
| $SO_2Et$ | Me | H | H | O | H | Ga |
| COMe | Me | H | H | O | H | Ga |
| COEt | Me | H | H | O | H | Ga |
| $CO_2H$ | Me | H | H | O | H | Gb |
| $CO_2Me$ | Me | H | H | O | H | Ga |
| $CO_2Et$ | Me | H | H | O | H | Ga |
| $CO_2Pr$-n | Me | H | H | O | H | Gb |
| $CO_2Bu$-n | Me | H | H | O | H | Gc |
| $CH_2Cl$ | Me | H | H | O | H | Ga |
| $CH_2Br$ | Me | H | H | O | H | Gb |
| $CH_2I$ | Me | H | H | O | H | Gb |
| $CH_2CF_3$ | Me | H | H | O | H | Ga |
| $CH_2CN$ | Me | H | H | O | H | Ga |
| $CH_2OH$ | Me | H | H | O | H | Gb |
| $CH_2OMe$ | Me | H | H | O | H | Ga |
| $CH_2OEt$ | Me | H | H | O | H | Ga |
| $CH_2OPr$-n | Me | H | H | O | H | Gb |
| $CH_2SMe$ | Me | H | H | O | H | Ga |
| $CH_2SEt$ | Me | H | H | O | H | Ga |
| $CH_2SPr$-n | Me | H | H | O | H | Gb |
| $CH_2SO_2Me$ | Me | H | H | O | H | Ga |
| $CH_2SO_2Et$ | Me | H | H | O | H | Ga |
| $CH_2COMe$ | Me | H | H | O | H | Ga |
| $CH_2COEt$ | Me | H | H | O | H | Ga |
| $CH_2COPr$-n | Me | H | H | O | H | Gb |
| $CH_2CO_2H$ | Me | H | H | O | H | Gb |
| $CH_2CO_2Me$ | Me | H | H | O | H | Ga |
| $CH_2CO_2Et$ | Me | H | H | O | H | Ga |

| $R_3$ | $R_2$ | $R_1$ | $R_4$ | X | M | Gn |
|---|---|---|---|---|---|---|
| $CH_2CO_2Pr$-n | Me | H | H | O | H | Gb |
| $CH_2OCH_2CH=CH_2$ | Me | H | H | O | H | Gb |
| $CH_2OCH_2C\equiv CH$ | Me | H | H | O | H | Gb |
| $CH=CHCO_2Me$ | Me | H | H | O | H | Ga |
| $CH=CHCO_2Et$ | Me | H | H | O | H | Ga |
| $CH=CHCO_2Pr$-n | Me | H | H | O | H | Gb |
| CH=CHCN | Me | H | H | O | H | Ga |
| Ph | Me | H | H | O | H | Ga |
| $CH_2Ph$ | Me | H | H | O | H | Ga |
| OPh | Me | H | H | O | H | Ga |
| SPh | Me | H | H | O | H | Ga |
| $SO_2Ph$ | Me | H | H | O | H | Ga |
| CONHMe | Me | H | H | O | H | Ga |
| CONHEt | Me | H | H | O | H | Ga |
| $CON(Me)_2$ | Me | H | H | O | H | Ga |
| $CON(Et)_2$ | Me | H | H | O | H | Ga |
| F | Et | H | H | O | H | Ga |
| Cl | Et | H | H | O | H | Ga |
| Br | Et | H | H | O | H | Ga |
| I | Et | H | H | O | H | Ga |
| $CH=CH_2$ | Et | H | H | O | H | Ga |
| $CH_2CH=CH_2$ | Et | H | H | O | H | Ga |
| $C\equiv CH$ | Et | H | H | O | H | Ga |
| $CH_2C\equiv CH$ | Et | H | H | O | H | Ga |
| $CH_2F$ | Et | H | H | O | H | Ga |
| $CHF_2$ | Et | H | H | O | H | Ga |
| CN | Et | H | H | O | H | Ga |
| $NH_2$ | Et | H | H | O | H | Ga |
| NHCOMe | Et | H | H | O | H | Ga |
| $CH_2NH_2$ | Et | H | H | O | H | Ga |
| $CH_2NHCOMe$ | Et | H | H | O | H | Ga |
| $CH_2OCHF_2$ | Et | H | H | O | H | Ga |
| $CF_3$ | Et | H | H | O | H | Ga |
| $NO_2$ | Et | H | H | O | H | Gb |
| OMe | Et | H | H | O | H | Ga |
| OEt | Et | H | H | O | H | Ga |
| OPr-n | Et | H | H | O | H | Gb |
| SMe | Et | H | H | O | H | Ga |
| SEt | Et | H | H | O | H | Ga |
| $SO_2Me$ | Et | H | H | O | H | Ga |

| $R_3$ | $R_2$ | $R_1$ | $R_4$ | X | M | Gn |
|---|---|---|---|---|---|---|
| $SO_2Et$ | Et | H | H | O | H | Ga |
| COMe | Et | H | H | O | H | Ga |
| COEt | Et | H | H | O | H | Ga |
| $CO_2H$ | Et | H | H | O | H | Gb |
| $CO_2Me$ | Et | H | H | O | H | Ga |
| $CO_2Et$ | Et | H | H | O | H | Ga |
| $CO_2Pr$-n | Et | H | H | O | H | Gb |
| $CO_2Bu$-n | Et | H | H | O | H | Gc |
| $CH_2Cl$ | Et | H | H | O | H | Ga |
| $CH_2Br$ | Et | H | H | O | H | Gb |
| $CH_2I$ | Et | H | H | O | H | Gb |
| $CH_2CF_3$ | Et | H | H | O | H | Ga |
| $CH_2CN$ | Et | H | H | O | H | Ga |
| $CH_2OH$ | Et | H | H | O | H | Gb |
| $CH_2OMe$ | Et | H | H | O | H | Ga |
| $CH_2OEt$ | Et | H | H | O | H | Ga |
| $CH_2OPr$-n | Et | H | H | O | H | Gb |
| $CH_2SMe$ | Et | H | H | O | H | Ga |
| $CH_2SEt$ | Et | H | H | O | H | Ga |
| $CH_2SPr$-n | Et | H | H | O | H | Gb |
| $CH_2SO_2Me$ | Et | H | H | O | H | Ga |
| $CH_2SO_2Et$ | Et | H | H | O | H | Ga |
| $CH_2COMe$ | Et | H | H | O | H | Ga |
| $CH_2COEt$ | Et | H | H | O | H | Ga |
| $CH_2COPr$-n | Et | H | H | O | H | Gb |
| $CH_2CO_2H$ | Et | H | H | O | H | Gb |
| $CH_2CO_2Me$ | Et | H | H | O | H | Ga |
| $CH_2CO_2Et$ | Et | H | H | O | H | Ga |
| $CH_2CO_2Pr$-n | Et | H | H | O | H | Gb |
| $CH_2OCH_2CH=CH_2$ | Et | H | H | O | H | Gb |
| $CH_2OCH_2C\equiv CH$ | Et | H | H | O | H | Gb |
| $CH=CHCO_2Me$ | Et | H | H | O | H | Ga |
| $CH=CHCO_2Et$ | Et | H | H | O | H | Ga |
| $CH=CHCO_2Pr$-n | Et | H | H | O | H | Gb |
| CH=CHCN | Et | H | H | O | H | Ga |
| Ph | Et | H | H | O | H | Ga |
| $CH_2Ph$ | Et | H | H | O | H | Ga |
| OPh | Et | H | H | O | H | Ga |
| SPh | Et | H | H | O | H | Ga |
| $SO_2Ph$ | Et | H | H | O | H | Ga |

| $R_3$ | $R_2$ | $R_1$ | $R_4$ | X | M | Gn |
|---|---|---|---|---|---|---|
| CONHMe | Et | H | H | O | H | Ga |
| CONHEt | Et | H | H | O | H | Ga |
| $CON(Me)_2$ | Et | H | H | O | H | Ga |
| $CON(Et)_2$ | Et | H | H | O | H | Ga |
| F | H | Pr-n | H | O | H | Ga |
| Cl | H | Pr-n | H | O | H | Ga |
| Br | H | Pr-n | H | O | H | Ga |
| I | H | Pr-n | H | O | H | Ga |
| $CH=CH_2$ | H | Pr-n | H | O | H | Ga |
| $CH_2CH=CH_2$ | H | Pr-n | H | O | H | Ga |
| $C\equiv CH$ | H | Pr-n | H | O | H | Ga |
| $CH_2C\equiv CH$ | H | Pr-n | H | O | H | Ga |
| $CH_2F$ | H | Pr-n | H | O | H | Ga |
| $CHF_2$ | H | Pr-n | H | O | H | Ga |
| CN | H | Pr-n | H | O | H | Ga |
| $NH_2$ | H | Pr-n | H | O | H | Ga |
| NHCOMe | H | Pr-n | H | O | H | Ga |
| $CH_2NH_2$ | H | Pr-n | H | O | H | Ga |
| $CH_2NHCOMe$ | H | Pr-n | H | O | H | Ga |
| $CH_2OCHF_2$ | H | Pr-n | H | O | H | Ga |
| $CF_3$ | H | Pr-n | H | O | H | Ga |
| $NO_2$ | H | Pr-n | H | O | H | Gb |
| OMe | H | Pr-n | H | O | H | Ga |
| OEt | H | Pr-n | H | O | H | Ga |
| OPr-n | H | Pr-n | H | O | H | Gb |
| SMe | H | Pr-n | H | O | H | Ga |
| SEt | H | Pr-n | H | O | H | Ga |
| $SO_2Me$ | H | Pr-n | H | O | H | Ga |
| $SO_2Et$ | H | Pr-n | H | O | H | Ga |
| COMe | H | Pr-n | H | O | H | Ga |
| COEt | H | Pr-n | H | O | H | Ga |
| $CO_2H$ | H | Pr-n | H | O | H | Gb |
| $CO_2Me$ | H | Pr-n | H | O | H | Ga |
| $CO_2Et$ | H | Pr-n | H | O | H | Ga |
| $CO_2Pr$-n | H | Pr-n | H | O | H | Gb |
| $CO_2Bu$-n | H | Pr-n | H | O | H | Gc |
| $CH_2Cl$ | H | Pr-n | H | O | H | Ga |
| $CH_2Br$ | H | Pr-n | H | O | H | Gb |
| $CH_2I$ | H | Pr-n | H | O | H | Gb |
| $CH_2CF_3$ | H | Pr-n | H | O | H | Ga |

| $R_3$ | $R_2$ | $R_1$ | $R_4$ | X | M | Gn |
|---|---|---|---|---|---|---|
| $CH_2CN$ | H | Pr-n | H | O | H | Ga |
| $CH_2OH$ | H | Pr-n | H | O | H | Gb |
| $CH_2OMe$ | H | Pr-n | H | O | H | Ga |
| $CH_2OEt$ | H | Pr-n | H | O | H | Ga |
| $CH_2OPr$-n | H | Pr-n | H | O | H | Gb |
| $CH_2SMe$ | H | Pr-n | H | O | H | Ga |
| $CH_2SEt$ | H | Pr-n | H | O | H | Ga |
| $CH_2SPr$-n | H | Pr-n | H | O | H | Gb |
| $CH_2SO_2Me$ | H | Pr-n | H | O | H | Ga |
| $CH_2SO_2Et$ | H | Pr-n | H | O | H | Ga |
| $CH_2COMe$ | H | Pr-n | H | O | H | Ga |
| $CH_2COEt$ | H | Pr-n | H | O | H | Ga |
| $CH_2COPr$-n | H | Pr-n | H | O | H | Gb |
| $CH_2CO_2H$ | H | Pr-n | H | O | H | Gb |
| $CH_2CO_2Me$ | H | Pr-n | H | O | H | Ga |
| $CH_2CO_2Et$ | H | Pr-n | H | O | H | Ga |
| $CH_2CO_2Pr$-n | H | Pr-n | H | O | H | Gb |
| $CH_2OCH_2CH=CH_2$ | H | Pr-n | H | O | H | Gb |
| $CH_2OCH_2C\equiv CH$ | H | Pr-n | H | O | H | Gb |
| $CH=CHCO_2Me$ | H | Pr-n | H | O | H | Ga |
| $CH=CHCO_2Et$ | H | Pr-n | H | O | H | Ga |
| $CH=CHCO_2Pr$-n | H | Pr-n | H | O | H | Gb |
| CH=CHCN | H | Pr-n | H | O | H | Ga |
| Ph | H | Pr-n | H | O | H | Ga |
| $CH_2Ph$ | H | Pr-n | H | O | H | Ga |
| OPh | H | Pr-n | H | O | H | Ga |
| SPh | H | Pr-n | H | O | H | Ga |
| $SO_2Ph$ | H | Pr-n | H | O | H | Ga |
| CONHMe | H | Pr-n | H | O | H | Ga |
| CONHEt | H | Pr-n | H | O | H | Ga |
| $CON(Me)_2$ | H | Pr-n | H | O | H | Ga |
| $CON(Et)_2$ | H | Pr-n | H | O | H | Ga |
| F | H | Cl | H | O | H | Ga |
| Cl | H | Cl | H | O | H | Ga |
| Br | H | Cl | H | O | H | Ga |
| I | H | Cl | H | O | H | Ga |
| $CH=CH_2$ | H | Cl | H | O | H | Ga |
| $CH_2CH=CH_2$ | H | Cl | H | O | H | Ga |
| $C\equiv CH$ | H | Cl | H | O | H | Ga |
| $CH_2C\equiv CH$ | H | Cl | H | O | H | Ga |

| $R_3$ | $R_2$ | $R_1$ | $R_4$ | X | M | Gn |
|---|---|---|---|---|---|---|
| $CH_2F$ | H | Cl | H | O | H | Ga |
| $CHF_2$ | H | Cl | H | O | H | Ga |
| CN | H | Cl | H | O | H | Ga |
| $NH_2$ | H | Cl | H | O | H | Ga |
| NHCOMe | H | Cl | H | O | H | Ga |
| $CH_2NH_2$ | H | Cl | H | O | H | Ga |
| $CH_2NHCOMe$ | H | Cl | H | O | H | Ga |
| $CH_2OCHF_2$ | H | Cl | H | O | H | Ga |
| $CF_3$- | H | Cl | H | O | H | Ga |
| $NO_2$ | H | Cl | H | O | H | Gb |
| OMe | H | Cl | H | O | H | Ga |
| OEt | H | Cl | H | O | H | Ga |
| OPr-n | H | Cl | H | O | H | Gb |
| SMe | H | Cl | H | O | H | Ga |
| SEt | H | Cl | H | O | H | Ga |
| $SO_2Me$ | H | Cl | H | O | H | Ga |
| $SO_2Et$ | H | Cl | H | O | H | Ga |
| COMe | H | Cl | H | O | H | Ga |
| COEt | H | Cl | H | O | H | Ga |
| $CO_2H$ | H | Cl | H | O | H | Gb |
| $CO_2Me$ | H | Cl | H | O | H | Ga |
| $CO_2Et$ | H | Cl | H | O | H | Ga |
| $CO_2Pr$-n | H | Cl | H | O | H | Gb |
| $CO_2Bu$-n | H | Cl | H | O | H | Gc |
| $CH_2Cl$ | H | Cl | H | O | H | Ga |
| $CH_2Br$ | H | Cl | H | O | H | Gb |
| $CH_2I$ | H | Cl | H | O | H | Gb |
| $CH_2CF_3$ | H | Cl | H | O | H | Ga |
| $CH_2CN$ | H | Cl | H | O | H | Ga |
| $CH_2OH$ | H | Cl | H | O | H | Gb |
| $CH_2OMe$ | H | Cl | H | O | H | Ga |
| $CH_2OEt$ | H | Cl | H | O | H | Ga |
| $CH_2OPr$-n | H | Cl | H | O | H | Gb |
| $CH_2SMe$ | H | Cl | H | O | H | Ga |
| $CH_2SEt$ | H | Cl | H | O | H | Ga |
| $CH_2SPr$-n | H | Cl | H | O | H | Gb |
| $CH_2SO_2Me$ | H | Cl | H | O | H | Ga |
| $CH_2SO_2Et$ | H | Cl | H | O | H | Ga |
| $CH_2COMe$ | H | Cl | H | O | H | Ga |
| $CH_2COEt$ | H | Cl | H | O | H | Ga |

| $R_3$ | $R_2$ | $R_1$ | $R_4$ | X | M | Gn |
|---|---|---|---|---|---|---|
| $CH_2COPr$-n | H | Cl | H | O | H | Gb |
| $CH_2CO_2H$ | H | Cl | H | O | H | Gb |
| $CH_2CO_2Me$ | H | Cl | H | O | H | Ga |
| $CH_2CO_2Et$ | H | Cl | H | O | H | Ga |
| $CH_2CO_2Pr$-n | H | Cl | H | O | H | Gb |
| $CH_2OCH_2CH=CH_2$ | H | Cl | H | O | H | Gb |
| $CH_2OCH_2C\equiv CH$ | H | Cl | H | O | H | Gb |
| $CH=CHCO_2Me$ | H | Cl | H | O | H | Ga |
| $CH=CHCO_2Et$ | H | Cl | H | O | H | Ga |
| $CH=CHCO_2Pr$-n | H | Cl | H | O | H | Gb |
| $CH=CHCN$ | H | Cl | H | O | H | Ga |
| Ph | H | Cl | H | O | H | Ga |
| $CH_2Ph$ | H | Cl | H | O | H | Ga |
| OPh | H | Cl | H | O | H | Ga |
| SPh | H | Cl | H | O | H | Ga |
| $SO_2Ph$ | H | Cl | H | O | H | Ga |
| CONHMe | H | Cl | H | O | H | Ga |
| CONHEt | H | Cl | H | O | H | Ga |
| $CON(Me)_2$ | H | Cl | H | O | H | Ga |
| $CON(Et)_2$ | H | Cl | H | O | H | Ga |
| F | H | $NO_2$ | H | O | H | Ga |
| Cl | H | $NO_2$ | H | O | H | Ga |
| Br | H | $NO_2$ | H | O | H | Ga |
| I | H | $NO_2$ | H | O | H | Ga |
| $CH=CH_2$ | H | $NO_2$ | H | O | H | Ga |
| $CH_2CH=CH_2$ | H | $NO_2$ | H | O | H | Ga |
| $C\equiv CH$ | H | $NO_2$ | H | O | H | Ga |
| $CH_2C\equiv CH$ | H | $NO_2$ | H | O | H | Ga |
| $CH_2F$ | H | $NO_2$ | H | O | H | Ga |
| $CHF_2$ | H | $NO_2$ | H | O | H | Ga |
| CN | H | $NO_2$ | H | O | H | Ga |
| $NH_2$ | H | $NO_2$ | H | O | H | Ga |
| NHCOMe | H | $NO_2$ | H | O | H | Ga |
| $CH_2NH_2$ | H | $NO_2$ | H | O | H | Ga |
| $CH_2NHCOMe$ | H | $NO_2$ | H | O | H | Ga |
| $CH_2OCHF_2$ | H | $NO_2$ | H | O | H | Ga |
| $CF_3$ | H | $NO_2$ | H | O | H | Ga |
| $NO_2$ | H | $NO_2$ | H | O | H | Gb |
| OMe | H | $NO_2$ | H | O | H | Ga |
| OEt | H | $NO_2$ | H | O | H | Ga |

| $R_3$ | $R_2$ | $R_1$ | $R_4$ | X | M | Gn |
|---|---|---|---|---|---|---|
| OPr-n | H | $NO_2$ | H | O | H | Gb |
| SMe | H | $NO_2$ | H | O | H | Ga |
| SEt | H | $NO_2$ | H | O | H | Ga |
| $SO_2Me$ | H | $NO_2$ | H | O | H | Ga |
| $SO_2Et$ | H | $NO_2$ | H | O | H | Ga |
| COMe | H | $NO_2$ | H | O | H | Ga |
| COEt | H | $NO_2$ | H | O | H | Ga |
| $CO_2H$ | H | $NO_2$ | H | O | H | Gb |
| $CO_2Me$ | H | $NO_2$ | H | O | H | Ga |
| $CO_2Et$ | H | $NO_2$ | H | O | H | Ga |
| $CO_2Pr$-n | H | $NO_2$ | H | O | H | Gb |
| $CO_2Bu$-n | H | $NO_2$ | H | O | H | Gc |
| $CH_2Cl$ | H | $NO_2$ | H | O | H | Ga |
| $CH_2Br$ | H | $NO_2$ | H | O | H | Gb |
| $CH_2I$ | H | $NO_2$ | H | O | H | Gb |
| $CH_2CF_3$ | H | $NO_2$ | H | O | H | Ga |
| $CH_2CN$ | H | $NO_2$ | H | O | H | Ga |
| $CH_2OH$ | H | $NO_2$ | H | O | H | Gb |
| $CH_2OMe$ | H | $NO_2$ | H | O | H | Ga |
| $CH_2OEt$ | H | $NO_2$ | H | O | H | Ga |
| $CH_2OPr$-n | H | $NO_2$ | H | O | H | Gb |
| $CH_2SMe$ | H | $NO_2$ | H | O | H | Ga |
| $CH_2SEt$ | H | $NO_2$ | H | O | H | Ga |
| $CH_2SPr$-n | H | $NO_2$ | H | O | H | Gb |
| $CH_2SO_2Me$ | H | $NO_2$ | H | O | H | Ga |
| $CH_2SO_2Et$ | H | $NO_2$ | H | O | H | Ga |
| $CH_2COMe$ | H | $NO_2$ | H | O | H | Ga |
| $CH_2COEt$ | H | $NO_2$ | H | O | H | Ga |
| $CH_2COPr$-n | H | $NO_2$ | H | O | H | Gb |
| $CH_2CO_2H$ | H | $NO_2$ | H | O | H | Gb |
| $CH_2CO_2Me$ | H | $NO_2$ | H | O | H | Ga |
| $CH_2CO_2Et$ | H | $NO_2$ | H | O | H | Ga |
| $CH_2CO_2Pr$-n | H | $NO_2$ | H | O | H | Gb |
| $CH_2OCH_2CH=CH_2$ | H | $NO_2$ | H | O | H | Gb |
| $CH_2OCH_2C\equiv CH$ | H | $NO_2$ | H | O | H | Gb |
| $CH=CHCO_2Me$ | H | $NO_2$ | H | O | H | Ga |
| $CH=CHCO_2Et$ | H | $NO_2$ | H | O | H | Ga |
| $CH=CHCO_2Pr$-n | H | $NO_2$ | H | O | H | Gb |
| CH=CHCN | H | $NO_2$ | H | O | H | Ga |
| Ph | H | $NO_2$ | H | O | H | Ga |

| $R_3$ | $R_2$ | $R_1$ | $R_4$ | X | M | Gn |
|---|---|---|---|---|---|---|
| $CH_2Ph$ | H | $NO_2$ | H | O | H | Ga |
| OPh | H | $NO_2$ | H | O | H | Ga |
| SPh | H | $NO_2$ | H | O | H | Ga |
| $SO_2Ph$ | H | $NO_2$ | H | O | H | Ga |
| CONHMe | H | $NO_2$ | H | O | H | Ga |
| CONHEt | H | $NO_2$ | H | O | H | Ga |
| $CON(Me)_2$ | H | $NO_2$ | H | O | H | Ga |
| $CON(Et)_2$ | H | $NO_2$ | H | O | H | Ga |
| F | H | $CF_3$ | H | O | H | Ga |
| Cl | H | $CF_3$ | H | O | H | Ga |
| Br | H | $CF_3$ | H | O | H | Ga |
| I | H | $CF_3$ | H | O | H | Ga |
| $CH=CH_2$ | H | $CF_3$ | H | O | H | Ga |
| $CH_2CH=CH_2$ | H | $CF_3$ | H | O | H | Ga |
| $C\equiv CH$ | H | $CF_3$ | H | O | H | Ga |
| $CH_2C\equiv CH$ | H | $CF_3$ | H | O | H | Ga |
| $CH_2F$ | H | $CF_3$ | H | O | H | Ga |
| $CHF_2$ | H | $CF_3$ | H | O | H | Ga |
| CN | H | $CF_3$ | H | O | H | Ga |
| $NH_2$ | H | $CF_3$ | H | O | H | Ga |
| NHCOMe | H | $CF_3$ | H | O | H | Ga |
| $CH_2NH_2$ | H | $CF_3$ | H | O | H | Ga |
| $CH_2NHCOMe$ | H | $CF_3$ | H | O | H | Ga |
| $CH_2OCHF_2$ | H | $CF_3$ | H | O | H | Ga |
| $CF_3$ | H | $CF_3$ | H | O | H | Ga |
| $NO_2$ | H | $CF_3$ | H | O | H | Gb |
| OMe | H | $CF_3$ | H | O | H | Ga |
| OEt | H | $CF_3$ | H | O | H | Ga |
| OPr-n | H | $CF_3$ | H | O | H | Gb |
| SMe | H | $CF_3$ | H | O | H | Ga |
| SEt | H | $CF_3$ | H | O | H | Ga |
| $SO_2Me$ | H | $CF_3$ | H | O | H | Ga |
| $SO_2Et$ | H | $CF_3$ | H | O | H | Ga |
| COMe | H | $CF_3$ | H | O | H | Ga |
| COEt | H | $CF_3$ | H | O | H | Ga |
| $CO_2H$ | H | $CF_3$ | H | O | H | Gb |
| $CO_2Me$ | H | $CF_3$ | H | O | H | Ga |
| $CO_2Et$ | H | $CF_3$ | H | O | H | Ga |
| $CO_2Pr$-n | H | $CF_3$ | H | O | H | Gb |
| $CO_2Bu$-n | H | $CF_3$ | H | O | H | Gc |

| $R_3$ | $R_2$ | $R_1$ | $R_4$ | X | M | Gn |
|---|---|---|---|---|---|---|
| $CH_2Cl$ | H | $CF_3$ | H | O | H | Ga |
| $CH_2Br$ | H | $CF_3$ | H | O | H | Gb |
| $CH_2I$ | H | $CF_3$ | H | O | H | Gb |
| $CH_2CF_3$ | H | $CF_3$ | H | O | H | Ga |
| $CH_2CN$ | H | $CF_3$ | H | O | H | Ga |
| $CH_2OH$ | H | $CF_3$ | H | O | H | Gb |
| $CH_2OMe$ | H | $CF_3$ | H | O | H | Ga |
| $CH_2OEt$ | H | $CF_3$ | H | O | H | Ga |
| $CH_2OPr$-n | H | $CF_3$ | H | O | H | Gb |
| $CH_2SMe$ | H | $CF_3$ | H | O | H | Ga |
| $CH_2SEt$ | H | $CF_3$ | H | O | H | Ga |
| $CH_2SPr$-n | H | $CF_3$ | H | O | H | Gb |
| $CH_2SO_2Me$ | H | $CF_3$ | H | O | H | Ga |
| $CH_2SO_2Et$ | H | $CF_3$ | H | O | H | Ga |
| $CH_2COMe$ | H | $CF_3$ | H | O | H | Ga |
| $CH_2COEt$ | H | $CF_3$ | H | O | H | Ga |
| $CH_2COPr$-n | H | $CF_3$ | H | O | H | Gb |
| $CH_2CO_2H$ | H | $CF_3$ | H | O | H | Gb |
| $CH_2CO_2Me$ | H | $CF_3$ | H | O | H | Ga |
| $CH_2CO_2Et$ | H | $CF_3$ | H | O | H | Ga |
| $CH_2CO_2Pr$-n | H | $CF_3$ | H | O | H | Gb |
| $CH_2OCH_2CH=CH_2$ | H | $CF_3$ | H | O | H | Gb |
| $CH_2OCH_2C \equiv CH$ | H | $CF_3$ | H | O | H | Gb |
| $CH=CHCO_2Me$ | H | $CF_3$ | H | O | H | Ga |
| $CH=CHCO_2Et$ | H | $CF_3$ | H | O | H | Ga |
| $CH=CHCO_2Pr$-n | H | $CF_3$ | H | O | H | Gb |
| $CH=CHCN$ | H | $CF_3$ | H | O | H | Ga |
| Ph | H | $CF_3$ | H | O | H | Ga |
| $CH_2Ph$ | H | $CF_3$ | H | O | H | Ga |
| OPh | H | $CF_3$ | H | O | H | Ga |
| SPh | H | $CF_3$ | H | O | H | Ga |
| $SO_2Ph$ | H | $CF_3$ | H | O | H | Ga |
| CONHMe | H | $CF_3$ | H | O | H | Ga |
| CONHEt | H | $CF_3$ | H | O | H | Ga |
| $CON(Me)_2$ | H | $CF_3$ | H | O | H | Ga |
| $CON(Et)_2$ | H | $CF_3$ | H | O | H | Ga |
| F | H | OMe | H | O | H | Ga |
| Cl | H | OMe | H | O | H | Ga |
| Br | H | OMe | H | O | H | Ga |
| I | H | OMe | H | O | H | Ga |

| $R_3$ | $R_2$ | $R_1$ | $R_4$ | X | M | Gn |
|---|---|---|---|---|---|---|
| $CH=CH_2$ | H | OMe | H | O | H | Ga |
| $CH_2CH=CH_2$ | H | OMe | H | O | H | Ga |
| $C\equiv CH$ | H | OMe | H | O | H | Ga |
| $CH_2C\equiv CH$ | H | OMe | H | O | H | Ga |
| $CH_2F$ | H | OMe | H | O | H | Ga |
| $CHF_2$ | H | OMe | H | O | H | Ga |
| CN | H | OMe | H | O | H | Ga |
| $NH_2$ | H | OMe | H | O | H | Ga |
| NHCOMe | H | OMe | H | O | H | Ga |
| $CH_2NH_2$ | H | OMe | H | O | H | Ga |
| $CH_2NHCOMe$ | H | OMe | H | O | H | Ga |
| $CH_2OCHF_2$ | H | OMe | H | O | H | Ga |
| $CF_3$ | H | OMe | H | O | H | Ga |
| $NO_2$ | H | OMe | H | O | H | Gb |
| OMe | H | OMe | H | O | H | Ga |
| OEt | H | OMe | H | O | H | Ga |
| OPr-n | H | OMe | H | O | H | Gb |
| SMe | H | OMe | H | O | H | Ga |
| SEt | H | OMe | H | O | H | Ga |
| $SO_2Me$ | H | OMe | H | O | H | Ga |
| $SO_2Et$ | H | OMe | H | O | H | Ga |
| COMe | H | OMe | H | O | H | Ga |
| COEt | H | OMe | H | O | H | Ga |
| $CO_2H$ | H | OMe | H | O | H | Gb |
| $CO_2Me$ | H | OMe | H | O | H | Ga |
| $CO_2Et$ | H | OMe | H | O | H | Ga |
| $CO_2Pr$-n | H | OMe | H | O | H | Gb |
| $CO_2Bu$-n | H | OMe | H | O | H | Gc |
| $CH_2Cl$ | H | OMe | H | O | H | Ga |
| $CH_2Br$ | H | OMe | H | O | H | Gb |
| $CH_2I$ | H | OMe | H | O | H | Gb |
| $CH_2CF_3$ | H | OMe | H | O | H | Ga |
| $CH_2CN$ | H | OMe | H | O | H | Ga |
| $CH_2OH$ | H | OMe | H | O | H | Gb |
| $CH_2OMe$ | H | OMe | H | O | H | Ga |
| $CH_2OEt$ | H | OMe | H | O | H | Ga |
| $CH_2OPr$-n | H | OMe | H | O | H | Gb |
| $CH_2SMe$ | H | OMe | H | O | H | Ga |
| $CH_2SEt$ | H | OMe | H | O | H | Ga |
| $CH_2SPr$-n | H | OMe | H | O | H | Gb |

| $R_3$ | $R_2$ | $R_1$ | $R_4$ | X | M | Gn |
|---|---|---|---|---|---|---|
| $CH_2SO_2Me$ | H | OMe | H | O | H | Ga |
| $CH_2SO_2Et$ | H | OMe | H | O | H | Ga |
| $CH_2COMe$ | H | OMe | H | O | H | Ga |
| $CH_2COEt$ | H | OMe | H | O | H | Ga |
| $CH_2COPr$-n | H | OMe | H | O | H | Gb |
| $CH_2CO_2H$ | H | OMe | H | O | H | Gb |
| $CH_2CO_2Me$ | H | OMe | H | O | H | Ga |
| $CH_2CO_2Et$ | H | OMe | H | O | H | Ga |
| $CH_2CO_2Pr$-n | H | OMe | H | O | H | Gb |
| $CH_2OCH_2CH=CH_2$ | H | OMe | H | O | H | Gb |
| $CH_2OCH_2C\equiv CH$ | H | OMe | H | O | H | Gb |
| $CH=CHCO_2Me$ | H | OMe | H | O | H | Ga |
| $CH=CHCO_2Et$ | H | OMe | H | O | H | Ga |
| $CH=CHCO_2Pr$-n | H | OMe | H | O | H | Gb |
| CH=CHCN | H | OMe | H | O | H | Ga |
| Ph | H | OMe | H | O | H | Ga |
| $CH_2Ph$ | H | OMe | H | O | H | Ga |
| OPh | H | OMe | H | O | H | Ga |
| SPh | H | OMe | H | O | H | Ga |
| $SO_2Ph$ | H | OMe | H | O | H | Ga |
| CONHMe | H | OMe | H | O | H | Ga |
| CONHEt | H | OMe | H | O | H | Ga |
| $CON(Me)_2$ | H | OMe | H | O | H | Ga |
| $CON(Et)_2$ | H | OMe | H | O | H | Ga |
| F | H | SMe | H | O | H | Ga |
| Cl | H | SMe | H | O | H | Ga |
| Br | H | SMe | H | O | H | Ga |
| I | H | SMe | H | O | H | Ga |
| $CH=CH_2$ | H | SMe | H | O | H | Ga |
| $CH_2CH=CH_2$ | H | SMe | H | O | H | Ga |
| $C\equiv CH$ | H | SMe | H | O | H | Ga |
| $CH_2C\equiv CH$ | H | SMe | H | O | H | Ga |
| $CH_2F$ | H | SMe | H | O | H | Ga |
| $CHF_2$ | H | SMe | H | O | H | Ga |
| CN | H | SMe | H | O | H | Ga |
| $NH_2$ | H | SMe | H | O | H | Ga |
| NHCOMe | H | SMe | H | O | H | Ga |
| $CH_2NH_2$ | H | SMe | H | O | H | Ga |
| $CH_2NHCOMe$ | H | SMe | H | O | H | Ga |
| $CH_2OCHF_2$ | H | SMe | H | O | H | Ga |

| $R_3$ | $R_2$ | $R_1$ | $R_4$ | X | M | Gn |
|---|---|---|---|---|---|---|
| $CF_3$ | H | SMe | H | O | H | Ga |
| $NO_2$ | H | SMe | H | O | H | Gb |
| OMe | H | SMe | H | O | H | Ga |
| OEt | H | SMe | H | O | H | Ga |
| OPr-n | H | SMe | H | O | H | Gb |
| SMe | H | SMe | H | O | H | Ga |
| SEt | H | SMe | H | O | H | Ga |
| $SO_2Me$ | H | SMe | H | O | H | Ga |
| $SO_2Et$ | H | SMe | H | O | H | Ga |
| COMe | H | SMe | H | O | H | Ga |
| COEt | H | SMe | H | O | H | Ga |
| $CO_2H$ | H | SMe | H | O | H | Gb |
| $CO_2Me$ | H | SMe | H | O | H | Ga |
| $CO_2Et$ | H | SMe | H | O | H | Ga |
| $CO_2Pr$-n | H | SMe | H | O | H | Gb |
| $CO_2Bu$-n | H | SMe | H | O | H | Gc |
| $CH_2Cl$ | H | SMe | H | O | H | Ga |
| $CH_2Br$ | H | SMe | H | O | H | Gb |
| $CH_2I$ | H | SMe | H | O | H | Gb |
| $CH_2CF_3$ | H | SMe | H | O | H | Ga |
| $CH_2CN$ | H | SMe | H | O | H | Ga |
| $CH_2OH$ | H | SMe | H | O | H | Gb |
| $CH_2OMe$ | H | SMe | H | O | H | Ga |
| $CH_2OEt$ | H | SMe | H | O | H | Ga |
| $CH_2OPr$-n | H | SMe | H | O | H | Gb |
| $CH_2SMe$ | H | SMe | H | O | H | Ga |
| $CH_2SEt$ | H | SMe | H | O | H | Ga |
| $CH_2SPr$-n | H | SMe | H | O | H | Gb |
| $CH_2SO_2Me$ | H | SMe | H | O | H | Ga |
| $CH_2SO_2Et$ | H | SMe | H | O | H | Ga |
| $CH_2COMe$ | H | SMe | H | O | H | Ga |
| $CH_2COEt$ | H | SMe | H | O | H | Ga |
| $CH_2COPr$-n | H | SMe | H | O | H | Gb |
| $CH_2CO_2H$ | H | SMe | H | O | H | Gb |
| $CH_2CO_2Me$ | H | SMe | H | O | H | Ga |
| $CH_2CO_2Et$ | H | SMe | H | O | H | Ga |
| $CH_2CO_2Pr$-n | H | SMe | H | O | H | Gb |
| $CH_2OCH_2CH=CH_2$ | H | SMe | H | O | H | Gb |
| $CH_2OCH_2C\equiv CH$ | H | SMe | H | O | H | Gb |
| $CH=CHCO_2Me$ | H | SMe | H | O | H | Ga |

| $R_3$ | $R_2$ | $R_1$ | $R_4$ | X | M | Gn |
|---|---|---|---|---|---|---|
| $CH=CHCO_2Et$ | H | SMe | H | O | H | Ga |
| $CH=CHCO_2Pr$-n | H | SMe | H | O | H | Gb |
| CH=CHCN | H | SMe | H | O | H | Ga |
| Ph | H | SMe | H | O | H | Ga |
| $CH_2Ph$ | H | SMe | H | O | H | Ga |
| OPh | H | SMe | H | O | H | Ga |
| SPh | H | SMe | H | O | H | Ga |
| $SO_2Ph$ | H | SMe | H | O | H | Ga |
| CONHMe | H | SMe | H | O | H | Ga |
| CONHEt | H | SMe | H | O | H | Ga |
| $CON(Me)_2$ | H | SMe | H | O | H | Ga |
| $CON(Et)_2$ | H | SMe | H | O | H | Ga |
| F | H | $SO_2Me$ | H | O | H | Ga |
| Cl | H | $SO_2Me$ | H | O | H | Ga |
| Br | H | $SO_2Me$ | H | O | H | Ga |
| I | H | $SO_2Me$ | H | O | H | Ga |
| $CH=CH_2$ | H | $SO_2Me$ | H | O | H | Ga |
| $CH_2CH=CH_2$ | H | $SO_2Me$ | H | O | H | Ga |
| C≡CH | H | $SO_2Me$ | H | O | H | Ga |
| $CH_2C≡CH$ | H | $SO_2Me$ | H | O | H | Ga |
| $CH_2F$ | H | $SO_2Me$ | H | O | H | Ga |
| $CHF_2$ | H | $SO_2Me$ | H | O | H | Ga |
| CN | H | $SO_2Me$ | H | O | H | Ga |
| $NH_2$ | H | $SO_2Me$ | H | O | H | Ga |
| NHCOMe | H | $SO_2Me$ | H | O | H | Ga |
| $CH_2NH_2$ | H | $SO_2Me$ | H | O | H | Ga |
| $CH_2NHCOMe$ | H | $SO_2Me$ | H | O | H | Ga |
| $CH_2OCHF_2$ | H | $SO_2Me$ | H | O | H | Ga |
| $CF_3$ | H | $SO_2Me$ | H | O | H | Ga |
| $NO_2$ | H | $SO_2Me$ | H | O | H | Gb |
| OMe | H | $SO_2Me$ | H | O | H | Ga |
| OEt | H | $SO_2Me$ | H | O | H | Ga |
| OPr-n | H | $SO_2Me$ | H | O | H | Gb |
| SMe | H | $SO_2Me$ | H | O | H | Ga |
| SEt | H | $SO_2Me$ | H | O | H | Ga |
| $SO_2Me$ | H | $SO_2Me$ | H | O | H | Ga |
| $SO_2Et$ | H | $SO_2Me$ | H | O | H | Ga |
| COMe | H | $SO_2Me$ | H | O | H | Ga |
| COEt | H | $SO_2Me$ | H | O | H | Ga |
| $CO_2H$ | H | $SO_2Me$ | H | O | H | Gb |

| $R_3$ | $R_2$ | $R_1$ | $R_4$ | X | M | Gn |
|---|---|---|---|---|---|---|
| $CO_2Me$ | H | $SO_2Me$ | H | O | H | Ga |
| $CO_2Et$ | H | $SO_2Me$ | H | O | H | Ga |
| $CO_2Pr$-n | H | $SO_2Me$ | H | O | H | Gb |
| $CO_2Bu$-n | H | $SO_2Me$ | H | O | H | Gc |
| $CH_2Cl$ | H | $SO_2Me$ | H | O | H | Ga |
| $CH_2Br$ | H | $SO_2Me$ | H | O | H | Gb |
| $CH_2I$ | H | $SO_2Me$ | H | O | H | Gb |
| $CH_2CF_3$ | H | $SO_2Me$ | H | O | H | Ga |
| $CH_2CN$ | H | $SO_2Me$ | H | O | H | Ga |
| $CH_2OH$ | H | $SO_2Me$ | H | O | H | Gb |
| $CH_2OMe$ | H | $SO_2Me$ | H | O | H | Ga |
| $CH_2OEt$ | H | $SO_2Me$ | H | O | H | Ga |
| $CH_2OPr$-n | H | $SO_2Me$ | H | O | H | Gb |
| $CH_2SMe$ | H | $SO_2Me$ | H | O | H | Ga |
| $CH_2SEt$ | H | $SO_2Me$ | H | O | H | Ga |
| $CH_2SPr$-n | H | $SO_2Me$ | H | O | H | Gb |
| $CH_2SO_2Me$ | H | $SO_2Me$ | H | O | H | Ga |
| $CH_2SO_2Et$ | H | $SO_2Me$ | H | O | H | Ga |
| $CH_2COMe$ | H | $SO_2Me$ | H | O | H | Ga |
| $CH_2COEt$ | H | $SO_2Me$ | H | O | H | Ga |
| $CH_2COPr$-n | H | $SO_2Me$ | H | O | H | Gb |
| $CH_2CO_2H$ | H | $SO_2Me$ | H | O | H | Gb |
| $CH_2CO_2Me$ | H | $SO_2Me$ | H | O | H | Ga |
| $CH_2CO_2Et$ | H | $SO_2Me$ | H | O | H | Ga |
| $CH_2CO_2Pr$-n | H | $SO_2Me$ | H | O | H | Gb |
| $CH_2OCH_2CH=CH_2$ | H | $SO_2Me$ | H | O | H | Gb |
| $CH_2OCH_2C\equiv CH$ | H | $SO_2Me$ | H | O | H | Gb |
| $CH=CHCO_2Me$ | H | $SO_2Me$ | H | O | H | Ga |
| $CH=CHCO_2Et$ | H | $SO_2Me$ | H | O | H | Ga |
| $CH=CHCO_2Pr$-n | H | $SO_2Me$ | H | O | H | Gb |
| $CH=CHCN$ | H | $SO_2Me$ | H | O | H | Ga |
| Ph | H | $SO_2Me$ | H | O | H | Ga |
| $CH_2Ph$ | H | $SO_2Me$ | H | O | H | Ga |
| OPh | H | $SO_2Me$ | H | O | H | Ga |
| SPh | H | $SO_2Me$ | H | O | H | Ga |
| $SO_2Ph$ | H | $SO_2Me$ | H | O | H | Ga |
| CONHMe | H | $SO_2Me$ | H | O | H | Ga |
| CONHEt | H | $SO_2Me$ | H | O | H | Ga |
| $CON(Me)_2$ | H | $SO_2Me$ | H | O | H | Ga |
| $CON(Et)_2$ | H | $SO_2Me$ | H | O | H | Ga |

| $R_3$ | $R_2$ | $R_1$ | $R_4$ | X | M | Gn |
|---|---|---|---|---|---|---|
| F | H | COMe | H | O | H | Ga |
| Cl | H | COMe | H | O | H | Ga |
| Br | H | COMe | H | O | H | Ga |
| I | H | COMe | H | O | H | Ga |
| $CH=CH_2$ | H | COMe | H | O | H | Ga |
| $CH_2CH=CH_2$ | H | COMe | H | O | H | Ga |
| $C\equiv CH$ | H | COMe | H | O | H | Ga |
| $CH_2C\equiv CH$ | H | COMe | H | O | H | Ga |
| $CH_2F$ | H | COMe | H | O | H | Ga |
| $CHF_2$ | H | COMe | H | O | H | Ga |
| CN | H | COMe | H | O | H | Ga |
| $NH_2$ | H | COMe | H | O | H | Ga |
| NHCOMe | H | COMe | H | O | H | Ga |
| $CH_2NH_2$ | H | COMe | H | O | H | Ga |
| $CH_2NHCOMe$ | H | COMe | H | O | H | Ga |
| $CH_2OCHF_2$ | H | COMe | H | O | H | Ga |
| $CF_3$ | H | COMe | H | O | H | Ga |
| $NO_2$ | H | COMe | H | O | H | Gb |
| OMe | H | COMe | H | O | H | Ga |
| OEt | H | COMe | H | O | H | Ga |
| OPr-n | H | COMe | H | O | H | Gb |
| SMe | H | COMe | H | O | H | Ga |
| SEt | H | COMe | H | O | H | Ga |
| $SO_2Me$ | H | COMe | H | O | H | Ga |
| $SO_2Et$ | H | COMe | H | O | H | Ga |
| COMe | H | COMe | H | O | H | Ga |
| COEt | H | COMe | H | O | H | Ga |
| $CO_2H$ | H | COMe | H | O | H | Gb |
| $CO_2Me$ | H | COMe | H | O | H | Ga |
| $CO_2Et$ | H | COMe | H | O | H | Ga |
| $CO_2Pr$-n | H | COMe | H | O | H | Gb |
| $CO_2Bu$-n | H | COMe | H | O | H | Gc |
| $CH_2Cl$ | H | COMe | H | O | H | Ga |
| $CH_2Br$ | H | COMe | H | O | H | Gb |
| $CH_2I$ | H | COMe | H | O | H | Gb |
| $CH_2CF_3$ | H | COMe | H | O | H | Ga |
| $CH_2CN$ | H | COMe | H | O | H | Ga |
| $CH_2OH$ | H | COMe | H | O | H | Gb |
| $CH_2OMe$ | H | COMe | H | O | H | Ga |
| $CH_2OEt$ | H | COMe | H | O | H | Ga |

| $R_3$ | $R_2$ | $R_1$ | $R_4$ | X | M | Gn |
|---|---|---|---|---|---|---|
| $CH_2OPr$-n | H | COMe | H | O | H | Gb |
| $CH_2SMe$ | H | COMe | H | O | H | Ga |
| $CH_2SEt$ | H | COMe | H | O | H | Ga |
| $CH_2SPr$-n | H | COMe | H | O | H | Gb |
| $CH_2SO_2Me$ | H | COMe | H | O | H | Ga |
| $CH_2SO_2Et$ | H | COMe | H | O | H | Ga |
| $CH_2COMe$ | H | COMe | H | O | H | Ga |
| $CH_2COEt$ | H | COMe | H | O | H | Ga |
| $CH_2COPr$-n | H | COMe | H | O | H | Gb |
| $CH_2CO_2H$ | H | COMe | H | O | H | Gb |
| $CH_2CO_2Me$ | H | COMe | H | O | H | Ga |
| $CH_2CO_2Et$ | H | COMe | H | O | H | Ga |
| $CH_2CO_2Pr$-n | H | COMe | H | O | H | Gb |
| $CH_2OCH_2CH=CH_2$ | H | COMe | H | O | H | Gb |
| $CH_2OCH_2C\equiv CH$ | H | COMe | H | O | H | Gb |
| $CH=CHCO_2Me$ | H | COMe | H | O | H | Ga |
| $CH=CHCO_2Et$ | H | COMe | H | O | H | Ga |
| $CH=CHCO_2Pr$-n | H | COMe | H | O | H | Gb |
| CH=CHCN | H | COMe | H | O | H | Ga |
| Ph | H | COMe | H | O | H | Ga |
| $CH_2Ph$ | H | COMe | H | O | H | Ga |
| OPh | H | COMe | H | O | H | Ga |
| SPh | H | COMe | H | O | H | Ga |
| $SO_2Ph$ | H | COMe | H | O | H | Ga |
| CONHMe | H | COMe | H | O | H | Ga |
| CONHEt | H | COMe | H | O | H | Ga |
| $CON(Me)_2$ | H | COMe | H | O | H | Ga |
| $CON(Et)_2$ | H | COMe | H | O | H | Ga |
| F | H | $CO_2Me$ | H | O | H | Ga |
| Cl | H | $CO_2Me$ | H | O | H | Ga |
| Br | H | $CO_2Me$ | H | O | H | Ga |
| I | H | $CO_2Me$ | H | O | H | Ga |
| $CH=CH_2$ | H | $CO_2Me$ | H | O | H | Ga |
| $CH_2CH=CH_2$ | H | $CO_2Me$ | H | O | H | Ga |
| $C\equiv CH$ | H | $CO_2Me$ | H | O | H | Ga |
| $CH_2C\equiv CH$ | H | $CO_2Me$ | H | O | H | Ga |
| $CH_2F$ | H | $CO_2Me$ | H | O | H | Ga |
| $CHF_2$ | H | $CO_2Me$ | H | O | H | Ga |
| CN | H | $CO_2Me$ | H | O | H | Ga |
| $NH_2$ | H | $CO_2Me$ | H | O | H | Ga |

| $R_3$ | $R_2$ | $R_1$ | $R_4$ | X | M | Gn |
|---|---|---|---|---|---|---|
| NHCOMe | H | $CO_2Me$ | H | O | H | Ga |
| $CH_2NH_2$ | H | $CO_2Me$ | H | O | H | Ga |
| $CH_2NHCOMe$ | H | $CO_2Me$ | H | O | H | Ga |
| $CH_2OCHF_2$ | H | $CO_2Me$ | H | O | H | Ga |
| $CF_3$ | H | $CO_2Me$ | H | O | H | Ga |
| $NO_2$ | H | $CO_2Me$ | H | O | H | Gb |
| OMe | H | $CO_2Me$ | H | O | H | Ga |
| OEt | H | $CO_2Me$ | H | O | H | Ga |
| OPr-n | H | $CO_2Me$ | H | O | H | Gb |
| SMe | H | $CO_2Me$ | H | O | H | Ga |
| SEt | H | $CO_2Me$ | H | O | H | Ga |
| $SO_2Me$ | H | $CO_2Me$ | H | O | H | Ga |
| $SO_2Et$ | H | $CO_2Me$ | H | O | H | Ga |
| COMe | H | $CO_2Me$ | H | O | H | Ga |
| COEt | H | $CO_2Me$ | H | O | H | Ga |
| $CO_2H$ | H | $CO_2Me$ | H | O | H | Gb |
| $CO_2Me$ | H | $CO_2Me$ | H | O | H | Ga |
| $CO_2Et$ | H | $CO_2Me$ | H | O | H | Ga |
| $CO_2Pr$-n | H | $CO_2Me$ | H | O | H | Gb |
| $CO_2Bu$-n | H | $CO_2Me$ | H | O | H | Gc |
| $CH_2Cl$ | H | $CO_2Me$ | H | O | H | Ga |
| $CH_2Br$ | H | $CO_2Me$ | H | O | H | Gb |
| $CH_2I$ | H | $CO_2Me$ | H | O | H | Gb |
| $CH_2CF_3$ | H | $CO_2Me$ | H | O | H | Ga |
| $CH_2CN$ | H | $CO_2Me$ | H | O | H | Ga |
| $CH_2OH$ | H | $CO_2Me$ | H | O | H | Gb |
| $CH_2OMe$ | H | $CO_2Me$ | H | O | H | Ga |
| $CH_2OEt$ | H | $CO_2Me$ | H | O | H | Ga |
| $CH_2OPr$-n | H | $CO_2Me$ | H | O | H | Gb |
| $CH_2SMe$ | H | $CO_2Me$ | H | O | H | Ga |
| $CH_2SEt$ | H | $CO_2Me$ | H | O | H | Ga |
| $CH_2SPr$-n | H | $CO_2Me$ | H | O | H | Gb |
| $CH_2SO_2Me$ | H | $CO_2Me$ | H | O | H | Ga |
| $CH_2SO_2Et$ | H | $CO_2Me$ | H | O | H | Ga |
| $CH_2COMe$ | H | $CO_2Me$ | H | O | H | Ga |
| $CH_2COEt$ | H | $CO_2Me$ | H | O | H | Ga |
| $CH_2COPr$-n | H | $CO_2Me$ | H | O | H | Gb |
| $CH_2CO_2H$ | H | $CO_2Me$ | H | O | H | Gb |
| $CH_2CO_2Me$ | H | $CO_2Me$ | H | O | H | Ga |
| $CH_2CO_2Et$ | H | $CO_2Me$ | H | O | H | Ga |

| $R_3$ | $R_2$ | $R_1$ | $R_4$ | X | M | Gn |
|---|---|---|---|---|---|---|
| $CH_2CO_2Pr$-n | H | $CO_2Me$ | H | O | H | Gb |
| $CH_2OCH_2CH=CH_2$ | H | $CO_2Me$ | H | O | H | Gb |
| $CH_2OCH_2C\equiv CH$ | H | $CO_2Me$ | H | O | H | Gb |
| $CH=CHCO_2Me$ | H | $CO_2Me$ | H | O | H | Ga |
| $CH=CHCO_2Et$ | H | $CO_2Me$ | H | O | H | Ga |
| $CH=CHCO_2Pr$-n | H | $CO_2Me$ | H | O | H | Gb |
| $CH=CHCN$ | H | $CO_2Me$ | H | O | H | Ga |
| Ph | H | $CO_2Me$ | H | O | H | Ga |
| $CH_2Ph$ | H | $CO_2Me$ | H | O | H | Ga |
| OPh | H | $CO_2Me$ | H | O | H | Ga |
| SPh | H | $CO_2Me$ | H | O | H | Ga |
| $SO_2Ph$ | H | $CO_2Me$ | H | O | H | Ga |
| CONHMe | H | $CO_2Me$ | H | O | H | Ga |
| CONHEt | H | $CO_2Me$ | H | O | H | Ga |
| $CON(Me)_2$ | H | $CO_2Me$ | H | O | H | Ga |
| $CON(Et)_2$ | H | $CO_2Me$ | H | O | H | Ga |
| F | H | $CO_2H$ | H | O | H | Ga |
| Cl | H | $CO_2H$ | H | O | H | Ga |
| Br | H | $CO_2H$ | H | O | H | Ga |
| I | H | $CO_2H$ | H | O | H | Ga |
| $CH=CH_2$ | H | $CO_2H$ | H | O | H | Ga |
| $CH_2CH=CH_2$ | H | $CO_2H$ | H | O | H | Ga |
| $C\equiv CH$ | H | $CO_2H$ | H | O | H | Ga |
| $CH_2C\equiv CH$ | H | $CO_2H$ | H | O | H | Ga |
| $CH_2F$ | H | $CO_2H$ | H | O | H | Ga |
| $CHF_2$ | H | $CO_2H$ | H | O | H | Ga |
| CN | H | $CO_2H$ | H | O | H | Ga |
| $NH_2$ | H | $CO_2H$ | H | O | H | Ga |
| NHCOMe | H | $CO_2H$ | H | O | H | Ga |
| $CH_2NH_2$ | H | $CO_2H$ | H | O | H | Ga |
| $CH_2NHCOMe$ | H | $CO_2H$ | H | O | H | Ga |
| $CH_2OCHF_2$ | H | $CO_2H$ | H | O | H | Ga |
| $CF_3$ | H | $CO_2H$ | H | O | H | Ga |
| $NO_2$ | H | $CO_2H$ | H | O | H | Gb |
| OMe | H | $CO_2H$ | H | O | H | Ga |
| OEt | H | $CO_2H$ | H | O | H | Ga |
| OPr-n | H | $CO_2H$ | H | O | H | Gb |
| SMe | H | $CO_2H$ | H | O | H | Ga |
| SEt | H | $CO_2H$ | H | O | H | Ga |
| $SO_2Me$ | H | $CO_2H$ | H | O | H | Ga |

| $R_3$ | $R_2$ | $R_1$ | $R_4$ | X | M | Gn |
|---|---|---|---|---|---|---|
| $SO_2Et$ | H | $CO_2H$ | H | O | H | Ga |
| COMe | H | $CO_2H$ | H | O | H | Ga |
| COEt | H | $CO_2H$ | H | O | H | Ga |
| $CO_2H$ | H | $CO_2H$ | H | O | H | Gb |
| $CO_2Me$ | H | $CO_2H$ | H | O | H | Ga |
| $CO_2Et$ | H | $CO_2H$ | H | O | H | Ga |
| $CO_2Pr$-n | H | $CO_2H$ | H | O | H | Gb |
| $CO_2Bu$-n | H | $CO_2H$ | H | O | H | Gc |
| $CH_2Cl$ | H | $CO_2H$ | H | O | H | Ga |
| $CH_2Br$ | H | $CO_2H$ | H | O | H | Gb |
| $CH_2I$ | H | $CO_2H$ | H | O | H | Gb |
| $CH_2CF_3$ | H | $CO_2H$ | H | O | H | Ga |
| $CH_2CN$ | H | $CO_2H$ | H | O | H | Ga |
| $CH_2OH$ | H | $CO_2H$ | H | O | H | Gb |
| $CH_2OMe$ | H | $CO_2H$ | H | O | H | Ga |
| $CH_2OEt$ | H | $CO_2H$ | H | O | H | Ga |
| $CH_2OPr$-n | H | $CO_2H$ | H | O | H | Gb |
| $CH_2SMe$ | H | $CO_2H$ | H | O | H | Ga |
| $CH_2SEt$ | H | $CO_2H$ | H | O | H | Ga |
| $CH_2SPr$-n | H | $CO_2H$ | H | O | H | Gb |
| $CH_2SO_2Me$ | H | $CO_2H$ | H | O | H | Ga |
| $CH_2SO_2Et$ | H | $CO_2H$ | H | O | H | Ga |
| $CH_2COMe$ | H | $CO_2H$ | H | O | H | Ga |
| $CH_2COEt$ | H | $CO_2H$ | H | O | H | Ga |
| $CH_2COPr$-n | H | $CO_2H$ | H | O | H | Gb |
| $CH_2CO_2H$ | H | $CO_2H$ | H | O | H | Gb |
| $CH_2CO_2Me$ | H | $CO_2H$ | H | O | H | Ga |
| $CH_2CO_2Et$ | H | $CO_2H$ | H | O | H | Ga |
| $CH_2CO_2Pr$-n | H | $CO_2H$ | H | O | H | Gb |
| $CH_2OCH_2CH=CH_2$ | H | $CO_2H$ | H | O | H | Gb |
| $CH_2OCH_2C\equiv CH$ | H | $CO_2H$ | H | O | H | Gb |
| $CH=CHCO_2Me$ | H | $CO_2H$ | H | O | H | Ga |
| $CH=CHCO_2Et$ | H | $CO_2H$ | H | O | H | Ga |
| $CH=CHCO_2Pr$-n | H | $CO_2H$ | H | O | H | Gb |
| $CH=CHCN$ | H | $CO_2H$ | H | O | H | Ga |
| Ph | H | $CO_2H$ | H | O | H | Ga |
| $CH_2Ph$ | H | $CO_2H$ | H | O | H | Ga |
| OPh | H | $CO_2H$ | H | O | H | Ga |
| SPh | H | $CO_2H$ | H | O | H | Ga |
| $SO_2Ph$ | H | $CO_2H$ | H | O | H | Ga |

| $R_3$ | $R_2$ | $R_1$ | $R_4$ | X | M | Gn |
|---|---|---|---|---|---|---|
| CONHMe | H | $CO_2H$ | H | O | H | Ga |
| CONHEt | H | $CO_2H$ | H | O | H | Ga |
| $CON(Me)_2$ | H | $CO_2H$ | H | O | H | Ga |
| $CON(Et)_2$ | H | $CO_2H$ | H | O | H | Ga |
| F | H | $CH_2F$ | H | O | H | Ga |
| Cl | H | $CH_2F$ | H | O | H | Ga |
| Br | H | $CH_2F$ | H | O | H | Ga |
| I | H | $CH_2F$ | H | O | H | Ga |
| $CH=CH_2$ | H | $CH_2F$ | H | O | H | Ga |
| $CH_2CH=CH_2$ | H | $CH_2F$ | H | O | H | Ga |
| $C\equiv CH$ | H | $CH_2F$ | H | O | H | Ga |
| $CH_2C\equiv CH$ | H | $CH_2F$ | H | O | H | Ga |
| $CH_2F$ | H | $CH_2F$ | H | O | H | Ga |
| $CHF_2$ | H | $CH_2F$ | H | O | H | Ga |
| CN | H | $CH_2F$ | H | O | H | Ga |
| $NH_2$ | H | $CH_2F$ | H | O | H | Ga |
| NHCOMe | H | $CH_2F$ | H | O | H | Ga |
| $CH_2NH_2$ | H | $CH_2F$ | H | O | H | Ga |
| $CH_2NHCOMe$ | H | $CH_2F$ | H | O | H | Ga |
| $CH_2OCHF_2$ | H | $CH_2F$ | H | O | H | Ga |
| $CF_3$ | H | $CH_2F$ | H | O | H | Ga |
| $NO_2$ | H | $CH_2F$ | H | O | H | Gb |
| OMe | H | $CH_2F$ | H | O | H | Ga |
| OEt | H | $CH_2F$ | H | O | H | Ga |
| OPr-n | H | $CH_2F$ | H | O | H | Gb |
| SMe | H | $CH_2F$ | H | O | H | Ga |
| SEt | H | $CH_2F$ | H | O | H | Ga |
| $SO_2Me$ | H | $CH_2F$ | H | O | H | Ga |
| $SO_2Et$ | H | $CH_2F$ | H | O | H | Ga |
| COMe | H | $CH_2F$ | H | O | H | Ga |
| COEt | H | $CH_2F$ | H | O | H | Ga |
| $CO_2H$ | H | $CH_2F$ | H | O | H | Gb |
| $CO_2Me$ | H | $CH_2F$ | H | O | H | Ga |
| $CO_2Et$ | H | $CH_2F$ | H | O | H | Ga |
| $CO_2Pr$-n | H | $CH_2F$ | H | O | H | Gb |
| $CO_2Bu$-n | H | $CH_2F$ | H | O | H | Gc |
| $CH_2Cl$ | H | $CH_2F$ | H | O | H | Ga |
| $CH_2Br$ | H | $CH_2F$ | H | O | H | Gb |
| $CH_2I$ | H | $CH_2F$ | H | O | H | Gb |
| $CH_2CF_3$ | H | $CH_2F$ | H | O | H | Ga |

| $R_3$ | $R_2$ | $R_1$ | $R_4$ | X | M | Gn |
|---|---|---|---|---|---|---|
| $CH_2CN$ | H | $CH_2F$ | H | O | H | Ga |
| $CH_2OH$ | H | $CH_2F$ | H | O | H | Gb |
| $CH_2OMe$ | H | $CH_2F$ | H | O | H | Ga |
| $CH_2OEt$ | H | $CH_2F$ | H | O | H | Ga |
| $CH_2OPr$-n | H | $CH_2F$ | H | O | H | Gb |
| $CH_2SMe$ | H | $CH_2F$ | H | O | H | Ga |
| $CH_2SEt$ | H | $CH_2F$ | H | O | H | Ga |
| $CH_2SPr$-n | H | $CH_2F$ | H | O | H | Gb |
| $CH_2SO_2Me$ | H | $CH_2F$ | H | O | H | Ga |
| $CH_2SO_2Et$ | H | $CH_2F$ | H | O | H | Ga |
| $CH_2COMe$ | H | $CH_2F$ | H | O | H | Ga |
| $CH_2COEt$ | H | $CH_2F$ | H | O | H | Ga |
| $CH_2COPr$-n | H | $CH_2F$ | H | O | H | Gb |
| $CH_2CO_2H$ | H | $CH_2F$ | H | O | H | Gb |
| $CH_2CO_2Me$ | H | $CH_2F$ | H | O | H | Ga |
| $CH_2CO_2Et$ | H | $CH_2F$ | H | O | H | Ga |
| $CH_2CO_2Pr$-n | H | $CH_2F$ | H | O | H | Gb |
| $CH_2OCH_2CH=CH_2$ | H | $CH_2F$ | H | O | H | Gb |
| $CH_2OCH_2C\equiv CH$ | H | $CH_2F$ | H | O | H | Gb |
| $CH=CHCO_2Me$ | H | $CH_2F$ | H | O | H | Ga |
| $CH=CHCO_2Et$ | H | $CH_2F$ | H | O | H | Ga |
| $CH=CHCO_2Pr$-n | H | $CH_2F$ | H | O | H | Gb |
| CH=CHCN | H | $CH_2F$ | H | O | H | Ga |
| Ph | H | $CH_2F$ | H | O | H | Ga |
| $CH_2Ph$ | H | $CH_2F$ | H | O | H | Ga |
| OPh | H | $CH_2F$ | H | O | H | Ga |
| SPh | H | $CH_2F$ | H | O | H | Ga |
| $SO_2Ph$ | H | $CH_2F$ | H | O | H | Ga |
| CONHMe | H | $CH_2F$ | H | O | H | Ga |
| CONHEt | H | $CH_2F$ | H | O | H | Ga |
| $CON(Me)_2$ | H | $CH_2F$ | H | O | H | Ga |
| $CON(Et)_2$ | H | $CH_2F$ | H | O | H | Ga |
| F | H | $CH_2Cl$ | H | O | H | Ga |
| Cl | H | $CH_2Cl$ | H | O | H | Ga |
| Br | H | $CH_2Cl$ | H | O | H | Ga |
| I | H | $CH_2Cl$ | H | O | H | Ga |
| $CH=CH_2$ | H | $CH_2Cl$ | H | O | H | Ga |
| $CH_2CH=CH_2$ | H | $CH_2Cl$ | H | O | H | Ga |
| $C\equiv CH$ | H | $CH_2Cl$ | H | O | H | Ga |
| $CH_2C\equiv CH$ | H | $CH_2Cl$ | H | O | H | Ga |

| $R_3$ | $R_2$ | $R_1$ | $R_4$ | X | M | Gn |
|---|---|---|---|---|---|---|
| $CH_2F$ | H | $CH_2Cl$ | H | O | H | Ga |
| $CHF_2$ | H | $CH_2Cl$ | H | O | H | Ga |
| CN | H | $CH_2Cl$ | H | O | H | Ga |
| $NH_2$ | H | $CH_2Cl$ | H | O | H | Ga |
| NHCOMe | H | $CH_2Cl$ | H | O | H | Ga |
| $CH_2NH_2$ | H | $CH_2Cl$ | H | O | H | Ga |
| $CH_2NHCOMe$ | H | $CH_2Cl$ | H | O | H | Ga |
| $CH_2OCHF_2$ | H | $CH_2Cl$ | H | O | H | Ga |
| $CF_3$ | H | $CH_2Cl$ | H | O | H | Ga |
| $NO_2$ | H | $CH_2Cl$ | H | O | H | Gb |
| OMe | H | $CH_2Cl$ | H | O | H | Ga |
| OEt | H | $CH_2Cl$ | H | O | H | Ga |
| OPr-n | H | $CH_2Cl$ | H | O | H | Gb |
| SMe | H | $CH_2Cl$ | H | O | H | Ga |
| SEt | H | $CH_2Cl$ | H | O | H | Ga |
| $SO_2Me$ | H | $CH_2Cl$ | H | O | H | Ga |
| $SO_2Et$ | H | $CH_2Cl$ | H | O | H | Ga |
| COMe | H | $CH_2Cl$ | H | O | H | Ga |
| COEt | H | $CH_2Cl$ | H | O | H | Ga |
| $CO_2H$ | H | $CH_2Cl$ | H | O | H | Gb |
| $CO_2Me$ | H | $CH_2Cl$ | H | O | H | Ga |
| $CO_2Et$ | H | $CH_2Cl$ | H | O | H | Ga |
| $CO_2Pr$-n | H | $CH_2Cl$ | H | O | H | Gb |
| $CO_2Bu$-n | H | $CH_2Cl$ | H | O | H | Gc |
| $CH_2Cl$ | H | $CH_2Cl$ | H | O | H | Ga |
| $CH_2Br$ | H | $CH_2Cl$ | H | O | H | Gb |
| $CH_2I$ | H | $CH_2Cl$ | H | O | H | Gb |
| $CH_2CF_3$ | H | $CH_2Cl$ | H | O | H | Ga |
| $CH_2CN$ | H | $CH_2Cl$ | H | O | H | Ga |
| $CH_2OH$ | H | $CH_2Cl$ | H | O | H | Gb |
| $CH_2OMe$ | H | $CH_2Cl$ | H | O | H | Ga |
| $CH_2OEt$ | H | $CH_2Cl$ | H | O | H | Ga |
| $CH_2OPr$-n | H | $CH_2Cl$ | H | O | H | Gb |
| $CH_2SMe$ | H | $CH_2Cl$ | H | O | H | Ga |
| $CH_2SEt$ | H | $CH_2Cl$ | H | O | H | Ga |
| $CH_2SPr$-n | H | $CH_2Cl$ | H | O | H | Gb |
| $CH_2SO_2Me$ | H | $CH_2Cl$ | H | O | H | Ga |
| $CH_2SO_2Et$ | H | $CH_2Cl$ | H | O | H | Ga |
| $CH_2COMe$ | H | $CH_2Cl$ | H | O | H | Ga |
| $CH_2COEt$ | H | $CH_2Cl$ | H | O | H | Ga |

| $R_3$ | $R_2$ | $R_1$ | $R_4$ | X | M | Gn |
|---|---|---|---|---|---|---|
| $CH_2COPr$-n | H | $CH_2Cl$ | H | O | H | Gb |
| $CH_2CO_2H$ | H | $CH_2Cl$ | H | O | H | Gb |
| $CH_2CO_2Me$ | H | $CH_2Cl$ | H | O | H | Ga |
| $CH_2CO_2Et$ | H | $CH_2Cl$ | H | O | H | Ga |
| $CH_2CO_2Pr$-n | H | $CH_2Cl$ | H | O | H | Gb |
| $CH_2OCH_2CH=CH_2$ | H | $CH_2Cl$ | H | O | H | Gb |
| $CH_2OCH_2C\equiv CH$ | H | $CH_2Cl$ | H | O | H | Gb |
| $CH=CHCO_2Me$ | H | $CH_2Cl$ | H | O | H | Ga |
| $CH=CHCO_2Et$ | H | $CH_2Cl$ | H | O | H | Ga |
| $CH=CHCO_2Pr$-n | H | $CH_2Cl$ | H | O | H | Gb |
| CH=CHCN | H | $CH_2Cl$ | H | O | H | Ga |
| Ph | H | $CH_2Cl$ | H | O | H | Ga |
| $CH_2Ph$ | H | $CH_2Cl$ | H | O | H | Ga |
| OPh | H | $CH_2Cl$ | H | O | H | Ga |
| SPh | H | $CH_2Cl$ | H | O | H | Ga |
| $SO_2Ph$ | H | $CH_2Cl$ | H | O | H | Ga |
| CONHMe | H | $CH_2Cl$ | H | O | H | Ga |
| CONHEt | H | $CH_2Cl$ | H | O | H | Ga |
| $CON(Me)_2$ | H | $CH_2Cl$ | H | O | H | Ga |
| $CON(Et)_2$ | H | $CH_2Cl$ | H | O | H | Ga |
| F | H | $CH_2OMe$ | H | O | H | Ga |
| Cl | H | $CH_2OMe$ | H | O | H | Ga |
| Br | H | $CH_2OMe$ | H | O | H | Ga |
| I | H | $CH_2OMe$ | H | O | H | Ga |
| $CH=CH_2$ | H | $CH_2OMe$ | H | O | H | Ga |
| $CH_2CH=CH_2$ | H | $CH_2OMe$ | H | O | H | Ga |
| $C\equiv CH$ | H | $CH_2OMe$ | H | O | H | Ga |
| $CH_2C\equiv CH$ | H | $CH_2OMe$ | H | O | H | Ga |
| $CH_2F$ | H | $CH_2OMe$ | H | O | H | Ga |
| $CHF_2$ | H | $CH_2OMe$ | H | O | H | Ga |
| CN | H | $CH_2OMe$ | H | O | H | Ga |
| $NH_2$ | H | $CH_2OMe$ | H | O | H | Ga |
| NHCOMe | H | $CH_2OMe$ | H | O | H | Ga |
| $CH_2NH_2$ | H | $CH_2OMe$ | H | O | H | Ga |
| $CH_2NHCOMe$ | H | $CH_2OMe$ | H | O | H | Ga |
| $CH_2OCHF_2$ | H | $CH_2OMe$ | H | O | H | Ga |
| $CF_3$ | H | $CH_2OMe$ | H | O | H | Ga |
| $NO_2$ | H | $CH_2OMe$ | H | O | H | Gb |
| OMe | H | $CH_2OMe$ | H | O | H | Ga |
| OEt | H | $CH_2OMe$ | H | O | H | Ga |

| $R_3$ | $R_2$ | $R_1$ | $R_4$ | X | M | Gn |
|---|---|---|---|---|---|---|
| OPr-n | H | $CH_2OMe$ | H | O | H | Gb |
| SMe | H | $CH_2OMe$ | H | O | H | Ga |
| SEt | H | $CH_2OMe$ | H | O | H | Ga |
| $SO_2Me$ | H | $CH_2OMe$ | H | O | H | Ga |
| $SO_2Et$ | H | $CH_2OMe$ | H | O | H | Ga |
| COMe | H | $CH_2OMe$ | H | O | H | Ga |
| COEt | H | $CH_2OMe$ | H | O | H | Ga |
| $CO_2H$ | H | $CH_2OMe$ | H | O | H | Gb |
| $CO_2Me$ | H | $CH_2OMe$ | H | O | H | Ga |
| $CO_2Et$ | H | $CH_2OMe$ | H | O | H | Ga |
| $CO_2Pr$-n | H | $CH_2OMe$ | H | O | H | Gb |
| $CO_2Bu$-n | H | $CH_2OMe$ | H | O | H | Gc |
| $CH_2Cl$ | H | $CH_2OMe$ | H | O | H | Ga |
| $CH_2Br$ | H | $CH_2OMe$ | H | O | H | Gb |
| $CH_2I$ | H | $CH_2OMe$ | H | O | H | Gb |
| $CH_2CF_3$ | H | $CH_2OMe$ | H | O | H | Ga |
| $CH_2CN$ | H | $CH_2OMe$ | H | O | H | Ga |
| $CH_2OH$ | H | $CH_2OMe$ | H | O | H | Gb |
| $CH_2OMe$ | H | $CH_2OMe$ | H | O | H | Ga |
| $CH_2OEt$ | H | $CH_2OMe$ | H | O | H | Ga |
| $CH_2OPr$-n | H | $CH_2OMe$ | H | O | H | Gb |
| $CH_2SMe$ | H | $CH_2OMe$ | H | O | H | Ga |
| $CH_2SEt$ | H | $CH_2OMe$ | H | O | H | Ga |
| $CH_2SPr$-n | H | $CH_2OMe$ | H | O | H | Gb |
| $CH_2SO_2Me$ | H | $CH_2OMe$ | H | O | H | Ga |
| $CH_2SO_2Et$ | H | $CH_2OMe$ | H | O | H | Ga |
| $CH_2COMe$ | H | $CH_2OMe$ | H | O | H | Ga |
| $CH_2COEt$ | H | $CH_2OMe$ | H | O | H | Ga |
| $CH_2COPr$-n | H | $CH_2OMe$ | H | O | H | Gb |
| $CH_2CO_2H$ | H | $CH_2OMe$ | H | O | H | Gb |
| $CH_2CO_2Me$ | H | $CH_2OMe$ | H | O | H | Ga |
| $CH_2CO_2Et$ | H | $CH_2OMe$ | H | O | H | Ga |
| $CH_2CO_2Pr$-n | H | $CH_2OMe$ | H | O | H | Gb |
| $CH_2OCH_2CH=CH_2$ | H | $CH_2OMe$ | H | O | H | Gb |
| $CH_2OCH_2C\equiv CH$ | H | $CH_2OMe$ | H | O | H | Gb |
| $CH=CHCO_2Me$ | H | $CH_2OMe$ | H | O | H | Ga |
| $CH=CHCO_2Et$ | H | $CH_2OMe$ | H | O | H | Ga |
| $CH=CHCO_2Pr$-n | H | $CH_2OMe$ | H | O | H | Gb |
| CH=CHCN | H | $CH_2OMe$ | H | O | H | Ga |
| Ph | H | $CH_2OMe$ | H | O | H | Ga |

| $R_3$ | $R_2$ | $R_1$ | $R_4$ | X | M | Gn |
|---|---|---|---|---|---|---|
| $CH_2Ph$ | H | $CH_2OMe$ | H | 0 | H | Ga |
| OPh | H | $CH_2OMe$ | H | 0 | H | Ga |
| SPh | H | $CH_2OMe$ | H | 0 | H | Ga |
| $SO_2Ph$ | H | $CH_2OMe$ | H | 0 | H | Ga |
| CONHMe | H | $CH_2OMe$ | H | 0 | H | Ga |
| CONHEt | H | $CH_2OMe$ | H | 0 | H | Ga |
| $CON(Me)_2$ | H | $CH_2OMe$ | H | 0 | H | Ga |
| $CON(Et)_2$ | H | $CH_2OMe$ | H | 0 | H | Ga |
| F | H | $CH_2SMe$ | H | 0 | H | Ga |
| Cl | H | $CH_2SMe$ | H | 0 | H | Ga |
| Br | H | $CH_2SMe$ | H | 0 | H | Ga |
| I | H | $CH_2SMe$ | H | 0 | H | Ga |
| $CH=CH_2$ | H | $CH_2SMe$ | H | 0 | H | Ga |
| $CH_2CH=CH_2$ | H | $CH_2SMe$ | H | 0 | H | Ga |
| $C\equiv CH$ | H | $CH_2SMe$ | H | 0 | H | Ga |
| $CH_2C\equiv CH$ | H | $CH_2SMe$ | H | 0 | H | Ga |
| $CH_2F$ | H | $CH_2SMe$ | H | 0 | H | Ga |
| $CHF_2$ | H | $CH_2SMe$ | H | 0 | H | Ga |
| CN | H | $CH_2SMe$ | H | 0 | H | Ga |
| $NH_2$ | H | $CH_2SMe$ | H | 0 | H | Ga |
| NHCOMe | H | $CH_2SMe$ | H | 0 | H | Ga |
| $CH_2NH_2$ | H | $CH_2SMe$ | H | 0 | H | Ga |
| $CH_2NHCOMe$ | H | $CH_2SMe$ | H | 0 | H | Ga |
| $CH_2OCHF_2$ | H | $CH_2SMe$ | H | 0 | H | Ga |
| $CF_3$ | H | $CH_2SMe$ | H | 0 | H | Ga |
| $NO_2$ | H | $CH_2SMe$ | H | 0 | H | Gb |
| OMe | H | $CH_2SMe$ | H | 0 | H | Ga |
| OEt | H | $CH_2SMe$ | H | 0 | H | Ga |
| OPr-n | H | $CH_2SMe$ | H | 0 | H | Gb |
| SMe | H | $CH_2SMe$ | H | 0 | H | Ga |
| SEt | H | $CH_2SMe$ | H | 0 | H | Ga |
| $SO_2Me$ | H | $CH_2SMe$ | H | 0 | H | Ga |
| $SO_2Et$ | H | $CH_2SMe$ | H | 0 | H | Ga |
| COMe | H | $CH_2SMe$ | H | 0 | H | Ga |
| COEt | H | $CH_2SMe$ | H | 0 | H | Ga |
| $CO_2H$ | H | $CH_2SMe$ | H | 0 | H | Gb |
| $CO_2Me$ | H | $CH_2SMe$ | H | 0 | H | Ga |
| $CO_2Et$ | H | $CH_2SMe$ | H | 0 | H | Ga |
| $CO_2Pr$-n | H | $CH_2SMe$ | H | 0 | H | Gb |
| $CO_2Bu$-n | H | $CH_2SMe$ | H | 0 | H | Gc |

| $R_3$ | $R_2$ | $R_1$ | $R_4$ | X | M | Gn |
|---|---|---|---|---|---|---|
| $CH_2Cl$ | H | $CH_2SMe$ | H | O | H | Ga |
| $CH_2Br$ | H | $CH_2SMe$ | H | O | H | Gb |
| $CH_2I$ | H | $CH_2SMe$ | H | O | H | Gb |
| $CH_2CF_3$ | H | $CH_2SMe$ | H | O | H | Ga |
| $CH_2CN$ | H | $CH_2SMe$ | H | O | H | Ga |
| $CH_2OH$ | H | $CH_2SMe$ | H | O | H | Gb |
| $CH_2OMe$ | H | $CH_2SMe$ | H | O | H | Ga |
| $CH_2OEt$ | H | $CH_2SMe$ | H | O | H | Ga |
| $CH_2OPr$-n | H | $CH_2SMe$ | H | O | H | Gb |
| $CH_2SMe$ | H | $CH_2SMe$ | H | O | H | Ga |
| $CH_2SEt$ | H | $CH_2SMe$ | H | O | H | Ga |
| $CH_2SPr$-n | H | $CH_2SMe$ | H | O | H | Gb |
| $CH_2SO_2Me$ | H | $CH_2SMe$ | H | O | H | Ga |
| $CH_2SO_2Et$ | H | $CH_2SMe$ | H | O | H | Ga |
| $CH_2COMe$ | H | $CH_2SMe$ | H | O | H | Ga |
| $CH_2COEt$ | H | $CH_2SMe$ | H | O | H | Ga |
| $CH_2COPr$-n | H | $CH_2SMe$ | H | O | H | Gb |
| $CH_2CO_2H$ | H | $CH_2SMe$ | H | O | H | Gb |
| $CH_2CO_2Me$ | H | $CH_2SMe$ | H | O | H | Ga |
| $CH_2CO_2Et$ | H | $CH_2SMe$ | H | O | H | Ga |
| $CH_2CO_2Pr$-n | H | $CH_2SMe$ | H | O | H | Gb |
| $CH_2OCH_2CH=CH_2$ | H | $CH_2SMe$ | H | O | H | Gb |
| $CH_2OCH_2C\equiv CH$ | H | $CH_2SMe$ | H | O | H | Gb |
| $CH=CHCO_2Me$ | H | $CH_2SMe$ | H | O | H | Ga |
| $CH=CHCO_2Et$ | H | $CH_2SMe$ | H | O | H | Ga |
| $CH=CHCO_2Pr$-n | H | $CH_2SMe$ | H | O | H | Gb |
| $CH=CHCN$ | H | $CH_2SMe$ | H | O | H | Ga |
| Ph | H | $CH_2SMe$ | H | O | H | Ga |
| $CH_2Ph$ | H | $CH_2SMe$ | H | O | H | Ga |
| OPh | H | $CH_2SMe$ | H | O | H | Ga |
| SPh | H | $CH_2SMe$ | H | O | H | Ga |
| $SO_2Ph$ | H | $CH_2SMe$ | H | O | H | Ga |
| CONHMe | H | $CH_2SMe$ | H | O | H | Ga |
| CONHEt | H | $CH_2SMe$ | H | O | H | Ga |
| $CON(Me)_2$ | H | $CH_2SMe$ | H | O | H | Ga |
| $CON(Et)_2$ | H | $CH_2SMe$ | H | O | H | Ga |
| F | H | $CH_2SO_2Me$ | H | O | H | Ga |
| Cl | H | $CH_2SO_2Me$ | H | O | H | Ga |
| Br | H | $CH_2SO_2Me$ | H | O | H | Ga |
| I | H | $CH_2SO_2Me$ | H | O | H | Ga |

| $R_3$ | $R_2$ | $R_1$ | $R_4$ | X | M | Gn |
|---|---|---|---|---|---|---|
| $CH=CH_2$ | H | $CH_2SO_2Me$ | H | O | H | Ga |
| $CH_2CH=CH_2$ | H | $CH_2SO_2Me$ | H | O | H | Ga |
| $C\equiv CH$ | H | $CH_2SO_2Me$ | H | O | H | Ga |
| $CH_2C\equiv CH$ | H | $CH_2SO_2Me$ | H | O | H | Ga |
| $CH_2F$ | H | $CH_2SO_2Me$ | H | O | H | Ga |
| $CHF_2$ | H | $CH_2SO_2Me$ | H | O | H | Ga |
| CN | H | $CH_2SO_2Me$ | H | O | H | Ga |
| $NH_2$ | H | $CH_2SO_2Me$ | H | O | H | Ga |
| NHCOMe | H | $CH_2SO_2Me$ | H | O | H | Ga |
| $CH_2NH_2$ | H | $CH_2SO_2Me$ | H | O | H | Ga |
| $CH_2NHCOMe$ | H | $CH_2SO_2Me$ | H | O | H | Ga |
| $CH_2OCHF_2$ | H | $CH_2SO_2Me$ | H | O | H | Ga |
| $CF_3$ | H | $CH_2SO_2Me$ | H | O | H | Ga |
| $NO_2$ | H | $CH_2SO_2Me$ | H | O | H | Gb |
| OMe | H | $CH_2SO_2Me$ | H | O | H | Ga |
| OEt | H | $CH_2SO_2Me$ | H | O | H | Ga |
| OPr-n | H | $CH_2SO_2Me$ | H | O | H | Gb |
| SMe | H | $CH_2SO_2Me$ | H | O | H | Ga |
| SEt | H | $CH_2SO_2Me$ | H | O | H | Ga |
| $SO_2Me$ | H | $CH_2SO_2Me$ | H | O | H | Ga |
| $SO_2Et$ | H | $CH_2SO_2Me$ | H | O | H | Ga |
| COMe | H | $CH_2SO_2Me$ | H | O | H | Ga |
| COEt | H | $CH_2SO_2Me$ | H | O | H | Ga |
| $CO_2H$ | H | $CH_2SO_2Me$ | H | O | H | Gb |
| $CO_2Me$ | H | $CH_2SO_2Me$ | H | O | H | Ga |
| $CO_2Et$ | H | $CH_2SO_2Me$ | H | O | H | Ga |
| $CO_2Pr$-n | H | $CH_2SO_2Me$ | H | O | H | Gb |
| $CO_2Bu$-n | H | $CH_2SO_2Me$ | H | O | H | Gc |
| $CH_2Cl$ | H | $CH_2SO_2Me$ | H | O | H | Ga |
| $CH_2Br$ | H | $CH_2SO_2Me$ | H | O | H | Gb |
| $CH_2I$ | H | $CH_2SO_2Me$ | H | O | H | Gb |
| $CH_2CF_3$ | H | $CH_2SO_2Me$ | H | O | H | Ga |
| $CH_2CN$ | H | $CH_2SO_2Me$ | H | O | H | Ga |
| $CH_2OH$ | H | $CH_2SO_2Me$ | H | O | H | Gb |
| $CH_2OMe$ | H | $CH_2SO_2Me$ | H | O | H | Ga |
| $CH_2OEt$ | H | $CH_2SO_2Me$ | H | O | H | Ga |
| $CH_2OPr$-n | H | $CH_2SO_2Me$ | H | O | H | Gb |
| $CH_2SMe$ | H | $CH_2SO_2Me$ | H | O | H | Ga |
| $CH_2SEt$ | H | $CH_2SO_2Me$ | H | O | H | Ga |
| $CH_2SPr$-n | H | $CH_2SO_2Me$ | H | O | H | Gb |

| $R_3$ | $R_2$ | $R_1$ | $R_4$ | X | M | Gn |
|---|---|---|---|---|---|---|
| $CH_2SO_2Me$ | H | $CH_2SO_2Me$ | H | O | H | Ga |
| $CH_2SO_2Et$ | H | $CH_2SO_2Me$ | H | O | H | Ga |
| $CH_2COMe$ | H | $CH_2SO_2Me$ | H | O | H | Ga |
| $CH_2COEt$ | H | $CH_2SO_2Me$ | H | O | H | Ga |
| $CH_2COPr$-n | H | $CH_2SO_2Me$ | H | O | H | Gb |
| $CH_2CO_2H$ | H | $CH_2SO_2Me$ | H | O | H | Gb |
| $CH_2CO_2Me$ | H | $CH_2SO_2Me$ | H | O | H | Ga |
| $CH_2CO_2Et$ | H | $CH_2SO_2Me$ | H | O | H | Ga |
| $CH_2CO_2Pr$-n | H | $CH_2SO_2Me$ | H | O | H | Gb |
| $CH_2OCH_2CH=CH_2$ | H | $CH_2SO_2Me$ | H | O | H | Gb |
| $CH_2OCH_2C\equiv CH$ | H | $CH_2SO_2Me$ | H | O | H | Gb |
| $CH=CHCO_2Me$ | H | $CH_2SO_2Me$ | H | O | H | Ga |
| $CH=CHCO_2Et$ | H | $CH_2SO_2Me$ | H | O | H | Ga |
| $CH=CHCO_2Pr$-n | H | $CH_2SO_2Me$ | H | O | H | Gb |
| CH=CHCN | H | $CH_2SO_2Me$ | H | O | H | Ga |
| Ph | H | $CH_2SO_2Me$ | H | O | H | Ga |
| $CH_2Ph$ | H | $CH_2SO_2Me$ | H | O | H | Ga |
| OPh | H | $CH_2SO_2Me$ | H | O | H | Ga |
| SPh | H | $CH_2SO_2Me$ | H | O | H | Ga |
| $SO_2Ph$ | H | $CH_2SO_2Me$ | H | O | H | Ga |
| CONHMe | H | $CH_2SO_2Me$ | H | O | H | Ga |
| CONHEt | H | $CH_2SO_2Me$ | H | O | H | Ga |
| $CON(Me)_2$ | H | $CH_2SO_2Me$ | H | O | H | Ga |
| $CON(Et)_2$ | H | $CH_2SO_2Me$ | H | O | H | Ga |
| F | H | Ph | H | O | H | Ga |
| Cl | H | Ph | H | O | H | Ga |
| Br | H | Ph | H | O | H | Ga |
| I | H | Ph | H | O | H | Ga |
| $CH=CH_2$ | H | Ph | H | O | H | Ga |
| $CH_2CH=CH_2$ | H | Ph | H | O | H | Ga |
| $C\equiv CH$ | H | Ph | H | O | H | Ga |
| $CH_2C\equiv CH$ | H | Ph | H | O | H | Ga |
| $CH_2F$ | H | Ph | H | O | H | Ga |
| $CHF_2$ | H | Ph | H | O | H | Ga |
| CN | H | Ph | H | O | H | Ga |
| $NH_2$ | H | Ph | H | O | H | Ga |
| NHCOMe | H | Ph | H | O | H | Ga |
| $CH_2NH_2$ | H | Ph | H | O | H | Ga |
| $CH_2NHCOMe$ | H | Ph | H | O | H | Ga |
| $CH_2OCHF_2$ | H | Ph | H | O | H | Ga |

| $R_3$ | $R_2$ | $R_1$ | $R_4$ | X | M | Gn |
|---|---|---|---|---|---|---|
| $CF_3$ | H | Ph | H | O | H | Ga |
| $NO_2$ | H | Ph | H | O | H | Gb |
| OMe | H | Ph | H | O | H | Ga |
| OEt | H | Ph | H | O | H | Ga |
| OPr-n | H | Ph | H | O | H | Gb |
| SMe | H | Ph | H | O | H | Ga |
| SEt | H | Ph | H | O | H | Ga |
| $SO_2Me$ | H | Ph | H | O | H | Ga |
| $SO_2Et$ | H | Ph | H | O | H | Ga |
| COMe | H | Ph | H | O | H | Ga |
| COEt | H | Ph | H | O | H | Ga |
| $CO_2H$ | H | Ph | H | O | H | Gb |
| $CO_2Me$ | H | Ph | H | O | H | Ga |
| $CO_2Et$ | H | Ph | H | O | H | Ga |
| $CO_2Pr$-n | H | Ph | H | O | H | Gb |
| $CO_2Bu$-n | H | Ph | H | O | H | Gc |
| $CH_2Cl$ | H | Ph | H | O | H | Ga |
| $CH_2Br$ | H | Ph | H | O | H | Gb |
| $CH_2I$ | H | Ph | H | O | H | Gb |
| $CH_2CF_3$ | H | Ph | H | O | H | Ga |
| $CH_2CN$ | H | Ph | H | O | H | Ga |
| $CH_2OH$ | H | Ph | H | O | H | Gb |
| $CH_2OMe$ | H | Ph | H | O | H | Ga |
| $CH_2OEt$ | H | Ph | H | O | H | Ga |
| $CH_2OPr$-n | H | Ph | H | O | H | Gb |
| $CH_2SMe$ | H | Ph | H | O | H | Ga |
| $CH_2SEt$ | H | Ph | H | O | H | Ga |
| $CH_2SPr$-n | H | Ph | H | O | H | Gb |
| $CH_2SO_2Me$ | H | Ph | H | O | H | Ga |
| $CH_2SO_2Et$ | H | Ph | H | O | H | Ga |
| $CH_2COMe$ | H | Ph | H | O | H | Ga |
| $CH_2COEt$ | H | Ph | H | O | H | Ga |
| $CH_2COPr$-n | H | Ph | H | O | H | Gb |
| $CH_2CO_2H$ | H | Ph | H | O | H | Gb |
| $CH_2CO_2Me$ | H | Ph | H | O | H | Ga |
| $CH_2CO_2Et$ | H | Ph | H | O | H | Ga |
| $CH_2CO_2Pr$-n | H | Ph | H | O | H | Gb |
| $CH_2OCH_2CH=CH_2$ | H | Ph | H | O | H | Gb |
| $CH_2OCH_2C\equiv CH$ | H | Ph | H | O | H | Gb |
| $CH=CHCO_2Me$ | H | Ph | H | O | H | Ga |

| $R_3$ | $R_2$ | $R_1$ | $R_4$ | X | M | Gn |
|---|---|---|---|---|---|---|
| $CH=CHCO_2Et$ | H | Ph | H | O | H | Ga |
| $CH=CHCO_2Pr\text{-}n$ | H | Ph | H | O | H | Gb |
| CH=CHCN | H | Ph | H | O | H | Ga |
| Ph | H | Ph | H | O | H | Ga |
| $CH_2Ph$ | H | Ph | H | O | H | Ga |
| OPh | H | Ph | H | O | H | Ga |
| SPh | H | Ph | H | O | H | Ga |
| $SO_2Ph$ | H | Ph | H | O | H | Ga |
| CONHMe | H | Ph | H | O | H | Ga |
| CONHEt | H | Ph | H | O | H | Ga |
| $CON(Me)_2$ | H | Ph | H | O | H | Ga |
| $CON(Et)_2$ | H | Ph | H | O | H | Ga |
| F | H | $CH_2Ph$ | H | O | H | Ga |
| Cl | H | $CH_2Ph$ | H | O | H | Ga |
| Br | H | $CH_2Ph$ | H | O | H | Ga |
| I | H | $CH_2Ph$ | H | O | H | Ga |
| $CH=CH_2$ | H | $CH_2Ph$ | H | O | H | Ga |
| $CH_2CH=CH_2$ | H | $CH_2Ph$ | H | O | H | Ga |
| $C\equiv CH$ | H | $CH_2Ph$ | H | O | H | Ga |
| $CH_2C\equiv CH$ | H | $CH_2Ph$ | H | O | H | Ga |
| $CH_2F$ | H | $CH_2Ph$ | H | O | H | Ga |
| $CHF_2$ | H | $CH_2Ph$ | H | O | H | Ga |
| CN | H | $CH_2Ph$ | H | O | H | Ga |
| $NH_2$ | H | $CH_2Ph$ | H | O | H | Ga |
| NHCOMe | H | $CH_2Ph$ | H | O | H | Ga |
| $CH_2NH_2$ | H | $CH_2Ph$ | H | O | H | Ga |
| $CH_2NHCOMe$ | H | $CH_2Ph$ | H | O | H | Ga |
| $CH_2OCHF_2$ | H | $CH_2Ph$ | H | O | H | Ga |
| $CF_3$ | H | $CH_2Ph$ | H | O | H | Ga |
| $NO_2$ | H | $CH_2Ph$ | H | O | H | Gb |
| OMe | H | $CH_2Ph$ | H | O | H | Ga |
| OEt | H | $CH_2Ph$ | H | O | H | Ga |
| OPr-n | H | $CH_2Ph$ | H | O | H | Gb |
| SMe | H | $CH_2Ph$ | H | O | H | Ga |
| SEt | H | $CH_2Ph$ | H | O | H | Ga |
| $SO_2Me$ | H | $CH_2Ph$ | H | O | H | Ga |
| $SO_2Et$ | H | $CH_2Ph$ | H | O | H | Ga |
| COMe | H | $CH_2Ph$ | H | O | H | Ga |
| COEt | H | $CH_2Ph$ | H | O | H | Ga |
| $CO_2H$ | H | $CH_2Ph$ | H | O | H | Gb |

| $R_3$ | $R_2$ | $R_1$ | $R_4$ | X | M | Gn |
|---|---|---|---|---|---|---|
| $CO_2Me$ | H | $CH_2Ph$ | H | O | H | Ga |
| $CO_2Et$ | H | $CH_2Ph$ | H | O | H | Ga |
| $CO_2Pr$-n | H | $CH_2Ph$ | H | O | H | Gb |
| $CO_2Bu$-n | H | $CH_2Ph$ | H | O | H | Gc |
| $CH_2Cl$ | H | $CH_2Ph$ | H | O | H | Ga |
| $CH_2Br$ | H | $CH_2Ph$ | H | O | H | Gb |
| $CH_2I$ | H | $CH_2Ph$ | H | O | H | Gb |
| $CH_2CF_3$ | H | $CH_2Ph$ | H | O | H | Ga |
| $CH_2CN$ | H | $CH_2Ph$ | H | O | H | Ga |
| $CH_2OH$ | H | $CH_2Ph$ | H | O | H | Gb |
| $CH_2OMe$ | H | $CH_2Ph$ | H | O | H | Ga |
| $CH_2OEt$ | H | $CH_2Ph$ | H | O | H | Ga |
| $CH_2OPr$-n | H | $CH_2Ph$ | H | O | H | Gb |
| $CH_2SMe$ | H | $CH_2Ph$ | H | O | H | Ga |
| $CH_2SEt$ | H | $CH_2Ph$ | H | O | H | Ga |
| $CH_2SPr$-n | H | $CH_2Ph$ | H | O | H | Gb |
| $CH_2SO_2Me$ | H | $CH_2Ph$ | H | O | H | Ga |
| $CH_2SO_2Et$ | H | $CH_2Ph$ | H | O | H | Ga |
| $CH_2COMe$ | H | $CH_2Ph$ | H | O | H | Ga |
| $CH_2COEt$ | H | $CH_2Ph$ | H | O | H | Ga |
| $CH_2COPr$-n | H | $CH_2Ph$ | H | O | H | Gb |
| $CH_2CO_2H$ | H | $CH_2Ph$ | H | O | H | Gb |
| $CH_2CO_2Me$ | H | $CH_2Ph$ | H | O | H | Ga |
| $CH_2CO_2Et$ | H | $CH_2Ph$ | H | O | H | Ga |
| $CH_2CO_2Pr$-n | H | $CH_2Ph$ | H | O | H | Gb |
| $CH_2OCH_2CH=CH_2$ | H | $CH_2Ph$ | H | O | H | Gb |
| $CH_2OCH_2C\equiv CH$ | H | $CH_2Ph$ | H | O | H | Gb |
| $CH=CHCO_2Me$ | H | $CH_2Ph$ | H | O | H | Ga |
| $CH=CHCO_2Et$ | H | $CH_2Ph$ | H | O | H | Ga |
| $CH=CHCO_2Pr$-n | H | $CH_2Ph$ | H | O | H | Gb |
| $CH=CHCN$ | H | $CH_2Ph$ | H | O | H | Ga |
| Ph | H | $CH_2Ph$ | H | O | H | Ga |
| $CH_2Ph$ | H | $CH_2Ph$ | H | O | H | Ga |
| OPh | H | $CH_2Ph$ | H | O | H | Ga |
| SPh | H | $CH_2Ph$ | H | O | H | Ga |
| $SO_2Ph$ | H | $CH_2Ph$ | H | O | H | Ga |
| CONHMe | H | $CH_2Ph$ | H | O | H | Ga |
| CONHEt | H | $CH_2Ph$ | H | O | H | Ga |
| $CON(Me)_2$ | H | $CH_2Ph$ | H | O | H | Ga |
| $CON(Et)_2$ | H | $CH_2Ph$ | H | O | H | Ga |

| $R_3$ | $R_2$ | $R_1$ | $R_4$ | X | M | Gn |
|---|---|---|---|---|---|---|
| F | H | OPh | H | O | H | Ga |
| Cl | H | OPh | H | O | H | Ga |
| Br | H | OPh | H | O | H | Ga |
| I | H | OPh | H | O | H | Ga |
| $CH=CH_2$ | H | OPh | H | O | H | Ga |
| $CH_2CH=CH_2$ | H | OPh | H | O | H | Ga |
| $C\equiv CH$ | H | OPh | H | O | H | Ga |
| $CH_2C\equiv CH$ | H | OPh | H | O | H | Ga |
| $CH_2F$ | H | OPh | H | O | H | Ga |
| $CHF_2$ | H | OPh | H | O | H | Ga |
| CN | H | OPh | H | O | H | Ga |
| $NH_2$ | H | OPh | H | O | H | Ga |
| NHCOMe | H | OPh | H | O | H | Ga |
| $CH_2NH_2$ | H | OPh | H | O | H | Ga |
| $CH_2NHCOMe$ | H | OPh | H | O | H | Ga |
| $CH_2OCHF_2$ | H | OPh | H | O | H | Ga |
| $CF_3$ | H | OPh | H | O | H | Ga |
| $NO_2$ | H | OPh | H | O | H | Gb |
| OMe | H | OPh | H | O | H | Ga |
| OEt | H | OPh | H | O | H | Ga |
| OPr-n | H | OPh | H | O | H | Gb |
| SMe | H | OPh | H | O | H | Ga |
| SEt | H | OPh | H | O | H | Ga |
| $SO_2Me$ | H | OPh | H | O | H | Ga |
| $SO_2Et$ | H | OPh | H | O | H | Ga |
| COMe | H | OPh | H | O | H | Ga |
| COEt | H | OPh | H | O | H | Ga |
| $CO_2H$ | H | OPh | H | O | H | Gb |
| $CO_2Me$ | H | OPh | H | O | H | Ga |
| $CO_2Et$ | H | OPh | H | O | H | Ga |
| $CO_2Pr$-n | H | OPh | H | O | H | Gb |
| $CO_2Bu$-n | H | OPh | H | O | H | Gc |
| $CH_2Cl$ | H | OPh | H | O | H | Ga |
| $CH_2Br$ | H | OPh | H | O | H | Gb |
| $CH_2I$ | H | OPh | H | O | H | Gb |
| $CH_2CF_3$ | H | OPh | H | O | H | Ga |
| $CH_2CN$ | H | OPh | H | O | H | Ga |
| $CH_2OH$ | H | OPh | H | O | H | Gb |
| $CH_2OMe$ | H | OPh | H | O | H | Ga |
| $CH_2OEt$ | H | OPh | H | O | H | Ga |

| $R_3$ | $R_2$ | $R_1$ | $R_4$ | X | M | Gn |
|---|---|---|---|---|---|---|
| $CH_2OPr$-n | H | OPh | H | O | H | Gb |
| $CH_2SMe$ | H | OPh | H | O | H | Ga |
| $CH_2SEt$ | H | OPh | H | O | H | Ga |
| $CH_2SPr$-n | H | OPh | H | O | H | Gb |
| $CH_2SO_2Me$ | H | OPh | H | O | H | Ga |
| $CH_2SO_2Et$ | H | OPh | H | O | H | Ga |
| $CH_2COMe$ | H | OPh | H | O | H | Ga |
| $CH_2COEt$ | H | OPh | H | O | H | Ga |
| $CH_2COPr$-n | H | OPh | H | O | H | Gb |
| $CH_2CO_2H$ | H | OPh | H | O | H | Gb |
| $CH_2CO_2Me$ | H | OPh | H | O | H | Ga |
| $CH_2CO_2Et$ | H | OPh | H | O | H | Ga |
| $CH_2CO_2Pr$-n | H | OPh | H | O | H | Gb |
| $CH_2OCH_2CH{=}CH_2$ | H | OPh | H | O | H | Gb |
| $CH_2OCH_2C{\equiv}CH$ | H | OPh | H | O | H | Gb |
| $CH{=}CHCO_2Me$ | H | OPh | H | O | H | Ga |
| $CH{=}CHCO_2Et$ | H | OPh | H | O | H | Ga |
| $CH{=}CHCO_2Pr$-n | H | OPh | H | O | H | Gb |
| $CH{=}CHCN$ | H | OPh | H | O | H | Ga |
| Ph | H | OPh | H | O | H | Ga |
| $CH_2Ph$ | H | OPh | H | O | H | Ga |
| OPh | H | OPh | H | O | H | Ga |
| SPh | H | OPh | H | O | H | Ga |
| $SO_2Ph$ | H | OPh | H | O | H | Ga |
| CONHMe | H | OPh | H | O | H | Ga |
| CONHEt | H | OPh | H | O | H | Ga |
| $CON(Me)_2$ | H | OPh | H | O | H | Ga |
| $CON(Et)_2$ | H | OPh | H | O | H | Ga |
| F | H | SPh | H | O | H | Ga |
| Cl | H | SPh | H | O | H | Ga |
| Br | H | SPh | H | O | H | Ga |
| I | H | SPh | H | O | H | Ga |
| $CH{=}CH_2$ | H | SPh | H | O | H | Ga |
| $CH_2CH{=}CH_2$ | H | SPh | H | O | H | Ga |
| $C{\equiv}CH$ | H | SPh | H | O | H | Ga |
| $CH_2C{\equiv}CH$ | H | SPh | H | O | H | Ga |
| $CH_2F$ | H | SPh | H | O | H | Ga |
| $CHF_2$ | H | SPh | H | O | H | Ga |
| CN | H | SPh | H | O | H | Ga |
| $NH_2$ | H | SPh | H | O | H | Ga |

| $R_3$ | $R_2$ | $R_1$ | $R_4$ | X | M | Gn |
|---|---|---|---|---|---|---|
| NHCOMe | H | SPh | H | O | H | Ga |
| $CH_2NH_2$ | H | SPh | H | O | H | Ga |
| $CH_2NHCOMe$ | H | SPh | H | O | H | Ga |
| $CH_2OCHF_2$ | H | SPh | H | O | H | Ga |
| $CF_3$ | H | SPh | H | O | H | Ga |
| $NO_2$ | H | SPh | H | O | H | Gb |
| OMe | H | SPh | H | O | H | Ga |
| OEt | H | SPh | H | O | H | Ga |
| OPr-n | H | SPh | H | O | H | Gb |
| SMe | H | SPh | H | O | H | Ga |
| SEt | H | SPh | H | O | H | Ga |
| $SO_2Me$ | H | SPh | H | O | H | Ga |
| $SO_2Et$ | H | SPh | H | O | H | Ga |
| COMe | H | SPh | H | O | H | Ga |
| COEt | H | SPh | H | O | H | Ga |
| $CO_2H$ | H | SPh | H | O | H | Gb |
| $CO_2Me$ | H | SPh | H | O | H | Ga |
| $CO_2Et$ | H | SPh | H | O | H | Ga |
| $CO_2Pr$-n | H | SPh | H | O | H | Gb |
| $CO_2Bu$-n | H | SPh | H | O | H | Gc |
| $CH_2Cl$ | H | SPh | H | O | H | Ga |
| $CH_2Br$ | H | SPh | H | O | H | Gb |
| $CH_2I$ | H | SPh | H | O | H | Gb |
| $CH_2CF_3$ | H | SPh | H | O | H | Ga |
| $CH_2CN$ | H | SPh | H | O | H | Ga |
| $CH_2OH$ | H | SPh | H | O | H | Gb |
| $CH_2OMe$ | H | SPh | H | O | H | Ga |
| $CH_2OEt$ | H | SPh | H | O | H | Ga |
| $CH_2OPr$-n | H | SPh | H | O | H | Gb |
| $CH_2SMe$ | H | SPh | H | O | H | Ga |
| $CH_2SEt$ | H | SPh | H | O | H | Ga |
| $CH_2SPr$-n | H | SPh | H | O | H | Gb |
| $CH_2SO_2Me$ | H | SPh | H | O | H | Ga |
| $CH_2SO_2Et$ | H | SPh | H | O | H | Ga |
| $CH_2COMe$ | H | SPh | H | O | H | Ga |
| $CH_2COEt$ | H | SPh | H | O | H | Ga |
| $CH_2COPr$-n | H | SPh | H | O | H | Gb |
| $CH_2CO_2H$ | H | SPh | H | O | H | Gb |
| $CH_2CO_2Me$ | H | SPh | H | O | H | Ga |
| $CH_2CO_2Et$ | H | SPh | H | O | H | Ga |

| $R_3$ | $R_2$ | $R_1$ | $R_4$ | X | M | Gn |
|---|---|---|---|---|---|---|
| $CH_2CO_2Pr$-n | H | SPh | H | O | H | Gb |
| $CH_2OCH_2CH=CH_2$ | H | SPh | H | O | H | Gb |
| $CH_2OCH_2C\equiv CH$ | H | SPh | H | O | H | Gb |
| $CH=CHCO_2Me$ | H | SPh | H | O | H | Ga |
| $CH=CHCO_2Et$ | H | SPh | H | O | H | Ga |
| $CH=CHCO_2Pr$-n | H | SPh | H | O | H | Gb |
| CH=CHCN | H | SPh | H | O | H | Ga |
| Ph | H | SPh | H | O | H | Ga |
| $CH_2Ph$ | H | SPh | H | O | H | Ga |
| OPh | H | SPh | H | O | H | Ga |
| SPh | H | SPh | H | O | H | Ga |
| $SO_2Ph$ | H | SPh | H | O | H | Ga |
| CONHMe | H | SPh | H | O | H | Ga |
| CONHEt | H | SPh | H | O | H | Ga |
| $CON(Me)_2$ | H | SPh | H | O | H | Ga |
| $CON(Et)_2$ | H | SPh | H | O | H | Ga |
| F | H | $SO_2Ph$ | H | O | H | Ga |
| Cl | H | $SO_2Ph$ | H | O | H | Ga |
| Br | H | $SO_2Ph$ | H | O | H | Ga |
| I | H | $SO_2Ph$ | H | O | H | Ga |
| $CH=CH_2$ | H | $SO_2Ph$ | H | O | H | Ga |
| $CH_2CH=CH_2$ | H | $SO_2Ph$ | H | O | H | Ga |
| $C\equiv CH$ | H | $SO_2Ph$ | H | O | H | Ga |
| $CH_2C\equiv CH$ | H | $SO_2Ph$ | H | O | H | Ga |
| $CH_2F$ | H | $SO_2Ph$ | H | O | H | Ga |
| $CHF_2$ | H | $SO_2Ph$ | H | O | H | Ga |
| CN | H | $SO_2Ph$ | H | O | H | Ga |
| $NH_2$ | H | $SO_2Ph$ | H | O | H | Ga |
| NHCOMe | H | $SO_2Ph$ | H | O | H | Ga |
| $CH_2NH_2$ | H | $SO_2Ph$ | H | O | H | Ga |
| $CH_2NHCOMe$ | H | $SO_2Ph$ | H | O | H | Ga |
| $CH_2OCHF_2$ | H | $SO_2Ph$ | H | O | H | Ga |
| $CF_3$ | H | $SO_2Ph$ | H | O | H | Ga |
| $NO_2$ | H | $SO_2Ph$ | H | O | H | Gb |
| OMe | H | $SO_2Ph$ | H | O | H | Ga |
| OEt | H | $SO_2Ph$ | H | O | H | Ga |
| OPr-n | H | $SO_2Ph$ | H | O | H | Gb |
| SMe | H | $SO_2Ph$ | H | O | H | Ga |
| SEt | H | $SO_2Ph$ | H | O | H | Ga |
| $SO_2Me$ | H | $SO_2Ph$ | H | O | H | Ga |

| $R_3$ | $R_2$ | $R_1$ | $R_4$ | X | M | Gn |
|---|---|---|---|---|---|---|
| $SO_2Et$ | H | $SO_2Ph$ | H | O | H | Ga |
| COMe | H | $SO_2Ph$ | H | O | H | Ga |
| COEt | H | $SO_2Ph$ | H | O | H | Ga |
| $CO_2H$ | H | $SO_2Ph$ | H | O | H | Gb |
| $CO_2Me$ | H | $SO_2Ph$ | H | O | H | Ga |
| $CO_2Et$ | H | $SO_2Ph$ | H | O | H | Ga |
| $CO_2Pr$-n | H | $SO_2Ph$ | H | O | H | Gb |
| $CO_2Bu$-n | H | $SO_2Ph$ | H | O | H | Gc |
| $CH_2Cl$ | H | $SO_2Ph$ | H | O | H | Ga |
| $CH_2Br$ | H | $SO_2Ph$ | H | O | H | Gb |
| $CH_2I$ | H | $SO_2Ph$ | H | O | H | Gb |
| $CH_2CF_3$ | H | $SO_2Ph$ | H | O | H | Ga |
| $CH_2CN$ | H | $SO_2Ph$ | H | O | H | Ga |
| $CH_2OH$ | H | $SO_2Ph$ | H | O | H | Gb |
| $CH_2OMe$ | H | $SO_2Ph$ | H | O | H | Ga |
| $CH_2OEt$ | H | $SO_2Ph$ | H | O | H | Ga |
| $CH_2OPr$-n | H | $SO_2Ph$ | H | O | H | Gb |
| $CH_2SMe$ | H | $SO_2Ph$ | H | O | H | Ga |
| $CH_2SEt$ | H | $SO_2Ph$ | H | O | H | Ga |
| $CH_2SPr$-n | H | $SO_2Ph$ | H | O | H | Gb |
| $CH_2SO_2Me$ | H | $SO_2Ph$ | H | O | H | Ga |
| $CH_2SO_2Et$ | H | $SO_2Ph$ | H | O | H | Ga |
| $CH_2COMe$ | H | $SO_2Ph$ | H | O | H | Ga |
| $CH_2COEt$ | H | $SO_2Ph$ | H | O | H | Ga |
| $CH_2COPr$-n | H | $SO_2Ph$ | H | O | H | Gb |
| $CH_2CO_2H$ | H | $SO_2Ph$ | H | O | H | Gb |
| $CH_2CO_2Me$ | H | $SO_2Ph$ | H | O | H | Ga |
| $CH_2CO_2Et$ | H | $SO_2Ph$ | H | O | H | Ga |
| $CH_2CO_2Pr$-n | H | $SO_2Ph$ | H | O | H | Gb |
| $CH_2OCH_2CH=CH_2$ | H | $SO_2Ph$ | H | O | H | Gb |
| $CH_2OCH_2C \equiv CH$ | H | $SO_2Ph$ | H | O | H | Gb |
| $CH=CHCO_2Me$ | H | $SO_2Ph$ | H | O | H | Ga |
| $CH=CHCO_2Et$ | H | $SO_2Ph$ | H | O | H | Ga |
| $CH=CHCO_2Pr$-n | H | $SO_2Ph$ | H | O | H | Gb |
| CH=CHCN | H | $SO_2Ph$ | H | O | H | Ga |
| Ph | H | $SO_2Ph$ | H | O | H | Ga |
| $CH_2Ph$ | H | $SO_2Ph$ | H | O | H | Ga |
| OPh | H | $SO_2Ph$ | H | O | H | Ga |
| SPh | H | $SO_2Ph$ | H | O | H | Ga |
| $SO_2Ph$ | H | $SO_2Ph$ | H | O | H | Ga |

| $R_3$ | $R_2$ | $R_1$ | $R_4$ | X | M | Gn |
|---|---|---|---|---|---|---|
| CONHMe | H | $SO_2Ph$ | H | O | H | Ga |
| CONHEt | H | $SO_2Ph$ | H | O | H | Ga |
| $CON(Me)_2$ | H | $SO_2Ph$ | H | O | H | Ga |
| $CON(Et)_2$ | H | $SO_2Ph$ | H | O | H | Ga |
| F | H | $CON(Et)_2$ | H | O | H | Ga |
| Cl | H | $CON(Et)_2$ | H | O | H | Ga |
| Br | H | $CON(Et)_2$ | H | O | H | Ga |
| I | H | $CON(Et)_2$ | H | O | H | Ga |
| $CH=CH_2$ | H | $CON(Et)_2$ | H | O | H | Ga |
| $CH_2CH=CH_2$ | H | $CON(Et)_2$ | H | O | H | Ga |
| $C\equiv CH$ | H | $CON(Et)_2$ | H | O | H | Ga |
| $CH_2C\equiv CH$ | H | $CON(Et)_2$ | H | O | H | Ga |
| $CH_2F$ | H | $CON(Et)_2$ | H | O | H | Ga |
| $CHF_2$ | H | $CON(Et)_2$ | H | O | H | Ga |
| CN | H | $CON(Et)_2$ | H | O | H | Ga |
| $NH_2$ | H | $CON(Et)_2$ | H | O | H | Ga |
| NHCOMe | H | $CON(Et)_2$ | H | O | H | Ga |
| $CH_2NH_2$ | H | $CON(Et)_2$ | H | O | H | Ga |
| $CH_2NHCOMe$ | H | $CON(Et)_2$ | H | O | H | Ga |
| $CH_2OCHF_2$ | H | $CON(Et)_2$ | H | O | H | Ga |
| $CF_3$ | H | $CON(Et)_2$ | H | O | H | Ga |
| $NO_2$ | H | $CON(Et)_2$ | H | O | H | Gb |
| OMe | H | $CON(Et)_2$ | H | O | H | Ga |
| OEt | H | $CON(Et)_2$ | H | O | H | Ga |
| OPr-n | H | $CON(Et)_2$ | H | O | H | Gb |
| SMe | H | $CON(Et)_2$ | H | O | H | Ga |
| SEt | H | $CON(Et)_2$ | H | O | H | Ga |
| $SO_2Me$ | H | $CON(Et)_2$ | H | O | H | Ga |
| $SO_2Et$ | H | $CON(Et)_2$ | H | O | H | Ga |
| COMe | H | $CON(Et)_2$ | H | O | H | Ga |
| COEt | H | $CON(Et)_2$ | H | O | H | Ga |
| $CO_2H$ | H | $CON(Et)_2$ | H | O | H | Gb |
| $CO_2Me$ | H | $CON(Et)_2$ | H | O | H | Ga |
| $CO_2Et$ | H | $CON(Et)_2$ | H | O | H | Ga |
| $CO_2Pr$-n | H | $CON(Et)_2$ | H | O | H | Gb |
| $CO_2Bu$-n | H | $CON(Et)_2$ | H | O | H | Gc |
| $CH_2Cl$ | H | $CON(Et)_2$ | H | O | H | Ga |
| $CH_2Br$ | H | $CON(Et)_2$ | H | O | H | Gb |
| $CH_2I$ | H | $CON(Et)_2$ | H | O | H | Gb |
| $CH_2CF_3$ | H | $CON(Et)_2$ | H | O | H | Ga |

| $R_3$ | $R_2$ | $R_1$ | $R_4$ | X | M | Gn |
|---|---|---|---|---|---|---|
| $CH_2CN$ | H | $CON(Et)_2$ | H | O | H | Ga |
| $CH_2OH$ | H | $CON(Et)_2$ | H | O | H | Gb |
| $CH_2OMe$ | H | $CON(Et)_2$ | H | O | H | Ga |
| $CH_2OEt$ | H | $CON(Et)_2$ | H | O | H | Ga |
| $CH_2OPr$-n | H | $CON(Et)_2$ | H | O | H | Gb |
| $CH_2SMe$ | H | $CON(Et)_2$ | H | O | H | Ga |
| $CH_2SEt$ | H | $CON(Et)_2$ | H | O | H | Ga |
| $CH_2SPr$-n | H | $CON(Et)_2$ | H | O | H | Gb |
| $CH_2SO_2Me$ | H | $CON(Et)_2$ | H | O | H | Ga |
| $CH_2SO_2Et$ | H | $CON(Et)_2$ | H | O | H | Ga |
| $CH_2COMe$ | H | $CON(Et)_2$ | H | O | H | Ga |
| $CH_2COEt$ | H | $CON(Et)_2$ | H | O | H | Ga |
| $CH_2COPr$-n | H | $CON(Et)_2$ | H | O | H | Gb |
| $CH_2CO_2H$ | H | $CON(Et)_2$ | H | O | H | Gb |
| $CH_2CO_2Me$ | H | $CON(Et)_2$ | H | O | H | Ga |
| $CH_2CO_2Et$ | H | $CON(Et)_2$ | H | O | H | Ga |
| $CH_2CO_2Pr$-n | H | $CON(Et)_2$ | H | O | H | Gb |
| $CH_2OCH_2CH=CH_2$ | H | $CON(Et)_2$ | H | O | H | Gb |
| $CH_2OCH_2C\equiv CH$ | H | $CON(Et)_2$ | H | O | H | Gb |
| $CH=CHCO_2Me$ | H | $CON(Et)_2$ | H | O | H | Ga |
| $CH=CHCO_2Et$ | H | $CON(Et)_2$ | H | O | H | Ga |
| $CH=CHCO_2Pr$-n | H | $CON(Et)_2$ | H | O | H | Gb |
| $CH=CHCN$ | H | $CON(Et)_2$ | H | O | H | Ga |
| Ph | H | $CON(Et)_2$ | H | O | H | Ga |
| $CH_2Ph$ | H | $CON(Et)_2$ | H | O | H | Ga |
| OPh | H | $CON(Et)_2$ | H | O | H | Ga |
| SPh | H | $CON(Et)_2$ | H | O | H | Ga |
| $SO_2Ph$ | H | $CON(Et)_2$ | H | O | H | Ga |
| CONHMe | H | $CON(Et)_2$ | H | O | H | Ga |
| CONHEt | H | $CON(Et)_2$ | H | O | H | Ga |
| $CON(Me)_2$ | H | $CON(Et)_2$ | H | O | H | Ga |
| $CON(Et)_2$ | H | $CON(Et)_2$ | H | O | H | Ga |
| F | H | CN | H | O | H | Ga |
| Cl | H | CN | H | O | H | Ga |
| Br | H | CN | H | O | H | Ga |
| I | H | CN | H | O | H | Ga |
| $CH=CH_2$ | H | CN | H | O | H | Ga |
| $CH_2CH=CH_2$ | H | CN | H | O | H | Ga |
| $C\equiv CH$ | H | CN | H | O | H | Ga |
| $CH_2C\equiv CH$ | H | CN | H | O | H | Ga |

| $R_3$ | $R_2$ | $R_1$ | $R_4$ | X | M | Gn |
|---|---|---|---|---|---|---|
| $CH_2F$ | H | CN | H | O | H | Ga |
| $CHF_2$ | H | CN | H | O | H | Ga |
| CN | H | CN | H | O | H | Ga |
| $NH_2$ | H | CN | H | O | H | Ga |
| NHCOMe | H | CN | H | O | H | Ga |
| $CH_2NH_2$ | H | CN | H | O | H | Ga |
| $CH_2NHCOMe$ | H | CN | H | O | H | Ga |
| $CH_2OCHF_2$ | H | CN | H | O | H | Ga |
| $CF_3$ | H | CN | H | O | H | Ga |
| $NO_2$ | H | CN | H | O | H | Gb |
| OMe | H | CN | H | O | H | Ga |
| OEt | H | CN | H | O | H | Ga |
| OPr-n | H | CN | H | O | H | Gb |
| SMe | H | CN | H | O | H | Ga |
| SEt | H | CN | H | O | H | Ga |
| $SO_2Me$ | H | CN | H | O | H | Ga |
| $SO_2Et$ | H | CN | H | O | H | Ga |
| COMe | H | CN | H | O | H | Ga |
| COEt | H | CN | H | O | H | Ga |
| $CO_2H$ | H | CN | H | O | H | Gb |
| $CO_2Me$ | H | CN | H | O | H | Ga |
| $CO_2Et$ | H | CN | H | O | H | Ga |
| $CO_2Pr$-n | H | CN | H | O | H | Gb |
| $CO_2Bu$-n | H | CN | H | O | H | Gc |
| $CH_2Cl$ | H | CN | H | O | H | Ga |
| $CH_2Br$ | H | CN | H | O | H | Gb |
| $CH_2I$ | H | CN | H | O | H | Gb |
| $CH_2CF_3$ | H | CN | H | O | H | Ga |
| $CH_2CN$ | H | CN | H | O | H | Ga |
| $CH_2OH$ | H | CN | H | O | H | Gb |
| $CH_2OMe$ | H | CN | H | O | H | Ga |
| $CH_2OEt$ | H | CN | H | O | H | Ga |
| $CH_2OPr$-n | H | CN | H | O | H | Gb |
| $CH_2SMe$ | H | CN | H | O | H | Ga |
| $CH_2SEt$ | H | CN | H | O | H | Ga |
| $CH_2SPr$-n | H | CN | H | O | H | Gb |
| $CH_2SO_2Me$ | H | CN | H | O | H | Ga |
| $CH_2SO_2Et$ | H | CN | H | O | H | Ga |
| $CH_2COMe$ | H | CN | H | O | H | Ga |
| $CH_2COEt$ | H | CN | H | O | H | Ga |

| $R_3$ | $R_2$ | $R_1$ | $R_4$ | X | M | Gn |
|---|---|---|---|---|---|---|
| $CH_2COPr$-n | H | CN | H | O | H | Gb |
| $CH_2CO_2H$ | H | CN | H | O | H | Gb |
| $CH_2CO_2Me$ | H | CN | H | O | H | Ga |
| $CH_2CO_2Et$ | H | CN | H | O | H | Ga |
| $CH_2CO_2Pr$-n | H | CN | H | O | H | Gb |
| $CH_2OCH_2CH=CH_2$ | H | CN | H | O | H | Gb |
| $CH_2OCH_2C\equiv CH$ | H | CN | H | O | H | Gb |
| $CH=CHCO_2Me$ | H | CN | H | O | H | Ga |
| $CH=CHCO_2Et$ | H | CN | H | O | H | Ga |
| $CH=CHCO_2Pr$-n | H | CN | H | O | H | Gb |
| CH=CHCN | H | CN | H | O | H | Ga |
| Ph | H | CN | H | O | H | Ga |
| $CH_2Ph$ | H | CN | H | O | H | Ga |
| OPh | H | CN | H | O | H | Ga |
| SPh | H | CN | H | O | H | Ga |
| $SO_2Ph$ | H | CN | H | O | H | Ga |
| CONHMe | H | CN | H | O | H | Ga |
| CONHEt | H | CN | H | O | H | Ga |
| $CON(Me)_2$ | H | CN | H | O | H | Ga |
| $CON(Et)_2$ | H | CN | H | O | H | Ga |
| F | H | Et | H | O | H | Ga |
| Cl | H | Et | H | O | H | Ga |
| Br | H | Et | H | O | H | Ga |
| I | H | Et | H | O | H | Ga |
| $CH=CH_2$ | H | Et | H | O | H | Ga |
| $CH_2CH=CH_2$ | H | Et | H | O | H | Ga |
| $C\equiv CH$ | H | Et | H | O | H | Ga |
| $CH_2C\equiv CH$ | H | Et | H | O | H | Ga |
| $CH_2F$ | H | Et | H | O | H | Ga |
| $CHF_2$ | H | Et | H | O | H | Ga |
| CN | H | Et | H | O | H | Ga |
| $NH_2$ | H | Et | H | O | H | Ga |
| NHCOMe | H | Et | H | O | H | Ga |
| $CH_2NH_2$ | H | Et | H | O | H | Ga |
| $CH_2NHCOMe$ | H | Et | H | O | H | Ga |
| $CH_2OCHF_2$ | H | Et | H | O | H | Ga |
| $CF_3$ | H | Et | H | O | H | Ga |
| $NO_2$ | H | Et | H | O | H | Gb |
| OMe | H | Et | H | O | H | Ga |
| OEt | H | Et | H | O | H | Ga |

| $R_3$ | $R_2$ | $R_1$ | $R_4$ | X | M | Gn |
|---|---|---|---|---|---|---|
| OPr-n | H | Et | H | O | H | Gb |
| SMe | H | Et | H | O | H | Ga |
| SEt | H | Et | H | O | H | Ga |
| $SO_2Me$ | H | Et | H | O | H | Ga |
| $SO_2Et$ | H | Et | H | O | H | Ga |
| COMe | H | Et | H | O | H | Ga |
| COEt | H | Et | H | O | H | Ga |
| $CO_2H$ | H | Et | H | O | H | Gb |
| $CO_2Me$ | H | Et | H | O | H | Ga |
| $CO_2Et$ | H | Et | H | O | H | Ga |
| $CO_2Pr$-n | H | Et | H | O | H | Gb |
| $CO_2Bu$-n | H | Et | H | O | H | Gc |
| $CH_2Cl$ | H | Et | H | O | H | Ga |
| $CH_2Br$ | H | Et | H | O | H | Gb |
| $CH_2I$ | H | Et | H | O | H | Gb |
| $CH_2CF_3$ | H | Et | H | O | H | Ga |
| $CH_2CN$ | H | Et | H | O | H | Ga |
| $CH_2OH$ | H | Et | H | O | H | Gb |
| $CH_2OMe$ | H | Et | H | O | H | Ga |
| $CH_2OEt$ | H | Et | H | O | H | Ga |
| $CH_2OPr$-n | H | Et | H | O | H | Gb |
| $CH_2SMe$ | H | Et | H | O | H | Ga |
| $CH_2SEt$ | H | Et | H | O | H | Ga |
| $CH_2SPr$-n | H | Et | H | O | H | Gb |
| $CH_2SO_2Me$ | H | Et | H | O | H | Ga |
| $CH_2SO_2Et$ | H | Et | H | O | H | Ga |
| $CH_2COMe$ | H | Et | H | O | H | Ga |
| $CH_2COEt$ | H | Et | H | O | H | Ga |
| $CH_2COPr$-n | H | Et | H | O | H | Gb |
| $CH_2CO_2H$ | H | Et | H | O | H | Gb |
| $CH_2CO_2Me$ | H | Et | H | O | H | Ga |
| $CH_2CO_2Et$ | H | Et | H | O | H | Ga |
| $CH_2CO_2Pr$-n | H | Et | H | O | H | Gb |
| $CH_2OCH_2CH=CH_2$ | H | Et | H | O | H | Gb |
| $CH_2OCH_2C\equiv CH$ | H | Et | H | O | H | Gb |
| $CH=CHCO_2Me$ | H | Et | H | O | H | Ga |
| $CH=CHCO_2Et$ | H | Et | H | O | H | Ga |
| $CH=CHCO_2Pr$-n | H | Et | H | O | H | Gb |
| CH=CHCN | H | Et | H | O | H | Ga |
| Ph | H | Et | H | O | H | Ga |

| $R_3$ | $R_2$ | $R_1$ | $R_4$ | X | M | Gn |
|---|---|---|---|---|---|---|
| $CH_2Ph$ | H | Et | H | O | H | Ga |
| OPh | H | Et | H | O | H | Ga |
| SPh | H | Et | H | O | H | Ga |
| $SO_2Ph$ | H | Et | H | O | H | Ga |
| CONHMe | H | Et | H | O | H | Ga |
| CONHEt | H | Et | H | O | H | Ga |
| $CON(Me)_2$ | H | Et | H | O | H | Ga |
| $CON(Et)_2$ | H | Et | H | O | H | Ga |

Table 8

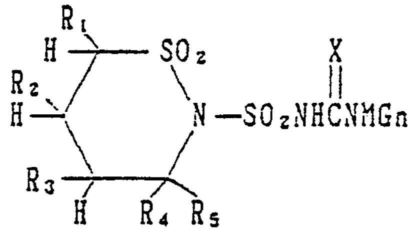

| $R_1$ | $R_2$ | $R_3$ | $R_4$ | $R_5$ | X | M | Gn |
|---|---|---|---|---|---|---|---|
| H | H | H | H | H | O | H | Ga |
| H | H | H | H | H | S | H | G3 |
| Me | H | H | H | H | O | H | Ga |
| Me | H | H | H | H | S | H | G3 |
| H | Me | H | H | H | O | H | Ga |
| H | Me | H | H | H | S | H | G3 |
| H | H | Me | H | H | O | H | Ga |
| H | H | Me | H | H | S | H | G3 |
| H | H | H | Me | H | O | H | Ga |
| H | H | H | Me | H | S | H | G3 |
| H | H | H | Me | H | O | Me | Gb |
| H | H | H | Me | H | O | Et | Gb |
| H | H | H | Me | H | O | $CH_2CH=CH_2$ | Gb |
| H | H | H | Me | H | O | $CH_2C\equiv CH$ | Gb |
| Et | H | H | H | H | O | H | Gb |
| Et | H | H | H | H | S | H | G3 |
| H | Et | H | H | H | O | H | Gb |
| H | Et | H | H | H | S | H | G3 |
| H | H | Et | H | H | O | H | Gb |
| H | H | Et | H | H | S | H | G3 |
| H | H | H | Et | H | O | H | Ga |
| H | H | H | Et | H | S | H | G3 |
| Pr-n | H | H | H | H | O | H | Gb |
| H | Pr-n | H | H | H | O | H | Gb |
| H | H | Pr-n | H | H | O | H | Gb |
| H | H | H | Pr-n | H | O | H | Gb |
| Pr-i | H | H | H | H | O | H | Gb |
| H | Pr-i | H | H | H | O | H | Gb |
| H | H | Pr-i | H | H | O | H | Gb |
| H | H | H | Pr-i | H | O | H | Gb |

| $R_1$ | $R_2$ | $R_3$ | $R_4$ | $R_5$ | X | M | Gn |
|---|---|---|---|---|---|---|---|
| Bu-n | H | H | H | H | O | H | Gc |
| H | Bu-n | H | H | H | O | H | Gc |
| H | H | Bu-n | H | H | O | H | Gc |
| H | H | H | Bu-n | H | O | H | Gc |
| H | H | H | Me | Me | O | H | Ga |
| H | H | H | Me | Me | S | H | G3 |
| H | H | H | Me | Me | O | Me | Gb |
| H | H | H | Me | Me | O | Et | Gb |
| H | H | H | Me | Me | O | $CH_2CH=CH_2$ | Gb |
| H | H | H | Me | Me | O | $CH_2C\equiv CH$ | Gb |
| Me | H | H | Me | Me | O | H | Ga |
| H | Me | H | Me | Me | O | H | Ga |
| H | H | Me | Me | Me | O | H | Ga |
| Et | H | H | Me | Me | O | H | Gb |
| H | Et | H | Me | Me | O | H | Gb |
| H | H | Et | Me | Me | O | H | Gb |
| Pr-n | H | H | Me | Me | O | H | Gb |
| H | Pr-n | H | Me | Me | O | H | Gb |
| H | H | Pr-n | Me | Me | O | H | Gb |
| Pr-i | H | H | Me | Me | O | H | Gb |
| H | Pr-i | H | Me | Me | O | H | Gb |
| H | H | Pr-i | Me | Me | O | H | Gb |
| Bu-n | H | H | Me | Me | O | H | Gc |
| H | Bu-n | H | Me | Me | O | H | Gc |
| H | H | Bu-n | Me | Me | O | H | Gc |
| Me | Me | H | Me | Me | O | H | Gb |
| Me | H | Me | Me | Me | O | H | Gb |
| H | Me | Me | Me | Me | O | H | Gb |
| Me | Me | Me | Me | Me | O | H | Gb |
| Et | Et | H | Me | Me | O | H | Gc |
| Et | H | Et | Me | Me | O | H | Gc |
| H | Et | Et | Me | Me | O | H | Gc |
| Et | Et | Et | Me | Me | O | H | Gc |
| Me | Et | H | Me | Me | O | H | Gc |
| Me | H | Et | Me | Me | O | H | Gc |
| H | Et | Me | Me | Me | O | H | Gc |
| H | Me | Et | Me | Me | O | H | Gc |
| Et | H | Me | Me | Me | O | H | Gc |
| Et | Me | H | Me | Me | O | H | Gc |
| H | H | H | Me | Et | O | H | Ga |

| $R_1$ | $R_2$ | $R_3$ | $R_4$ | $R_5$ | X | M | Gn |
|---|---|---|---|---|---|---|---|
| H | H | H | Me | Et | S | H | G3 |
| Me | H | H | Me | Et | 0 | H | Ga |
| H | Me | H | Me | Et | 0 | H | Ga |
| H | H | Me | Me | Et | 0 | H | Ga |
| Et | H | H | Me | Et | 0 | H | Gb |
| H | Et | H | Me | Et | 0 | H | Gb |
| H | H | Et | Me | Et | 0 | H | Gb |
| Pr-n | H | H | Me | Et | 0 | H | Gb |
| H | Pr-n | H | Me | Et | 0 | H | Gb |
| H | H | Pr-n | Me | Et | 0 | H | Gb |
| Pr-i | H | H | Me | Et | 0 | H | Gb |
| H | Pr-i | H | Me | Et | 0 | H | Gb |
| H | H | Pr-i | Me | Et | 0 | H | Gb |
| Bu-n | H | H | Me | Et | 0 | H | Gc |
| H | Bu-n | H | Me | Et | 0 | H | Gc |
| H | H | Bu-n | Me | Et | 0 | H | Gc |
| Me | Me | H | Me | Et | 0 | H | Gb |
| Me | H | Me | Me | Et | 0 | H | Gb |
| H | Me | Me | Me | Et | 0 | H | Gb |
| Me | Me | Me | Me | Et | 0 | H | Gb |
| Et | Et | H | Me | Et | 0 | H | Gc |
| Et | H | Et | Me | Et | 0 | H | Gc |
| H | Et | Et | Me | Et | 0 | H | Gc |
| Et | Et | Et | Me | Et | 0 | H | Gc |
| Me | Et | H | Me | Et | 0 | H | Gc |
| Me | H | Et | Me | Et | 0 | H | Gc |
| H | Et | Me | Me | Et | 0 | H | Gc |
| H | Me | Et | Me | Et | 0 | H | Gc |
| Et | H | Me | Me | Et | 0 | H | Gc |
| Et | Me | H | Me | Et | 0 | H | Gc |
| H | H | H | Me | Pr-n | S | H | G3 |
| H | H | H | Me | Pr-n | 0 | H | Ga |
| Me | H | H | Me | Pr-n | 0 | H | Ga |
| H | Me | H | Me | Pr-n | 0 | H | Ga |
| H | H | Me | Me | Pr-n | 0 | H | Ga |
| Et | H | H | Me | Pr-n | 0 | H | Gb |
| H | Et | H | Me | Pr-n | 0 | H | Gb |
| H | H | Et | Me | Pr-n | 0 | H | Gb |
| Pr-n | H | H | Me | Pr-n | 0 | H | Gb |

| $R_1$ | $R_2$ | $R_3$ | $R_4$ | $R_5$ | X | M | Gn |
|---|---|---|---|---|---|---|---|
| H | Pr-n | H | Me | Pr-n | O | H | Gb |
| H | H | Pr-n | Me | Pr-n | O | H | Gb |
| Pr-i | H | H | Me | Pr-n | O | H | Gb |
| H | Pr-i | H | Me | Pr-n | O | H | Gb |
| H | H | Pr-i | Me | Pr-n | O | H | Gb |
| Bu-n | H | H | Me | Pr-n | O | H | Gc |
| H | Bu-n | H | Me | Pr-n | O | H | Gc |
| H | H | Bu-n | Me | Pr-n | O | H | Gc |
| Me | Me | H | Me | Pr-n | O | H | Gb |
| Me | H | Me | Me | Pr-n | O | H | Gb |
| H | Me | Me | Me | Pr-n | O | H | Gb |
| Me | Me | Me | Me | Pr-n | O | H | Gb |
| Et | Et | H | Me | Pr-n | O | H | Gc |
| Et | H | Et | Me | Pr-n | O | H | Gc |
| H | Et | Et | Me | Pr-n | O | H | Gc |
| Et | Et | Et | Me | Pr-n | O | H | Gc |
| Me | Et | H | Me | Pr-n | O | H | Gc |
| Me | H | Et | Me | Pr-n | O | H | Gc |
| H | Et | Me | Me | Pr-n | O | H | Gc |
| H | Me | Et | Me | Pr-n | O | H | Gc |
| Et | H | Me | Me | Pr-n | O | H | Gc |
| Et | Me | H | Me | Pr-n | O | H | Gc |
| H | H | H | Me | Pr-i | O | H | Gb |
| H | H | H | Me | Pr-i | S | H | G3 |
| Me | H | H | Me | Pr-i | O | H | Ga |
| H | Me | H | Me | Pr-i | O | H | Ga |
| H | H | Me | Me | Pr-i | O | H | Ga |
| Et | H | H | Me | Pr-i | O | H | Gb |
| H | Et | H | Me | Pr-i | O | H | Gb |
| H | H | Et | Me | Pr-i | O | H | Gb |
| Pr-n | H | H | Me | Pr-i | O | H | Gb |
| H | Pr-n | H | Me | Pr-i | O | H | Gb |
| H | H | Pr-n | Me | Pr-i | O | H | Gb |
| Pr-i | H | H | Me | Pr-i | O | H | Gb |
| H | Pr-i | H | Me | Pr-i | O | H | Gb |
| H | H | Pr-i | Me | Pr-i | O | H | Gb |
| Bu-n | H | H | Me | Pr-i | O | H | Gc |
| H | Bu-n | H | Me | Pr-i | O | H | Gc |

| $R_1$ | $R_2$ | $R_3$ | $R_4$ | $R_5$ | X | M | Gn |
|---|---|---|---|---|---|---|---|
| H | H | Bu-n | Me | Pr-i | O | H | Gc |
| Me | Me | H | Me | Pr-i | O | H | Gb |
| Me | H | Me | Me | Pr-i | O | H | Gb |
| H | Me | Me | Me | Pr-i | O | H | Gb |
| Me | Me | Me | Me | Pr-i | O | H | Gb |
| Et | Et | H | Me | Pr-i | O | H | Gc |
| Et | H | Et | Me | Pr-i | O | H | Gc |
| H | Et | Et | Me | Pr-i | O | H | Gc |
| Et | Et | Et | Me | Pr-i | O | H | Gc |
| Me | Et | H | Me | Pr-i | O | H | Gc |
| Me | H | Et | Me | Pr-i | O | H | Gc |
| H | Et | Me | Me | Pr-i | O | H | Gc |
| H | Me | Et | Me | Pr-i | O | H | Gc |
| Et | H | Me | Me | Pr-i | O | H | Gc |
| Et | Me | H | Me | Pr-i | O | H | Gc |
| H | H | H | Et | Et | O | H | Gb |
| H | H | H | Et | Et | S | H | G3 |
| Me | H | H | Et | Et | O | H | Ga |
| H | Me | H | Et | Et | O | H | Ga |
| H | H | Me | Et | Et | O | H | Ga |
| Et | H | H | Et | Et | O | H | Gb |
| H | Et | H | Et | Et | O | H | Gb |
| H | H | Et | Et | Et | O | H | Gb |
| Pr-n | H | H | Et | Et | O | H | Gb |
| H | Pr-n | H | Et | Et | O | H | Gb |
| H | H | Pr-n | Et | Et | O | H | Gb |
| Pr-i | H | H | Et | Et | O | H | Gb |
| H | Pr-i | H | Et | Et | O | H | Gb |
| H | H | Pr-i | Et | Et | O | H | Gb |
| Bu-n | H | H | Et | Et | O | H | Gc |
| H | Bu-n | H | Et | Et | O | H | Gc |
| H | H | Bu-n | Et | Et | O | H | Gc |
| Me | Me | H | Et | Et | O | H | Gb |
| Me | H | Me | Et | Et | O | H | Gb |
| H | Me | Me | Et | Et | O | H | Gb |
| Me | Me | Me | Et | Et | O | H | Gb |
| Et | Et | H | Et | Et | O | H | Gc |
| Et | H | Et | Et | Et | O | H | Gc |

| $R_1$ | $R_2$ | $R_3$ | $R_4$ | $R_5$ | X | M | Gn |
|---|---|---|---|---|---|---|---|
| H | Et | Et | Et | Et | O | H | Gc |
| Et | Et | Et | Et | Et | O | H | Gc |
| Me | Et | H | Et | Et | O | H | Gc |
| Me | H | Et | Et | Et | O | H | Gc |
| H | Et | Me | Et | Et | O | H | Gc |
| H | Me | Et | Et | Et | O | H | Gc |
| Et | H | Me | Et | Et | O | H | Gc |
| Et | Me | H | Et | Et | O | H | Gc |
| F | H | H | Me | Me | O | H | Gb |
| Cl | H | H | Me | Me | O | H | Gb |
| Br | H | H | Me | Me | O | H | Gb |
| I | H | H | Me | Me | O | H | Gc |
| $CH=CH_2$ | H | H | Me | Me | O | H | Gb |
| $CH_2CH=CH_2$ | H | H | Me | Me | O | H | Gb |
| $C\equiv CH$ | H | H | Me | Me | O | H | Gb |
| $CH_2C\equiv CH$ | H | H | Me | Me | O | H | Gb |
| $CH_2F$ | H | H | Me | Me | O | H | Gb |
| $CHF_2$ | H | H | Me | Me | O | H | Gb |
| CN | H | H | Me | Me | O | H | Gb |
| $NH_2$ | H | H | Me | Me | O | H | Gb |
| NHCOMe | H | H | Me | Me | O | H | Gb |
| $CH_2NH_2$ | H | H | Me | Me | O | H | Gb |
| $CH_2NHCOMe$ | H | H | Me | Me | O | H | Gb |
| $CH_2OCHF_2$ | H | H | Me | Me | O | H | Gb |
| $CF_3$ | H | H | Me | Me | O | H | Gb |
| $NO_2$ | H | H | Me | Me | O | H | Gb |
| OMe | H | H | Me | Me | O | H | Ga |
| OEt | H | H | Me | Me | O | H | Ga |
| OPr-n | H | H | Me | Me | O | H | Gb |
| SMe | H | H | Me | Me | O | H | Ga |
| SEt | H | H | Me | Me | O | H | Ga |
| $SO_2Me$ | H | H | Me | Me | O | H | Ga |
| COMe | H | H | Me | Me | O | H | Ga |
| COEt | H | H | Me | Me | O | H | Ga |
| $CO_2H$ | H | H | Me | Me | O | H | Ga |
| $CO_2Me$ | H | H | Me | Me | O | H | Ga |
| $CO_2Et$ | H | H | Me | Me | O | H | Ga |
| $CO_2Pr$-n | H | H | Me | Me | O | H | Gb |

| $R_1$ | $R_2$ | $R_3$ | $R_4$ | $R_5$ | X | M | Gn |
|---|---|---|---|---|---|---|---|
| $CO_2Bu$-n | H | H | Me | Me | O | H | Gc |
| $CH_2Cl$ | H | H | Me | Me | O | H | Ga |
| $CH_2Br$ | H | H | Me | Me | O | H | Gb |
| $CH_2I$ | H | H | Me | Me | O | H | Gb |
| $CH_2CF_3$ | H | H | Me | Me | O | H | Gb |
| $CH_2CN$ | H | H | Me | Me | O | H | Ga |
| $CH_2OH$ | H | H | Me | Me | O | H | Gb |
| $CH_2OMe$ | H | H | Me | Me | O | H | Ga |
| $CH_2OEt$ | H | H | Me | Me | O | H | Ga |
| $CH_2OPr$-n | H | H | Me | Me | O | H | Gb |
| $CH_2SMe$ | H | H | Me | Me | O | H | Ga |
| $CH_2SEt$ | H | H | Me | Me | O | H | Ga |
| $CH_2SO_2Me$ | H | H | Me | Me | O | H | Ga |
| $CH_2SO_2Et$ | H | H | Me | Me | O | H | Ga |
| $CH_2SPr$-n | H | H | Me | Me | O | H | Gb |
| $CH_2SPr$-i | H | H | Me | Me | O | H | Gb |
| $CH_2COMe$ | H | H | Me | Me | O | H | Ga |
| $CH_2COEt$ | H | H | Me | Me | O | H | Ga |
| $CH_2COPr$-n | H | H | Me | Me | O | H | Gb |
| $CH_2CO_2H$ | H | H | Me | Me | O | H | Ga |
| $CH_2CO_2Me$ | H | H | Me | Me | O | H | Ga |
| $CH_2CO_2Et$ | H | H | Me | Me | O | H | Ga |
| $CH_2CO_2Pr$-n | H | H | Me | Me | O | H | Gb |
| $CH_2OCH_2CH=CH_2$ | H | H | Me | Me | O | H | Gb |
| $CH_2OCH_2C\equiv CH$ | H | H | Me | Me | O | H | Gb |
| $CH=CHCO_2Me$ | H | H | Me | Me | O | H | Ga |
| $CH=CHCO_2Et$ | H | H | Me | Me | O | H | Ga |
| $CH=CHCO_2Pr$-n | H | H | Me | Me | O | H | Gb |
| CH=CHCN | H | H | Me | Me | O | H | Ga |
| Ph | H | H | Me | Me | O | H | Ga |
| $CH_2Ph$ | H | H | Me | Me | O | H | Ga |
| OPh | H | H | Me | Me | O | H | Ga |
| SPh | H | H | Me | Me | O | H | Ga |
| $SO_2Ph$ | H | H | Me | Me | O | H | Ga |
| CONHMe | H | H | Me | Me | O | H | Ga |
| CONHEt | H | H | Me | Me | O | H | Ga |
| $CON(Me)_2$ | H | H | Me | Me | O | H | Ga |
| $CON(Et)_2$ | H | H | Me | Me | O | H | Ga |
| $CON(Pr\text{-}n)_2$ | H | H | Me | Me | O | H | Gb |
| H | F | H | Me | Me | O | H | Gb |

| $R_1$ | $R_2$ | $R_3$ | $R_4$ | $R_5$ | X | M | Gn |
|---|---|---|---|---|---|---|---|
| H | Cl | H | Me | Me | O | H | Gb |
| H | Br | H | Me | Me | O | H | Gb |
| H | I | H | Me | Me | O | H | Gc |
| H | $CH=CH_2$ | H | Me | Me | O | H | Gb |
| H | $CH_2CH=CH_2$ | H | Me | Me | O | H | Gb |
| H | $C\equiv CH$ | H | Me | Me | O | H | Gb |
| H | $CH_2C\equiv CH$ | H | Me | Me | O | H | Gb |
| H | $CH_2F$ | H | Me | Me | O | H | Gb |
| H | $CHF_2$ | H | Me | Me | O | H | Gb |
| H | CN | H | Me | Me | O | H | Gb |
| H | $NH_2$ | H | Me | Me | O | H | Gb |
| H | NHCOMe | H | Me | Me | O | H | Gb |
| H | $CH_2NH_2$ | H | Me | Me | O | H | Gb |
| H | $CH_2NHCOMe$ | H | Me | Me | O | H | Gb |
| H | $CH_2OCHF_2$ | H | Me | Me | O | H | Gb |
| H | $CF_3$ | H | Me | Me | O | H | Gb |
| H | $NO_2$ | H | Me | Me | O | H | Gb |
| H | OMe | H | Me | Me | O | H | Ga |
| H | OEt | H | Me | Me | O | H | Ga |
| H | OPr-n | H | Me | Me | O | H | Gb |
| H | SMe | H | Me | Me | O | H | Ga |
| H | SEt | H | Me | Me | O | H | Ga |
| H | $SO_2Me$ | H | Me | Me | O | H | Ga |
| H | COMe | H | Me | Me | O | H | Ga |
| H | COEt | H | Me | Me | O | H | Ga |
| H | $CO_2H$ | H | Me | Me | O | H | Ga |
| H | $CO_2Me$ | H | Me | Me | O | H | Ga |
| H | $CO_2Et$ | H | Me | Me | O | H | Ga |
| H | $CO_2Pr$-n | H | Me | Me | O | H | Gb |
| H | $CO_2Bu$-n | H | Me | Me | O | H | Gc |
| H | $CH_2Cl$ | H | Me | Me | O | H | Ga |
| H | $CH_2Br$ | H | Me | Me | O | H | Gb |
| H | $CH_2I$ | H | Me | Me | O | H | Gb |
| H | $CH_2CF_3$ | H | Me | Me | O | H | Gb |
| H | $CH_2CN$ | H | Me | Me | O | H | Ga |
| H | $CH_2OH$ | H | Me | Me | O | H | Gb |
| H | $CH_2OMe$ | H | Me | Me | O | H | Ga |
| H | $CH_2OEt$ | H | Me | Me | O | H | Ga |
| H | $CH_2OPr$-n | H | Me | Me | O | H | Gb |
| H | $CH_2SMe$ | H | Me | Me | O | H | Ga |

| $R_1$ | $R_2$ | $R_3$ | $R_4$ | $R_5$ | X | M | Gn |
|---|---|---|---|---|---|---|---|
| H | $CH_2SEt$ | H | Me | Me | O | H | Ga |
| H | $CH_2SO_2Me$ | H | Me | Me | O | H | Ga |
| H | $CH_2SO_2Et$ | H | Me | Me | O | H | Ga |
| H | $CH_2SPr$-n | H | Me | Me | O | H | Gb |
| H | $CH_2SPr$-i | H | Me | Me | O | H | Gb |
| H | $CH_2COMe$ | H | Me | Me | O | H | Ga |
| H | $CH_2COEt$ | H | Me | Me | O | H | Ga |
| H | $CH_2COPr$-n | H | Me | Me | O | H | Gb |
| H | $CH_2CO_2H$ | H | Me | Me | O | H | Ga |
| H | $CH_2CO_2Me$ | H | Me | Me | O | H | Ga |
| H | $CH_2CO_2Et$ | H | Me | Me | O | H | Ga |
| H | $CH_2CO_2Pr$-n | H | Me | Me | O | H | Gb |
| H | $CH_2OCH_2CH=CH_2$ | H | Me | Me | O | H | Gb |
| H | $CH_2OCH_2C\equiv CH$ | H | Me | Me | O | H | Gb |
| H | $CH=CHCO_2Me$ | H | Me | Me | O | H | Ga |
| H | $CH=CHCO_2Et$ | H | Me | Me | O | H | Ga |
| H | $CH=CHCO_2Pr$-n | H | Me | Me | O | H | Gb |
| H | CH=CHCN | H | Me | Me | O | H | Ga |
| H | Ph | H | Me | Me | O | H | Ga |
| H | $CH_2Ph$ | H | Me | Me | O | H | Ga |
| H | OPh | H | Me | Me | O | H | Ga |
| H | SPh | H | Me | Me | O | H | Ga |
| H | $SO_2Ph$ | H | Me | Me | O | H | Ga |
| H | CONHMe | H | Me | Me | O | H | Ga |
| H | CONHEt | H | Me | Me | O | H | Ga |
| H | $CON(Me)_2$ | H | Me | Me | O | H | Ga |
| H | $CON(Et)_2$ | H | Me | Me | O | H | Ga |
| H | $CON(Pr\text{-}n)_2$ | H | Me | Me | O | H | Gb |
| H | H | F | Me | Me | O | H | Gb |
| H | H | Cl | Me | Me | O | H | Gb |
| H | H | Br | Me | Me | O | H | Gb |
| H | H | I | Me | Me | O | H | Gc |
| H | H | $CH=CH_2$ | Me | Me | O | H | Gb |
| H | H | $CH_2CH=CH_2$ | Me | Me | O | H | Gb |
| H | H | $C\equiv CH$ | Me | Me | O | H | Gb |
| H | H | $CH_2C\equiv CH$ | Me | Me | O | H | Gb |
| H | H | $CH_2F$ | Me | Me | O | H | Gb |
| H | H | $CHF_2$ | Me | Me | O | H | Gb |
| H | H | CN | Me | Me | O | H | Gb |
| H | H | $NH_2$ | Me | Me | O | H | Gb |

| $R_1$ | $R_2$ | $R_3$ | $R_4$ | $R_5$ | X | M | Gn |
|---|---|---|---|---|---|---|---|
| H | H | NHCOMe | Me | Me | O | H | Gb |
| H | H | $CH_2NH_2$ | Me | Me | O | H | Gb |
| H | H | $CH_2NHCOMe$ | Me | Me | O | H | Gb |
| H | H | $CH_2OCHF_2$ | Me | Me | O | H | Gb |
| H | H | $CF_3$ | Me | Me | O | H | Gb |
| H | H | $NO_2$ | Me | Me | O | H | Gb |
| H | H | OMe | Me | Me | O | H | Ga |
| H | H | OEt | Me | Me | O | H | Ga |
| H | H | OPr-n | Me | Me | O | H | Gb |
| H | H | SMe | Me | Me | O | H | Ga |
| H | H | SEt | Me | Me | O | H | Ga |
| H | H | $SO_2Me$ | Me | Me | O | H | Ga |
| H | H | COMe | Me | Me | O | H | Ga |
| H | H | COEt | Me | Me | O | H | Ga |
| H | H | $CO_2H$ | Me | Me | O | H | Ga |
| H | H | $CO_2Me$ | Me | Me | O | H | Ga |
| H | H | $CO_2Et$ | Me | Me | O | H | Ga |
| H | H | $CO_2Pr$-n | Me | Me | O | H | Gb |
| H | H | $CO_2Bu$-n | Me | Me | O | H | Gc |
| H | H | $CH_2Cl$ | Me | Me | O | H | Ga |
| H | H | $CH_2Br$ | Me | Me | O | H | Gb |
| H | H | $CH_2I$ | Me | Me | O | H | Gb |
| H | H | $CH_2CF_3$ | Me | Me | O | H | Gb |
| H | H | $CH_2CN$ | Me | Me | O | H | Ga |
| H | H | $CH_2OH$ | Me | Me | O | H | Gb |
| H | H | $CH_2OMe$ | Me | Me | O | H | Ga |
| H | H | $CH_2OEt$ | Me | Me | O | H | Ga |
| H | H | $CH_2OPr$-n | Me | Me | O | H | Gb |
| H | H | $CH_2SMe$ | Me | Me | O | H | Ga |
| H | H | $CH_2SEt$ | Me | Me | O | H | Ga |
| H | H | $CH_2SO_2Me$ | Me | Me | O | H | Ga |
| H | H | $CH_2SO_2Et$ | Me | Me | O | H | Ga |
| H | H | $CH_2SPr$-n | Me | Me | O | H | Gb |
| H | H | $CH_2SPr$-i | Me | Me | O | H | Gb |
| H | H | $CH_2COMe$ | Me | Me | O | H | Ga |
| H | H | $CH_2COEt$ | Me | Me | O | H | Ga |
| H | H | $CH_2COPr$-n | Me | Me | O | H | Gb |
| H | H | $CH_2CO_2H$ | Me | Me | O | H | Ga |
| H | H | $CH_2CO_2Me$ | Me | Me | O | H | Ga |
| H | H | $CH_2CO_2Et$ | Me | Me | O | H | Ga |

| $R_1$ | $R_2$ | $R_3$ | $R_4$ | $R_5$ | X | M | Gn |
|---|---|---|---|---|---|---|---|
| H | H | $CH_2CO_2Pr$-n | Me | Me | O | H | Gb |
| H | H | $CH_2OCH_2CH{=}CH_2$ | Me | Me | O | H | Gb |
| H | H | $CH_2OCH_2C\equiv CH$ | Me | Me | O | H | Gb |
| H | H | $CH{=}CHCO_2Me$ | Me | Me | O | H | Ga |
| H | H | $CH{=}CHCO_2Et$ | Me | Me | O | H | Ga |
| H | H | $CH{=}CHCO_2Pr$-n | Me | Me | O | H | Gb |
| H | H | CH=CHCN | Me | Me | O | H | Ga |
| H | H | Ph | Me | Me | O | H | Ga |
| H | H | $CH_2Ph$ | Me | Me | O | H | Ga |
| H | H | OPh | Me | Me | O | H | Ga |
| H | H | SPh | Me | Me | O | H | Ga |
| H | H | $SO_2Ph$ | Me | Me | O | H | Ga |
| H | H | CONHMe | Me | Me | O | H | Ga |
| H | H | CONHEt | Me | Me | O | H | Ga |
| H | H | $CON(Me)_2$ | Me | Me | O | H | Ga |
| H | H | $CON(Et)_2$ | Me | Me | O | H | Ga |
| H | H | $CON(Pr$-$n)_2$ | Me | Me | O | H | Gb |
| F | H | H | Me | Et | O | H | Gb |
| Cl | H | H | Me | Et | O | H | Gb |
| Br | H | H | Me | Et | O | H | Gb |
| I | H | H | Me | Et | O | H | Gc |
| $CH{=}CH_2$ | H | H | Me | Et | O | H | Gb |
| $CH_2CH{=}CH_2$ | H | H | Me | Et | O | H | Gb |
| $C\equiv CH$ | H | H | Me | Et | O | H | Gb |
| $CH_2C\equiv CH$ | H | H | Me | Et | O | H | Gb |
| $CH_2F$ | H | H | Me | Et | O | H | Gb |
| $CHF_2$ | H | H | Me | Et | O | H | Gb |
| CN | H | H | Me | Et | O | H | Gb |
| $NH_2$ | H | H | Me | Et | O | H | Gb |
| NHCOMe | H | H | Me | Et | O | H | Gb |
| $CH_2NH_2$ | H | H | Me | Et | O | H | Gb |
| $CH_2NHCOMe$ | H | H | Me | Et | O | H | Gb |
| $CH_2OCHF_2$ | H | H | Me | Et | O | H | Gb |
| $CF_3$ | H | H | Me | Et | O | H | Gb |
| $NO_2$ | H | H | Me | Et | O | H | Gb |
| OMe | H | H | Me | Et | O | H | Ga |
| OEt | H | H | Me | Et | O | H | Ga |
| OPr-n | H | H | Me | Et | O | H | Gb |
| SMe | H | H | Me | Et | O | H | Ga |
| SEt | H | H | Me | Et | O | H | Ga |

| $R_1$ | $R_2$ | $R_3$ | $R_4$ | $R_5$ | X | M | Gn |
|---|---|---|---|---|---|---|---|
| $SO_2Me$ | H | H | Me | Et | O | H | Ga |
| COMe | H | H | Me | Et | O | H | Ga |
| COEt | H | H | Me | Et | O | H | Ga |
| $CO_2H$ | H | H | Me | Et | O | H | Ga |
| $CO_2Me$ | H | H | Me | Et | O | H | Ga |
| $CO_2Et$ | H | H | Me | Et | O | H | Ga |
| $CO_2Pr$-n | H | H | Me | Et | O | H | Gb |
| $CO_2Bu$-n | H | H | Me | Et | O | H | Gc |
| $CH_2Cl$ | H | H | Me | Et | O | H | Ga |
| $CH_2Br$ | H | H | Me | Et | O | H | Gb |
| $CH_2I$ | H | H | Me | Et | O | H | Gb |
| $CH_2CF_3$ | H | H | Me | Et | O | H | Gb |
| $CH_2CN$ | H | H | Me | Et | O | H | Ga |
| $CH_2OH$ | H | H | Me | Et | O | H | Gb |
| $CH_2OMe$ | H | H | Me | Et | O | H | Ga |
| $CH_2OEt$ | H | H | Me | Et | O | H | Ga |
| $CH_2OPr$-n | H | H | Me | Et | O | H | Gb |
| $CH_2SMe$ | H | H | Me | Et | O | H | Ga |
| $CH_2SEt$ | H | H | Me | Et | O | H | Ga |
| $CH_2SO_2Me$ | H | H | Me | Et | O | H | Ga |
| $CH_2SO_2Et$ | H | H | Me | Et | O | H | Ga |
| $CH_2SPr$-n | H | H | Me | Et | O | H | Gb |
| $CH_2SPr$-i | H | H | Me | Et | O | H | Gb |
| $CH_2COMe$ | H | H | Me | Et | O | H | Ga |
| $CH_2COEt$ | H | H | Me | Et | O | H | Ga |
| $CH_2COPr$-n | H | H | Me | Et | O | H | Gb |
| $CH_2CO_2H$ | H | H | Me | Et | O | H | Ga |
| $CH_2CO_2Me$ | H | H | Me | Et | O | H | Ga |
| $CH_2CO_2Et$ | H | H | Me | Et | O | H | Ga |
| $CH_2CO_2Pr$-n | H | H | Me | Et | O | H | Gb |
| $CH_2OCH_2CH=CH_2$ | H | H | Me | Et | O | H | Gb |
| $CH_2OCH_2C \equiv CH$ | H | H | Me | Et | O | H | Gb |
| $CH=CHCO_2Me$ | H | H | Me | Et | O | H | Ga |
| $CH=CHCO_2Et$ | H | H | Me | Et | O | H | Ga |
| $CH=CHCO_2Pr$-n | H | H | Me | Et | O | H | Gb |
| CH=CHCN | H | H | Me | Et | O | H | Ga |
| Ph | H | H | Me | Et | O | H | Ga |
| $CH_2Ph$ | H | H | Me | Et | O | H | Ga |
| OPh | H | H | Me | Et | O | H | Ga |
| SPh | H | H | Me | Et | O | H | Ga |

| $R_1$ | $R_2$ | $R_3$ | $R_4$ | $R_5$ | X | M | Gn |
|---|---|---|---|---|---|---|---|
| $SO_2Ph$ | H | H | Me | Et | O | H | Ga |
| CONHMe | H | H | Me | Et | O | H | Ga |
| CONHEt | H | H | Me | Et | O | H | Ga |
| $CON(Me)_2$ | H | H | Me | Et | O | H | Ga |
| $CON(Et)_2$ | H | H | Me | Et | O | H | Ga |
| $CON(Pr\text{-}n)_2$ | H | H | Me | Et | O | H | Gb |
| H | H | H | F | H | O | H | Gb |
| H | H | H | F | H | S | H | G3 |
| H | H | H | Cl | H | O | H | Gb |
| H | H | H | Cl | H | S | H | G3 |
| H | H | H | Br | H | O | H | Gb |
| H | H | H | Br | H | S | H | G3 |
| H | H | H | I | H | O | H | Gc |
| H | H | H | $CH=CH_2$ | H | O | H | Gb |
| H | H | H | $CH=CH_2$ | H | S | H | G3 |
| H | H | H | $CH_2CH=CH_2$ | H | O | H | Gb |
| H | H | H | $C\equiv CH$ | H | O | H | Gb |
| H | H | H | $CH_2C\equiv CH$ | H | O | H | Gb |
| H | H | H | $CH_2F$ | H | O | H | Gb |
| H | H | H | $CH_2F$ | H | S | H | G3 |
| H | H | H | $CHF_2$ | H | O | H | Gb |
| H | H | H | $CHF_2$ | H | S | H | G3 |
| H | H | H | CN | H | O | H | Gb |
| H | H | H | CN | H | S | H | G3 |
| H | H | H | $NH_2$ | H | O | H | Gb |
| H | H | H | NHCOMe | H | O | H | Gb |
| H | H | H | $CH_2NH_2$ | H | O | H | Gb |
| H | H | H | $CH_2NHCOMe$ | H | O | H | Gb |
| H | H | H | $CH_2OCHF_2$ | H | O | H | Gb |
| H | H | H | $CH_2OCHF_2$ | H | S | H | G3 |
| H | H | H | $CF_3$ | H | O | H | Gb |
| H | H | H | $CF_3$ | H | S | H | G3 |
| H | H | H | $CF_3$ | H | O | Me | Gb |
| H | H | H | $CF_3$ | H | O | Et | Gb |
| H | H | H | $CF_3$ | H | O | $CH_2CH=CH_2$ | Gb |
| H | H | H | $CF_3$ | H | O | $CH_2C\equiv CH$ | Gb |
| H | H | H | $NO_2$ | H | O | H | Gb |
| H | H | H | $NO_2$ | H | S | H | G3 |
| H | H | H | OMe | H | O | H | Ga |
| H | H | H | OMe | H | S | H | G3 |

| $R_1$ | $R_2$ | $R_3$ | $R_4$ | $R_5$ | X | M | Gn |
|---|---|---|---|---|---|---|---|
| H | H | H | OMe | H | O | Me | Gb |
| H | H | H | OMe | H | O | Et | Gb |
| H | H | H | OMe | H | O | $CH_2CH=CH_2$ | Gb |
| H | H | H | OMe | H | O | $CH_2C\equiv CH$ | Gb |
| H | H | H | OEt | H | O | H | Ga |
| H | H | H | OEt | H | S | H | G3 |
| H | H | H | OPr-n | H | O | H | Gb |
| H | H | H | OPr-n | H | S | H | G3 |
| H | H | H | SMe | H | O | H | Ga |
| H | H | H | SMe | H | S | H | G3 |
| H | H | H | SEt | H | O | H | Ga |
| H | H | H | SEt | H | S | H | G3 |
| H | H | H | SPr-n | H | O | H | Gb |
| H | H | H | SPr-n | H | S | H | G3 |
| H | H | H | SBu-n | H | O | H | Gc |
| H | H | H | SPen-n | H | O | H | Gc |
| H | H | H | $SO_2Me$ | H | O | H | Ga |
| H | H | H | $SO_2Me$ | H | S | H | G3 |
| H | H | H | $SO_2Et$ | H | O | H | Ga |
| H | H | H | $SO_2Et$ | H | S | H | G3 |
| H | H | H | $SO_2Pr$-n | H | O | H | Gb |
| H | H | H | $SO_2Pr$-n | H | S | H | G3 |
| H | H | H | COMe | H | O | H | Ga |
| H | H | H | COMe | H | S | H | G3 |
| H | H | H | COEt | H | O | H | Ga |
| H | H | H | COEt | H | S | H | G3 |
| H | H | H | COPr-n | H | O | H | Gb |
| H | H | H | COPr-n | H | S | H | G3 |
| H | H | H | $CO_2H$ | H | O | H | Ga |
| H | H | H | $CO_2H$ | H | S | H | G3 |
| H | H | H | $CO_2Me$ | H | O | H | Ga |
| H | H | H | $CO_2Me$ | H | S | H | G3 |
| H | H | H | $CO_2Me$ | H | O | Me | Gb |
| H | H | H | $CO_2Me$ | H | O | Et | Gb |
| H | H | H | $CO_2Me$ | H | O | $CH_2CH=CH_2$ | Gb |
| H | H | H | $CO_2Me$ | H | O | $CH_2C\equiv CH$ | Gb |
| H | H | H | $CO_2Et$ | H | O | H | Ga |
| H | H | H | $CO_2Et$ | H | S | H | G3 |
| H | H | H | $CO_2Pr$-n | H | O | H | Gb |
| H | H | H | $CO_2Pr$-n | H | S | H | G3 |

| $R_1$ | $R_2$ | $R_3$ | $R_4$ | $R_5$ | X | M | Gn |
|---|---|---|---|---|---|---|---|
| H | H | H | $CO_2Pr$-i | H | O | H | Gb |
| H | H | H | $CO_2Pr$-i | H | S | H | G3 |
| H | H | H | $CO_2Bu$-n | H | O | H | Gc |
| H | H | H | $CO_2Bu$-t | H | O | H | Gc |
| H | H | H | $CO_2Bu$-sec | H | O | H | Gc |
| H | H | H | $CO_2Pen$-n | H | O | H | Gc |
| H | H | H | $CH_2Cl$ | H | O | H | Ga |
| H | H | H | $CH_2Cl$ | H | S | H | G3 |
| H | H | H | $CH_2Br$ | H | O | H | Gb |
| H | H | H | $CH_2Br$ | H | S | H | G3 |
| H | H | H | $CH_2I$ | H | O | H | Gb |
| H | H | H | $CH_2I$ | H | S | H | G3 |
| H | H | H | $CH_2CF_3$ | H | O | H | Gb |
| H | H | H | $CH_2CF_3$ | H | S | H | G3 |
| H | H | H | $CH_2CN$ | H | O | H | Ga |
| H | H | H | $CH_2CN$ | H | S | H | G3 |
| H | H | H | $CH_2OH$ | H | O | H | Gb |
| H | H | H | $CH_2OH$ | H | S | H | G3 |
| H | H | H | $CH_2OMe$ | H | O | H | Ga |
| H | H | H | $CH_2OMe$ | H | S | H | G3 |
| H | H | H | $CH_2OMe$ | H | O | Me | Gb |
| H | H | H | $CH_2OMe$ | H | O | Et | Gb |
| H | H | H | $CH_2OMe$ | H | O | $CH_2CH{=}CH_2$ | Gb |
| H | H | H | $CH_2OMe$ | H | O | $CH_2C{\equiv}CH$ | Gb |
| H | H | H | $CH_2OEt$ | H | O | H | Ga |
| H | H | H | $CH_2OEt$ | H | S | H | G3 |
| H | H | H | $CH_2OPr$-n | H | O | H | Gb |
| H | H | H | $CH_2OPr$-n | H | S | H | G3 |
| H | H | H | $CH_2OPr$-i | H | O | H | Gb |
| H | H | H | $CH_2OPr$-i | H | S | H | G3 |
| H | H | H | $CH_2SMe$ | H | O | H | Ga |
| H | H | H | $CH_2SMe$ | H | S | H | G3 |
| H | H | H | $CH_2SEt$ | H | O | H | Ga |
| H | H | H | $CH_2SEt$ | H | S | H | G3 |
| H | H | H | $CH_2SO_2Me$ | H | O | H | Ga |
| H | H | H | $CH_2SO_2Et$ | H | O | H | Ga |
| H | H | H | $CH_2SO_2Me$ | H | S | H | G3 |
| H | H | H | $CH_2SO_2Et$ | H | S | H | G3 |
| H | H | H | $CH_2SPr$-n | H | O | H | Gb |
| H | H | H | $CH_2SPr$-n | H | S | H | G3 |

| $R_1$ | $R_2$ | $R_3$ | $R_4$ | $R_5$ | X | M | Gn |
|---|---|---|---|---|---|---|---|
| H | H | H | $CH_2SPr$-i | H | O | H | Gb |
| H | H | H | $CH_2SPr$-i | H | S | H | G3 |
| H | H | H | $CH_2COMe$ | H | O | H | Ga |
| H | H | H | $CH_2COMe$ | H | S | H | G3 |
| H | H | H | $CH_2COEt$ | H | O | H | Ga |
| H | H | H | $CH_2COEt$ | H | S | H | G3 |
| H | H | H | $CH_2COPr$-n | H | O | H | Gb |
| H | H | H | $CH_2COPr$-n | H | S | H | G3 |
| H | H | H | $CH_2COPr$-i | H | O | H | Gb |
| H | H | H | $CH_2COPr$-i | H | S | H | G3 |
| H | H | H | $CH_2CO_2H$ | H | O | H | Ga |
| H | H | H | $CH_2CO_2H$ | H | S | H | G3 |
| H | H | H | $CH_2CO_2Me$ | H | O | H | Ga |
| H | H | H | $CH_2CO_2Me$ | H | S | H | G3 |
| H | H | H | $CH_2CO_2Et$ | H | O | H | Ga |
| H | H | H | $CH_2CO_2Et$ | H | S | H | G3 |
| H | H | H | $CH_2CO_2Pr$-n | H | O | H | Gb |
| H | H | H | $CH_2CO_2Pr$-n | H | S | H | G3 |
| H | H | H | $CH_2CO_2Pr$-i | H | O | H | Gb |
| H | H | H | $CH_2CO_2Pr$-i | H | S | H | G3 |
| H | H | H | $CH_2OCH_2CH=CH_2$ | H | O | H | Gb |
| H | H | H | $CH_2OCH_2CH=CH_2$ | H | S | H | G3 |
| H | H | H | $CH_2OCH_2C\equiv CH$ | H | O | H | Gb |
| H | H | H | $CH_2OCH_2C\equiv CH$ | H | S | H | G3 |
| H | H | H | $CH=CHCO_2Me$ | H | O | H | Ga |
| H | H | H | $CH=CHCO_2Me$ | H | S | H | G3 |
| H | H | H | $CH=CHCO_2Et$ | H | O | H | Ga |
| H | H | H | $CH=CHCO_2Et$ | H | S | H | G3 |
| H | H | H | $CH=CHCO_2Pr$-n | H | O | H | Gb |
| H | H | H | $CH=CHCO_2Pr$-n | H | S | H | G3 |
| H | H | H | $CH=CHCO_2Pr$-i | H | O | H | Gb |
| H | H | H | $CH=CHCO_2Pr$-i | H | S | H | G3 |
| H | H | H | CH=CHCN | H | O | H | Ga |
| H | H | H | CH=CHCN | H | S | H | G3 |
| H | H | H | Ph | H | O | H | Ga |
| H | H | H | Ph | H | S | H | G3 |
| H | H | H | Ph | H | O | Me | Gb |
| H | H | H | Ph | H | O | Et | Gb |
| H | H | H | Ph | H | O | $CH_2CH=CH_2$ | Gb |
| H | H | H | Ph | H | O | $CH_2C\equiv CH$ | Gb |

| $R_1$ | $R_2$ | $R_3$ | $R_4$ | $R_5$ | X | M | Gn |
|---|---|---|---|---|---|---|---|
| H | H | H | Ph-2-F | H | O | H | Ga |
| H | H | H | Ph-2-F | H | S | H | G3 |
| H | H | H | Ph-3-F | H | O | H | Gb |
| H | H | H | Ph-4-F | H | O | H | Gb |
| H | H | H | Ph-2-Cl | H | O | H | Ga |
| H | H | H | Ph-2-Cl | H | S | H | G3 |
| H | H | H | Ph-3-Cl | H | O | H | Gb |
| H | H | H | Ph-4-Cl | H | O | H | Gb |
| H | H | H | Ph-2-Br | H | O | H | Ga |
| H | H | H | Ph-2-Br | H | S | H | G3 |
| H | H | H | Ph-3-Br | H | O | H | Gb |
| H | H | H | Ph-4-Br | H | O | H | Gb |
| H | H | H | Ph-2-$CF_3$ | H | O | H | Ga |
| H | H | H | Ph-2-$CF_3$ | H | S | H | G3 |
| H | H | H | Ph-3-$CF_3$ | H | O | H | Gb |
| H | H | H | Ph-4-$CF_3$ | H | O | H | Gb |
| H | H | H | Ph-2-Me | H | O | H | Ga |
| H | H | H | Ph-2-Me | H | S | H | G3 |
| H | H | H | Ph-3-Me | H | O | H | Gb |
| H | H | H | Ph-4-Me | H | O | H | Gb |
| H | H | H | Ph-2-Et | H | O | H | Ga |
| H | H | H | Ph-2-Et | H | S | H | G3 |
| H | H | H | Ph-2-OMe | H | O | H | Ga |
| H | H | H | Ph-2-OMe | H | S | H | G3 |
| H | H | H | Ph-3-OMe | H | O | H | Gb |
| H | H | H | Ph-4-OMe | H | O | H | Gb |
| H | H | H | Ph-2-OEt | H | O | H | Ga |
| H | H | H | Ph-2-OEt | H | S | H | G3 |
| H | H | H | Ph-2-$CO_2$Me | H | O | H | Ga |
| H | H | H | Ph-2-$CO_2$Me | H | S | H | G3 |
| H | H | H | Ph-2-$CO_2$Et | H | O | H | Ga |
| H | H | H | Ph-2-$CO_2$Et | H | S | H | G3 |
| H | H | H | Ph-3-$CO_2$Me | H | O | H | Gb |
| H | H | H | Ph-4-$CO_2$Me | H | O | H | Gb |
| H | H | H | $CH_2$Ph | H | O | H | Ga |
| H | H | H | $CH_2$Ph | H | S | H | G3 |
| H | H | H | $CH_2$Ph | H | O | Me | Gb |
| H | H | H | $CH_2$Ph | H | O | Et | Gb |
| H | H | H | $CH_2$Ph | H | O | $CH_2CH=CH_2$ | Gb |
| H | H | H | $CH_2$Ph | H | O | $CH_2C{\equiv}CH$ | Gb |

| $R_1$ | $R_2$ | $R_3$ | $R_4$ | $R_5$ | X | M | Gn |
|---|---|---|---|---|---|---|---|
| H | H | H | $CH_2Ph$-2-F | H | O | H | Ga |
| H | H | H | $CH_2Ph$-2-F | H | S | H | G3 |
| H | H | H | $CH_2Ph$-3-F | H | O | H | Gb |
| H | H | H | $CH_2Ph$-4-F | H | O | H | Gb |
| H | H | H | $CH_2Ph$-2-Cl | H | O | H | Ga |
| H | H | H | $CH_2Ph$-2-Cl | H | S | H | G3 |
| H | H | H | $CH_2Ph$-3-Cl | H | O | H | Gb |
| H | H | H | $CH_2Ph$-4-Cl | H | O | H | Gb |
| H | H | H | $CH_2Ph$-2-Br | H | O | H | Ga |
| H | H | H | $CH_2Ph$-2-Br | H | S | H | G3 |
| H | H | H | $CH_2Ph$-3-Br | H | O | H | Gb |
| H | H | H | $CH_2Ph$-4-Br | H | O | H | Gb |
| H | H | H | $CH_2Ph$-2-$CF_3$ | H | O | H | Ga |
| H | H | H | $CH_2Ph$-2-$CF_3$ | H | S | H | G3 |
| H | H | H | $CH_2Ph$-3-$CF_3$ | H | O | H | Gb |
| H | H | H | $CH_2Ph$-4-$CF_3$ | H | O | H | Gb |
| H | H | H | $CH_2Ph$-2-Me | H | O | H | Ga |
| H | H | H | $CH_2Ph$-2-Me | H | S | H | G3 |
| H | H | H | $CH_2Ph$-3-Me | H | O | H | Gb |
| H | H | H | $CH_2Ph$-4-Me | H | O | H | Gb |
| H | H | H | $CH_2Ph$-2-Et | H | O | H | Ga |
| H | H | H | $CH_2Ph$-2-Et | H | S | H | G3 |
| H | H | H | $CH_2Ph$-2-OMe | H | O | H | Ga |
| H | H | H | $CH_2Ph$-2-OMe | H | S | H | G3 |
| H | H | H | $CH_2Ph$-3-OMe | H | O | H | Gb |
| H | H | H | $CH_2Ph$-4-OMe | H | O | H | Gb |
| H | H | H | $CH_2Ph$-2-OEt | H | O | H | Ga |
| H | H | H | $CH_2Ph$-2-OEt | H | S | H | G3 |
| H | H | H | $CH_2Ph$-2-$CO_2Me$ | H | O | H | Ga |
| H | H | H | $CH_2Ph$-2-$CO_2Me$ | H | S | H | G3 |
| H | H | H | $CH_2Ph$-2-$CO_2Et$ | H | O | H | Ga |
| H | H | H | $CH_2Ph$-2-$CO_2Et$ | H | S | H | G3 |
| H | H | H | $CH_2Ph$-3-$CO_2Me$ | H | O | H | Gb |
| H | H | H | $CH_2Ph$-4-$CO_2Me$ | H | O | H | Gb |
| H | H | H | OPh | H | O | H | Ga |
| H | H | H | OPh | H | S | H | G3 |
| H | H | H | OPh | H | O | Me | Gb |
| H | H | H | OPh | H | O | Et | Gb |
| H | H | H | OPh | H | O | $CH_2CH=CH_2$ | Gb |
| H | H | H | OPh | H | O | $CH_2C\equiv CH$ | Gb |

| $R_1$ | $R_2$ | $R_3$ | $R_4$ | $R_5$ | X | M | Gn |
|---|---|---|---|---|---|---|---|
| H | H | H | OPh-2-Cl | H | O | H | Ga |
| H | H | H | OPh-2-Cl | H | S | H | G3 |
| H | H | H | OPh-3-Cl | H | O | H | Gb |
| H | H | H | OPh-4-Cl | H | O | H | Gb |
| H | H | H | OPh-2-F | H | O | H | Ga |
| H | H | H | OPh-2-F | H | S | H | G3 |
| H | H | H | OPh-3-F | H | O | H | Gb |
| H | H | H | OPh-4-F | H | O | H | Gb |
| H | H | H | OPh-2-$CF_3$ | H | O | H | Ga |
| H | H | H | OPh-2-$CF_3$ | H | S | H | G3 |
| H | H | H | OPh-3-$CF_3$ | H | O | H | Gb |
| H | H | H | OPh-4-$CF_3$ | H | O | H | Gb |
| H | H | H | OPh-2-Me | H | O | H | Ga |
| H | H | H | OPh-2-Me | H | S | H | G3 |
| H | H | H | OPh-3-Me | H | O | H | Gb |
| H | H | H | OPh-4-Me | H | O | H | Gb |
| H | H | H | OPh-2-Et | H | O | H | Ga |
| H | H | H | OPh-2-Et | H | S | H | G3 |
| H | H | H | OPh-2-OMe | H | O | H | Ga |
| H | H | H | OPh-2-OMe | H | S | H | G3 |
| H | H | H | OPh-3-OMe | H | O | H | Gb |
| H | H | H | OPh-4-OMe | H | O | H | Gb |
| H | H | H | OPh-2-OEt | H | O | H | Ga |
| H | H | H | OPh-2-OEt | H | S | H | G3 |
| H | H | H | OPh-2-$CO_2$Me | H | O | H | Ga |
| H | H | H | OPh-2-$CO_2$Me | H | S | H | G3 |
| H | H | H | OPh-3-$CO_2$Me | H | O | H | Gb |
| H | H | H | OPh-4-$CO_2$Me | H | O | H | Gb |
| H | H | H | OPh-2-$CO_2$Et | H | O | H | Ga |
| H | H | H | OPh-2-$CO_2$Et | H | S | H | G3 |
| H | H | H | SPh | H | O | H | Ga |
| H | H | H | SPh | H | S | H | G3 |
| H | H | H | SPh-2-Cl | H | O | H | Ga |
| H | H | H | SPh-2-Cl | H | S | H | G3 |
| H | H | H | SPh-3-Cl | H | O | H | Gb |
| H | H | H | SPh-4-Cl | H | O | H | Gb |
| H | H | H | SPh-2-F | H | O | H | Ga |
| H | H | H | SPh-2-F | H | S | H | G3 |
| H | H | H | SPh-3-F | H | O | H | Gb |
| H | H | H | SPh-4-F | H | O | H | Gb |

| $R_1$ | $R_2$ | $R_3$ | $R_4$ | $R_5$ | X | M | Gn |
|---|---|---|---|---|---|---|---|
| H | H | H | SPh-2-$CF_3$ | H | O | H | Ga |
| H | H | H | SPh-2-$CF_3$ | H | S | H | G3 |
| H | H | H | SPh-3-$CF_3$ | H | O | H | Gb |
| H | H | H | SPh-4-$CF_3$ | H | O | H | Gb |
| H | H | H | SPh-2-Me | H | O | H | Ga |
| H | H | H | SPh-2-Me | H | S | H | G3 |
| H | H | H | SPh-3-Me | H | O | H | Gb |
| H | H | H | SPh-4-Me | H | O | H | Gb |
| H- | H | H | SPh-2-Et | H | O | H | Ga |
| H | H | H | SPh-2-Et | H | S | H | G3 |
| H | H | H | SPh-2-OMe | H | O | H | Ga |
| H | H | H | SPh-2-OMe | H | S | H | G3 |
| H | H | H | SPh-3-OMe | H | O | H | Gb |
| H | H | H | SPh-4-OMe | H | O | H | Gb |
| H | H | H | SPh-2-OEt | H | O | H | Ga |
| H | H | H | SPh-2-OEt | H | S | H | G3 |
| H | H | H | SPh-2-$CO_2$Me | H | O | H | G3 |
| H | H | H | SPh-2-$CO_2$Me | H | S | H | G3 |
| H | H | H | SPh-3-$CO_2$Me | H | O | H | Gb |
| H | H | H | SPh-4-$CO_2$Me | H | O | H | Gb |
| H | H | H | SPh-2-$CO_2$Et | H | O | H | Ga |
| H | H | H | SPh-2-$CO_2$Et | H | S | H | G3 |
| H | H | H | $SO_2$Ph | H | O | H | Ga |
| H | H | H | $SO_2$Ph | H | S | H | G3 |
| H | H | H | $SO_2$Ph-2-Cl | H | O | H | Ga |
| H | H | H | $SO_2$Ph-2-Cl | H | S | H | G3 |
| H | H | H | $SO_2$Ph-3-Cl | H | O | H | Gb |
| H | H | H | $SO_2$Ph-4-Cl | H | O | H | Gb |
| H | H | H | $SO_2$Ph-2-F | H | O | H | Ga |
| H | H | H | $SO_2$Ph-2-F | H | S | H | G3 |
| H | H | H | $SO_2$Ph-3-F | H | O | H | Gb |
| H | H | H | $SO_2$Ph-4-F | H | O | H | Gb |
| H | H | H | $SO_2$Ph-2-$CF_3$ | H | O | H | Ga |
| H | H | H | $SO_2$Ph-2-$CF_3$ | H | S | H | G3 |
| H | H | H | $SO_2$Ph-3-$CF_3$ | H | O | H | Gb |
| H | H | H | $SO_2$Ph-4-$CF_3$ | H | O | H | Gb |
| H | H | H | $SO_2$Ph-2-Me | H | O | H | Ga |
| H | H | H | $SO_2$Ph-2-Me | H | S | H | G3 |
| H | H | H | $SO_2$Ph-3-Me | H | O | H | Gb |
| H | H | H | $SO_2$Ph-4-Me | H | O | H | Gb |

| $R_1$ | $R_2$ | $R_3$ | $R_4$ | $R_5$ | X | M | Gn |
|---|---|---|---|---|---|---|---|
| H | H | H | $SO_2$Ph-2-Et | H | O | H | Ga |
| H | H | H | $SO_2$Ph-2-Et | H | S | H | G3 |
| H | H | H | $SO_2$Ph-2-OMe | H | O | H | Ga |
| H | H | H | $SO_2$Ph-2-OMe | H | S | H | G3 |
| H | H | H | $SO_2$Ph-3-OMe | H | O | H | Gb |
| H | H | H | $SO_2$Ph-4-OMe | H | O | H | Gb |
| H | H | H | $SO_2$Ph-2-OEt | H | O | H | Ga |
| H | H | H | $SO_2$Ph-2-OEt | H | S | H | G3 |
| H | H | H | $SO_2$Ph-2-$CO_2$Me | H | O | H | Ga |
| H | H | H | $SO_2$Ph-2-$CO_2$Me | H | S | H | G3 |
| H | H | H | $SO_2$Ph-3-$CO_2$Me | H | O | H | Gb |
| H | H | H | $SO_2$Ph-4-$CO_2$Me | H | O | H | Gb |
| H | H | H | $SO_2$Ph-2-$CO_2$Et | H | O | H | Ga |
| H | H | H | $SO_2$Ph-2-$CO_2$Et | H | S | H | G3 |
| H | H | H | CONHMe | H | O | H | Ga |
| H | H | H | CONHMe | H | S | H | G3 |
| H | H | H | CONHEt | H | O | H | Ga |
| H | H | H | CONHEt | H | S | H | G3 |
| H | H | H | CONHPr-n | H | O | H | Gb |
| H | H | H | CONHPr-n | H | S | H | G3 |
| H | H | H | CONHPr-i | H | O | H | Gb |
| H | H | H | CONHPr-i | H | S | H | G3 |
| H | H | H | CONHBu-n | H | O | H | Gb |
| H | H | H | CONHBu-sec | H | O | H | Gb |
| H | H | H | CONHBu-t | H | O | H | Gb |
| H | H | H | CON(Me)$_2$ | H | O | H | Ga |
| H | H | H | CON(Me)$_2$ | H | S | H | G3 |
| H | H | H | CON(Et)$_2$ | H | O | H | Ga |
| H | H | H | CON(Et)$_2$ | H | S | H | G3 |
| H | H | H | CON(Pr-n)$_2$ | H | O | H | Gb |
| H | H | H | CON(Pr-n)$_2$ | H | S | H | G3 |
| F | H | H | H | H | O | H | Gb |
| F | H | H | H | H | S | H | G3 |
| Cl | H | H | H | H | O | H | Gb |
| Cl | H | H | H | H | S | H | G3 |
| Br | H | H | H | H | O | H | Gb |
| Br | H | H | H | H | S | H | G3 |
| I | H | H | H | H | O | H | Gc |
| CH=$CH_2$ | H | H | H | H | O | H | Gb |
| CH=$CH_2$ | H | H | H | H | S | H | G3 |

| $R_1$ | $R_2$ | $R_3$ | $R_4$ | $R_5$ | X | M | Gn |
|---|---|---|---|---|---|---|---|
| $CH_2CH=CH_2$ | H | H | H | H | O | H | Gb |
| $C\equiv CH$ | H | H | H | H | O | H | Gb |
| $CH_2C\equiv CH$ | H | H | H | H | O | H | Gb |
| $CH_2F$ | H | H | H | H | O | H | Gb |
| $CH_2F$ | H | H | H | H | S | H | G3 |
| $CHF_2$ | H | H | H | H | O | H | Gb |
| $CHF_2$ | H | H | H | H | S | H | G3 |
| CN | H | H | H | H | O | H | Gb |
| CN | H | H | H | H | S | H | G3 |
| $NH_2$ | H | H | H | H | O | H | Gb |
| NHCOMe | H | H | H | H | O | H | Gb |
| $CH_2NH_2$ | H | H | H | H | O | H | Gb |
| $CH_2NHCOMe$ | H | H | H | H | O | H | Gb |
| $CH_2OCHF_2$ | H | H | H | H | O | H | Gb |
| $CH_2OCHF_2$ | H | H | H | H | S | H | G3 |
| $CF_3$ | H | H | H | H | O | H | Gb |
| $CF_3$ | H | H | H | H | S | H | G3 |
| $CF_3$ | H | H | H | H | O | Me | Gb |
| $CF_3$ | H | H | H | H | O | Et | Gb |
| $CF_3$ | H | H | H | H | O | $CH_2CH=CH_2$ | Gb |
| $CF_3$ | H | H | H | H | O | $CH_2C\equiv CH$ | Gb |
| $NO_2$ | H | H | H | H | O | H | Gb |
| $NO_2$ | H | H | H | H | S | H | G3 |
| OMe | H | H | H | H | O | H | Ga |
| OMe | H | H | H | H | S | H | G3 |
| OMe | H | H | H | H | O | Me | Gb |
| OMe | H | H | H | H | O | Et | Gb |
| OMe | H | H | H | H | O | $CH_2CH=CH_2$ | Gb |
| OMe | H | H | H | H | O | $CH_2C\equiv CH$ | Gb |
| OEt | H | H | H | H | O | H | Ga |
| OEt | H | H | H | H | S | H | G3 |
| OPr-n | H | H | H | H | O | H | Gb |
| OPr-n | H | H | H | H | S | H | G3 |
| SMe | H | H | H | H | O | H | Ga |
| SMe | H | H | H | H | S | H | G3 |
| SEt | H | H | H | H | O | H | Ga |
| SEt | H | H | H | H | S | H | G3 |
| SPr-n | H | H | H | H | O | H | Gb |
| SPr-n | H | H | H | H | S | H | G3 |
| SBu-n | H | H | H | H | O | H | Gc |

| $R_1$ | $R_2$ | $R_3$ | $R_4$ | $R_5$ | X | M | Gn |
|---|---|---|---|---|---|---|---|
| SPen-n | H | H | H | H | O | H | Gc |
| $SO_2Me$ | H | H | H | H | O | H | Ga |
| $SO_2Me$ | H | H | H | H | S | H | G3 |
| $SO_2Et$ | H | H | H | H | O | H | Ga |
| $SO_2Et$ | H | H | H | H | S | H | G3 |
| $SO_2Pr$-n | H | H | H | H | O | H | Gb |
| $SO_2Pr$-n | H | H | H | H | S | H | G3 |
| COMe | H | H | H | H | O | H | Ga |
| COMe | H | H | H | H | S | H | G3 |
| COEt | H | H | H | H | O | H | Ga |
| COEt | H | H | H | H | S | H | G3 |
| COPr-n | H | H | H | H | O | H | Gb |
| COPr-n | H | H | H | H | S | H | G3 |
| $CO_2H$ | H | H | H | H | O | H | Ga |
| $CO_2H$ | H | H | H | H | S | H | G3 |
| $CO_2Me$ | H | H | H | H | O | H | Ga |
| $CO_2Me$ | H | H | H | H | S | H | G3 |
| $CO_2Me$ | H | H | H | H | O | Me | Gb |
| $CO_2Me$ | H | H | H | H | O | Et | Gb |
| $CO_2Me$ | H | H | H | H | O | $CH_2CH=CH_2$ | Gb |
| $CO_2Me$ | H | H | H | H | O | $CH_2C\equiv CH$ | Gb |
| $CO_2Et$ | H | H | H | H | O | H | Ga |
| $CO_2Et$ | H | H | H | H | S | H | G3 |
| $CO_2Pr$-n | H | H | H | H | O | H | Gb |
| $CO_2Pr$-n | H | H | H | H | S | H | G3 |
| $CO_2Pr$-i | H | H | H | H | O | H | Gb |
| $CO_2Pr$-i | H | H | H | H | S | H | G3 |
| $CO_2Bu$-n | H | H | H | H | O | H | Gc |
| $CO_2Bu$-t | H | H | H | H | O | H | Gc |
| $CO_2Bu$-sec | H | H | H | H | O | H | Gc |
| $CO_2Pen$-n | H | H | H | H | O | H | Gc |
| $CH_2Cl$ | H | H | H | H | O | H | Ga |
| $CH_2Cl$ | H | H | H | H | S | H | G3 |
| $CH_2Br$ | H | H | H | H | O | H | Gb |
| $CH_2Br$ | H | H | H | H | S | H | G3 |
| $CH_2I$ | H | H | H | H | O | H | Gb |
| $CH_2I$ | H | H | H | H | S | H | G3 |
| $CH_2CF_3$ | H | H | H | H | O | H | Gb |
| $CH_2CF_3$ | H | H | H | H | S | H | G3 |
| $CH_2CN$ | H | H | H | H | O | H | Ga |

| $R_1$ | $R_2$ | $R_3$ | $R_4$ | $R_5$ | X | M | Gn |
|---|---|---|---|---|---|---|---|
| $CH_2CN$ | H | H | H | H | S | H | G3 |
| $CH_2OH$ | H | H | H | H | O | H | Gb |
| $CH_2OH$ | H | H | H | H | S | H | G3 |
| $CH_2OMe$ | H | H | H | H | O | H | Ga |
| $CH_2OMe$ | H | H | H | H | S | H | G3 |
| $CH_2OMe$ | H | H | H | H | O | Me | Gb |
| $CH_2OMe$ | H | H | H | H | O | Et | Gb |
| $CH_2OMe$ | H | H | H | H | O | $CH_2CH{=}CH_2$ | Gb |
| $CH_2OMe$ | H | H | H | H | O | $CH_2C{\equiv}CH$ | Gb |
| $CH_2OEt$ | H | H | H | H | O | H | Ga |
| $CH_2OEt$ | H | H | H | H | S | H | G3 |
| $CH_2OPr$-n | H | H | H | H | O | H | Gb |
| $CH_2OPr$-n | H | H | H | H | S | H | G3 |
| $CH_2OPr$-i | H | H | H | H | O | H | Gb |
| $CH_2OPr$-i | H | H | H | H | S | H | G3 |
| $CH_2SMe$ | H | H | H | H | O | H | Ga |
| $CH_2SMe$ | H | H | H | H | S | H | G3 |
| $CH_2SEt$ | H | H | H | H | O | H | Ga |
| $CH_2SEt$ | H | H | H | H | S | H | G3 |
| $CH_2SO_2Me$ | H | H | H | H | O | H | Ga |
| $CH_2SO_2Et$ | H | H | H | H | O | H | Ga |
| $CH_2SO_2Me$ | H | H | H | H | S | H | G3 |
| $CH_2SO_2Et$ | H | H | H | H | S | H | G3 |
| $CH_2SPr$-n | H | H | H | H | O | H | Gb |
| $CH_2SPr$-n | H | H | H | H | S | H | G3 |
| $CH_2SPr$-i | H | H | H | H | O | H | Gb |
| $CH_2SPr$-i | H | H | H | H | S | H | G3 |
| $CH_2COMe$ | H | H | H | H | O | H | Ga |
| $CH_2COMe$ | H | H | H | H | S | H | G3 |
| $CH_2COEt$ | H | H | H | H | O | H | Ga |
| $CH_2COEt$ | H | H | H | H | S | H | G3 |
| $CH_2COPr$-n | H | H | H | H | O | H | Gb |
| $CH_2COPr$-n | H | H | H | H | S | H | G3 |
| $CH_2COPr$-i | H | H | H | H | O | H | Gb |
| $CH_2COPr$-i | H | H | H | H | S | H | G3 |
| $CH_2CO_2H$ | H | H | H | H | O | H | Ga |
| $CH_2CO_2H$ | H | H | H | H | S | H | G3 |
| $CH_2CO_2Me$ | H | H | H | H | O | H- | Ga |
| $CH_2CO_2Me$ | H | H | H | H | S | H | G3 |
| $CH_2CO_2Et$ | H | H | H | H | O | H | Ga |

| $R_1$ | $R_2$ | $R_3$ | $R_4$ | $R_5$ | X | M | Gn |
|---|---|---|---|---|---|---|---|
| $CH_2CO_2Et$ | H | H | H | H | S | H | G3 |
| $CH_2CO_2Pr$-n | H | H | H | H | O | H | Gb |
| $CH_2CO_2Pr$-n | H | H | H | H | S | H | G3 |
| $CH_2CO_2Pr$-i | H | H | H | H | O | H | Gb |
| $CH_2CO_2Pr$-i | H | H | H | H | S | H | G3 |
| $CH_2OCH_2CH=CH_2$ | H | H | H | H | O | H | Gb |
| $CH_2OCH_2CH=CH_2$ | H | H | H | H | S | H | G3 |
| $CH_2OCH_2C\equiv CH$ | H | H | H | H | O | H | Gb |
| $CH_2OCH_2C\equiv CH$ | H | H | H | H | S | H | G3 |
| $CH=CHCO_2Me$ | H | H | H | H | O | H | Ga |
| $CH=CHCO_2Me$ | H | H | H | H | S | H | G3 |
| $CH=CHCO_2Et$ | H | H | H | H | O | H | Ga |
| $CH=CHCO_2Et$ | H | H | H | H | S | H | G3 |
| $CH=CHCO_2Pr$-n | H | H | H | H | O | H | Gb |
| $CH=CHCO_2Pr$-n | H | H | H | H | S | H | G3 |
| $CH=CHCO_2Pr$-i | H | H | H | H | O | H | Gb |
| $CH=CHCO_2Pr$-i | H | H | H | H | S | H | G3 |
| CH=CHCN | H | H | H | H | O | H | Ga |
| CH=CHCN | H | H | H | H | S | H | G3 |
| Ph | H | H | H | H | O | H | Ga |
| Ph | H | H | H | H | S | H | G3 |
| Ph | H | H | H | H | O | Me | Gb |
| Ph | H | H | H | H | O | Et | Gb |
| Ph | H | H | H | H | O | $CH_2CH=CH_2$ | Gb |
| Ph | H | H | H | H | O | $CH_2C\equiv CH$ | Gb |
| Ph-2-F | H | H | H | H | O | H | Ga |
| Ph-2-F | H | H | H | H | S | H | G3 |
| Ph-3-F | H | H | H | H | O | H | Gb |
| Ph-4-F | H | H | H | H | O | H | Gb |
| Ph-2-Cl | H | H | H | H | O | H | Ga |
| Ph-2-Cl | H | H | H | H | S | H | G3 |
| Ph-3-Cl | H | H | H | H | O | H | Gb |
| Ph-4-Cl | H | H | H | H | O | H | Gb |
| Ph-2-Br | H | H | H | H | O | H | Ga |
| Ph-2-Br | H | H | H | H | S | H | G3 |
| Ph-3-Br | H | H | H | H | O | H | Gb |
| Ph-4-Br | H | H | H | H | O | H | Gb |
| Ph-2-$CF_3$ | H | H | H | H | O | H | Ga |
| Ph-2-$CF_3$ | H | H | H | H | S | H | G3 |
| Ph-3-$CF_3$ | H | H | H | H | O | H | Gb |

| $R_1$ | $R_2$ | $R_3$ | $R_4$ | $R_5$ | X | M | Gn |
|---|---|---|---|---|---|---|---|
| Ph-4-$CF_3$ | H | H | H | H | O | H | Gb |
| Ph-2-Me | H | H | H | H | O | H | Ga |
| Ph-2-Me | H | H | H | H | S | H | G3 |
| Ph-3-Me | H | H | H | H | O | H | Gb |
| Ph-4-Me | H | H | H | H | O | H | Gb |
| Ph-2-Et | H | H | H | H | O | H | Ga |
| Ph-2-Et | H | H | H | H | S | H | G3 |
| Ph-2-OMe | H | H | H | H | O | H | Ga |
| Ph-2-OMe | H | H | H | H | S | H | G3 |
| Ph-3-OMe | H | H | H | H | O | H | Gb |
| Ph-4-OMe | H | H | H | H | O | H | Gb |
| Ph-2-OEt | H | H | H | H | O | H | Ga |
| Ph-2-OEt | H | H | H | H | S | H | G3 |
| Ph-2-$CO_2$Me | H | H | H | H | O | H | Ga |
| Ph-2-$CO_2$Me | H | H | H | H | S | H | G3 |
| Ph-2-$CO_2$Et | H | H | H | H | O | H | Ga |
| Ph-2-$CO_2$Et | H | H | H | H | S | H | G3 |
| Ph-3-$CO_2$Me | H | H | H | H | O | H | Gb |
| Ph-4-$CO_2$Me | H | H | H | H | O | H | Gb |
| $CH_2$Ph | H | H | H | H | O | H | Ga |
| $CH_2$Ph | H | H | H | H | S | H | G3 |
| $CH_2$Ph | H | H | H | H | O | Me | Gb |
| $CH_2$Ph | H | H | H | H | O | Et | Gb |
| $CH_2$Ph | H | H | H | H | O | $CH_2CH=CH_2$ | Gb |
| $CH_2$Ph | H | H | H | H | O | $CH_2C\equiv CH$ | Gb |
| $CH_2$Ph-2-F | H | H | H | H | O | H | Ga |
| $CH_2$Ph-2-F | H | H | H | H | S | H | G3 |
| $CH_2$Ph-3-F | H | H | H | H | O | H | Gb |
| $CH_2$Ph-4-F | H | H | H | H | O | H | Gb |
| $CH_2$Ph-2-Cl | H | H | H | H | O | H | Ga |
| $CH_2$Ph-2-Cl | H | H | H | H | S | H | G3 |
| $CH_2$Ph-3-Cl | H | H | H | H | O | H | Gb |
| $CH_2$Ph-4-Cl | H | H | H | H | O | H | Gb |
| $CH_2$Ph-2-Br | H | H | H | H | O | H | Ga |
| $CH_2$Ph-2-Br | H | H | H | H | S | H | G3 |
| $CH_2$Ph-3-Br | H | H | H | H | O | H | Gb |
| $CH_2$Ph-4-Br | H | H | H | H | O | H | Gb |
| $CH_2$Ph-2-$CF_3$ | H | H | H | H | O | H | Ga |
| $CH_2$Ph-2-$CF_3$ | H | H | H | H | S | H | G3 |
| $CH_2$Ph-3-$CF_3$ | H | H | H | H | O | H | Gb |

| $R_1$ | $R_2$ | $R_3$ | $R_4$ | $R_5$ | X | M | Gn |
|---|---|---|---|---|---|---|---|
| $CH_2Ph$-4-$CF_3$ | H | H | H | H | O | H | Gb |
| $CH_2Ph$-2-Me | H | H | H | H | O | H | Ga |
| $CH_2Ph$-2-Me | H | H | H | H | S | H | G3 |
| $CH_2Ph$-3-Me | H | H | H | H | O | H | Gb |
| $CH_2Ph$-4-Me | H | H | H | H | O | H | Gb |
| $CH_2Ph$-2-Et | H | H | H | H | O | H | Ga |
| $CH_2Ph$-2-Et | H | H | H | H | S | H | G3 |
| $CH_2Ph$-2-OMe | H | H | H | H | O | H | Ga |
| $CH_2Ph$-2-OMe | H | H | H | H | S | H | G3 |
| $CH_2Ph$-3-OMe | H | H | H | H | O | H | Gb |
| $CH_2Ph$-4-OMe | H | H | H | H | O | H | Gb |
| $CH_2Ph$-2-OEt | H | H | H | H | O | H | Ga |
| $CH_2Ph$-2-OEt | H | H | H | H | S | H | G3 |
| $CH_2Ph$-2-$CO_2Me$ | H | H | H | H | O | H | Ga |
| $CH_2Ph$-2-$CO_2Me$ | H | H | H | H | S | H | G3 |
| $CH_2Ph$-2-$CO_2Et$ | H | H | H | H | O | H | Ga |
| $CH_2Ph$-2-$CO_2Et$ | H | H | H | H | S | H | G3 |
| $CH_2Ph$-3-$CO_2Me$ | H | H | H | H | O | H | Gb |
| $CH_2Ph$-4-$CO_2Me$ | H | H | H | H | O | H | Gb |
| OPh | H | H | H | H | O | H | Ga |
| OPh | H | H | H | H | S | H | G3 |
| OPh | H | H | H | H | O | Me | Gb |
| OPh | H | H | H | H | O | Et | Gb |
| OPh | H | H | H | H | O | $CH_2CH=CH_2$ | Gb |
| OPh | H | H | H | H | O | $CH_2C\equiv CH$ | Gb |
| OPh-2-Cl | H | H | H | H | O | H | Ga |
| OPh-2-Cl | H | H | H | H | S | H | G3 |
| OPh-3-Cl | H | H | H | H | O | H | Gb |
| OPh-4-Cl | H | H | H | H | O | H | Gb |
| OPh-2-F | H | H | H | H | O | H | Ga |
| OPh-2-F | H | H | H | H | S | H | G3 |
| OPh-3-F | H | H | H | H | O | H | Gb |
| OPh-4-F | H | H | H | H | O | H | Gb |
| OPh-2-$CF_3$ | H | H | H | H | O | H | Ga |
| OPh-2-$CF_3$ | H | H | H | H | S | H | G3 |
| OPh-3-$CF_3$ | H | H | H | H | O | H | Gb |
| OPh-4-$CF_3$ | H | H | H | H | O | H | Gb |
| OPh-2-Me | H | H | H | H | O | H | Ga |
| OPh-2-Me | H | H | H | H | S | H | G3 |
| OPh-3-Me | H | H | H | H | O | H | Gb |

| $R_1$ | $R_2$ | $R_3$ | $R_4$ | $R_5$ | X | M | Gn |
|---|---|---|---|---|---|---|---|
| OPh-4-Me | H | H | H | H | O | H | Gb |
| OPh-2-Et | H | H | H | H | O | H | Ga |
| OPh-2-Et | H | H | H | H | S | H | G3 |
| OPh-2-OMe | H | H | H | H | O | H | Ga |
| OPh-2-OMe | H | H | H | H | S | H | G3 |
| OPh-3-OMe | H | H | H | H | O | H | Gb |
| OPh-4-OMe | H | H | H | H | O | H | Gb |
| OPh-2-OEt | H | H | H | H | O | H | Ga |
| OPh-2-OEt | H | H | H | H | S | H | G3 |
| OPh-2-$CO_2$Me | H | H | H | H | O | H | Ga |
| OPh-2-$CO_2$Me | H | H | H | H | S | H | G3 |
| OPh-3-$CO_2$Me | H | H | H | H | O | H | Gb |
| OPh-4-$CO_2$Me | H | H | H | H | O | H | Gb |
| OPh-2-$CO_2$Et | H | H | H | H | O | H | Ga |
| OPh-2-$CO_2$Et | H | H | H | H | S | H | G3 |
| SPh | H | H | H | H | O | H | Ga |
| SPh | H | H | H | H | S | H | G3 |
| SPh-2-Cl | H | H | H | H | O | H | Ga |
| SPh-2-Cl | H | H | H | H | S | H | G3 |
| SPh-3-Cl | H | H | H | H | O | H | Gb |
| SPh-4-Cl | H | H | H | H | O | H | Gb |
| SPh-2-F | H | H | H | H | O | H | Ga |
| SPh-2-F | H | H | H | H | S | H | G3 |
| SPh-3-F | H | H | H | H | O | H | Gb |
| SPh-4-F | H | H | H | H | O | H | Gb |
| SPh-2-$CF_3$ | H | H | H | H | O | H | Ga |
| SPh-2-$CF_3$ | H | H | H | H | S | H | G3 |
| SPh-3-$CF_3$ | H | H | H | H | O | H | Gb |
| SPh-4-$CF_3$ | H | H | H | H | O | H | Gb |
| SPh-2-Me | H | H | H | H | O | H | Ga |
| SPh-2-Me | H | H | H | H | S | H | G3 |
| SPh-3-Me | H | H | H | H | O | H | Gb |
| SPh-4-Me | H | H | H | H | O | H | Gb |
| SPh-2-Et | H | H | H | H | O | H | Ga |
| SPh-2-Et | H | H | H | H | S | H | G3 |
| SPh-2-OMe | H | H | H | H | O | H | Ga |
| SPh-2-OMe | H | H | H | H | S | H | G3 |
| SPh-3-OMe | H | H | H | H | O | H | Gb |
| SPh-4-OMe | H | H | H | H | O | H | Gb |
| SPh-2-OEt | H | H | H | H | O | H | Ga |

| $R_1$ | $R_2$ | $R_3$ | $R_4$ | $R_5$ | X | M | Gn |
|---|---|---|---|---|---|---|---|
| SPh-2-OEt | H | H | H | H | S | H | G3 |
| SPh-2-$CO_2$Me | H | H | H | H | O | H | G3 |
| SPh-2-$CO_2$Me | H | H | H | H | S | H | G3 |
| SPh-3-$CO_2$Me | H | H | H | H | O | H | Gb |
| SPh-4-$CO_2$Me | H | H | H | H | O | H | Gb |
| SPh-2-$CO_2$Et | H | H | H | H | O | H | Ga |
| SPh-2-$CO_2$Et | H | H | H | H | S | H | G3 |
| $SO_2$Ph | H | H | H | H | O | H | Ga |
| $SO_2$Ph | H | H | H | H | S | H | G3 |
| $SO_2$Ph-2-Cl | H | H | H | H | O | H | Ga |
| $SO_2$Ph-2-Cl | H | H | H | H | S | H | G3 |
| $SO_2$Ph-3-Cl | H | H | H | H | O | H | Gb |
| $SO_2$Ph-4-Cl | H | H | H | H | O | H | Gb |
| $SO_2$Ph-2-F | H | H | H | H | O | H | Ga |
| $SO_2$Ph-2-F | H | H | H | H | S | H | G3 |
| $SO_2$Ph-3-F | H | H | H | H | O | H | Gb |
| $SO_2$Ph-4-F | H | H | H | H | O | H | Gb |
| $SO_2$Ph-2-$CF_3$ | H | H | H | H | O | H | Ga |
| $SO_2$Ph-2-$CF_3$ | H | H | H | H | S | H | G3 |
| $SO_2$Ph-3-$CF_3$ | H | H | H | H | O | H | Gb |
| $SO_2$Ph-4-$CF_3$ | H | H | H | H | O | H | Gb |
| $SO_2$Ph-2-Me | H | H | H | H | O | H | Ga |
| $SO_2$Ph-2-Me | H | H | H | H | S | H | G3 |
| $SO_2$Ph-3-Me | H | H | H | H | O | H | Gb |
| $SO_2$Ph-4-Me | H | H | H | H | O | H | Gb |
| $SO_2$Ph-2-Et | H | H | H | H | O | H | Ga |
| $SO_2$Ph-2-Et | H | H | H | H | S | H | G3 |
| $SO_2$Ph-2-OMe | H | H | H | H | O | H | Ga |
| $SO_2$Ph-2-OMe | H | H | H | H | S | H | G3 |
| $SO_2$Ph-3-OMe | H | H | H | H | O | H | Gb |
| $SO_2$Ph-4-OMe | H | H | H | H | O | H | Gb |
| $SO_2$Ph-2-OEt | H | H | H | H | O | H | Ga |
| $SO_2$Ph-2-OEt | H | H | H | H | S | H | G3 |
| $SO_2$Ph-2-$CO_2$Me | H | H | H | H | O | H | Ga |
| $SO_2$Ph-2-$CO_2$Me | H | H | H | H | S | H | G3 |
| $SO_2$Ph-3-$CO_2$Me | H | H | H | H | O | H | Gb |
| $SO_2$Ph-4-$CO_2$Me | H | H | H | H | O | H | Gb |
| $SO_2$Ph-2-$CO_2$Et | H | H | H | H | O | H | Ga |
| $SO_2$Ph-2-$CO_2$Et | H | H | H | H | S | H | G3 |
| CONHMe | H | H | H | H | O | H | Ga |

| $R_1$ | $R_2$ | $R_3$ | $R_4$ | $R_5$ | X | M | Gn |
|---|---|---|---|---|---|---|---|
| CONHMe | H | H | H | H | S | H | G3 |
| CONHEt | H | H | H | H | O | H | Ga |
| CONHEt | H | H | H | H | S | H | G3 |
| CONHPr-n | H | H | H | H | O | H | Gb |
| CONHPr-n | H | H | H | H | S | H | G3 |
| CONHPr-i | H | H | H | H | O | H | Gb |
| CONHPr-i | H | H | H | H | S | H | G3 |
| CONHBu-n | H | H | H | H | O | H | Gb |
| CONHBu-sec | H | H | H | H | O | H | Gb |
| CONHBu-t | H | H | H | H | O | H | Gb |
| $CON(Me)_2$ | H | H | H | H | O | H | Ga |
| $CON(Me)_2$ | H | H | H | H | S | H | G3 |
| $CON(Et)_2$ | H | H | H | H | O | H | Ga |
| $CON(Et)_2$ | H | H | H | H | S | H | G3 |
| $CON(Pr\text{-}n)_2$ | H | H | H | H | O | H | Gb |
| $CON(Pr\text{-}n)_2$ | H | H | H | H | S | H | G3 |
| H | F | H | H | H | O | H | Gb |
| H | F | H | H | H | S | H | G3 |
| H | Cl | H | H | H | O | H | Gb |
| H | Cl | H | H | H | S | H | G3 |
| H | Br | H | H | H | O | H | Gb |
| H | Br | H | H | H | S | H | G3 |
| H | I | H | H | H | O | H | Gc |
| H | $CH=CH_2$ | H | H | H | O | H | Gb |
| H | $CH=CH_2$ | H | H | H | S | H | G3 |
| H | $CH_2CH=CH_2$ | H | H | H | O | H | Gb |
| H | $C\equiv CH$ | H | H | H | O | H | Gb |
| H | $CH_2C\equiv CH$ | H | H | H | O | H | Gb |
| H | $CH_2F$ | H | H | H | O | H | Gb |
| H | $CH_2F$ | H | H | H | S | H | G3 |
| H | $CHF_2$ | H | H | H | O | H | Gb |
| H | $CHF_2$ | H | H | H | S | H | G3 |
| H | CN | H | H | H | O | H | Gb |
| H | CN | H | H | H | S | H | G3 |
| H | $NH_2$ | H | H | H | O | H | Gb |
| H | NHCOMe | H | H | H | O | H | Gb |
| H | $CH_2NH_2$ | H | H | H | O | H | Gb |
| H | $CH_2NHCOMe$ | H | H | H | O | H | Gb |
| H | $CH_2OCHF_2$ | H | H | H | O | H | Gb |
| H | $CH_2OCHF_2$ | H | H | H | S | H | G3 |

| $R_1$ | $R_2$ | $R_3$ | $R_4$ | $R_5$ | X | M | Gn |
|---|---|---|---|---|---|---|---|
| H | $CF_3$ | H | H | H | O | H | Gb |
| H | $CF_3$ | H | H | H | S | H | G3 |
| H | $CF_3$ | H | H | H | O | Me | Gb |
| H | $CF_3$ | H | H | H | O | Et | Gb |
| H | $CF_3$ | H | H | H | O | $CH_2CH=CH_2$ | Gb |
| H | $CF_3$ | H | H | H | O | $CH_2C\equiv CH$ | Gb |
| H | $NO_2$ | H | H | H | O | H | Gb |
| H | $NO_2$ | H | H | H | S | H | G3 |
| H | OMe | H | H | H | O | H | Ga |
| H | OMe | H | H | H | S | H | G3 |
| H | OMe | H | H | H | O | Me | Gb |
| H | OMe | H | H | H | O | Et | Gb |
| H | OMe | H | H | H | O | $CH_2CH=CH_2$ | Gb |
| H | OMe | H | H | H | O | $CH_2C\equiv CH$ | Gb |
| H | OEt | H | H | H | O | H | Ga |
| H | OEt | H | H | H | S | H | G3 |
| H | OPr-n | H | H | H | O | H | Gb |
| H | OPr-n | H | H | H | S | H | G3 |
| H | SMe | H | H | H | O | H | Ga |
| H | SMe | H | H | H | S | H | G3 |
| H | SEt | H | H | H | O | H | Ga |
| H | SEt | H | H | H | S | H | G3 |
| H | SPr-n | H | H | H | O | H | Gb |
| H | SPr-n | H | H | H | S | H | G3 |
| H | SBu-n | H | H | H | O | H | Gc |
| H | SPen-n | H | H | H | O | H | Gc |
| H | $SO_2Me$ | H | H | H | O | H | Ga |
| H | $SO_2Me$ | H | H | H | S | H | G3 |
| H | $SO_2Et$ | H | H | H | O | H | Ga |
| H | $SO_2Et$ | H | H | H | S | H | G3 |
| H | $SO_2Pr$-n | H | H | H | O | H | Gb |
| H | $SO_2Pr$-n | H | H | H | S | H | G3 |
| H | COMe | H | H | H | O | H | Ga |
| H | COMe | H | H | H | S | H | G3 |
| H | COEt | H | H | H | O | H | Ga |
| H | COEt | H | H | H | S | H | G3 |
| H | COPr-n | H | H | H | O | H | Gb |
| H | COPr-n | H | H | H | S | H | G3 |
| H | $CO_2H$ | H | H | H | O | H | Ga |
| H | $CO_2H$ | H | H | H | S | H | G3 |

| $R_1$ | $R_2$ | $R_3$ | $R_4$ | $R_5$ | X | M | Gn |
|---|---|---|---|---|---|---|---|
| H | $CO_2Me$ | H | H | H | O | H | Ga |
| H | $CO_2Me$ | H | H | H | S | H | G3 |
| H | $CO_2Me$ | H | H | H | O | Me | Gb |
| H | $CO_2Me$ | H | H | H | O | Et | Gb |
| H | $CO_2Me$ | H | H | H | O | $CH_2CH=CH_2$ | Gb |
| H | $CO_2Me$ | H | H | H | O | $CH_2C\equiv CH$ | Gb |
| H | $CO_2Et$ | H | H | H | O | H | Ga |
| H | $CO_2Et$ | H | H | H | S | H | G3 |
| H | $CO_2Pr$-n | H | H | H | O | H | Gb |
| H | $CO_2Pr$-n | H | H | H | S | H | G3 |
| H | $CO_2Pr$-i | H | H | H | O | H | Gb |
| H | $CO_2Pr$-i | H | H | H | S | H | G3 |
| H | $CO_2Bu$-n | H | H | H | O | H | Gc |
| H | $CO_2Bu$-t | H | H | H | O | H | Gc |
| H | $CO_2Bu$-sec | H | H | H | O | H | Gc |
| H | $CO_2Pen$-n | H | H | H | O | H | Gc |
| H | $CH_2Cl$ | H | H | H | O | H | Ga |
| H | $CH_2Cl$ | H | H | H | S | H | G3 |
| H | $CH_2Br$ | H | H | H | O | H | Gb |
| H | $CH_2Br$ | H | H | H | S | H | G3 |
| H | $CH_2I$ | H | H | H | O | H | Gb |
| H | $CH_2I$ | H | H | H | S | H | G3 |
| H | $CH_2CF_3$ | H | H | H | O | H | Gb |
| H | $CH_2CF_3$ | H | H | H | S | H | G3 |
| H | $CH_2CN$ | H | H | H | O | H | Ga |
| H | $CH_2CN$ | H | H | H | S | H | G3 |
| H | $CH_2OH$ | H | H | H | O | H | Gb |
| H | $CH_2OH$ | H | H | H | S | H | G3 |
| H | $CH_2OMe$ | H | H | H | O | H | Ga |
| H | $CH_2OMe$ | H | H | H | S | H | G3 |
| H | $CH_2OMe$ | H | H | H | O | Me | Gb |
| H | $CH_2OMe$ | H | H | H | O | Et | Gb |
| H | $CH_2OMe$ | H | H | H | O | $CH_2CH=CH_2$ | Gb |
| H | $CH_2OMe$ | H | H | H | O | $CH_2C\equiv CH$ | Gb |
| H | $CH_2OEt$ | H | H | H | O | H | Ga |
| H | $CH_2OEt$ | H | H | H | S | H | G3 |
| H | $CH_2OPr$-n | H | H | H | O | H | Gb |
| H | $CH_2OPr$-n | H | H | H | S | H | G3 |
| H | $CH_2OPr$-i | H | H | H | O | H | Gb |
| H | $CH_2OPr$-i | H | H | H | S | H | G3 |

| $R_1$ | $R_2$ | $R_3$ | $R_4$ | $R_5$ | X | M | Gn |
|---|---|---|---|---|---|---|---|
| H | $CH_2SMe$ | H | H | H | O | H | Ga |
| H | $CH_2SMe$ | H | H | H | S | H | G3 |
| H | $CH_2SEt$ | H | H | H | O | H | Ga |
| H | $CH_2SEt$ | H | H | H | S | H | G3 |
| H | $CH_2SO_2Me$ | H | H | H | O | H | Ga |
| H | $CH_2SO_2Et$ | H | H | H | O | H | Ga |
| H | $CH_2SO_2Me$ | H | H | H | S | H | G3 |
| H | $CH_2SO_2Et$ | H | H | H | S | H | G3 |
| H | $CH_2SPr$-n | H | H | H | O | H | Gb |
| H | $CH_2SPr$-n | H | H | H | S | H | G3 |
| H | $CH_2SPr$-i | H | H | H | O | H | Gb |
| H | $CH_2SPr$-i | H | H | H | S | H | G3 |
| H | $CH_2COMe$ | H | H | H | O | H | Ga |
| H | $CH_2COMe$ | H | H | H | S | H | G3 |
| H | $CH_2COEt$ | H | H | H | O | H | Ga |
| H | $CH_2COEt$ | H | H | H | S | H | G3 |
| H | $CH_2COPr$-n | H | H | H | O | H | Gb |
| H | $CH_2COPr$-n | H | H | H | S | H | G3 |
| H | $CH_2COPr$-i | H | H | H | O | H | Gb |
| H | $CH_2COPr$-i | H | H | H | S | H | G3 |
| H | $CH_2CO_2H$ | H | H | H | O | H | Ga |
| H | $CH_2CO_2H$ | H | H | H | S | H | G3 |
| H | $CH_2CO_2Me$ | H | H | H | O | H | Ga |
| H | $CH_2CO_2Me$ | H | H | H | S | H | G3 |
| H | $CH_2CO_2Et$ | H | H | H | O | H | Ga |
| H | $CH_2CO_2Et$ | H | H | H | S | H | G3 |
| H | $CH_2CO_2Pr$-n | H | H | H | O | H | Gb |
| H | $CH_2CO_2Pr$-n | H | H | H | S | H | G3 |
| H | $CH_2CO_2Pr$-i | H | H | H | O | H | Gb |
| H | $CH_2CO_2Pr$-i | H | H | H | S | H | G3 |
| H | $CH_2OCH_2CH=CH_2$ | H | H | H | O | H | Gb |
| H | $CH_2OCH_2CH=CH_2$ | H | H | H | S | H | G3 |
| H | $CH_2OCH_2C\equiv CH$ | H | H | H | O | H | Gb |
| H | $CH_2OCH_2C\equiv CH$ | H | H | H | S | H | G3 |
| H | $CH=CHCO_2Me$ | H | H | H | O | H | Ga |
| H | $CH=CHCO_2Me$ | H | H | H | S | H | G3 |
| H | $CH=CHCO_2Et$ | H | H | H | O | H | Ga |
| H | $CH=CHCO_2Et$ | H | H | H | S | H | G3 |
| H | $CH=CHCO_2Pr$-n | H | H | H | O | H | Gb |
| H | $CH=CHCO_2Pr$-n | H | H | H | S | H | G3 |

| $R_1$ | $R_2$ | $R_3$ | $R_4$ | $R_5$ | X | M | Gn |
|---|---|---|---|---|---|---|---|
| H | $CH=CHCO_2Pr$-i | H | H | H | O | H | Gb |
| H | $CH=CHCO_2Pr$-i | H | H | H | S | H | G3 |
| H | CH=CHCN | H | H | H | O | H | Ga |
| H | CH=CHCN | H | H | H | S | H | G3 |
| H | Ph | H | H | H | O | H | Ga |
| H | Ph | H | H | H | S | H | G3 |
| H | Ph | H | H | H | O | Me | Gb |
| H | Ph | H | H | H | O | Et | Gb |
| H | Ph | H | H | H | O | $CH_2CH=CH_2$ | Gb |
| H | Ph | H | H | H | O | $CH_2C\equiv CH$ | Gb |
| H | Ph-2-F | H | H | H | O | H | Ga |
| H | Ph-2-F | H | H | H | S | H | G3 |
| H | Ph-3-F | H | H | H | O | H | Gb |
| H | Ph-4-F | H | H | H | O | H | Gb |
| H | Ph-2-Cl | H | H | H | O | H | Ga |
| H | Ph-2-Cl | H | H | H | S | H | G3 |
| H | Ph-3-Cl | H | H | H | O | H | Gb |
| H | Ph-4-Cl | H | H | H | O | H | Gb |
| H | Ph-2-Br | H | H | H | O | H | Ga |
| H | Ph-2-Br | H | H | H | S | H | G3 |
| H | Ph-3-Br | H | H | H | O | H | Gb |
| H | Ph-4-Br | H | H | H | O | H | Gb |
| H | $Ph-2-CF_3$ | H | H | H | O | H | Ga |
| H | $Ph-2-CF_3$ | H | H | H | S | H | G3 |
| H | $Ph-3-CF_3$ | H | H | H | O | H | Gb |
| H | $Ph-4-CF_3$ | H | H | H | O | H | Gb |
| H | Ph-2-Me | H | H | H | O | H | Ga |
| H | Ph-2-Me | H | H | H | S | H | G3 |
| H | Ph-3-Me | H | H | H | O | H | Gb |
| H | Ph-4-Me | H | H | H | O | H | Gb |
| H | Ph-2-Et | H | H | H | O | H | Ga |
| H | Ph-2-Et | H | H | H | S | H | G3 |
| H | Ph-2-OMe | H | H | H | O | H | Ga |
| H | Ph-2-OMe | H | H | H | S | H | G3 |
| H | Ph-3-OMe | H | H | H | O | H | Gb |
| H | Ph-4-OMe | H | H | H | O | H | Gb |
| H | Ph-2-OEt | H | H | H | O | H | Ga |
| H | Ph-2-OEt | H | H | H | S | H | G3 |
| H | $Ph-2-CO_2Me$ | H | H | H | O | H | Ga |
| H | $Ph-2-CO_2Me$ | H | H | H | S | H | G3 |

| $R_1$ | $R_2$ | $R_3$ | $R_4$ | $R_5$ | X | M | Gn |
|---|---|---|---|---|---|---|---|
| H | Ph-2-$CO_2$Et | H | H | H | O | H | Ga |
| H | Ph-2-$CO_2$Et | H | H | H | S | H | G3 |
| H | Ph-3-$CO_2$Me | H | H | H | O | H | Gb |
| H | Ph-4-$CO_2$Me | H | H | H | O | H | Gb |
| H | $CH_2$Ph | H | H | H | O | H | Ga |
| H | $CH_2$Ph | H | H | H | S | H | G3 |
| H | $CH_2$Ph | H | H | H | O | Me | Gb |
| H | $CH_2$Ph | H | H | H | O | Et | Gb |
| H | $CH_2$Ph | H | H | H | O | $CH_2CH=CH_2$ | Gb |
| H | $CH_2$Ph | H | H | H | O | $CH_2C\equiv CH$ | Gb |
| H | $CH_2$Ph-2-F | H | H | H | O | H | Ga |
| H | $CH_2$Ph-2-F | H | H | H | S | H | G3 |
| H | $CH_2$Ph-3-F | H | H | H | O | H | Gb |
| H | $CH_2$Ph-4-F | H | H | H | O | H | Gb |
| H | $CH_2$Ph-2-Cl | H | H | H | O | H | Ga |
| H | $CH_2$Ph-2-Cl | H | H | H | S | H | G3 |
| H | $CH_2$Ph-3-Cl | H | H | H | O | H | Gb |
| H | $CH_2$Ph-4-Cl | H | H | H | O | H | Gb |
| H | $CH_2$Ph-2-Br | H | H | H | O | H | Ga |
| H | $CH_2$Ph-2-Br | H | H | H | S | H | G3 |
| H | $CH_2$Ph-3-Br | H | H | H | O | H | Gb |
| H | $CH_2$Ph-4-Br | H | H | H | O | H | Gb |
| H | $CH_2$Ph-2-$CF_3$ | H | H | H | O | H | Ga |
| H | $CH_2$Ph-2-$CF_3$ | H | H | H | S | H | G3 |
| H | $CH_2$Ph-3-$CF_3$ | H | H | H | O | H | Gb |
| H | $CH_2$Ph-4-$CF_3$ | H | H | H | O | H | Gb |
| H | $CH_2$Ph-2-Me | H | H | H | O | H | Ga |
| H | $CH_2$Ph-2-Me | H | H | H | S | H | G3 |
| H | $CH_2$Ph-3-Me | H | H | H | O | H | Gb |
| H | $CH_2$Ph-4-Me | H | H | H | O | H | Gb |
| H | $CH_2$Ph-2-Et | H | H | H | O | H | Ga |
| H | $CH_2$Ph-2-Et | H | H | H | S | H | G3 |
| H | $CH_2$Ph-2-OMe | H | H | H | O | H | Ga |
| H | $CH_2$Ph-2-OMe | H | H | H | S | H | G3 |
| H | $CH_2$Ph-3-OMe | H | H | H | O | H | Gb |
| H | $CH_2$Ph-4-OMe | H | H | H | O | H | Gb |
| H | $CH_2$Ph-2-OEt | H | H | H | O | H | Ga |
| H | $CH_2$Ph-2-OEt | H | H | H | S | H | G3 |
| H | $CH_2$Ph-2-$CO_2$Me | H | H | H | O | H | Ga |
| H | $CH_2$Ph-2-$CO_2$Me | H | H | H | S | H | G3 |

| $R_1$ | $R_2$ | $R_3$ | $R_4$ | $R_5$ | X | M | Gn |
|---|---|---|---|---|---|---|---|
| H | $CH_2Ph$-2-$CO_2Et$ | H | H | H | O | H | Ga |
| H | $CH_2Ph$-2-$CO_2Et$ | H | H | H | S | H | G3 |
| H | $CH_2Ph$-3-$CO_2Me$ | H | H | H | O | H | Gb |
| H | $CH_2Ph$-4-$CO_2Me$ | H | H | H | O | H | Gb |
| H | OPh | H | H | H | O | H | Ga |
| H | OPh | H | H | H | S | H | G3 |
| H | OPh | H | H | H | O | Me | Gb |
| H | OPh | H | H | H | O | Et | Gb |
| H | OPh | H | H | H | O | $CH_2CH=CH_2$ | Gb |
| H | OPh | H | H | H | O | $CH_2C\equiv CH$ | Gb |
| H | OPh-2-Cl | H | H | H | O | H | Ga |
| H | OPh-2-Cl | H | H | H | S | H | G3 |
| H | OPh-3-Cl | H | H | H | O | H | Gb |
| H | OPh-4-Cl | H | H | H | O | H | Gb |
| H | OPh-2-F | H | H | H | O | H | Ga |
| H | OPh-2-F | H | H | H | S | H | G3 |
| H | OPh-3-F | H | H | H | O | H | Gb |
| H | OPh-4-F | H | H | H | O | H | Gb |
| H | OPh-2-$CF_3$ | H | H | H | O | H | Ga |
| H | OPh-2-$CF_3$ | H | H | H | S | H | G3 |
| H | OPh-3-$CF_3$ | H | H | H | O | H | Gb |
| H | OPh-4-$CF_3$ | H | H | H | O | H | Gb |
| H | OPh-2-Me | H | H | H | O | H | Ga |
| H | OPh-2-Me | H | H | H | S | H | G3 |
| H | OPh-3-Me | H | H | H | O | H | Gb |
| H | OPh-4-Me | H | H | H | O | H | Gb |
| H | OPh-2-Et | H | H | H | O | H | Ga |
| H | OPh-2-Et | H | H | H | S | H | G3 |
| H | OPh-2-OMe | H | H | H | O | H | Ga |
| H | OPh-2-OMe | H | H | H | S | H | G3 |
| H | OPh-3-OMe | H | H | H | O | H | Gb |
| H | OPh-4-OMe | H | H | H | O | H | Gb |
| H | OPh-2-OEt | H | H | H | O | H | Ga |
| H | OPh-2-OEt | H | H | H | S | H | G3 |
| H | OPh-2-$CO_2Me$ | H | H | H | O | H | Ga |
| H | OPh-2-$CO_2Me$ | H | H | H | S | H | G3 |
| H | OPh-3-$CO_2Me$ | H | H | H | O | H | Gb |
| H | OPh-4-$CO_2Me$ | H | H | H | O | H | Gb |
| H | OPh-2-$CO_2Et$ | H | H | H | O | H | Ga |
| H | OPh-2-$CO_2Et$ | H | H | H | S | H | G3 |

| $R_1$ | $R_2$ | $R_3$ | $R_4$ | $R_5$ | X | M | Gn |
|---|---|---|---|---|---|---|---|
| H | SPh | H | H | H | O | H | Ga |
| H | SPh | H | H | H | S | H | G3 |
| H | SPh-2-Cl | H | H | H | O | H | Ga |
| H | SPh-2-Cl | H | H | H | S | H | G3 |
| H | SPh-3-Cl | H | H | H | O | H | Gb |
| H | SPh-4-Cl | H | H | H | O | H | Gb |
| H | SPh-2-F | H | H | H | O | H | Ga |
| H | SPh-2-F | H | H | H | S | H | G3 |
| H | SPh-3-F | H | H | H | O | H | Gb |
| H | SPh-4-F | H | H | H | O | H | Gb |
| H | SPh-2-$CF_3$ | H | H | H | O | H | Ga |
| H | SPh-2-$CF_3$ | H | H | H | S | H | G3 |
| H | SPh-3-$CF_3$ | H | H | H | O | H | Gb |
| H | SPh-4-$CF_3$ | H | H | H | O | H | Gb |
| H | SPh-2-Me | H | H | H | O | H | Ga |
| H | SPh-2-Me | H | H | H | S | H | G3 |
| H | SPh-3-Me | H | H | H | O | H | Gb |
| H | SPh-4-Me | H | H | H | O | H | Gb |
| H | SPh-2-Et | H | H | H | O | H | Ga |
| H | SPh-2-Et | H | H | H | S | H | G3 |
| H | SPh-2-OMe | H | H | H | O | H | Ga |
| H | SPh-2-OMe | H | H | H | S | H | G3 |
| H | SPh-3-OMe | H | H | H | O | H | Gb |
| H | SPh-4-OMe | H | H | H | O | H | Gb |
| H | SPh-2-OEt | H | H | H | O | H | Ga |
| H | SPh-2-OEt | H | H | H | S | H | G3 |
| H | SPh-2-$CO_2$Me | H | H | H | O | H | G3 |
| H | SPh-2-$CO_2$Me | H | H | H | S | H | G3 |
| H | SPh-3-$CO_2$Me | H | H | H | O | H | Gb |
| H | SPh-4-$CO_2$Me | H | H | H | O | H | Gb |
| H | SPh-2-$CO_2$Et | H | H | H | O | H | Ga |
| H | SPh-2-$CO_2$Et | H | H | H | S | H | G3 |
| H | $SO_2$Ph | H | H | H | O | H | Ga |
| H | $SO_2$Ph | H | H | H | S | H | G3 |
| H | $SO_2$Ph-2-Cl | H | H | H | O | H | Ga |
| H | $SO_2$Ph-2-Cl | H | H | H | S | H | G3 |
| H | $SO_2$Ph-3-Cl | H | H | H | O | H | Gb |
| H | $SO_2$Ph-4-Cl | H | H | H | O | H | Gb |
| H | $SO_2$Ph-2-F | H | H | H | O | H | Ga |
| H | $SO_2$Ph-2-F | H | H | H | S | H | G3 |

| $R_1$ | $R_2$ | $R_3$ | $R_4$ | $R_5$ | X | M | Gn |
|---|---|---|---|---|---|---|---|
| H | $SO_2Ph$-3-F | H | H | H | O | H | Gb |
| H | $SO_2Ph$-4-F | H | H | H | O | H | Gb |
| H | $SO_2Ph$-2-$CF_3$ | H | H | H | O | H | Ga |
| H | $SO_2Ph$-2-$CF_3$ | H | H | H | S | H | G3 |
| H | $SO_2Ph$-3-$CF_3$ | H | H | H | O | H | Gb |
| H | $SO_2Ph$-4-$CF_3$ | H | H | H | O | H | Gb |
| H | $SO_2Ph$-2-Me | H | H | H | O | H | Ga |
| H | $SO_2Ph$-2-Me | H | H | H | S | H | G3 |
| H | $SO_2Ph$-3-Me | H | H | H | O | H | Gb |
| H | $SO_2Ph$-4-Me | H | H | H | O | H | Gb |
| H | $SO_2Ph$-2-Et | H | H | H | O | H | Ga |
| H | $SO_2Ph$-2-Et | H | H | H | S | H | G3 |
| H | $SO_2Ph$-2-OMe | H | H | H | O | H | Ga |
| H | $SO_2Ph$-2-OMe | H | H | H | S | H | G3 |
| H | $SO_2Ph$-3-OMe | H | H | H | O | H | Gb |
| H | $SO_2Ph$-4-OMe | H | H | H | O | H | Gb |
| H | $SO_2Ph$-2-OEt | H | H | H | O | H | Ga |
| H | $SO_2Ph$-2-OEt | H | H | H | S | H | G3 |
| H | $SO_2Ph$-2-$CO_2Me$ | H | H | H | O | H | Ga |
| H | $SO_2Ph$-2-$CO_2Me$ | H | H | H | S | H | G3 |
| H | $SO_2Ph$-3-$CO_2Me$ | H | H | H | O | H | Gb |
| H | $SO_2Ph$-4-$CO_2Me$ | H | H | H | O | H | Gb |
| H | $SO_2Ph$-2-$CO_2Et$ | H | H | H | O | H | Ga |
| H | $SO_2Ph$-2-$CO_2Et$ | H | H | H | S | H | G3 |
| H | CONHMe | H | H | H | O | H | Ga |
| H | CONHMe | H | H | H | S | H | G3 |
| H | CONHEt | H | H | H | O | H | Ga |
| H | CONHEt | H | H | H | S | H | G3 |
| H | CONHPr-n | H | H | H | O | H | Gb |
| H | CONHPr-n | H | H | H | S | H | G3 |
| H | CONHPr-i | H | H | H | O | H | Gb |
| H | CONHPr-i | H | H | H | S | H | G3 |
| H | CONHBu-n | H | H | H | O | H | Gb |
| H | CONHBu-sec | H | H | H | O | H | Gb |
| H | CONHBu-t | H | H | H | O | H | Gb |
| H | CON(Me)$_2$ | H | H | H | O | H | Ga |
| H | CON(Me)$_2$ | H | H | H | S | H | G3 |
| H | CON(Et)$_2$ | H | H | H | O | H | Ga |
| H | CON(Et)$_2$ | H | H | H | S | H | G3 |
| H | CON(Pr-n)$_2$ | H | H | H | O | H | Gb |

| $R_1$ | $R_2$ | $R_3$ | $R_4$ | $R_5$ | X | M | Gn |
|---|---|---|---|---|---|---|---|
| H | $CON(Pr\text{-}n)_2$ | H | H | H | S | H | G3 |
| H | H | F | H | H | O | H | Gb |
| H | H | F | H | H | S | H | G3 |
| H | H | Cl | H | H | O | H | Gb |
| H | H | Cl | H | H | S | H | G3 |
| H | H | Br | H | H | O | H | Gb |
| H | H | Br | H | H | S | H | G3 |
| H | H | I | H | H | O | H | Gc |
| H | H | $CH=CH_2$ | H | H | O | H | Gb |
| H | H | $CH=CH_2$ | H | H | S | H | G3 |
| H | H | $CH_2CH=CH_2$ | H | H | O | H | Gb |
| H | H | $C\equiv CH$ | H | H | O | H | Gb |
| H | H | $CH_2C\equiv CH$ | H | H | O | H | Gb |
| H | H | $CH_2F$ | H | H | O | H | Gb |
| H | H | $CH_2F$ | H | H | S | H | G3 |
| H | H | $CHF_2$ | H | H | O | H | Gb |
| H | H | $CHF_2$ | H | H | S | H | G3 |
| H | H | CN | H | H | O | H | Gb |
| H | H | CN | H | H | S | H | G3 |
| H | H | $NH_2$ | H | H | O | H | Gb |
| H | H | NHCOMe | H | H | O | H | Gb |
| H | H | $CH_2NH_2$ | H | H | O | H | Gb |
| H | H | $CH_2NHCOMe$ | H | H | O | H | Gb |
| H | H | $CH_2OCHF_2$ | H | H | O | H | Gb |
| H | H | $CH_2OCHF_2$ | H | H | S | H | G3 |
| H | H | $CF_3$ | H | H | O | H | Gb |
| H | H | $CF_3$ | H | H | S | H | G3 |
| H | H | $CF_3$ | H | H | O | Me | Gb |
| H | H | $CF_3$ | H | H | O | Et | Gb |
| H | H | $CF_3$ | H | H | O | $CH_2CH=CH_2$ | Gb |
| H | H | $CF_3$ | H | H | O | $CH_2C\equiv CH$ | Gb |
| H | H | $NO_2$ | H | H | O | H | Gb |
| H | H | $NO_2$ | H | H | S | H | G3 |
| H | H | OMe | H | H | O | H | Ga |
| H | H | OMe | H | H | S | H | G3 |
| H | H | OMe | H | H | O | Me | Gb |
| H | H | OMe | H | H | O | Et | Gb |
| H | H | OMe | H | H | O | $CH_2CH=CH_2$ | Gb |
| H | H | OMe | H | H | O | $CH_2C\equiv CH$ | Gb |
| H | H | OEt | H | H | O | H | Ga |

| $R_1$ | $R_2$ | $R_3$ | $R_4$ | $R_5$ | X | M | Gn |
|---|---|---|---|---|---|---|---|
| H | H | OEt | H | H | S | H | G3 |
| H | H | OPr-n | H | H | O | H | Gb |
| H | H | OPr-n | H | H | S | H | G3 |
| H | H | SMe | H | H | O | H | Ga |
| H | H | SMe | H | H | S | H | G3 |
| H | H | SEt | H | H | O | H | Ga |
| H | H | SEt | H | H | S | H | G3 |
| H | H | SPr-n | H | H | O | H | Gb |
| H | H | SPr-n | H | H | S | H | G3 |
| H | H | SBu-n | H | H | O | H | Gc |
| H | H | SPen-n | H | H | O | H | Gc |
| H | H | $SO_2Me$ | H | H | O | H | Ga |
| H | H | $SO_2Me$ | H | H | S | H | G3 |
| H | H | $SO_2Et$ | H | H | O | H | Ga |
| H | H | $SO_2Et$ | H | H | S | H | G3 |
| H | H | $SO_2Pr$-n | H | H | O | H | Gb |
| H | H | $SO_2Pr$-n | H | H | S | H | G3 |
| H | H | COMe | H | H | O | H | Ga |
| H | H | COMe | H | H | S | H | G3 |
| H | H | COEt | H | H | O | H | Ga |
| H | H | COEt | H | H | S | H | G3 |
| H | H | COPr-n | H | H | O | H | Gb |
| H | H | COPr-n | H | H | S | H | G3 |
| H | H | $CO_2H$ | H | H | O | H | Ga |
| H | H | $CO_2H$ | H | H | S | H | G3 |
| H | H | $CO_2Me$ | H | H | O | H | Ga |
| H | H | $CO_2Me$ | H | H | S | H | G3 |
| H | H | $CO_2Me$ | H | H | O | Me | Gb |
| H | H | $CO_2Me$ | H | H | O | Et | Gb |
| H | H | $CO_2Me$ | H | H | O | $CH_2CH=CH_2$ | Gb |
| H | H | $CO_2Me$ | H | H | O | $CH_2C\equiv CH$ | Gb |
| H | H | $CO_2Et$ | H | H | O | H | Ga |
| H | H | $CO_2Et$ | H | H | S | H | G3 |
| H | H | $CO_2Pr$-n | H | H | O | H | Gb |
| H | H | $CO_2Pr$-n | H | H | S | H | G3 |
| H | H | $CO_2Pr$-i | H | H | O | H | Gb |
| H | H | $CO_2Pr$-i | H | H | S | H | G3 |
| H | H | $CO_2Bu$-n | H | H | O | H | Gc |
| H | H | $CO_2Bu$-t | H | H | O | H | Gc |
| H | H | $CO_2Bu$-sec | H | H | O | H | Gc |

| $R_1$ | $R_2$ | $R_3$ | $R_4$ | $R_5$ | X | M | Gn |
|---|---|---|---|---|---|---|---|
| H | H | $CO_2$Pen-n | H | H | O | H | Gc |
| H | H | $CH_2Cl$ | H | H | O | H | Ga |
| H | H | $CH_2Cl$ | H | H | S | H | G3 |
| H | H | $CH_2Br$ | H | H | O | H | Gb |
| H | H | $CH_2Br$ | H | H | S | H | G3 |
| H | H | $CH_2I$ | H | H | O | H | Gb |
| H | H | $CH_2I$ | H | H | S | H | G3 |
| H | H | $CH_2CF_3$ | H | H | O | H | Gb |
| H | H | $CH_2CF_3$ | H | H | S | H | G3 |
| H | H | $CH_2CN$ | H | H | O | H | Ga |
| H | H | $CH_2CN$ | H | H | S | H | G3 |
| H | H | $CH_2OH$ | H | H | O | H | Gb |
| H | H | $CH_2OH$ | H | H | S | H | G3 |
| H | H | $CH_2OMe$ | H | H | O | H | Ga |
| H | H | $CH_2OMe$ | H | H | S | H | G3 |
| H | H | $CH_2OMe$ | H | H | O | Me | Gb |
| H | H | $CH_2OMe$ | H | H | O | Et | Gb |
| H | H | $CH_2OMe$ | H | H | O | $CH_2CH=CH_2$ | Gb |
| H | H | $CH_2OMe$ | H | H | O | $CH_2C\equiv CH$ | Gb |
| H | H | $CH_2OEt$ | H | H | O | H | Ga |
| H | H | $CH_2OEt$ | H | H | S | H | G3 |
| H | H | $CH_2OPr$-n | H | H | O | H | Gb |
| H | H | $CH_2OPr$-n | H | H | S | H | G3 |
| H | H | $CH_2OPr$-i | H | H | O | H | Gb |
| H | H | $CH_2OPr$-i | H | H | S | H | G3 |
| H | H | $CH_2SMe$ | H | H | O | H | Ga |
| H | H | $CH_2SMe$ | H | H | S | H | G3 |
| H | H | $CH_2SEt$ | H | H | O | H | Ga |
| H | H | $CH_2SEt$ | H | H | S | H | G3 |
| H | H | $CH_2SO_2Me$ | H | H | O | H | Ga |
| H | H | $CH_2SO_2Et$ | H | H | O | H | Ga |
| H | H | $CH_2SO_2Me$ | H | H | S | H | G3 |
| H | H | $CH_2SO_2Et$ | H | H | S | H | G3 |
| H | H | $CH_2SPr$-n | H | H | O | H | Gb |
| H | H | $CH_2SPr$-n | H | H | S | H | G3 |
| H | H | $CH_2SPr$-i | H | H | O | H | Gb |
| H | H | $CH_2SPr$-i | H | H | S | H | G3 |
| H | H | $CH_2COMe$ | H | H | O | H | Ga |
| H | H | $CH_2COMe$ | H | H | S | H | G3 |
| H | H | $CH_2COEt$ | H | H | O | H | Ga |

| $R_1$ | $R_2$ | $R_3$ | $R_4$ | $R_5$ | X | M | Gn |
|---|---|---|---|---|---|---|---|
| H | H | $CH_2COEt$ | H | H | S | H | G3 |
| H | H | $CH_2COPr$-n | H | H | O | H | Gb |
| H | H | $CH_2COPr$-n | H | H | S | H | G3 |
| H | H | $CH_2COPr$-i | H | H | O | H | Gb |
| H | H | $CH_2COPr$-i | H | H | S | H | G3 |
| H | H | $CH_2CO_2H$ | H | H | O | H | Ga |
| H | H | $CH_2CO_2H$ | H | H | S | H | G3 |
| H | H | $CH_2CO_2Me$ | H | H | O | H | Ga |
| H | H | $CH_2CO_2Me$ | H | H | S | H | G3 |
| H | H | $CH_2CO_2Et$ | H | H | O | H | Ga |
| H | H | $CH_2CO_2Et$ | H | H | S | H | G3 |
| H | H | $CH_2CO_2Pr$-n | H | H | O | H | Gb |
| H | H | $CH_2CO_2Pr$-n | H | H | S | H | G3 |
| H | H | $CH_2CO_2Pr$-i | H | H | O | H | Gb |
| H | H | $CH_2CO_2Pr$-i | H | H | S | H | G3 |
| H | H | $CH_2OCH_2CH=CH_2$ | H | H | O | H | Gb |
| H | H | $CH_2OCH_2CH=CH_2$ | H | H | S | H | G3 |
| H | H | $CH_2OCH_2C\equiv CH$ | H | H | O | H | Gb |
| H | H | $CH_2OCH_2C\equiv CH$ | H | H | S | H | G3 |
| H | H | $CH=CHCO_2Me$ | H | H | O | H | Ga |
| H | H | $CH=CHCO_2Me$ | H | H | S | H | G3 |
| H | H | $CH=CHCO_2Et$ | H | H | O | H | Ga |
| H | H | $CH=CHCO_2Et$ | H | H | S | H | G3 |
| H | H | $CH=CHCO_2Pr$-n | H | H | O | H | Gb |
| H | H | $CH=CHCO_2Pr$-n | H | H | S | H | G3 |
| H | H | $CH=CHCO_2Pr$-i | H | H | O | H | Gb |
| H | H | $CH=CHCO_2Pr$-i | H | H | S | H | G3 |
| H | H | CH=CHCN | H | H | O | H | Ga |
| H | H | CH=CHCN | H | H | S | H | G3 |
| H | H | Ph | H | H | O | H | Ga |
| H | H | Ph | H | H | S | H | G3 |
| H | H | Ph | H | H | O | Me | Gb |
| H | H | Ph | H | H | O | Et | Gb |
| H | H | Ph | H | H | O | $CH_2CH=CH_2$ | Gb |
| H | H | Ph | H | H | O | $CH_2C\equiv CH$ | Gb |
| H | H | Ph-2-F | H | H | O | H | Ga |
| H | H | Ph-2-F | H | H | S | H | G3 |
| H | H | Ph-3-F | H | H | O | H | Gb |
| H | H | Ph-4-F | H | H | O | H | Gb |
| H | H | Ph-2-Cl | H | H | O | H | Ga |

| $R_1$ | $R_2$ | $R_3$ | $R_4$ | $R_5$ | X | M | Gn |
|---|---|---|---|---|---|---|---|
| H | H | Ph-2-Cl | H | H | S | H | G3 |
| H | H | Ph-3-Cl | H | H | O | H | Gb |
| H | H | Ph-4-Cl | H | H | O | H | Gb |
| H | H | Ph-2-Br | H | H | O | H | Ga |
| H | H | Ph-2-Br | H | H | S | H | G3 |
| H | H | Ph-3-Br | H | H | O | H | Gb |
| H | H | Ph-4-Br | H | H | O | H | Gb |
| H | H | Ph-2-$CF_3$ | H | H | O | H | Ga |
| H | H | Ph-2-$CF_3$ | H | H | S | H | G3 |
| H | H | Ph-3-$CF_3$ | H | H | O | H | Gb |
| H | H | Ph-4-$CF_3$ | H | H | O | H | Gb |
| H | H | Ph-2-Me | H | H | O | H | Ga |
| H | H | Ph-2-Me | H | H | S | H | G3 |
| H | H | Ph-3-Me | H | H | O | H | Gb |
| H | H | Ph-4-Me | H | H | O | H | Gb |
| H | H | Ph-2-Et | H | H | O | H | Ga |
| H | H | Ph-2-Et | H | H | S | H | G3 |
| H | H | Ph-2-OMe | H | H | O | H | Ga |
| H | H | Ph-2-OMe | H | H | S | H | G3 |
| H | H | Ph-3-OMe | H | H | O | H | Gb |
| H | H | Ph-4-OMe | H | H | O | H | Gb |
| H | H | Ph-2-OEt | H | H | O | H | Ga |
| H | H | Ph-2-OEt | H | H | S | H | G3 |
| H | H | Ph-2-$CO_2$Me | H | H | O | H | Ga |
| H | H | Ph-2-$CO_2$Me | H | H | S | H | G3 |
| H | H | Ph-2-$CO_2$Et | H | H | O | H | Ga |
| H | H | Ph-2-$CO_2$Et | H | H | S | H | G3 |
| H | H | Ph-3-$CO_2$Me | H | H | O | H | Gb |
| H | H | Ph-4-$CO_2$Me | H | H | O | H | Gb |
| H | H | $CH_2$Ph | H | H | O | H | Ga |
| H | H | $CH_2$Ph | H | H | S | H | G3 |
| H | H | $CH_2$Ph | H | H | O | Me | Gb |
| H | H | $CH_2$Ph | H | H | O | Et | Gb |
| H | H | $CH_2$Ph | H | H | O | $CH_2CH=CH_2$ | Gb |
| H | H | $CH_2$Ph | H | H | O | $CH_2C \equiv CH$ | Gb |
| H | H | $CH_2$Ph-2-F | H | H | O | H | Ga |
| H | H | $CH_2$Ph-2-F | H | H | S | H | G3 |
| H | H | $CH_2$Ph-3-F | H | H | O | H | Gb |
| H | H | $CH_2$Ph-4-F | H | H | O | H | Gb |
| H | H | $CH_2$Ph-2-Cl | H | H | O | H | Ga |

| $R_1$ | $R_2$ | $R_3$ | $R_4$ | $R_5$ | X | M | Gn |
|---|---|---|---|---|---|---|---|
| H | H | $CH_2Ph$-2-Cl | H | H | S | H | G3 |
| H | H | $CH_2Ph$-3-Cl | H | H | O | H | Gb |
| H | H | $CH_2Ph$-4-Cl | H | H | O | H | Gb |
| H | H | $CH_2Ph$-2-Br | H | H | O | H | Ga |
| H | H | $CH_2Ph$-2-Br | H | H | S | H | G3 |
| H | H | $CH_2Ph$-3-Br | H | H | O | H | Gb |
| H | H | $CH_2Ph$-4-Br | H | H | O | H | Gb |
| H | H | $CH_2Ph$-2-$CF_3$ | H | H | O | H | Ga |
| H | H | $CH_2Ph$-2-$CF_3$ | H | H | S | H | G3 |
| H | H | $CH_2Ph$-3-$CF_3$ | H | H | O | H | Gb |
| H | H | $CH_2Ph$-4-$CF_3$ | H | H | O | H | Gb |
| H | H | $CH_2Ph$-2-Me | H | H | O | H | Ga |
| H | H | $CH_2Ph$-2-Me | H | H | S | H | G3 |
| H | H | $CH_2Ph$-3-Me | H | H | O | H | Gb |
| H | H | $CH_2Ph$-4-Me | H | H | O | H | Gb |
| H | H | $CH_2Ph$-2-Et | H | H | O | H | Ga |
| H | H | $CH_2Ph$-2-Et | H | H | S | H | G3 |
| H | H | $CH_2Ph$-2-OMe | H | H | O | H | Ga |
| H | H | $CH_2Ph$-2-OMe | H | H | S | H | G3 |
| H | H | $CH_2Ph$-3-OMe | H | H | O | H | Gb |
| H | H | $CH_2Ph$-4-OMe | H | H | O | H | Gb |
| H | H | $CH_2Ph$-2-OEt | H | H | O | H | Ga |
| H | H | $CH_2Ph$-2-OEt | H | H | S | H | G3 |
| H | H | $CH_2Ph$-2-$CO_2Me$ | H | H | O | H | Ga |
| H | H | $CH_2Ph$-2-$CO_2Me$ | H | H | S | H | G3 |
| H | H | $CH_2Ph$-2-$CO_2Et$ | H | H | O | H | Ga |
| H | H | $CH_2Ph$-2-$CO_2Et$ | H | H | S | H | G3 |
| H | H | $CH_2Ph$-3-$CO_2Me$ | H | H | O | H | Gb |
| H | H | $CH_2Ph$-4-$CO_2Me$ | H | H | O | H | Gb |
| H | H | OPh | H | H | O | H | Ga |
| H | H | OPh | H | H | S | H | G3 |
| H | H | OPh | H | H | O | Me | Gb |
| H | H | OPh | H | H | O | Et | Gb |
| H | H | OPh | H | H | O | $CH_2CH=CH_2$ | Gb |
| H | H | OPh | H | H | O | $CH_2C\equiv CH$ | Gb |
| H | H | OPh-2-Cl | H | H | O | H | Ga |
| H | H | OPh-2-Cl | H | H | S | H | G3 |
| H | H | OPh-3-Cl | H | H | O | H | Gb |
| H | H | OPh-4-Cl | H | H | O | H | Gb |
| H | H | OPh-2-F | H | H | O | H | Ga |

| $R_1$ | $R_2$ | $R_3$ | $R_4$ | $R_5$ | X | M | Gn |
|---|---|---|---|---|---|---|---|
| H | H | OPh-2-F | H | H | S | H | G3 |
| H | H | OPh-3-F | H | H | O | H | Gb |
| H | H | OPh-4-F | H | H | O | H | Gb |
| H | H | OPh-2-$CF_3$ | H | H | O | H | Ga |
| H | H | OPh-2-$CF_3$ | H | H | S | H | G3 |
| H | H | OPh-3-$CF_3$ | H | H | O | H | Gb |
| H | H | OPh-4-$CF_3$ | H | H | O | H | Gb |
| H | H | OPh-2-Me | H | H | O | H | Ga |
| H | H | OPh-2-Me | H | H | S | H | G3 |
| H | H | OPh-3-Me | H | H | O | H | Gb |
| H | H | OPh-4-Me | H | H | O | H | Gb |
| H | H | OPh-2-Et | H | H | O | H | Ga |
| H | H | OPh-2-Et | H | H | S | H | G3 |
| H | H | OPh-2-OMe | H | H | O | H | Ga |
| H | H | OPh-2-OMe | H | H | S | H | G3 |
| H | H | OPh-3-OMe | H | H | O | H | Gb |
| H | H | OPh-4-OMe | H | H | O | H | Gb |
| H | H | OPh-2-OEt | H | H | O | H | Ga |
| H | H | OPh-2-OEt | H | H | S | H | G3 |
| H | H | OPh-2-$CO_2$Me | H | H | O | H | Ga |
| H | H | OPh-2-$CO_2$Me | H | H | S | H | G3 |
| H | H | OPh-3-$CO_2$Me | H | H | O | H | Gb |
| H | H | OPh-4-$CO_2$Me | H | H | O | H | Gb |
| H | H | OPh-2-$CO_2$Et | H | H | O | H | Ga |
| H | H | OPh-2-$CO_2$Et | H | H | S | H | G3 |
| H | H | SPh | H | H | O | H | Ga |
| H | H | SPh | H | H | S | H | G3 |
| H | H | SPh-2-Cl | H | H | O | H | Ga |
| H | H | SPh-2-Cl | H | H | S | H | G3 |
| H | H | SPh-3-Cl | H | H | O | H | Gb |
| H | H | SPh-4-Cl | H | H | O | H | Gb |
| H | H | SPh-2-F | H | H | O | H | Ga |
| H | H | SPh-2-F | H | H | S | H | G3 |
| H | H | SPh-3-F | H | H | O | H | Gb |
| H | H | SPh-4-F | H | H | O | H | Gb |
| H | H | SPh-2-$CF_3$ | H | H | O | H | Ga |
| H | H | SPh-2-$CF_3$ | H | H | S | H | G3 |
| H | H | SPh-3-$CF_3$ | H | H | O | H | Gb |
| H | H | SPh-4-$CF_3$ | H | H | O | H | Gb |
| H | H | SPh-2-Me | H | H | O | H | Ga |

| $R_1$ | $R_2$ | $R_3$ | $R_4$ | $R_5$ | X | M | Gn |
|---|---|---|---|---|---|---|---|
| H | H | SPh-2-Me | H | H | S | H | G3 |
| H | H | SPh-3-Me | H | H | O | H | Gb |
| H | H | SPh-4-Me | H | H | O | H | Gb |
| H | H | SPh-2-Et | H | H | O | H | Ga |
| H | H | SPh-2-Et | H | H | S | H | G3 |
| H | H | SPh-2-OMe | H | H | O | H | Ga |
| H | H | SPh-2-OMe | H | H | S | H | G3 |
| H | H | SPh-3-OMe | H | H | O | H | Gb |
| H | H | SPh-4-OMe | H | H | O | H | Gb |
| H | H | SPh-2-OEt | H | H | O | H | Ga |
| H | H | SPh-2-OEt | H | H | S | H | G3 |
| H | H | SPh-2-$CO_2$Me | H | H | O | H | G3 |
| H | H | SPh-2-$CO_2$Me | H | H | S | H | G3 |
| H | H | SPh-3-$CO_2$Me | H | H | O | H | Gb |
| H | H | SPh-4-$CO_2$Me | H | H | O | H | Gb |
| H | H | SPh-2-$CO_2$Et | H | H | O | H | Ga |
| H | H | SPh-2-$CO_2$Et | H | H | S | H | G3 |
| H | H | $SO_2$Ph | H | H | O | H | Ga |
| H | H | $SO_2$Ph | H | H | S | H | G3 |
| H | H | $SO_2$Ph-2-Cl | H | H | O | H | Ga |
| H | H | $SO_2$Ph-2-Cl | H | H | S | H | G3 |
| H | H | $SO_2$Ph-3-Cl | H | H | O | H | Gb |
| H | H | $SO_2$Ph-4-Cl | H | H | O | H | Gb |
| H | H | $SO_2$Ph-2-F | H | H | O | H | Ga |
| H | H | $SO_2$Ph-2-F | H | H | S | H | G3 |
| H | H | $SO_2$Ph-3-F | H | H | O | H | Gb |
| H | H | $SO_2$Ph-4-F | H | H | O | H | Gb |
| H | H | $SO_2$Ph-2-$CF_3$ | H | H | O | H | Ga |
| H | H | $SO_2$Ph-2-$CF_3$ | H | H | S | H | G3 |
| H | H | $SO_2$Ph-3-$CF_3$ | H | H | O | H | Gb |
| H | H | $SO_2$Ph-4-$CF_3$ | H | H | O | H | Gb |
| H | H | $SO_2$Ph-2-Me | H | H | O | H | Ga |
| H | H | $SO_2$Ph-2-Me | H | H | S | H | G3 |
| H | H | $SO_2$Ph-3-Me | H | H | O | H | Gb |
| H | H | $SO_2$Ph-4-Me | H | H | O | H | Gb |
| H | H | $SO_2$Ph-2-Et | H | H | O | H | Ga |
| H | H | $SO_2$Ph-2-Et | H | H | S | H | G3 |
| H | H | $SO_2$Ph-2-OMe | H | H | O | H | Ga |
| H | H | $SO_2$Ph-2-OMe | H | H | S | H | G3 |
| H | H | $SO_2$Ph-3-OMe | H | H | O | H | Gb |

| $R_1$ | $R_2$ | $R_3$ | $R_4$ | $R_5$ | X | M | Gn |
|---|---|---|---|---|---|---|---|
| H | H | $SO_2$Ph-4-OMe | H | H | O | H | Gb |
| H | H | $SO_2$Ph-2-OEt | H | H | O | H | Ga |
| H | H | $SO_2$Ph-2-OEt | H | H | S | H | G3 |
| H | H | $SO_2$Ph-2-$CO_2$Me | H | H | O | H | Ga |
| H | H | $SO_2$Ph-2-$CO_2$Me | H | H | S | H | G3 |
| H | H | $SO_2$Ph-3-$CO_2$Me | H | H | O | H | Gb |
| H | H | $SO_2$Ph-4-$CO_2$Me | H | H | O | H | Gb |
| H | H | $SO_2$Ph-2-$CO_2$Et | H | H | O | H | Ga |
| H | H | $SO_2$Ph-2-$CO_2$Et | H | H | S | H | G3 |
| H | H | CONHMe | H | H | O | H | Ga |
| H | H | CONHMe | H | H | S | H | G3 |
| H | H | CONHEt | H | H | O | H | Ga |
| H | H | CONHEt | H | H | S | H | G3 |
| H | H | CONHPr-n | H | H | O | H | Gb |
| H | H | CONHPr-n | H | H | S | H | G3 |
| H | H | CONHPr-i | H | H | O | H | Gb |
| H | H | CONHPr-i | H | H | S | H | G3 |
| H | H | CONHBu-n | H | H | O | H | Gb |
| H | H | CONHBu-sec | H | H | O | H | Gb |
| H | H | CONHBu-t | H | H | O | H | Gb |
| H | H | CON(Me)$_2$ | H | H | O | H | Ga |
| H | H | CON(Me)$_2$ | H | H | S | H | G3 |
| H | H | CON(Et)$_2$ | H | H | O | H | Ga |
| H | H | CON(Et)$_2$ | H | H | S | H | G3 |
| H | H | CON(Pr-n)$_2$ | H | H | O | H | Gb |
| H | H | CON(Pr-n)$_2$ | H | H | S | H | G3 |

| $R_1$ | $R_2$ | $R_3$ | $R_4$ | $R_5$ | X | M | Gn |
|---|---|---|---|---|---|---|---|
| Me | Me | H | H | H | O | H | Ga |
| Me | Me | H | H | H | S | H | G3 |
| Me | H | Me | H | H | O | H | Ga |
| Me | H | Me | H | H | S | H | G3 |
| Me | H | H | Me | H | O | H | Ga |
| Me | H | H | Me | H | S | H | G3 |
| Me | Et | H | H | H | O | H | Gb |
| Me | H | Et | H | H | O | H | Gb |
| Me | H | H | Et | H | O | H | Gb |
| Me | Pr-n | H | H | H | O | H | Gb |
| Me | H | Pr-n | H | H | O | H | Gb |
| Me | H | H | Pr-n | H | O | H | Gb |
| Me | Pr-i | H | H | H | O | H | Gb |
| Me | H | Pr-i | H | H | O | H | Gb |
| Me | H | H | Pr-i | H | O | H | Gb |
| Et | Me | H | H | H | O | H | Ga |
| Et | Me | H | H | H | S | H | G3 |
| Et | H | Me | H | H | O | H | Ga |
| Et | H | Me | H | H | S | H | G3 |
| Et | H | H | Me | H | O | H | Ga |
| Et | H | H | Me | H | S | H | G3 |
| Et | Et | H | H | H | O | H | Gb |
| Et | H | Et | H | H | O | H | Gb |
| Et | H | H | Et | H | O | H | Gb |
| Et | Pr-n | H | H | H | O | H | Gb |
| Et | H | Pr-n | H | H | O | H | Gb |
| Et | H | H | Pr-n | H | O | H | Gb |
| Et | Pr-i | H | H | H | O | H | Gb |
| Et | H | Pr-i | H | H | O | H | Gb |
| Et | H | H | Pr-i | H | O | H | Gb |
| Pr-n | Me | H | H | H | O | H | Ga |
| Pr-n | Me | H | H | H | S | H | G3 |
| Pr-n | H | Me | H | H | O | H | Ga |
| Pr-n | H | Me | H | H | S | H | G3 |
| Pr-n | H | H | Me | H | O | H | Ga |
| Pr-n | H | H | Me | H | S | H | G3 |
| Pr-n | Et | H | H | H | O | H | Gb |
| Pr-n | H | Et | H | H | O | H | Gb |
| Pr-n | H | H | Et | H | O | H | Gb |
| Pr-n | Pr-n | H | H | H | O | H | Gb |

| $R_1$ | $R_2$ | $R_3$ | $R_4$ | $R_5$ | X | M | Gn |
|---|---|---|---|---|---|---|---|
| Pr-n | H | Pr-n | H | H | O | H | Gb |
| Pr-n | H | H | Pr-n | H | O | H | Gb |
| Pr-n | Pr-i | H | H | H | O | H | Gb |
| Pr-n | H | Pr-i | H | H | O | H | Gb |
| Pr-n | H | H | Pr-i | H | O | H | Gb |
| Pr-i | Me | H | H | H | O | H | Ga |
| Pr-i | Me | H | H | H | S | H | G3 |
| Pr-i | H | Me | H | H | O | H | Ga |
| Pr-i | H | Me | H | H | S | H | G3 |
| Pr-i | H | H | Me | H | O | H | Ga |
| Pr-i | H | H | Me | H | S | H | G3 |
| Pr-i | Et | H | H | H | O | H | Gb |
| Pr-i | H | Et | H | H | O | H | Gb |
| Pr-i | H | H | Et | H | O | H | Gb |
| Pr-i | Pr-n | H | H | H | O | H | Gb |
| Pr-i | H | Pr-n | H | H | O | H | Gb |
| Pr-i | H | H | Pr-n | H | O | H | Gb |
| Pr-i | Pr-i | H | H | H | O | H | Gb |
| Pr-i | H | Pr-i | H | H | O | H | Gb |
| Pr-i | H | H | Pr-i | H | O | H | Gb |
| H | Me | Me | H | H | O | H | Ga |
| H | Me | Me | H | H | S | H | G3 |
| H | Me | H | Me | H | O | H | Ga |
| H | Me | H | Me | H | S | H | G3 |
| H | Me | Et | H | H | O | H | Gb |
| H | Me | Et | H | H | S | H | G3 |
| H | Me | H | Et | H | O | H | Gb |
| H | Me | H | Et | H | S | H | G3 |
| H | Me | Pr-n | H | H | O | H | Gb |
| H | Me | Pr-i | H | H | O | H | Gb |
| H | Me | H | Pr-n | H | O | H | Gb |
| H | Me | H | Pr-i | H | O | H | Gb |
| H | Et | Me | H | H | O | H | Ga |
| H | Et | Me | H | H | S | H | G3 |
| H | Et | H | Me | H | O | H | Ga |
| H | Et | H | Me | H | S | H | G3 |
| H | Et | Et | H | H | O | H | Gb |
| H | Et | Et | H | H | S | H | G3 |
| H | Et | H | Et | H | O | H | Gb |
| H | Et | H | Et | H | S | H | G3 |

| $R_1$ | $R_2$ | $R_3$ | $R_4$ | $R_5$ | X | M | Gn |
|---|---|---|---|---|---|---|---|
| H | Et | Pr-n | H | H | O | H | Gb |
| H | Et | Pr-i | H | H | O | H | Gb |
| H | Et | H | Pr-n | H | O | H | Gb |
| H | Et | H | Pr-i | H | O | H | Gb |
| H | Pr-n | Me | H | H | O | H | Ga |
| H | Pr-n | Me | H | H | S | H | G3 |
| H | Pr-n | H | Me | H | O | H | Ga |
| H | Pr-n | H | Me | H | S | H | G3 |
| H | Pr-n | Et | H | H | O | H | Gb |
| H | Pr-n | Et | H | H | S | H | G3 |
| H | Pr-n | H | Et | H | O | H | Gb |
| H | Pr-n | H | Et | H | S | H | G3 |
| H | Pr-n | Pr-n | H | H | O | H | Gb |
| H | Pr-n | Pr-i | H | H | O | H | Gb |
| H | Pr-n | H | Pr-n | H | O | H | Gb |
| H | Pr-n | H | Pr-i | H | O | H | Gb |
| H | Pr-i | Me | H | H | O | H | Ga |
| H | Pr-i | Me | H | H | S | H | G3 |
| H | Pr-i | H | Me | H | O | H | Ga |
| H | Pr-i | H | Me | H | S | H | G3 |
| H | Pr-i | Et | H | H | O | H | Gb |
| H | Pr-i | Et | H | H | S | H | G3 |
| H | Pr-i | H | Et | H | O | H | Gb |
| H | Pr-i | H | Et | H | S | H | G3 |
| H | Pr-i | Pr-n | H | H | O | H | Gb |
| H | Pr-i | Pr-i | H | H | O | H | Gb |
| H | Pr-i | H | Pr-n | H | O | H | Gb |
| H | Pr-i | H | Pr-i | H | O | H | Gb |
| H | H | Me | Me | H | O | H | Ga |
| H | H | Me | Me | H | S | H | G3 |
| H | H | Me | Et | H | O | H | Gb |
| H | H | Me | Pr-n | H | O | H | Gb |
| H | H | Me | Pr-i | H | O | H | Gb |
| H | H | Et | Me | H | O | H | Ga |
| H | H | Et | Me | H | S | H | G3 |
| H | H | Et | Et | H | O | H | Gb |
| H | H | Et | Pr-n | H | O | H | Gb |
| H | H | Et | Pr-i | H | O | H | Gb |
| H | H | Pr-n | Me | H | O | H | Ga |
| H | H | Pr-n | Me | H | S | H | G3 |

| $R_1$ | $R_2$ | $R_3$ | $R_4$ | $R_5$ | X | M | Gn |
|---|---|---|---|---|---|---|---|
| H | H | Pr-n | Et | H | O | H | Gb |
| H | H | Pr-n | Pr-n | H | O | H | Gb |
| H | H | Pr-n | Pr-i | H | O | H | Gb |
| H | H | Pr-i | Me | H | O | H | Ga |
| H | H | Pr-i | Me | H | S | H | G3 |
| H | H | Pr-i | Et | H | O | H | Gb |
| H | H | Pr-i | Pr-n | H | O | H | Gb |
| H | H | Pr-i | Pr-i | H | O | H | Gb |
| Me | Me | Me | H | H | O | H | Gb |
| Me | Me | Et | H | H | O | H | Gb |
| Me | Me | Pr-n | H | H | O | H | Gc |
| Me | Me | Pr-i | H | H | O | H | Gc |
| Me | Et | Me | H | H | O | H | Gb |
| Me | Et | Et | H | H | O | H | Gb |
| Me | Et | Pr-n | H | H | O | H | Gc |
| Me | Et | Pr-i | H | H | O | H | Gc |
| Me | Pr-n | Me | H | H | O | H | Gb |
| Me | Pr-n | Et | H | H | O | H | Gb |
| Me | Pr-n | Pr-n | H | H | O | H | Gc |
| Me | Pr-n | Pr-i | H | H | O | H | Gc |
| Me | Pr-i | Me | H | H | O | H | Gb |
| Me | Pr-i | Et | H | H | O | H | Gb |
| Me | Pr-i | Pr-n | H | H | O | H | Gc |
| Me | Pr-i | Pr-i | H | H | O | H | Gc |
| Et | Me | Me | H | H | O | H | Gb |
| Et | Me | Et | H | H | O | H | Gb |
| Et | Me | Pr-n | H | H | O | H | Gc |
| Et | Me | Pr-i | H | H | O | H | Gc |
| Et | Et | Me | H | H | O | H | Gb |
| Et | Et | Et | H | H | O | H | Gb |
| Et | Et | Pr-n | H | H | O | H | Gc |
| Et | Et | Pr-i | H | H | O | H | Gc |
| Et | Pr-n | Me | H | H | O | H | Gb |
| Et | Pr-n | Et | H | H | O | H | Gb |
| Et | Pr-n | Pr-n | H | H | O | H | Gc |
| Et | Pr-n | Pr-i | H | H | O | H | Gc |
| Et | Pr-i | Me | H | H | O | H | Gb |
| Et | Pr-i | Et | H | H | O | H | Gb |
| Et | Pr-i | Pr-n | H | H | O | H | Gc |
| Et | Pr-i | Pr-i | H | H | O | H | Gc |

| $R_1$ | $R_2$ | $R_3$ | $R_4$ | $R_5$ | X | M | Gn |
|---|---|---|---|---|---|---|---|
| Pr-n | Me | Me | H | H | O | H | Gb |
| Pr-n | Me | Et | H | H | O | H | Gb |
| Pr-n | Me | Pr-n | H | H | O | H | Gc |
| Pr-n | Me | Pr-i | H | H | O | H | Gc |
| Pr-n | Et | Me | H | H | O | H | Gb |
| Pr-n | Et | Et | H | H | O | H | Gb |
| Pr-n | Et | Pr-n | H | H | O | H | Gc |
| Pr-n | Et | Pr-i | H | H | O | H | Gc |
| Pr-n | Pr-n | Me | H | H | O | H | Gb |
| Pr-n | Pr-n | Et | H | H | O | H | Gb |
| Pr-n | Pr-n | Pr-n | H | H | O | H | Gc |
| Pr-n | Pr-n | Pr-i | H | H | O | H | Gc |
| Pr-n | Pr-i | Me | H | H | O | H | Gb |
| Pr-n | Pr-i | Et | H | H | O | H | Gb |
| Pr-n | Pr-i | Pr-n | H | H | O | H | Gc |
| Pr-n | Pr-i | Pr-i | H | H | O | H | Gc |
| Pr-i | Me | Me | H | H | O | H | Gb |
| Pr-i | Me | Et | H | H | O | H | Gb |
| Pr-i | Me | Pr-n | H | H | O | H | Gc |
| Pr-i | Me | Pr-i | H | H | O | H | Gc |
| Pr-i | Et | Me | H | H | O | H | Gb |
| Pr-i | Et | Et | H | H | O | H | Gb |
| Pr-i | Et | Pr-n | H | H | O | H | Gc |
| Pr-i | Et | Pr-i | H | H | O | H | Gc |
| Pr-i | Pr-n | Me | H | H | O | H | Gb |
| Pr-i | Pr-n | Et | H | H | O | H | Gb |
| Pr-i | Pr-n | Pr-n | H | H | O | H | Gc |
| Pr-i | Pr-n | Pr-i | H | H | O | H | Gc |
| Pr-i | Pr-i | Me | H | H | O | H | Gb |
| Pr-i | Pr-i | Et | H | H | O | H | Gb |
| Pr-i | Pr-i | Pr-n | H | H | O | H | Gc |
| Pr-i | Pr-i | Pr-i | H | H | O | H | Gc |
| Me | H | Me | Me | H | O | H | Gb |
| Me | H | Me | Et | H | O | H | Gb |
| Me | H | Me | Pr-n | H | O | H | Gc |
| Me | H | Me | Pr-i | H | O | H | Gc |
| Me | H | Et | Me | H | O | H | Gb |
| Me | H | Et | Et | H | O | H | Gb |
| Me | H | Et | Pr-n | H | O | H | Gc |
| Me | H | Et | Pr-i | H | O | H | Gc |

| $R_1$ | $R_2$ | $R_3$ | $R_4$ | $R_5$ | X | M | Gn |
|---|---|---|---|---|---|---|---|
| Me | H | Pr-n | Me | H | O | H | Gb |
| Me | H | Pr-n | Et | H | O | H | Gb |
| Me | H | Pr-n | Pr-n | H | O | H | Gc |
| Me | H | Pr-n | Pr-i | H | O | H | Gc |
| Me | H | Pr-i | Me | H | O | H | Gb |
| Me | H | Pr-i | Et | H | O | H | Gb |
| Me | H | Pr-i | Pr-n | H | O | H | Gc |
| Me | H | Pr-i | Pr-i | H | O | H | Gc |
| Et | H | Me | Me | H | O | H | Gb |
| Et | H | Me | Et | H | O | H | Gb |
| Et | H | Me | Pr-n | H | O | H | Gc |
| Et | H | Me | Pr-i | H | O | H | Gc |
| Et | H | Et | Me | H | O | H | Gb |
| Et | H | Et | Et | H | O | H | Gb |
| Et | H | Et | Pr-n | H | O | H | Gc |
| Et | H | Et | Pr-i | H | O | H | Gc |
| Et | H | Pr-n | Me | H | O | H | Gb |
| Et | H | Pr-n | Et | H | O | H | Gb |
| Et | H | Pr-n | Pr-n | H | O | H | Gc |
| Et | H | Pr-n | Pr-i | H | O | H | Gc |
| Et | H | Pr-i | Me | H | O | H | Gb |
| Et | H | Pr-i | Et | H | O | H | Gb |
| Et | H | Pr-i | Pr-n | H | O | H | Gc |
| Et | H | Pr-i | Pr-i | H | O | H | Gc |
| Pr-n | H | Me | Me | H | O | H | Gb |
| Pr-n | H | Me | Et | H | O | H | Gb |
| Pr-n | H | Me | Pr-n | H | O | H | Gc |
| Pr-n | H | Me | Pr-i | H | O | H | Gc |
| Pr-n | H | Et | Me | H | O | H | Gb |
| Pr-n | H | Et | Et | H | O | H | Gb |
| Pr-n | H | Et | Pr-n | H | O | H | Gc |
| Pr-n | H | Et | Pr-i | H | O | H | Gc |
| Pr-n | H | Pr-n | Me | H | O | H | Gb |
| Pr-n | H | Pr-n | Et | H | O | H | Gb |
| Pr-n | H | Pr-n | Pr-n | H | O | H | Gc |
| Pr-n | H | Pr-n | Pr-i | H | O | H | Gc |
| Pr-n | H | Pr-i | Me | H | O | H | Gb |
| Pr-n | H | Pr-i | Et | H | O | H | Gb |
| Pr-n | H | Pr-i | Pr-n | H | O | H | Gc |
| Pr-n | H | Pr-i | Pr-i | H | O | H | Gc |

| $R_1$ | $R_2$ | $R_3$ | $R_4$ | $R_5$ | X | M | Gn |
|---|---|---|---|---|---|---|---|
| Pr-i | H | Me | Me | H | O | H | Gb |
| Pr-i | H | Me | Et | H | O | H | Gb |
| Pr-i | H | Me | Pr-n | H | O | H | Gc |
| Pr-i | H | Me | Pr-i | H | O | H | Gc |
| Pr-i | H | Et | Me | H | O | H | Gb |
| Pr-i | H | Et | Et | H | O | H | Gb |
| Pr-i | H | Et | Pr-n | H | O | H | Gc |
| Pr-i | H | Et | Pr-i | H | O | H | Gc |
| Pr-i | H | Pr-n | Me | H | O | H | Gb |
| Pr-i | H | Pr-n | Et | H | O | H | Gb |
| Pr-i | H | Pr-n | Pr-n | H | O | H | Gc |
| Pr-i | H | Pr-n | Pr-i | H | O | H | Gc |
| Pr-i | H | Pr-i | Me | H | O | H | Gb |
| Pr-i | H | Pr-i | Et | H | O | H | Gb |
| Pr-i | H | Pr-i | Pr-n | H | O | H | Gc |
| Pr-i | H | Pr-i | Pr-i | H | O | H | Gc |
| Me | Me | H | Me | H | O | H | Gb |
| Me | Me | H | Et | H | O | H | Gb |
| Me | Me | H | Pr-n | H | O | H | Gc |
| Me | Me | H | Pr-i | H | O | H | Gc |
| Me | Et | H | Me | H | O | H | Gb |
| Me | Et | H | Et | H | O | H | Gb |
| Me | Et | H | Pr-n | H | O | H | Gc |
| Me | Et | H | Pr-i | H | O | H | Gc |
| Me | Pr-n | H | Me | H | O | H | Gb |
| Me | Pr-n | H | Et | H | O | H | Gb |
| Me | Pr-n | H | Pr-n | H | O | H | Gc |
| Me | Pr-n | H | Pr-i | H | O | H | Gc |
| Me | Pr-i | H | Me | H | O | H | Gb |
| Me | Pr-i | H | Et | H | O | H | Gb |
| Me | Pr-i | H | Pr-n | H | O | H | Gc |
| Me | Pr-i | H | Pr-i | H | O | H | Gc |
| Et | Me | H | Me | H | O | H | Gb |
| Et | Me | H | Et | H | O | H | Gb |
| Et | Me | H | Pr-n | H | O | H | Gc |
| Et | Me | H | Pr-i | H | O | H | Gc |
| Et | Et | H | Me | H | O | H | Gb |
| Et | Et | H | Et | H | O | H | Gb |
| Et | Et | H | Pr-n | H | O | H | Gc |
| Et | Et | H | Pr-i | H | O | H | Gc |

| $R_1$ | $R_2$ | $R_3$ | $R_4$ | $R_5$ | X | M | Gn |
|---|---|---|---|---|---|---|---|
| Et | Pr-n | H | Me | H | O | H | Gb |
| Et | Pr-n | H | Et | H | O | H | Gb |
| Et | Pr-n | H | Pr-n | H | O | H | Gc |
| Et | Pr-n | H | Pr-i | H | O | H | Gc |
| Et | Pr-i | H | Me | H | O | H | Gb |
| Et | Pr-i | H | Et | H | O | H | Gb |
| Et | Pr-i | H | Pr-n | H | O | H | Gc |
| Et | Pr-i | H | Pr-i | H | O | H | Gc |
| Pr-n | Me | H | Me | H | O | H | Gb |
| Pr-n | Me | H | Et | H | O | H | Gb |
| Pr-n | Me | H | Pr-n | H | O | H | Gc |
| Pr-n | Me | H | Pr-i | H | O | H | Gc |
| Pr-n | Et | H | Me | H | O | H | Gb |
| Pr-n | Et | H | Et | H | O | H | Gb |
| Pr-n | Et | H | Pr-n | H | O | H | Gc |
| Pr-n | Et | H | Pr-i | H | O | H | Gc |
| Pr-n | Pr-n | H | Me | H | O | H | Gb |
| Pr-n | Pr-n | H | Et | H | O | H | Gb |
| Pr-n | Pr-n | H | Pr-n | H | O | H | Gc |
| Pr-n | Pr-n | H | Pr-i | H | O | H | Gc |
| Pr-n | Pr-i | H | Me | H | O | H | Gb |
| Pr-n | Pr-i | H | Et | H | O | H | Gb |
| Pr-n | Pr-i | H | Pr-n | H | O | H | Gc |
| Pr-n | Pr-i | H | Pr-i | H | O | H | Gc |
| Pr-i | Me | H | Me | H | O | H | Gb |
| Pr-i | Me | H | Et | H | O | H | Gb |
| Pr-i | Me | H | Pr-n | H | O | H | Gc |
| Pr-i | Me | H | Pr-i | H | O | H | Gc |
| Pr-i | Et | H | Me | H | O | H | Gb |
| Pr-i | Et | H | Et | H | O | H | Gb |
| Pr-i | Et | H | Pr-n | H | O | H | Gc |
| Pr-i | Et | H | Pr-i | H | O | H | Gc |
| Pr-i | Pr-n | H | Me | H | O | H | Gb |
| Pr-i | Pr-n | H | Et | H | O | H | Gb |
| Pr-i | Pr-n | H | Pr-n | H | O | H | Gc |
| Pr-i | Pr-n | H | Pr-i | H | O | H | Gc |
| Pr-i | Pr-i | H | Me | H | O | H | Gb |
| Pr-i | Pr-i | H | Et | H | O | H | Gb |
| Pr-i | Pr-i | H | Pr-n | H | O | H | Gc |
| Pr-i | Pr-i | H | Pr-i | H | O | H | Gc |

| $R_1$ | $R_2$ | $R_3$ | $R_4$ | $R_5$ | X | M | Gn |
|---|---|---|---|---|---|---|---|
| H | Me | Me | Me | H | O | H | Gb |
| H | Me | Me | Et | H | O | H | Gb |
| H | Me | Me | Pr-n | H | O | H | Gc |
| H | Me | Me | Pr-i | H | O | H | Gc |
| H | Me | Et | Me | H | O | H | Gb |
| H | Me | Et | Et | H | O | H | Gb |
| H | Me | Et | Pr-n | H | O | H | Gc |
| H | Me | Et | Pr-i | H | O | H | Gc |
| H | Me | Pr-n | Me | H | O | H | Gb |
| H | Me | Pr-n | Et | H | O | H | Gb |
| H | Me | Pr-n | Pr-n | H | O | H | Gc |
| H | Me | Pr-n | Pr-i | H | O | H | Gc |
| H | Me | Pr-i | Me | H | O | H | Gb |
| H | Me | Pr-i | Et | H | O | H | Gb |
| H | Me | Pr-i | Pr-n | H | O | H | Gc |
| H | Me | Pr-i | Pr-i | H | O | H | Gc |
| H | Et | Me | Me | H | O | H | Gb |
| H | Et | Me | Et | H | O | H | Gb |
| H | Et | Me | Pr-n | H | O | H | Gc |
| H | Et | Me | Pr-i | H | O | H | Gc |
| H | Et | Et | Me | H | O | H | Gb |
| H | Et | Et | Et | H | O | H | Gb |
| H | Et | Et | Pr-n | H | O | H | Gc |
| H | Et | Et | Pr-i | H | O | H | Gc |
| H | Et | Pr-n | Me | H | O | H | Gb |
| H | Et | Pr-n | Et | H | O | H | Gb |
| H | Et | Pr-n | Pr-n | H | O | H | Gc |
| H | Et | Pr-n | Pr-i | H | O | H | Gc |
| H | Et | Pr-i | Me | H | O | H | Gb |
| H | Et | Pr-i | Et | H | O | H | Gb |
| H | Et | Pr-i | Pr-n | H | O | H | Gc |
| H | Et | Pr-i | Pr-i | H | O | H | Gc |
| H | Pr-n | Me | Me | H | O | H | Gb |
| H | Pr-n | Me | Et | H | O | H | Gb |
| H | Pr-n | Me | Pr-n | H | O | H | Gc |
| H | Pr-n | Me | Pr-i | H | O | H | Gc |
| H | Pr-n | Et | Me | H | O | H | Gb |
| H | Pr-n | Et | Et | H | O | H | Gb |
| H | Pr-n | Et | Pr-n | H | O | H | Gc |
| H | Pr-n | Et | Pr-i | H | O | H | Gc |

| $R_1$ | $R_2$ | $R_3$ | $R_4$ | $R_5$ | X | M | Gn |
|---|---|---|---|---|---|---|---|
| H | Pr-n | Pr-n | Me | H | O | H | Gb |
| H | Pr-n | Pr-n | Et | H | O | H | Gb |
| H | Pr-n | Pr-n | Pr-n | H | O | H | Gc |
| H | Pr-n | Pr-n | Pr-i | H | O | H | Gc |
| H | Pr-n | Pr-i | Me | H | O | H | Gb |
| H | Pr-n | Pr-i | Et | H | O | H | Gb |
| H | Pr-n | Pr-i | Pr-n | H | O | H | Gc |
| H | Pr-n | Pr-i | Pr-i | H | O | H | Gc |
| H | Pr-i | Me | Me | H | O | H | Gb |
| H | Pr-i | Me | Et | H | O | H | Gb |
| H | Pr-i | Me | Pr-n | H | O | H | Gc |
| H | Pr-i | Me | Pr-i | H | O | H | Gc |
| H | Pr-i | Et | Me | H | O | H | Gb |
| H | Pr-i | Et | Et | H | O | H | Gb |
| H | Pr-i | Et | Pr-n | H | O | H | Gc |
| H | Pr-i | Et | Pr-i | H | O | H | Gc |
| H | Pr-i | Pr-n | Me | H | O | H | Gb |
| H | Pr-i | Pr-n | Et | H | O | H | Gb |
| H | Pr-i | Pr-n | Pr-n | H | O | H | Gc |
| H | Pr-i | Pr-n | Pr-i | H | O | H | Gc |
| H | Pr-i | Pr-i | Me | H | O | H | Gb |
| H | Pr-i | Pr-i | Et | H | O | H | Gb |
| H | Pr-i | Pr-i | Pr-n | H | O | H | Gc |
| H | Pr-i | Pr-i | Pr-i | H | O | H | Gc |
| Me | Me | Me | Me | H | O | H | Gb |
| F | H | H | Me | H | O | H | Gb |
| Cl | H | H | Me | H | O | H | Gb |
| Br | H | H | Me | H | O | H | Gb |
| I | H | H | Me | H | O | H | Gc |
| $CH=CH_2$ | H | H | Me | H | O | H | Gb |
| $CH_2CH=CH_2$ | H | H | Me | H | O | H | Gb |
| $C\equiv CH$ | H | H | Me | H | O | H | Gb |
| $CH_2C\equiv CH$ | H | H | Me | H | O | H | Gb |
| $CH_2F$ | H | H | Me | H | O | H | Gb |
| $CHF_2$ | H | H | Me | H | O | H | Gb |
| CN | H | H | Me | H | O | H | Gb |
| $NH_2$ | H | H | Me | H | O | H | Gb |
| NHCOMe | H | H | Me | H | O | H | Gb |
| $CH_2NH_2$ | H | H | Me | H | O | H | Gb |
| $CH_2NHCOMe$ | H | H | Me | H | O | H | Gb |

| $R_1$ | $R_2$ | $R_3$ | $R_4$ | $R_5$ | X | M | Gn |
|---|---|---|---|---|---|---|---|
| $CH_2OCHF_2$ | H | H | Me | H | O | H | Gb |
| $CF_3$ | H | H | Me | H | O | H | Gb |
| $NO_2$ | H | H | Me | H | O | H | Gb |
| OMe | H | H | Me | H | O | H | Ga |
| OEt | H | H | Me | H | O | H | Ga |
| OPr-n | H | H | Me | H | O | H | Gb |
| SMe | H | H | Me | H | O | H | Ga |
| SEt | H | H | Me | H | O | H | Ga |
| $SO_2Me$ | H | H | Me | H | O | H | Ga |
| COMe | H | H | Me | H | O | H | Ga |
| COEt | H | H | Me | H | O | H | Ga |
| $CO_2H$ | H | H | Me | H | O | H | Ga |
| $CO_2Me$ | H | H | Me | H | O | H | Ga |
| $CO_2Et$ | H | H | Me | H | O | H | Ga |
| $CO_2Pr$-n | H | H | Me | H | O | H | Gb |
| $CO_2Bu$-n | H | H | Me | H | O | H | Gc |
| $CH_2Cl$ | H | H | Me | H | O | H | Ga |
| $CH_2Br$ | H | H | Me | H | O | H | Gb |
| $CH_2I$ | H | H | Me | H | O | H | Gb |
| $CH_2CF_3$ | H | H | Me | H | O | H | Gb |
| $CH_2CN$ | H | H | Me | H | O | H | Ga |
| $CH_2OH$ | H | H | Me | H | O | H | Gb |
| $CH_2OMe$ | H | H | Me | H | O | H | Ga |
| $CH_2OEt$ | H | H | Me | H | O | H | Ga |
| $CH_2OPr$-n | H | H | Me | H | O | H | Gb |
| $CH_2SMe$ | H | H | Me | H | O | H | Ga |
| $CH_2SEt$ | H | H | Me | H | O | H | Ga |
| $CH_2SO_2Me$ | H | H | Me | H | O | H | Ga |
| $CH_2SO_2Et$ | H | H | Me | H | O | H | Ga |
| $CH_2SPr$-n | H | H | Me | H | O | H | Gb |
| $CH_2SPr$-i | H | H | Me | H | O | H | Gb |
| $CH_2COMe$ | H | H | Me | H | O | H | Ga |
| $CH_2COEt$ | H | H | Me | H | O | H | Ga |
| $CH_2COPr$-n | H | H | Me | H | O | H | Gb |
| $CH_2CO_2H$ | H | H | Me | H | O | H | Ga |
| $CH_2CO_2Me$ | H | H | Me | H | O | H | Ga |
| $CH_2CO_2Et$ | H | H | Me | H | O | H | Ga |
| $CH_2CO_2Pr$-n | H | H | Me | H | O | H | Gb |
| $CH_2OCH_2CH=CH_2$ | H | H | Me | H | O | H | Gb |
| $CH_2OCH_2C\equiv CH$ | H | H | Me | H | O | H | Gb |

| $R_1$ | $R_2$ | $R_3$ | $R_4$ | $R_5$ | X | M | Gn |
|---|---|---|---|---|---|---|---|
| $CH=CHCO_2Me$ | H | H | Me | H | O | H | Ga |
| $CH=CHCO_2Et$ | H | H | Me | H | O | H | Ga |
| $CH=CHCO_2Pr$-n | H | H | Me | H | O | H | Gb |
| CH=CHCN | H | H | Me | H | O | H | Ga |
| Ph | H | H | Me | H | O | H | Ga |
| $CH_2Ph$ | H | H | Me | H | O | H | Ga |
| OPh | H | H | Me | H | O | H | Ga |
| SPh | H | H | Me | H | O | H | Ga |
| $SO_2Ph$ | H | H | Me | H | O | H | Ga |
| CONHMe | H | H | Me | H | O | H | Ga |
| CONHEt | H | H | Me | H | O | H | Ga |
| $CON(Me)_2$ | H | H | Me | H | O | H | Ga |
| $CON(Et)_2$ | H | H | Me | H | O | H | Ga |
| $CON(Pr\text{-}n)_2$ | H | H | Me | H | O | H | Gb |
| F | H | H | Et | H | O | H | Gb |
| Cl | H | H | Et | H | O | H | Gb |
| Br | H | H | Et | H | O | H | Gb |
| I | H | H | Et | H | O | H | Gc |
| $CH=CH_2$ | H | H | Et | H | O | H | Gb |
| $CH_2CH=CH_2$ | H | H | Et | H | O | H | Gb |
| C≡CH | H | H | Et | H | O | H | Gb |
| $CH_2C≡CH$ | H | H | Et | H | O | H | Gb |
| $CH_2F$ | H | H | Et | H | O | H | Gb |
| $CHF_2$ | H | H | Et | H | O | H | Gb |
| CN | H | H | Et | H | O | H | Gb |
| $NH_2$ | H | H | Et | H | O | H | Gb |
| NHCOMe | H | H | Et | H | O | H | Gb |
| $CH_2NH_2$ | H | H | Et | H | O | H | Gb |
| $CH_2NHCOMe$ | H | H | Et | H | O | H | Gb |
| $CH_2OCHF_2$ | H | H | Et | H | O | H | Gb |
| $CF_3$ | H | H | Et | H | O | H | Gb |
| $NO_2$ | H | H | Et | H | O | H | Gb |
| OMe | H | H | Et | H | O | H | Ga |
| OEt | H | H | Et | H | O | H | Ga |
| OPr-n | H | H | Et | H | O | H | Gb |
| SMe | H | H | Et | H | O | H | Ga |
| SEt | H | H | Et | H | O | H | Ga |
| $SO_2Me$ | H | H | Et | H | O | H | Ga |
| COMe | H | H | Et | H | O | H | Ga |
| COEt | H | H | Et | H | O | H | Ga |

| $R_1$ | $R_2$ | $R_3$ | $R_4$ | $R_5$ | X | M | Gn |
|---|---|---|---|---|---|---|---|
| $CO_2H$ | H | H | Et | H | O | H | Ga |
| $CO_2Me$ | H | H | Et | H | O | H | Ga |
| $CO_2Et$ | H | H | Et | H | O | H | Ga |
| $CO_2Pr$-n | H | H | Et | H | O | H | Gb |
| $CO_2Bu$-n | H | H | Et | H | O | H | Gc |
| $CH_2Cl$ | H | H | Et | H | O | H | Ga |
| $CH_2Br$ | H | H | Et | H | O | H | Gb |
| $CH_2I$ | H | H | Et | H | O | H | Gb |
| $CH_2CF_3$ | H | H | Et | H | O | H | Gb |
| $CH_2CN$ | H | H | Et | H | O | H | Ga |
| $CH_2OH$ | H | H | Et | H | O | H | Gb |
| $CH_2OMe$ | H | H | Et | H | O | H | Ga |
| $CH_2OEt$ | H | H | Et | H | O | H | Ga |
| $CH_2OPr$-n | H | H | Et | H | O | H | Gb |
| $CH_2SMe$ | H | H | Et | H | O | H | Ga |
| $CH_2SEt$ | H | H | Et | H | O | H | Ga |
| $CH_2SO_2Me$ | H | H | Et | H | O | H | Ga |
| $CH_2SO_2Et$ | H | H | Et | H | O | H | Ga |
| $CH_2SPr$-n | H | H | Et | H | O | H | Gb |
| $CH_2SPr$-i | H | H | Et | H | O | H | Gb |
| $CH_2COMe$ | H | H | Et | H | O | H | Ga |
| $CH_2COEt$ | H | H | Et | H | O | H | Ga |
| $CH_2COPr$-n | H | H | Et | H | O | H | Gb |
| $CH_2CO_2H$ | H | H | Et | H | O | H | Ga |
| $CH_2CO_2Me$ | H | H | Et | H | O | H | Ga |
| $CH_2CO_2Et$ | H | H | Et | H | O | H | Ga |
| $CH_2CO_2Pr$-n | H | H | Et | H | O | H | Gb |
| $CH_2OCH_2CH=CH_2$ | H | H | Et | H | O | H | Gb |
| $CH_2OCH_2C\equiv CH$ | H | H | Et | H | O | H | Gb |
| $CH=CHCO_2Me$ | H | H | Et | H | O | H | Ga |
| $CH=CHCO_2Et$ | H | H | Et | H | O | H | Ga |
| $CH=CHCO_2Pr$-n | H | H | Et | H | O | H | Gb |
| CH=CHCN | H | H | Et | H | O | H | Ga |
| Ph | H | H | Et | H | O | H | Ga |
| $CH_2Ph$ | H | H | Et | H | O | H | Ga |
| OPh | H | H | Et | H | O | H | Ga |
| SPh | H | H | Et | H | O | H | Ga |
| $SO_2Ph$ | H | H | Et | H | O | H | Ga |
| CONHMe | H | H | Et | H | O | H | Ga |
| CONHEt | H | H | Et | H | O | H | Ga |

| $R_1$ | $R_2$ | $R_3$ | $R_4$ | $R_5$ | X | M | Gn |
|---|---|---|---|---|---|---|---|
| $CON(Me)_2$ | H | H | Et | H | O | H | Ga |
| $CON(Et)_2$ | H | H | Et | H | O | H | Ga |
| $CON(Pr\text{-}n)_2$ | H | H | Et | H | O | H | Gb |
| F | H | H | Cl | H | O | H | Gb |
| Cl | H | H | Cl | H | O | H | Gb |
| Br | H | H | Cl | H | O | H | Gb |
| I | H | H | Cl | H | O | H | Gc |
| $CH=CH_2$ | H | H | Cl | H | O | H | Gb |
| $CH_2CH=CH_2$ | H | H | Cl | H | O | H | Gb |
| $C\equiv CH$ | H | H | Cl | H | O | H | Gb |
| $CH_2C\equiv CH$ | H | H | Cl | H | O | H | Gb |
| $CH_2F$ | H | H | Cl | H | O | H | Gb |
| $CHF_2$ | H | H | Cl | H | O | H | Gb |
| CN | H | H | Cl | H | O | H | Gb |
| $NH_2$ | H | H | Cl | H | O | H | Gb |
| NHCOMe | H | H | Cl | H | O | H | Gb |
| $CH_2NH_2$ | H | H | Cl | H | O | H | Gb |
| $CH_2NHCOMe$ | H | H | Cl | H | O | H | Gb |
| $CH_2OCHF_2$ | H | H | Cl | H | O | H | Gb |
| $CF_3$ | H | H | Cl | H | O | H | Gb |
| $NO_2$ | H | H | Cl | H | O | H | Gb |
| OMe | H | H | Cl | H | O | H | Ga |
| OEt | H | H | Cl | H | O | H | Ga |
| OPr-n | H | H | Cl | H | O | H | Gb |
| SMe | H | H | Cl | H | O | H | Ga |
| SEt | H | H | Cl | H | O | H | Ga |
| $SO_2Me$ | H | H | Cl | H | O | H | Ga |
| COMe | H | H | Cl | H | O | H | Ga |
| COEt | H | H | Cl | H | O | H | Ga |
| $CO_2H$ | H | H | Cl | H | O | H | Ga |
| $CO_2Me$ | H | H | Cl | H | O | H | Ga |
| $CO_2Et$ | H | H | Cl | H | O | H | Ga |
| $CO_2Pr\text{-}n$ | H | H | Cl | H | O | H | Gb |
| $CO_2Bu\text{-}n$ | H | H | Cl | H | O | H | Gc |
| $CH_2Cl$ | H | H | Cl | H | O | H | Ga |
| $CH_2Br$ | H | H | Cl | H | O | H | Gb |
| $CH_2I$ | H | H | Cl | H | O | H | Gb |
| $CH_2CF_3$ | H | H | Cl | H | O | H | Gb |
| $CH_2CN$ | H | H | Cl | H | O | H | Ga |
| $CH_2OH$ | H | H | Cl | H | O | H | Gb |

| $R_1$ | $R_2$ | $R_3$ | $R_4$ | $R_5$ | X | M | Gn |
|---|---|---|---|---|---|---|---|
| $CH_2OMe$ | H | H | Cl | H | O | H | Ga |
| $CH_2OEt$ | H | H | Cl | H | O | H | Ga |
| $CH_2OPr$-n | H | H | Cl | H | O | H | Gb |
| $CH_2SMe$ | H | H | Cl | H | O | H | Ga |
| $CH_2SEt$ | H | H | Cl | H | O | H | Ga |
| $CH_2SO_2Me$ | H | H | Cl | H | O | H | Ga |
| $CH_2SO_2Et$ | H | H | Cl | H | O | H | Ga |
| $CH_2SPr$-n | H | H | Cl | H | O | H | Gb |
| $CH_2SPr$-i | H | H | Cl | H | O | H | Gb |
| $CH_2COMe$ | H | H | Cl | H | O | H | Ga |
| $CH_2COEt$ | H | H | Cl | H | O | H | Ga |
| $CH_2COPr$-n | H | H | Cl | H | O | H | Gb |
| $CH_2CO_2H$ | H | H | Cl | H | O | H | Ga |
| $CH_2CO_2Me$ | H | H | Cl | H | O | H | Ga |
| $CH_2CO_2Et$ | H | H | Cl | H | O | H | Ga |
| $CH_2CO_2Pr$-n | H | H | Cl | H | O | H | Gb |
| $CH_2OCH_2CH{=}CH_2$ | H | H | Cl | H | O | H | Gb |
| $CH_2OCH_2C{\equiv}CH$ | H | H | Cl | H | O | H | Gb |
| $CH{=}CHCO_2Me$ | H | H | Cl | H | O | H | Ga |
| $CH{=}CHCO_2Et$ | H | H | Cl | H | O | H | Ga |
| $CH{=}CHCO_2Pr$-n | H | H | Cl | H | O | H | Gb |
| $CH{=}CHCN$ | H | H | Cl | H | O | H | Ga |
| Ph | H | H | Cl | H | O | H | Ga |
| $CH_2Ph$ | H | H | Cl | H | O | H | Ga |
| OPh | H | H | Cl | H | O | H | Ga |
| SPh | H | H | Cl | H | O | H | Ga |
| $SO_2Ph$ | H | H | Cl | H | O | H | Ga |
| CONHMe | H | H | Cl | H | O | H | Ga |
| CONHEt | H | H | Cl | H | O | H | Ga |
| $CON(Me)_2$ | H | H | Cl | H | O | H | Ga |
| $CON(Et)_2$ | H | H | Cl | H | O | H | Ga |
| $CON(Pr\text{-}n)_2$ | H | H | Cl | H | O | H | Gb |
| F | H | H | $CO_2Me$ | H | O | H | Gb |
| Cl | H | H | $CO_2Me$ | H | O | H | Gb |
| Br | H | H | $CO_2Me$ | H | O | H | Gb |
| I | H | H | $CO_2Me$ | H | O | H | Gc |
| $CH{=}CH_2$ | H | H | $CO_2Me$ | H | O | H | Gb |
| $CH_2CH{=}CH_2$ | H | H | $CO_2Me$ | H | O | H | Gb |
| $C{\equiv}CH$ | H | H | $CO_2Me$ | H | O | H | Gb |
| $CH_2C{\equiv}CH$ | H | H | $CO_2Me$ | H | O | H | Gb |

| $R_1$ | $R_2$ | $R_3$ | $R_4$ | $R_5$ | X | M | Gn |
|---|---|---|---|---|---|---|---|
| $CH_2F$ | H | H | $CO_2Me$ | H | O | H | Gb |
| $CHF_2$ | H | H | $CO_2Me$ | H | O | H | Gb |
| CN | H | H | $CO_2Me$ | H | O | H | Gb |
| $NH_2$ | H | H | $CO_2Me$ | H | O | H | Gb |
| NHCOMe | H | H | $CO_2Me$ | H | O | H | Gb |
| $CH_2NH_2$ | H | H | $CO_2Me$ | H | O | H | Gb |
| $CH_2NHCOMe$ | H | H | $CO_2Me$ | H | O | H | Gb |
| $CH_2OCHF_2$ | H | H | $CO_2Me$ | H | O | H | Gb |
| $CF_3$ | H | H | $CO_2Me$ | H | O | H | Gb |
| $NO_2$ | H | H | $CO_2Me$ | H | O | H | Gb |
| OMe | H | H | $CO_2Me$ | H | O | H | Ga |
| OEt | H | H | $CO_2Me$ | H | O | H | Ga |
| OPr-n | H | H | $CO_2Me$ | H | O | H | Gb |
| SMe | H | H | $CO_2Me$ | H | O | H | Ga |
| SEt | H | H | $CO_2Me$ | H | O | H | Ga |
| $SO_2Me$ | H | H | $CO_2Me$ | H | O | H | Ga |
| COMe | H | H | $CO_2Me$ | H | O | H | Ga |
| COEt | H | H | $CO_2Me$ | H | O | H | Ga |
| $CO_2H$ | H | H | $CO_2Me$ | H | O | H | Ga |
| $CO_2Me$ | H | H | $CO_2Me$ | H | O | H | Ga |
| $CO_2Et$ | H | H | $CO_2Me$ | H | O | H | Ga |
| $CO_2Pr$-n | H | H | $CO_2Me$ | H | O | H | Gb |
| $CO_2Bu$-n | H | H | $CO_2Me$ | H | O | H | Gc |
| $CH_2Cl$ | H | H | $CO_2Me$ | H | O | H | Ga |
| $CH_2Br$ | H | H | $CO_2Me$ | H | O | H | Gb |
| $CH_2I$ | H | H | $CO_2Me$ | H | O | H | Gb |
| $CH_2CF_3$ | H | H | $CO_2Me$ | H | O | H | Gb |
| $CH_2CN$ | H | H | $CO_2Me$ | H | O | H | Ga |
| $CH_2OH$ | H | H | $CO_2Me$ | H | O | H | Gb |
| $CH_2OMe$ | H | H | $CO_2Me$ | H | O | H | Ga |
| $CH_2OEt$ | H | H | $CO_2Me$ | H | O | H | Ga |
| $CH_2OPr$-n | H | H | $CO_2Me$ | H | O | H | Gb |
| $CH_2SMe$ | H | H | $CO_2Me$ | H | O | H | Ga |
| $CH_2SEt$ | H | H | $CO_2Me$ | H | O | H | Ga |
| $CH_2SO_2Me$ | H | H | $CO_2Me$ | H | O | H | Ga |
| $CH_2SO_2Et$ | H | H | $CO_2Me$ | H | O | H | Ga |
| $CH_2SPr$-n | H | H | $CO_2Me$ | H | O | H | Gb |
| $CH_2SPr$-i | H | H | $CO_2Me$ | H | O | H | Gb |
| $CH_2COMe$ | H | H | $CO_2Me$ | H | O | H | Ga |
| $CH_2COEt$ | H | H | $CO_2Me$ | H | O | H | Ga |

| $R_1$ | $R_2$ | $R_3$ | $R_4$ | $R_5$ | X | M | Gn |
|---|---|---|---|---|---|---|---|
| $CH_2COPr$-n | H | H | $CO_2Me$ | H | O | H | Gb |
| $CH_2CO_2H$ | H | H | $CO_2Me$ | H | O | H | Ga |
| $CH_2CO_2Me$ | H | H | $CO_2Me$ | H | O | H | Ga |
| $CH_2CO_2Et$ | H | H | $CO_2Me$ | H | O | H | Ga |
| $CH_2CO_2Pr$-n | H | H | $CO_2Me$ | H | O | H | Gb |
| $CH_2OCH_2CH{=}CH_2$ | H | H | $CO_2Me$ | H | O | H | Gb |
| $CH_2OCH_2C{\equiv}CH$ | H | H | $CO_2Me$ | H | O | H | Gb |
| $CH{=}CHCO_2Me$ | H | H | $CO_2Me$ | H | O | H | Ga |
| $CH{=}CHCO_2Et$ | H | H | $CO_2Me$ | H | O | H | Ga |
| $CH{=}CHCO_2Pr$-n | H | H | $CO_2Me$ | H | O | H | Gb |
| $CH{=}CHCN$ | H | H | $CO_2Me$ | H | O | H | Ga |
| Ph | H | H | $CO_2Me$ | H | O | H | Ga |
| $CH_2Ph$ | H | H | $CO_2Me$ | H | O | H | Ga |
| OPh | H | H | $CO_2Me$ | H | O | H | Ga |
| SPh | H | H | $CO_2Me$ | H | O | H | Ga |
| $SO_2Ph$ | H | H | $CO_2Me$ | H | O | H | Ga |
| CONHMe | H | H | $CO_2Me$ | H | O | H | Ga |
| CONHEt | H | H | $CO_2Me$ | H | O | H | Ga |
| $CON(Me)_2$ | H | H | $CO_2Me$ | H | O | H | Ga |
| $CON(Et)_2$ | H | H | $CO_2Me$ | H | O | H | Ga |
| $CON(Pr\text{-}n)_2$ | H | H | $CO_2Me$ | H | O | H | Gb |
| F | H | H | $CH_2Cl$ | H | O | H | Gb |
| Cl | H | H | $CH_2Cl$ | H | O | H | Gb |
| Br | H | H | $CH_2Cl$ | H | O | H | Gb |
| I | H | H | $CH_2Cl$ | H | O | H | Gc |
| $CH{=}CH_2$ | H | H | $CH_2Cl$ | H | O | H | Gb |
| $CH_2CH{=}CH_2$ | H | H | $CH_2Cl$ | H | O | H | Gb |
| $C{\equiv}CH$ | H | H | $CH_2Cl$ | H | O | H | Gb |
| $CH_2C{\equiv}CH$ | H | H | $CH_2Cl$ | H | O | H | Gb |
| $CH_2F$ | H | H | $CH_2Cl$ | H | O | H | Gb |
| $CHF_2$ | H | H | $CH_2Cl$ | H | O | H | Gb |
| CN | H | H | $CH_2Cl$ | H | O | H | Gb |
| $NH_2$ | H | H | $CH_2Cl$ | H | O | H | Gb |
| NHCOMe | H | H | $CH_2Cl$ | H | O | H | Gb |
| $CH_2NH_2$ | H | H | $CH_2Cl$ | H | O | H | Gb |
| $CH_2NHCOMe$ | H | H | $CH_2Cl$ | H | O | H | Gb |
| $CH_2OCHF_2$ | H | H | $CH_2Cl$ | H | O | H | Gb |
| $CF_3$ | H | H | $CH_2Cl$ | H | O | H | Gb |
| $NO_2$ | H | H | $CH_2Cl$ | H | O | H | Gb |
| OMe | H | H | $CH_2Cl$ | H | O | H | Ga |

| $R_1$ | $R_2$ | $R_3$ | $R_4$ | $R_5$ | X | M | Gn |
|---|---|---|---|---|---|---|---|
| OEt | H | H | $CH_2Cl$ | H | O | H | Ga |
| OPr-n | H | H | $CH_2Cl$ | H | O | H | Gb |
| SMe | H | H | $CH_2Cl$ | H | O | H | Ga |
| SEt | H | H | $CH_2Cl$ | H | O | H | Ga |
| $SO_2Me$ | H | H | $CH_2Cl$ | H | O | H | Ga |
| COMe | H | H | $CH_2Cl$ | H | O | H | Ga |
| COEt | H | H | $CH_2Cl$ | H | O | H | Ga |
| $CO_2H$ | H | H | $CH_2Cl$ | H | O | H | Ga |
| $CO_2Me$ | H | H | $CH_2Cl$ | H | O | H | Ga |
| $CO_2Et$ | H | H | $CH_2Cl$ | H | O | H | Ga |
| $CO_2Pr$-n | H | H | $CH_2Cl$ | H | O | H | Gb |
| $CO_2Bu$-n | H | H | $CH_2Cl$ | H | O | H | Gc |
| $CH_2Cl$ | H | H | $CH_2Cl$ | H | O | H | Ga |
| $CH_2Br$ | H | H | $CH_2Cl$ | H | O | H | Gb |
| $CH_2I$ | H | H | $CH_2Cl$ | H | O | H | Gb |
| $CH_2CF_3$ | H | H | $CH_2Cl$ | H | O | H | Gb |
| $CH_2CN$ | H | H | $CH_2Cl$ | H | O | H | Ga |
| $CH_2OH$ | H | H | $CH_2Cl$ | H | O | H | Gb |
| $CH_2OMe$ | H | H | $CH_2Cl$ | H | O | H | Ga |
| $CH_2OEt$ | H | H | $CH_2Cl$ | H | O | H | Ga |
| $CH_2OPr$-n | H | H | $CH_2Cl$ | H | O | H | Gb |
| $CH_2SMe$ | H | H | $CH_2Cl$ | H | O | H | Ga |
| $CH_2SEt$ | H | H | $CH_2Cl$ | H | O | H | Ga |
| $CH_2SO_2Me$ | H | H | $CH_2Cl$ | H | O | H | Ga |
| $CH_2SO_2Et$ | H | H | $CH_2Cl$ | H | O | H | Ga |
| $CH_2SPr$-n | H | H | $CH_2Cl$ | H | O | H | Gb |
| $CH_2SPr$-i | H | H | $CH_2Cl$ | H | O | H | Gb |
| $CH_2COMe$ | H | H | $CH_2Cl$ | H | O | H | Ga |
| $CH_2COEt$ | H | H | $CH_2Cl$ | H | O | H | Ga |
| $CH_2COPr$-n | H | H | $CH_2Cl$ | H | O | H | Gb |
| $CH_2CO_2H$ | H | H | $CH_2Cl$ | H | O | H | Ga |
| $CH_2CO_2Me$ | H | H | $CH_2Cl$ | H | O | H | Ga |
| $CH_2CO_2Et$ | H | H | $CH_2Cl$ | H | O | H | Ga |
| $CH_2CO_2Pr$-n | H | H | $CH_2Cl$ | H | O | H | Gb |
| $CH_2OCH_2CH=CH_2$ | H | H | $CH_2Cl$ | H | O | H | Gb |
| $CH_2OCH_2C{\equiv}CH$ | H | H | $CH_2Cl$ | H | O | H | Gb |
| $CH=CHCO_2Me$ | H | H | $CH_2Cl$ | H | O | H | Ga |
| $CH=CHCO_2Et$ | H | H | $CH_2Cl$ | H | O | H | Ga |
| $CH=CHCO_2Pr$-n | H | H | $CH_2Cl$ | H | O | H | Gb |
| $CH=CHCN$ | H | H | $CH_2Cl$ | H | O | H | Ga |

| $R_1$ | $R_2$ | $R_3$ | $R_4$ | $R_5$ | X | M | Gn |
|---|---|---|---|---|---|---|---|
| Ph | H | H | $CH_2Cl$ | H | O | H | Ga |
| $CH_2Ph$ | H | H | $CH_2Cl$ | H | O | H | Ga |
| OPh | H | H | $CH_2Cl$ | H | O | H | Ga |
| SPh | H | H | $CH_2Cl$ | H | O | H | Ga |
| $SO_2Ph$ | H | H | $CH_2Cl$ | H | O | H | Ga |
| CONHMe | H | H | $CH_2Cl$ | H | O | H | Ga |
| CONHEt | H | H | $CH_2Cl$ | H | O | H | Ga |
| $CON(Me)_2$ | H | H | $CH_2Cl$ | H | O | H | Ga |
| $CON(Et)_2$ | H | H | $CH_2Cl$ | H | O | H | Ga |
| $CON(Pr\text{-}n)_2$ | H | H | $CH_2Cl$ | H | O | H | Gb |
| F | H | H | $CH_2OMe$ | H | O | H | Gb |
| Cl | H | H | $CH_2OMe$ | H | O | H | Gb |
| Br | H | H | $CH_2OMe$ | H | O | H | Gb |
| I | H | H | $CH_2OMe$ | H | O | H | Gc |
| $CH=CH_2$ | H | H | $CH_2OMe$ | H | O | H | Gb |
| $CH_2CH=CH_2$ | H | H | $CH_2OMe$ | H | O | H | Gb |
| $C\equiv CH$ | H | H | $CH_2OMe$ | H | O | H | Gb |
| $CH_2C\equiv CH$ | H | H | $CH_2OMe$ | H | O | H | Gb |
| $CH_2F$ | H | H | $CH_2OMe$ | H | O | H | Gb |
| $CHF_2$ | H | H | $CH_2OMe$ | H | O | H | Gb |
| CN | H | H | $CH_2OMe$ | H | O | H | Gb |
| $NH_2$ | H | H | $CH_2OMe$ | H | O | H | Gb |
| NHCOMe | H | H | $CH_2OMe$ | H | O | H | Gb |
| $CH_2NH_2$ | H | H | $CH_2OMe$ | H | O | H | Gb |
| $CH_2NHCOMe$ | H | H | $CH_2OMe$ | H | O | H | Gb |
| $CH_2OCHF_2$ | H | H | $CH_2OMe$ | H | O | H | Gb |
| $CF_3$ | H | H | $CH_2OMe$ | H | O | H | Gb |
| $NO_2$ | H | H | $CH_2OMe$ | H | O | H | Gb |
| OMe | H | H | $CH_2OMe$ | H | O | H | Ga |
| OEt | H | H | $CH_2OMe$ | H | O | H | Ga |
| OPr-n | H | H | $CH_2OMe$ | H | O | H | Gb |
| SMe | H | H | $CH_2OMe$ | H | O | H | Ga |
| SEt | H | H | $CH_2OMe$ | H | O | H | Ga |
| $SO_2Me$ | H | H | $CH_2OMe$ | H | O | H | Ga |
| COMe | H | H | $CH_2OMe$ | H | O | H | Ga |
| COEt | H | H | $CH_2OMe$ | H | O | H | Ga |
| $CO_2H$ | H | H | $CH_2OMe$ | H | O | H | Ga |
| $CO_2Me$ | H | H | $CH_2OMe$ | H | O | H | Ga |
| $CO_2Et$ | H | H | $CH_2OMe$ | H | O | H | Ga |
| $CO_2Pr\text{-}n$ | H | H | $CH_2OMe$ | H | O | H | Gb |

| $R_1$ | $R_2$ | $R_3$ | $R_4$ | $R_5$ | X | M | Gn |
|---|---|---|---|---|---|---|---|
| $CO_2Bu$-n | H | H | $CH_2OMe$ | H | O | H | Gc |
| $CH_2Cl$ | H | H | $CH_2OMe$ | H | O | H | Ga |
| $CH_2Br$ | H | H | $CH_2OMe$ | H | O | H | Gb |
| $CH_2I$ | H | H | $CH_2OMe$ | H | O | H | Gb |
| $CH_2CF_3$ | H | H | $CH_2OMe$ | H | O | H | Gb |
| $CH_2CN$ | H | H | $CH_2OMe$ | H | O | H | Ga |
| $CH_2OH$ | H | H | $CH_2OMe$ | H | O | H | Gb |
| $CH_2OMe$ | H | H | $CH_2OMe$ | H | O | H | Ga |
| $CH_2OEt$ | H | H | $CH_2OMe$ | H | O | H | Ga |
| $CH_2OPr$-n | H | H | $CH_2OMe$ | H | O | H | Gb |
| $CH_2SMe$ | H | H | $CH_2OMe$ | H | O | H | Ga |
| $CH_2SEt$ | H | H | $CH_2OMe$ | H | O | H | Ga |
| $CH_2SO_2Me$ | H | H | $CH_2OMe$ | H | O | H | Ga |
| $CH_2SO_2Et$ | H | H | $CH_2OMe$ | H | O | H | Ga |
| $CH_2SPr$-n | H | H | $CH_2OMe$ | H | O | H | Gb |
| $CH_2SPr$-i | H | H | $CH_2OMe$ | H | O | H | Gb |
| $CH_2COMe$ | H | H | $CH_2OMe$ | H | O | H | Ga |
| $CH_2COEt$ | H | H | $CH_2OMe$ | H | O | H | Ga |
| $CH_2COPr$-n | H | H | $CH_2OMe$ | H | O | H | Gb |
| $CH_2CO_2H$ | H | H | $CH_2OMe$ | H | O | H | Ga |
| $CH_2CO_2Me$ | H | H | $CH_2OMe$ | H | O | H | Ga |
| $CH_2CO_2Et$ | H | H | $CH_2OMe$ | H | O | H | Ga |
| $CH_2CO_2Pr$-n | H | H | $CH_2OMe$ | H | O | H | Gb |
| $CH_2OCH_2CH=CH_2$ | H | H | $CH_2OMe$ | H | O | H | Gb |
| $CH_2OCH_2C\equiv CH$ | H | H | $CH_2OMe$ | H | O | H | Gb |
| $CH=CHCO_2Me$ | H | H | $CH_2OMe$ | H | O | H | Ga |
| $CH=CHCO_2Et$ | H | H | $CH_2OMe$ | H | O | H | Ga |
| $CH=CHCO_2Pr$-n | H | H | $CH_2OMe$ | H | O | H | Gb |
| $CH=CHCN$ | H | H | $CH_2OMe$ | H | O | H | Ga |
| Ph | H | H | $CH_2OMe$ | H | O | H | Ga |
| $CH_2Ph$ | H | H | $CH_2OMe$ | H | O | H | Ga |
| OPh | H | H | $CH_2OMe$ | H | O | H | Ga |
| SPh | H | H | $CH_2OMe$ | H | O | H | Ga |
| $SO_2Ph$ | H | H | $CH_2OMe$ | H | O | H | Ga |
| CONHMe | H | H | $CH_2OMe$ | H | O | H | Ga |
| CONHEt | H | H | $CH_2OMe$ | H | O | H | Ga |
| $CON(Me)_2$ | H | H | $CH_2OMe$ | H | O | H | Ga |
| $CON(Et)_2$ | H | H | $CH_2OMe$ | H | O | H | Ga |
| $CON(Pr$-$n)_2$ | H | H | $CH_2OMe$ | H | O | H | Gb |
| F | H | H | $CH_2SMe$ | H | O | H | Gb |

| $R_1$ | $R_2$ | $R_3$ | $R_4$ | $R_5$ | X | M | Gn |
|---|---|---|---|---|---|---|---|
| Cl | H | H | $CH_2SMe$ | H | O | H | Gb |
| Br | H | H | $CH_2SMe$ | H | O | H | Gb |
| I | H | H | $CH_2SMe$ | H | O | H | Gc |
| $CH=CH_2$ | H | H | $CH_2SMe$ | H | O | H | Gb |
| $CH_2CH=CH_2$ | H | H | $CH_2SMe$ | H | O | H | Gb |
| $C\equiv CH$ | H | H | $CH_2SMe$ | H | O | H | Gb |
| $CH_2C\equiv CH$ | H | H | $CH_2SMe$ | H | O | H | Gb |
| $CH_2F$ | H | H | $CH_2SMe$ | H | O | H | Gb |
| $CHF_2$ | H | H | $CH_2SMe$ | H | O | H | Gb |
| CN | H | H | $CH_2SMe$ | H | O | H | Gb |
| $NH_2$ | H | H | $CH_2SMe$ | H | O | H | Gb |
| NHCOMe | H | H | $CH_2SMe$ | H | O | H | Gb |
| $CH_2NH_2$ | H | H | $CH_2SMe$ | H | O | H | Gb |
| $CH_2NHCOMe$ | H | H | $CH_2SMe$ | H | O | H | Gb |
| $CH_2OCHF_2$ | H | H | $CH_2SMe$ | H | O | H | Gb |
| $CF_3$ | H | H | $CH_2SMe$ | H | O | H | Gb |
| $NO_2$ | H | H | $CH_2SMe$ | H | O | H | Gb |
| OMe | H | H | $CH_2SMe$ | H | O | H | Ga |
| OEt | H | H | $CH_2SMe$ | H | O | H | Ga |
| OPr-n | H | H | $CH_2SMe$ | H | O | H | Gb |
| SMe | H | H | $CH_2SMe$ | H | O | H | Ga |
| SEt | H | H | $CH_2SMe$ | H | O | H | Ga |
| $SO_2Me$ | H | H | $CH_2SMe$ | H | O | H | Ga |
| COMe | H | H | $CH_2SMe$ | H | O | H | Ga |
| COEt | H | H | $CH_2SMe$ | H | O | H | Ga |
| $CO_2H$ | H | H | $CH_2SMe$ | H | O | H | Ga |
| $CO_2Me$ | H | H | $CH_2SMe$ | H | O | H | Ga |
| $CO_2Et$ | H | H | $CH_2SMe$ | H | O | H | Ga |
| $CO_2Pr$-n | H | H | $CH_2SMe$ | H | O | H | Gb |
| $CO_2Bu$-n | H | H | $CH_2SMe$ | H | O | H | Gc |
| $CH_2Cl$ | H | H | $CH_2SMe$ | H | O | H | Ga |
| $CH_2Br$ | H | H | $CH_2SMe$ | H | O | H | Gb |
| $CH_2I$ | H | H | $CH_2SMe$ | H | O | H | Gb |
| $CH_2CF_3$ | H | H | $CH_2SMe$ | H | O | H | Gb |
| $CH_2CN$ | H | H | $CH_2SMe$ | H | O | H | Ga |
| $CH_2OH$ | H | H | $CH_2SMe$ | H | O | H | Gb |
| $CH_2OMe$ | H | H | $CH_2SMe$ | H | O | H | Ga |
| $CH_2OEt$ | H | H | $CH_2SMe$ | H | O | H | Ga |
| $CH_2OPr$-n | H | H | $CH_2SMe$ | H | O | H | Gb |
| $CH_2SMe$ | H | H | $CH_2SMe$ | H | O | H | Ga |

| $R_1$ | $R_2$ | $R_3$ | $R_4$ | $R_5$ | X | M | Gn |
|---|---|---|---|---|---|---|---|
| $CH_2SEt$ | H | H | $CH_2SMe$ | H | O | H | Ga |
| $CH_2SO_2Me$ | H | H | $CH_2SMe$ | H | O | H | Ga |
| $CH_2SO_2Et$ | H | H | $CH_2SMe$ | H | O | H | Ga |
| $CH_2SPr$-n | H | H | $CH_2SMe$ | H | O | H | Gb |
| $CH_2SPr$-i | H | H | $CH_2SMe$ | H | O | H | Gb |
| $CH_2COMe$ | H | H | $CH_2SMe$ | H | O | H | Ga |
| $CH_2COEt$ | H | H | $CH_2SMe$ | H | O | H | Ga |
| $CH_2COPr$-n | H | H | $CH_2SMe$ | H | O | H | Gb |
| $CH_2CO_2H$ | H | H | $CH_2SMe$ | H | O | H | Ga |
| $CH_2CO_2Me$ | H | H | $CH_2SMe$ | H | O | H | Ga |
| $CH_2CO_2Et$ | H | H | $CH_2SMe$ | H | O | H | Ga |
| $CH_2CO_2Pr$-n | H | H | $CH_2SMe$ | H | O | H | Gb |
| $CH_2OCH_2CH=CH_2$ | H | H | $CH_2SMe$ | H | O | H | Gb |
| $CH_2OCH_2C\equiv CH$ | H | H | $CH_2SMe$ | H | O | H | Gb |
| $CH=CHCO_2Me$ | H | H | $CH_2SMe$ | H | O | H | Ga |
| $CH=CHCO_2Et$ | H | H | $CH_2SMe$ | H | O | H | Ga |
| $CH=CHCO_2Pr$-n | H | H | $CH_2SMe$ | H | O | H | Gb |
| CH=CHCN | H | H | $CH_2SMe$ | H | O | H | Ga |
| Ph | H | H | $CH_2SMe$ | H | O | H | Ga |
| $CH_2Ph$ | H | H | $CH_2SMe$ | H | O | H | Ga |
| OPh | H | H | $CH_2SMe$ | H | O | H | Ga |
| SPh | H | H | $CH_2SMe$ | H | O | H | Ga |
| $SO_2Ph$ | H | H | $CH_2SMe$ | H | O | H | Ga |
| CONHMe | H | H | $CH_2SMe$ | H | O | H | Ga |
| CONHEt | H | H | $CH_2SMe$ | H | O | H | Ga |
| $CON(Me)_2$ | H | H | $CH_2SMe$ | H | O | H | Ga |
| $CON(Et)_2$ | H | H | $CH_2SMe$ | H | O | H | Ga |
| $CON(Pr\text{-}n)_2$ | H | H | $CH_2SMe$ | H | O | H | Gb |
| F | H | H | CN | H | O | H | Gb |
| Cl | H | H | CN | H | O | H | Gb |
| Br | H | H | CN | H | O | H | Gb |
| I | H | H | CN | H | O | H | Gc |
| $CH=CH_2$ | H | H | CN | H | O | H | Gb |
| $CH_2CH=CH_2$ | H | H | CN | H | O | H | Gb |
| $C\equiv CH$ | H | H | CN | H | O | H | Gb |
| $CH_2C\equiv CH$ | H | H | CN | H | O | H | Gb |
| $CH_2F$ | H | H | CN | H | O | H | Gb |
| $CHF_2$ | H | H | CN | H | O | H | Gb |
| CN | H | H | CN | H | O | H | Gb |
| $NH_2$ | H | H | CN | H | O | H | Gb |

| $R_1$ | $R_2$ | $R_3$ | $R_4$ | $R_5$ | X | M | Gn |
|---|---|---|---|---|---|---|---|
| NHCOMe | H | H | CN | H | O | H | Gb |
| $CH_2NH_2$ | H | H | CN | H | O | H | Gb |
| $CH_2NHCOMe$ | H | H | CN | H | O | H | Gb |
| $CH_2OCHF_2$ | H | H | CN | H | O | H | Gb |
| $CF_3$ | H | H | CN | H | O | H | Gb |
| $NO_2$ | H | H | CN | H | O | H | Gb |
| OMe | H | H | CN | H | O | H | Ga |
| OEt | H | H | CN | H | O | H | Ga |
| OPr-n | H | H | CN | H | O | H | Gb |
| SMe | H | H | CN | H | O | H | Ga |
| SEt | H | H | CN | H | O | H | Ga |
| $SO_2Me$ | H | H | CN | H | O | H | Ga |
| COMe | H | H | CN | H | O | H | Ga |
| COEt | H | H | CN | H | O | H | Ga |
| $CO_2H$ | H | H | CN | H | O | H | Ga |
| $CO_2Me$ | H | H | CN | H | O | H | Ga |
| $CO_2Et$ | H | H | CN | H | O | H | Ga |
| $CO_2Pr$-n | H | H | CN | H | O | H | Gb |
| $CO_2Bu$-n | H | H | CN | H | O | H | Gc |
| $CH_2Cl$ | H | H | CN | H | O | H | Ga |
| $CH_2Br$ | H | H | CN | H | O | H | Gb |
| $CH_2I$ | H | H | CN | H | O | H | Gb |
| $CH_2CF_3$ | H | H | CN | H | O | H | Gb |
| $CH_2CN$ | H | H | CN | H | O | H | Ga |
| $CH_2OH$ | H | H | CN | H | O | H | Gb |
| $CH_2OMe$ | H | H | CN | H | O | H | Ga |
| $CH_2OEt$ | H | H | CN | H | O | H | Ga |
| $CH_2OPr$-n | H | H | CN | H | O | H | Gb |
| $CH_2SMe$ | H | H | CN | H | O | H | Ga |
| $CH_2SEt$ | H | H | CN | H | O | H | Ga |
| $CH_2SO_2Me$ | H | H | CN | H | O | H | Ga |
| $CH_2SO_2Et$ | H | H | CN | H | O | H | Ga |
| $CH_2SPr$-n | H | H | CN | H | O | H | Gb |
| $CH_2SPr$-i | H | H | CN | H | O | H | Gb |
| $CH_2COMe$ | H | H | CN | H | O | H | Ga |
| $CH_2COEt$ | H | H | CN | H | O | H | Ga |
| $CH_2COPr$-n | H | H | CN | H | O | H | Gb |
| $CH_2CO_2H$ | H | H | CN | H | O | H | Ga |
| $CH_2CO_2Me$ | H | H | CN | H | O | H | Ga |
| $CH_2CO_2Et$ | H | H | CN | H | O | H | Ga |

| $R_1$ | $R_2$ | $R_3$ | $R_4$ | $R_5$ | X | M | Gn |
|---|---|---|---|---|---|---|---|
| $CH_2CO_2Pr$-n | H | H | CN | H | O | H | Gb |
| $CH_2OCH_2CH=CH_2$ | H | H | CN | H | O | H | Gb |
| $CH_2OCH_2C\equiv CH$ | H | H | CN | H | O | H | Gb |
| $CH=CHCO_2Me$ | H | H | CN | H | O | H | Ga |
| $CH=CHCO_2Et$ | H | H | CN | H | O | H | Ga |
| $CH=CHCO_2Pr$-n | H | H | CN | H | O | H | Gb |
| CH=CHCN | H | H | CN | H | O | H | Ga |
| Ph | H | H | CN | H | O | H | Ga |
| $CH_2Ph$ | H | H | CN | H | O | H | Ga |
| OPh | H | H | CN | H | O | H | Ga |
| SPh | H | H | CN | H | O | H | Ga |
| $SO_2Ph$ | H | H | CN | H | O | H | Ga |
| CONHMe | H | H | CN | H | O | H | Ga |
| CONHEt | H | H | CN | H | O | H | Ga |
| $CON(Me)_2$ | H | H | CN | H | O | H | Ga |
| $CON(Et)_2$ | H | H | CN | H | O | H | Ga |
| $CON(Pr\text{-}n)_2$ | H | H | CN | H | O | H | Gb |

| $R_4$ | $R_3$ | $R_2$ | $R_1$ | $R_5$ | X | M | Gn |
|---|---|---|---|---|---|---|---|
| F | H | H | Me | H | O | H | Gb |
| Cl | H | H | Me | H | O | H | Gb |
| Br | H | H | Me | H | O | H | Gb |
| I | H | H | Me | H | O | H | Gc |
| $CH=CH_2$ | H | H | Me | H | O | H | Gb |
| $CH_2CH=CH_2$ | H | H | Me | H | O | H | Gb |
| $C\equiv CH$ | H | H | Me | H | O | H | Gb |
| $CH_2C\equiv CH$ | H | H | Me | H | O | H | Gb |
| $CH_2F$ | H | H | Me | H | O | H | Gb |
| $CHF_2$ | H | H | Me | H | O | H | Gb |
| CN | H | H | Me | H | O | H | Gb |
| $NH_2$ | H | H | Me | H | O | H | Gb |
| NHCOMe | H | H | Me | H | O | H | Gb |
| $CH_2NH_2$ | H | H | Me | H | O | H | Gb |

| $R_4$ | $R_3$ | $R_2$ | $R_1$ | $R_5$ | X | M | Gn |
|---|---|---|---|---|---|---|---|
| $CH_2NHCOMe$ | H | H | Me | H | O | H | Gb |
| $CH_2OCHF_2$ | H | H | Me | H | O | H | Gb |
| $CF_3$ | H | H | Me | H | O | H | Gb |
| $NO_2$ | H | H | Me | H | O | H | Gb |
| OMe | H | H | Me | H | O | H | Ga |
| OEt | H | H | Me | H | O | H | Ga |
| OPr-n | H | H | Me | H | O | H | Gb |
| SMe | H | H | Me | H | O | H | Ga |
| SEt | H | H | Me | H | O | H | Ga |
| $SO_2Me$ | H | H | Me | H | O | H | Ga |
| COMe | H | H | Me | H | O | H | Ga |
| COEt | H | H | Me | H | O | H | Ga |
| $CO_2H$ | H | H | Me | H | O | H | Ga |
| $CO_2Me$ | H | H | Me | H | O | H | Ga |
| $CO_2Et$ | H | H | Me | H | O | H | Ga |
| $CO_2Pr$-n | H | H | Me | H | O | H | Gb |
| $CO_2Bu$-n | H | H | Me | H | O | H | Gc |
| $CH_2Cl$ | H | H | Me | H | O | H | Ga |
| $CH_2Br$ | H | H | Me | H | O | H | Gb |
| $CH_2I$ | H | H | Me | H | O | H | Gb |
| $CH_2CF_3$ | H | H | Me | H | O | H | Gb |
| $CH_2CN$ | H | H | Me | H | O | H | Ga |
| $CH_2OH$ | H | H | Me | H | O | H | Gb |
| $CH_2OMe$ | H | H | Me | H | O | H | Ga |
| $CH_2OEt$ | H | H | Me | H | O | H | Ga |
| $CH_2OPr$-n | H | H | Me | H | O | H | Gb |
| $CH_2SMe$ | H | H | Me | H | O | H | Ga |
| $CH_2SEt$ | H | H | Me | H | O | H | Ga |
| $CH_2SO_2Me$ | H | H | Me | H | O | H | Ga |
| $CH_2SO_2Et$ | H | H | Me | H | O | H | Ga |
| $CH_2SPr$-n | H | H | Me | H | O | H | Gb |
| $CH_2SPr$-i | H | H | Me | H | O | H | Gb |
| $CH_2COMe$ | H | H | Me | H | O | H | Ga |
| $CH_2COEt$ | H | H | Me | H | O | H | Ga |
| $CH_2COPr$-n | H | H | Me | H | O | H | Gb |
| $CH_2CO_2H$ | H | H | Me | H | O | H | Ga |
| $CH_2CO_2Me$ | H | H | Me | H | O | H | Ga |
| $CH_2CO_2Et$ | H | H | Me | H | O | H | Ga |
| $CH_2CO_2Pr$-n | H | H | Me | H | O | H | Gb |
| $CH_2OCH_2CH=CH_2$ | H | H | Me | H | O | H | Gb |

| $R_4$ | $R_3$ | $R_2$ | $R_1$ | $R_5$ | X | M | Gn |
|---|---|---|---|---|---|---|---|
| $CH_2OCH_2C{\equiv}CH$ | H | H | Me | H | O | H | Gb |
| $CH{=}CHCO_2Me$ | H | H | Me | H | O | H | Ga |
| $CH{=}CHCO_2Et$ | H | H | Me | H | O | H | Ga |
| $CH{=}CHCO_2Pr$-n | H | H | Me | H | O | H | Gb |
| CH=CHCN | H | H | Me | H | O | H | Ga |
| Ph | H | H | Me | H | O | H | Ga |
| $CH_2Ph$ | H | H | Me | H | O | H | Ga |
| OPh | H | H | Me | H | O | H | Ga |
| SPh | H | H | Me | H | O | H | Ga |
| $SO_2Ph$ | H | H | Me | H | O | H | Ga |
| CONHMe | H | H | Me | H | O | H | Ga |
| CONHEt | H | H | Me | H | O | H | Ga |
| $CON(Me)_2$ | H | H | Me | H | O | H | Ga |
| $CON(Et)_2$ | H | H | Me | H | O | H | Ga |
| $CON(Pr\text{-}n)_2$ | H | H | Me | H | O | H | Gb |
| F | H | H | Et | H | O | H | Gb |
| Cl | H | H | Et | H | O | H | Gb |
| Br | H | H | Et | H | O | H | Gb |
| I | H | H | Et | H | O | H | Gc |
| $CH{=}CH_2$ | H | H | Et | H | O | H | Gb |
| $CH_2CH{=}CH_2$ | H | H | Et | H | O | H | Gb |
| $C{\equiv}CH$ | H | H | Et | H | O | H | Gb |
| $CH_2C{\equiv}CH$ | H | H | Et | H | O | H | Gb |
| $CH_2F$ | H | H | Et | H | O | H | Gb |
| $CHF_2$ | H | H | Et | H | O | H | Gb |
| CN | H | H | Et | H | O | H | Gb |
| $NH_2$ | H | H | Et | H | O | H | Gb |
| NHCOMe | H | H | Et | H | O | H | Gb |
| $CH_2NH_2$ | H | H | Et | H | O | H | Gb |
| $CH_2NHCOMe$ | H | H | Et | H | O | H | Gb |
| $CH_2OCHF_2$ | H | H | Et | H | O | H | Gb |
| $CF_3$ | H | H | Et | H | O | H | Gb |
| $NO_2$ | H | H | Et | H | O | H | Gb |
| OMe | H | H | Et | H | O | H | Ga |
| OEt | H | H | Et | H | O | H | Ga |
| OPr-n | H | H | Et | H | O | H | Gb |
| SMe | H | H | Et | H | O | H | Ga |
| SEt | H | H | Et | H | O | H | Ga |
| $SO_2Me$ | H | H | Et | H | O | H | Ga |
| COMe | H | H | Et | H | O | H | Ga |

| $R_4$ | $R_3$ | $R_2$ | $R_1$ | $R_5$ | X | M | Gn |
|---|---|---|---|---|---|---|---|
| COEt | H | H | Et | H | O | H | Ga |
| $CO_2H$ | H | H | Et | H | O | H | Ga |
| $CO_2Me$ | H | H | Et | H | O | H | Ga |
| $CO_2Et$ | H | H | Et | H | O | H | Ga |
| $CO_2Pr$-n | H | H | Et | H | O | H | Gb |
| $CO_2Bu$-n | H | H | Et | H | O | H | Gc |
| $CH_2Cl$ | H | H | Et | H | O | H | Ga |
| $CH_2Br$ | H | H | Et | H | O | H | Gb |
| $CH_2I$ | H | H | Et | H | O | H | Gb |
| $CH_2CF_3$ | H | H | Et | H | O | H | Gb |
| $CH_2CN$ | H | H | Et | H | O | H | Ga |
| $CH_2OH$ | H | H | Et | H | O | H | Gb |
| $CH_2OMe$ | H | H | Et | H | O | H | Ga |
| $CH_2OEt$ | H | H | Et | H | O | H | Ga |
| $CH_2OPr$-n | H | H | Et | H | O | H | Gb |
| $CH_2SMe$ | H | H | Et | H | O | H | Ga |
| $CH_2SEt$ | H | H | Et | H | O | H | Ga |
| $CH_2SO_2Me$ | H | H | Et | H | O | H | Ga |
| $CH_2SO_2Et$ | H | H | Et | H | O | H | Ga |
| $CH_2SPr$-n | H | H | Et | H | O | H | Gb |
| $CH_2SPr$-i | H | H | Et | H | O | H | Gb |
| $CH_2COMe$ | H | H | Et | H | O | H | Ga |
| $CH_2COEt$ | H | H | Et | H | O | H | Ga |
| $CH_2COPr$-n | H | H | Et | H | O | H | Gb |
| $CH_2CO_2H$ | H | H | Et | H | O | H | Ga |
| $CH_2CO_2Me$ | H | H | Et | H | O | H | Ga |
| $CH_2CO_2Et$ | H | H | Et | H | O | H | Ga |
| $CH_2CO_2Pr$-n | H | H | Et | H | O | H | Gb |
| $CH_2OCH_2CH=CH_2$ | H | H | Et | H | O | H | Gb |
| $CH_2OCH_2C\equiv CH$ | H | H | Et | H | O | H | Gb |
| $CH=CHCO_2Me$ | H | H | Et | H | O | H | Ga |
| $CH=CHCO_2Et$ | H | H | Et | H | O | H | Ga |
| $CH=CHCO_2Pr$-n | H | H | Et | H | O | H | Gb |
| CH=CHCN | H | H | Et | H | O | H | Ga |
| Ph | H | H | Et | H | O | H | Ga |
| $CH_2Ph$ | H | H | Et | H | O | H | Ga |
| OPh | H | H | Et | H | O | H | Ga |
| SPh | H | H | Et | H | O | H | Ga |
| $SO_2Ph$ | H | H | Et | H | O | H | Ga |
| CONHMe | H | H | Et | H | O | H | Ga |

| $R_4$ | $R_3$ | $R_2$ | $R_1$ | $R_5$ | X | M | Gn |
|---|---|---|---|---|---|---|---|
| CONHEt | H | H | Et | H | O | H | Ga |
| $CON(Me)_2$ | H | H | Et | H | O | H | Ga |
| $CON(Et)_2$ | H | H | Et | H | O | H | Ga |
| $CON(Pr\text{-}n)_2$ | H | H | Et | H | O | H | Gb |
| F | H | H | Cl | H | O | H | Gb |
| Cl | H | H | Cl | H | O | H | Gb |
| Br | H | H | Cl | H | O | H | Gb |
| I | H | H | Cl | H | O | H | Gc |
| $CH=CH_2$ | H | H | Cl | H | O | H | Gb |
| $CH_2CH=CH_2$ | H | H | Cl | H | O | H | Gb |
| $C\equiv CH$ | H | H | Cl | H | O | H | Gb |
| $CH_2C\equiv CH$ | H | H | Cl | H | O | H | Gb |
| $CH_2F$ | H | H | Cl | H | O | H | Gb |
| $CHF_2$ | H | H | Cl | H | O | H | Gb |
| CN | H | H | Cl | H | O | H | Gb |
| $NH_2$ | H | H | Cl | H | O | H | Gb |
| NHCOMe | H | H | Cl | H | O | H | Gb |
| $CH_2NH_2$ | H | H | Cl | H | O | H | Gb |
| $CH_2NHCOMe$ | H | H | Cl | H | O | H | Gb |
| $CH_2OCHF_2$ | H | H | Cl | H | O | H | Gb |
| $CF_3$ | H | H | Cl | H | O | H | Gb |
| $NO_2$ | H | H | Cl | H | O | H | Gb |
| OMe | H | H | Cl | H | O | H | Ga |
| OEt | H | H | Cl | H | O | H | Ga |
| OPr-n | H | H | Cl | H | O | H | Gb |
| SMe | H | H | Cl | H | O | H | Ga |
| SEt | H | H | Cl | H | O | H | Ga |
| $SO_2Me$ | H | H | Cl | H | O | H | Ga |
| COMe | H | H | Cl | H | O | H | Ga |
| COEt | H | H | Cl | H | O | H | Ga |
| $CO_2H$ | H | H | Cl | H | O | H | Ga |
| $CO_2Me$ | H | H | Cl | H | O | H | Ga |
| $CO_2Et$ | H | H | Cl | H | O | H | Ga |
| $CO_2Pr\text{-}n$ | H | H | Cl | H | O | H | Gb |
| $CO_2Bu\text{-}n$ | H | H | Cl | H | O | H | Gc |
| $CH_2Cl$ | H | H | Cl | H | O | H | Ga |
| $CH_2Br$ | H | H | Cl | H | O | H | Gb |
| $CH_2I$ | H | H | Cl | H | O | H | Gb |
| $CH_2CF_3$ | H | H | Cl | H | O | H | Gb |
| $CH_2CN$ | H | H | Cl | H | O | H | Ga |

| $R_4$ | $R_3$ | $R_2$ | $R_1$ | $R_5$ | X | M | Gn |
|---|---|---|---|---|---|---|---|
| $CH_2OH$ | H | H | Cl | H | O | H | Gb |
| $CH_2OMe$ | H | H | Cl | H | O | H | Ga |
| $CH_2OEt$ | H | H | Cl | H | O | H | Ga |
| $CH_2OPr$-n | H | H | Cl | H | O | H | Gb |
| $CH_2SMe$ | H | H | Cl | H | O | H | Ga |
| $CH_2SEt$ | H | H | Cl | H | O | H | Ga |
| $CH_2SO_2Me$ | H | H | Cl | H | O | H | Ga |
| $CH_2SO_2Et$ | H | H | Cl | H | O | H | Ga |
| $CH_2SPr$-n | H | H | Cl | H | O | H | Gb |
| $CH_2SPr$-i | H | H | Cl | H | O | H | Gb |
| $CH_2COMe$ | H | H | Cl | H | O | H | Ga |
| $CH_2COEt$ | H | H | Cl | H | O | H | Ga |
| $CH_2COPr$-n | H | H | Cl | H | O | H | Gb |
| $CH_2CO_2H$ | H | H | Cl | H | O | H | Ga |
| $CH_2CO_2Me$ | H | H | Cl | H | O | H | Ga |
| $CH_2CO_2Et$ | H | H | Cl | H | O | H | Ga |
| $CH_2CO_2Pr$-n | H | H | Cl | H | O | H | Gb |
| $CH_2OCH_2CH=CH_2$ | H | H | Cl | H | O | H | Gb |
| $CH_2OCH_2C\equiv CH$ | H | H | Cl | H | O | H | Gb |
| $CH=CHCO_2Me$ | H | H | Cl | H | O | H | Ga |
| $CH=CHCO_2Et$ | H | H | Cl | H | O | H | Ga |
| $CH=CHCO_2Pr$-n | H | H | Cl | H | O | H | Gb |
| CH=CHCN | H | H | Cl | H | O | H | Ga |
| Ph | H | H | Cl | H | O | H | Ga |
| $CH_2Ph$ | H | H | Cl | H | O | H | Ga |
| OPh | H | H | Cl | H | O | H | Ga |
| SPh | H | H | Cl | H | O | H | Ga |
| $SO_2Ph$ | H | H | Cl | H | O | H | Ga |
| CONHMe | H | H | Cl | H | O | H | Ga |
| CONHEt | H | H | Cl | H | O | H | Ga |
| $CON(Me)_2$ | H | H | Cl | H | O | H | Ga |
| $CON(Et)_2$ | H | H | Cl | H | O | H | Ga |
| $CON(Pr$-n$)_2$ | H | H | Cl | H | O | H | Gb |
| F | H | H | $CO_2Me$ | H | O | H | Gb |
| Cl | H | H | $CO_2Me$ | H | O | H | Gb |
| Br | H | H | $CO_2Me$ | H | O | H | Gb |
| I | H | H | $CO_2Me$ | H | O | H | Gc |
| $CH=CH_2$ | H | H | $CO_2Me$ | H | O | H | Gb |
| $CH_2CH=CH_2$ | H | H | $CO_2Me$ | H | O | H | Gb |
| $C\equiv CH$ | H | H | $CO_2Me$ | H | O | H | Gb |

| $R_4$ | $R_3$ | $R_2$ | $R_1$ | $R_5$ | X | M | Gn |
|---|---|---|---|---|---|---|---|
| $CH_2C{\equiv}CH$ | H | H | $CO_2Me$ | H | O | H | Gb |
| $CH_2F$ | H | H | $CO_2Me$ | H | O | H | Gb |
| $CHF_2$ | H | H | $CO_2Me$ | H | O | H | Gb |
| CN | H | H | $CO_2Me$ | H | O | H | Gb |
| $NH_2$ | H | H | $CO_2Me$ | H | O | H | Gb |
| NHCOMe | H | H | $CO_2Me$ | H | O | H | Gb |
| $CH_2NH_2$ | H | H | $CO_2Me$ | H | O | H | Gb |
| $CH_2NHCOMe$ | H | H | $CO_2Me$ | H | O | H | Gb |
| $CH_2OCHF_2$ | H | H | $CO_2Me$ | H | O | H | Gb |
| $CF_3$ | H | H | $CO_2Me$ | H | O | H | Gb |
| $NO_2$ | H | H | $CO_2Me$ | H | O | H | Gb |
| OMe | H | H | $CO_2Me$ | H | O | H | Ga |
| OEt | H | H | $CO_2Me$ | H | O | H | Ga |
| OPr-n | H | H | $CO_2Me$ | H | O | H | Gb |
| SMe | H | H | $CO_2Me$ | H | O | H | Ga |
| SEt | H | H | $CO_2Me$ | H | O | H | Ga |
| $SO_2Me$ | H | H | $CO_2Me$ | H | O | H | Ga |
| COMe | H | H | $CO_2Me$ | H | O | H | Ga |
| COEt | H | H | $CO_2Me$ | H | O | H | Ga |
| $CO_2H$ | H | H | $CO_2Me$ | H | O | H | Ga |
| $CO_2Me$ | H | H | $CO_2Me$ | H | O | H | Ga |
| $CO_2Et$ | H | H | $CO_2Me$ | H | O | H | Ga |
| $CO_2Pr$-n | H | H | $CO_2Me$ | H | O | H | Gb |
| $CO_2Bu$-n | H | H | $CO_2Me$ | H | O | H | Gc |
| $CH_2Cl$ | H | H | $CO_2Me$ | H | O | H | Ga |
| $CH_2Br$ | H | H | $CO_2Me$ | H | O | H | Gb |
| $CH_2I$ | H | H | $CO_2Me$ | H | O | H | Gb |
| $CH_2CF_3$ | H | H | $CO_2Me$ | H | O | H | Gb |
| $CH_2CN$ | H | H | $CO_2Me$ | H | O | H | Ga |
| $CH_2OH$ | H | H | $CO_2Me$ | H | O | H | Gb |
| $CH_2OMe$ | H | H | $CO_2Me$ | H | O | H | Ga |
| $CH_2OEt$ | H | H | $CO_2Me$ | H | O | H | Ga |
| $CH_2OPr$-n | H | H | $CO_2Me$ | H | O | H | Gb |
| $CH_2SMe$ | H | H | $CO_2Me$ | H | O | H | Ga |
| $CH_2SEt$ | H | H | $CO_2Me$ | H | O | H | Ga |
| $CH_2SO_2Me$ | H | H | $CO_2Me$ | H | O | H | Ga |
| $CH_2SO_2Et$ | H | H | $CO_2Me$ | H | O | H | Ga |
| $CH_2SPr$-n | H | H | $CO_2Me$ | H | O | H | Gb |
| $CH_2SPr$-i | H | H | $CO_2Me$ | H | O | H | Gb |
| $CH_2COMe$ | H | H | $CO_2Me$ | H | O | H | Ga |

| $R_4$ | $R_3$ | $R_2$ | $R_1$ | $R_5$ | X | M | Gn |
|---|---|---|---|---|---|---|---|
| $CH_2COEt$ | H | H | $CO_2Me$ | H | O | H | Ga |
| $CH_2COPr$-n | H | H | $CO_2Me$ | H | O | H | Gb |
| $CH_2CO_2H$ | H | H | $CO_2Me$ | H | O | H | Ga |
| $CH_2CO_2Me$ | H | H | $CO_2Me$ | H | O | H | Ga |
| $CH_2CO_2Et$ | H | H | $CO_2Me$ | H | O | H | Ga |
| $CH_2CO_2Pr$-n | H | H | $CO_2Me$ | H | O | H | Gb |
| $CH_2OCH_2CH=CH_2$ | H | H | $CO_2Me$ | H | O | H | Gb |
| $CH_2OCH_2C\equiv CH$ | H | H | $CO_2Me$ | H | O | H | Gb |
| $CH=CHCO_2Me$ | H | H | $CO_2Me$ | H | O | H | Ga |
| $CH=CHCO_2Et$ | H | H | $CO_2Me$ | H | O | H | Ga |
| $CH=CHCO_2Pr$-n | H | H | $CO_2Me$ | H | O | H | Gb |
| $CH=CHCN$ | H | H | $CO_2Me$ | H | O | H | Ga |
| Ph | H | H | $CO_2Me$ | H | O | H | Ga |
| $CH_2Ph$ | H | H | $CO_2Me$ | H | O | H | Ga |
| OPh | H | H | $CO_2Me$ | H | O | H | Ga |
| SPh | H | H | $CO_2Me$ | H | O | H | Ga |
| $SO_2Ph$ | H | H | $CO_2Me$ | H | O | H | Ga |
| CONHMe | H | H | $CO_2Me$ | H | O | H | Ga |
| CONHEt | H | H | $CO_2Me$ | H | O | H | Ga |
| $CON(Me)_2$ | H | H | $CO_2Me$ | H | O | H | Ga |
| $CON(Et)_2$ | H | H | $CO_2Me$ | H | O | H | Ga |
| $CON(Pr$-n$)_2$ | H | H | $CO_2Me$ | H | O | H | Gb |
| F | H | H | $CH_2Cl$ | H | O | H | Gb |
| Cl | H | H | $CH_2Cl$ | H | O | H | Gb |
| Br | H | H | $CH_2Cl$ | H | O | H | Gb |
| I | H | H | $CH_2Cl$ | H | O | H | Gc |
| $CH=CH_2$ | H | H | $CH_2Cl$ | H | O | H | Gb |
| $CH_2CH=CH_2$ | H | H | $CH_2Cl$ | H | O | H | Gb |
| $C\equiv CH$ | H | H | $CH_2Cl$ | H | O | H | Gb |
| $CH_2C\equiv CH$ | H | H | $CH_2Cl$ | H | O | H | Gb |
| $CH_2F$ | H | H | $CH_2Cl$ | H | O | H | Gb |
| $CHF_2$ | H | H | $CH_2Cl$ | H | O | H | Gb |
| CN | H | H | $CH_2Cl$ | H | O | H | Gb |
| $NH_2$ | H | H | $CH_2Cl$ | H | O | H | Gb |
| NHCOMe | H | H | $CH_2Cl$ | H | O | H | Gb |
| $CH_2NH_2$ | H | H | $CH_2Cl$ | H | O | H | Gb |
| $CH_2NHCOMe$ | H | H | $CH_2Cl$ | H | O | H | Gb |
| $CH_2OCHF_2$ | H | H | $CH_2Cl$ | H | O | H | Gb |
| $CF_3$ | H | H | $CH_2Cl$ | H | O | H | Gb |
| $NO_2$ | H | H | $CH_2Cl$ | H | O | H | Gb |

| $R_4$ | $R_3$ | $R_2$ | $R_1$ | $R_5$ | X | M | Gn |
|---|---|---|---|---|---|---|---|
| OMe | H | H | $CH_2Cl$ | H | O | H | Ga |
| OEt | H | H | $CH_2Cl$ | H | O | H | Ga |
| OPr-n | H | H | $CH_2Cl$ | H | O | H | Gb |
| SMe | H | H | $CH_2Cl$ | H | O | H | Ga |
| SEt | H | H | $CH_2Cl$ | H | O | H | Ga |
| $SO_2Me$ | H | H | $CH_2Cl$ | H | O | H | Ga |
| COMe | H | H | $CH_2Cl$ | H | O | H | Ga |
| COEt | H | H | $CH_2Cl$ | H | O | H | Ga |
| $CO_2H$ | H | H | $CH_2Cl$ | H | O | H | Ga |
| $CO_2Me$ | H | H | $CH_2Cl$ | H | O | H | Ga |
| $CO_2Et$ | H | H | $CH_2Cl$ | H | O | H | Ga |
| $CO_2Pr$-n | H | H | $CH_2Cl$ | H | O | H | Gb |
| $CO_2Bu$-n | H | H | $CH_2Cl$ | H | O | H | Gc |
| $CH_2Cl$ | H | H | $CH_2Cl$ | H | O | H | Ga |
| $CH_2Br$ | H | H | $CH_2Cl$ | H | O | H | Gb |
| $CH_2I$ | H | H | $CH_2Cl$ | H | O | H | Gb |
| $CH_2CF_3$ | H | H | $CH_2Cl$ | H | O | H | Gb |
| $CH_2CN$ | H | H | $CH_2Cl$ | H | O | H | Ga |
| $CH_2OH$ | H | H | $CH_2Cl$ | H | O | H | Gb |
| $CH_2OMe$ | H | H | $CH_2Cl$ | H | O | H | Ga |
| $CH_2OEt$ | H | H | $CH_2Cl$ | H | O | H | Ga |
| $CH_2OPr$-n | H | H | $CH_2Cl$ | H | O | H | Gb |
| $CH_2SMe$ | H | H | $CH_2Cl$ | H | O | H | Ga |
| $CH_2SEt$ | H | H | $CH_2Cl$ | H | O | H | Ga |
| $CH_2SO_2Me$ | H | H | $CH_2Cl$ | H | O | H | Ga |
| $CH_2SO_2Et$ | H | H | $CH_2Cl$ | H | O | H | Ga |
| $CH_2SPr$-n | H | H | $CH_2Cl$ | H | O | H | Gb |
| $CH_2SPr$-i | H | H | $CH_2Cl$ | H | O | H | Gb |
| $CH_2COMe$ | H | H | $CH_2Cl$ | H | O | H | Ga |
| $CH_2COEt$ | H | H | $CH_2Cl$ | H | O | H | Ga |
| $CH_2COPr$-n | H | H | $CH_2Cl$ | H | O | H | Gb |
| $CH_2CO_2H$ | H | H | $CH_2Cl$ | H | O | H | Ga |
| $CH_2CO_2Me$ | H | H | $CH_2Cl$ | H | O | H | Ga |
| $CH_2CO_2Et$ | H | H | $CH_2Cl$ | H | O | H | Ga |
| $CH_2CO_2Pr$-n | H | H | $CH_2Cl$ | H | O | H | Gb |
| $CH_2OCH_2CH=CH_2$ | H | H | $CH_2Cl$ | H | O | H | Gb |
| $CH_2OCH_2C\equiv CH$ | H | H | $CH_2Cl$ | H | O | H | Gb |
| $CH=CHCO_2Me$ | H | H | $CH_2Cl$ | H | O | H | Ga |
| $CH=CHCO_2Et$ | H | H | $CH_2Cl$ | H | O | H | Ga |
| $CH=CHCO_2Pr$-n | H | H | $CH_2Cl$ | H | O | H | Gb |

| $R_4$ | $R_3$ | $R_2$ | $R_1$ | $R_5$ | X | M | Gn |
|---|---|---|---|---|---|---|---|
| CH=CHCN | H | H | $CH_2Cl$ | H | O | H | Ga |
| Ph | H | H | $CH_2Cl$ | H | O | H | Ga |
| $CH_2Ph$ | H | H | $CH_2Cl$ | H | O | H | Ga |
| OPh | H | H | $CH_2Cl$ | H | O | H | Ga |
| SPh | H | H | $CH_2Cl$ | H | O | H | Ga |
| $SO_2Ph$ | H | H | $CH_2Cl$ | H | O | H | Ga |
| CONHMe | H | H | $CH_2Cl$ | H | O | H | Ga |
| CONHEt | H | H | $CH_2Cl$ | H | O | H | Ga |
| $CON(Me)_2$ | H | H | $CH_2Cl$ | H | O | H | Ga |
| $CON(Et)_2$ | H | H | $CH_2Cl$ | H | O | H | Ga |
| $CON(Pr-n)_2$ | H | H | $CH_2Cl$ | H | O | H | Gb |
| F | H | H | $CH_2OMe$ | H | O | H | Gb |
| Cl | H | H | $CH_2OMe$ | H | O | H | Gb |
| Br | H | H | $CH_2OMe$ | H | O | H | Gb |
| I | H | H | $CH_2OMe$ | H | O | H | Gc |
| $CH=CH_2$ | H | H | $CH_2OMe$ | H | O | H | Gb |
| $CH_2CH=CH_2$ | H | H | $CH_2OMe$ | H | O | H | Gb |
| $C\equiv CH$ | H | H | $CH_2OMe$ | H | O | H | Gb |
| $CH_2C\equiv CH$ | H | H | $CH_2OMe$ | H | O | H | Gb |
| $CH_2F$ | H | H | $CH_2OMe$ | H | O | H | Gb |
| $CHF_2$ | H | H | $CH_2OMe$ | H | O | H | Gb |
| CN | H | H | $CH_2OMe$ | H | O | H | Gb |
| $NH_2$ | H | H | $CH_2OMe$ | H | O | H | Gb |
| NHCOMe | H | H | $CH_2OMe$ | H | O | H | Gb |
| $CH_2NH_2$ | H | H | $CH_2OMe$ | H | O | H | Gb |
| $CH_2NHCOMe$ | H | H | $CH_2OMe$ | H | O | H | Gb |
| $CH_2OCHF_2$ | H | H | $CH_2OMe$ | H | O | H | Gb |
| $CF_3$ | H | H | $CH_2OMe$ | H | O | H | Gb |
| $NO_2$ | H | H | $CH_2OMe$ | H | O | H | Gb |
| OMe | H | H | $CH_2OMe$ | H | O | H | Ga |
| OEt | H | H | $CH_2OMe$ | H | O | H | Ga |
| OPr-n | H | H | $CH_2OMe$ | H | O | H | Gb |
| SMe | H | H | $CH_2OMe$ | H | O | H | Ga |
| SEt | H | H | $CH_2OMe$ | H | O | H | Ga |
| $SO_2Me$ | H | H | $CH_2OMe$ | H | O | H | Ga |
| COMe | H | H | $CH_2OMe$ | H | O | H | Ga |
| COEt | H | H | $CH_2OMe$ | H | O | H | Ga |
| $CO_2H$ | H | H | $CH_2OMe$ | H | O | H | Ga |
| $CO_2Me$ | H | H | $CH_2OMe$ | H | O | H | Ga |
| $CO_2Et$ | H | H | $CH_2OMe$ | H | O | H | Ga |

| $R_4$ | $R_3$ | $R_2$ | $R_1$ | $R_5$ | X | M | Gn |
|---|---|---|---|---|---|---|---|
| $CO_2Pr$-n | H | H | $CH_2OMe$ | H | O | H | Gb |
| $CO_2Bu$-n | H | H | $CH_2OMe$ | H | O | H | Gc |
| $CH_2Cl$ | H | H | $CH_2OMe$ | H | O | H | Ga |
| $CH_2Br$ | H | H | $CH_2OMe$ | H | O | H | Gb |
| $CH_2I$ | H | H | $CH_2OMe$ | H | O | H | Gb |
| $CH_2CF_3$ | H | H | $CH_2OMe$ | H | O | H | Gb |
| $CH_2CN$ | H | H | $CH_2OMe$ | H | O | H | Ga |
| $CH_2OH$ | H | H | $CH_2OMe$ | H | O | H | Gb |
| $CH_2OMe$ | H | H | $CH_2OMe$ | H | O | H | Ga |
| $CH_2OEt$ | H | H | $CH_2OMe$ | H | O | H | Ga |
| $CH_2OPr$-n | H | H | $CH_2OMe$ | H | O | H | Gb |
| $CH_2SMe$ | H | H | $CH_2OMe$ | H | O | H | Ga |
| $CH_2SEt$ | H | H | $CH_2OMe$ | H | O | H | Ga |
| $CH_2SO_2Me$ | H | H | $CH_2OMe$ | H | O | H | Ga |
| $CH_2SO_2Et$ | H | H | $CH_2OMe$ | H | O | H | Ga |
| $CH_2SPr$-n | H | H | $CH_2OMe$ | H | O | H | Gb |
| $CH_2SPr$-i | H | H | $CH_2OMe$ | H | O | H | Gb |
| $CH_2COMe$ | H | H | $CH_2OMe$ | H | O | H | Ga |
| $CH_2COEt$ | H | H | $CH_2OMe$ | H | O | H | Ga |
| $CH_2COPr$-n | H | H | $CH_2OMe$ | H | O | H | Gb |
| $CH_2CO_2H$ | H | H | $CH_2OMe$ | H | O | H | Ga |
| $CH_2CO_2Me$ | H | H | $CH_2OMe$ | H | O | H | Ga |
| $CH_2CO_2Et$ | H | H | $CH_2OMe$ | H | O | H | Ga |
| $CH_2CO_2Pr$-n | H | H | $CH_2OMe$ | H | O | H | Gb |
| $CH_2OCH_2CH=CH_2$ | H | H | $CH_2OMe$ | H | O | H | Gb |
| $CH_2OCH_2C\equiv CH$ | H | H | $CH_2OMe$ | H | O | H | Gb |
| $CH=CHCO_2Me$ | H | H | $CH_2OMe$ | H | O | H | Ga |
| $CH=CHCO_2Et$ | H | H | $CH_2OMe$ | H | O | H | Ga |
| $CH=CHCO_2Pr$-n | H | H | $CH_2OMe$ | H | O | H | Gb |
| $CH=CHCN$ | H | H | $CH_2OMe$ | H | O | H | Ga |
| Ph | H | H | $CH_2OMe$ | H | O | H | Ga |
| $CH_2Ph$ | H | H | $CH_2OMe$ | H | O | H | Ga |
| OPh | H | H | $CH_2OMe$ | H | O | H | Ga |
| SPh | H | H | $CH_2OMe$ | H | O | H | Ga |
| $SO_2Ph$ | H | H | $CH_2OMe$ | H | O | H | Ga |
| CONHMe | H | H | $CH_2OMe$ | H | O | H | Ga |
| CONHEt | H | H | $CH_2OMe$ | H | O | H | Ga |
| $CON(Me)_2$ | H | H | $CH_2OMe$ | H | O | H | Ga |
| $CON(Et)_2$ | H | H | $CH_2OMe$ | H | O | H | Ga |
| $CON(Pr$-n$)_2$ | H | H | $CH_2OMe$ | H | O | H | Gb |

| $R_4$ | $R_3$ | $R_2$ | $R_1$ | $R_5$ | X | M | Gn |
|---|---|---|---|---|---|---|---|
| F | H | H | $CH_2SMe$ | H | O | H | Gb |
| Cl | H | H | $CH_2SMe$ | H | O | H | Gb |
| Br | H | H | $CH_2SMe$ | H | O | H | Gb |
| I | H | H | $CH_2SMe$ | H | O | H | Gc |
| $CH=CH_2$ | H | H | $CH_2SMe$ | H | O | H | Gb |
| $CH_2CH=CH_2$ | H | H | $CH_2SMe$ | H | O | H | Gb |
| $C\equiv CH$ | H | H | $CH_2SMe$ | H | O | H | Gb |
| $CH_2C\equiv CH$ | H | H | $CH_2SMe$ | H | O | H | Gb |
| $CH_2F$ | H | H | $CH_2SMe$ | H | O | H | Gb |
| $CHF_2$ | H | H | $CH_2SMe$ | H | O | H | Gb |
| CN | H | H | $CH_2SMe$ | H | O | H | Gb |
| $NH_2$ | H | H | $CH_2SMe$ | H | O | H | Gb |
| NHCOMe | H | H | $CH_2SMe$ | H | O | H | Gb |
| $CH_2NH_2$ | H | H | $CH_2SMe$ | H | O | H | Gb |
| $CH_2NHCOMe$ | H | H | $CH_2SMe$ | H | O | H | Gb |
| $CH_2OCHF_2$ | H | H | $CH_2SMe$ | H | O | H | Gb |
| $CF_3$ | H | H | $CH_2SMe$ | H | O | H | Gb |
| $NO_2$ | H | H | $CH_2SMe$ | H | O | H | Gb |
| OMe | H | H | $CH_2SMe$ | H | O | H | Ga |
| OEt | H | H | $CH_2SMe$ | H | O | H | Ga |
| OPr-n | H | H | $CH_2SMe$ | H | O | H | Gb |
| SMe | H | H | $CH_2SMe$ | H | O | H | Ga |
| SEt | H | H | $CH_2SMe$ | H | O | H | Ga |
| $SO_2Me$ | H | H | $CH_2SMe$ | H | O | H | Ga |
| COMe | H | H | $CH_2SMe$ | H | O | H | Ga |
| COEt | H | H | $CH_2SMe$ | H | O | H | Ga |
| $CO_2H$ | H | H | $CH_2SMe$ | H | O | H | Ga |
| $CO_2Me$ | H | H | $CH_2SMe$ | H | O | H | Ga |
| $CO_2Et$ | H | H | $CH_2SMe$ | H | O | H | Ga |
| $CO_2Pr$-n | H | H | $CH_2SMe$ | H | O | H | Gb |
| $CO_2Bu$-n | H | H | $CH_2SMe$ | H | O | H | Gc |
| $CH_2Cl$ | H | H | $CH_2SMe$ | H | O | H | Ga |
| $CH_2Br$ | H | H | $CH_2SMe$ | H | O | H | Gb |
| $CH_2I$ | H | H | $CH_2SMe$ | H | O | H | Gb |
| $CH_2CF_3$ | H | H | $CH_2SMe$ | H | O | H | Gb |
| $CH_2CN$ | H | H | $CH_2SMe$ | H | O | H | Ga |
| $CH_2OH$ | H | H | $CH_2SMe$ | H | O | H | Gb |
| $CH_2OMe$ | H | H | $CH_2SMe$ | H | O | H | Ga |
| $CH_2OEt$ | H | H | $CH_2SMe$ | H | O | H | Ga |
| $CH_2OPr$-n | H | H | $CH_2SMe$ | H | O | H | Gb |

| $R_4$ | $R_3$ | $R_2$ | $R_1$ | $R_5$ | X | M | Gn |
|---|---|---|---|---|---|---|---|
| $CH_2SMe$ | H | H | $CH_2SMe$ | H | O | H | Ga |
| $CH_2SEt$ | H | H | $CH_2SMe$ | H | O | H | Ga |
| $CH_2SO_2Me$ | H | H | $CH_2SMe$ | H | O | H | Ga |
| $CH_2SO_2Et$ | H | H | $CH_2SMe$ | H | O | H | Ga |
| $CH_2SPr$-n | H | H | $CH_2SMe$ | H | O | H | Gb |
| $CH_2SPr$-i | H | H | $CH_2SMe$ | H | O | H | Gb |
| $CH_2COMe$ | H | H | $CH_2SMe$ | H | O | H | Ga |
| $CH_2COEt$ | H | H | $CH_2SMe$ | H | O | H | Ga |
| $CH_2COPr$-n | H | H | $CH_2SMe$ | H | O | H | Gb |
| $CH_2CO_2H$ | H | H | $CH_2SMe$ | H | O | H | Ga |
| $CH_2CO_2Me$ | H | H | $CH_2SMe$ | H | O | H | Ga |
| $CH_2CO_2Et$ | H | H | $CH_2SMe$ | H | O | H | Ga |
| $CH_2CO_2Pr$-n | H | H | $CH_2SMe$ | H | O | H | Gb |
| $CH_2OCH_2CH=CH_2$ | H | H | $CH_2SMe$ | H | O | H | Gb |
| $CH_2OCH_2C\equiv CH$ | H | H | $CH_2SMe$ | H | O | H | Gb |
| $CH=CHCO_2Me$ | H | H | $CH_2SMe$ | H | O | H | Ga |
| $CH=CHCO_2Et$ | H | H | $CH_2SMe$ | H | O | H | Ga |
| $CH=CHCO_2Pr$-n | H | H | $CH_2SMe$ | H | O | H | Gb |
| CH=CHCN | H | H | $CH_2SMe$ | H | O | H | Ga |
| Ph | H | H | $CH_2SMe$ | H | O | H | Ga |
| $CH_2Ph$ | H | H | $CH_2SMe$ | H | O | H | Ga |
| OPh | H | H | $CH_2SMe$ | H | O | H | Ga |
| SPh | H | H | $CH_2SMe$ | H | O | H | Ga |
| $SO_2Ph$ | H | H | $CH_2SMe$ | H | O | H | Ga |
| CONHMe | H | H | $CH_2SMe$ | H | O | H | Ga |
| CONHEt | H | H | $CH_2SMe$ | H | O | H | Ga |
| $CON(Me)_2$ | H | H | $CH_2SMe$ | H | O | H | Ga |
| $CON(Et)_2$ | H | H | $CH_2SMe$ | H | O | H | Ga |
| $CON(Pr\text{-}n)_2$ | H | H | $CH_2SMe$ | H | O | H | Gb |
| F | H | H | CN | H | O | H | Gb |
| Cl | H | H | CN | H | O | H | Gb |
| Br | H | H | CN | H | O | H | Gb |
| I | H | H | CN | H | O | H | Gc |
| $CH=CH_2$ | H | H | CN | H | O | H | Gb |
| $CH_2CH=CH_2$ | H | H | CN | H | O | H | Gb |
| $C\equiv CH$ | H | H | CN | H | O | H | Gb |
| $CH_2C\equiv CH$ | H | H | CN | H | O | H | Gb |
| $CH_2F$ | H | H | CN | H | O | H | Gb |
| $CHF_2$ | H | H | CN | H | O | H | Gb |
| CN | H | H | CN | H | O | H | Gb |

| $R_4$ | $R_3$ | $R_2$ | $R_1$ | $R_5$ | X | M | Gn |
|---|---|---|---|---|---|---|---|
| $NH_2$ | H | H | CN | H | O | H | Gb |
| NHCOMe | H | H | CN | H | O | H | Gb |
| $CH_2NH_2$ | H | H | CN | H | O | H | Gb |
| $CH_2NHCOMe$ | H | H | CN | H | O | H | Gb |
| $CH_2OCHF_2$ | H | H | CN | H | O | H | Gb |
| $CF_3$ | H | H | CN | H | O | H | Gb |
| $NO_2$ | H | H | CN | H | O | H | Gb |
| OMe | H | H | CN | H | O | H | Ga |
| OEt | H | H | CN | H | O | H | Ga |
| OPr-n | H | H | CN | H | O | H | Gb |
| SMe | H | H | CN | H | O | H | Ga |
| SEt | H | H | CN | H | O | H | Ga |
| $SO_2Me$ | H | H | CN | H | O | H | Ga |
| COMe | H | H | CN | H | O | H | Ga |
| COEt | H | H | CN | H | O | H | Ga |
| $CO_2H$ | H | H | CN | H | O | H | Ga |
| $CO_2Me$ | H | H | CN | H | O | H | Ga |
| $CO_2Et$ | H | H | CN | H | O | H | Ga |
| $CO_2Pr$-n | H | H | CN | H | O | H | Gb |
| $CO_2Bu$-n | H | H | CN | H | O | H | Gc |
| $CH_2Cl$ | H | H | CN | H | O | H | Ga |
| $CH_2Br$ | H | H | CN | H | O | H | Gb |
| $CH_2I$ | H | H | CN | H | O | H | Gb |
| $CH_2CF_3$ | H | H | CN | H | O | H | Gb |
| $CH_2CN$ | H | H | CN | H | O | H | Ga |
| $CH_2OH$ | H | H | CN | H | O | H | Gb |
| $CH_2OMe$ | H | H | CN | H | O | H | Ga |
| $CH_2OEt$ | H | H | CN | H | O | H | Ga |
| $CH_2OPr$-n | H | H | CN | H | O | H | Gb |
| $CH_2SMe$ | H | H | CN | H | O | H | Ga |
| $CH_2SEt$ | H | H | CN | H | O | H | Ga |
| $CH_2SO_2Me$ | H | H | CN | H | O | H | Ga |
| $CH_2SO_2Et$ | H | H | CN | H | O | H | Ga |
| $CH_2SPr$-n | H | H | CN | H | O | H | Gb |
| $CH_2SPr$-i | H | H | CN | H | O | H | Gb |
| $CH_2COMe$ | H | H | CN | H | O | H | Ga |
| $CH_2COEt$ | H | H | CN | H | O | H | Ga |
| $CH_2COPr$-n | H | H | CN | H | O | H | Gb |
| $CH_2CO_2H$ | H | H | CN | H | O | H | Ga |
| $CH_2CO_2Me$ | H | H | CN | H | O | H | Ga |

| $R_4$ | $R_3$ | $R_2$ | $R_1$ | $R_5$ | X | M | Gn |
|---|---|---|---|---|---|---|---|
| $CH_2CO_2Et$ | H | H | CN | H | O | H | Ga |
| $CH_2CO_2Pr$-n | H | H | CN | H | O | H | Gb |
| $CH_2OCH_2CH=CH_2$ | H | H | CN | H | O | H | Gb |
| $CH_2OCH_2C\equiv CH$ | H | H | CN | H | O | H | Gb |
| $CH=CHCO_2Me$ | H | H | CN | H | O | H | Ga |
| $CH=CHCO_2Et$ | H | H | CN | H | O | H | Ga |
| $CH=CHCO_2Pr$-n | H | H | CN | H | O | H | Gb |
| CH=CHCN | H | H | CN | H | O | H | Ga |
| Ph | H | H | CN | H | O | H | Ga |
| $CH_2Ph$ | H | H | CN | H | O | H | Ga |
| OPh | H | H | CN | H | O | H | Ga |
| SPh | H | H | CN | H | O | H | Ga |
| $SO_2Ph$ | H | H | CN | H | O | H | Ga |
| CONHMe | H | H | CN | H | O | H | Ga |
| CONHEt | H | H | CN | H | O | H | Ga |
| $CON(Me)_2$ | H | H | CN | H | O | H | Ga |
| $CON(Et)_2$ | H | H | CN | H | O | H | Ga |
| $CON(Pr$-$n)_2$ | H | H | CN | H | O | H | Gb |
| H | H | Cl | Cl | H | O | H | Gb |
| H | Cl | H | Cl | H | O | H | Gb |
| Cl | H | H | Cl | H | O | H | Gb |
| H | Cl | Cl | H | H | O | H | Gb |
| Cl | H | Cl | H | H | O | H | Gb |
| Cl | Cl | H | H | H | O | H | Gb |
| H | H | Cl | Me | H | O | H | Gc |
| H | Cl | H | Me | H | O | H | Gc |
| Me | H | Cl | H | H | O | H | Gc |
| Me | Cl | H | H | H | O | H | Gc |
| H | H | Cl | Et | H | O | H | Gc |
| H | Cl | H | Et | H | O | H | Gc |
| Et | H | Cl | H | H | O | H | Gc |
| Et | Cl | H | H | H | O | H | Gc |
| H | H | Br | Br | H | O | H | Gb |
| H | Br | H | Br | H | O | H | Gb |
| Br | H | H | Br | H | O | H | Gb |
| H | Br | Br | H | H | O | H | Gb |
| Br | H | Br | H | H | O | H | Gb |
| Br | Br | H | H | H | O | H | Gb |

Table 9

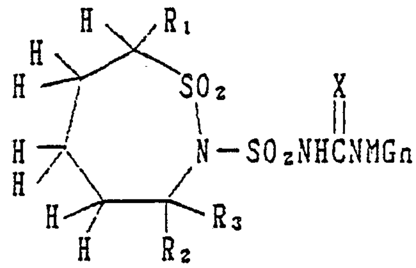

| $R_1$ | $R_2$ | $R_3$ | X | M | Gn |
|---|---|---|---|---|---|
| H | H | H | O | H | Ga |
| H | H | H | S | H | G3 |
| H | H | H | O | Me | Gb |
| H | H | H | O | Et | Gb |
| H | H | H | O | $CH_2CH=CH_2$ | Gb |
| H | H | H | O | $CH_2C\equiv CH$ | Gb |
| Me | H | H | O | H | Ga |
| Me | H | H | S | H | G3 |
| H | Me | H | O | H | Ga |
| H | Me | H | S | H | G3 |
| Et | H | H | O | H | Ga |
| Et | H | H | S | H | G3 |
| H | Et | H | O | H | Ga |
| H | Et | H | S | H | G3 |
| Pr-n | H | H | O | H | Ga |
| Pr-n | H | H | S | H | G3 |
| H | Pr-n | H | O | H | Gb |
| H | Pr-n | H | S | H | G3 |
| Pr-i | H | H | O | H | Gb |
| H | Pr-i | H | O | H | Gb |
| Bu-n | H | H | O | H | Gb |
| H | Bu-n | H | O | H | Gb |
| Me | Me | H | O | H | Ga |
| Me | Me | H | S | H | G3 |
| Me | Et | H | O | H | Ga |
| Me | Et | H | S | H | G3 |
| Me | Pr-n | H | O | H | Gb |
| Me | Pr-i | H | O | H | Gb |
| Et | Me | H | O | H | Gb |

| $R_1$ | $R_2$ | $R_3$ | X | M | Gn |
|---|---|---|---|---|---|
| Et | Me | H | S | H | G3 |
| Et | Et | H | O | H | Gb |
| Et | Et | H | S | H | G3 |
| Et | Pr-n | H | O | H | Gb |
| Et | Pr-i | H | O | H | Gb |
| Pr-n | Me | H | O | H | Gb |
| Pr-n | Et | H | O | H | Gb |
| Pr-n | Pr-n | H | O | H | Gb |
| Pr-n | Pr-i | H | O | H | Gb |
| Pr-i | Me | H | O | H | Gb |
| Pr-i | Et | H | O | H | Gb |
| Pr-i | Pr-n | H | O | H | Gb |
| Pr-i | Pr-i | H | O | H | Gb |
| H | Me | Me | O | H | Ga |
| H | Me | Me | S | H | G3 |
| H | Me | Et | O | H | Ga |
| H | Me | Et | S | H | G3 |
| H | Me | Pr-n | O | H | Gb |
| H | Me | Pr-i | O | H | Gb |
| H | Et | Et | O | H | Gb |
| H | Et | Et | S | H | G3 |
| H | Et | Pr-n | O | H | Gb |
| Me | Me | Me | O | H | Ga |
| Me | Me | Me | S | H | G3 |
| Et | Me | Me | O | H | Ga |
| Et | Me | Me | S | H | G3 |
| Pr-n | Me | Me | O | H | Gb |
| Pr-i | Me | Me | O | H | Gb |
| H | Cl | H | O | H | Ga |
| H | Cl | H | S | H | G3 |
| H | Br | H | O | H | Gb |
| H | Br | H | S | H | G3 |
| H | I | H | O | H | Gb |
| H | I | H | S | H | G3 |
| H | $CF_3$ | H | O | H | Ga |
| H | $CF_3$ | H | S | H | G3 |
| H | $NO_2$ | H | O | H | Ga |
| H | $NO_2$ | H | S | H | G3 |
| H | OMe | H | O | H | Ga |
| H | OMe | H | S | H | G3 |

| $R_1$ | $R_2$ | $R_3$ | X | M | Gn |
|---|---|---|---|---|---|
| H | SMe | H | O | H | Ga |
| H | SMe | H | S | H | G3 |
| H | $SO_2Me$ | H | O | H | Ga |
| H | $SO_2Me$ | H | S | H | G3 |
| H | COMe | H | O | H | Ga |
| H | COMe | H | S | H | G3 |
| H | $CO_2H$ | H | O | H | Ga |
| H | $CO_2H$ | H | S | H | G3 |
| H | $CO_2Me$ | H | O | H | Ga |
| H | $CO_2Me$ | H | S | H | G3 |
| H | $CO_2Et$ | H | O | H | Ga |
| H | $CO_2Et$ | H | S | H | G3 |
| H | $CH_2Cl$ | H | O | H | Ga |
| H | $CH_2Cl$ | H | S | H | G3 |
| H | $CH_2Br$ | H | O | H | Gb |
| H | $CH_2Br$ | H | S | H | G3 |
| H | $CH_2I$ | H | O | H | Gb |
| H | $CH_2I$ | H | S | H | G3 |
| H | $CH_2CF_3$ | H | O | H | Ga |
| H | $CH_2CF_3$ | H | S | H | G3 |
| H | $CH_2CN$ | H | O | H | Ga |
| H | $CH_2CN$ | H | S | H | G3 |
| H | $CH_2OH$ | H | O | H | Gb |
| H | $CH_2OMe$ | H | O | H | Ga |
| H | $CH_2OMe$ | H | S | H | G3 |
| H | $CH_2OEt$ | H | O | H | Ga |
| H | $CH_2OEt$ | H | S | H | G3 |
| H | $CH_2SMe$ | H | O | H | Ga |
| H | $CH_2SMe$ | H | S | H | G3 |
| H | $CH_2SEt$ | H | O | H | Ga |
| H | $CH_2SEt$ | H | S | H | G3 |
| H | $CH_2COMe$ | H | O | H | Ga |
| H | $CH_2COMe$ | H | S | H | G3 |
| H | $CH_2COEt$ | H | O | H | Ga |
| H | $CH_2COEt$ | H | S | H | G3 |
| H | $CH_2CO_2H$ | H | O | H | Ga |
| H | $CH_2CO_2H$ | H | S | H | G3 |
| H | $CH_2CO_2Me$ | H | O | H | Ga |
| H | $CH_2CO_2Me$ | H | S | H | G3 |
| H | $CH_2CO_2Et$ | H | O | H | Ga |

| $R_1$ | $R_2$ | $R_3$ | X | M | Gn |
|---|---|---|---|---|---|
| H | $CH_2CO_2Et$ | H | S | H | G3 |
| H | $CH_2OCH_2CH=CH_2$ | H | O | H | Gb |
| H | $CH_2OCH_2CH=CH_2$ | H | S | H | G3 |
| H | $CH_2OCH_2C\equiv CH$ | H | O | H | Gb |
| H | $CH_2OCH_2C\equiv CH$ | H | S | H | G3 |
| H | $CH=CHCO_2Me$ | H | O | H | Ga |
| H | $CH=CHCO_2Me$ | H | S | H | G3 |
| H | $CH=CHCO_2Et$ | H | O | H | Ga |
| H | $CH=CHCO_2Et$ | H | S | H | G3 |
| H | CH=CHCN | H | O | H | Ga |
| H | CH=CHCN | H | S | H | G3 |
| H | Ph | H | O | H | Ga |
| H | Ph | H | S | H | G3 |
| H | Ph-2-Cl | H | O | H | Ga |
| H | Ph-2-Cl | H | S | H | G3 |
| H | Ph-3-Cl | H | O | H | Gb |
| H | Ph-4-Cl | H | O | H | Gb |
| H | $CH_2Ph$ | H | O | H | Ga |
| H | $CH_2Ph$ | H | S | H | G3 |
| H | $CH_2Ph$-2-Cl | H | O | H | Ga |
| H | $CH_2Ph$-2-Cl | H | S | H | G3 |
| H | $CH_2Ph$-3-Cl | H | O | H | Gb |
| H | $CH_2Ph$-4-Cl | H | O | H | Gb |
| Me | Cl | H | O | H | Ga |
| Me | Cl | H | S | H | G3 |
| Me | Br | H | O | H | Gb |
| Me | Br | H | S | H | G3 |
| Me | I | H | O | H | Gb |
| Me | I | H | S | H | G3 |
| Me | $CF_3$ | H | O | H | Ga |
| Me | $CF_3$ | H | S | H | G3 |
| Me | $NO_2$ | H | O | H | Ga |
| Me | $NO_2$ | H | S | H | G3 |
| Me | OMe | H | O | H | Ga |
| Me | OMe | H | S | H | G3 |
| Me | SMe | H | O | H | Ga |
| Me | SMe | H | S | H | G3 |
| Me | $SO_2Me$ | H | O | H | Ga |
| Me | $SO_2Me$ | H | S | H | G3 |
| Me | COMe | H | O | H | Ga |

| $R_1$ | $R_2$ | $R_3$ | X | M | Gn |
|---|---|---|---|---|---|
| Me | COMe | H | S | H | G3 |
| Me | $CO_2H$ | H | O | H | Ga |
| Me | $CO_2H$ | H | S | H | G3 |
| Me | $CO_2Me$ | H | O | H | Ga |
| Me | $CO_2Me$ | H | S | H | G3 |
| Me | $CO_2Et$ | H | O | H | Ga |
| Me | $CO_2Et$ | H | S | H | G3 |
| Me | $CH_2Cl$ | H | O | H | Ga |
| Me | $CH_2Cl$ | H | S | H | G3 |
| Me | $CH_2Br$ | H | O | H | Gb |
| Me | $CH_2Br$ | H | S | H | G3 |
| Me | $CH_2I$ | H | O | H | Gb |
| Me | $CH_2I$ | H | S | H | G3 |
| Me | $CH_2CF_3$ | H | O | H | Ga |
| Me | $CH_2CF_3$ | H | S | H | G3 |
| Me | $CH_2CN$ | H | O | H | Ga |
| Me | $CH_2CN$ | H | S | H | G3 |
| Me | $CH_2OH$ | H | O | H | Gb |
| Me | $CH_2OMe$ | H | O | H | Ga |
| Me | $CH_2OMe$ | H | S | H | G3 |
| Me | $CH_2OEt$ | H | O | H | Ga |
| Me | $CH_2OEt$ | H | S | H | G3 |
| Me | $CH_2SMe$ | H | O | H | Ga |
| Me | $CH_2SMe$ | H | S | H | G3 |
| Me | $CH_2SEt$ | H | O | H | Ga |
| Me | $CH_2SEt$ | H | S | H | G3 |
| Me | $CH_2COMe$ | H | O | H | Ga |
| Me | $CH_2COMe$ | H | S | H | G3 |
| Me | $CH_2COEt$ | H | O | H | Ga |
| Me | $CH_2COEt$ | H | S | H | G3 |
| Me | $CH_2CO_2H$ | H | O | H | Ga |
| Me | $CH_2CO_2H$ | H | S | H | G3 |
| Me | $CH_2CO_2Me$ | H | O | H | Ga |
| Me | $CH_2CO_2Me$ | H | S | H | G3 |
| Me | $CH_2CO_2Et$ | H | O | H | Ga |
| Me | $CH_2CO_2Et$ | H | S | H | G3 |
| Me | $CH_2OCH_2CH=CH_2$ | H | O | H | Gb |
| Me | $CH_2OCH_2CH=CH_2$ | H | S | H | G3 |
| Me | $CH_2OCH_2C\equiv CH$ | H | O | H | Gb |
| Me | $CH_2OCH_2C\equiv CH$ | H | S | H | G3 |

| $R_1$ | $R_2$ | $R_3$ | X | M | Gn |
|---|---|---|---|---|---|
| Me | $CH=CHCO_2Me$ | H | O | H | Ga |
| Me | $CH=CHCO_2Me$ | H | S | H | G3 |
| Me | $CH=CHCO_2Et$ | H | O | H | Ga |
| Me | $CH=CHCO_2Et$ | H | S | H | G3 |
| Me | CH=CHCN | H | O | H | Ga |
| Me | CH=CHCN | H | S | H | G3 |
| Me | Ph | H | O | H | Ga |
| Me | Ph | H | S | H | G3 |
| Me | Ph-2-Cl | H | O | H | Ga |
| Me | Ph-2-Cl | H | S | H | G3 |
| Me | Ph-3-Cl | H | O | H | Gb |
| Me | Ph-4-Cl | H | O | H | Gb |
| Me | $CH_2Ph$ | H | O | H | Ga |
| Me | $CH_2Ph$ | H | S | H | G3 |
| Me | $CH_2Ph$-2-Cl | H | O | H | Ga |
| Me | $CH_2Ph$-2-Cl | H | S | H | G3 |
| Me | $CH_2Ph$-3-Cl | H | O | H | Gb |
| Me | $CH_2Ph$-4-Cl | H | O | H | Gb |
| Et | Cl | H | O | H | Ga |
| Et | Cl | H | S | H | G3 |
| Et | Br | H | O | H | Gb |
| Et | Br | H | S | H | G3 |
| Et | I | H | O | H | Gb |
| Et | I | H | S | H | G3 |
| Et | $CF_3$ | H | O | H | Ga |
| Et | $CF_3$ | H | S | H | G3 |
| Et | $NO_2$ | H | O | H | Ga |
| Et | $NO_2$ | H | S | H | G3 |
| Et | OMe | H | O | H | Ga |
| Et | OMe | H | S | H | G3 |
| Et | SMe | H | O | H | Ga |
| Et | SMe | H | S | H | G3 |
| Et | $SO_2Me$ | H | O | H | Ga |
| Et | $SO_2Me$ | H | S | H | G3 |
| Et | COMe | H | O | H | Ga |
| Et | COMe | H | S | H | G3 |
| Et | $CO_2H$ | H | O | H | Ga |
| Et | $CO_2H$ | H | S | H | G3 |
| Et | $CO_2Me$ | H | O | H | Ga |
| Et | $CO_2Me$ | H | S | H | G3 |

| $R_1$ | $R_2$ | $R_3$ | X | M | Gn |
|---|---|---|---|---|---|
| Et | $CO_2Et$ | H | O | H | Ga |
| Et | $CO_2Et$ | H | S | H | G3 |
| Et | $CH_2Cl$ | H | O | H | Ga |
| Et | $CH_2Cl$ | H | S | H | G3 |
| Et | $CH_2Br$ | H | O | H | Gb |
| Et | $CH_2Br$ | H | S | H | G3 |
| Et | $CH_2I$ | H | O | H | Gb |
| Et | $CH_2I$ | H | S | H | G3 |
| Et | $CH_2CF_3$ | H | O | H | Ga |
| Et | $CH_2CF_3$ | H | S | H | G3 |
| Et | $CH_2CN$ | H | O | H | Ga |
| Et | $CH_2CN$ | H | S | H | G3 |
| Et | $CH_2OH$ | H | O | H | Gb |
| Et | $CH_2OMe$ | H | O | H | Ga |
| Et | $CH_2OMe$ | H | S | H | G3 |
| Et | $CH_2OEt$ | H | O | H | Ga |
| Et | $CH_2OEt$ | H | S | H | G3 |
| Et | $CH_2SMe$ | H | O | H | Ga |
| Et | $CH_2SMe$ | H | S | H | G3 |
| Et | $CH_2SEt$ | H | O | H | Ga |
| Et | $CH_2SEt$ | H | S | H | G3 |
| Et | $CH_2COMe$ | H | O | H | Ga |
| Et | $CH_2COMe$ | H | S | H | G3 |
| Et | $CH_2COEt$ | H | O | H | Ga |
| Et | $CH_2COEt$ | H | S | H | G3 |
| Et | $CH_2CO_2H$ | H | O | H | Ga |
| Et | $CH_2CO_2H$ | H | S | H | G3 |
| Et | $CH_2CO_2Me$ | H | O | H | Ga |
| Et | $CH_2CO_2Me$ | H | S | H | G3 |
| Et | $CH_2CO_2Et$ | H | O | H | Ga |
| Et | $CH_2CO_2Et$ | H | S | H | G3 |
| Et | $CH_2OCH_2CH=CH_2$ | H | O | H | Gb |
| Et | $CH_2OCH_2CH=CH_2$ | H | S | H | G3 |
| Et | $CH_2OCH_2C\equiv CH$ | H | O | H | Gb |
| Et | $CH_2OCH_2C\equiv CH$ | H | S | H | G3 |
| Et | $CH=CHCO_2Me$ | H | O | H | Ga |
| Et | $CH=CHCO_2Me$ | H | S | H | G3 |
| Et | $CH=CHCO_2Et$ | H | O | H | Ga |
| Et | $CH=CHCO_2Et$ | H | S | H | G3 |
| Et | $CH=CHCN$ | H | O | H | Ga |

| $R_1$ | $R_2$ | $R_3$ | X | M | Gn |
|---|---|---|---|---|---|
| Et | CH=CHCN | H | S | H | G3 |
| Et | Ph | H | O | H | Ga |
| Et | Ph | H | S | H | G3 |
| Et | Ph-2-Cl | H | O | H | Ga |
| Et | Ph-2-Cl | H | S | H | G3 |
| Et | Ph-3-Cl | H | O | H | Gb |
| Et | Ph-4-Cl | H | O | H | Gb |
| Et | $CH_2Ph$ | H | O | H | Ga |
| Et | $CH_2Ph$ | H | S | H | G3 |
| Et | $CH_2Ph$-2-Cl | H | O | H | Ga |
| Et | $CH_2Ph$-2-Cl | H | S | H | G3 |
| Et | $CH_2Ph$-3-Cl | H | O | H | Gb |
| Et | $CH_2Ph$-4-Cl | H | O | H | Gb |
| Cl | Me | H | O | H | Ga |
| Cl | Me | H | S | H | G3 |
| Br | Me | H | O | H | Gb |
| Br | Me | H | S | H | G3 |
| I | Me | H | O | H | Gb |
| I | Me | H | S | H | G3 |
| $CF_3$ | Me | H | O | H | Ga |
| $CF_3$ | Me | H | S | H | G3 |
| $NO_2$ | Me | H | O | H | Ga |
| $NO_2$ | Me | H | S | H | G3 |
| OMe | Me | H | O | H | Ga |
| OMe | Me | H | S | H | G3 |
| SMe | Me | H | O | H | Ga |
| SMe | Me | H | S | H | G3 |
| $SO_2Me$ | Me | H | O | H | Ga |
| $SO_2Me$ | Me | H | S | H | G3 |
| COMe | Me | H | O | H | Ga |
| COMe | Me | H | S | H | G3 |
| $CO_2H$ | Me | H | O | H | Ga |
| $CO_2H$ | Me | H | S | H | G3 |
| $CO_2Me$ | Me | H | O | H | Ga |
| $CO_2Me$ | Me | H | S | H | G3 |
| $CO_2Et$ | Me | H | O | H | Ga |
| $CO_2Et$ | Me | H | S | H | G3 |
| $CH_2Cl$ | Me | H | O | H | Ga |
| $CH_2Cl$ | Me | H | S | H | G3 |
| $CH_2Br$ | Me | H | O | H | Gb |

| $R_1$ | $R_2$ | $R_3$ | X | M | Gn |
|---|---|---|---|---|---|
| $CH_2Br$ | Me | H | S | H | G3 |
| $CH_2I$ | Me | H | O | H | Gb |
| $CH_2I$ | Me | H | S | H | G3 |
| $CH_2CF_3$ | Me | H | O | H | Ga |
| $CH_2CF_3$ | Me | H | S | H | G3 |
| $CH_2CN$ | Me | H | O | H | Ga |
| $CH_2CN$ | Me | H | S | H | G3 |
| $CH_2OH$ | Me | H | O | H | Gb |
| $CH_2OMe$ | Me | H | O | H | Ga |
| $CH_2OMe$ | Me | H | S | H | G3 |
| $CH_2OEt$ | Me | H | O | H | Ga |
| $CH_2OEt$ | Me | H | S | H | G3 |
| $CH_2SMe$ | Me | H | O | H | Ga |
| $CH_2SMe$ | Me | H | S | H | G3 |
| $CH_2SEt$ | Me | H | O | H | Ga |
| $CH_2SEt$ | Me | H | S | H | G3 |
| $CH_2COMe$ | Me | H | O | H | Ga |
| $CH_2COMe$ | Me | H | S | H | G3 |
| $CH_2COEt$ | Me | H | O | H | Ga |
| $CH_2COEt$ | Me | H | S | H | G3 |
| $CH_2CO_2H$ | Me | H | O | H | Ga |
| $CH_2CO_2H$ | Me | H | S | H | G3 |
| $CH_2CO_2Me$ | Me | H | O | H | Ga |
| $CH_2CO_2Me$ | Me | H | S | H | G3 |
| $CH_2CO_2Et$ | Me | H | O | H | Ga |
| $CH_2CO_2Et$ | Me | H | S | H | G3 |
| $CH_2OCH_2CH=CH_2$ | Me | H | O | H | Gb |
| $CH_2OCH_2CH=CH_2$ | Me | H | S | H | G3 |
| $CH_2OCH_2C\equiv CH$ | Me | H | O | H | Gb |
| $CH_2OCH_2C\equiv CH$ | Me | H | S | H | G3 |
| $CH=CHCO_2Me$ | Me | H | O | H | Ga |
| $CH=CHCO_2Me$ | Me | H | S | H | G3 |
| $CH=CHCO_2Et$ | Me | H | O | H | Ga |
| $CH=CHCO_2Et$ | Me | H | S | H | G3 |
| CH=CHCN | Me | H | O | H | Ga |
| CH=CHCN | Me | H | S | H | G3 |
| Ph | Me | H | O | H | Ga |
| Ph | Me | H | S | H | G3 |
| Ph-2-Cl | Me | H | O | H | Ga |
| Ph-2-Cl | Me | H | S | H | G3 |

| $R_1$ | $R_2$ | $R_3$ | X | M | Gn |
|---|---|---|---|---|---|
| Ph-3-Cl | Me | H | O | H | Gb |
| Ph-4-Cl | Me | H | O | H | Gb |
| $CH_2Ph$ | Me | H | O | H | Ga |
| $CH_2Ph$ | Me | H | S | H | G3 |
| $CH_2Ph$-2-Cl | Me | H | O | H | Ga |
| $CH_2Ph$-2-Cl | Me | H | S | H | G3 |
| $CH_2Ph$-3-Cl | Me | H | O | H | Gb |
| $CH_2Ph$-4-Cl | Me | H | O | H | Gb |
| Cl | Et | H | O | H | Ga |
| Cl | Et | H | S | H | G3 |
| Br | Et | H | O | H | Gb |
| Br | Et | H | S | H | G3 |
| I | Et | H | O | H | Gb |
| I | Et | H | S | H | G3 |
| $CF_3$ | Et | H | O | H | Ga |
| $CF_3$ | Et | H | S | H | G3 |
| $NO_2$ | Et | H | O | H | Ga |
| $NO_2$ | Et | H | S | H | G3 |
| OMe | Et | H | O | H | Ga |
| OMe | Et | H | S | H | G3 |
| SMe | Et | H | O | H | Ga |
| SMe | Et | H | S | H | G3 |
| $SO_2Me$ | Et | H | O | H | Ga |
| $SO_2Me$ | Et | H | S | H | G3 |
| COMe | Et | H | O | H | Ga |
| COMe | Et | H | S | H | G3 |
| $CO_2H$ | Et | H | O | H | Ga |
| $CO_2H$ | Et | H | S | H | G3 |
| $CO_2Me$ | Et | H | O | H | Ga |
| $CO_2Me$ | Et | H | S | H | G3 |
| $CO_2Et$ | Et | H | O | H | Ga |
| $CO_2Et$ | Et | H | S | H | G3 |
| $CH_2Cl$ | Et | H | O | H | Ga |
| $CH_2Cl$ | Et | H | S | H | G3 |
| $CH_2Br$ | Et | H | O | H | Gb |
| $CH_2Br$ | Et | H | S | H | G3 |
| $CH_2I$ | Et | H | O | H | Gb |
| $CH_2I$ | Et | H | S | H | G3 |
| $CH_2CF_3$ | Et | H | O | H | Ga |
| $CH_2CF_3$ | Et | H | S | H | G3 |

| $R_1$ | $R_2$ | $R_3$ | X | M | Gn |
|---|---|---|---|---|---|
| $CH_2CN$ | Et | H | O | H | Ga |
| $CH_2CN$ | Et | H | S | H | G3 |
| $CH_2OH$ | Et | H | O | H | Gb |
| $CH_2OMe$ | Et | H | O | H | Ga |
| $CH_2OMe$ | Et | H | S | H | G3 |
| $CH_2OEt$ | Et | H | O | H | Ga |
| $CH_2OEt$ | Et | H | S | H | G3 |
| $CH_2SMe$ | Et | H | O | H | Ga |
| $CH_2SMe$ | Et | H | S | H | G3 |
| $CH_2SEt$ | Et | H | O | H | Ga |
| $CH_2SEt$ | Et | H | S | H | G3 |
| $CH_2COMe$ | Et | H | O | H | Ga |
| $CH_2COMe$ | Et | H | S | H | G3 |
| $CH_2COEt$ | Et | H | O | H | Ga |
| $CH_2COEt$ | Et | H | S | H | G3 |
| $CH_2CO_2H$ | Et | H | O | H | Ga |
| $CH_2CO_2H$ | Et | H | S | H | G3 |
| $CH_2CO_2Me$ | Et | H | O | H | Ga |
| $CH_2CO_2Me$ | Et | H | S | H | G3 |
| $CH_2CO_2Et$ | Et | H | O | H | Ga |
| $CH_2CO_2Et$ | Et | H | S | H | G3 |
| $CH_2OCH_2CH=CH_2$ | Et | H | O | H | Gb |
| $CH_2OCH_2CH=CH_2$ | Et | H | S | H | G3 |
| $CH_2OCH_2C\equiv CH$ | Et | H | O | H | Gb |
| $CH_2OCH_2C\equiv CH$ | Et | H | S | H | G3 |
| $CH=CHCO_2Me$ | Et | H | O | H | Ga |
| $CH=CHCO_2Me$ | Et | H | S | H | G3 |
| $CH=CHCO_2Et$ | Et | H | O | H | Ga |
| $CH=CHCO_2Et$ | Et | H | S | H | G3 |
| CH=CHCN | Et | H | O | H | Ga |
| CH=CHCN | Et | H | S | H | G3 |
| Ph | Et | H | O | H | Ga |
| Ph | Et | H | S | H | G3 |
| Ph-2-Cl | Et | H | O | H | Ga |
| Ph-2-Cl | Et | H | S | H | G3 |
| Ph-3-Cl | Et | H | O | H | Gb |
| Ph-4-Cl | Et | H | O | H | Gb |
| $CH_2Ph$ | Et | H | O | H | Ga |
| $CH_2Ph$ | Et | H | S | H | G3 |
| $CH_2Ph$-2-Cl | Et | H | O | H | Ga |

| $R_1$ | $R_2$ | $R_3$ | X | M | Gn |
|---|---|---|---|---|---|
| $CH_2Ph$-2-Cl | Et | H | S | H | G3 |
| $CH_2Ph$-3-Cl | Et | H | O | H | Gb |
| $CH_2Ph$-4-Cl | Et | H | O | H | Gb |

Table 10

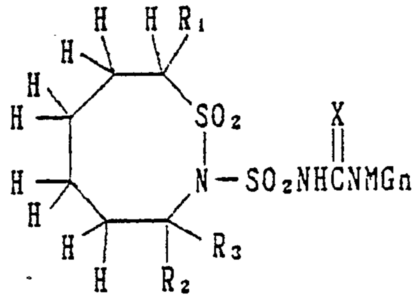

| $R_1$ | $R_2$ | $R_3$ | X | M | Gn |
|---|---|---|---|---|---|
| H | H | H | O | H | Ga |
| H | H | H | S | H | G3 |
| H | H | H | O | Me | Gb |
| H | H | H | O | Et | Gb |
| H | H | H | O | $CH_2CH=CH_2$ | Gb |
| H | H | H | O | $CH_2C\equiv CH$ | Gb |
| Me | H | H | O | H | Ga |
| Me | H | H | S | H | G3 |
| H | Me | H | O | H | Ga |
| H | Me | H | S | H | G3 |
| Et | H | H | O | H | Gb |
| Et | H | H | S | H | G3 |
| H | Et | H | O | H | Ga |
| H | Et | H | S | H | G3 |
| Pr-n | H | H | O | H | Gb |
| H | Pr-n | H | O | H | Gb |
| H | Pr-n | H | S | H | G3 |
| Me | Me | H | O | H | Ga |
| Me | Me | H | S | H | G3 |
| Me | Et | H | O | H | Gb |

| $R_1$ | $R_2$ | $R_3$ | X | M | Gn |
|---|---|---|---|---|---|
| Me | Pr-n | H | 0 | H | Gb |
| Me | Pr-i | H | 0 | H | Gb |
| Et | Me | H | 0 | H | Gb |
| Et | Et | H | 0 | H | Gb |
| Et | Pr-n | H | 0 | H | Gb |
| Et | Pr-i | H | 0 | H | Gb |
| Pr-n | Me | H | 0 | H | Gb |
| Pr-n | Et | H | 0 | H | Gb |
| Pr-n | Pr-n | H | 0 | H | Gb |
| Pr-n | Pr-i | H | 0 | H | Gb |
| Pr-i | Me | H | 0 | H | Gb |
| Pr-i | Et | H | 0 | H | Gb |
| Pr-i | Pr-n | H | 0 | H | Gb |
| Pr-i | Pr-i | H | 0 | H | Gb |
| H | Me | Me | 0 | H | Ga |
| H | Me | Me | S | H | G3 |
| H | Me | Et | 0 | H | Ga |
| H | Me | Et | S | H | G3 |
| H | Me | Pr-n | 0 | H | Gb |
| H | Me | Pr-i | 0 | H | Gb |
| H | Me | Pr-i | S | H | G3 |
| H | Et | Et | 0 | H | Gb |
| H | Et | Pr-n | 0 | H | Gb |
| Me | Me | Me | 0 | H | Ga |
| Me | Me | Me | S | H | G3 |
| Et | Me | Me | 0 | H | Gb |
| Pr-n | Me | Me | 0 | H | Gb |
| Pr-i | Me | Me | 0 | H | Gb |
| H | Ph | H | 0 | H | Ga |
| H | Ph | H | S | H | G3 |
| H | $CH_2Ph$ | H | 0 | H | Ga |
| H | $CH_2Ph$ | H | S | H | G3 |
| Me | Ph | H | 0 | H | Ga |
| Me | Ph | H | S | H | G3 |
| Me | $CH_2Ph$ | H | 0 | H | Ga |
| Me | $CH_2Ph$ | H | S | H | G3 |
| Et | Ph | H | 0 | H | Ga |

| $R_1$ | $R_2$ | $R_3$ | X | M | Gn |
|---|---|---|---|---|---|
| Et | Ph | H | S | H | G3 |
| Et | $CH_2Ph$ | H | O | H | Ga |
| Et | $CH_2Ph$ | H | S | H | G3 |
| Ph | Me | H | O | H | Ga |
| Ph | Me | H | S | H | G3 |
| $CH_2Ph$ | Me | H | O | H | Ga |
| $CH_2Ph$ | Me | H | S | H | G3 |
| Ph | Et | H | O | H | Ga |
| Ph | Et | H | S | H | G3 |
| $CH_2Ph$ | Et | H | O | H | Ga |
| $CH_2Ph$ | Et | H | S | H | G3 |

When the compound of the present invention is to be used as a herbicide, it is usually mixed with a suitable carrier, for instance, a solid carrier such as clay, talc, bentonite or diatomaceous earth, or a liquid carrier such as water, an alcohol (such as methanol or ethanol), an aromatic hydrocarbon (such as benzene, toluene or xylene), a chlorinated hydrocarbon, an ether, a ketone, an ester (such as ethyl acetate) or an acid amide (such as dimethylformamide). If desired, an emulsifier, a dispersing agent, a suspending agent, a penetrating agent, a spreader or a stabilizer may be added to prepare an optional formulation such as a liquid formulation, an emulsifiable concentrate, a wettable powder, a dust, a granule or a flowable.

Now, Formulation Examples of the herbicides containing the compounds of the present invention as active ingredients, will be given. However, it should be understood that the present invention is by no means restricted to such specific Examples. In the following Formulation Examples, "parts" means "parts by weight".

| FORMULATION EXAMPLE 1: Wettable powder | |
|---|---|
| Compound No. 1 of the present invention | 20 parts |
| Zeeklite A (tradename for a kaolin-type clay, manufactured by Zeeklite Industries, Co., Ltd.) | 76 parts |
| Sorpol 5039 (tradename for a mixture of a nonionic surfactant and an anionic surfactant, manufactured by Toho Chemical Co., Ltd.) | 2 parts |
| Carplex (tradename for a coagulation-preventing agent composed of a mixture of a surfactant and fine silica powder, manufactured by Shionogi Pharmaceutical Co., Ltd.) | 2 parts |

The above ingredients are homogeneously pulverized and mixed to form a wettable powder.

| FORMULATION EXAMPLE 2: Wettable powder | |
|---|---|
| Compound No. 14 of the present invention | 40 parts |
| Zeeklite A (tradename for a kaolin-type clay, manufactured by Zeeklite Industries, Co., Ltd.) | 54 parts |
| Sorpol 5039 (tradename for a mixture of a nonionic surfactant and an anionic surfactant, manufactured by Toho Chemical Co., Ltd.) | 2 parts |
| Carplex (tradename for a coagulation-preventing agent composed of a mixture of a surfactant and fine silica powder, manufactured by Shionogi Pharmaceutical Co., Ltd.) | 4 parts |

The above ingredients are homogeneously pulverized and mixed to form a wettable powder.

| FORMULATION EXAMPLE 3: Emulsifiable concentrate | |
|---|---|
| Compound No. 2 of the present invention | 5 parts |
| Xylene | 75 parts |
| N,N-dimethylformamide | 15 parts |
| Sorpol 2680 (tradename for a mixture of a nonionic surfactant and an anionic surfactant, manufactured by Toho Chemical Co., Ltd.) | 5 parts |

The above ingredients are homogeneously mixed to obtain an emulsifiable concentrate. In use, the above emulsifiable concentrate is diluted from 10 to 10,000 times and applied so that the dose of the active ingredient will be from 0.005 to 10 kg per hectare.

| FORMULATION EXAMPLE 4: Flowable | |
|---|---|
| Compound No. 7 of the present invention | 25 parts |
| Agrizole S-710 (tradename for a nonionic surfactant, manufactured by Kao Corporation) | 10 parts |
| Runox 1000C (tradename for an anionic surfactant, manufactured by Toho Chemical Co., Ltd.) | 0.5 part |
| 1% Rodopol water (tradename for a thickener, manufacture by Rhone-Poulenc) | 20 parts |
| Water | 44.5 parts |

The above ingredients are homogeneously mixed to form a flowable.

| FORMULATION EXAMPLE 5: Flowable | |
|---|---|
| Compound No. 6 of the present invention | 40 parts |
| Agrizole S-710 (tradename for a nonionic surfactant, manufactured by Kao Corporation) | 10 parts |
| Runox 1000C (tradename for an anionic surfactant, manufactured by Toho Chemical Co., Ltd.) | 0.5 parts |
| 1% Rodopol water (tradename for a thickener, manufacture by Rhone-Poulenc) | 20 parts |
| Water | 29.5 parts |

The above ingredients are homogeneously mixed to form a flowable.

| FORMULATION EXAMPLE 6: Granule | |
|---|---|
| Compound No. 22 of the present invention | 1 part |
| Bentonite | 55 parts |
| Talc | 44 parts |

The above ingredients are uniformly pulverized and mixed. Then, the mixture is kneaded and stirred by an addition of a small amount of water, granulated by a granulator and dried to obtain granules.

Further, if desired, other herbicides, various insecticides, bacteriocides, plant regulating agents or synergism agents may be combined at the time of the preparation of the formulations or at the time of the application of the herbicides.

As other herbicides to be combined with the herbicide of the present invention, there may be mentioned, for instance, compounds disclosed in Farm Chemicals Handbook, the 73rd Edition (1987).

The compounds of the present inventin are applicable not only to agricultural and horticultural fields such as upland fields, paddy fields and orchards, but also to non-agricultural fields such as athletic fields, vacant fields and railway sides for the control of various weeds. The dose in their application varies depending upon the application site, the season for application, the type of weeds, the type of crop plants, etc. However, it is usually within a range of from 0.005 to 10 kg per hectare.

Now, the herbicidal activities of the compounds of the present invention will be described with respect to specific Test Examples. The compound Nos. used in the Test Examples correspond to the aforementioned Compound Nos.

TEST EXAMPLE 1: Test on the herbicidal effects in soil treatment

A plastic box having a length of 15 cm, a width of 22 cm and a depth of 6 cm was filled with a sterilized diluvium soil, and seeds of Echinochloa crus-galli, Digitaria adscendens, Cyperus micoiria, Solanum nigrum, Galinsoga ciliate, Rorippa indica, Oryza sativa, Zea mays, Triticum vulgare, Glycine max and Gossypium ssp. were sown. The soil was covered thereon in the thickness of about 1.5 cm, and then a herbicide solution was applied onto the surface of the soil uniformly so that the active ingredient is distributed at a predetermined concentration. The herbicide solution was prepared by diluting the wettable powder as described in the foregoing Formulation Examples with water and applied onto the entire soil surface by means of a small spray. Four weeks after the application of the herbicide solution, the herbicidal effects against each weed were determined on the basis of the following standard ratings. The results thereby obtained are shown in Table 11. (Compound Nos. correspond to the Compound Nos. presented in Examples.)

Some compounds of the present invention have selectivity for certain crop plants.

Standard ratings:

5: Growth control rate of more than 90%
(almost completely withered)
4: Growth control rate of from 70 to 90%
3: Growth control rate of from 40 to 70%
2: Growth control rate of from 20 to 40%
1: Growth control rate of from 5 to 20%
0: Growth control rate of less than 5%
(almost non-effective)

The above growth control rates were calculated by the following equation:

Growth control rate (%) = $(1-\frac{T}{N}) \times 100$

where T: Weight of the weed grown above the soil surface of the treated area
N: Weight of the weed grown above the soil surface of the non-treated area

TEST EXAMPLE 2: Test on the herbicidal effects in foliage treatment

A plastic box having a length of 15 cm, a width of 22 cm and a depth of 6 cm was filled with a sterilized diluvium soil, and seeds of Echinochloa crus-galli, Digitaria adscendens, Cyperus microiria, Solanum nigrum, Galinsoga ciliate, Rorippa indica, Oryza sativa, Zea mays, Triticum vulgare, Glycine max, Gossypium spp. and Beta vulgaris were spot-wisely sown. Then, the soil was covered thereon in a thickness of about 1.5 cm. When the various weeds and crops grew to the 2 to 3 leaf stage, a herbicide solution was uniformly sprayed on the foliages so that the active ingredient is applied in a predetermined concentration.

The herbicide solution was prepared by diluting the wettable powder as described in the above Formulation Examples with water and applied onto the entire surface of the foliages of the weeds and the crop plants by a small spray. Four weeks after the application of the herbicide solution, the herbicidal effects against each weed were determined on the basis of the standard ratings described in Test Example 1. The results are shown in Table 12. (Compound Nos. correspond to the Compound Nos. presented in Examples.)

In Tables 11 and 12, the following abbreviations are used.

Dose: Dose of active ingredient (g/are)
EC: Echinochloa crus-galli (barnyardgrass)
DI: Digitaria adscendens (large crabgrass)
CY: Cyperus microiria (annual-sedge)
SO: Solanum nigrum (brack nightshade)
GA: Galinsoga ciliate (hairly galinsoga)
RO: Rorippa indica
OY: Oryza sativa (rice)
ZE: Zea mays (corn)
TR: Triticum vulgare (wheat)

GY: Glycine max (soybean)
GO: Gossypium spp. (cotton)
BE: Beta vulgaris (beet)

Table 11

| Compound No. | Dose | EC | DI | CY | SO | GA | RO | OY | ZE | TR | GY | GO |
|---|---|---|---|---|---|---|---|---|---|---|---|---|
| 1 | 1.6 | 5 | 5 | 5 | 5 | 5 | 5 | 5 | 5 | 0 | 5 | 0 |
| | 3.2 | 5 | 5 | 5 | 5 | 5 | 5 | 5 | 5 | 1 | 5 | 1 |
| | 6.3 | 5 | 5 | 5 | 5 | 5 | 5 | 5 | 5 | 2 | 5 | 2 |
| 3 | 1.6 | 3 | 5 | 5 | 4 | 5 | 5 | 5 | 5 | 2 | 5 | 0 |
| | 3.2 | 4 | 5 | 5 | 5 | 5 | 5 | 5 | 5 | 3 | 5 | 1 |
| | 6.3 | 5 | 5 | 5 | 5 | 5 | 5 | 5 | 5 | 4 | 5 | 2 |
| 4 | 1.6 | 4 | 5 | 5 | 5 | 5 | 5 | 5 | 5 | 0 | 5 | 0 |
| | 3.2 | 5 | 5 | 5 | 5 | 5 | 5 | 5 | 5 | 1 | 5 | 1 |
| | 6.3 | 5 | 5 | 5 | 5 | 5 | 5 | 5 | 5 | 2 | 5 | 2 |
| 5 | 1.6 | 4 | 5 | 5 | 5 | 5 | 5 | 5 | 4 | 0 | 4 | 0 |
| | 3.2 | 5 | 5 | 5 | 5 | 5 | 5 | 5 | 5 | 1 | 5 | 0 |
| | 6.3 | 5 | 5 | 5 | 5 | 5 | 5 | 5 | 5 | 2 | 5 | 0 |
| 6 | 6.3 | 3 | 5 | 4 | 4 | 4 | 5 | 5 | 5 | 0 | 4 | 0 |
| 7 | 6.3 | 5 | 5 | 5 | 5 | 5 | 5 | 5 | 5 | 3 | 5 | 5 |

Table 11 (continued)

| Compound No. | Dose | EC | DI | CY | SO | GA | RO | OY | ZE | TR | GY | GO |
|---|---|---|---|---|---|---|---|---|---|---|---|---|
| 8 | 6.3 | 3 | 4 | 5 | 3 | 5 | 5 | 5 | 5 | 1 | 5 | 1 |
| 9 | 6.3 | 5 | 4 | 5 | 5 | 5 | 5 | 5 | 5 | 2 | 5 | 5 |
| 10 | 6.3 | 4 | 4 | 5 | 5 | 5 | 5 | 5 | 5 | 3 | 5 | 0 |
| 12 | 6.3 | 3 | 4 | 5 | 3 | 5 | 5 | 5 | 4 | 3 | 4 | 0 |
| 14 | 1.6 | 5 | 5 | 5 | 4 | 5 | 5 | 5 | 5 | 5 | 5 | 0 |
| | 3.2 | 5 | 5 | 5 | 5 | 5 | 5 | 5 | 5 | 5 | 5 | 1 |
| | 6.3 | 5 | 5 | 5 | 5 | 5 | 5 | 5 | 5 | 5 | 5 | 2 |
| 15 | 1.6 | 4 | 4 | 5 | 3 | 5 | 5 | 5 | 5 | 5 | 4 | 0 |
| | 3.2 | 5 | 5 | 5 | 4 | 5 | 5 | 5 | 5 | 5 | 5 | 0 |
| | 6.3 | 5 | 5 | 5 | 5 | 5 | 5 | 5 | 5 | 5 | 5 | 1 |
| 16 | 1.6 | 5 | 5 | 5 | 5 | 5 | 5 | 5 | 5 | 5 | 5 | 0 |
| | 3.2 | 5 | 5 | 5 | 5 | 5 | 5 | 5 | 5 | 5 | 5 | 0 |
| | 6.3 | 5 | 5 | 5 | 5 | 5 | 5 | 5 | 5 | 5 | 5 | 1 |
| 17 | 1.6 | 3 | 4 | 5 | 4 | 5 | 5 | 5 | 1 | 2 | 5 | 1 |
| | 3.2 | 4 | 5 | 5 | 5 | 5 | 5 | 5 | 2 | 3 | 5 | 2 |
| | 6.3 | 5 | 5 | 5 | 5 | 5 | 5 | 5 | 3 | 4 | 5 | 3 |

Table 11 (continued)

| Compound No. | Dose | EC | DI | CY | SO | GA | RO | OY | ZE | TR | GY | GO |
|---|---|---|---|---|---|---|---|---|---|---|---|---|
| 20 | 6.3 | 5 | 5 | 5 | 5 | 5 | 5 | 5 | 1 | 1 | 1 | 0 |
| | 1.6 | 5 | 5 | 5 | 3 | 5 | 5 | 5 | 5 | 5 | 5 | 5 |
| 21 | 3.2 | 5 | 5 | 5 | 4 | 5 | 5 | 5 | 5 | 5 | 5 | 5 |
| | 6.3 | 5 | 5 | 5 | 5 | 5 | 5 | 5 | 5 | 5 | 5 | 5 |
| | 1.6 | 5 | 5 | 5 | 2 | 5 | 5 | 5 | 4 | 2 | 0 | 0 |
| 22 | 3.2 | 5 | 5 | 5 | 3 | 5 | 5 | 5 | 5 | 3 | 1 | 0 |
| | 6.3 | 5 | 5 | 5 | 4 | 5 | 5 | 5 | 5 | 4 | 3 | 1 |
| 23 | 6.3 | 4 | 3 | 5 | 4 | 5 | 5 | 5 | 2 | 1 | 3 | 2 |
| 24 | 6.3 | 4 | 4 | 5 | 2 | 5 | 5 | 5 | 2 | 4 | 2 | 2 |
| 27 | 6.3 | 3 | 2 | 5 | 3 | 5 | 5 | 3 | 4 | 0 | 4 | 3 |
| 30 | 6.3 | 3 | 2 | 4 | 4 | 5 | 5 | 5 | 5 | 2 | 4 | 3 |
| 35 | 6.3 | 5 | 5 | 5 | 4 | 4 | 5 | 5 | 2 | 0 | 4 | 0 |
| 37 | 6.3 | 2 | 3 | 4 | 3 | 4 | 4 | 5 | 3 | 0 | 4 | 2 |
| | 0.4 | 5 | 5 | 5 | 5 | 5 | 5 | 5 | 5 | 1 | 5 | 1 |
| 38 | 0.8 | 5 | 5 | 5 | 5 | 5 | 5 | 5 | 5 | 2 | 5 | 2 |
| | 1.6 | 5 | 5 | 5 | 5 | 5 | 5 | 5 | 5 | 3 | 5 | 3 |

Table 11 (continued)

| Compound No. | Dose | EC | DI | CY | SO | GA | RO | OY | ZE | TR | GY | GO |
|---|---|---|---|---|---|---|---|---|---|---|---|---|
| 39 | 0.4 | 2 | 3 | 5 | 2 | 5 | 5 | 5 | 2 | 0 | 3 | 0 |
| | 0.8 | 4 | 4 | 5 | 3 | 5 | 5 | 5 | 3 | 0 | 4 | 1 |
| | 1.6 | 5 | 5 | 5 | 4 | 5 | 5 | 5 | 4 | 1 | 5 | 2 |
| 40 | 1.6 | 3 | 5 | 5 | 5 | 5 | 5 | 5 | 2 | 0 | 3 | 0 |
| 41 | 1.6 | 3 | 3 | 4 | 5 | 5 | 5 | 5 | 2 | 0 | 0 | 0 |
| 42 | 6.3 | 1 | 1 | 4 | 3 | 5 | 5 | 4 | 0 | 0 | 0 | 0 |
| 43 | 0.4 | 5 | 5 | 5 | 5 | 5 | 5 | 5 | 5 | 5 | 5 | 5 |
| | 0.8 | 5 | 5 | 5 | 5 | 5 | 5 | 5 | 5 | 5 | 5 | 5 |
| | 1.6 | 5 | 5 | 5 | 5 | 5 | 5 | 5 | 5 | 5 | 5 | 5 |
| 44 | 0.4 | 2 | 5 | 5 | 3 | 5 | 5 | 5 | 3 | 4 | 3 | 0 |
| | 0.8 | 3 | 5 | 5 | 4 | 5 | 5 | 5 | 4 | 5 | 4 | 1 |
| | 1.6 | 4 | 5 | 5 | 5 | 5 | 5 | 5 | 5 | 5 | 5 | 3 |
| 45 | 0.4 | 2 | 5 | 5 | 4 | 5 | 5 | 5 | 4 | 5 | 4 | 3 |
| | 0.8 | 4 | 5 | 5 | 5 | 5 | 5 | 5 | 5 | 5 | 5 | 4 |
| | 1.6 | 5 | 5 | 5 | 5 | 5 | 5 | 5 | 5 | 5 | 5 | 5 |

Table 11 (continued)

| Compound No. | Dose | EC | DI | CY | SO | GA | RO | OY | ZE | TR | GY | GO |
|---|---|---|---|---|---|---|---|---|---|---|---|---|
| 46 | 0.4 | 3 | 5 | 4 | 4 | 5 | 5 | 5 | 3 | 1 | 2 | 3 |
| | 0.8 | 4 | 5 | 5 | 5 | 5 | 5 | 5 | 4 | 2 | 3 | 4 |
| | 1.6 | 5 | 5 | 5 | 5 | 5 | 5 | 5 | 5 | 3 | 4 | 5 |
| 47 | 1.6 | 2 | 2 | 5 | 3 | 5 | 5 | 5 | 2 | 1 | 2 | 0 |
| | 3.2 | 3 | 3 | 5 | 4 | 5 | 5 | 5 | 3 | 2 | 3 | 1 |
| | 6.3 | 5 | 5 | 5 | 5 | 5 | 5 | 5 | 4 | 4 | 4 | 2 |
| 49 | 1.6 | 4 | 5 | 5 | 3 | 5 | 5 | 5 | 1 | 0 | 2 | 1 |
| | 3.2 | 5 | 5 | 5 | 4 | 5 | 5 | 5 | 2 | 1 | 3 | 2 |
| | 6.3 | 5 | 5 | 5 | 5 | 5 | 5 | 5 | 3 | 2 | 4 | 3 |
| 50 | 6.3 | 3 | 5 | 5 | 4 | 5 | 5 | 5 | 3 | 0 | 3 | 2 |
| 51 | 6.3 | 2 | 4 | 5 | 3 | 5 | 5 | 4 | 2 | 0 | 2 | 1 |
| 52 | 1.6 | 2 | 1 | 5 | 2 | 5 | 5 | 4 | 1 | 2 | 3 | 1 |
| | 3.2 | 2 | 2 | 5 | 4 | 5 | 5 | 5 | 2 | 3 | 4 | 2 |
| | 6.3 | 3 | 3 | 5 | 5 | 5 | 5 | 5 | 3 | 4 | 5 | 3 |

Table 11 (continued)

| Compound No. | Dose | EC | DI | CY | SO | GA | RO | OY | ZE | TR | GY | GO |
|---|---|---|---|---|---|---|---|---|---|---|---|---|
| 53 | 1.6 | 3 | 4 | 4 | 4 | 5 | 5 | 5 | 2 | 3 | 4 | 3 |
| 54 | 6.3 | 4 | 3 | 3 | 3 | 4 | 5 | 5 | 4 | 4 | 2 | 1 |
| | 0.4 | 5 | 5 | 5 | 2 | 5 | 5 | 5 | 3 | 0 | 4 | 0 |
| 56 | 0.8 | 5 | 5 | 5 | 3 | 5 | 5 | 5 | 4 | 1 | 5 | 0 |
| | 1.6 | 5 | 5 | 5 | 4 | 5 | 5 | 5 | 5 | 3 | 5 | 1 |
| | 1.6 | 3 | 4 | 4 | 2 | 5 | 5 | 4 | 3 | 0 | 3 | 0 |
| 57 | 3.2 | 4 | 5 | 5 | 3 | 5 | 5 | 5 | 4 | 1 | 4 | 1 |
| | 6.3 | 5 | 5 | 5 | 4 | 5 | 5 | 5 | 5 | 3 | 5 | 2 |
| 59 | 6.3 | 3 | 2 | 5 | 5 | 5 | 5 | 5 | 3 | 0 | 0 | 2 |
| 61 | 6.3 | 3 | 2 | 5 | 5 | 5 | 5 | 5 | 3 | 0 | 0 | 2 |
| 62 | 6.3 | 5 | 5 | 5 | 5 | 5 | 5 | 5 | 4 | 2 | 5 | 5 |
| 64 | 6.3 | 2 | 5 | 5 | 5 | 5 | 5 | 0 | 0 | 0 | 2 | 0 |
| | 1.6 | 3 | 4 | 4 | 2 | 3 | 3 | 4 | 3 | 0 | 0 | 0 |
| 65 | 3.2 | 4 | 5 | 5 | 3 | 4 | 4 | 5 | 4 | 1 | 0 | 0 |
| | 6.3 | 5 | 5 | 5 | 4 | 5 | 5 | 5 | 4 | 2 | 1 | 1 |

Table 11 (continued)

| Compound No. | Dose | EC | DI | CY | SO | GA | RO | OY | ZE | TR | GY | GO |
|---|---|---|---|---|---|---|---|---|---|---|---|---|
| 67 | 1.6 | 3 | 4 | 3 | 2 | 5 | 5 | 5 | 0 | 1 | 1 | 0 |
| | 3.2 | 4 | 5 | 4 | 3 | 5 | 5 | 5 | 1 | 2 | 2 | 0 |
| | 6.3 | 5 | 5 | 5 | 4 | 5 | 5 | 5 | 2 | 3 | 3 | 0 |
| 72 | 1.6 | 5 | 5 | 5 | 3 | 4 | 5 | 5 | 2 | 0 | 1 | 1 |
| | 3.2 | 5 | 5 | 5 | 4 | 5 | 5 | 5 | 4 | 1 | 2 | 2 |
| | 6.3 | 5 | 5 | 5 | 5 | 5 | 5 | 5 | 5 | 2 | 4 | 3 |
| 73 | 1.6 | 4 | 5 | 5 | 4 | 5 | 5 | 5 | 4 | 4 | 2 | 2 |
| | 3.2 | 5 | 5 | 5 | 5 | 5 | 5 | 5 | 5 | 5 | 3 | 3 |
| | 6.3 | 5 | 5 | 5 | 5 | 5 | 5 | 5 | 5 | 5 | 4 | 4 |
| 74 | 0.8 | 2 | 4 | 4 | 2 | 5 | 5 | 5 | 3 | 4 | 3 | 0 |
| | 1.6 | 4 | 5 | 5 | 4 | 5 | 5 | 5 | 4 | 5 | 4 | 1 |
| | 3.2 | 5 | 5 | 5 | 5 | 5 | 5 | 5 | 5 | 5 | 5 | 2 |
| 75 | 1.6 | 2 | 4 | 4 | 2 | 5 | 5 | 1 | 0 | 0 | 1 | 2 |
| | 3.2 | 3 | 5 | 5 | 3 | 5 | 5 | 2 | 1 | 0 | 2 | 3 |
| | 6.3 | 4 | 5 | 5 | 4 | 5 | 5 | 3 | 2 | 2 | 3 | 4 |

Table 11 (continued)

| Compound No. | Dose | EC | DI | CY | SO | GA | RO | OY | ZE | TR | GY | GO |
|---|---|---|---|---|---|---|---|---|---|---|---|---|
| 77 | 0.4 | 5 | 5 | 5 | 5 | 5 | 5 | 5 | 5 | 5 | 4 | 3 |
| | 0.8 | 5 | 5 | 5 | 5 | 5 | 5 | 5 | 5 | 5 | 5 | 4 |
| | 1.6 | 5 | 5 | 5 | 5 | 5 | 5 | 5 | 5 | 5 | 5 | 5 |
| 78 | 0.4 | 4 | 5 | 5 | 3 | 5 | 5 | 5 | 4 | 5 | 3 | 2 |
| | 0.8 | 5 | 5 | 5 | 4 | 5 | 5 | 5 | 5 | 5 | 4 | 3 |
| | 1.6 | 5 | 5 | 5 | 5 | 5 | 5 | 5 | 5 | 5 | 5 | 4 |
| 79 | 0.4 | 3 | 5 | 5 | 5 | 5 | 5 | 5 | 1 | 2 | 1 | 1 |
| | 0.8 | 4 | 5 | 5 | 5 | 5 | 5 | 5 | 2 | 4 | 2 | 1 |
| | 1.6 | 5 | 5 | 5 | 5 | 5 | 5 | 5 | 4 | 5 | 4 | 2 |

Table 12

| Compound No. | Dose | EC | DI | CY | SO | GA | RO | OY | ZE | TR | GY | GO | BE |
|---|---|---|---|---|---|---|---|---|---|---|---|---|---|
| 1 | 1.6 | 5 | 5 | 5 | 5 | 5 | 5 | 5 | 5 | 0 | 5 | 5 | 5 |
| | 3.2 | 5 | 5 | 5 | 5 | 5 | 5 | 5 | 5 | 1 | 5 | 5 | 5 |
| | 6.3 | 5 | 5 | 5 | 5 | 5 | 5 | 5 | 5 | 2 | 5 | 5 | 5 |
| 2 | 6.3 | 3 | 2 | 5 | 5 | 5 | 5 | 2 | 1 | 0 | 4 | 2 | 5 |
| 3 | 1.6 | 5 | 4 | 5 | 5 | 5 | 5 | 5 | 5 | 1 | 5 | 3 | 5 |
| | 3.2 | 5 | 5 | 5 | 5 | 5 | 5 | 5 | 5 | 2 | 5 | 4 | 5 |
| | 6.3 | 5 | 5 | 5 | 5 | 5 | 5 | 5 | 5 | 3 | 5 | 5 | 5 |
| 4 | 1.6 | 5 | 4 | 5 | 5 | 5 | 5 | 5 | 4 | 0 | 5 | 5 | 5 |
| | 3.2 | 5 | 5 | 5 | 5 | 5 | 5 | 5 | 5 | 0 | 5 | 5 | 5 |
| | 6.3 | 5 | 5 | 5 | 5 | 5 | 5 | 5 | 5 | 1 | 5 | 5 | 5 |
| 5 | 1.6 | 5 | 4 | 4 | 5 | 5 | 5 | 5 | 3 | 0 | 4 | 2 | 5 |
| | 3.2 | 5 | 5 | 5 | 5 | 5 | 5 | 5 | 4 | 0 | 5 | 3 | 5 |
| | 6.3 | 5 | 5 | 5 | 5 | 5 | 5 | 5 | 5 | 1 | 5 | 4 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| 6 | 6.3 | 5 | 4 | 5 | 5 | 5 | 5 | 5 | 4 | 0 | 4 | 1 | 5 |
| 7 | 6.3 | 5 | 4 | 5 | 5 | 5 | 5 | 5 | 5 | 2 | 5 | 5 | 5 |
| 8 | 6.3 | 4 | 3 | 5 | 4 | 5 | 5 | 5 | 5 | 1 | 5 | 4 | 5 |
| 9 | 6.3 | 5 | 4 | 5 | 5 | 5 | 5 | 5 | 5 | 1 | 5 | 5 | 5 |
| 10 | 6.3 | 5 | 4 | 5 | 5 | 5 | 5 | 5 | 5 | 2 | 5 | 2 | 5 |
| 12 | 6.3 | 5 | 3 | 3 | 5 | 5 | 5 | 5 | 3 | 3 | 4 | 1 | 5 |
| 13 | 6.3 | 3 | 2 | 3 | 5 | 5 | 5 | 4 | 3 | 3 | 3 | 1 | 5 |
| 14 | 1.6 | 5 | 3 | 5 | 5 | 5 | 5 | 5 | 5 | 5 | 5 | 1 | 5 |
|  | 3.2 | 5 | 4 | 5 | 5 | 5 | 5 | 5 | 5 | 5 | 5 | 2 | 5 |
|  | 6.3 | 5 | 5 | 5 | 5 | 5 | 5 | 5 | 5 | 5 | 5 | 3 | 5 |
| 15 | 1.6 | 5 | 2 | 4 | 3 | 5 | 5 | 2 | 3 | 2 | 5 | 0 | 5 |
|  | 3.2 | 5 | 3 | 5 | 4 | 5 | 5 | 3 | 4 | 3 | 5 | 1 | 5 |
|  | 6.3 | 5 | 4 | 5 | 5 | 5 | 5 | 4 | 5 | 4 | 5 | 2 | 5 |
| 16 | 1.6 | 5 | 3 | 3 | 5 | 5 | 5 | 4 | 3 | 2 | 5 | 1 | 5 |
|  | 3.2 | 5 | 4 | 4 | 5 | 5 | 5 | 5 | 4 | 3 | 5 | 2 | 5 |
|  | 6.3 | 5 | 5 | 5 | 5 | 5 | 5 | 5 | 5 | 4 | 5 | 3 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| | 1.6 | 5 | 2 | 2 | 3 | 4 | 4 | 5 | 1 | 2 | 5 | 1 | 4 |
| 17 | 3.2 | 5 | 3 | 3 | 4 | 5 | 5 | 5 | 2 | 3 | 5 | 2 | 5 |
| | 6.3 | 5 | 4 | 4 | 5 | 5 | 5 | 5 | 3 | 4 | 5 | 3 | 5 |
| 20 | 6.3 | 5 | 2 | 3 | 5 | 5 | 5 | 2 | 3 | 1 | 5 | 1 | 4 |
| | 1.6 | 5 | 2 | 3 | 3 | 5 | 5 | 5 | 4 | 5 | 5 | 1 | 5 |
| 21 | 3.2 | 5 | 3 | 4 | 4 | 5 | 5 | 5 | 5 | 5 | 5 | 2 | 5 |
| | 6.3 | 5 | 4 | 5 | 5 | 5 | 5 | 5 | 5 | 5 | 5 | 3 | 5 |
| | 1.6 | 5 | 1 | 3 | 3 | 3 | 5 | 2 | 4 | 2 | 4 | 0 | 5 |
| 22 | 3.2 | 5 | 2 | 4 | 4 | 4 | 5 | 3 | 5 | 3 | 5 | 0 | 5 |
| | 6.3 | 5 | 3 | 5 | 5 | 5 | 5 | 4 | 5 | 4 | 5 | 1 | 5 |
| 23 | 6.3 | 4 | 3 | 3 | 4 | 5 | 5 | 3 | 4 | 1 | 4 | 1 | 5 |
| 24 | 6.3 | 4 | 3 | 3 | 3 | 4 | 5 | 3 | 3 | 1 | 4 | 2 | 5 |
| 27 | 6.3 | 4 | 2 | 4 | 5 | 5 | 5 | 5 | 3 | 0 | 5 | 2 | 5 |
| 28 | 6.3 | 3 | 2 | 4 | 4 | 5 | 5 | 2 | 1 | 0 | 5 | 2 | 5 |
| 30 | 6.3 | 5 | 3 | 5 | 5 | 5 | 5 | 5 | 5 | 3 | 5 | 5 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| 31 | 6.3 | 4 | 3 | 5 | 4 | 5 | 5 | 5 | 3 | 2 | 5 | 5 | 5 |
| 35 | 6.3 | 4 | 3 | 3 | 4 | 5 | 5 | 5 | 5 | 2 | 5 | 2 | 5 |
| 37 | 6.3 | 4 | 3 | 3 | 5 | 5 | 5 | 5 | 5 | 0 | 5 | 5 | 5 |
| 38 | 0.4 | 5 | 5 | 5 | 5 | 5 | 5 | 5 | 4 | 0 | 5 | 5 | 5 |
| | 0.8 | 5 | 5 | 5 | 5 | 5 | 5 | 5 | 5 | 1 | 5 | 5 | 5 |
| | 1.6 | 5 | 5 | 5 | 5 | 5 | 5 | 5 | 5 | 2 | 5 | 5 | 5 |
| 39 | 0.4 | 4 | 3 | 5 | 5 | 5 | 5 | 4 | 4 | 0 | 5 | 4 | 5 |
| | 0.8 | 5 | 4 | 5 | 5 | 5 | 5 | 5 | 5 | 1 | 5 | 5 | 5 |
| | 1.6 | 5 | 5 | 5 | 5 | 5 | 5 | 5 | 5 | 2 | 5 | 5 | 5 |
| 40 | 1.6 | 5 | 5 | 5 | 5 | 5 | 5 | 4 | 3 | 1 | 4 | 3 | 5 |
| 41 | 1.6 | 4 | 3 | 3 | 5 | 5 | 5 | 4 | 2 | 0 | 3 | 3 | 5 |
| 42 | 6.3 | 1 | 1 | 3 | 5 | 5 | 5 | 3 | 0 | 0 | 4 | 2 | 5 |
| 43 | 0.4 | 5 | 4 | 5 | 5 | 5 | 5 | 5 | 5 | 5 | 5 | 5 | 4 |
| | 0.8 | 5 | 5 | 5 | 5 | 5 | 5 | 5 | 5 | 5 | 5 | 5 | 5 |
| | 1.6 | 5 | 5 | 5 | 5 | 5 | 5 | 5 | 5 | 5 | 5 | 5 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| | 0.4 | 3 | 2 | 2 | 5 | 5 | 5 | 5 | 3 | 4 | 5 | 4 | 3 |
| 44 | 0.8 | 4 | 2 | 4 | 5 | 5 | 5 | 5 | 4 | 5 | 5 | 5 | 4 |
| | 1.6 | 5 | 3 | 5 | 5 | 5 | 5 | 5 | 5 | 5 | 5 | 5 | 5 |
| | 0.4 | 3 | 4 | 4 | 5 | 5 | 5 | 4 | 4 | 4 | 4 | 4 | 3 |
| 45 | 0.8 | 4 | 5 | 5 | 5 | 5 | 5 | 5 | 5 | 5 | 5 | 5 | 4 |
| | 1.6 | 5 | 5 | 5 | 5 | 5 | 5 | 5 | 5 | 5 | 5 | 5 | 5 |
| | 0.4 | 4 | 3 | 4 | 5 | 5 | 5 | 3 | 4 | 2 | 3 | 4 | 3 |
| 46 | 0.8 | 5 | 4 | 5 | 5 | 5 | 5 | 4 | 5 | 3 | 4 | 5 | 4 |
| | 1.6 | 5 | 5 | 5 | 5 | 5 | 5 | 5 | 5 | 4 | 5 | 5 | 5 |
| | 1.6 | 2 | 0 | 5 | 3 | 5 | 5 | 4 | 3 | 0 | 3 | 3 | 5 |
| 47 | 3.2 | 3 | 1 | 5 | 4 | 5 | 5 | 5 | 4 | 0 | 4 | 4 | 5 |
| | 6.3 | 4 | 2 | 5 | 5 | 5 | 5 | 5 | 5 | 1 | 5 | 5 | 5 |
| | 1.6 | 4 | 2 | 5 | 5 | 5 | 5 | 4 | 4 | 0 | 5 | 3 | 5 |
| 49 | 3.2 | 5 | 3 | 5 | 5 | 5 | 5 | 5 | 5 | 1 | 5 | 4 | 5 |
| | 6.3 | 5 | 4 | 5 | 5 | 5 | 5 | 5 | 5 | 2 | 5 | 5 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| 50 | 6.3 | 3 | 2 | 5 | 5 | 5 | 5 | 5 | 3 | 2 | 5 | 4 | 5 |
| 51 | 6.3 | 3 | 2 | 5 | 4 | 4 | 5 | 4 | 4 | 0 | 5 | 3 | 5 |
| 52 | 1.6 | 1 | 1 | 5 | 4 | 5 | 5 | 3 | 3 | 0 | 4 | 4 | 5 |
| | 3.2 | 2 | 2 | 5 | 5 | 5 | 5 | 4 | 4 | 1 | 5 | 5 | 5 |
| | 6.3 | 3 | 3 | 5 | 5 | 5 | 5 | 5 | 5 | 2 | 5 | 5 | 5 |
| 53 | 1.6 | 3 | 2 | 5 | 5 | 5 | 5 | 4 | 4 | 2 | 5 | 4 | 5 |
| 54 | 6.3 | 4 | 3 | 4 | 5 | 5 | 5 | 5 | 4 | 4 | 5 | 4 | 5 |
| 56 | 0.4 | 4 | 3 | 5 | 3 | 5 | 5 | 4 | 4 | 0 | 5 | 4 | 5 |
| | 0.8 | 5 | 4 | 5 | 4 | 5 | 5 | 5 | 5 | 0 | 5 | 5 | 5 |
| | 1.6 | 5 | 5 | 5 | 5 | 5 | 5 | 5 | 5 | 1 | 5 | 5 | 5 |
| 57 | 1.6 | 4 | 3 | 4 | 5 | 5 | 5 | 3 | 3 | 1 | 5 | 4 | 5 |
| | 3.2 | 5 | 4 | 5 | 5 | 5 | 5 | 4 | 4 | 2 | 5 | 5 | 5 |
| | 6.3 | 5 | 5 | 5 | 5 | 5 | 5 | 5 | 5 | 3 | 5 | 5 | 5 |
| 59 | 6.3 | 3 | 2 | 5 | 5 | 5 | 5 | 4 | 3 | 0 | 1 | 4 | 5 |
| 61 | 6.3 | 3 | 2 | 5 | 5 | 5 | 5 | 4 | 3 | 0 | 1 | 4 | 2 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| 62 | 6.3 | 5 | 4 | 5 | 5 | 5 | 5 | 5 | 4 | 2 | 5 | 5 | 5 |
| 64 | 6.3 | 3 | 3 | 4 | 5 | 5 | 5 | 0 | 1 | 1 | 3 | 4 | 4 |
| | 1.6 | 2 | 2 | 5 | 3 | 5 | 3 | 4 | 2 | 2 | 0 | 3 | 3 |
| 65 | 3.2 | 3 | 3 | 5 | 4 | 5 | 4 | 5 | 3 | 3 | 1 | 4 | 4 |
| | 6.3 | 4 | 4 | 5 | 5 | 5 | 5 | 5 | 4 | 4 | 2 | 5 | 4 |
| | 1.6 | 2 | 2 | 5 | 2 | 4 | 4 | 3 | 3 | 0 | 0 | 3 | 2 |
| 67 | 3.2 | 3 | 3 | 5 | 3 | 5 | 5 | 4 | 4 | 1 | 1 | 4 | 3 |
| | 6.3 | 4 | 4 | 5 | 4 | 5 | 5 | 4 | 4 | 2 | 2 | 4 | 4 |
| | 1.6 | 1 | 1 | 5 | 5 | 5 | 5 | 5 | 0 | 0 | 2 | 2 | 3 |
| 68 | 3.2 | 2 | 2 | 5 | 5 | 5 | 5 | 5 | 1 | 0 | 3 | 3 | 4 |
| | 6.3 | 3 | 3 | 5 | 5 | 5 | 5 | 5 | 2 | 0 | 4 | 4 | 5 |
| 71 | 6.3 | 5 | 3 | 5 | 5 | 5 | 5 | 5 | 4 | 4 | 5 | 2 | 4 |
| | 1.6 | 3 | 1 | 3 | 5 | 5 | 4 | 5 | 2 | 0 | 5 | 3 | 5 |
| 72 | 3.2 | 4 | 2 | 4 | 5 | 5 | 5 | 5 | 3 | 1 | 5 | 4 | 5 |
| | 6.3 | 5 | 3 | 5 | 5 | 5 | 5 | 5 | 4 | 2 | 5 | 5 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| | 1.6 | 5 | 2 | 5 | 5 | 5 | 5 | 5 | 4 | 3 | 4 | 5 | 5 |
| 73 | 3.2 | 5 | 3 | 5 | 5 | 5 | 5 | 5 | 5 | 4 | 5 | 5 | 5 |
| | 6.3 | 5 | 4 | 5 | 5 | 5 | 5 | 5 | 5 | 5 | 5 | 5 | 5 |
| | 0.8 | 3 | 2 | 3 | 3 | 5 | 5 | 4 | 4 | 4 | 5 | 2 | 3 |
| 74 | 1.6 | 4 | 3 | 4 | 4 | 5 | 5 | 5 | 5 | 5 | 5 | 3 | 4 |
| | 3.2 | 5 | 4 | 5 | 5 | 5 | 5 | 5 | 5 | 5 | 5 | 4 | 5 |
| | 1.6 | 3 | 2 | 4 | 4 | 5 | 3 | 5 | 4 | 4 | 5 | 2 | 4 |
| 75 | 3.2 | 4 | 3 | 5 | 5 | 5 | 4 | 5 | 5 | 5 | 5 | 2 | 5 |
| | 6.3 | 5 | 4 | 5 | 5 | 5 | 5 | 5 | 5 | 5 | 5 | 3 | 5 |
| | 0.4 | 4 | 3 | 4 | 5 | 5 | 5 | 5 | 5 | 5 | 5 | 5 | 5 |
| 77 | 0.8 | 5 | 4 | 5 | 5 | 5 | 5 | 5 | 5 | 5 | 5 | 5 | 5 |
| | 1.6 | 5 | 5 | 5 | 5 | 5 | 5 | 5 | 5 | 5 | 5 | 5 | 5 |

Table 12 (continued)

| Compound No. | Dose | EC | DI | CY | SO | GA | RO | OY | ZE | TR | GY | GO | BE |
|---|---|---|---|---|---|---|---|---|---|---|---|---|---|
| | 0.4 | 3 | 3 | 4 | 4 | 5 | 5 | 5 | 5 | 4 | 5 | 3 | 4 |
| 78 | 0.8 | 4 | 4 | 5 | 5 | 5 | 5 | 5 | 5 | 5 | 5 | 4 | 5 |
| | 1.6 | 5 | 5 | 5 | 5 | 5 | 5 | 5 | 5 | 5 | 5 | 5 | 5 |
| | 0.4 | 2 | 2 | 3 | 5 | 4 | 3 | 3 | 3 | 3 | 4 | 3 | 4 |
| 79 | 0.8 | 3 | 3 | 4 | 5 | 5 | 5 | 4 | 4 | 4 | 5 | 4 | 5 |
| | 1.6 | 4 | 4 | 5 | 5 | 5 | 5 | 5 | 5 | 5 | 5 | 5 | 5 |

## Claims

1. A sultamsulfonamide derivative having the formula:

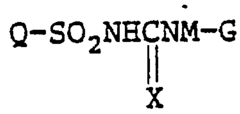

(I)

wherein Q is

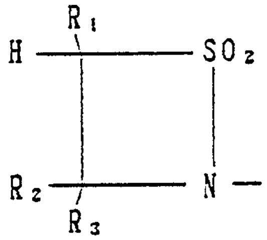

,

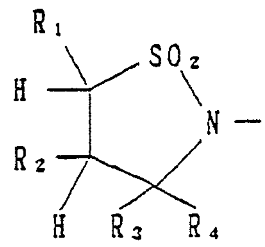

,

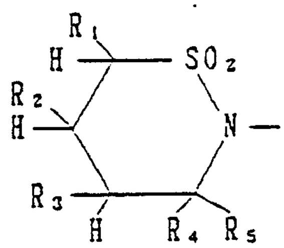

,

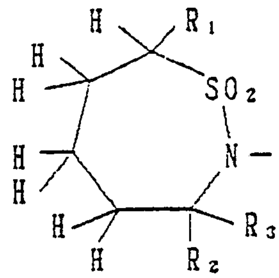

or

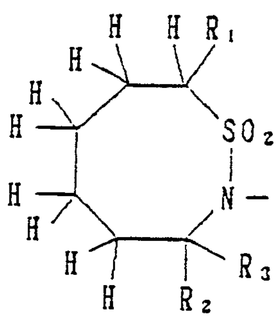

wherein each of $R_1$, $R_2$, $R_3$, $R_4$ and $R_5$ is hydrogen, $C_1$-$C_6$ alkyl, $C_2$-$C_6$ alkenyl, $C_2$-$C_6$ alkynyl, halogen, trifluoromethyl, nitro, cyano, amino, $C_1$-$C_6$ alkoxy, phenoxy (provided that this phenoxy may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylthio, phenylthio (provided that this phenylthio may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylsulfonyl, benzenesulfonyl (provided that this benzenesulfonyl may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylcarbonyl, carboxylic acid, $C_1$-$C_6$ alkoxycarbonyl, $C_1$-$C_6$ monoalkylaminocarbonyl, $C_1$-$C_6$ dialkylaminocarbonyl, $C_2$-$C_6$ acylamino, $C_1$-$C_6$ halogenoalkyl, $C_1$-$C_6$ alkyl substituted by cyano, $C_1$-$C_6$ alkyl substituted by hydroxyl, $C_1$-$C_6$ alkyl substituted by amino, $C_1$-$C_6$ alkyl substituted by $C_1$-$C_6$ alkoxy, $C_1$-$C_6$ alkyl substituted by $C_1$-$C_6$ halogenoalkoxy, $C_1$-$C_6$ alkyl substituted by $C_1$-$C_6$ alkylthio, $C_1$-$C_6$ alkyl substituted by $C_1$-$C_6$ alkylsulfonyl, $C_1$-$C_6$ alkyl substituted by $C_1$-$C_6$ alkylcarbonyl, $C_1$-$C_6$ alkyl substituted by carboxylic acid, $C_1$-$C_6$ alkyl substituted by $C_1$-$C_6$ alkoxycarbonyl, $C_1$-$C_6$ alkyl substituted by $C_2$-$C_6$ alkenyloxy, $C_1$-$C_6$ alkyl substituted by $C_2$-$C_6$ alkynyloxy, $C_1$-$C_6$ alkyl substituted by $C_2$-$C_6$ acylamino, $C_1$-$C_6$ alkyl substituted by $C_1$-$C_6$ alkylsulfonyloxy, $C_2$-$C_6$ alkenyl substituted by $C_1$-$C_6$ alkoxycarbonyl, $C_2$-$C_6$ alkynyl substituted by cyano, phenyl (provided that this phenyl may be substituted by halogen, trifluoromethyl, $C_1$-$C_6$ alkyl, $C_1$-$C_6$ alkoxy or $C_1$-$C_6$ alkoxycarbonyl) or $C_1$-$C_6$ alkyl substituted by phenyl (provided that this phenyl may be substituted by halogen, trifluoromethyl, $C_1$-$C_6$ alkyl, $C_1$-$C_6$ alkoxy or $C_1$-$C_6$ alkoxycarbonyl); X is oxygen or

sulfur; M is hydrogen, $C_1$-$C_6$ alkyl, $C_2$-$C_6$ alkenyl or $C_2$-$C_6$ alkynyl; and G is

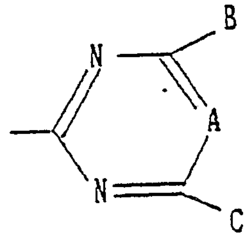

wherein A is CH or nitrogen, and each of B and C is $C_1$-$C_4$ alkyl, $C_1$-$C_4$ alkoxy, $C_1$-$C_4$ halogenoalkyl, $C_1$-$C_4$ halogenoalkoxy, halogen or $C_1$-$C_4$ monoalkylamino, or an agriculturally useful salt thereof.

2. A compound having the formula:

$Q$-$SO_2NH_2$ (II)

wherein Q is

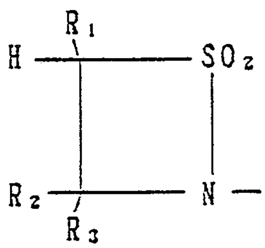

,

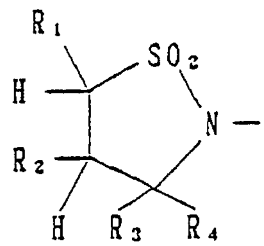

,

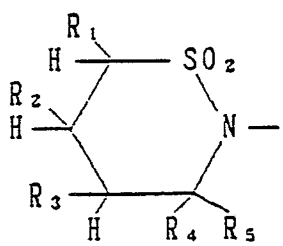

,

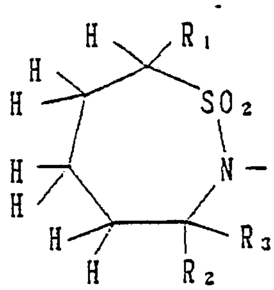

or

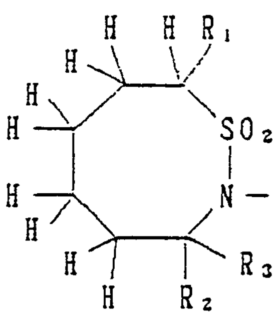

wherein each of $R_1$, $R_2$, $R_3$, $R_4$ and $R_5$ is hydrogen, $C_1$-$C_6$ alkyl, $C_2$-$C_6$ alkenyl, $C_2$-$C_6$ alkynyl, halogen, trifluoromethyl, nitro, cyano, amino, $C_1$-$C_6$ alkoxy, phenoxy (provided that this phenoxy may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylthio, phenylthio (provided that this phenylthio may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylsulfonyl, benzenesulfonyl (provided that this benzenesulfonyl may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylcarbonyl, carboxylic acid, $C_1$-$C_6$ alkoxycarbonyl, $C_1$-$C_6$ monoalkylaminocarbonyl, $C_1$-$C_6$ dialkylaminocarbonyl, $C_2$-$C_6$ acylamino, $C_1$-$C_6$ halogenoalkyl, $C_1$-$C_6$ alkyl substituted by cyano, $C_1$-$C_6$ alkyl substituted by hydroxyl, $C_1$-$C_6$ alkyl substituted by amino, $C_1$-$C_6$ alkyl substituted by $C_1$-$C_6$ alkoxy, $C_1$-$C_6$ alkyl substituted

by $C_1$-$C_6$ halogenoalkoxy, $C_1$-$C_6$ alkyl substituted by $C_1$-$C_6$ alkylthio, $C_1$-$C_6$ alkyl substituted by $C_1$-$C_6$ alkylsulfonyl, $C_1$-$C_6$ alkyl substituted by $C_1$-$C_6$ alkylcarbonyl, $C_1$-$C_6$ alkyl substituted by carboxylic acid, $C_1$-$C_6$ alkyl substituted by $C_1$-$C_6$ alkoxycarbonyl, $C_1$-$C_6$ alkyl substituted by $C_2$-$C_6$ alkenyloxy, $C_1$-$C_6$ alkyl substituted by $C_2$-$C_6$ alkynylcxy, $C_1$-$C_6$ alkyl substituted by $C_2$-$C_6$ acylamino, $C_1$-$C_6$ alkyl substituted by $C_1$-$C_6$ alkylsulfonyloxy, $C_2$-$C_6$ alkenyl substituted by $C_1$-$C_6$ alkoxycarbonyl, $C_2$-$C_6$ alkynyl substituted by cyano, phenyl (provided that this phenyl may be substituted by halogen, trifluoromethyl, $C_1$-$C_6$ alkyl, $C_1$-$C_6$ alkoxy or $C_1$-$C_6$ alkoxycarbonyl) or $C_1$-$C_6$ alkyl substituted by phenyl (provided that this phenyl may be substituted by halogen, trifluoromethyl, $C_1$-$C_6$ alkyl, $C_1$-$C_6$ alkoxy or $C_1$-$C_6$ alkoxycarbonyl).

3. A compound having the formula:

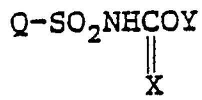

(III)

wherein Q is

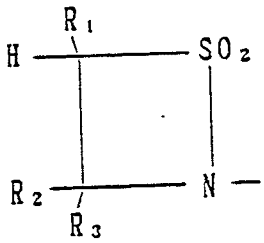

,

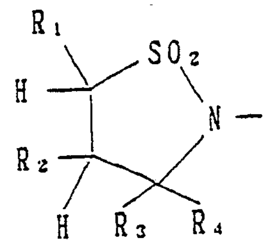

,

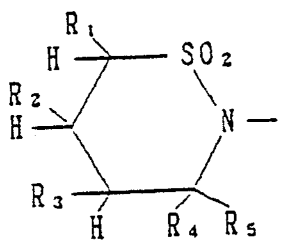

,

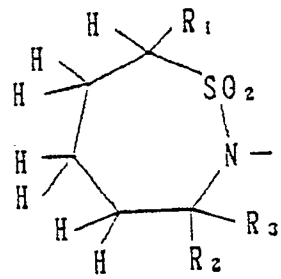

or

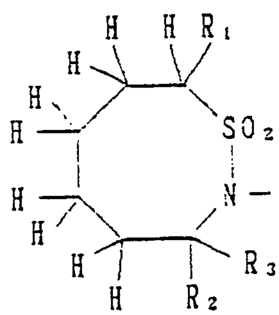

wherein each of $R_1$, $R_2$, $R_3$, $R_4$ and $R_5$ is hydrogen, $C_1$-$C_6$ alkyl, $C_2$-$C_6$ alkenyl, $C_2$-$C_6$ alkynyl, halogen, trifluoromethyl, nitro, cyano, amino, $C_1$-$C_6$ alkoxy, phenoxy (provided that this phenoxy may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylthio, phenylthio (provided that this phenylthio may be substituted by halogen, trifluoromethyl, $C_1$-$C_6$ alkyl, $C_1$-$C_6$ alkoxy or $C_1$-$C_6$ alkoxycarbonyl), $C_1$-$C_6$ alkylsulfonyl, benzenesulfonyl (provided that this benzenesulfonyl may be substituted by halogen, trifluoromethyl, $C_1$-$C_6$ alkyl. $C_1$-$C_6$ alkoxy or $C_1$-$C_6$ alkoxycarbonyl), $C_1$-$C_6$ alkylcarbonyl, carboxylic acid, $C_1$-$C_6$ alkoxycarbonyl, $C_1$-$C_6$ monoalkylaminocarbonyl, $C_1$-$C_6$ dialkylaminocarbonyl, $C_2$-$C_6$ acylamino, $C_1$-$C_6$ halogenoalkyl, $C_1$-$C_6$ alkyl substituted by cyano, $C_1$-$C_6$ alkyl substituted by hydroxyl, $C_1$-$C_6$ alkyl substituted by amino, $C_1$-$C_6$ alkyl substituted by $C_1$-$C_6$ alkoxy, $C_1$-$C_6$ alkyl substituted by $C_1$-$C_6$ halogenoalkoxy, $C_1$-$C_6$ alkyl substituted by $C_1$-$C_6$ alkylthio, $C_1$-$C_6$ alkyl substituted by $C_1$-$C_6$ alkylsulfonyl, $C_1$-$C_6$ alkyl substituted by $C_1$-$C_6$ alkylcarbonyl, $C_1$-$C_6$ alkyl substituted by carboxylic acid, $C_1$-$C_6$ alkyl substituted by $C_1$-$C_6$ alkoxycarbonyl, $C_1$-$C_6$ alkyl substituted by $C_2$-$C_6$ alkenyloxy, $C_1$-$C_6$ alkyl

substituted by $C_2$-$C_6$ alkynyloxy, $C_1$-$C_6$ alkyl substituted by $C_2$-$C_6$ acylamino, $C_1$-$C_6$ alkyl substituted by $C_1$-$C_6$ alkylsulfonyloxy, $C_2$-$C_6$ alkenyl substituted by $C_1$-$C_6$ alkoxycarbonyl, $C_2$-$C_6$ alkynyl substituted by cyano, phenyl (provided that this phenyl may be substituted by halogen, trifluoromethyl, $C_1$-$C_6$ alkyl, $C_1$-$C_6$ alkoxy or $C_1$-$C_6$ alkoxycarbonyl) or $C_1$-$C_6$ alkyl substituted by phenyl (provided that this phenyl may be substituted by halogen, trifluoromethyl, $C_1$-$C_6$ alkyl, $C_1$-$C_6$ alkoxy or $C_1$-$C_6$ alkoxycarbonyl); X is oxygen or sulfur; and Y is $C_1$-$C_6$ alkyl or phenyl.

4. A selective herbicide comprising a herbicidally effective amount of a compound having the formula I as defined in Claim 1 or its salt and an agricultural carrier or diluent.